# EUROPEAN PATENT SPECIFICATION

(11) **EP 2 198 005 B1**
(45) Date of publication and mention of the grant of the patent: **07.05.2014**
(21) Application number: 08835263.8
(22) Date of filing: 03.10.2008
(51) Int. Cl.: C12N 1/00, C12P 7/64, C12N 15/00

(54) **PEROXISOME BIOGENESIS FACTOR PROTEIN (PEX) DISRUPTIONS FOR ALTERING THE CONTENT OF POLYUNSATURATED FATTY ACIDS AND THE TOTAL LIPID CONTENT IN OLEAGINOUS EUKARYOTIC ORGANISMS**
PEROXISOMALES BIOGENESE-FAKTORPROTEIN (PEX)-INAKTIVIERUNGEN ZUR ÄNDERUNG DES GEHALTS AN MEHRFACH UNGESÄTTIGTEN FETTSÄUREN UND DES GESAMTFETTGEHALTS IN ÖLIGEN EUKARYOTEN ORGANISMEN
PERTURBATIONS DES PROTÉINES DES FACTEURS DE BIOGENÈSE DU PEROXYSOME (PEX) POUR MODIFIER LA TENEUR EN ACIDES GRAS POLYINSATURÉS ET LA TENEUR EN LIPIDES TOTAUX DANS DES ORGANISMES EUCARYOTES OLÉAGINEUX

(30) Priority: 03.10.2007 US 977174 P
(43) Date of publication of application: 23.06.2010
(73) Proprietor: E. I. du Pont de Nemours and Company, Wilmington, DE 19898 (US)
(72) Inventor: XUE, Zhixiong, Chadds Ford, Pennsylvania 19317-9728 (US); ZHU, Quinn, Qun, West Chester, Pennsylvania 19382 (US); YADAV, Narendra, S., Chadds Ford, Pennsylvania 19317 (US); SHARPE, Pamela, L., Wilmington, Delaware 19808 (US); HONG, Seung-Pyo, Hockessin, Delaware 19707 (US)
(74) Representative: Dzieglewska, Hanna Eva
(86) International application number: PCT/US2008/078671
(87) International publication number: WO 2009/046248

(56) References cited:
- WO-A-2005/003322
- US-A1- 2005 043 527
- LIN YUN ET AL: "The peroxisome deficient Arabidopsis mutant sse1 exhibits impaired fatty acid synthesis" PLANT PHYSIOLOGY (ROCKVILLE), vol. 135, no. 2, June 2004 (2004-06), pages 814-827, XP002506374 ISSN: 0032-0889 cited in the application
- SUMITA TORU ET AL: "Peroxisome deficiency represses the expression of n-alkane-inducible YlALK1 encoding cytochrome P450ALK1 in Yarrowia lipolytica" FEMS MICROBIOLOGY LETTERS, vol. 214, no. 1, 27 August 2002 (2002-08-27), pages 31-38, XP002506375 ISSN: 0378-1097
- EITZEN GARY A ET AL: "Enlarged peroxisomes are present in oleic acid-grown Yarrowia lipolytica overexpressing the PEX16 gene encoding an intraperoxisomal peripheral membrane peroxine" JOURNAL OF CELL BIOLOGY, vol. 137, no. 6, 1997, pages 1265-1278, XP002506376 ISSN: 0021-9525 cited in the application
- BASCOM ROGER A ET AL: "Peroxisome biogenesis occurs in an unsynchronized manner in close association with the endoplasmic reticulum in temperature-sensitive Yarrowia lipolytica Pex3p mutants." MOLECULAR BIOLOGY OF THE CELL, vol. 14, no. 3, March 2003 (2003-03), pages 939-957, XP002506377 ISSN: 1059-1524 cited in the application

## Description

### FIELD OF THE INVENTION

This invention is in the field of biotechnology. More specifically, this invention pertains to methods useful for manipulating the polyunsaturated fatty acid (PUFA) composition and lipid content of eukaryotic organisms, based on disruption of peroxisome biogenesis factor (Pex) proteins.

### BACKGROUND OF THE INVENTION

The health benefits associated with polyunsaturated fatty acids ["PUFAs"], especially ω-3 and ω-6 PUFAs, have been well documented. In order to find ways to produce large-scale quantities of ω-3 and ω-6 PUFAs, researchers have directed their work toward the discovery of genes and the understanding of the encoded biosynthetic pathways that result in lipids and fatty acids.

One effort to produce these PUFAs has introduced ω-3/ω-6 PUFA biosynthetic pathways into organisms that do not natively produce ω-3/ω-6 PUFAs. One such organism that has been extensively manipulated is the non-oleaginous yeast, *Saccharomyces cerevisiae.* However, none of the preliminary results demonstrating limited production of linoleic acid ["LA"], γ-linolenic acid ["GLA"], α-linolenic acid ["ALA"], stearidonic acid ["STA"] and/or eicosapentaenoic acid ["EPA"] are suitable for commercial exploitation.

Other efforts to produce large-scale quantities of ω-3/ω-6 PUFAs have cultivated microbial organisms that natively produce the fatty acid of choice, e.g., heterotrophic diatoms *Cyclotella* sp. and *Nitzschia* sp., *Pseudomonas, Alteromonas* or *Shewanella* species, filamentous fungi of the genus *Pythium,* or *Mortierella elongata, M. exigua* or *M*. *hygrophila.*

All these efforts suffer from an inability to substantially improve the yield of oil or to control the characteristics of the oil composition produced, since the fermentations rely on the natural abilities of the microbes themselves.

Commonly owned U.S. Pat. No. 7,238,482 describes the use of the oleaginous yeast *Yarrowia lipolytica* as a production host for the production of PUFAs. Oleaginous yeast are defined as those yeast that are naturally capable of oil synthesis and accumulation, where greater than 25% of the cellular dry weight is typical. Optimization of the production host has been described in the art (see for example Int'l. App. Pub. No. WO 2006/033723, U.S. Pat. App. Pub. No. 2006-0094092, U.S. Pat. App. Pub. No. 2006-0115881, and U.S. Pat. App. Pub. No. 2006-0110806). The recombinant strains described therein comprise various chimeric genes expressing multiple copies of heterologous desaturases, elongases and acyltransferases and optionally comprise various native desaturase and acyltransferase knockouts to enable PUFA synthesis and accumulation. Further optimization of the host cell is needed for commercial production of PUFAs.

Lin Y. et al suggest that peroxisomes are required for both catabolic and anabolic lipid metabolism (Plant Physiology, 135:814-827 (2004)). However, this hypothesis was based on studies with a homolog of Pex16p in *Arabidopsis* mutants that had both abnormal peroxisome biogenesis and fatty acid synthesis (i.e., a reduction of oil to approximately 10-16% of wild type in *sse1* seeds was reported). Binns, D. et al. (J. Cell Biol., 173(5):719-731 (2006)) also document an intimate collaboration between peroxisomes and lipid bodies in *Saccharomyces cerevisiae.* But, previous studies of Pex knockouts have not been performed in a PUFA-producing organism.

Applicants have solved the stated problem of optimizing host cells for commercial production of PUFAs by the unpredictable mechanism of disruption of peroxisome biogenesis factor proteins in a PUFA-producing orgamism, which leads to the unpredictable result of an increase in the amount of PUFAs, as a percent of total fatty acids, in a recombinant PUFA-producing strain of *Y. lipolytica.* Novel strains containing disruptions in peroxisome biogenesis factor proteins are described herein.

### SUMMARY OF THE INVENTION

Described herein are methods of increasing the weight percent of at least one polyunsaturated fatty acid ["PUFA"] relative to the weight percent of total fatty acids ["TFAs"] in an oleaginous eukaryotic organism having a total lipid content, a total lipid fraction and an oil fraction, comprising:
a) providing an oleaginous eukaryotic organism comprising:
   1) genes encoding a functional polyunsaturated fatty acid biosynthetic pathway; and
   2) a disruption in a native gene encoding a peroxisome biogenesis factor protein, thereby providing a PEX-disrupted organism, and
b) growing the PEX-disrupted organism under conditions as to increase the weight percent of at least one polyunsaturated fatty acid relative to the weight percent of total fatty acids in the total lipid fraction or in the oil fraction, when compared to the weight percent of the at least one polyunsaturated fatty acid relative to the weight percent of total fatty acids in the total lipid fraction or in the oil fraction in the oleaginous eukaryotic organism in which no native gene encoding a peroxisome biogenesis factor protein has been disrupted.

This method of increasing may also be used to increase the percent of at least one polyunsaturated fatty acid ["PUFA"] relative to the dry cell weight ["DCW"] by applying the same steps (a) and (b).

In some of the methods described here, the weight percent of the PUFA relative to the weight percent of the TFAs is increased at least 1.3 fold.

In some of the described methods, the total lipid content in the PEX-disrupted organism may be increased or decreased compared with that of an oleaginous eukaryote having no disruption in a native PEX gene.

In any of these methods, the increased PUFA may be a single PUFA or a combination of PUFAs. In either case, the increased PUFA or increased combination of PUFAs can include linoleic acid, conjugated linoleic acid, γ-linolenic acid, dihomo-γ-linolenic acid, arachidonic acid, docosatetraenoic acid, ω-6 docosapentaenoic acid, α-linolenic acid, stearidonic acid, eicosatetraenoic acid, eicosapentaenoic acid, ω-3 docosapentaenoic acid, eicosadienoic acid, eicosatrienoic acid, docosahexaenoic acid, hydroxylated or expoxy fatty acids of these, a C₁₈ polyunsaturated fatty acid or a combination of these, a C₂₀ polyunsaturated fatty acid or a combination of these, a combination of C₂₀-₂₂ polyunsaturated fatty acids and a C₂₂ polyunsaturated fatty acid or a combination of these.

In any of these methods, the PEX-disrupted organism may be a member of the following: *Yarrowia, Candida, Rhodotorula, Rhodosporidium, Cryptococcus, Trichosporon, Lipomyces, Mortierella Thraustochytrium, Schizochytrium,* and *Saccharomyces* having the property of oleaginy. And, in any of the described methods, the PUFA biosynthetic pathway includes genes that encodes any or a combination of the following enzymes: Δ9 desaturase, Δ12 desaturase, Δ6 desaturase, Δ5 desaturase, Δ17 desaturase, Δ8 desaturase, Δ15 desaturase, Δ4 desaturase, C_{14/16}elongase, C_{16/18}elongase, C_{18/20} elongase, C_{20/22} elongase and Δ9 elongase.

The disruption may occur in a PEX gene that encodes a peroxisome biogenesis factor protein that includes the following: Pex1p, Pex 2p, Pex3p, Pex3Bp, Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21p, Pex21 Bp, Pex22p, Pex22p-like and Pex26p. And in any of these methods, the disruption may be a gene knockout or a deletion in a portion of the gene that encodes the C-terminal portion of the protein. In some of these methods, the deletion is in the portion of the gene encoding the C-terminal portion of the C₃HC₄ zinc ring finger motif of the protein.

Also described herein is the oil fraction or the total lipid fraction in a PEX-disrupted organism, which has experienced an increase in the weight percent of at least one PUFA accomolished by the method of Claim 1. Described herein is also a PEX-disrupted *Yarrowia lipolytica,* having a disruption in a native gene encoding Pex3p or Pex10p or Pex16p. This *Y*. *lipolytica* may have ATCC designation ATCC PTA-8614 (strain Y4128).

### BIOLOGICAL DEPOSITS

The following biological materials have been deposited with the American Type Culture Collection (ATCC), 10801 University Boulevard, Manassas, VA 20110-2209, and bear the following designations, accession numbers and dates of deposit.

| Biological Material | Accession No. | Date of Deposit |
|---|---|---|
| *Yarrowia lipolytica* Y2047 | ATCC PTA-7186 | October 26, 2005 |
| *Yarrowia lipolytica* Y2201 | ATCC PTA-7185 | October 26, 2005 |
| *Yarrowia lipolytica* Y2096 | ATCC PTA-7184 | October 26, 2005 |
| *Yarrowia lipolytica* Y3000 | ATCC PTA-7187 | October 26, 2005 |
| *Yarrowia lipolytica* Y4128 | ATCC PTA-8614 | August 23, 2007 |
| *Yarrowia lipolytica* Y4127 | ATCC PTA-8802 | November 29, 2007 |

The biological materials listed above were deposited under the terms of the Budapest Treaty on the International Recognition of the Deposit of Microorganisms for the Purposes of Patent Procedure. The listed deposit will be maintained in the indicated international depository for at least 30 years and will be made available to the public upon the grant of a patent disclosing it. The availability of a deposit does not constitute a license to practice the subject invention in derogation of patent rights granted by government action.

### BRIEF DESCRIPTION OF THE DRAWINGS AND SEQUENCE LISTINGS

FIG. 1 consists of FIG. 1A and FIG 1B, which together illustrate the ω-3/ω-6 fatty acid biosynthetic pathway, and should be viewed together when considering the description of this pathway below.
FIG. 2A provides an alignment of the C₃HC₄ zinc ring finger motifs of the *Yarrowia lipolytica* Pex10p (i.e., amino acids 327-364 of SEQ ID NO:10 [GenBank Accession No. CAG81606]), the *Yarrowia lipolytica* Pex2p (i.e., amino acids 266-323 of SEQ ID NO:2 [GenBank Accession No. CAG77647]) and the *Yarrowia lipolytica* Pex12p (i.e., amino acids 342-391 of SEQ ID NO:11 [GenBank Accession No. CAG81532]), with cysteine and histidine residues of the conserved C₃HC₄ zinc ring finger motif indicated by astericks.
FIG. 2B schematically illustrates the proposed interaction between various amino acid residues of the *Y. lipolytica* Pex10p C₃HC₄ finger motif and the two zinc ions to which they bind.
FIG. 3A diagrams the development of *Yarrowia lipolytica* strain Y4128, producing 37.6% EPA in the total lipid fraction.
FIG. 3B provides a plasmid map for pZP3-Pa777U.
FIG. 4 provides plasmid maps for the following: (A) pY117; and, (B) pZP2-2988.
FIG. 5 provides plasmid maps for the following: (A) pZKUE3S; and, (B) pFBAIN-MOD-1.
FIG. 6 provides plasmid maps for the following: (A) pFBAIN-PEX10; and, (B) pEXP-MOD-1.
FIG. 7A provides a plasmid map for pPEX10-1. FIG. 7B diagrams the development of *Yarrowia lipolytica* strain Y4184U.
FIG. 8 provides plasmid maps for the following: (A) pZKL1-2SP98C; and, (B) pZKL2-5U89GC.
FIG. 9 provides plasmid maps for the following: (A) pYPS161; and, (B) pYRH13.
FIG. 10 diagrams the development of *Yarrowia lipolytica* strain Y4305U3.
FIG. 11 provides plasmid maps for the following: (A) pZKUM; and, (B) pZKD2-5U89A2.
FIG. 12 provides plasmid maps for the following: (A) pY87; and, (B) pY157.

The invention can be more fully understood from the following detailed description and the accompanying sequence descriptions, which form a part of this application.

The following sequences comply with 37 C.F.R. §1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.

SEQ ID NOs:1-86 are primers, ORFs encoding genes or proteins (or portions thereof), or plasmids, as identified in Table 1.

**Table 1**

| Summary Of Nucleic Acid And Protein SEQ ID Numbers | | |
|---|---|---|
| **Description and Abbreviation** | **Nucleic acid SEQ ID NO.** | **Protein SEQ ID NO.** |
| *Yarrowia lipolytica* Pex1 p (GenBank Accession No. CAG82178) | -- | 1 (1024 AA) |
| *Yarrowia lipolytica* Pex2p (GenBank Accession No. CAG77647) | -- | 2 (381 ALA) |
| *Yarrowia lipolytica* Pex3p (GenBank Accession No. CAG78565) | -- | 3 (431 AA) |
| *Yarrowia lipolytica* Pex3Bp (GenBank Accession No. CAG83356) | -- | 4 (395 AA) |
| *Yarrowia lipolytica* Pex4p (GenBank Accession No. CAG79130) | -- | 5 (153 AA) |
| *Yarrowia lipolytica* Pex5p (GenBank Accession No. CAG78803) | -- | 6 (598 AA) |
| *Yarrowia lipolytica* Pex6p (GenBank Accession No. CAG82306) | -- | 7 (1024 AA) |
| *Yarrowia lipolytica* Pex7p (GenBank Accession No. CAG78389) | -- | 8 (356 AA) |
| *Yarrovvia lipolytica* Pex8p (GenBank Accession No. CAG80447) | -- | 9 (671 AA) |
| *Yarrowia lipolytica* Pex10p (GenBank Accession No. CAG81606) | -- | 10 (377 AA) |
| *Yarrowia lipolytica* Pex12p (GenBank Accession No. CAG81532) | -- | 11 (408 AA) |
| *Yarrowia lipolytica* Pex13p (GenBank Accession No. CAG81789) | -- | 12 (412 AA) |
| *Yarrowia lipolytica* Pex14p (GenBank Accession No. CAG79323) | -- | 13 (380 AA) |
| *Yarrowia lipolytica* Pex16p (GenBank Accession No. CAG79622) | -- | 14 (391 AA) |
| *Yarrowia lipolytica* Pex17p (GenBank Accession No. CAG84025) | -- | 15 (225 AA) |
| *Yarrowia lipolytica* Pex19p (GenBank Accession No. AAK84827) | -- | 16 (324 AA) |
| *Yarrowia lipolytica* Pex20p (GenBank | -- | 17 |
| Accession No. CAG79226) | | (417 AA) |
| *Yarrowia lipolytica* Pex22p (GenBank Accession No. CAG77876) | - | 18 (195 AA) |
| *Yarrowia lipolytica* Pex26p (GenBank Accession No. NC_006072, antisense translation of nucleotides 117230-118387) | - | 19 (386 AA) |
| Contig comprising *Yarrowia lipolytica* Pex10 gene encoding peroxisomal biogenesis factor protein (Pex10p) (GenBank Accession No. AB036770) | 20 (3387 bp) | - |
| *Yarrowia lipolytica* Pex10 (GenBank Accession No. AB036770, nucleotides 1038-2171) (the protein sequence is 100% identical to SEQ ID NO:10) | 21 (1134 bp) | 22 (377 AA) |
| *Yarrowia lipolytica* Pex10 (GenBank Accession No. AJ012084, which corresponds to nucleotides 1107-2171 of GenBank Accession No. AB036770) (the first 23 amino acids are truncated with respect to the protein sequences of SEQ ID NOs:10 and 22) | 23 (1065 bp) | 24 (354 AA) |
| *Yarrowia lipolytica* Pex10p C₃HC₄ zinc ring finger motif (i.e., amino acids 327-364 of SEQ ID NO:10) | - | 25 (38 AA) |
| *Yarrowia lipolytica* truncated Pex10p (GenBank Accession No. CAG81606 [SEQ ID NO:10], with C-terminal 32 amino acid deletion) | - | 26 (345 AA) |
| *Yarrowia lipolytica* mutant acetohydroxyacid synthase (AHAS) gene comprising a W497L mutation | 27 (2987 bp) | -- |
| Plasmid pZP3-Pa777U | 28 (13,066 bp) | -- |
| Plasmid pY117 | 29 (9570 bp) | -- |
| Plasmid pZP2-2988 | 30 (15,743 bp) | -- |
| Plasmid pZKUE3S | (6303 bp) | 31 |
| Primer pZP-GW-5-1 | 32 | -- |
| Primer pZP-GW-5-2 | 33 | |
| Primer pZP-GW-5-3 | 34 | -- |
| Primer pZP-GW-5-4 | 35 | -- |
| Primer pZP-GW-3-1 | 36 | -- |
| Primer pZP-GW-3-2 | 37 | -- |
| Primer pZP-GW-3-3 | 38 | -- |
| Primer pZP-GW-3-4 | 39 | -- |
| Genome Walker adaptor [top strand] | 40 | - |
| Genome Walker adaptor [bottom strand] | 41 | - |
| Nested adaptor primer | 42 | -- |
| Primer Per10 F1 | 43 | -- |
| Primer ZPGW-5-5 | 44 | -- |
| Primer Per10 R | 45 | -- |
| Plasmid pFBAIN-MOD-1 | 46 (7222 bp) | -- |
| Plasmid pFBAIn-PEX10 | 47 (8133 bp) | -- |
| Primer PEX10-R-BsiWI | 48 | -- |
| Primer PEX10-F1-Sall | 49 | -- |
| Primer PEX10-F2-Sall | 50 | -- |
| Plasmid pEXP-MOD1 | 51 (7277 bp) | -- |
| Plasmid pPEX10-1 | 52 (7559 bp) | -- |
| Plasmid pPEX10-2 | 53 (8051 bp) | -- |
| Plasmid pZKL1-2SP98C | 54 (15,877 bp) | -- |
| Plasmid pZKL2-5U89GC | 55 (15,812 bp) | -- |
| Plasmid pYPS161 | 56 (7966 bp) | -- |
| Primer Pex-10del1 3'.Forward | -- | -- |
| Primer Pex-10del2 5'.Reverse | 58 | -- |
| Plasmid pYRH13 | 59 (8673 bp) | -- |
| Primer PEX16Fii | 60 | -- |
| Primer PEX16Rii | 61 | -- |
| Primer 3UTR-URA3 | 62 | -- |
| Primer Pex16-conf | 63 | -- |
| Real time PCR primer ef-324F | 64 | -- |
| Real time PCR primer ef-392R | 65 | -- |
| Real time PCR primer Pex16-741 F | 66 | -- |
| Real time PCR primer Pex16-802R | 67 | -- |
| Nucleotide portion of TaqMan probe ef-345T | 68 | -- |
| Nucleotide portion of TaqMan probe PEX16-760T | 69 | -- |
| Plasmid pZKUM | 70 (4313 bp) | -- |
| Plasmid pZKD2-5U89A2 | 71 (15,966 bp) | |
| *Yarrowia lipolytica* diacylglycerol acyltransferase (DGAT2) (U.S. Patent 7,267,976) | 72 (2119 bp) | 73 (514 AA) |
| Synthetic Δ12 desaturase derived from *Fusarium moniliforme,* codon-optimized for expression in *Yarrowia lipolytica* ("FmD12S") | 74 (1434 bp) | 75 (477 AA) |
| Synthetic mutant Δ8 desaturase ("EgD8M"), derived from *Euglena gracilis* ("EgD8S"; U.S. Patent 7,256,033) | 76 (1272 bp) | 77 (422 AA) |
| Synthetic Δ9 elongase derived from *Eutreptiella* sp. CCMP389 codon-optimized for expression in *Yarrowia lipolytica* ("E389D9eS") | 78 (792 bp) | 79 (263 AA) |
| Synthetic Δ5 desaturase derived from *Euglena gracilis,* codon-optimized for expression in *Yarrowia lipolytica* ("EgD5S") | 80 (1350 bp) | 81 (449 AA) |
| Plasmid pY157 | 82 (6356 bp) | -- |
| Plasmid pY87 | 83 (5910 bp) | -- |
| *Escherichia coli* LoxP recombination site, recognized by a Cre recombinase enzyme | 84 (34 bp) | -- |
| Primer UP 768 | 85 | -- |
| Primer LP 769 | 86 | -- |

### DETAILED DESCRIPTION OF THE INVENTION

Described herein are generalized methods to manipulate the concentration (as a percent of total fatty acids) and content (as a percent of the dry cell weight) of long-chain polyunsaturated fatty acids ["LC- PUFAs"] in PUFA-producing eukaryotic organisms. These methods rely on disruption of a native peroxisome biogenesis factor ["Pex"] protein within the host having native or genetically-engineered ability to produce PUFAs, including fungi and yeast.

PUFAs, or derivatives thereof, are used as dietary substitutes, or supplements, particularly infant formulas, for patients undergoing intravenous feeding or for preventing or treating malnutrition. For example, PUFAs may be incorporated into cooking oils, fats or margarines and ingested as part of a consumer's typical diet, thereby giving the consumer desired dietary supplementation. Further, PUFAs may also be incorporated into infant formulas, nutritional supplements or other food products and may find use as anti-inflammatory or cholesterol lowering agents. Optionally, the compositions may be used for pharmaceutical use, either human or veterinary.

### Definitions

In this disclosure, a number of terms and abbreviations are used. The following definitions are provided.

"Open reading frame" is abbreviated as "ORF".

"Polymerase chain reaction" is abbreviated as "PCR".

"American Type Culture Collection" is abbreviated as "ATCC".

"Polyunsaturated fatty acid(s)" is abbreviated as "PUFA(s)".

"Triacylglycerols" are abbreviated as "TAGs".

"Total fatty acids" are abbreviated as "TFAs".

"Fatty acid methyl esters" are abbreviated as "FAMEs".

"Dry cell weight" is abbreviated as "DCW".

The term "invention" or "present invention" as used herein is not meant to be limiting but applies generally to any of the inventions defined in the claims or described herein.

The term "peroxisomes" refers to ubiquitous organelles found in all eukaryotic cells. They have a single lipid bilayer membrane that separates their contents from the cytosol and that contains various membrane proteins essential to the functions described below. Peroxisomes selectively import proteins via an "extended shuttle mechanism". More specifically, there are at least 32 known peroxisomal proteins, also known as peroxins, which participate in the process of importing proteins by means of ATP hydrolysis through the peroxisomal membrane. Some peroxins comprise a specific protein signal, i.e., a peroxisomal targeting signal or "PTS", at either the N-terminus or C-terminus to signal that importation through the peroxisomal membrane should occur. Once cellular proteins are imported into the peroxisome, they are typically subjected to some means of degradation. For example, peroxisomes contain oxidative enzymes, such as catalase, D-amino acid oxidase and uric acid oxidase, that enable degradation of substances that are toxic to the cell. Alternatively, peroxisomes breakdown fatty acid molecules to produce free molecules of acetyl-CoA which are exported back to the cytosol, in a process called β-oxidation.

The terms "peroxisome biogenesis factor protein", "peroxin" and "Pex protein" are interchangeable and refer to proteins involved in peroxisome biogenesis and/or that participate in the process of importing cellular proteins by means of ATP hydrolysis through the peroxisomal membrane. The acronym of a gene that encodes any of these proteins is "Pex gene". A system for nomenclature of Pex genes is described by Distel et al., J. Cell Biol., 135:1-3 (1996). At least 32 different Pex genes have been identified so far in various eukaryotic organisms. Many Pex genes have been isolated from the analysis of mutants that demonstrated abnormal peroxisomal functions or structures. Based on a review by Kiel, J. A. K. W., et al. (Traffic, 7:1291-1303 (2006)), wherein *in silico* analysis of the genomic sequences of 17 different fungal species was performed, the following Pex proteins were identified: Pex1p, Pex2p, Pex3p, Pex3Bp, Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21 p, Pex21 Bp, Pex22p, Pex22p-like and Pex26p. Thus, each of these proteins is referred to herein as a "Pex protein", a "peroxin" or a "peroxisome biogenesis factor protein", and is encoded by at least one "Pex gene".

The term "conserved domain" or "motif' refers to a set of amino acids conserved at specific positions along an aligned sequence of evolutionarily related proteins. While amino acids at other positions can vary between homologous proteins, amino acids that are highly conserved at specific positions indicate amino acids that are essential in the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family. Of relevance herein, Pex2p, Pex10p and Pex12p all share a cysteine-rich motif near their carboxyl termini, known as a C₃HC₄ zinc ring finger motif. This motif appears to be required for their activities, involved in protein docking and translocation into the peroxisome (Kiel, J. A. K. W., et al., Traffic, 7:1291-1303 (2006)).

The term "C₃HC₄ zinc ring finger motif" or "C₃HC₄ motif' generically refers to a conserved cysteine-rich motif that binds two zinc ions, identified by the presence of a sequence of amino acids as set forth in Formula I:

**Formula I:** CX₂CX₉₋₂₇CX₁₋₃HX₂CX₂CX₄₋₄₈CX₂C

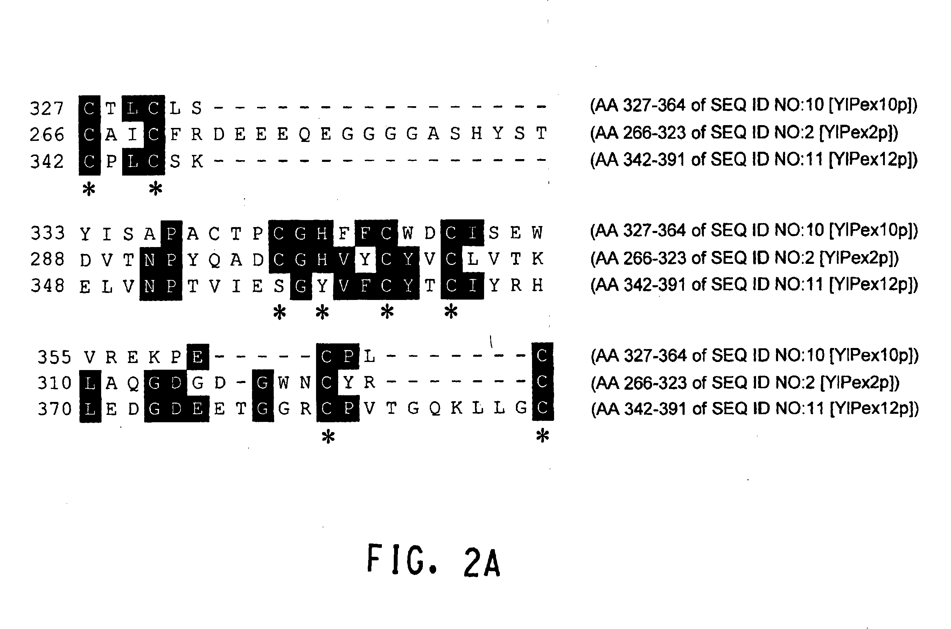

The C₃HC₄ zinc ring finger motif within the *Yarrowia lipolytica* gene encoding the peroxisome biogenesis factor 10 protein, i.e., YlPex10p, is located between amino acids 327-364 of SEQ ID NO:10 and is defined by a CX₂CX₁₁CX₁HX₂CX₂CX₁₀CX₂C motif (SEQ ID NO:25). The C₃HC₄ zinc ring finger motif within the *Y*. *lipolytica* gene encoding the peroxisome biogenesis factor 2 protein, i.e., YlPex2p, is located between amino acids 266-323 of SEQ ID NO:2. The *Y. lipolytica* peroxisome biogenesis factor 12 protein, i.e., YlPex12p, contains an imperfect C₃HC₄ ring-finger motif located between amino acids 342-391 of SEQ ID NO:11. The protein sequences corresponding to the C₃HC₄ zinc ring finger motif of YlPex10, YlPex2 and YlPex12 are aligned in FIG. 2A; asterisks denote the conserved cysteine or histidine residues of the motif.

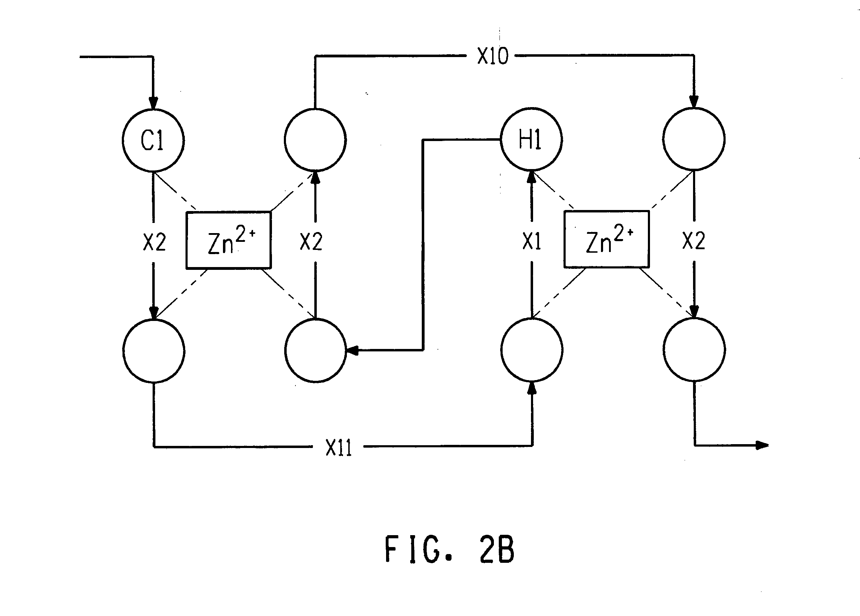

YlPex10, YlPex2 and YlPex12 are thought to form a ring finger complex by protein-protein interaction. The proposed interaction between the cystine and histidine residues of the YlPex10p C₃HC₄ finger motif with two zinc residues is schematically diagrammed in FIG. 2B.

The term "Pex10" refers to the gene encoding the peroxisome biogenesis factor 10 protein or peroxisomal assembly protein Peroxin 10, wherein the peroxin protein is hereinafter referred to as "Pex10p". The function of Pex10p has not been clearly elucidated, although studies in other organisms have revealed that Pex10 products are localized in the peroxisomal membrane and are essential to the normal functioning of the organelle. A C₃HC₄ zinc ring finger motif appears to be conserved in the C-terminal region of Pex10p (Kalish, J. E. et al., Mol. Cell Biol., 15:6406-6419 (1995); Tan, X. et al., J. Cell Biol., 128:307-319 (1995); Warren, D. S., et al., Am. J. Hum. Genet., 63:347-359 (1998)) and is required for enzymatic activity.

The term "YlPex10" refers to the *Yarrowia lipolytica* gene encoding the peroxisome biogenesis factor 10 protein, wherein the protein is hereinafter referred to as "YlPex10p". This particular peroxin was recently studied by Sumita et al. (FEMS Microbiol. Lett., 214:31-38 (2002)). The nucleotide sequence of YlPex10 was registered in GenBank under multiple accession numbers, including GenBank Accession No. CAG81606 (SEQ ID NO:10), No. AB036770 (SEQ ID NOs:20, 21 and 22) and No. AJ012084 (SEQ ID NOs:23 and 24). The YlPex10p sequence set forth in SEQ ID NO:24 is 354 amino acids in length. In contrast, the YlPex10p sequences set forth in SEQ ID NO:10 and SEQ ID NO:22 are each 377 amino acids in length, as the 100% identical sequences possess an additional 23 amino acids at the N-terminus of the protein (corresponding to a different start codon than that identified in GenBank Accession No. AJ012084 (SEQ ID NO:24)).

The term "Pex3" refers to the gene encoding the peroxisome biogenesis factor 3 protein or peroxisomal assembly protein Peroxin 3, wherein the peroxin protein is hereinafter referred to as "Pex3p". Although mechanistic details concerning the function of Pex3p have not been clearly resolved, it is clear that Pex3p is a peroxisomal integral membrane protein required early in peroxisome biogenesis for formation of the peroxisomal membrane (see, e.g., Baerends, R. J. et al., J. Biol. Chem., 271:8887-8894 (1996); Bascom, R.A. et al, Mol. Biol. Cell, 14:939-957 (2003)).

The term "YlPex3" refers to the *Yarrowia lipolytica* gene encoding the peroxisome biogenesis factor 3 protein, wherein the protein is hereinafter referred to as "YlPex3p". The nucleotide sequence of YlPex3 was registered in GenBank as Accession No. CAG78565 (SEQ ID NO:3).

The term "Pex16" refers to the gene encoding the peroxisome biogenesis factor 16 protein or peroxisomal assembly protein Peroxin 16, wherein the peroxin protein is hereinafter referred to as "Pex16p". The function of Pex16p has not been clearly elucidated, although studies in various organisms have revealed that Pex16 products play a role in the formation of the peroxisomal membrane and regulation of peroxisomal proliferation (Platta, H.W. and R. Erdmann, Trends Cell Biol., 17(10):474-484 (2007)).

The term "YlPex16" refers to the *Yarrowia lipolytica* gene encoding the peroxisome biogenesis factor 16 protein, wherein the protein is hereinafter referred to as "YlPex16p". This particular peroxin was described by Elizen G. A., et al. (J. Cell Biol., 137:1265-1278 (1997)) and Titorenko, V. I. et al. (Mol. Cell Biol., 17:5210-5226 (1997)). The nucleotide sequence of YlPex16 was registered in GenBank as Accession No. CAG79622 (SEQ ID NO:14).

The term "disruption" in or in connection with a native Pex gene refers to an insertion, deletion, or targeted mutation within a portion of that gene, that results in either a complete gene knockout such that the gene is deleted from the genome and no protein is translated or a translated Pex protein having an insertion, deletion, amino acid substitution or other targeted mutation. The location of the disruption in the protein may be, for example, within the N-terminal portion of the protein or within the C-terminal portion of the protein. The disrupted Pex protein will have impaired activity with respect to the Pex protein that was not disrupted, and can be non-functional. A disruption in a native gene encoding a Pex protein also includes alternate means that result in low or lack of expression of the Pex protein, such as could result via manipulating the regulatory sequences, transcription and translation factors and/or signal transduction pathways or by use of sense, antisense or RNAi technology, etc.

As used herein, the term "Pex-disrupted organism" refers to any oleaginous eukaryotic organism comprising genes that encode a functional polyunsaturated fatty acid biosynthetic pathway and having a disruption, as defined above, in a native gene that encodes a peroxisome biogenesis factor protein,

The term "'lipids" refer to any fat-soluble (i.e., lipophilic), naturally-occurring molecule. Lipids are a diverse group of compounds that have many key biological functions, such as structural components of cell membranes, energy storage sources and intermediates in signaling pathways. Lipids may be broadly defined as hydrophobic or amphiphilic small molecules that originate entirely or in part from either ketoacyl or isoprene groups. A general overview of lipids, based on the Lipid Metabolites and Pathways Strategy (LIPID MAPS) classification system (National Institute of General Medical Sciences, Bethesda, MD), is shown below in Table 2.

**Table 2**

| Overview Of Lipid Classes | | |
|---|---|---|
| Structural Building Block | Lipid Category | Examples Of Lipid Classes |
| Derived from condensation of ketoacyl subunits | Fatty Acyls | Includes fatty acids, eicosanoids, fatty esters and fatty amides |
| | Glycerolipids | Includes mainly of mono-, di- and trisubstituted glycerols, the most well-known being the fatty acid esters of glycerol ["triacylglycerols"] |
| | Glycerophospholipids or Phospholipids | Includes phosphatidylcholine, phosphatidylethanolamine, phosphatidylserine, phosphatidylinositols and phosphatidic acids |
| | Sphingolipids | Includes ceramides, phosphosphingolipids (e.g., sphingomyelins), glycosphingolipids (e.g., gangliosides), sphingosine, cerebrosides |
| | Saccharolipids | Includes acylaminosugars, acylaminosugar glycans, acyltrehaloses, acyltrehalose glycans |
| | Polyketides | Includes halogenated acetogenins, polyenes, linear tetracyclines, polyether antibiotics, flavonoids, aromatic polyketides |
| Derived from condensation of isoprene subunits | Sterol Lipids | Includes sterols (e.g., cholesterol), C18 steroids (e.g., estrogens), C19 steroids (e.g., androgens), C21 steroids (e.g., progestogens, glucocorticoids and mineral-ocorticoids), secosteroids, bile acids |
| | Prenol Lipids | Includes isoprenoids, carotenoids, quinones, hydroquinones, polyprenols, hopanoids |

The term "total lipid fraction" of cells herein refers to all esterified fatty acids of the cell. Various subfractions within the total lipid fraction can be isolated, including the triacylglycerol ["oil"] fraction, phosphatidylcholine fraction and the phosphatidyletanolamine fraction, although this is by no means inclusive of all sub-fractions.

"Lipid bodies" refer to lipid droplets that are bound by a monolayer of phospholipid and, usually, by specific proteins. These organelles are sites where most organisms transport/store neutral lipids. Lipid bodies are thought to arise from microdomains of the endoplasmic reticulum that contain TAG biosynthesis enzymes. Their synthesis and size appear to be controlled by specific protein components.

"Neutral lipids" refer to those lipids commonly found in cells in lipid bodies as storage fats and oils and are so called because at cellular pH, the lipids bear no charged groups. Generally, they are completely nonpolar with no affinity for water. Neutral lipids generally refer to mono-, di-, and/or triesters of glycerol with fatty acids, also called monoacylglycerol, diacylglycerol or triacylglycerol, respectively, or collectively, acylglycerols. A hydrolysis reaction must occur to release free fatty acids from acylglycerols.

The terms "triacylglycerols" ["TAGs"] and "oil" are interchangeable and refer to neutral lipids composed of three fatty acyl residues esterified to a glycerol molecule. TAGs can contain long chain PUFAs, as well as shorter saturated and unsaturated fatty acids and longer chain saturated fatty acids. The TAG fraction of cells is also referred to as the "oil fraction", and "oil biosynthesis" generically refers to the synthesis of TAGs in the cell. The oil or TAG fraction is a sub-fraction of the total lipid fraction, although also it constitutes a major part of the total lipid content, measured as the weight of total fatty acids in the cell as a percent of the dry cell weight [see below], in oleaginous organisms. The fatty acid composition in the oil ["TAG"] fraction and the fatty acid composition of the total lipid fraction are generally similar. Thus, an increase or decrease in the concentration of PUFAs in the total lipid fraction will correspond with an increase or decrease in the concentration of PUFAs in the oil [" TAG"] fraction, and vice versa.

The term "total fatty acids" ["TFAs"] herein refer to the sum of all cellular fatty acids that can be derivitized to fatty acid methyl esters ["FAMEs"] by the base transesterification method (as known in the art) in a given sample, which may be the total lipid fraction or the oil fraction, for example. Thus, total fatty acids include fatty acids from neutral and polar lipid fractions, including the phosphatidylcholine fraction, the phosphatidyletanolamine fraction and the diacylglycerol, monoacylglycerol and triacylglycerol ["TAG or oil"] fractions but not free fatty acids.

3The term "total lipid content" of cells is a measure of TFAs as a percent of the dry cell weight ["DCW"]. Thus, total lipid content ["TFAs % DCW"] is equivalent to, e.g., milligrams of total fatty acids per 100 milligrams of DCW.

Generally, the concentration of a fatty acid is expressed herein as a weight percent of TFAs ["% TFAs"], e.g., milligrams of the given fatty acid per 100 milligrams of TFAs. Unless otherwise specifically stated in the disclosure herein, reference to the percent of a given fatty acid with respect to total lipids is equivalent to concentration of the fatty acid as % TFAs (e.g., % EPA of total lipids is equivalent to EPA % TFAs).

In some cases, it is useful to express the content of a given fatty acid(s) in a cell as its percent of the dry cell weight ["% DCW"]. Thus, for example, eicosapentaenoic acid % DCW would be determined according to the following formula: (eicosapentaenoic acid % TFAs) * (TFA % DCW)]/100.

The terms "lipid profile" and "lipid composition" are interchangeable and refer to the amount of an individual fatty acid contained in a particular lipid fraction, such as in the total lipid fraction or the oil ["TAG"] fraction, wherein the amount is expressed as a percent of TFAs. The sum of each individual fatty acid present in the mixture should be 100.

As used herein, the term "fold increase" refers to an increase obtained by multiplying by a number. For example, multiplying by 1.3 a quantity, an amount, a concentration, a weight percent, etc. provides a 1.3 fold increase.

The term "fatty acids" refers to long chain aliphatic acids (alkanoic acids) of varying chain lengths, from about C₁₂ to C₂₂, although both longer and shorter chain-length acids are known. The predominant chain lengths are between C₁₆ and C₂₂. The structure of a fatty acid is represented by a simple notation system of "X:Y", where X is the total number of carbon ["C"] atoms in the particular fatty acid and Y is the number of double bonds. Additional details concerning the differentiation between "saturated fatty acids" versus "unsaturated fatty acids", "monounsaturated fatty acids" versus "polyunsaturated fatty acids" ["PUFAs"], and "omega-6 fatty acids" ["ω-6" or "*n*-6"] versus "omega-3 fatty acids" ["ω-3" or "*n*-3"] are provided in U.S. Pat. No. 7,238,482.

Nomenclature used to describe PUFAs herein is given in Table 3. In the column titled "Shorthand Notation", the omega-reference system is used to indicate the number of carbons, the number of double bonds and the position of the double bond closest to the omega carbon, counting from the omega carbon, which is numbered 1 for this purpose. The remainder of the Table summarizes the common names of ω-3 and ω-6 fatty acids and their precursors, the abbreviations that are used throughout the specification and the chemical name of each compound.

**Table 3**

| Nomenclature of Polyunsaturated Fatty Acids And Precursors | | | |
|---|---|---|---|
| Common Name | Abbreviation | Chemical Name | Shorthand Notation |
| Myristic | -- | Tetradecanoic | 14:0 |
| Palmitic | Palmitate | Hexadecanoic | 16:0 |
| Palmitoleic | -- | 9-hexadecenoic | 16:1 |
| Stearic | -- | Octadecanoic | 18:0 |
| Oleic | -- | *cis*-9-octadecenoic | 18:1 |
| Linoleic | LA | *cis*-9, 12-octadecadienoic | 18:2 ω-6 |
| γ-Linolenic | GLA | *cis-*6*,* 9, 12-octadecatrienoic | 18:3 ω-6 |
| Eicosadienoic | EDA | *cis*-11, 14-eicosadienoic | 20:2 ω-6 |
| Dihomo-γ-Linolenic | DGLA | *cis*-8, 11, 14-eicosatrienoic | 20:3 ω-6 |
| Arachidonic | ARA | *cis-5,* 8, 11, 14-eicosatetraenoic | 20:4 ω-6 |
| α-Linolenic | ALA | *cis*-9, 12, 15-octadecatrienoic | 18:3 ω-3 |
| Stearidonic | STA | *cis*-6, 9, 12, 15-octadecatetraenoic | 18:4 ω-3 |
| Eicosatrienoic | ETrA | *cis-*11*,* 14, 17-eicosatrienoic | 20:3 ω-3 |
| Sciadonic | SCI | *cis-5,* 11, 14-eicosatrienoic | 20:3b ω-6 |
| Juniperonic | JUP | *cis*-5, 11, 14, 17-eicosatetraenoic | 20:4b ω-3 |
| Eicosatetraenoic | ETA | *cis-*8*,* 11, 14, 17-eicosatetraenoic | 20:4 ω-3 |
| Eicosapentaenoic | EPA | *cis-*5, 8, 11, 14, 17-eicosapentaenoic | 20:5 ω-3 |
| Docosatrienoic | DRA | *cis*-10,13,16-docosatrienoic | 22:3 ω-3 |
| Docosatetraenoic | DTA | *cis*-7, 10, 13, 16-docosatetraenoic | 22:4 ω-3 |
| Docosapentaenoic | DPAn-6 | *cis*-4, 7, 10, 13, 16-docosapentaenoic | 22:5 ω-6 |
| Docosapentaenoic | DPA | *cis-*7*,* 10, 13, 16, 19-docosapentaenoic | 22:5 ω-3 |
| Docosahexaenoic | DHA | *cis-*4*,* 7, 10, 13, 16, 19-docosahexaenoic | 22:6 ω-3 |

Although the ω-3/ω-6 PUFAs listed in Table 3 are the most likely to be accumulated in the oil fractions of oleaginous yeast using the methods described herein, this list should not be construed as limiting or as complete.

As used herein, the terms "a combination of polyunsaturated fatty acids" or "any combination of polyunsaturated fatty acids" refers to a mixture of any two or more of the polyunsaturated fatty acids listed above in Table 3. Such combination has the attributes of a concentration and of a weight percent that can be measured relative to a variety of concentrations or weight percents in the cell, including relative to the weight percent of the total fatty acids in the cell.

A metabolic pathway, or biosynthetic pathway, in a biochemical sense, can be regarded as a series of chemical reactions occurring in order within a cell, catalyzed by enzymes, to achieve either the formation of a metabolic product to be used or stored by the cell, or the initiation of another metabolic pathway, which is termed "flux generating step". Many of these pathways are elaborate, and involve a step by step modification of the initial substance to shape it into a product having the exact chemical structure desired.

The term "PUFA biosynthetic pathway" refers to a metabolic process that converts oleic acid to ω-6 fatty acids such as LA, EDA, GLA, DGLA, ARA, DRA, DTA and DPAn-6 and ω-3 fatty acids such as ALA, STA, ETrA, ETA, EPA, DPA and DHA. This process is well described in the literature. See e.g., Int'. App. Pub.No. WO 2006/052870. Briefly, this process involves elongation of the carbon chain through the addition of carbon atoms and desaturation of the elongated molecule through the addition of double bonds, via a series of special elongation and desaturation enzymes termed "PUFA biosynthetic pathway enzymes" that are present in the endoplasmic reticulum membrane. More specifically, "PUFA biosynthetic pathway enzymes" refer to any of the following enzymes (and genes which encode them) associated with the biosynthesis of a PUFA, including: a Δ4 desaturase, a Δ5 desaturase, a Δ6 desaturase, a Δ12 desaturase, a Δ15 desaturase, a Δ17 desaturase, a Δ9 desaturase, a Δ8 desaturase, a Δ9 elongase, a C_{14/16} elongase, a C_{16/18} elongase, a C_{18/20} elongase and/or a C_{20/22} elongase.

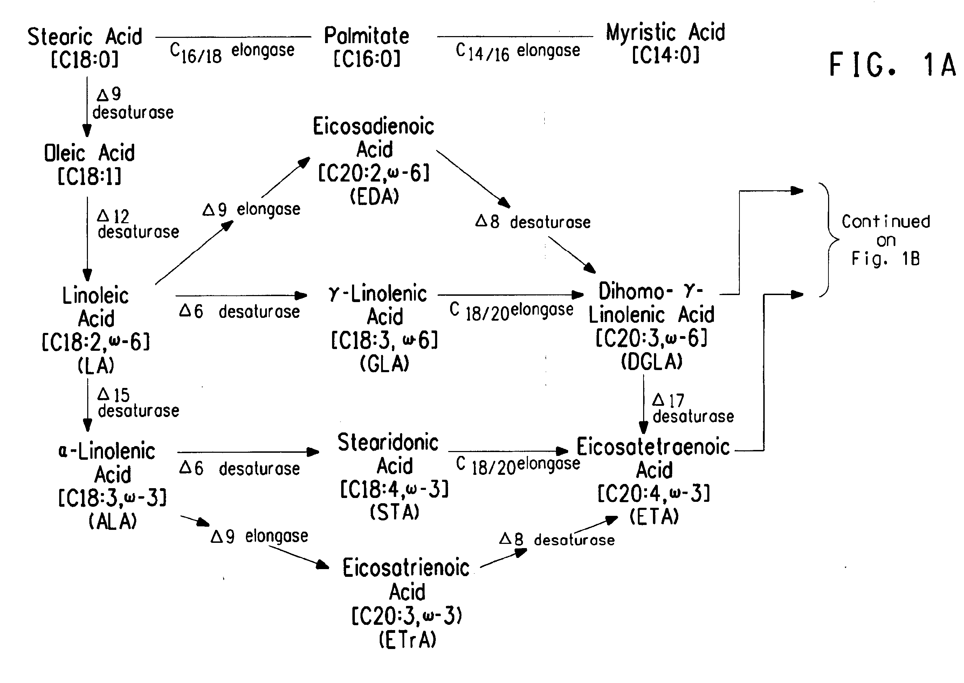

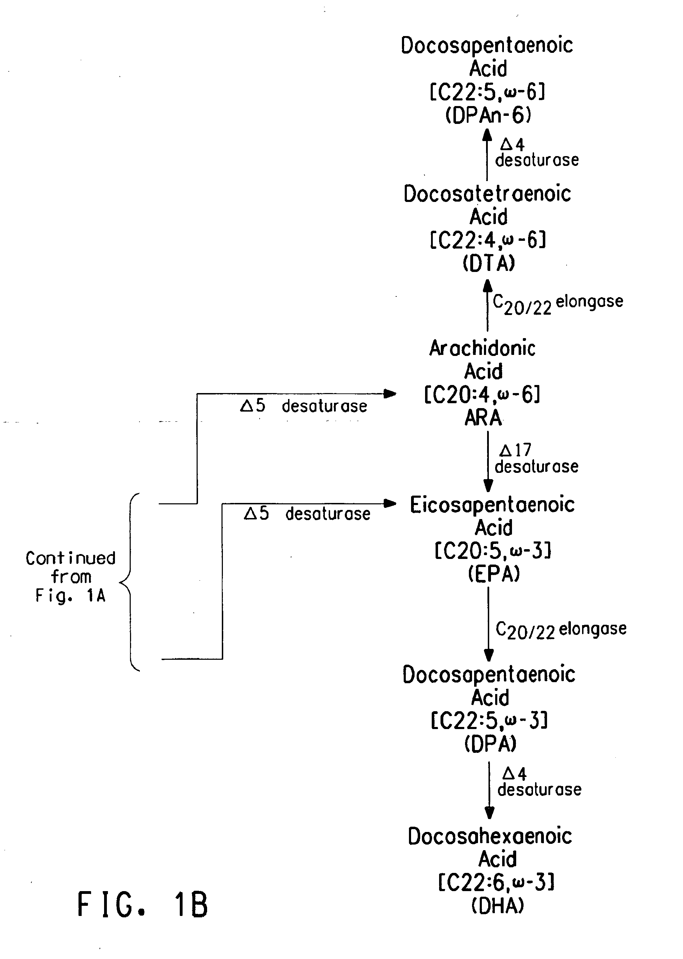

The term "ω-3/ω-6 fatty acid biosynthetic pathway" refers to a set of genes which, when expressed under the appropriate conditions, encode enzymes that catalyze the production of either or both ω-3 and ω-6 fatty acids. Typically the genes involved in the ω-3/ω-6 fatty acid biosynthetic pathway encode PUFA biosynthetic pathway enzymes. A representative pathway is illustrated in FIG. 1, providing for the conversion of myristic acid through various intermediates to DHA, which demonstrates how both ω-3 and ω-6 fatty acids may be produced from a common source. The pathway is naturally divided into two portions, such that one portion generates only ω-3 fatty acids and the other portion, only ω-6 fatty acids. That portion that generates only ω-3 fatty acids is referred to herein as the ω-3 fatty acid biosynthetic pathway, whereas that portion that generates only ω-6 fatty acids is referred to herein as the ω-6 fatty acid biosynthetic pathway.

The term "functional" as used herein relating to the ω-3/ω-6 fatty acid biosynthetic pathway, means that some (or all) of the genes in the pathway express active enzymes, resulting in *in vivo* catalysis or substrate conversion. It should be understood that "ω-3/ω-6 fatty acid biosynthetic pathway" or "functional ω-3/ω-6 fatty acid biosynthetic pathway" does not imply that all of the genes listed in the above paragraph are required, as a number of fatty acid products require only the expression of a subset of the genes of this pathway.

The term "Δ6 desaturase/Δ6 elongase pathway" refers to a PUFA biosynthetic pathway that minimally includes at least one Δ6 desaturase and at least one C_{18/20} elongase, thereby enabling biosynthesis of DGLA and/or ETA from LA and ALA, respectively, with GLA and/or STA as intermediate fatty acids. With expression of other desaturases and elongases, ARA, EPA, DPA and DHA may also be synthesized.

The term "Δ9 elongase/Δ8 desaturase pathway" refers to a PUFA biosynthetic pathway that minimally includes at least one Δ9 elongase and at least one Δ8 desaturase, thereby enabling biosynthesis of DGLA and/or ETA from LA and ALA, respectively, with EDA and/or ETrA as intermediate fatty acids. With expression of other desaturases and elongases, ARA, EPA, DPA and DHA may also be synthesized.

The term "desaturase" refers to a polypeptide that can desaturate adjoining carbons in a fatty acid by removing a hydrogen from one of the adjoining carbons and thereby introducing a double bond between them. Desaturation produces a fatty acid or precursor of interest. Despite use of the omega-reference system throughout the specification to refer to specific fatty acids, it is more convenient to indicate the activity of a desaturase by counting from the carboxyl end of the substrate using the delta-system. Of particular interest herein are: 1) Δ5 desaturases that catalyze the conversion of the substrate fatty acid, DGLA, to ARA and/or of the substrate fatty acid, ETA, to EPA; 2) Δ17 desaturases that desaturate a fatty acid between the 17^{th} and 18^{th} carbon atom numbered from the carboxyl-terminal end of the molecule and which, for example, catalyze the conversion of the substrate fatty acid, ARA, to EPA and/or the conversion of the substrate fatty acid, DGLA, to ETA; 3) Δ6 desaturases that catalyze the conversion of the substrate fatty acid, LA, to GLA and/or the conversion of the substrate fatty acid, ALA, to STA; 4) Δ12 desaturases that catalyze the conversion of the substrate fatty acid, oleic acid, to LA; 5) Δ15 desaturases that catalyze the conversion of the substrate fatty acid, LA, to ALA and/or the conversion of the substrate fatty acid, GLA, to STA; 6) Δ4 desaturases that catalyze the conversion of the substrate fatty acid, DPA, to DHA and/or the conversion of the substrate fatty acid, DTA, to DPAn-6; 7) Δ8 desaturases that catalyze the conversion of the substrate fatty acid, EDA, to DGLA and/or the conversion of the substrate fatty acid, ETrA, to ETA; and, 8) Δ9 desaturases that catalyze the conversion of the substrate fatty acid, palmitate, to palmitoleic acid (16:1) and/or the conversion of the substrate fatty acid, stearic acid, to oleic acid. Δ15 and Δ17 desaturases are also occasionally referred to as "omega-3 desaturases", "w-3 desaturases", and/or "ω-3 desaturases", based on their ability to convert ω-6 fatty acids into their ω-3 counterparts (e.g., conversion of LA into ALA and ARA into EPA, respectively). It may be desirable to empirically determine the specificity of a particular fatty acid desaturase by transforming a suitable host with the gene for the fatty acid desaturase and determining its effect on the fatty acid profile of the host.

The term "elongase" refers to a polypeptide that can elongate a fatty acid carbon chain to produce an acid 2 carbons longer than the fatty acid substrate that the elongase acts upon. This process of elongation occurs in a multi-step mechanism in association with fatty acid synthase, as described in U.S. Pat. App. Pub. No. 2005/0132442 and Int'l App. Pub. No. WO 2005/047480. Examples of reactions catalyzed by elongase systems are the conversion of GLA to DGLA, STA to ETA and EPA to DPA. In general, the substrate selectivity of elongases is somewhat broad but segregated by both chain length and the degree and type of unsaturation. For example, a C_{14/16} elongase utilizes a C₁₄ substrate e.g., myristic acid, a C_{16/18} elongase utilizes a C₁₆ substrate e.g., palmitate, a C_{18/20} elongase [also known as a Δ6 elongase as the terms can be used interchangeably] utilizes a C₁₈ substrate e.g., GLA or STA, and a C_{20/22} elongase utilizes a C₂₀ substrate e.g., EPA. In like manner, a Δ9 elongase is able to catalyze the conversion of LA and ALA to EDA and ETrA, respectively. It is important to note that some elongases have broad specificity and thus a single enzyme may be capable of catalyzing several elongase reactions. For example a single enzyme may thus act as both a C_{16/18} elongase and a C_{18/20} elongase.

The terms "conversion efficiency" and "percent substrate conversion" refer to the efficiency by which a particular enzyme, such as a desaturase, can convert substrate to product. The conversion efficiency is measured according to the following formula: ([product]/[substrate + product])*100, where 'product' includes the immediate product and all products in the pathway derived from it.

The term "oleaginous" refers to those organisms that tend to store their energy source in the form of oil (Weete, In: Fungal Lipid Biochemistry, 2nd Ed., Plenum, 1980).

The term "oleaginous yeast" refers to those microorganisms classified as yeasts that can make oil, that is, TAGs. Generally, the cellular oil or TAG content of oleaginous microorganisms follows a sigmoid curve, wherein the concentration of lipid increases until it reaches a maximum at the late logarithmic or early stationary growth phase and then gradually decreases during the late stationary and death phases (Yongmanitchai and Ward, Appl. Environ. Microbiol., 57:419-25 (1991)). Oleaginous microorganisms as referred to herein typically accumulate in excess of about 25% of their dry cell weight as oil or TAGs. Examples of oleaginous yeast include, but are not limited to, the following genera: *Yarrowia, Candida, Rhodotorula, Rhodosporidium, Cryptococcus, Trichosporon* and *Lipomyces.*

As used herein, the terms "isolated nucleic acid fragment" and "isolated nucleic acid molecule" are used interchangeably and refer to a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA.

A nucleic acid fragment is "hybridizable" to another nucleic acid fragment, such as a cDNA, genomic DNA, or RNA molecule, when a single-stranded form of the nucleic acid fragment can anneal to the other nucleic acid fragment under the appropriate conditions of temperature and solution ionic strength. Hybridization and washing conditions are well known and exemplified in Sambrook, J., Fritsch, E. F. and Maniatis, T. Molecular Cloning: A Laboratory Manual, 2nd ed., Cold Spring Harbor Laboratory: Cold Spring Harbor, NY (1989).

A "substantial portion" of an amino acid or nucleotide sequence is that portion comprising enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Basic Local Alignment Search Tool; Altschul, S. F., et al., J. Mol. Biol., 215:403-410 (1993)). In general, a sequence of ten or more contiguous amino acids or of thirty or more contiguous nucleotides is necessary in order to putatively identify a polypeptide or nucleic acid sequence as homologous to a known protein or gene. Moreover, with respect to nucleotide sequences, gene specific oligonucleotide probes comprising 20-30 contiguous nucleotides may be used in sequence-dependent methods of gene identification (e.g., Southern hybridization) and isolation, such as *in situ* hybridization of microbial colonies or bacteriophage plaques. In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence.

The term "complementary" is used to describe the relationship between nucleotide bases that are capable of hybridizing to one another. For example, with respect to DNA, adenosine is complementary to thymine and cytosine is complementary to guanine.

The terms "homology" and "homologous" are used interchangeably herein. They refer to nucleic acid fragments wherein changes in one or more nucleotide bases do not affect the ability of the nucleic acid fragment to mediate gene expression or produce a certain phenotype. These terms also refer to modifications of the Pex nucleic acid fragments described herein, such as deletion or insertion of one or more nucleotides that do not substantially alter the functional properties of the resulting nucleic acid fragment relative to the initial, unmodified fragment.

Moreover, the skilled artisan recognizes that homologous nucleic acid sequences are also defined by their ability to hybridize, under moderately stringent conditions, such as 0.5 X SSC, 0.1 % SDS, 60 °C, with the sequences exemplified herein, or to any portion of the nucleotide sequences disclosed herein and which are functionally equivalent thereto.

"Codon degeneracy" refers to the nature in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

"Synthetic genes" can be assembled from oligonucleotide building blocks that are chemically synthesized using procedures known to those skilled in the art. These oligonucleotide building blocks are annealed and then ligated to form gene segments that are then enzymatically assembled to construct the entire gene. Accordingly, the genes can be tailored for optimal gene expression based on optimization of nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell, where sequence information is available.

"Gene" refers to a nucleic acid fragment that expresses a specific protein, and which may refer to the coding region alone or may include regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene that is introduced into the host organism by gene transfer. Foreign genes can comprise native genes inserted into a non-native organism, native genes introduced into a new location within the native host, or chimeric genes. A "transgene" is a gene that has been introduced into the genome by a transformation procedure. A "codon-optimized gene" is a gene having its frequency of codon usage designed to mimic the frequency of preferred codon usage of the host cell.

"Coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, enhancers, silencers, 5' untranslated leader sequence (e.g., between the transcription start site and the translation initiation codon), introns, polyadenylation recognition sequences, RNA processing sites, effector binding sites and stem-loop structures.

"Promoter" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental or physiological conditions. Promoters that cause a gene to be expressed in most cell types at most times are commonly referred to as "constitutive promoters". It is further recognized that since in most cases the exact boundaries of regulatory sequences have not been completely defined, DNA fragments of different lengths may have identical promoter activity.

The terms "3' non-coding sequences" and "transcription terminator" refer to DNA sequences located downstream of a coding sequence. This includes polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The 3' region can influence the transcription, RNA processing or stability, or translation of the associated coding sequence.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from post-transcriptional processing of the primary transcript and is referred to as the mature RNA. "Messenger RNA" or "mRNA" refers to the RNA that is without introns and which can be translated into protein by the cell. "cDNA" refers to a double-stranded DNA that is complementary to, and derived from, mRNA. "Sense" RNA refers to RNA transcript that includes the mRNA and so can be translated into protein by the cell. "Antisense RNA" refers to a RNA transcript that is complementary to all or part of a target primary transcript or mRNA and that blocks the expression of a target gene (U.S. Pat. No. 5,107,065; Int'l. App. Pub. No. WO 99/28508). The complementarity of an antisense RNA may be with any part of the specific gene transcript, i.e., at the 5' non-coding sequence, 3' non-coding sequence, or the coding sequence. "Functional RNA" refers to antisense RNA, ribozyme RNA, or other RNA that is not translated and yet has an effect on cellular processes.

The term "operably linked" refers to the association of nucleic acid sequences on a single nucleic acid fragment so that the function of one is affected by the other. For example, a promoter is operably linked with a coding sequence when it is capable of affecting the expression of that coding sequence. That is, the coding sequence is under the transcriptional control of the promoter. Coding sequences can be operably linked to regulatory sequences in sense or antisense orientation.

The term "expression", as used herein, refers to the transcription and stable accumulation of sense (mRNA) or antisense RNA derived from nucleic acid fragments. Expression may also refer to translation of mRNA into a polypeptide.

"Mature" protein refers to a post-translationally processed polypeptide, i.e., one from which any pre- or pro-peptides present in the primary translation product have been removed. "Precursor" protein refers to the primary product of translation of mRNA, i.e., with pre- and pro-peptides still present. Pre- and pro-peptides may be, but are not limited to, intracellular localization signals.

"Transformation" refers to the transfer of a nucleic acid molecule into a host organism, resulting in genetically stable inheritance. The nucleic acid molecule may be a plasmid that replicates autonomously, for example, or, it may integrate into the genome of the host organism. Host organisms containing the transformed nucleic acid fragments are referred to as "transgenic" or recombinant" or "transformed" organisms.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms.

The terms "plasmid" and "vector" refer to an extra chromosomal element often carrying genes that are not part of the central metabolism of the cell, and usually in the form of circular double-stranded DNA fragments. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction that is capable of introducing an expression cassette(s) into a cell.

The term "expression cassette" refers to a fragment of DNA comprising the coding sequence of a selected gene and regulatory sequences preceding (5' non-coding sequences) and following (3' non-coding sequences) the coding sequence that are required for expression of the selected gene product. Thus, an expression cassette is typically composed of: 1) a promoter sequence; 2) a coding sequence, i.e., open reading frame ["ORF"] and, 3) a 3' untranslated region, i.e., a terminator that in eukaryotes usually contains a polyadenylation site. The expression cassette(s) is usually included within a vector, to facilitate cloning and transformation. Different expression cassettes can be transformed into different organisms including bacteria, yeast, plants and mammalian cells, as long as the correct regulatory sequences are used for each host.

The term "percent identity" refers to a relationship between two or more polypeptide sequences or two or more polynucleotide sequences, as determined by comparing the sequences. "Identity" also means the degree of sequence relatedness between polypeptide or polynucleotide sequences, as the case may be, as determined by the percentage of match between compared sequences. "Percent identity" and "percent similarity" can be readily calculated by known methods, including but not limited to those described in: 1) Computational Molecular Biology (Lesk, A. M., Ed.) Oxford University: NY (1988); 2) Biocomputing: Informatics and Genome Projects (Smith, D. W., Ed.) Academic: NY (1993); 3) Computer Analysis of Sequence Data, Part I (Griffin, A. M., and Griffin, H. G., Eds.) Humania: NJ (1994); 4) Sequence Analysis in Molecular Biology (von Heinje, G., Ed.) Academic (1987); and, 5) Sequence Analysis Primer (Gribskov, M. and Devereux, J., Eds.) Stockton: NY (1991).

Preferred methods to determine percent identity are designed to give the best match between the sequences tested. Methods to determine percent identity and percent similarity are codified in publicly available computer programs. Sequence alignments and percent identity calculations may be performed using the MegAlign^{™} program of the LASERGENE bioinformatics computing suite (DNASTAR Inc., Madison, WI). Multiple alignment of the sequences is performed using the "Clustal method of alignment" which encompasses several varieties of the algorithm including the "Clustal V method of alignment" and the "Clustal W method of alignment" (described by Higgins and Sharp, CABIOS, 5:151-153 (1989); Higgins, D.G. et al., Comput. Appl. Biosci., 8:189-191 (1992)) and found in the MegAlign^{™} v6.1 program of the LASERGENE bioinformatics computing suite (DNASTAR Inc.). After alignment of the sequences using either Clustal program, it is possible to obtain a "percent identity" by viewing the "sequence distances" table in the program.

It is well understood by one skilled in the art that various measures of sequence percent identity are useful in identifying polypeptides, from other species, wherein such polypeptides have the same or similar function or activity. Useful examples of percent identities include, but are not limited to, 50%, 55%, 60%, 65%, 70%, 75%, 80%, 85%, 90%, or 95%, or any integer percentage from 50% to 100%. Indeed, any integer amino acid identity from 50% to 100% may be useful in describing suitable nucleic acid fragments (isolated polynucleotides) encoding polypeptides in methods and host cells described herein, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%. In some cases, suitable nucleic acid fragments (isolated polynucleotides) encode polypeptides that are at least about 70% identical, preferably at least about 75% identical, and more preferably at least about 80% identical to the amino acid sequences reported herein. Preferred nucleic acid fragments encode amino acid sequences that are at least about 85% identical to the amino acid sequences reported herein. More preferred nucleic acid fragments encode amino acid sequences that are at least about 90% identical to the amino acid sequences reported herein. Most preferred are nucleic acid fragments that encode amino acid sequences that are at least about 95% identical to the amino acid sequences reported herein.

Suitable nucleic acid fragments not only have the above homologies but typically encode a polypeptide having at least 50 amino acids, preferably at least 100 amino acids, more preferably at least 150 amino acids, still more preferably at least 200 amino acids, and most preferably at least 250 amino acids.

The term "sequence analysis software" refers to any computer algorithm or software program that is useful for the analysis of nucleotide or amino acid sequences. "Sequence analysis software" may be commercially available or independently developed. Typical sequence analysis software include, but is not limited to: 1) the GCG suite of programs (Wisconsin Package Version 9.0, Genetics Computer Group (GCG), Madison, WI); 2) BLASTP, BLASTN, BLASTX (Altschul et al., J. Mol. Biol., 215:403-410 (1990)); 3) DNASTAR (DNASTAR, Inc. Madison, WI); 4) Sequencher (Gene Codes Corporation, Ann Arbor, MI); and, 5) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within this description, whenever sequence analysis software is used for analysis, the analytical results are based on the "default values" of the program referenced, unless otherwise specified. As used herein "default values" means any set of values or parameters that originally load with the software when first initialized.

Standard recombinant DNA and molecular cloning techniques used herein are well known in the art and are described by Sambrook, J., Fritsch, E. F. and Maniatis, T., Molecular Cloning: A Laboratory Manual, 2nd ed., Cold Spring Harbor Laboratory: Cold Spring Harbor, NY (1989) (hereinafter "Maniatis"); by Silhavy, T. J., Bennan, M. L. and Enquist, L. W., Experiments with Gene Fusions, Cold Spring Harbor Laboratory: Cold Spring Harbor, NY (1984); and by Ausubel, F. M. et al., Current Protocols in Molecular Biology, published by Greene Publishing Assoc. and Wiley-Interscience, Hoboken, NJ (1987).

### An Overview: Biosynthesis Of Fatty Acids And Triacylglycerols

In general, lipid accumulation in oleaginous microorganisms is triggered in response to the overall carbon to nitrogen ratio present in the growth medium. This process, leading to the *de novo* synthesis of free palmitate (16:0) in oleaginous microorganisms, is described in detail in U.S. Pat. No. 7,238,482. Palmitate is the precursor of longer-chain saturated and unsaturated fatty acid derivates, which are formed through the action of elongases and desaturases (FIG. 1).

TAGs, the primary storage unit for fatty acids, are formed by a series of reactions that involve: 1) esterification of one molecule of acyl-CoA to glycerol-3-phosphate via an acyltransferase to produce lysophosphatidic acid; 2) esterification of a second molecule of acyl-CoA via an acyltransferase to yield 1 ,2-diacylglycerol phosphate, commonly identified as phosphatidic acid; 3) removal of a phosphate by phosphatidic acid phosphatase to yield 1,2-diacylglycerol ["DAG"]; and, 4) addition of a third fatty acid by the action of an acyltransferase to form the TAG.

A wide spectrum of fatty acids can be incorporated into TAGs, including saturated and unsaturated fatty acids and short-chain and long-chain fatty acids. Some non-limiting examples of fatty acids that can be incorporated into TAGs by acyltransferases include: capric (10:0), lauric (12:0), myristic (14:0), palmitic (16:0), palmitoleic (16:1), stearic (18:0), oleic (18:1), vaccenic (18:1), LA (18:2), eleostearic (18:3), GLA (18:3), ALA (18:3), STA (18:4), arachidic (20:0), EDA (20:2), DGLA (20:3), ETrA (20:3), ARA (20:4), ETA (20:4), EPA (20:5), behenic (22:0), DPA (22:5), DHA (22:6), lignoceric (24:0), nervonic (24:1), cerotic (26:0) and montanic (28:0) fatty acids. In the methods and host cells described herein, incorporation of "long-chain" PUFAs into TAGs may be most desirable, wherein long-chain PUFAs include any fatty acid derived from an 18:1 substrate having at least 18 carbons in length, i.e., C₁₈ or greater. This also includes hydroxylated fatty acids, expoxy fatty acids and conjugated linoleic acid.

Although most PUFAs are incorporated into TAGs as neutral lipids and are stored in lipid bodies, it is important to note that a measurement of the total PUFAs within an oleaginous organism should include those PUFAs that are located in the phosphatidylcholine fraction, phosphatidyl-etanolamine fraction, and triacylglycerol, also known as the TAG or oil, fraction.

### Biosynthesis Of Omega Fatty Acids

The metabolic process wherein oleic acid is converted to ω-3/ω-6 fatty acids involves elongation of the carbon chain through the addition of carbon atoms and desaturation of the molecule through the addition of double bonds. This requires a series of special desaturation and elongation enzymes present in the endoplasmic reticulim membrane. However, as seen in FIG. 1 and as described below, there are often multiple alternate pathways for production of a specific ω-3/ω-6 fatty acid.

Specifically, FIG. 1 depicts the pathways described below. All pathways require the initial conversion of oleic acid to linoleic acid ["LA"], the first of the ω-6 fatty acids, by a Δ12 desaturase. Then, using the "Δ6 desaturase/Δ6 elongase pathway" and LA as substrate, long-chain ω-6 fatty acids are formed as follows: 1) LA is converted to γ-linolenic acid ["GLA"] by a Δ6 desaturase; 2) GLA is converted to dihomo-γ-linolenic acid ["DGLA"] by a C_{18/20} elongase; 3) DGLA is converted to arachidonic acid ["ARA"] by a Δ5 desaturase; 4) ARA is converted to docosatetraenoic acid ["DTA"] by a C_{20/22} elongase; and, 5) DTA is converted to docosapentaenoic acid ["DPAn-6"] by a Δ4 desaturase.

Alternatively, the "Δ6 desaturase/Δ6 elongase pathway" can use α-linolenic acid ["ALA"] as substrate to produce long-chain ω-3 fatty acids as follows: 1) LA is converted to ALA, the first of the ω-3 fatty acids, by a Δ15 desaturase; 2) ALA is converted to stearidonic acid ["STA"] by a Δ6 desaturase; 3) STA is converted to eicosatetraenoic acid ["ETA"] by a C_{18/20} elongase; 4) ETA is converted to eicosapentaenoic acid ["EPA"] by a Δ5 desaturase; 5) EPA is converted to docosapentaenoic acid ["DPA"] by a C_{20/22} elongase; and, 6) DPA is converted to docosahexaenoic acid ["DHA"] by a Δ4 desaturase. Optionally, ω-6 fatty acids may be converted to ω-3 fatty acids. For example, ETA and EPA are produced from DGLA and ARA, respectively, by Δ17 desaturase activity.

Alternate pathways for the biosynthesis of ω-3/ω-6 fatty acids utilize Δ9 elongase and Δ8 desaturase, that is, the "Δ9 elongase/ Δ8 desaturase pathway". More specifically, LA and ALA may be converted to EDA and ETrA, respectively, by a Δ9 elongase. A Δ8 desaturase then converts EDA to DGLA and/or ETrA to ETA. Downstream PUFAs are subsequently formed as described above.

The host organism herein must possess the ability to produce PUFAs, either naturally or via techniques of genetic engineering. Although many microorganisms can synthesize PUFAs (including ω-3/ω-6 fatty acids) in the ordinary course of cellular metabolism, some of whom could be commercially cultured, few to none of these organisms produce oils having a desired oil content and composition for use in pharmaceuticals, dietary substitutes, medical foods, nutritional supplements, other food products, industrial oleochemicals or other end-use applications. Thus, there is increasing emphasis on the ability to engineer microorganisms for production of "designer" lipids and oils, wherein the fatty acid content and composition are carefully specified by genetic engineering. On this basis, it is expected that the host likely comprises heterologous genes encoding a functional PUFA biosynthetic pathway but not necessarily.

If the host organism does not natively produce the desired PUFAs or possess the desired lipid profile, one skilled in the art is familiar with the considerations and techniques necessary to introduce one or more expression cassettes encoding appropriate enzymes for PUFA biosynthesis into the host organism of choice. Numerous teachings are provided in the literature to one of skill for so introducing such expression cassettes into various host organisms. Some references using the host organism *Yarrowia lipolytica* are provided as follows: U.S. Pat. No. 7,238,482, Int'l. App. Pub. No. WO 2006/033723, Pat. Appl. Pub. No. US-2006-0094092, Pat. Appl. Pub. No. US-2006-0115881-A1 and Pat. Appl. Pub. No. US-2006-0110806-A1. This list is not exhaustive and should not be construed as limiting.

Briefly, a variety of ω-3/ω-6 PUFA products can be produced prior to their transfer to TAGs, depending on the fatty acid substrate and the particular genes of the ω-3/ω-6 fatty acid biosynthetic pathway that are present in or transformed into the host cell. As such, production of the desired fatty acid product can occur directly or indirectly. Direct production occurs when the fatty acid substrate is converted directly into the desired fatty acid product without any intermediate steps or pathway intermediates. Indirect production occurs when multiple genes encoding the PUFA biosynthetic pathway may be used in combination such that a series of reactions occur to produce a desired PUFA. Specifically, it may be desirable to transform an oleaginous yeast with an expression cassette comprising a Δ12 desaturase, Δ6 desaturase, a C_{18/20} elongase, a Δ5 desaturase and a Δ17 desaturase for the overproduction of EPA. See U.S. Pat. No. 7,238,482 and Int'l. App. Pub. No. WO 2006/052870. As is well known to one skilled in the art, various other combinations of genes encoding enzymes of the PUFA biosynthetic pathway may be useful to express in an oleaginous organism (see FIG. 1). The particular genes included within a particular expression cassette depend on the host organism, its PUFA profile and/or desaturase/elongase profile, the availability of substrate and the desired end product(s).

A number of candidate genes having the desired desaturase and/or elongase activities can be identified according to publicly available literature, such as GenBank, the patent literature, and experimental analysis of organisms having the ability to produce PUFAs. Useful desaturase and elongase sequences may be derived from any source, e.g., isolated from a natural source such as from bacteria, algae, fungi, oomycete, yeast, plants, animals, etc., produced via a semi-synthetic route or synthesized *de novo.* Following the identification of these candidate genes, considerations for choosing a specific polypeptide having desaturase or elongase activity include: 1) the substrate specificity of the polypeptide; 2) whether the polypeptide or a component thereof is a rate-limiting enzyme; 3) whether the desaturase or elongase is essential for synthesis of a desired PUFA; 4) co-factors required by the polypeptide; and/or, 5) whether the polypeptide is modified after its production, such as by a kinase or a prenyltransferase.

The expressed polypeptide preferably has parameters compatible with the biochemical environment of its location in the host cell. See U.S. Pat. No. 7,238,482. It may also be useful to consider the conversion efficiency of each particular desaturase and/or elongase. More specifically, since each enzyme rarely functions with 100% efficiency to convert substrate to product, the final lipid profile of un-purified oils produced in a host cell is typically a mixture of various PUFAs consisting of the desired ω-3/ω-6 fatty acid, as well as various upstream intermediary PUFAs. Thus, the conversion efficiency of each enzyme is also a variable to consider when optimizing biosynthesis of a desired fatty acid.

### Peroxisome Biogenesis And Pex Genes

As previously described, peroxisomes are ubiquitous organelles found in all eukaryotic cells. Their primary role is the degradation of various substances within a localized organelle of the cell, such as toxic compounds, fatty acids, etc. For example, the process of β-oxidation, wherein fatty acid molecules are broken down to ultimately produce free molecules of acetyl-CoA (which are exported back to the cytosol), can occur in peroxisomes. Although the process of β-oxidation in mitochondria results in ATP synthesis, β-oxidation in peroxisomes causes the transfer of high-potential electrons to O₂ and results in the formation of H₂O₂, which is subsequently converted to water and O₂ by peroxisome catalases. Very long chain, such as C₁₈ to C₂₂, fatty acids undergo initial β-oxidation in peroxisomes, followed by mitochondrial β-oxidation.

The proteins responsible for importing proteins by means of ATP hydrolysis through the peroxisomal membrane are known as peroxisome biogenesis factor proteins, or "peroxins". These peroxisome biogenesis factor proteins also include those proteins involved in peroxisome biogenesis/assembly. The gene acronym for peroxisome biogenesis factor proteins is Pex; and, a system for nomenclature is described by Distel et al., J. Cell Biol., 135:1-3 (1996). At least 32 different Pex genes have been identified so far in various eukaryotic organisms. In fungi, however, the recent review of Kiel et al. (Traffic, 7:1291-1303 (2006)) suggests that the minimal requirement for peroxisome biogenesis/matrix protein import is numbered as 17, thereby requiring only Pex1p, Pex2p, Pex3p, Pex4p, Pex5p, Pex6p, Pex7p, Pex8p, Pex10p, Pex12p, Pex13p, Pex14p, Pex17p, Pex19p, Pex20p, Pex22p and Pe26p. These proteins act in a coordinated fashion to proliferate (duplicate) peroxisomes and import proteins via translocation into peroxisomes (reviewed in Waterham, H.R. and J.M. Cregg. BioEssays. 19(1):57-66 (1996)).

Many Pex genes were initially isolated from the analysis of mutants that demonstrated abnormal peroxisomal functions or structures. With the availability of complete genome sequences, however, it is becoming increasingly easy to identify Pex genes via computer sequence searches based on homology. Kiel et al. (Traffic, 7:1291-1303 (2006)) cite strong conservation of the peroxisome biogenesis machinery, despite occasional low sequence similarity. More specifically, within the yeast and filamentous fungi, their data indicate that almost all Pex proteins identified thus far are conserved. Table 4, below, shows peroxisome biogenesis factor proteins identified by Kiel et al. (*supra*) in *Saccharomyces cerevisiae, Candida glabrata, Ashbya gossypii*, *Kluyveromyces lactis, Candida albicans, Debaryomyces hansenii, Pichia pastoris, Hansenula - polymorpha, Yarrowia lipolytica, Aspergillus fumigatus, Aspergillus nidulans, Penicillium chrysogenum*, *Magnaporthe grisea, Neurospora crassa, Gibberella zeae, Ustilago maydis, Cryptococcus neoformans var. neoformans* and *Schizosaccharomyces pombe.*

**Table 4**

| GenBank Accession Numbers Of Fungal Peroxisome Biogenesis Factor Proteins [Recreated From Table 2 of Kiel et al., (Traffic, 7:1291-1303 (2006))] | | | | | | | | | |
|---|---|---|---|---|---|---|---|---|---|
| | ***Saccharomyces cerevisiae*** | ***Candida glabrata*** | ***Ashbya gossypii*** | ***Kluyveromyces lactis*** | ***Candida albicans*** | ***Debaryomyces hansenii*** | ***Pichia pastoris*** | ***Hansenula polymorpha*** | ***Yarrowia lipolytica*** |
| **Pex1p** | CAA82041 | CAG60131 | AAS53742 | CAH02218 | EAL02496 | CAG89689 | CAA85450 | AAD52811 | CAG82178 |
| **Pex2p** | CAA89508 | CAG60461 | AAS50677 | CAH00186 | EAK95929 | CAG85956 | CAA65646 | AAT97412 | CAG77647 |
| **Pex3p** | AAB64764 | CAG62379 | AAS52217 | CAG99801 | EAK94771 | CAG89890 | CAA96530 | AAC49471 | CAG78565 |
| **Pex3bp** | - | - | - | - | - | - | na | - | CAG83356 |
| **Pex4p** | CAA97146 | CAG60639 | AAS53685 | CAG99212 | EAL03336 | CAG87262 | AAA53634 | AAC16238 | CAG79130 |
| **Pex5p** | CAA89730 | CAG61665 | AAS53824 | CAH01742 | EAK94251 | CAG89098 | AAB40613 | AAC49040 | CAG78803 |
| **Pex5Bp** | - | CAG61076 | - | - | - | - | na | - | - |
| **Pex5Cp** | CAA89120 (Ymr018wp) | - | - | - | - | - | na | - | - |
| **Pex5/20p** | - | - | - | - | - | - | na | - | - |
| **Pex5Rp** | - | - | - | - | - | - | na | - | - |
| **Pex6p** | AAA16574 | CAG58438 | AAS54884 | CAG99125 | EAK95956 | CAG87108 | CAA80278 | AAD52812 | CAG82306 |
| **Pex7p** | CAA57183 | CAG57936 | AAS54301 | CAG99215 | EAK95226 | CAG87150 | AAC08303 | ABA64462 | CAG78389 |
| **Pex8p** | CAA97079 | CAG61238 | AAS52889 | CAH01253 | EAK91777, EAK91778* | CAG89446 | AAC41653 | CAA82928 | CAG80447 |
| **Pex9p** | ORF wrongly identified | - | - | - | - | - | - | - | - |
| **Pex10p** | AAB64453 | CAG62699 | AAS53069 | CAG99788 | Translation of AACQ-01000128, nucleotides 37281-36306 (contains intron) | CAG89101 | AAB09086 | CAA86101 | CAG81606 |
| **Pex12p** | CAA89129 | CAG62649 | AAS50837 | CAG99378 | EAL00707 | CAG84342 | AAC49402 | AAM66157 | CAG81532 |
| **Pex13p** | AAB46885 | CAG57840 | AAS51456 | CAG99931 | EAK97421 | CAG86337 | AAB09087 | DQ345349 | CAG81789 |
| **Pex14p** | AAS56829 | CAG58828 | AAS54871 | CAG99440 | EAK90926 | CAG91028 | AAG28574 | AAB40596 | CAG79323 |
| **Pex15p** | CAA99046 | CAG58938 | AAS51506 | CAG98135 | - | - | na | - | - |
| **Pex16p** | - | - | - | - | - | - | na | - | CAG79622 |
| **Pex17p** | CAA96116 | CAG61398 | AAS50595 | CAH01010 | EAK95385 | CAG86168 | AAF19606 | DQ345350 | CAG84025 |
| **Pex14/17p** | - | - | - | - | - | - | na | - | - |
| **Pex18p** | AAB68992 | - | - | - | - | - | na | - | - |
| **Pex19p** | CAA98630 | CAG58359 | AAS52741 | CAG99258 | EAK97275 | CAG84799 | AAD43507 | AAK84070 | AAK84827 |
| **Pex20p** | - | - | - | - | EAK91603, EAK94766* | CAG87898 | AAX11696 | AAX14715 | CAG79226 |
| **Pex21p** | CAA97267 | CAG59241 | AAS51769 | CAG99735 | - | - | na | - | - |
| **Pex21Bp** | - | CAG60281 | - | - | - | - | na | - | - |
| **Pex22p** | AAC04978 | CAG60970 | AAS52329 | CAG97800 | EAK91040 | CAG88727 | AAD45664 | DQ384616 | CAG77876 |
| **Pex22p-like** | - | - | - | - | - | na | - | - | EAL90994 |
| **Pex26p** | - | - | - | - | EAK91093 | CAG88929 | na | DQ645588 | Antisense translation of NC_006072, nucleotides 117230-118387 |

| | ***Aspergillus fumigatus*** | ***Aspergillus nidulans*** | ***Penicillium chrysogenum*** | ***Magnaporthe grisea*** | ***Neurospora crassa*** | ***Gibberella zeae*** | ***Ustilago maydis*** | ***Cryptococcus neoformans var. neoformans*** | ***Schizosaccharomyces pombe*** |
|---|---|---|---|---|---|---|---|---|---|
| **Pex1p** | EAL93310 | EAA57740 | AAG09748 | XP_364454 | EAA34641 | EAA76787 | EAK85195 | AAW43248 | CAA19256 |
| **Pex2p** | EAL88068 | EAA58944 | DQ793192 | XP_368589 | EAA35361 | EAA70670 | EAK81310 | AAW40683 | CAA16981 |
| **Pex3p** | EAL91965 | EAA64392 | DQ793193 | XP_369909 | EAA33751 | EAA76989 | EAK87104 | AAW42444 | CAB10141 |
| **Pex3Bp** | - | - | - | - | - | - | - | - | - |
| **Pex4p** | EAL87211 | Translation of AACD0-1000130, nucleotides 150195-150738 (contains intron) | DQ793194 | XP_369064 | EAA34737 | EAA76379 | Translation of AACP0-1000006, nucleotides 97041-96550 (contains-intron) | - | CAB91184 |
| **Pex5p** | EAL85289 | EAA63772 | AAR12222 | XP_360528 | EAA36111 | EAA68640 | EAK83659 | AAW46349 | CAA22179 |
| **Pex5Bp** | - | - | - | - | - | - | - | - | - |
| **Pex5Cp** | - | - | - | - | - | - | - | - | - |
| **Pex5/20p** | - | - | - | - | - | - | EAK82973 | AAW41849 | - |
| **Pex5Rp** | - | - | - | - | - | - | - | - | - |
| **Pex6p** | EAL92776 | EAA63496 | AAG09749 | XP_368715 | EAA36040 | EAA73732 | EAK83459 | AAW45333 | CAB11501 |
| **Pex7p** | EAL90870 | EAA65909 | DQ793195 | XP_363555 | AAN39560 | EAA74171 | EAK84499 | AAW41119 | P78798 |
| **Pex8p** | EAL93137 | EAA57947 | DQ793196 | XP_359449 | EAA27783 | EAA77627 | EAK83936 | AAW43468 | CAB53406 |
| **Pex9p** | - | - | - | - | - | - | - | - | - |
| **Pex10p** | EAL87045 | EAA62774 | DQ793197 | XP_369099 | EAA34967 | EAA76761 | EAK83811 | AAW45079 | CAB51769 |
| **Pex12p** | EAL93972 | EAA61357 | DQ793198 | XP_363845 | EAA32773 | EAA76413 | EAK81282 | AAW46724 | CAD27496 |
| **Pex13p** | EAL85282 | EAA63824 | DQ793199 | XP_369087 | EAA35785 | EAA68396 | EAK84395 | AAW42381 | CAB16740 |
| **Pex14p** | EAL92562 | EAA61046 | DQ793200 | XP_368216 | EAA28304 | EAA76904 | EAK83123 | AAW46857 | CAA18656 |
| **Pex15p** | - | - | - | - | - | - | - | - | - |
| **Pex16p** | EAL88469 | EAA62294 | DQ793201 | XP_364166 | EAA34648 | EAA71849 | EAK82801 | AAW43797 | CAA22819 |
| **Pex17p** | See Pex14/17p | - | - | - | - | - | - | - | - |
| **Pex14/17p** | EAL93590 | EAA58642 | DQ793202 | XP 368163 | EAA27748 | EAA73655 | EAK81127 | - | - |
| **Pex18p** | - | - | - | - | - | - | - | - | - |
| **Pex19p** | EAL92487 | EAA60977 | DQ793203 | XP_368273 | EAA31855 | EAA70162 | EAK86072 | AAW42876 | CAA97344 |
| **Pex20p** | EAL90176 | EAA60479 | DQ793204 | XP_368606 | AAN39561 | EAA76911 | - | - | - |
| **Pex21p** | - | - | - | - | - | - | - | - | - |
| **Pex21 Bp** | - | - | - | - | - | - | - | - | - |
| **Pex22p** | - | - | - | - | - | - | - | - | - |
| **Pex22p-like** | EAL90994 | EAA66006 | DQ793205 | XP_365689 | EAA26537 | Translation of AACM0-1000080, nucleotides 4362-3039 (contains intron) | - | - | - |
| **Pex26p** | EAL93994 | EAA61336 | DQ793206 | XP 359606 | EAA28582 | EAA76391 | - | - | - |

| | | | | | | | | | |
|---|---|---|---|---|---|---|---|---|---|
| * Partial ORFs encoded on non-overlapping contigs. | | | | | | | | | |

Mutations of Pex genes leading to impaired peroxisome biogenesis result in severe metabolic and developmental disturbances in yeasts, human and plants (Eckert, J. H. and R. Erdmann, Rev. Physiol. Biochem Pharmacol 147:75-121 (2003); Weller, S. et al., Annual Review of Genomics and Human Genetics, 4:165-211 (2003); Wanders, R. J., Am. J. Med. Genet., 126A:355-375 (2004); Mano, S. and M. Nishimura, Vitam Horm., 72:111-154 (2005); Wanders, J. A., and H. R. Waterham, Annu. Rev. Biochem., 75:295-332 (2006); Fujiki, Yukio. Peroxisome Biogenesis Disorders. In, Encyclopedia of Life Sciences. John Wiley & Sons, 2006). For example, X-linked adrenoleukodystrophy ["X-ALD"] and Zellweger syndrome, as well as several less severe forms of the disease, can result from single enzyme deficiencies and/or peroxisomal biogenesis disorders.

Within the yeast, *Yarrowia lipolytica,* a variety of different Pex genes have been isolated and characterized, as identified in Table 4 above. More specifically, Bascom, R.A. et al. (Mol. Biol. Cell, 14:939-957 (2003)) describe YIPex3p; Szilard, R. K. et al. (J. Cell Biol., 131:1453-1469 (1995)) describe YIPex5p; Nuttley, W. M. et al. (J. Biol. Chem., 269:556-566 (1994)) describe YIPex6p; Elizen G. A., et al. (J. Biol. Chem., 270:1429-1436 (1995)) describe YIPex9p; Elizen G. A., et al. (J. Cell Biol., 137:1265-1278 (1997)) and Titorenko, V. I. et al. (Mol. Cell Biol., 17:5210-5226 (1997)) describe YIPex16p; Lambkin, G.R. and R.A. Rachubinski (Mol. Biol. Cell., 12(11):3353-3364 (2001)) describe YIPex19; and Titorenko V.I., et al. (J. Ce Biol., 142:403-420 (1998)) and Smith J.J. and R.A. Rachubinski (J. Cell Biol. 276:1618-1625 (2001)) describe YIPex20p.

Of initial interest herein was YIPex10p (GenBank Accession No. CAG81606, No. AB036770 and No. AJ012084). It was demonstrated in Sumita et al. (FEMS Microbiol. Lett., 214:31-38 (2002) that: 1) YIPex10p functions as a component of the peroxisome; and, 2) the C₃HC₄ zinc ring finger motif of YIPex10p was essential for the protein's function as determined via creation of C341 S, C346S and H343W point mutations, followed by analysis of growth.

Studies of the C₃HC₄ zinc ring finger motif of Pex10 have been done in other organisms with similar results. For example, point mutations that alter conserved residues in the Pex10p C₃HC₄ motif of *Pichia pastoris* were found to abolish function of the protein (Kalish, J. E. et al., Mol. Cell Biol., 15:6406-6419 (1995)). Similarly, after functional complementation assays in fibroblast cell lines, Warren D. S., et al. (Hum. Mutat., 15(6):509-521 (2000)) concluded that the C₃HC₄ motif was critical for Pex10p function. Several studies show that loss of function of Pex10p in *Arabidopsis* causes embryo lethality at the heart stage (Hu, J., et al., Science, 297:405-409 (2002); Schmumann, U. et al., Proc. Natl. Acad. Sci. U.S.A., 100:9626-9631 (2003); Sparkes, I.A., et al., Plant Physiol., 133:1809-1819 (2003); Fan, J. et al., Plant Physiol., 139:231-239 (2005)). In follow-up research, Schemann, U. et al. (Proc. Natl. Acad. Sci. U.S.A., 104:1069-1074 (2007)) investigated the function of Pex10p in nonlethal partial loss-of-function *Arabidopsis* mutants. Specifically, four T-DNA insertion lines expressing Pex10p with a dysfunctional C₃HC₄ motif wer created in an *Arabidopsis* wildtype background. Mutant plants demonstrated impaired leaf peroxisomes and the authors suggest that inactivation of the ring finger motif in Pex10p eliminated protein interaction required for attachment of peroxisomes to chloroplasts and movement of metabolites between peroxisomes and chloroplasts.

Although studies have not identified essential domains in other Pex proteins, research has looked at the effect of various Pex mutants to learn the strategies and the molecular mechanisms evolutionarily diverse organisms use for assembling, maintaining, propagating and inheriting the peroxisome, an organelle known for its role in lipid metabolism. For example, Bascom, R.A. et al. has performed knockout and overexpression of the *Yarrowia lipolytica* Pex3p (Mol. Biol. Cell, 14:939-957 (2003)). The knockout cells did not contain wildtype perixosomes but instead had numerous small vesicles; overexpression resulted in cells with fewer, larger and clustered peroxisomes. They hypothesized that Pex3p is involved in the initiation of peroxisome assembly by sequestering components of peroxisome biogenesis, i.e., peroxisome targeting signal (PTS) 1 and 2 import machineries. Similarly, for Guo, T. et al., knockout of the Y *lipolytica* Pex16p resulted in excessive proliferation of immature peroxisomal vesicles and significantly decreased the rate and efficiency of their conversion to mature peroxisomes (J. Cell Biol.,162:1255-1266 (2003)), while overexpression resulted in few but enlarged peroxisomes (Eitzen et al., J. Cell Biol., 137:1265-1278 (1997)). Guo et al. concluded Pex16p negatively regulated the membrane scission event required for division of early peroxisomal precursors.

Despite the advances summarized above, many details concerning the roles of various Pex proteins, their interaction with one another and the biogenesis/assembly mechanism in peroxisomes remains to be elucidated. As such, the data described in the Application, wherein mutation within the C₃HC₄ motif of YIPex10p or knockout of YIPex3p, YIPex10p or YIPex16p results in creation of a *Yarrowia lipolytica* mutant that has an increased capacity to incorporate PUFAs, especially long-chain PUFAs such as C₂₀ to C₂₂ molecules, into the total lipid fraction and in the oil fraction in the cell, is a novel observation that does not yet find validation in studies with other plants or animals.

It has been suggested that peroxisomes are required for both catabolic and anabolic lipid metabolism (Lin, Y. et al., Plant Physiology, 135:814-827 (2004)); however, this hypothesis was based on studies with a homolog of Pex16p. More specifically, Lin, Y. et al. (*supra*) reported that Arabidopsis *Shrunken Seed 1 (*s*se1*) mutants had both abnormal peroxisome biogenesis and fatty acid synthesis, based on a reduction of oil to approximately 10-16% of wild type in sse1 seeds. Binns, D. et al. (J. Cell Biol., 173(5):719-731 (2006)) examined the peroxisome-lipid body interactions in *Saccharomyces cerevisiae* and determined that extensive physical contact between the two organelles promotes coupling of lipolysis within lipid bodies with peroxisomal fatty acid oxidation. More specifically, ratios of free fatty acids to TAGs were examined in various Pex knockouts and found to be increased relative to the wildtype. Clearly, further investigation will be necessary to understand the metabolic roles of peroxisomes and in particular of Pex3p, Pex10p and Pex16p proteins.

Without wishing to be held to any particular explanation or theory, it is hypothesized that disruption or knockout of a Pex gene within an oleaginous yeast cell affects both the catabolic and anabolic lipid metabolism that naturally occurs in peroxisomes or is affected by peroxisomes. Disruption or knockout results in an increase in the amount of PUFAs in the total lipid fraction and in the oil fraction, as a percent of total fatty acids, as compared with an oleaginous yeast whose native peroxisome biogenesis factor protein has not been disrupted. In some cases, an increase in the amount of PUFAs in the total lipid fraction and in the oil fraction as a percent of dry cell weight, and/or an increase in the total lipid content as a percent of dry cell weight, is also observed. It is hypothesized that this generalized mechanism is applicable within all eukaryotic organisms, such as algae, fungi, oomycetes, yeast, euglenoids, stramenopiles, plants and some mammalian systems, since all comprise peroxisomes.

### Identification And Isolation Of Pex Homologs

When the sequence of a particular Pex gene or protein within a preferred host organism is not known, one skilled in the art recognizes that it will be most desirable to identify and isolate these genes, or portions of them prior to, regulating the activity of the encoded proteins, which regulation in turn facilitates altering the amount, as a percent of total fatty acids, of PUFAs incorporated into the total lipid fraction and in the oil fraction of the eukaryote Sequence knowledge of the preferred host's Pex genes also facilitates disruption of the homologous chromosomal genes by targeted disruption.

The Pex sequences in Table 4, or portions of them, may be used to search for Pex homologs in the same or other algal, fungal, oomycete, euglenoid, stramenopiles, yeast or plant species using sequence analysis software. In general, such computer software matches similar sequences by assigning degrees of homology to various substitutions, deletions, and other modifications. Use of software algorithms, such as the BLASTP method of alignment with a low complexity filter and the following parameters: Expect value = 10, matrix = Blosum 62 (Altschul, et al., Nucleic Acids Res. 25:3389-3402 (1997)), is well-known for comparing any Pex protein in Table 4 against a database of nucleic or protein sequences and thereby identifying similar known sequences within a preferred host organism.

Use of a software algorithm to comb through databases of known sequences is particularly suitable for the isolation of homologs having a relatively low percent identity to publicly available Pex sequences, such as those described in Table 4. It is predictable that isolation would be relatively easier for Pex homologs of at least about 70%-85% identity to publicly available Pex sequences. Further, those sequences that are at least about 85%-90% identical would be particularly suitable for isolation and those sequences that are at least about 90%-95% identical would be the most facilely isolated.

Some Pex homologs have also been isolated by the use of motifs unique to the Pex enzymes. For example, it is well known that Pex2p, Pex10p and Pex12p all share a cysteine-rich motif near their carboxyl termini, known as a C₃HC₄ zinc ring finger motif (FIG. 2A). This region of "conserved domain" corresponds to a set of amino acids that are highly conserved at specific positions and likely represents a region of the Pex protein that is essential to the structure, stability or activity of the protein. Motifs are identified by their high degree of conservation in aligned sequences of a family of protein homologues. As unique "signatures", they can determine if a protein with a newly determined sequence belongs to a previously identified protein family. These motifs are useful as diagnostic tools for the rapid identification of novel Pex2, Pex10 and/or Pex12 genes, respectively.

Alternatively, the publicly available Pex sequences or their motifs may be hybridization reagents for the identification of homologs. The basic components of a nucleic acid hybridization test include a probe, a sample suspected of containing the gene or gene fragment of interest, and a specific hybridization method. Probes are typically single-stranded nucleic acid sequences that are complementary to the nucleic acid sequences to be detected. Probes are hybridizable to the nucleic acid sequence to be detected. Although probe length can vary from 5 bases to tens of thousands of bases, typically a probe length of about 15 bases to about 30 bases is suitable. Only part of the probe molecule need be complementary to the nucleic acid sequence to be detected. In addition, the complementarity between the probe and the target sequence need not be perfect. Hybridization does occur between imperfectly complementary molecules with the result that a certain fraction of the bases in the hybridized region are not paired with the proper complementary base.

Hybridization methods are well known. Typically the probe and the sample must be mixed under conditions that permit nucleic acid hybridization. This involves contacting the probe and sample in the presence of an inorganic or organic salt under the proper concentration and temperature conditions. The probe and sample nucleic acids must be in contact for a long enough time that any possible hybridization between the probe and the sample nucleic acid occurs. The concentration of probe or target in the mixture determine the time necessary for hybridization to occur. The higher the concentration of the probe or target, the shorter the hybridization incubation time needed. Optionally, a chaotropic agent may be added, such as guanidinium chloride, guanidinium thiocyanate, sodium thiocyanate, lithium tetrachloroacetate, sodium perchlorate, rubidium tetrachloroacetate, potassium iodide or cesium trifluoroacetate. If desired, one can add formamide to the hybridization mixture, typically 30-50% (v/v) ["by volume"].

Various hybridization solutions can be employed. Typically, these comprise from about 20 to 60% volume, preferably 30%, of a polar organic solvent. A common hybridization solution employs about 30-50% v/v formamide, about 0.15 to 1 M sodium chloride, about 0.05 to 0.1 M buffers (e.g., sodium citrate, Tris-HCl, PIPES or HEPES (pH range about 6-9)), about 0.05 to 0.2% detergent (e.g., sodium dodecylsulfate), or between 0.5-20 mM EDTA, FICOLL (Pharmacia Inc.) (about 300-500 kdal), polyvinylpyrrolidone (about 250-500 kdal), and serum albumin. Also included in the typical hybridization solution are unlabeled carrier nucleic acids from about 0.1 to 5 mg/mL, fragmented nucleic DNA such as calf thymus or salmon sperm DNA or yeast RNA, and optionally from about 0.5 to 2% wt/vol ["weight by volume"] glycine. Other additives may be included, such as volume exclusion agents that include polar water-soluble or swellable agents (e.g., polyethylene glycol), anionic polymers (e.g., polyacrylate or polymethylacrylate) and anionic saccharidic polymers, such as dextran sulfate.

Nucleic acid hybridization is adaptable to a variety of assay formats. One of the most suitable is the sandwich assay format. The sandwich assay is particularly adaptable to hybridization under non-denaturing conditions. A primary component of a sandwich-type assay is a solid support. The solid support has adsorbed or covalently coupled to it immobilized nucleic acid probe that is unlabeled and complementary to one portion of the sequence.

Any of the Pex nucleic acid fragments or any identified homologs may be used to isolate genes encoding homologous proteins from the same or other algal, fungal, oomycete, euglenoid, stramenopiles, yeast or plant species. Isolation of homologous genes using sequence-dependent protocols is well known in the art. Examples of sequence-dependent protocols include, but are not limited to: 1) methods of nucleic acid hybridization; 2) methods of DNA and RNA amplification, as exemplified by various uses of nucleic acid amplification technologies, such as polymerase chain reaction ["PCR"] (U.S. Pat. No.4,683,202); ligase chain reaction ["LCR"] (Tabor, S. et al., Proc. Natl. Acad. Sci. U.S.A., 82:1074 (1985)); or strand displacement amplification ["SDA"] (Walker, et al., Proc. Natl. Acad. Sci. U.S.A., 89:392 (1992)); and, 3) methods of library construction and screening by complementation.

For example, genes encoding proteins or polypeptides similar to publicly available Pex genes or their motifs could be isolated directly by using all or a portion of those publicly available nucleic acid fragments as DNA hybridization probes to screen libraries from any desired organism using well known methods. Specific oligonucleotide probes based upon the publicly available nucleic acid sequences can be designed and synthesized by methods known in the art (Maniatis, *supra*). Moreover, the entire sequences can be used directly to synthesize DNA probes by methods known to the skilled artisan, such as random primers DNA labeling, nick translation or end labeling techniques, or RNA probes using available *in vitro* transcription systems. In addition, specific primers can be designed and used to amplify a part or the full length of the publicly available sequences or their motifs. The resulting amplification products can be labeled directly during amplification reactions or labeled after amplification reactions, and used as probes to isolate full-length DNA fragments under conditions of appropriate stringency.

Typically, in PCR-type amplification techniques, the primers have different sequences and are not complementary to each other. Depending on the desired test conditions, the sequences of the primers should be designed to provide for both efficient and faithful replication of the target nucleic acid. Methods of PCR primer design are common and well known (Thein and Wallace, "The use of oligonucleotides as specific hybridization probes in the Diagnosis of Genetic Disorders", in Human Genetic Diseases: A Practical Approach, K. E. Davis Ed., (1986) pp 33-50, IRL: Herndon, VA; Rychlik, W., In Methods in Molecular Biology, White, B. A. Ed., (1993) Vol. 15, pp 31-39, PCR Protocols: Current Methods and Applications. Humania: Totowa, NJ).

Generally two short segments of available Pex sequences may be used in PCR protocols to amplify longer nucleic acid fragments encoding homologous genes from DNA or RNA. PCR may also be performed on a library of cloned nucleic acid fragments wherein the sequence of one primer is derived from the available nucleic acid fragments or their motifs. The sequence of the other primer takes advantage of the presence of the polyadenylic acid tracts to the 3' end of the mRNA precursor encoding genes

Alternatively, the second primer sequence may be based upon sequences derived from the cloning vector. For example, the skilled artisan can follow the RACE protocol (Frohman et al., Proc. Natl. Acad. Sci. U.S.A., 85:8998 (1988)) to generate cDNAs by using PCR to amplify copies of the region between a single point in the transcript and the 3' or 5' end. Primers oriented in the 3' and 5' directions can be designed from the available sequences. Using commercially available 3' RACE or 5' RACE systems (e.g BRL, Gaithersburg, MD), specific 3' or 5' cDNA fragments can be isolated (Ohara et al., Proc. Natl. Acad. Sci. U.S.A., 86:5673 (1989); Loh et al., Science, 243:217 (1989)).

Based on any of these well-known methods just discussed, it would be possible to identify and/or isolate Pex gene homologs in any preferred eukaryotic organism of choice. The activity of any putative Pex gene can readily be confirmed by targeted disruption of the endogenous gene within the PUFA-producing host organism, since the lipid profiles of the total lipid fraction and of the oil fraction are modified relative to those within an organism lacking the targeted Pex gene disruption.

### Increasing The Amount Of PUFAs In The Total Lipid Fraction And In The Oil Fraction Via Disruption Of A Native Peroxisome Biogenesis Factor Protein

As noted above, the present disclosure relates to the following described methods for increasing the weight percent of one PUFA or a combination of PUFAs in an oleaginous yeast or fungus, comprising:
a) providing an oleaginous yeast or fungus comprising a disruption in a native gene encoding a peroxisome biogenesis factor protein, which creates a PEX-disruption organism; and genes encoding a functional PUFA biosynthetic pathway; and,
b) growing the yeast or fungus of (a) under conditions wherein the weight percent of one PUFA or a combination of PUFAs is increased in the total lipid fraction and in the oil fraction relative to the weight percent of the total fatty acids, when compared with those weight percents in an oleaginous yeast or fungus whose native peroxisome biogenesis factor protein has not been disrupted.

The amount of PUFAs that increases as a percent of total fatty acids can be: 1) the PUFA that is the desired end product of a functional PUFA biosynthetic pathway, as opposed to PUFA intermediates or by-products; 2) C₂₀ to C₂₂ PUFAs; and/or, 3) total PUFAs.

In addition to the increase in the weight percent of one or a combination of PUFAs relative to the weight percent of the total fatty acids, in some cases, the total lipid content (TFA % DCW) of the cell may be increased or decreased. What this means is that regardless of whether the disruption in the PEX gene causes the amount of total lipids in the PEX-disrupted cell to increase or decrease, the disruption always causes the weight percent of a PUFA or of a combination of PUFAs to increase.

Another method provided herein relates to a disruption in a native gene encoding a peroxisome biogenesis factor protein, wherein said disruption can result in an increase in the percent of one PUFA or a combination of PUFAs relative to the dry cell weight when compared to that percent in a parental strain whose native Pex protein had not been disrupted or that was expressing a "replacement" copy of the disrupted native Pex protein.

In preferred aspects of the method above, the disruption in a native gene encoding a peroxisome biogenesis factor protein results in an increase in the amount of the PUFA that is the desired end product of a functional PUFA biosynthetic pathway, as opposed to PUFA intermediates or by-products, as a percent of dry cell weight relative to the parental strain whose native Pex protein had not been disrupted or the parental strain that was expressing a "replacement" copy of the disrupted native Pex protein. In some cases, the increase in the percent of a combination of PUFAs relative to the dry cell weight is a combination of C₂₀ to C₂₂ PUFAs or the total PUFAs.

Also described herein are organisms produced by these methods, comprising a disruption of at least one peroxisome biogenesis factor protein. Lipids and oils obtained from these organisms, products obtained from the processing of the lipids and oil, use of these lipids and oil in foods, animal feeds or industrial applications and/or use of the by-products in foods or animal feeds are also described.

The peroxisome biogenesis factor protein for any of these methods may be selected from the group consisting of: Pex1 p, Pex2p, Pex3p, Pex3Bp Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21 p, Pex21 B, Pex22p, Pex22p-like and Pex26p (and protein homologs thereof). In some preferred methods described herein, the disrupted peroxisome biogenesis factor protein is selected from the group consisting of: Pex2p, Pex3p, Pex10p, Pex12p and/or Pex16p. In some more preferred methods, however, the disrupted peroxisome biogenesis factor protein is selected from the group consisting of: Pex3p, Pex10p and/or Pex16p.

The disruption in the native gene encoding a peroxisome biogenesis factor protein can be an insertion, deletion, or targeted mutation within a portion of the gene, such as within the N-terminal portion of the protein or within the C-terminal portion of the protein. Alternatively, the disruption can result in a complete gene knockout such that the gene is eliminated from the host cell genome. Or, the disruption could be a targeted mutation that results in a non-functional protein.

### Disruption Methodologies

The invention includes disruption in a native gene encoding a peroxisome biogenesis factor protein within a preferred host cell. Although numerous techniques are available to one of skill in the art to achieve disruption, generally the endogenous activity of a particular gene can be reduced or eliminated by the following techniques, for example: 1) disrupting the gene through insertion, substitution and/or deletion of all or part of the target gene; or 2) manipulating the regulatory sequences controlling the expression of the protein. Both of these techniques are discussed below. However, one skilled in the art appreciates that these are well described in the existing literature and are not limiting to the methods, host cells, and products described herein. One skilled in the art also appreciates the most appropriate technique for use with any particular oleaginous yeast.

**Disruption Via Insertion, Substitution And/Or Deletion: For gene** disruption, a foreign DNA fragment, typically a selectable marker gene, is inserted into the structural gene. This interrupts the the coding sequence of the structural gene and causes inactivatation of that gene. Transformation of the disruption cassette into the host cell results in replacement of the functional native gene by homologous recombination with the non-functional disrupted gene. See, for example: Hamilton et al., J. Bacteriol., 171:4617-4622 (1989); Balbas et al., Gene, 136:211-213 (1993); Gueldener et al., Nucleic Acids Res., 24:2519-2524 (1996); and Smith et al., Methods Mol. Cell. Biol., 5:270-277(1996). One skilled in the art appreciates the many variations of the general method of gene targeting, which admits of positive or negative selection, creation of gene knockouts, and insertion of exogenous DNA sequences into specific genome sites in mammalian systems, plant cells, filamentous fungi, algae, oomycetes, euglenoids, stramenopiles, yeast and/or microbial systems.

In contrast, a non-specific method of gene disruption is the use of transposable elements or transposons. Transposons are genetic elements that insert randomly into DNA but can be later retrieved on the basis of sequence to determine the locus of insertion. Both *in vivo* and *in vitro* transposition techniques are known and involve the use of a transposable element in combination with a transposase enzyme. When the transposable element or transposon is contacted with a nucleic acid fragment in the presence of the transposase, the transposable elements randomly inserts into the nucleic acid fragment. The technique is useful for random mutagenesis and for gene isolation, since the disrupted gene may be identified on the bas of the sequence of the transposable element. Kits for *in vitro* transposition a commercially available and include: the Primer Island Transposition Kit, available from Perkin Elmer Applied Biosystems, Branchburg, NJ, based upon the yeast Ty1 element; the Genome Priming System, available from New England Biolabs, Beverly, MA, based upon the bacterial transposon Tn7; and EZ::TN Transposon Insertion Systems, available from Epicentre Technologie Madison, WI, based upon the Tn5 bacterial transposable element.

**Manipulation Of Pex Regulatory Sequences:** As is well known in the art, the regulatory sequences associated with a coding sequence include transcriptional and translational "control" nucleotide sequences located upstream (5' non-coding sequences), within, or downstream (3' non-coding sequences) of the coding sequence, and which influence the transcription, RNA processing or stability, or translation of the associated coding sequence Thus, manipulation of a Pex gene's regulatory sequences may refer to manipulation of the promoters, silencers, 5' untranslated leader sequences (between the transcription start site and the translation initiation codon), introns, enhancers, initiation control regions, polyadenylation recognition sequences, RNA processing sites, effector binding sites and stem-loop structures of the particular Pex gene. In all cases, however, the result of the manipulation is down-regulation of the Pex gene's expression, which promotes increased amount of PUFAs in the total lipid fraction and in the oil fraction, as a percent of total fatty acids, as compared with an oleaginous yeast whose native peroxisome biogenesis factor protein has not been disrupted.

For example, the promoter of a Pex10 gene could be deleted or disrupted. Alternatively, the native promoter driving expression of a Pex10 gene may be substituted with a heterologous promoter having diminished promoter activity with respect to that of the native promoter. Methods useful for manipulating regulatory sequences are well known.

The skilled person is able to use these and other well known techniques to disrupt a native peroxisome biogenesis factor protein within the host cells described herein, filamentous fungi and yeast.

One skilled in the art is able to discern the optimum means to disrupt the native Pex gene to achieve an increased amount of PUFAs that accumulate in the total lipid fraction and in the oil fraction, as a percent of total fatty acids, as compared with a eukaryotic organisms whose native peroxisome biogenesis factor protein has not been disrupted.

### Metabolic Engineering Of ω-3 And/Or ω-6 Fatty Acid Biosynthesis

In addition to the methods described herein for disruption of a native peroxisome biogenesis factor protein, it may also be useful to manipulate ω-3 and/or ω-6 fatty acid biosynthesis. This may require metabolic engineering directly within the PUFA biosynthetic pathway or additional manipulation of pathways that contribute carbon to the PUFA biosynthetic pathway.

Techniques useful for up-regulating desirable biochemical pathways and down-regulating undesirable biochemical pathways are well known in the art. For example, biochemical pathways competing with the ω-3 and/or ω-6 fatty acid biosynthetic pathways for energy or carbon, or native PUFA biosynthetic pathway enzymes that interfere with production of a particular PUFA end-product, may be eliminated by gene disruption or down-regulated by other means, such as antisense mRNA and zinc-finger targeting technologies.

The following discuss altering the PUFA biosynthetic pathway as a means to increase GLA, ARA, EPA or DHA, respectively, and desirable manipulations in the TAG biosynthetic pathway and in the TAG degradation pathway: Int'l. App. Pub. No. WO 2006/033723, Int'l. App. Pub. No. WO 2006/055322 [U.S. Pat. Appl. Pub. No. 2006-0094092-A1], Int'l. App. Pub. No. WO 2006/052870 [U.S. Pat. Appl. Pub. No. 2006-0115881-Al and Int'l. App. Pub. No. WO 2006/052871 [U.S. Pat. Appl. Pub. No. 2006-0110806-A1], respectively.

### Expression Systems, Cassettes, Vectors And Transformation Of Host Cells

It may be necessary to create and introduce a recombinant construct into the eukaryotic host, which may be filamentous fungi and yeast, to result in disruption of a native peroxisome biogenesis factor protein and/or introduction of genes encoding a PUFA biosynthetic pathway. One of skill in the art appreciates standard resource materials that describe: 1) specific conditions and procedures for construction, manipulation and isolation of macromolecules, such as DNA molecules, plasmids, etc.; 2) generation of recombinant DNA fragments and recombinant expression constructs; and 3) screening and isolating of clones. See Sambrook et al., Molecular Cloning: A Laboratory Manual, 2nd ed., Cold Spring Harbor Laboratory: Cold Spring Harbor, NY (1989); Maliga et al., Methods in Plant Molecular Biology, Cold Spring Harbor, NY (1995); Birren et al., Genome Analysis: Detecting Genes, v. 1, Cold Spring Harbor, NY (1998); Birren et al. Genome Analysis: Analyzing DNA, v. 2, Cold Spring Harbor: NY (1998); Plant Molecular Biology: A Laboratory Manual, Clark, ed. Springer: NY (1997).

In general, the choice of sequences included in the construct depends on the desired expression products, the nature of the host cell and the proposed means of separating transformed cells versus non-transformed cells. The skilled artisan is well aware of the genetic elements that must be present on the plasmid vector to successfully transform, select and propagate host cells containing the chimeric gene. Typically, however, the vector or cassette contains sequences directing transcription and translation of the relevant gene(s), a selectable marker and sequences allowing autonomous replication or chromosomal integration. Suitable vectors comprise a region 5 of the gene that controls transcriptional initiation, i.e., a promoter, and a region 3' of the DNA fragment that controls transcriptional termination, i.e., a terminator. It is most preferred when both control regions are derived from genes from the transformed host cell.

Initiation control regions or promoters useful for driving expression of heterologous genes or portions of them in the desired host cell are numerous and well known. These control regions may comprise a promoter, enhancer, silencer, intron sequences, 3' UTR and/or 5' UTR regions, and protein and/or RNA stabilizing elements. Such elements may vary in their strength and specificity. Virtually any promoter (i.e., native, synthetic, or chimeric) capable of directing expression of these genes in the selected host cell is suitable. Expression in a host cell can occur in an induced or constitutive fashion. Induced expression occurs by inducing the activity of a regulatable promoter operably linked to the Pex gene of interest. Constitutive expression occurs by the use of a constituitive promoter operably linked to the gene of interest.

When the host cell is, for example, yeast, transcriptional and translational regions functional in yeast cells are provided, particularly from the host species. See Int'l. App. Pub. No. WO 2006/052870 for preferred transcriptional initiation regulatory regions for use in *Yarrowia lipolytica.* Any of a number of regulatory sequences may be used, depending on whether constitutive or induced transcription is desired, the efficiency of the promoter in expressing the ORF of interest, the ease of construction, etc.

3' non-coding sequences encoding transcription termination signals, i.e., a "termination region", must be provided in a recombinant construct and may be from the 3' region of the gene from which the initiation region was obtained or from a different gene. A large number of termination regions are known and function satisfactorily in a variety of hosts when utilized in both the same and different genera and species from which they were derived. The termination region is selected more for convenience rather than for any particular property. Termination regions may also be derived from various genes native to the preferred hosts.

Particularly useful termination regions for use in yeast are those derived from a yeast gene, particularly *Saccharomyces, Schizosaccharomyces, Candida, Yarrowia* or *Kluyveromyces.* The 3'-regions of mammalian genes encoding γ-interferon and α-2 interferon are also known to function in yeast. The 3'-region can also be synthetic, as one of skill in the art can utilize available information to design and synthesize a 3'-region sequence that functions as a transcription terminator. A termination region may be unnecessary, but is highly preferred.

The vector may comprise a selectable and/or scorable marker, in addition to the regulatory elements described above. Preferably, the marker gene is an antibiotic resistance gene such that treating cells with the antibiotic causes inhibition of growth, or death, of untransformed cells and uninhibited growth of transformed cells. For selection of yeast transformants, any marker that functions in yeast is useful with resistance to kanamycin, hygromycin and the amino glycoside G418 and the ability to grow on media lacking uracil, lysine, histine or leucine being particularly useful.

Merely inserting a gene into a cloning vector does not ensure its expression at the desired rate, concentration, amount, etc. In response to the need for a high expression rate, many specialized expression vectors have been created by manipulating a number of different genetic elements that control transcription, RNA stability, translation, protein stability and location, oxygen limitation, and secretion from the host cell. Some of the manipulated features include: the nature of the relevant transcriptional promoter and terminator sequences, the number of copies of the cloned gene and whether the gene is plasmid-borne or integrated into the genome of the host cell, the final cellular location of the synthesized foreign protein, the efficiency of translation and correct folding of the protein in the host organism, the intrinsic stability of the mRNA and protein of the cloned gene within the host cell and the codon usage within the cloned gene, such that its frequency approaches the frequency of preferred codon usage of the host cell. Each of these may be used in the methods and host cells described herein to further optimize expression of PUFA biosynthetic pathway genes and to diminish expression of a native Pex gene.

After a recombinant construct is created, e.g., comprising a chimeric gene comprising a promoter, ORF and terminator, suitable for disruption or knock out of a native peroxisome biogenesis factor protein and/or expression of genes encoding a PUFA biosynthetic pathway activity, it is placed in a plasmid vector capable of autonomous replication in the host cell or is directly integrated into the genome of the host cell. Integration of expression cassettes can occur randomly within the host genome or can be targeted through the use of constructs containing regions of homology with the host genome sufficient to target recombination with the host locus. Where constructs are targeted to an endogenous locus, all or some of the transcriptional and translational regulatory regions can be provided by the endogenous locus.

When two or more genes are expressed from separate replicating vectors, each vector may have a different means of selection and should lack homology to the other construct(s) to maintain stable expression and prevent reassortment of elements among constructs. Judicious choice of regulatory regions, selection means and method of propagation of the introduced construct(s) can be experimentally determined so that all introduced genes are expressed at the necessary levels to provide for synthesis of the desired products.

Constructs comprising the gene of interest may be introduced into a host cell by any standard technique. These techniques include transformation, e.g., lithium acetate transformation (Methods in Enzymology, 194:186-187 (1991)), protoplast fusion, biolistic impact, electroporation, microinjection, vacuum filtration or any other method that introduces the gene of interest into the host cell.

For convenience, a host cell that has been manipulated by any method to take up a DNA sequence, for example, in an expression cassette, is referred to herein as "transformed" or "recombinant". The transformed host will have at least one copy of the expression construct and may have two or more, depending upon whether the gene is integrated into the genome, amplified, or is present on an extrachromosomal element having multiple copy numbers.

The transformed host cell can be identified by selection for a marker contained on the introduced construct. Alternatively, a separate marker construct may be co-transformed with the desired construct, as many transformation techniques introduce many DNA molecules into host cells. Typically, transformed hosts are selected for their ability to grow on selective media. Selective media may incorporate an antibiotic or lack a factor necessary for growth of the untransformed host, such as a nutrient or growth factor. An introduced marker gene may confer antibiotic resistance, or encode an essential growth factor or enzyme, thereby permitting growth on selective media when expressed in the transformed host. Selection of a transformed host can also occur when the expressed marker protein can be detected, either directly or indirectly. The marker protein may be expressed alone or as a fusion to another protein. The marker protein can be detected by its enzymatic activity (e.g., β-galactosidase can convert the substrate X-gal ["5-bromo-4-chloro-3-indolyl-β-D-galactopyranoside"] to a colored product luciferase can convert luciferin to a light-emitting product) or its light-producing or modifying characteristics (e.g., the green fluorescent protein of *Aequorea victoria* fluoresces when illuminated with blue light). Alternatively, antibodies can be used to detect the marker protein or a molecular tag on, fo example, a protein of interest. Cells expressing the marker protein or tag can be selected, for example, visually, or by techniques such as fluorescence-activated cell sorting or panning using antibodies.

Regardless of the selected host or expression construct, multiple transformants must be screened to obtain a strain or plant line displaying the desired expression level, regulation and pattern, as different independent transformation events result in different levels and patterns of expression (Jones et al., EMBO J., 4:2411-2418 (1985); De Almeida et al., Mol. Gen. Genetics, 218:78-86 (1989)). Such screening may be accomplished by Southern analysis of DNA blots (Southern, J. Mol. Biol., 98:503 (1975)), Northern analysis of mRNA expression (Kroczek, J. Chromatogr. Biomed. Appl., 618(1-2):133-145 (1993)), Western and/or Elisa analyses of protein expression, phenotypic analysis or GC analysis of the PUFA products.

### Preferred Eukaryotic Host Organisms

A variety of eukaryotic organisms are suitable as host herein, to thereby yield a transformant host organism comprising a disruption in a native peroxisome biogenesis factor protein and genes encoding a PUFA biosynthetic pathway, wherein the transformed eukaryotic host organism has an increased amount of PUFAs incorporated into the total lipid fraction and in the oil fraction, as a percent of total fatty acids, as compared to a eukaryotic organism whose native peroxisome biogenesis factor protein has not been disrupted. Various mammalian systems, plant cells, fungi, algae, oomycetes yeasts, stramenopiles and/or euglenoids may be useful hosts. Non-oleaginous organisms also have utility herein such that, when one of their native PEX genes is disrupted, an increase in the weight percent of at least one polyunsaturated fatty acid relative to the weight percent of total fatty acids in the total lipid fraction or in the oil fraction will be experienced and may lead to a 1.3 fold increase in the PUFA. Additionally, the percent of the PUFA may be increased relative to the dry cell weight in the non-oleaginous organism. Alternatively, a non-oleaginous organism can be genetically modified to become oleaginous, e.g., yeast such as *Saccharomyces cerevisiae.*

Oleaginous organisms are naturally capable of oil synthesis and accumulation, wherein the total oil content typically comprises greater than about 25% of the cellular dry weight. Various algae, moss, fungi, yeast, stramenopiles and plants are naturally classified as oleaginous.

Oleaginous microbes include those algal, stramenopile and fungal organisms that naturally produce ω-3/ω-6 PUFAs. For example, ARA, EPA and/or DHA is produced via *Cyclotella* sp., *Nitzschia* sp., *Pythium, Thraustochytrium* sp., *Schizochytrium* sp. and *Mortierella.* The method of transformation of M. *alpina* is described by Mackenzie et al. (Appl. Environ. Microbiol., 66:4655 (2000)). Similarly, methods for transformation of Thraustochytriales microorganisms (e.g., *Thraustochytrium, Schizochytrium*) are disclosed in U.S. Pat. No. 7,001,772.

Preferred are oleaginous yeast, including those that naturally produce and those genetically engineered to produce ω-3/ω-6 PUFAs. Genera typically identified as oleaginous yeast include, but are not limited to: *Yarrowia, Candida, Rhodotorula, Rhodosporidium, Cryptococcus, Trichosporon* and *Lipomyces.* More specifically, illustrative oil-synthesizing yeasts include: *Rhodosporidium toruloides, Lipomyces starkeyii, L. lipoferus, Candida revkaufi, C. pulcherrima, C. tropicalis, C. utilis, Trichosporon pullans, T. cutaneum, Rhodotorula glutinus, R. graminis* and *Yarrowia lipolytica* (formerly classified as *Candida lipolytica*).

Most preferred is the oleaginous yeast *Yarrowia lipolytica;* and, in a further embodiment, most preferred are the *Y. lipolytica* strains designated as ATCC #76982, ATCC #20362, ATCC #8862, ATCC #18944 and/or LGAM S(7)1 (Papanikolaou S., and Aggelis G., Bioresour. Technol., 82(1):43-9 (2002)).

Specific teachings relating to transformation of *Yarrowia lipolytica* include U.S. Pat. No. 4,880,741 and U.S. Pat. No. 5,071,764 and Chen, D. C et al. (Appl. Microbiol. Biotechnol., 48(2):232-235 (1997)), while suitable selection techniques are described in U.S. Pat. No. 7,238,482 and Int'l. App. Pub. Nos. WO 2005/003310 and WO 2006/052870.

The preferred method of expressing genes in *Yarrowia lipolytica* is by integration of linear DNA into the genome of the host. Integration into multiple locations within the genome can be particularly useful when high level expression of genes are desired, such as in the *Ura3 locus* (GenBank Accession No. AJ306421), the *Leu2* gene locus (GenBank Accession No. AF260230), the *Lys5* gene locus (GenBank Accession No. M34929), the *Aco2 gene* locus (GenBank Accession No. AJ001300), the *Pox3* gene locus (Pox3: GenBank Accession No. XP_503244 or Aco3: GenBank Accession No. AJ001301), the Δ12 desaturase gene locus (U.S. Pat. No. 7,214,491), th *Lip1* gene locus (GenBank Accession No. Z50020), the *Lip2* gene locus (GenBank Accession No. AJ012632), the *SCP2 gene* locus (GenBank Accession No. AJ431362), the *Pex3* gene locus (GenBank Accession No. CAG78565), the *Pex16* gene locus (GenBank Accession No. CAG79622) and/or the *Pex10* gene locus (GenBank Accession No. CAG81606).

Preferred selection methods for use in *Yarrowia lipolytica* are resistance to kanamycin, hygromycin and the amino glycoside G418, as well as ability to grow on media lacking uracil, leucine, lysine, tryptophan or histidine. 5-fluoroorotic acid [5-fluorouracil-6-carboxylic acid monohydrate or "5-FOA"] may also be used for selection of yeast *Ura*⁻ mutants. This compound is toxic to yeast cells that possess a functioning URA3 gene encoding orotidine 5'-monophosphate decarboxylase [OMP decarboxylase]; thus, based on this toxicity, 5-FOA is especially useful for the selection and identification of *Ura*⁻ mutant yeast strains (Bartel, P.L. and Fields, S., Yeast 2 Hybrid System, Oxford University: New York, v. 7, pp 109-147, 1997; see also Int'l. App. Pub. No. WO 2006/052870 for 5-FOA use in *Yarrowia*)*.*

An alternate preferred selection method for use in *Yarrowia* relies on a dominant, non-antibiotic marker for *Yarrowia lipolytica* based on sulfonylurea (chlorimuron ethyl; E. I. duPont de Nemours & Co., Inc., Wilmington, DE) resistance. More specifically, the marker gene is a native acetohydroxyacid synthase ("AHAS" or acetolactate synthase; E.C. 4.1.3.18) that has a single amino acid change, i.e., W497L, that confers sulfonyl urea herbicide resistance (Int'l. App. Pub. No. WO 2006/052870). AHAS is the first common enzyme in the pathway for the biosynthesis of branched-chain amino acids, i.e., valine, leucine, isoleucine, and it is the target of the sulfonylurea and imidazolinone herbicides.

### Fermentation Processes For Polyunsaturated Fatty Acid Production

The transformed host cell is grown under conditions that optimize expression of PUFA biosynthetic genes and produce the greatest and most economical yield of desired PUFAs. In general, media conditions may be optimized by modifying the type and amount of carbon source, the type and amount of nitrogen source, the carbon-to-nitrogen ratio, the amount of different mineral ions, the oxygen level, growth temperature, pH, length of the biomass production phase, length of the oil accumulation phase and the time and method of cell harvest. Oleaginous yeast of interest, such as *Yarrowia lipolytica,* are generally grown in a complex medium such as yeast extract-peptone-dextrose broth (YPD) or a defined minimal media that lacks a component necessary for growth and forces selection of the desired expression cassettes (e.g., Yeast Nitrogen Base (DIFCO Laboratories, Detroit, MI)).

Fermentation media for the methods and host cells described herein must contain a suitable carbon source such as are taught in U.S. Pat. No. 7,238,482. Suitable sources of carbon encompass a wide variety of sources with sugars, glycerol and/or fatty acids being preferred. Most preferred is glucose and/or fatty acids containing between 10-22 carbons.

Nitrogen may be supplied from an inorganic (e.g., (NH₄)₂SO₄) or organic (e.g., urea or glutamate) source. In addition to appropriate carbon and nitrogen sources, the fermentation media must also contain suitable minerals, salts, cofactors, buffers, vitamins and other components known to those skilled in the art suitable for the growth of the oleaginous yeast and the promotion of the enzymatic pathways of PUFA production. Particular attention is given to several metal ions, such as Fe⁺², Cu⁺², Mn⁺², Co⁺², Zn⁺² and Mg⁺², that promote synthesis of lipids and PUFAs (Nakahara, T. et al., Ind. Appl. Single Cell Oils, D. J. Kyle and R. Colin, eds. pp 61-97 (1992)).

Preferred growth media for the methods and host cells described herein are common commercially prepared media, such as Yeast Nitrogen Base (DIFCO Laboratories, Detroit, MI). Other defined or synthetic growth media may also be used and the appropriate medium for growth of the transformant host cells is well known in microbiology or fermentation science A suitable pH range for the fermentation is typically between about pH 4.0 to pH 8.0, wherein pH 5.5 to pH 7.5 is preferred as the range for the initial growth conditions. The fermentation may be conducted under aerobic or anaerobic conditions, wherein microaerobic conditions are preferred.

Typically, accumulation of increased amounts of PUFAs and TAGs in oleaginous yeast cells requires a two-stage process, since the metabolic state must be "balanced" between growth and synthesis/storage of fats. Thus, most preferably, a two-stage fermentation process is necessary for the production of oils in oleaginous yeast. This approach is described in U.S. Pat. No. 7,238,482, as are various suitable fermentation process designs (i.e., batch, fed-batch and continuous) and considerations during growth.

### Purification And Processing Of PUFA Oils

Fatty acids, including PUFAs, may be found in the host organisms as free fatty acids or in esterified forms such as acylglycerols, phospholipids, sulfolipids or glycolipids. These fatty acids may be extracted from the host cells through a variety of means well-known in the art. One review of extraction techniques, quality analysis and acceptability standards for yeast lipids is that of Z. Jacobs (Critical Reviews in Biotechnology, 12(5/6):463-491 (1992)). A brief review of downstream processing is also available by A. Singh and O. Ward (Adv. Appl. Microbiol., 45:271-312 (1997)).

In general, means for the purification of fatty acids (including PUFAs) may include extraction (e.g., U.S. Pat. No. 6,797,303 and U.S. Pat. No. 5,648,564) with organic solvents, sonication, supercritical fluid extraction (e.g., using carbon dioxide), saponification and physical means such as presses, or combinations thereof. See U.S. Pat. No. 7,238,482.

### Oils For Use In Foodstuffs. Health Food Products. Pharmaceuticals And Animal Feeds

The market place contains many food and feed products, incorporating ω-3 and/or ω-6 fatty acids, particularly ALA, GLA, ARA, EPA, DPA and DHA. It is contemplated that the microbial biomass comprising long-chain PUFAs, partially purified microbial biomass comprising PUFAs, purified microbial oil comprising PUFAs, and/or purified PUFAs made by the methods and host cells described herein impart health benefits, upon ingestion of foods or feed improved by their addition. These oils can be added to food analogs, drinks meat products, cereal products, baked foods, snack foods and dairy products to name a few. See U.S. Pat. App. Pub. No. 2006/0094092.

These compositions may impart health benefits by being added to medical foods including medical nutritionals, dietary supplements, infant formula and pharmaceuticals. The skilled artisan will appreciate the amount of the oils to be added to food, feed, dietary supplements, nutriceuticals, pharmaceuticals, and other ingestible products as to impart health benefits. Health benefits from ingestion of these oils are described in the art, known to the skilled artisan and continuously investigated. Such an amount is referred to herein as an "effective" amount and depends on, among other things, the nature of the ingested products containing these oils and the physical conditions they are intended to address.

### DESCRIPTION OF PREFERRED EMBODIMENTS

As demonstrated in the Examples and summarized in Table 5, *infra,* disruptions in the C-terminal portion of the C₃HC₄ zinc ring finger motif of YIPex10p, deletion of the entire chromosomal YIPex10 gene or of the entire chromosomal YIPex16 gene, deletion of both the entire chromosomal YIPex10 and the YIPex16 gene, and deletion of the entire chromosomal YIPex3 gene all resulted in an engineered PUFA-producing strain of *Yarrowia lipolytica* that had an increased weight percent of PUFAs as a percent of total fatty acids, relative to the parental strain whose native Pex protein had no disruption. Expression of an extrachromosomal YIPex10p in an engineered EPA-producing strain of *Yarrowia lipolytica* that possessed a disruption in the genomic Pex10p and an increased amount of PUFAs in the total lipid fractior and in the oil fraction reversed the effect.

Table 5 compiles data from Examples 3, 4, 5, 7, 9, 11 and 12, such that trends concerning total lipid content ["TFAs % DCW"], concentration of a given fatty acid(s) expressed as a weight percent of total fatty acids ["% TFAs"], and content of a given fatty acid(s) as its percent of the dry cell weight ["% DCW"] can be deduced, based on the presence/absence of a Pex disruption or knockout. "Desired PUFA % TFAs" and "Desired PUFA % DCW" quantify the particular concentration or content, respectively, of the desired PUFA product (i.e., DGLA or EPA)that the engineered PUFA biosynthetic pathway was designed to produce. "All PUFAs" includes LA, ALA, EDA, DGLA, ETrA, ETA and EPA, while "C20 PUFAs" is limited to EDA DGLA, ETrA, ETA and EPA.

**Table 5**

| PUFA % TFAs and % DCW In *Yarrowia lipolytica* Strains With Mutant Pex Genes | | | | | | | | | |
|---|---|---|---|---|---|---|---|---|---|
| Example | Strain | Genomic Pex Gene | TFA % DCW | % TFAs | | | % DCW | | |
| | | | | Desired PUFA | All PUFAs | C20 PUFAs | Desired PUFA | All PUFAs | C20 PUFAs |
| 3,4 | Y4086 | Wildtype Pex10 | 28.6 | 9.8 [EPA] | 60.1 | 25.2 | 2.8 [EPA] | 17.2 | 7.2 |
| | Y4128 | Mutant* Pex10 | 11.2 | 42.8 [EPA] | 79.3 | 57.9 | 4.8 [EPA] | 8.9 | 6.4 |
| 5 | Y4128U1 + pFBAIn- PEX10 | Mutant* Pex10 + Plasmid Wildtype Pex10 within chimeric FBAINm::Pex10::Pex20 gene | 29.2 | 10.8 [EPA] | 60 | 27.3 | 3.1 [EPA] | 17.5 | 8.0 |
| | Y4128U1 + pPEX10-1 | Mutant* Pex10 + Plasmid Wildtype Pex10 within Pex10-5' (500 bp):: Pex10::Pex10-3' gene | 27.1 | 10.7 [EPA] | 60.1 | 26.7 | 2.9 [EPA] | 16.2 | 7.2 |
| | Y4128U1 + pPEX10-2 | Mutant* Pex10 + Plasmid Wildtype Pex10 within Pex10-5' (991 bp):: Pex10::Pex10-3' gene | 28.5 | 10.8 [EPA] | 59 | 26.9 | 3.1 [EPA] | 16.8 | 7.7 |
| | Y4128U1 + control | Mutant* Pex10 | 22.8 | 27.7 [EPA] | 62.6 | 42.3 | 6.3 [EPA] | 14.2 | 9.6 |
| 7 | Y4184U | Wildtype Pex10 | 11.8 | 20.6 [EPA] | nq^{◆} | nq^{◆} | 2.4 [EDA] | nq^{◆} | nq^{◆} |
| | | | 8.8 | 23.2 [EPA] | nq^{◆} | nq^{◆} | 2.0 [EPA] | nq^{◆} | nq^{◆} |
| | Y4184U | Mutant Pex10 | 17.6 | 43. [EPA]2 | nq^{◆} | nq^{◆} | 7.6 [EPA] | nq^{◆} | nq^{◆} |
| | ΔPex10 | | 13.2 | 46.1 [EPA] | nq^{◆} | nq^{◆} | 6.1 [EPA] | nq^{◆} | nq^{◆} |
| 9 | Y4036 (avg) | Wildtype Pex16 | Nq^{◆} | 23.4 [DGLA] | 61.5 | 33.7 | nq^{◆} | nq^{◆} | nq^{◆} |
| | Y4036 (ΔPex16) (avg) | Mutant Pex16 | Nq^{◆} | 43.4 [DGLA] | 69.1 | 49.1 | nq^{◆} | nq^{◆} | nq^{◆} |
| 11 | Y4305U (Δpex10) (avg) | Mutant Pex10 and Wildtype Pex16 | 30 | 44.7 [EPA] | 76.6 | 55.4 | 13.4 [EPA] | 23.0 | 16.6 |
| | Y4305 (ΔPex10, ΔPex16) (avg) | Mutant Pex10, Mutant Pex16 | 30 | 48.3 [EPA] | 79.0 | 57.7 | 14.5 [EPA] | 23.7 | 17.3 |
| 12 | Y4036 | Wildtype Pex3 | 4.7 | 19 [DGLAI | 57 | 27 | 0.9 [DGLA] | 2.7 | 1.3 |
| | Y4036 (ΔPex3) | Mutant Pex3 | 6.1 | 46 [DGLA] | 68 | 56 | 2.8 [DGLA] | 4.4 | 3.4 |
| | | | 5.9 | 46 [DGLA] | 68 | 56 | 2.7 [DGLA] | 4.0 | 3.3 |

| | | | | | | | | | |
|---|---|---|---|---|---|---|---|---|---|
| * Pex10 disruption in Y4128 results in a truncated protein, wherein the last 32 amino acids of the C-terminus (corresponding to the C-terminal portion of the C₃HC₄ zinc ring finger motif) are not present. ^{◆} nq = not quantified | | | | | | | | | |

Although data cannot be directly compared between Examples, as a result of different *Yarrowia* strains and growth conditions, the following conclusions can be drawn (relative to the parental strain whose native Pex protein had not been disrupted or the parental strain that was expressing a "replacement" copy of the disrupted native Pex protein):
1) Pex disruption in a PUFA-producing *Yarrowia* results in an increase in the weight percent of a single PUFA, for example EPA or DLGA, relative to the weight percent of total fatty acids (% TFAs) in the total lipid fraction and in the oil fraction;
2) Pex disruption in a PUFA-producing *Yarrowia* results in an increase in the weight percent of C₂₀ PUFAs relative to the weight percent of total fatty acids in the total lipid fraction and in the oil fraction;
3) By the extension of point 1), Pex disruption in a PUFA-producing *Yarrowia* results in an increase in the amount of any and all combinations of PUFAs relative to the weight percent of total fatty acids in the total lipid fraction and in the oil fraction; and
4) Pex disruption in a PUFA-producing *Yarrowia* results in an increase in the percent of a single PUFA, for example EPA or DLGA, relative to the dry cell weight.

Variable results are observed when comparing the effects of Pex disruptions in "All PUFAs % DCW", "C20 PUFAs % DCW" and TFA % DCW Specifically, in some cases, the Pex disruption in the PUFA-producing *Yarrowia* results in an increased amount of C₂₀ PUFAs or All PUFAs, as a percent of dry cell weight, in the total limpid fraction and in the oil fraction (relative to the parental strain whose native Pex protein had not been disrupted). In other cases, there is a diminished amount of C₂₀ PUFAs or All PUFAs, as a percent of dry cell weight, in the total lipid fraction and in the oil fraction (relative to the parental strain whose native Pex protein had not been disrupted). Similar results are observed with respect to the total lipid content (TFA % DCW), in that the effect of the Pex disruption can either result in an increase in total lipid content or a decrease.

Although each of the above generalizations are of interest, it is particularly useful to examine the effect of the Pex disruptions on the ratio of the desired PUFA which the organism was engineered to produce relative to the amount of total PUFAs.

For example, 54% of the PUFAs (as a % TFAs) were EPA in strain Y4128 containing the Pex10 disruption that resulted in truncation of the last 32 amino acids of the C-terminus, while only 16.3% of the PUFAs (as a % TFAs) were EPA in the parent strain, Y4086. Thus, the disruption was responsible for a 3.3-fold increase in the amount of the desired PUFA (as % TFAs) (Examples 3, 4). In a similar manner, 62.8% of the PUFAs (as a % TFAs) were DGLA in strain Y4036 (ΔPex16), while only 38.1% the PUFAs (a a % TFAs) were DGLA in Y4036-a 1.65 fold increase (Example 9). And, 67.7% of the PUFAs (as a % TFAs) were DGLA in strain Y4036 (ΔPex3), while only 33.3% the PUFAs (as a % TFAs) were DGLA in Y4036-a 2.0 fold increase (Example 12). These results support the hypothesis that the Pex disruption results in a selective increase in the amount, as a % TFAs, of the desired PUFA which the organism was engineered to produce in the total lipid and oil fractions.

Less significant selectivity is observed when examining the effect of Pex disruptions on the ratio of C20 PUFAs relative to the amount of total PUFAs. For example, 73% of the PUFAs (as a % TFAs) were C20 PUFAs in strain Y4128 containing the Pex10 disruption, while only 42% of the PUFAs (as a % TFAs) were C20 PUFAs in strain Y4086. Thus, the disruption was responsible for a 1.7-fold increase in the amount of C20 PUFAs that accumulated in the total lipid and oil fractions, relative to the total PUFAs (Examples 3, 4). In a similar manner, 71% of the PUFAs (as a % TFAs) were C20 PUFAs in strain Y4036 (ΔPex16), while only 54.8% the PUFAs (as a % TFAs) were C20 PUFAs in Y4036-a 1.3 fold increase (Example 9). And, 82.4% of the PUFAs (as a % TFAs) were C20 PUFAs in strain Y4036 (ΔPex3), while only 47.4% the PUFAs (as a % TFAs) were C20 PUFAs in Y4036-a 1.7 fold increase (Example 12).

On the basis of the teachings and results described herein, it is expected that the feasibility and commercial utility of utilizing various disruptions in native genes encoding peroxisome biogenesis factor proteins as a means to increase the amount of PUFAs produced in a PUFA-producing eukaryotic organism will be appreciated. The PUFA-producing eukaryotic organism can synthesize a variety of ω-3 and/or ω-6 PUFAs, using either the Δ9 elongase/Δ8 desaturase pathway or the Δ6 desaturase/ Δ6 elongase pathway.

### EXAMPLES

The present invention is further described in the following Examples, which illustrate reductions to practice of the invention but do not completely define all of its possible variations.

### GENERAL METHODS

Standard recombinant DNA and molecular cloning techniques used in the Examples are well known in the art and are described by: 1) Sambrook, J., Fritsch, E. F. and Maniatis, T., Molecular Cloning: A Laboratory Manual; Cold Spring Harbor Laboratory: Cold Spring Harbor, NY (1989) (Maniatis); 2) T. J. Silhavy, M. L. Bennan, and L. W. Enquist, Experiments with Gene Fusions; Cold Spring Harbor Laboratory: Cold Spring Harbor, NY (1984); and 3) Ausubel, F. M. et al., Current Protocols in Molecular Biology, published by Greene Publishing Assoc. and Wiley-Interscience, Hoboken, NJ (1987).

Materials and methods suitable for the maintenance and growth of microbial cultures are well known in the art. Techniques suitable for use in the following examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, Eds American Society for Microbiology: Washington, D.C. (1994)); or by Thomas D. Brock in Biotechnology: A Textbook of Industrial Microbiology, 2nd ed., Sinauer Associates: Sunderland, MA (1989). All reagents, restriction enzymes and materials used for the growth and maintenance of microbial cells were obtained from Aldrich Chemicals (Milwaukee, WI), DIFCO Laboratories (Detroit, MI), New England Biolabs, Inc. (Beverly, MA), GIBCO/BRL (Gaithersburg, MD), or Sigma Chemical Company (St. Louis, MO), unless otherwise specified. *E*. *coli* strains were typically grown at 37 °C on Luria Bertani (LB) plates.

General molecular cloning was performed according to standard methods (Sambrook et al., *supra*). DNA sequence was generated on an ABI Automatic sequencer using dye terminator technology (U.S. Pat. No. 5,366,860; EP 272,007) using a combination of vector and insert-specific primers. Sequence editing was performed in Sequencher (Gene Codes Corporation, Ann Arbor, MI). All sequences represent coverage at least two times in both directions. Unless otherwise indicated herein comparisons of genetic sequences were accomplished using DNASTAR software (DNASTAR Inc., Madison, WI).

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kB" means kilobase(s).

### Nomenclature For Expression Cassettes:

The structure of an expression cassette is represented by a simple notation system of "X::Y::Z", wherein X describes the promoter fragment, Y describes the gene fragment, and Z describes the terminator fragment, which are all operably linked to one another.

### Transformation And Cultivation Of Yarrowia lipolytica

*Yarrowia lipolytica* strain ATCC #20362 was purchased from the American Type Culture Collection (Rockville, MD). *Yarrowia lipolytica* strains were routinely grown at 28-30 °C in several media, according to the recipes shown below. Agar plates were prepared as required by addition of 20 g/L agar to each liquid media, according to standard methodology.
YPD agar medium (per liter): 10 g of yeast extract [Difco], 20 g of Bacto peptone [Difco], and 20 g of glucose.
Basic Minimal Media (MM) (per liter): 20 g glucose, 1.7 g yeast nitrogen base without amino acids, 1.0 g proline, and pH 6.1 (not adjusted).
Minimal Media + Uracil (MM+uracil or MMU (per liter): Prepare MM media as above and add 0.1 g uracil and 0.1 g uridine.
Minimal Media + Uracil + Sulfonylurea (MMU+SU) (per liter): Prepare MMU media as above and add 280 mg sulfonylurea.
Minimal Media + Leucine + Lysine (MMLeuLys) (per liter): Prepare MM media as above and add 0.1 g leucine and 0.1 g lysine.
Minimal Media + 5-Fluoroorotic Acid (MM + 5-FOA) (per liter): 20 g glucose, 6.7 g Yeast Nitrogen base, 75 mg uracil, 75 mg uridine and appropriate amount of FOA (Zymo Research Corp., Orange, CA), based on FOA activity testing against a range of concentrations from 100 mg/L to 1000 mg/L (since variation occurs within each batch received from the supplier).
High Glucose Media (HGM) (per liter): 80 glucose, 2.58 g KH₂PO₄ and 5.36 g K₂HPO₄, pH 7.5 (do not need to adjust).
Fermentation medium without Yeast Extract (FM without YE) (per liter): 6.70 g Yeast Nitrogen base, 6.00 g KH₂PO₄, 2.00 g K₂HPO₄, 1.50 g MgSO₄*7H₂O and 20 g Glucose.
Fermentation medium (FM) (per liter): Prepare FM without YE media as above and add 5.00 g Yeast extract (BBL).
Synthetic Dextrose Media (SD) (per liter): 6.7 g Yeast Nitrogen base with ammonium sulfate and without amino acids; and 20 g glucose.
Complete Minimal Glucose Broth Minus Uracil (CSM-Ura): Catalog No. C8140, Teknova, Hollister, CA (0.13% amino acid dropout powder minus uracil. 0.17% yeast nitrogen base, 0.5%(NH₄)₂SO₄, 2.0% glucose).

Transformation of *Y. lipolytica* was performed according to the method of Chen, D. C. et al. (Appl. Microbiol.Biotechnol., 48(2):232-235 (1997)), unless otherwise noted. Briefly, *Yarrowia* was streaked onto a YPD plate and grown at 30 °C for approximately 18 hr. Several large loopfuls of cells were scraped from the plate and resuspended in 1 mL of transformation buffer containing: 2.25 mL of 50% PEG, average MW 3350; 0.125 mL of 2 M Li acetate, pH 6.0; and 0.125 mL of 2 M DTT. Then, approximately 500 ng of linearized plasmid DNA was incubated in 100 µl of resuspended cells, and maintained at 39 °C for 1 hr with vortex mixing at 15 min intervals. The cells were plated onto selection media plates and maintained at 30 °C for 2 to 3 days.

### Fatty Acid Analysis Of Yarrowia lipolytica

For fatty acid analysis, cells were collected by centrifugation and lipids were extracted as described in Bligh, E. G. & Dyer, W. J. (Can. J. Biochem. Physiol., 37:911-917 (1959)). Fatty acid methyl esters ["FAMEs"] were prepared by transesterification of the lipid extract with sodium methoxide (Roughan, G., and Nishida I., Arch Biochem Biophys., 276(1):38-46 (1990)) and subsequently analyzed with a Hewlett-Packard 6890 GC fitted with a 30-m X 0.25 mm (i.d.) HP-INNOWAX (Hewlett-Packard) column. The oven temperature was from 170 °C (25 min hold) to 185 °C at 3.53C/min.

For direct base transesterification, *Yarrowia* culture (3 mL) was harvested, washed once in distilled water, and dried under vacuum in a Speed-Vac for 5-10 min. Sodium methoxide (100 µl of 1 %) was added to th sample, and then the sample was vortexed and rocked for 20 min. After adding 3 drops of 1 M NaCl and 400 µl hexane, the sample was vortexed and spun. The upper layer was removed and analyzed by GC as described above.

### EXAMPLE 1

### Generation Of Yarrowia lipolytica Strain Y4086 To Produce About 14% EPA Of Total Lipids Via The Δ9 Elongase/Δ8 Desaturase Pathway

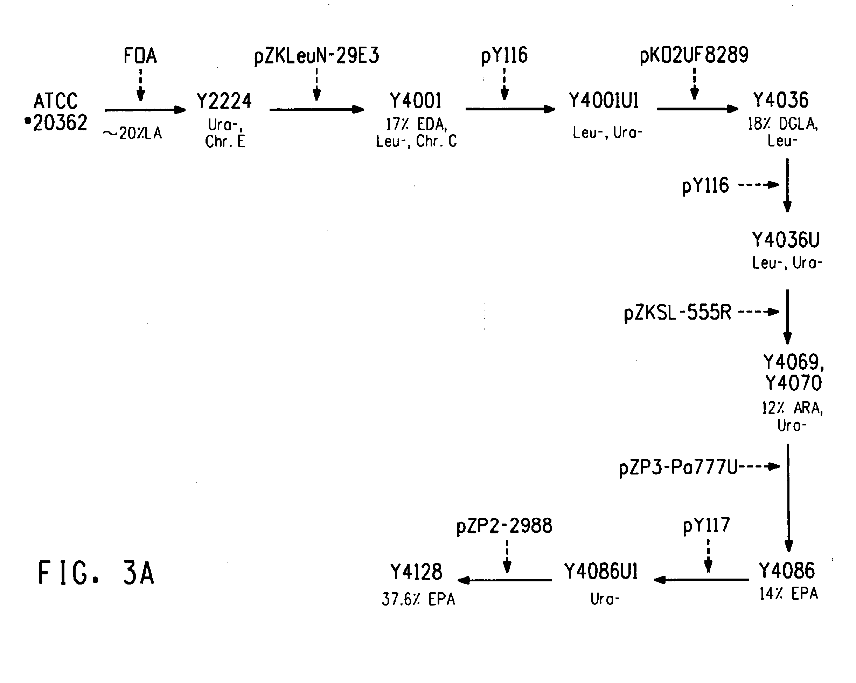

The present Example describes the construction of strain Y4086, derived from *Yarrowia lipolytica* ATCC #20362, capable of producing about 14% EPA relative to the total lipids via expression of a Δ9 elongase/Δ8 desaturase pathway (FIG. 3A).

The development of strain Y4086 required the construction of strain Y2224 (a FOA resistant mutant from an autonomous mutation of the *Ura3* gene of wildtype *Yarrowia* strain ATCC #20362), strain Y4001 (producing 17% EDA with a *Leu*- phenotype), strain Y4001 U (*Leu-* and *Ura-* phenotype strain Y4036 (producing 18% DGLA with a *Leu-* phenotype), strain Y4036U (*Leu-* and *Ura-* phenotype) and strain Y4070 (producing 12% ARA with *a Ura* phenotype). Further details regarding the construction of strains Y2224, Y4001, Y4001 U, Y4036, Y4036U and Y4070 are described in Example 7 of Int'l. App. Pub. No. WO 2008/073367.

The final genotype of strain Y4070 with respect to wildtype *Yarrowia lipolytica* ATCC #20362 was *Ura3-, unknown 1-, unknown 3-, Leu+, Lys+,* GPD::FmD12::Pex20, YAT1::FmD12::OCT, YAT1::ME3S::Pex16, GPAT::EgD9e::Lip2, EXP1::EgD9eS::Lip1, FBAINm::EgD9eS::Lip2, FBAINm::EgDBM::Pex20, EXP1::EgD8M::Pex16, FBAIN::EgD5::Aco, EXP1::EgD5S::Pex20, YAT1::RD5S::OCT (wherein FmD12 is a *Fusarium moniliforme* Δ12 desaturase gene [Int'l. App. Pub. No. WO 2005/047485]; ME3S is a codon-optimized C_{16/18} elongase gene, derived from *Mortierella alpina* [Int'l. App. Pub. No. WO 2007/046817]; EgD9e is a *Euglena gracilis* Δ9 elongase gene [Int'l. App. Pub. No. WO 2007/061742]; EgD9eS is a codon-optimized Δ9 elongase gene, derived from *Euglena gracilis* [Int'l. App. Pub. No. WO 2007/061742]; EgD8M is a synthetic mutant Δ8 desaturase [Int'l. App. Pub. No. WO 2008/073271], derived from *Euglena gracilis* [U.S. Pat. No. 7,256,033]; EgD5 is a *Euglena gracilis* Δ5 desaturase [U.S. Pat. App. Pub. US 2007-0292924-A1]; EgD5S is a codon-optimized Δ5 desaturase gene, derived from *Euglena gracilis* [U.S. Pat. App. Pub. No. 2007-0292924] and RD5S is a codon-optimized Δ5 desaturase, derived from *Peridinium sp.* CCMP626 [U.S. Pat. App. Pub. No. 2007-0271632]).

### Generation Of Y4086 Strain To Produce About 14% EPA Of Total Lipids

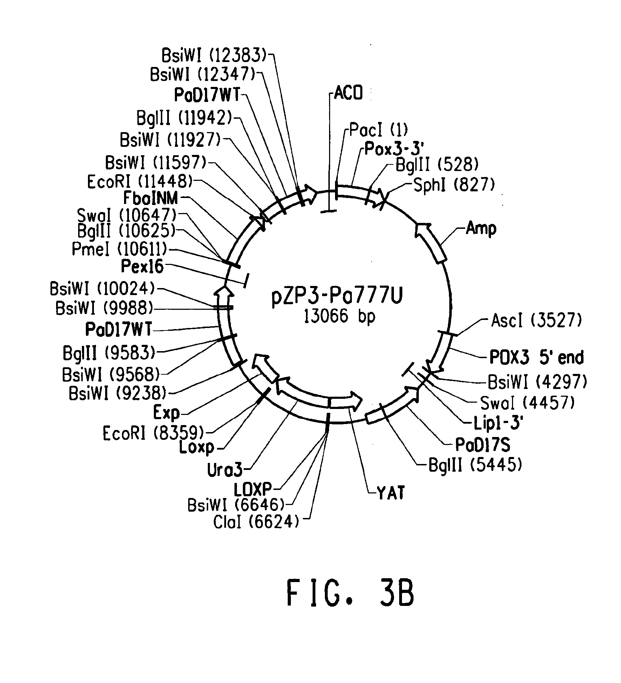

Construct pZP3-Pa777U (FIG. 3B; SEQ ID NO:28), described in Table 19 of Int'l. App. Pub. No. WO 2008/07336 was generated to integrate three Δ17 desaturase genes into the *Pox3* loci (GenBank Accession No. AJ001301 of strain Y4070, to thereby enable production of EPA. The Δ17 desaturase genes were PaD17, a *Pythium aphanidermatum* Δ17 desaturase (Int'l. App. Pub. No. WO 2008/054565), and PaD17S, a codon-optimized Δ17 desaturase derived from *Pythium aphanidermatum* (Int'l. App. Pub. No. WO 2008/054565).

The pZP3-Pa777U plasmid was digested with *AscI*/*SphI,* and then used for transformation of strain Y4070 according to the General Methods. The transformant cells were plated onto MM plates and maintained at 30 °C for 2 to 3 days. Single colonies were re-streaked onto MM plates, and then inoculated into liquid MMLeuLys at 30 °C and shaken at 250 rpm/min for 2 days. The cells were collected by centrifugation, lipids were extracted, and FAMEs were prepared by trans-esterification, and subsequently analyzed with a Hewlett-Packard 6890 GC.

GC analyses showed the presence of EPA in the transformants containing the 3 chimeric genes of pZP3-Pa777U, but not in the parent Y407 strain. Most of the selected 96 strains produced 10-13% EPA of total lipids. There were 2 strains (i.e., #58 and #79) that produced about 14.2% and 13.8% EPA of total lipids. These two strains were designated as Y4085 and Y4086, respectively.

The final genotype of strain Y4086 with respect to wildtype *Yarrowia lipolytica* ATCC #20362 was *Ura3*+*, Leu*+*, Lys*+*, unknown 1-, unknown 2-, YALI0F24167g-,* GPD::FmD12::Pex20, YAT1::FmD12::OCT, YAT1::ME3S::Pex16, GPAT::EgD9e::Lip2, EXPI::EgD9eS::Lip1, FBAINm::EgD9eS::Lip2, FBAINm::EgD8M::Pex20, EXP1::EgD8M::Pex16, FBAIN::EgD5::Aco, EXP1::EgD5S::Pex20, YAT1::RD5S::OCT, YAT1::PaD17S::Lip1, EXP1::PaD17::Pex16, FBAINm::PaD17::Aco.

### EXAMPLE 2

### Generation Of Yarrowia lipolytica Strain Y4128 To Produce About 37% EPA Of Total Lipids Via The Δ9 Elongase/Δ8 Desaturase Pathway

The present Example describes the construction of strain Y4128, derived from *Yarrowia lipolytica* ATCC #20362, capable of producing about 37.6% EPA relative to the total lipids (i.e., greater than a 2-fold increase in EPA concentration as percent of total fatty acids with respect to Y4086; FIG. 3A).

The development of strain Y4128 required the construction of strains Y2224, Y4001, Y4001 U, Y4036, Y4036U, Y4070 and Y4086 (described in Example 1), as well as construction of strain Y4086U1 *(Ura-).*

### Generation Of Strain Y4086U1 (Ura-)

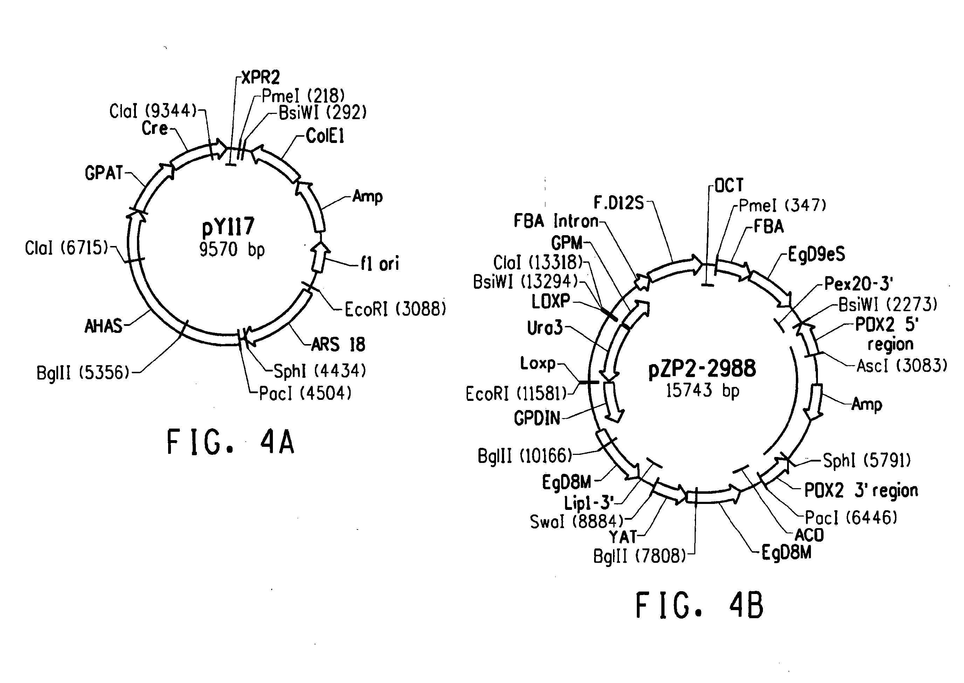

Strain Y4086U1 was created via temporary expression of the *Cre* recombinase enzyme in construct pY117 (FIG. 4A; SEQ ID NO:29; described in Table 20 of Int'l. App. Pub. No. WO 2008/073367) within strain Y4086 to produce a *Ura-* phenotype. This released the LoxP sandwiched *Ura3* gene from the genome. The mutated *Yarrowia* acetohydroxyacid synthase ["AHAS"; E.C. 4.1.3.18] enzyme (i.e., GenBank Accession No. XP_501277, comprising a W497L mutation as set forth in SEQ ID NO:27; see Int'l. App. Pub. No. WO 2006/052870) in plasmid pY117 conferred sulfonyl urea herbicide resistance (SU^{R}), which was used as a positive screening marker.

Plasmid pY117 was used to transform strain Y4086 according to the General Methods. Following transformation, the cells were plated onto MMU+SU (280 µg/mL sulfonylurea; also known as chlorimuron ethyl, E. I. duPont de Nemours & Co., Inc., Wilmington, DE) plates and maintained at 30 °C for 2 to 3 days. The individual SU^{R} colonies grown on MMU+SU plates were picked, and streaked into YPD liquid media at 30°C and shaken at 250 rpm/min for 1 day to cure the pY117 plasmid. The grown cultures were streaked onto MMU plates. After two days at 30 °C, the individual colonies were re-streaked onto MM and MMU plates. Those colonies that could grow on MMU, but not on MM plates were selected. Two of these strains with *Ura* phenotypes were designated as Y4086U1 and Y4086U2.

### Generation Of Y4128 Strain To Produce About 37% EPA Of Total Lipids

Construct pZP2-2988 (FIG. 4B; SEQ ID NO:30; described in Table 21 of Int'l. App. Pub. No. WO 2008/073367) was generated to integrate one Δ12 desaturase gene (i.e., FmD12S, a codon-optimized Δ12 desaturase gene derived from *Fusarium moniliforme* [Int'l. App. Pub. No. WO 2005/047485]), two Δ8 desaturase genes (i.e., EgD8M) and one Δ9 elongase gene (i.e., EgD9eS) into the Pox2 loci (GenBank Accession No. AJ001300) of strain Y4086U1, to thereby enable higher level production of EPA. The pZP2-2988 plasmid was digested with *AscIlSphI,* and then used for transformation of strain Y4086U1 according to the General Methods. The transformant cells were plated onto MM plates and maintained at 30 °C for 2 to 3 days. Single colonies were re-streaked onto MM plates, and then inoculated into liquid MMLeuLys at 30°C and shaken at 250 rpm/min for 2 days. The cells were collected by centrifugation, resuspended in HGM and then shaken at 250 rpm/min for 5 days. The cells were collected by centrifugation, lipids were extracted, and FAMEs were prepared by trans-esterification, and subsequently analyzed with a Hewlett-Packard 6890 GC.

GC analyses showed that most of the selected 96 strains produced 12 15.6% EPA of total lipids. There were 2 strains (i.e., #37 within Group I and #33 within Group II) that produced about 37.6% and 16.3% EPA of total lipids. These two strains were designated as Y4128 and Y4129, respectively

The final genotype of strain Y4128 with respect to wildtype *Yarrowia lipolytica* ATCC #20362 was: *YALI0F24167g-, Pex10-, unknown 1-, unknown* 2-, GPD::FmD12::Pex20, YAT1::FmD12::OCT, GPM/FBAIN::FmD12S::OCT, YAT1::ME3S::Pex16, GPAT::EgD9e::Lip2, EXP1::EgD9eS::Lip1, FBAINm::EgD9eS::Lip2, FBA::EgD9eS::Pex20, FBAINm::EgD8M::Pex20, EXP1::EgD8M::Pex16, GPDIN::EgD8M::Lip1, YAT1::EgD8M::Aco, FBAIN::EgD5::Aco, EXP1::EgD5S::Pex20, YAT1::RD5S::OCT, YAT1::PaD17S::Lip1, EXP1::PaD17::Pex16, FBAINm::PaD17::Aco.

*Yarrowia lipolytica* strain Y4128 was deposited with the American Type Culture Collection on August 23, 2007 and bears the designation ATCC PTA 8614.

### Generation Of Y4128U Strains With A Ura- Phenotype

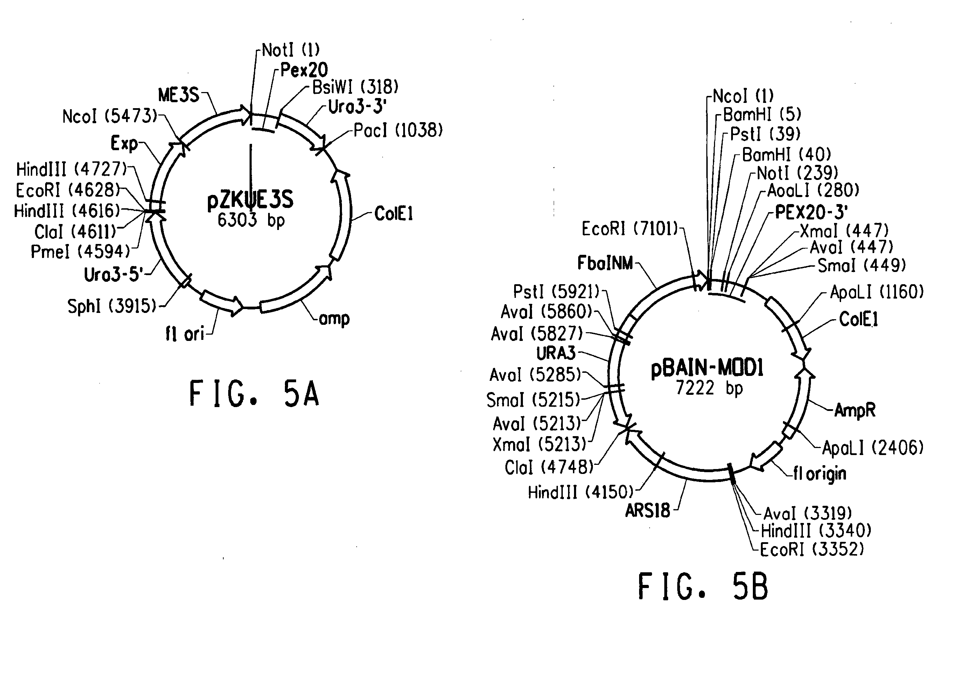

In order to disrupt the Ura3 gene in strain Y4128, construct pZKUE3S (FIG. 5A; SEQ ID NO:31; described in Table 22 of Int'l. App. Pub. No. WO 2008/073367) was created to integrate a EXP1::ME3S::Pex20 chimeric gene into the *Ura3* gene of strain Y4128. Plasmid pZKUE3S was digested with *Sph*I/*PacI,* and then used to transform strain Y4128 according to the General Methods. Following transformation, cells were plated onto MM + 5-FOA selection plates and maintained at 30 °C for 2 to 3 days.

A total of 24 transformants grown on MM + 5-FOA selection plates were picked and re-streaked onto fresh MM + 5-FOA plates. The cells were stripped from the plates, lipids were extracted, and FAMEs were prepared by trans-esterification, and subsequently analyzed with a Hewlett-Packard 6890 GC.

GC analyses showed the presence of between 10-15% EPA in all of the transformants with pZKUE3S from plates. The strains identified as #3, #4, #10, #12, #19 and #21 that produced 12.9%, 14.4%, 15.2%, 15.4%, 14% and 10.9% EPA of total lipids were designated as Y4128U1, Y4128U2, Y4128U3, Y4128U4, Y4128U5 and Y4128U6, respectively (collectively, Y4128U).

The discrepancy in the % EPA quantified in Y4128 (37.6%) versus Y4128U (average 13.8%) is based on differing growth conditions. Specifically, the former culture was analyzed following two days of growth in liquid culture, while the latter culture was analyzed after growth on an agar plate. The Applicants have observed a 2-3 fold increase in % EPA, when comparing results from agar plates to those in liquid culture. Thus, although results are not directly comparable, both Y4128 and Y4128U strains demonstrate production of EPA.

### EXAMPLE 3

### Determination Of Total Lipid Content Of Yarrowia lipolytica Strain Y4128

The total amount of lipid produced by strain Y4128 and the percentage of each fatty acid species in the lipid were measured by GC analysis. Specifically, total lipids were extracted, and FAMEs were prepared by trans-esterification, and subsequently analyzed with a Hewlett-Packard 6890 GC, as described in the General Methods.

Dry cell weight was determined by collecting cells from 10 mL of culture via centrifugation, washing the cells with water once to remove residual medium, drying the cells in a vacuum oven at 80 °C overnight, and weighing the dried cells. The total amount of FAMEs in a sample was determined by comparing the areas of all peaks in the GC profile with the peak area of an added known amount of internal standard C15:0 fatty acid.

Based on the above analyses, lipid content as a percentage of dry cell weight (DCW) and lipid composition was determined for strains Y4086 and Y4128. Strain Y4128 had decreased lipid content with respect to strain Y4086 (11.2 TFAs % DCW versus 28.6 TFAs % DCW). In contrast, strain Y4128 had elevated EPA concentrations among lipids with respect to strain Y4086, as shown below in Table 6. Fatty acids are identified as 18:0 (stearic acid), 18:1 (oleic acid), LA, ALA, EDA, DGLA, ETrA, ETA and EPA; fatty acid compositions were expressed as the weight percent (wt. %) of total fatty acid (TFAs).

**Table 6**

| Lipid Composition In *Yarrowia lipolytica* Strains Y4086 And Y4128 | | | | | | | | | |
|---|---|---|---|---|---|---|---|---|---|
| Sample | 18:0 | 18:1 | 18:2 [LA] | 18:3 (n-3) [ALA] | 20:2 [EDA] | 20:3 (n-6) [DGLA] | 20: 3 (n-3) [ETrA] | 20:4 (n-3) [ETA] | 20:5 (n-3) [EPA] |
| Y4086 | 4.6 | 26.8 | 28.0 | 6.9 | 7.6 | 0.9 | 4.9 | 2.0 | 9.8 |
| Y4128 | 1.8 | 6.7 | 19.6 | 1.8 | 4.2 | 3.4 | 1.5 | 6.0 | 42.8 |

EPA content in the cell, expressed as mg EPA/g dry cell and calculated according to the following formula: (% of EPA/Lipid) * (% of Lipid /dry cell weight) * 0.1, increased from 28 mg EPA/g DCW in strain Y4086 to 47.9 mg EPA/g DCW in strain Y4128.

Thus, the results in Table 6 showed that compared to the parent strain Y4086, strain Y4128 had a lower total lipid content (TFAs % DCW) (11.2% versus 28.6%), higher EPA % TFAs (42.8% versus 9.8%), and higher **EPA % DCW (4.8% versus 2.8%).** Additionally, strain Y4128 had a 3.3-fold increase in the amount of EPA relative to the total PUFAs (54% of the PUFAs [as a % TFAs] versus 16.3% of the PUFAs [as a % TFAs]) and a 1.7-fold increase in the amount of C20 PUFAs relative to the total PUFAs (73% of the PUFAs [as a % TFAs] versus 42% of the PUFAs [as a % TFAs]).

### EXAMPLE 4

### Determination Of The Integration Site Of pZP2-2988 In Yarrowia lipolytica

### Strain Y4128 As A Pex10 Integration

The genomic integration site of pZP2-2988 in strain Y4128 was determined by genome walking using the Universal GenomeWalker™ Kit from Clontech (Palo Alto, CA), following the manufacturer's recommended protocol. Based on the sequence of the plasmid, the following primers were designed for genome walking: pZP-GW-5-1 (SEQ ID NO:32), pZP-GW-5-2 (SEQ ID NO:33), pZP-GW-5-3 (SEQ ID NO:34), pZP-GW-5-4 (SEQ ID NO:35), pZP-GW-3-1 (SEQ ID NO:36), pZP-GW-3-2 (SEQ ID NO:37), pZP-GW-3-3 (SEQ ID NO:38) and pZP-GW-3-4 (SEQ ID NO:39).

Genomic DNA was prepared from strain Y4128 using the Qiagen Miniprep kit with a modified protocol. Cells were scraped off a YPD medium plate into a 1.5 mL microfuge tube. Cell pellet (100 µl) was resuspended with 250 µl of buffer P1 containing 0.125 M β-mercaptoethanol and 1 mg/mL zymolyase 20T (MP Biomedicals, Inc., Solon, OH). The cell suspension was incubated at 37 °C for 30 min. Buffer P2 (250 µl) was then added to the tube After mixing by inverting the tube for several times, 350 µl of buffer N3 was added. The mixture was then centrifuged at 14,000 rpm for 5 min in a microfuge. Supernatant was poured into a Qiagen miniprep spin colomn, and centrifuged for 1 min. The column was washed once by adding 0.75 mL of buffer PE, followed by centrifugation at 14,000 rpm for 1 min. The column was dried by further centrifugation at 14,000 rpm for 1 min. Genomic DNA was eluted by adding 50 µl of buffer EB to the column, allowed to sit for 1 min and centrifuged at 14,000 rpm for 1 min.

Purified genomic DNA was used for genome walking. The DNA was digested with restriction enzymes *Dra*I*, Eco*RV, *Pvu*II and *Stu*I separately, according to the protocol of the GenomeWalker kit. For each digestion, the reaction mixture contained 10 µl of 10X restriction buffer, 10 µl of the appropriate restriction enzyme and 8 µg of genomic DNA in a total volume of 100 µl. The reaction mixtures were incubated at 37 °C for 4 hrs. The digested DNA samples were then purified using Qiagen PCR purification kit following the manufacturer's protocol exactly. DNA samples were eluted in 16 µl water. Purified, digested genomic DNA samples were then ligated to the genome walker adaptor (*infra*). Each ligation mixture contained 1.9 µl of the genome walker adaptor, 1.6 µl of 10X ligation buffer, 0.5 µl T4 DNA ligase. and 4 µl of the digested DNA. The reaction mixtures were incubated at 16 °C overnight. Then, 72 µl of 50 mM TrisHCl, 1 mM EDTA, pH 7.5 were added to each ligation mixture.

For 5'-end genome walking, four PCR reactions were carried out using 1 µl of each ligation mixture individually as template. In addition, each reaction mixture contained 1 µl of 10 µM primer pZP-GW-5-1 (SEQ ID NO:32), 1 µl of 10 µM kit-supplied Genome Walker adaptor, 41 µl water, 5 µl 10X cDNA PCR reaction buffer and 1 µl Advantage cDNA polymerase mix from Clontech. The sequence of the Genome Walker adaptor (SEQ ID NOs:40 [top strand] and 41 [bottom strand]), is shown below:

The PCR conditions were as follows: 95 °C for 1 min, followed by 30 cycles a 95 °C for 20 sec and 68 °C for 3 min, followed by a final extension at 68 °C for 7 min. The PCR products were each diluted 1:100 and 1 µl of the diluted PCR product used as template for a second round of PCR. The conditions were exactly the same except that pZP-GW-5-2 (SEQ ID NO:33) replaced pZP-GW-5-1 (SEQ ID NO:32).

For 3'-end genome walking, four PCR reactions were carried out as above, except primer pZP-GW-3-1 (SEQ ID NO:36) and nested adaptor primer (SEQ ID NO:42) were used. The PCR products were similarly diluted and used as template for a second round of PCR, using pZP-GW-3-2 (SEQ ID NO:37) to replace pZP-GW-3-1 (SEQ ID NO:36).

PCR products were analyzed by gel electrophoresis. One reaction product, using EcoRV digested genomic DNA as template and the primers pZP-GW-3-2 and nested adaptor primer, generated a ∼1.6 kB fragment. This fragment was isolated, purified with a Qiagen gel purification kit and cloned into pCR2.1-TOPO. Sequence analysis showed that the fragment included both part of plasmid pZP2-2988 and the *Yarrowia* genomic DNA from chromosome C. The junction between them was at nucleotide position 139826 of chromosome C. This was inside the coding region of the Pex10 gene (GenBank Accession No. CAG81606; SEQ ID NO:10).

To determine the 5' end of the junction, PCR amplification was performed using genomic DNA from strain Y4128 as the template and primers Per10 F1 (SEQ ID NO:43) and ZPGW-5-5 (SEQ ID NO:44). The reaction mixture included 1 µl each of 20 µM primer, 1 µl genomic DNA, 22 µl water and 25 µl TaKaRa ExTaq 2X premix (TaKaRa Bio Inc., Otsu Shiga, Japan). The thermocycler conditions were: 94 °C for 1 min, followed by 30 cycles of 94 °C for 20 sec, 55 °C for 20 sec and 72 °C for 2 min, followed by final extension at 72 °C for 7 min. A 1.6 kB DNA fragment was amplified and cloned into pCR2.1-TOPO. Sequence analysis showed that it was a chimeric fragment between *Yarrowia* genomic DNA from chromosome C and pZP2-2988. The junction was at nucleotide position 139817 of chromosome C. µThus, a 10 nucleotide segment of chromosome C was replaced by the AscI/SphI fragment from pZP2-2988 (FIG. 4B) in strain Y4128. As a result, Pex10 in strain Y4128 was lacking the last 32 amino acids of the encoded protein.

Based on the above conclusions, the Y4128U strains isolated in Example 2 (*supra*) are referred to subsequently as Δpex10 strains. For clarity, strain Y4128U1 is equivalent to strain Y4128U1 (Δpex10).

### EXAMPLE 5

### Plasmid Expression Of Pex10 In Yarrowia lipolytica Strain Y4128U1 (Δpex10)

Three plasm ids that carried the Y. *lipolytica* Pex10 gene were constructed: 1) pFBAIn-PEX10 allowed the expression of the Pex10 ORF under the control of the FBAINm promoter; and, 2) pPEX10-1 and pPEX10-2 allowed the expression of Pex10 under control of the native Pex10 promoter, although pPEX10-1 used a shorter version (~500 bp) while pPEX10-2 used a longer version (~900 bp) of the promoter. Following construction of these expression plasmids and transformation, the effect of Pex10 plasmid expression on total oil and on EPA level in the Y. *lipolytica* strain Y4128U1 (Δpex10) was determined. Deletion of Pex10 resulted in an increased amount of EPA as a percent of TFAs, but a reduced amount of total lipid, as percent of DCW, in the cell.

### Construction Of pFBAIn-PEX10, pPEX10-1 And pPEX10-2

To construct pFBAIn-PEX10, the primers Per10 F1 (SEQ ID NO:43) and Per10 R (SEQ ID NO:45) were used to amplify the coding region of the Pex10 gene using Y. *lipolytica* genomic DNA as template. The PCR reaction mixture contained 1 µl each of 20 µM primers, 1 µl of *Y. lipolytica* genomic DNA (~100 ng), 25 µl ExTaq 2X premix and 22 µl water. The reaction was carried out as follows: 94 °C for 1 min, followed by 30 cycles of 94 °C for 20 sec, 55 °C for 20 sec and 72 °C for 90 sec, followed by a final extension of 72 °C for 7 min. The PCR product, a 1168 bp DNA fragment, was purified with a Qiagen PCR purification kit, digested with NcoI and *Not*I, and cloned into pFBAIn-MOD-1 (SEQ ID NO:46; FIG. 5B) digested with the same two restriction enzymes.

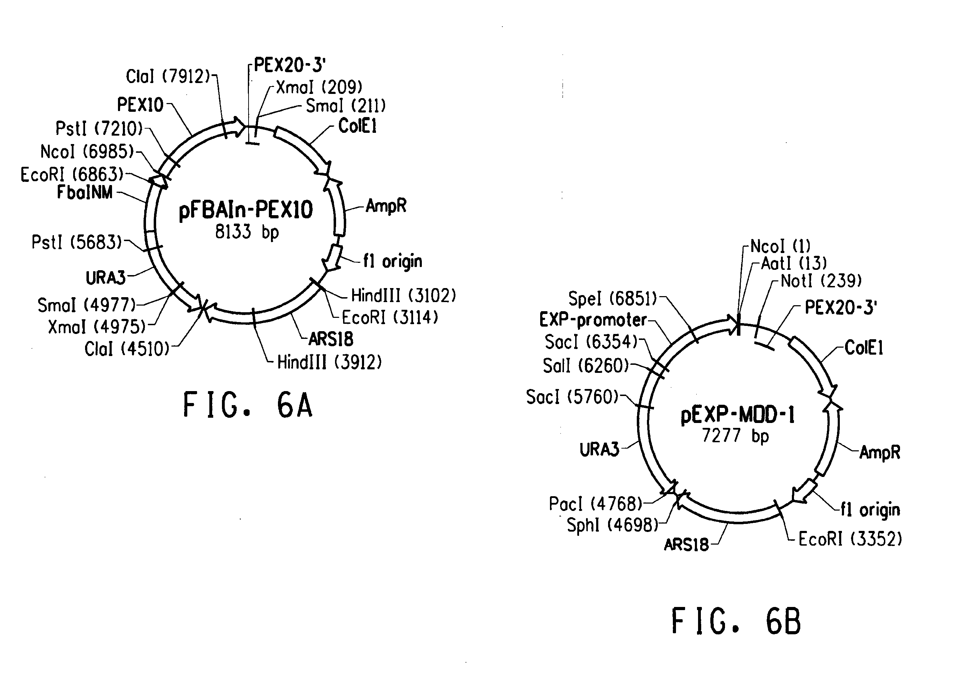

Of the 8 individual clones subjected to sequence analysis, 2 had the correct sequence of Pex10 with no errors. The components of pFBAIn-PEX10 (SEQ ID NO:47; FIG. 6A) are listed below in Table 7.

**Table 7**

| Components Of Plasmid pFBAIn-PEX10 (SEQ ID NO:47) | |
|---|---|
| RE Sites And Nucleotides Within SEQ ID NO:47 | Description Of Fragment And Chimeric Gene Components |
| *Bgl*II-*Bsi*WI (6040-318) | FBAINm::Pex10::Pex20, comprising: |
| | • FBAINm: *Yarrowia lipolytica* FBAINm promoter (U.S. Pat. No. 7,202,356); |
| | • Pex10: *Y. lipolytica* Pex10 ORF (GenBank Accession No. AB036770, nucleotides 1038-2171; SEQ ID NO:21); |
| | • Pex20: Pex20 terminator sequence from *Yarrowia* Pex20 gene (GenBank Accession No. AF054613) |
| *Pac*I-*Bgl*II (4530-6040) | *Yarrowia URA3* (GenBank Accession No. AJ306421) |
| (3123-4487) | *Yarrowia* autonomous replicating sequence 18 (ARS18; GenBank Accession No. A17608) |
| (2464-2864) | *E. coli* f1 origin of replication |
| (1424-2284) | Ampicillin-resistance gene (Amp^{R}) for selection in *E. coli* |
| (474-1354) | *ColE1* plasmid origin of replication |

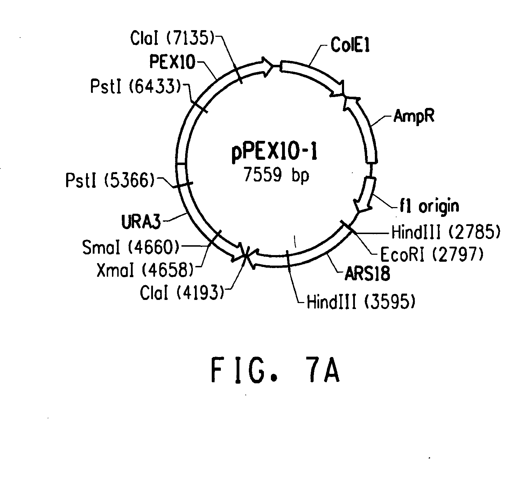

To construct pPEX10-1 and pPEX10-2, primers PEX10-R-BsiWI (SEQ ID NO:48), PEX10-F1-SalI (SEQ ID NO:49) and PEX10-F2-SalI (SEQ ID NO:50) were designed and synthesized. PCR amplification using genomic *Yarrowia lipolytica* DNA and primers PEX10-R-BsiWI and PEX10-F1-SalI generated a 1873 bp fragment containing the Pex10 ORF, 500 bp of the 5' upstream region and 215 bp of the 3' downstream region of the Pex10 gene, flanked by *Sal*I and *Bsi*WI restriction sites at either end. This fragment was purified with the Qiagen PCR purification kit, digested with *Sa*II and *Bsi*WI, and cloned into pEXP-MOD-1 (SEQ ID NO:51; FIG. 6B) digested with the same two enzymes to generate pPEX10-1 (SEQ ID NO:52; FIG. 7A). Plasmid pEXP-MOD1 is similar to pFBAIn-MOD-1 (SEQ ID NO:46; FIG. 5B) except that the FBAINm promoter in the latter was replaced with the EXP1 promoter. Table 8 lists the components of pPEX10-1.

**Table 8**

| Components Of Plasmid pPEX10-1 (SEQ ID NO:52) | |
|---|---|
| RE Sites And Nucleotides Within SEQ ID NO:52 | Description Of Fragment And Chimeric Gene Components |
| *Sal*I-*Bsi*WI) (5705-1) | Pex10-5'::Pex10::Pex10-3', comprising: |
| | • Pex10-5': 500 bp of the 5' promoter region of *Yarrowia lipolytica* Pex10 gene; |
| | • Pex10: *Yarrowia lipolytica* Pex10 ORF (GenBank Accession No. AB036770, nucleotides 1038-2171; SEQ ID NO:21); |
| | • Pex10-3': 215 bp of Pex10 terminator sequence from *Yarrowia* Pex10 gene (GenBank Accession No. AB036770) |
| | [Note the entire Pex10-5'::Pex10::Pex10-3' expression cassette is labeled collectively as "PEX10" in the Figure] |
| *Pac*I-*Sal*I (4216-5703) | *Yarrowia URA3* gene (GenBank Accession No. AJ306421) |
| (2806-4170) | *Yarrowia* autonomous replicating sequence 18 (ARS18; GenBank Accession No. A17608) |
| (2147-2547) | *E. coli* f1 origin of replication |
| (1107-1967) | Ampicillin-resistance gene (Amp^{R}) for selection in *E. coli* |
| (157-1037) | *ColE1* plasmid origin of replication |

PCR amplification of *Yarrowia lipolytica* genomic DNA using PEX10-R-BsiWI (SEQ ID NO:48) and PEX10-F2-SalI (SEQ ID NO:50) generated a 2365 bp fragment containing the PEX10 ORF, 991 bp of the 5' upstream region and 215 bp of the 3' downstream region of the Pex10 gene, flanked by *Sal*I and *Bsi*WI restriction sites at either end. This fragment was purified with a Qiagen PCR purification kit, digested with *Sal*I and *Bsi*WI, and cloned into similarly digested pEXP-MOD-1. This resulted in synthesis of pPEX10-2 (SEQ ID NO:53), whose construction is analogous to that of plasmid pPEX10-1 (Table 8, *supra*), with the exception of the longer Pex10-5' promoter in the chimeric Pex10-5'::Pex10::Pex10-3'gene.

### Expression Of Pex10 In Strain Y4128U1 (Δpex10)

Plasmids pFBAIN-MOD-1 (control; SEQ ID NO:46), pFBAIn-PEX10 (SEQ ID NO:47), pPEX10-1 (SEQ ID NO:52) and pPEX10-2 (SEQ ID NO:53) were transformed into Y4128U1 (Δpex10) according to the protocol in the General Methods. Transformants were plated on MM plates. The total lipid content and fatty acid composition of transformants carrying the above plasmids were analyzed as described in Example 3.

Lipid content as a percentage of dry cell weight (DCW) and lipid composition are shown below in Table 9. Specifically, fatty acids are identified as 18:0 (stearic acid), 18:1 (oleic acid), LA, ALA, EDA, DGLA, ETrA ETA and EPA; fatty acid compositions were expressed as the weight percent (wt. %) of total fatty acids.

**Table 9**

| Lipid Composition In *Yarrowia lipolytica* Strain Y4128U1 (Δpex10) Transformed With Various Pex10 Plasmids | | | | | | | | | | |
|---|---|---|---|---|---|---|---|---|---|---|
| Plasmid | TFA % DCW | 18:0 | 18:1 | 18:2 [LA] | 18:3 (ω3) [ALA] | 20:2 [EDA] | 20:3 (ω6) [DGLA] | 20: 3 (ω3) [ETrA] | 20:4 (ω3) [ETA] | 20:5 (ω3) [EPA] |
| pFBAIN-MOD-1 | 22.8 | 1.9 | 9.6 | 18.3 | 2.0 | 4.3 | 2.3 | 2.1 | 5.9 | 27.7 |
| pFBAIN-PEX10 | 29.2 | 4.0 | 24.9 | 25.1 | 7.6 | 6.6 | 1.0 | 5.3 | 3.6 | 10.8 |
| pPEX10-1 | 27.1 | 3.9 | 25.0 | 25.2 | 8.2 | 6.4 | 0.9 | 5.2 | 3.5 | 10.7 |
| pPEX10-2 | 28.5 | 4.3 | 25.4 | 24.5 | 7.6 | 6.4 | 1.0 | 5.3 | 3.4 | 10.8 |

The results in Table 9 showed that expression of Pex10 in Y4128U1 (Δpex10), either from the native Y. *lipolytica* Pex10 promoter or from the Y. *lipolytica* FBAINm promoter, reduced the percent of EPA back to the level of Y4086 while increasing the total lipid content (TFA % DCW) up to the level of Y4086 (see data of Table 6 for comparison). EPA content per gram of dry cell changed from 63.2 mg in the case of the control sample (i.e., cells carrying pFBAIn-MOD-1) to 31.5 mg in cells carrying pFBAIn-PEX10, 29 mg in cells carrying pPEX10-1 and 30.8 mg in cells carrying pPEX10-2. These results demonstrated that disruption of the ring-finger domain of Pex10 increased the amount of EPA but reduced the total lipid content in the cell.

Thus, the results in Table 9 showed that compared to Y4128U1 (Δpex10) transformant with control plasmid, all transformants with Pex10 expressing plasmids showed higher lipid content (TFAs % DCW) (>27% versus 22.8%), lower EPA % TFAs (ca. 10.8% versus 27.7%), and lower EPA % DCW (<3.1% versus 6.3%). Additionally, strain Y4128U1 (Δpex10) transformant with control plasmid, as compared to those transformants with Pex10 expressing plasmids, had a 2.5-fold increase in the amount of EPA relative to the total PUFAs (44% of the PUFAs [as a % TFAs] versus 17.5% (avg) of the PUFAs [as a % TFAs]) and a 1.5-fold increase in the amount of C20 PUFAs relative to the total PUFAs (67% of the PUFAs [as a % TFAs] versus 44% (avg) of the PUFAs [as a % TFAs]).

### EXAMPLE 6

### Generation Of Y4184U Strain To Produce EPA

*Y. lipolytica* strain Y4184U was used as the host in Example 7, *infra.* Strain Y4184U was derived from Y. *lipolytica* ATCC #20362, and is capable of producing EPA via expression of a Δ9 elongase/Δ8 desaturase pathway. The strain has a *Ura-* phenotype and its construction is described in Example 7 of Int'I. App. Pub. No. WO 2008/073367.

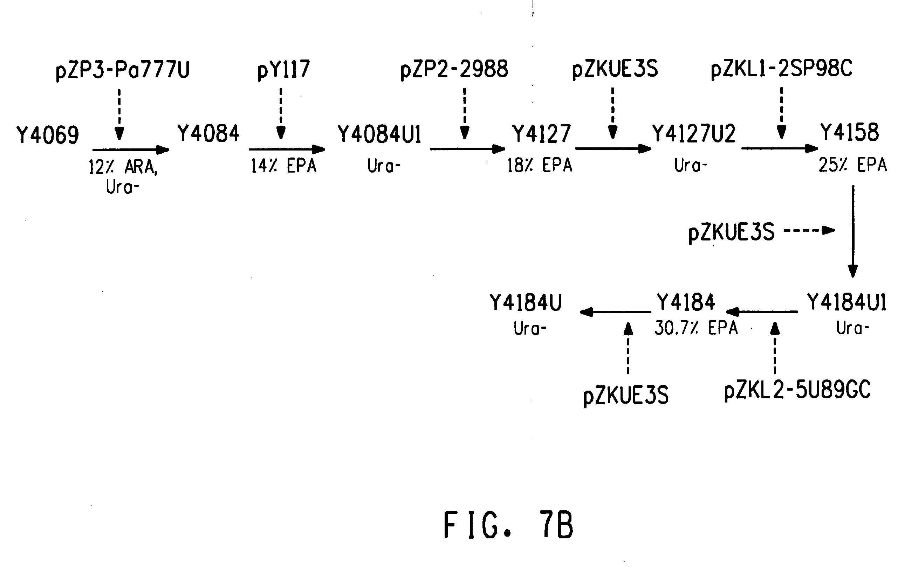

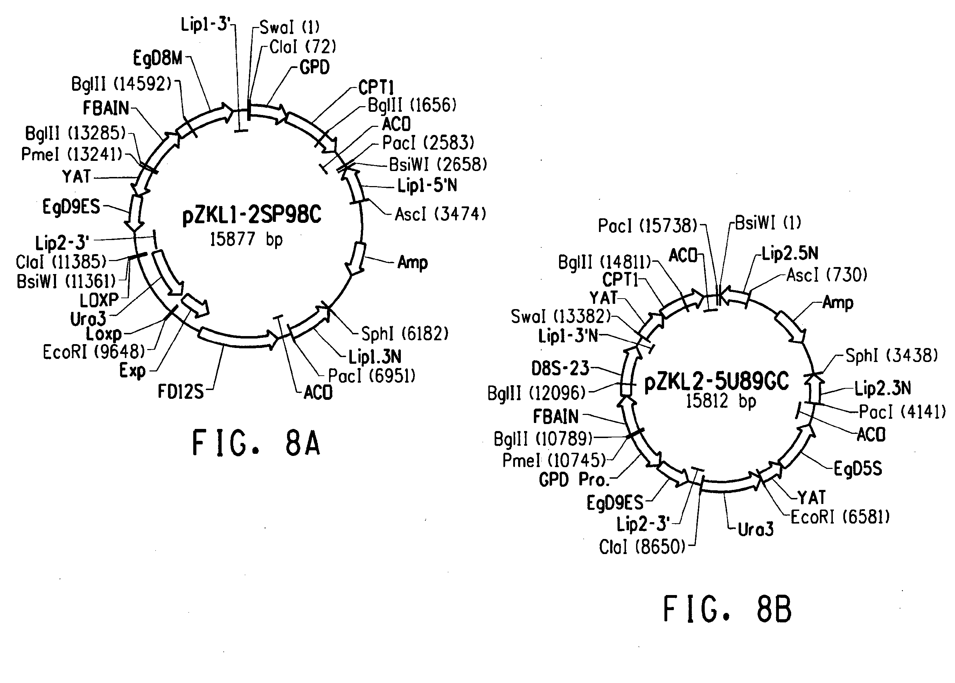

In summary, however, the development of strain Y4184U required the construction of strain Y2224, strain Y4001, strain Y4001 U, strain Y4036, strain Y4036U and strain Y4069 (*supra*, Example 1). Further development of strain Y4184U (diagrammed in Figure 7B) required generation of strain Y4084, strain Y4084U1, strain Y4127 (deposited with the American Type Culture Collection on November 29, 2007, under accession number ATCC PTA-8802), strain Y4127U2, strain Y4158, strain Y4158U1 and strain Y4184, The plasmid construct pZKL1-2SP98C, used for transformation of strain Y4127U2, is diagrammed in FIG. 8A (SEQ ID NO:54; described in Table 23 of Int'I. App. Pub. No. WO 2008/073367). Plasmid pZKL2-5U89GC, used for transformation of strain Y4158U1, is shown in FIG. 8B (SEQ ID NO:55; described in Table 24 of Int'I. App. Pub. No. WO 2008/073367).

The final genotype of strain Y4184 (producing 31 % EPA of total lipids) with respect to wildtype *Yarrowia lipolytica* ATCC #20362 was *unknown 1*-, *unknown 2-, unknown 4-, unknown 5-, unknown 6-, unknown 7-,* YAT1::ME3S::Pex16, EXP1::ME3S::Pex20 (2 copies), GPAT::EgD9e::Lip2, FBAINm::EgD9eS::Lip2, EXP1::EgD9eS::Lip1, FBA::EgD9eS::Pex20, YAT1::EgD9eS::Lip2, GPD::EgD9eS::Lip2, GPDIN::EgD8M::Lip1, YAT1::EgD8M::Aco, EXP1::EgD8M::Pex16, FBAINm::EgD8M::Pex20, FBAIN::EgD8M::Lip1 (2 copies), GPM/FBAIN::FmD12S::Oct, EXP1::FmD12S::Aco, YAT1::FmD12::Oct, GPD::FmD12::Pex20, EXP1::EgD5S::Pex20, YAT1::EgDSS::Aco, YAT1::Rd5S::Oct, FBAIN::EgD5::Aco, FBAINm::PaD17::Aco, EXP1::PaD17::Pex16, YAT1 ::PaD17S::Lip1, YATI::YICPT1::Aco, GPD::YICPT1::Aco (wherein FmD12 is a *Fusarium moniliforme* Δ12 desaturase gene [Int'I. App. Pub. No. WO 2005/047485]; FmD12S is a codon-optimized Δ12 desaturase gene, derived from *Fusarium moniliforme* [Int'I. App. Pub. No. WO 2005/047485]; ME3S is a codon-optimized C_{16/18} elongase gene, derived from *Mortierella alpina* [Int'I. App. Pub. No. WO 2007/046817]; EgD9e is a *Euglena gracilis* Δ9 elongase gene [Int'I. App. Pub. No. WO 2007/061742]; EgD9eS is a codon-optimized Δ9 elongase gene, derived from *Euglena gracilis* [Int'I. App. Pub. No. WO 2007/061742]; EgDBM is a synthetic mutant Δ8 desaturase [Int'I. App. Pub. No. WO 2008/073271], derived from *Euglena gracilis* [U.S. Pat. No. 7,256,033]; EgD5 is a *Euglena gracilis* Δ5 desaturase [U.S. Pat. App. Pub. US 2007-0292924-A1]; EgD5S is a codon-optimized Δ5 desaturase gene, derived from *Euglena gracilis* [U.S. Pat. App. Pub. No. 2007-0292924] RD5S is a codon-optimized Δ5 desaturase, derived from *Peridinium sp.* CCMP626 [U.S. Pat. App. Pub. No. 2007-0271632]; PaD17 is a *Pythium aphanidermatum* Δ17 desaturase [Int'I. App. Pub. No. WO 2008/054565]; PaD17S is a codon-optimized Δ17 desaturase, derived from *Pythium aphanidermatum* [Int'I. App. Pub. No. WO 2008/054565]; and, YICPT1 is a *Yarrowia lipolytica* diacylglycerol cholinephosphotransferase gene [Int'I. App. Pub. No. WO 2006/052870]).

In order to disrupt the Ura3 gene in strain Y4184, construct pZKUE3S (FIG. 5A; SEQ ID NO:31; described in Table 22 of Int'I. App. Pub. No. WO 2008/073367) was used to integrate a EXP1::ME3S::Pex20 chimeric gene into the *Ura3* gene of strain Y4184 to result in strains Y4184U1 (11.2% EPA of total lipids), Y4184U2 (10.6% EPA of total lipids) and Y4184U4 (15.5% EPA of total lipids), respectively (collectively, Y4184U).

### EXAMPLE 7

### Chromosomal Deletion Of Pex10 In Yarrowia lipolytica Strain Y4184U4 Increases Accumulation Of EPA And Total Lipid Content

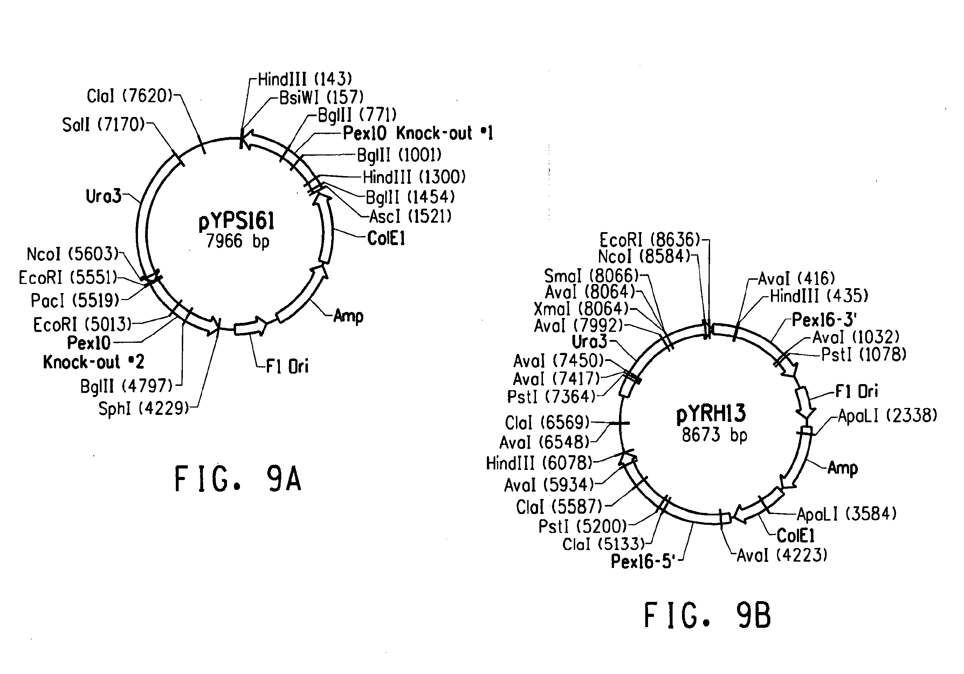

Construct pYPS161 (FIG. 9A, SEQ ID NO:56) was used to knock out the chromosomal Pex10 gene from the EPA-producing *Yarrowia* strain Y4184U4 (Example 6). Transformation of *Y. lipolytica strain* Y4184U4 with the Pex10 knock out construct resulted in creation of strain Y4184 (□pex10). The effect of the Pex10 knockout on total oil and on EPA level was determined and compared. Specifically, knockout of Pex10 resulted in an increase percentage of EPA (as % TFAs and % DCW) and an increased total lipid content in the cell.

### Construct pYSP161

The construct pYPS161 contained the following components:

**Table 10**

| Description of Plasmid pYPS161 (SEQ ID NO:56) | |
|---|---|
| RE Sites And Nucleotides Within SEQ ID NO:56 | Description Of Fragment And Chimeric Gene Components |
| *Asc*I/*BsiW*I (1521-157) | 1364 bp Pex10 knockout fragment #1 of *Yarrowia* Pex10 gene (GenBank Accession No. AB036770) |
| *Pac*I/*Sph*I (5519-4229) | 1290 bp Pex10 knockout fragment #2 of *Yarrowia* Pex10 gene (GenBank Accession No. AB036770) |
| *Sal*I/*Eco*RI (7170-5551) | *Yarrowia URA3* gene (GenBank Accession No. AJ306421) |
| 2451-1571 | *ColE1* plasmid origin of replication |
| 3369-2509 | ampicillin-resistance gene (Amp^{R}) for selection in *E. coli* |
| 3977-3577 | *E. coli* f1 origin of replication |

### Generation Of Yarrowia lipotytica Knockout Strain Y4184 (ΔPex10)

Standard protocols were used to transform *Yarrowia lipolytica strain* Y4184U4 (Example 6) with the purified 5.3 kB *Asc*I/*Sph*I fragment of Pex10 knockout construct pYPS161 (*supra*), and a cells alone control was also prepared. There were about 200 to 250 colonies present for each of the experimental transformations, while there were no colonies present on the cells alone plates (per expectations).

Colony PCR was used to screen for cells having the Pex10 deletion. Specifically, the PCR reaction was performed using MasterAmp Taq polymerase (Epicentre Technologies, Madison, WI) following standard protocols, using PCR primers Pex-10del1 3'.Forward (SEQ ID NO:57) and Pex-10de12 5'.Reverse (SEQ ID NO:58). The PCR reaction conditions were 94°C for 5 min, followed by 30 cycles at 94°C for 30 sec, 60 °C for 30 sec and 72 °C for 2 min, followed by a final extension at 72 °C for 6 min. The reaction was then held at 4 °C. If the Pex10 knockout construct integrated within the Pex10 region, a single PCR product 2.8 kB in size was expected to be produced. In contrast, if the strain integrated the Pex10 knockout construct in a chromosomal region other than the Pex10 region, then two PCR fragments, i.e., 2.8 kB and 1.1 kB, would be generated. Of the 288 colonies screened, the majority had the Pex10 knockout construct integrated at a random site. Only one of the 288 colonies contained the Pex10 knockout. This strain was designated Y4184 (Apex10).

### Evaluation Of Yarrowia lipotvtica Strains Y4184 And Y4184 (ΔPex10) For Total Oil And EPA Production

To evaluate the effect of the Pex10 knockout on the percent of PUFAs in the total lipid fraction and the total lipid content in the cells, strains Y4184 and Y4184 (Δpex10) were grown under comparable oleaginous conditions. Specfiically, cultures were grown at a starting OD₆₀₀ of ~0.1 in 25 mL of eithe fermentation media (FM) or FM medium without Yeast Extract (FM without YE) in a 250 mL flask for 48 hrs. The cells were harvested by centrifugation for 10 min at 8000 rpm in a 50 mL conical tube. The supernatant was discarded and the cells were re-suspended in 25 mL of HGM and transferred to a new 250 mL flask. The cells were incubated with aeration for an additional 120 hrs at 30 °C.

To determine the dry cell weight (DCW), the cells from 5 mL of the FM grown cultures and 10 mL of the FM without YE-grown cultures were processed. The cultured cells were centrifuged for 10 min at 4300 rpm. The pellet was re-suspended using 10 mL of saline and was centrifuged under the same conditions for a second time. The pellet was then re-suspended using 1 mL of sterile H₂O (three times) and was transferred to a pre-weighed aluminum pan. The cells were dried overnight in a vacuum oven at 80 °C. The weight of the cells was determined.

The total lipid content and fatty acid composition of transformants carrying the above plasmids were analyzed as described in Example 3. DCW, total lipid content (TFAs % DCW), total EPA % TFAs, and EPA % DCW are shown below in Table 11.

**Table 11 Lipid Composition In Y. lipolytica Strains Y4184 And Y4184 (ΔPex10)**

| Media | Strain | DCW | TFAs % DCW | EPA % TFAs | EPA % DCW |
|---|---|---|---|---|---|
| FM | Y4184 | 11.5 | 11.8 | 20.6 | 2.4 |
| | Y4184 (ΔPex10) | 11.5 | 17.6 | 43.2 | 7.6 |
| FM without YE | Y4184 | 4.6 | 8.8 | 23.2 | 2.0 |
| | Y4184 (ΔPex10) | 4.0 | 13.2 | 46.1 | 6.1 |

The results in Table 11 showed that knockout of the chromosomal Pex10 gene in Y4184 (ΔPex10) increased the percent of EPA (as % TFAs and as % DCW) and increased the total oil content, as compared to the percent of EPA and total oil content in strain Y4184 whose native Pex10p had not been knocked out. More specifically, in FM media, there was about 109% increase in EPA (% TFAs), about 216% increase in EPA productivity (% DCW) and about 49% increase in total oil (TFAs % DCW). In FM without YE media, there was about 100% increase in EPA (% TFAs), about 205% increase in EPA productivity (% DCW) and about 50% increase in total oil (TFAs % DCW).

Thus, the results in Table 11 showed that in FM medium, compared to the parent strain Y4184, Y4184 (ΔPex10) strain had higher lipid content (TFAs % DCW) (17.6% versus 11.8%), higher EPA % TFAs (43.2% versus 20.6%), and higher EPA % DCW (7.6% versus 2.4%). Similarly, in FM medium without YE, compared to the parent strain Y4184, Y4184 (ΔPex10) strain had higher lipid content (TFAs % DCW) (13.2% versus 8.8%), higher EPA % TFAs (46.1% versus 23.2%), and higher EPA % DCW (6.1% versus 2.0%).

### EXAMPLE 8 (Prophetic)

### Chromosomal Knockout Of Alternate Pex Genes In PUFA-Producing Strains Of Yarrowia lipolytica

The present Example describes various strains of *Yarrowia lipolytica* that have been engineered to produce ω-3/ω-6 PUFAs. It is contemplated that any of these Y. *lipolytica* host strains could be engineered to produce an increased amount of ω-3/ω-6 PUFAs in the total lipid fraction and in the oil fraction, if the chromosomal gene encoding Pex1p, Pex2p, Pex3p, Pex3Bp, Pex4p, Pex5p, Pex6p, Pex7p, Pex8p, Pex12p, Pex13p, Pex14p, Pex16p, Pex17p, Pex19p, Pex20p, Pex22p or Pex26p was disrupted using the methodology of Example 7, *supra.*

More specifically, a variety of *Yarrowia lipolytica* strains have been engineered by the Applicant's Assignee to produce high concentrations of various ω-3/ω-6 PUFAs via expression of a heterologous Δ6 desaturase/Δ6 elongase PUFA pathway or a heterologous Δ9 elongase/Δ8 desaturase PUFA pathway.

### Summary Of Representative Yarrowia lipolytica Strains Producing ω-3/ω-6 PUFAs

Although some representative strains are summarized in the Table below, the disclosure of *Yarrowia lipolytica* strains producing ω-3/ω-6 PUFAs is not limited in any way to the strains therein. Instead, all of the teachings provided in the present Application, in addition to the following commonly owned and co-pending applications, are useful for development of a suitable *Yarrowia lipolytica* strain engineered to produce ω-3/ω-6 PUFAs. These specifically include the following Applicants' Assignee's co-pending patents and applications: U.S. Pat. No. 7,125,672, U.S. Pat. No. 7,189,559, U.S. Pat. No. 7,192,762, U.S. Pat. No. 7,198,937, U.S. Pat. No. 7,202,356, U.S. Pat. No. 7,214,491, U.S. Pat. No. 7,238,482, U.S. Pat. No. 7,256,033, U.S. Pat. No. 7,259,255, U.S. Pat. No. 7,264,949, U.S. Pat. No. 7,267,976, U.S. Pat. No. 7,273,746, U.S. Pat. App. No. 10/985254 and No. 10/985691 (filed November 10, 2004), U.S. Pat. App. No. 11/183664 (filed July 18, 2005), U.S. Pat. App. No. 11 /185301 (filed July 20, 2005), U.S. Pat. App. No. 11 /190750 (filed July 27, 2005), U.S. Pat. App. No. 11/198975 (filed August 8, 2005), U.S. Pat. App. No. 11/253882 (filed October 19, 2005), U.S. Pat. App. No. 11/264784 and No. 11/264737 (filed November 1, 2005), U.S. Pat. App. No. 11/265761 (filed November 2, 2005), U.S. Pat. App. No. 11/601563 and No. 11/601564 (filed November 16, 2006), U.S. Pat. App. No. 11/635258 (filed December 7, 2006), U.S. Pat. App. No. 11/613420 (filed December 20, 2006 U.S. Pat. App. No. 11/787772 (filed April 18, 2007), U.S. Pat. App. No. 11/737772 (filed April 20, 2007), U.S. Pat. App. No. 11/740298 (filed April 26 2007), U.S. Pat. App. No. 12/111237 (filed April 29, 2008), U.S. Pat. App. No. 11/748629 and No. 11/748637 (filed May 15, 2007), U.S. Pat. App. No. 11/779915 (filed July 19, 2007), U.S. Pat. App. No. 60/991266 (filed November 30. 2007), U.S. Pat. App. No. 11/952243 (filed December 7, 2007), U.S. Pat. App. No. 61/041716 (filed April 2, 2008), U.S. Pat. App. No. 12/061738 (filed April 3, 2008), U.S. Pat. App. No. 12/099811 (filed April 9, 2008), U.S. Pat. App. No. 12/102879 (filed April 15, 2008), U.S. Pat. App. No. 12/111237 (filed April 29, 2008), U.S. Pat. App. No. 61/055511 (filed May 23, 2008) and U.S. Pat. App. No. 61/093007 (filed August 29, 2008).

**Table 12: Lipid Profile Of Representative Yarrowia lipolytica Strains Engineered To Produce ω-3/ω-6 PUFAs**

| **Strain** | **Reference** | **ATCC Deposit No.** | **Fatty Acid Content (As A Percent [%] of Total Fatty Acids)** | | | | | | | | | | | | | | Lipid % dew |
|---|---|---|---|---|---|---|---|---|---|---|---|---|---|---|---|---|---|
| | | | 16:0 | 16:1 | 18:0 | 18:1 | 18:2 | 18:3 (ALA) | GLA | 20:2 | DGLA | ARA | ETA | EPA | DPA | DHA | |
| Wildtype | US 2006-0035351-A1; WO2006/033723 | #76982 | 14 | 11 | 3.5 | 34.8 | 31 | -- | 0 | -- | -- | -- | -- | -- | -- | -- | -- |
| pDMW208 | | -- | 11.9 | 8.6 | 1.5 | 24.4 | 17.8 | - | 25.9 | -- | -- | -- | -- | -- | -- | -- | -- |
| pDMW208D62 | | -- | 16.2 | 1.5 | 0.1 | 17.8 | 22.2 | -- | 34 | -- | -- | -- | -- | -- | -- | -- | -- |
| M4 | US 2006-0115881-A1; W02006/052870 | -- | 15 | 4 | 2 | 5 | 27 | -- | 35 | -- | 8 | 0 | 0 | 0 | -- | -- | -- |
| Y2034 | US 2006-0094092-A1; W02006/055322 | -- | 13.1 | 8.1 | 1.7 | 7.4 | 14.8 | -- | 25.2 | -- | 8.3 | 11.2 | -- | -- | | -- | -- |
| Y2047 | | PTA-7186 | 15.9 | 6.6 | 0.7 | 8.9 | 16.6 | -- | 29.7 | -- | 0 | 10.9 | -- | -- | -- | -- | -- |
| Y2214 | | -- | 7.9 | 15.3 | 0 | 13.7 | 37.5 | -- | 0 | -- | 7.9 | 14 | -- | -- | -- | -- | -- |
| EU | US 2006-0115881-A1; WO2006/052870 | -- | 19 | 10.3 | 2.3 | 15.8 | 12 | -- | 18.7 | -- | 5.7 | 0.2 | 3 | 10.3 | -- | -- | 36 |
| Y2072 | | -- | 7.6 | 4.1 | 2.2 | 16.8 | 13.9 | -- | 27.8 | -- | 3.7 | 1.7 | 2.2 | 15 | -- | -- | -- |
| Y2102 | | -- | 9 | 3 | 3.5 | 5.6 | 18.6 | -- | 29.6 | -- | 3.8 | 2.8 | 2.3 | 18.4 | -- | -- | -- |
| Y2088 | | -- | 17 | 4.5 | 3 | 2.5 | 10 | -- | 20 | -- | 3 | 2.8 | 1.7 | 20 | -- | -- | -- |
| Y2089 | | -- | 7.9 | 3.4 | 2.5 | 9.9 | 14.3 | -- | 37.5 | -- | 2.5 | 1.8 | 1.6 | 17.6 | -- | -- | - |
| Y2095 | | -- | 13 | 0 | 2.6 | 5.1 | 16 | -- | 29.1 | -- | 3.1 | 1.9 | 2.7 | 19.3 | -- | -- | -- |
| Y2090 | | -- | 6 | 1 | 6.1 | 7.7 | 12.6 | -- | 26.4 | -- | 6.7 | 2.4 | 3.6 | 26.6 | -- | -- | 22.9 |
| Y2096 | | PTA-7184 | 8.1 | 1 | 6.3 | 8.5 | 11.5 | -- | 25 | -- | 5.8 | 2.1 | 2.5 | 28.1 | -- | -- | 20.8 |
| Y2201 | | PTA-7185 | 11 | 16.1 | 0.7 | 18.4 | 27 | -- | -- | 3.3 | 3.3 | 1 | 3.8 | 9 | -- | -- | -- |
| Y3000 | US 2006-0110806-A1; W020061052871 | PTA-7187 | 5.9 | 1.2 | 5.5 | 7.7 | 11.7 | -- | 30.1 | -- | 2.6 | 1.2 | 1.2 | 4.7 | 18.3 | 5.6 | -- |
| Y4001 | WO2008/073367 | -- | 4.3 | 4.4 | 3.9 | 35.9 | 23 | 0 | -- | 23.8 | 0 | 0 | 0 | -- | -- | -- | |
| Y4036 | | -- | 7.7 | 3.6 | 1.1 | 14.2 | 32.6 | 0 | -- | 15.6 | 18.2 | 0 | 0 | -- | -- | -- | |
| Y4070 | | -- | 8 | 5.3 | 3.5 | 14.6 | 42.1 | 0 | -- | 6.7 | 2.4 | 11.9 | -- | -- | -- | -- | |
| Y4158 | | -- | 3.2 | 1.2 | 2.7 | 14.5 | 30.4 | 5.3 | -- | 6.2 | 3.1 | 0.3 | 14 | 20.5 | -- | -- | 27.3 |
| Y4184 | | -- | 3.1 | 1.5 | 1.8 | 8.7 | 31.5 | 4.9 | -- | 5.6 | 2.9 | 0.6 | 2.4 | 28.9 | -- | -- | 23.9 |

### Chromosomal Knockout Of Pex Genes

Following selection of a preferred *Yarrowia lipolytica* strain producing the desired ω-3/ω-6 PUFA (or combination of PUFAs thereof), one of skill in the art could readily engineer a suitable knockout construct, similar to pYPS161 in Example 7, to result in knockout of a chromosomal Pex gene upon transformation into the parental *Y. lipolytica* strain. Preferred Pex gene would include: YIPex1 p (GenBank Accession No. CAG82178; SEQ ID NO:1) YIPex2p (GenBank Accession No. CAG77647; SEQ ID NO:2), YIPex3p (GenBank Accession No. CAG78565; SEQ ID NO:3), YIPex3Bp (GenBank Accession No. CAG83356; SEQ ID NO:4), YIPex4p (GenBank Accession No. CAG79130; SEQ ID NO:5), YIPex5p (GenBank Accession No. CAG78803; SEQ ID NO:6), YIPex6p (GenBank Accession No. CAG82306; SEQ ID NO:7), YIPex7p (GenBank Accession No. CAG78389; SEQ ID NO:8), YIPex8p (GenBank Accession No. CAG80447; SEQ ID NO:9), YIPex12p (GenBank Accession No. CAG81532; SEQ ID NO:11), YIPex13p (GenBank Accession No. CAG81789; SEQ ID NO:12), YIPex14p (GenBank Accession No. CAG79323; SEQ ID NO:13), YIPex16p (GenBank Accession No. CAG79622; SEQ ID NO:14), YIPex17p (GenBank Accession No. CAG84025 SEQ ID NO:15), YIPex19p (GenBank Accession No. AAK84827; SEQ ID NO:16), YIPex20p (GenBank Accession No. CAG79226; SEQ ID NO:17), YIPex22p (GenBank Accession No. CAG77876; SEQ ID NO:18) and YIPex26p (GenBank Accession No. NC_006072, antisense translation of nucleotides 117230-118387; SEQ ID NO:19).

It would be expected that the chromosomal disruption of Pex would result in an increased amount of PUFAs in the total lipid fraction and in the oiL fraction, as a percent of total fatty acids, as compared with a eukaryotic organism whose native peroxisome biogenesis factor protein has not been disrupted, wherein the amount of PUFAs can be: 1) the PUFA that is the desired end product of a functional PUFA biosynthetic pathway, as opposed to PUFA intermediates or by-products, 2) C₂₀ and C₂₂ PUFAs, and/or 3) total PUFAs. Preferred results not only achieve an increase in the amount of PUFAs as a percent of total fatty acids but also result in an increased amount of PUFAs as a percent of dry cell weight, as compared with a eukaryotic organism whose native peroxisome biogenesis factor protein has not been disrupted. Again, the amount of PUFAs can be: 1) the PUFA that is the desired end product of a functional PUFA biosynthetic pathway, as opposed to PUFA intermediates or by-products, 2) the C₂₀ and C₂₂ PUFAs, and/or 3) the total PUFAs. In some cases, the total lipid content also increases, relative to that of a eukaryotic organism whose native peroxisome biogenesis factor protein has not been disrupted.

### EXAMPLE 9

### Chromosomal Deletion Of Pex16 In Yarrowia lipolytica Strain Y4036U Increases Percent DGLA Accumulated

The present Example describes use of construct pYRH13 (FIG. 9B; SEQ ID NO:59) to knock out the chromosomal Pex16 gene in the DGLA-producing *Yarrowia* strain Y4036U (Example 1). Transformation of Y. *lipolytica strain* Y4036U with the Pex16 knockout construct resulted in creation of strain Y4036U (Δpex16). The effect of the Pex16 knockout on DGLA level was determined and compared. Specifically, knockout of Pex16 resulted in an increased percentage of DGLA as a percent of total fatty acids in the cell.

### Construct pYRH13

Plasmid pYRH13 was derived from plasmid pYPS161 (FIG. 9A, SEQ ID NO:56; Example 7). Specifically, a 1982 bp 5' promoter region of the *Yarrowia lipolytica* Pex16 gene (GenBank Accession No. CAG79622) replaced the *As*cI/*Bsi*WI fragment of pYPS161 and a 448 bp 3' terminator region of the *Yarrowia lipolytica* Pex16 gene (GenBank Accession No. CAG79622) replaced the *PacI*/*SphI* fragment of pYPS161 to produce pYRH13 (SEQ ID NO:59; FIG.9B).

### Generation Of Yarrowia lipotytica Knockout Strain Y4036 (ΔPex16)

Standard protocols were used to transform *Yarrowia lipolytica* strain Y4036U (Example 1) with the purified 6.0 kB *Asc*I/*Sph*I fragment of Pex16 knockout construct pYRH13.

To screen for cells having the Pex16 deletion, colony PCR was performed using *Taq* polymerase (Invitrogen; Carlsbad, CA) and the PCR primers PEX16Fii (SEQ ID NO:60) and PEX16Rii (SEQ ID NO:61). This set of primers was designed to amplify a 1.1 kB region of the intact Pex16 gene, and therefore the Pex16 deleted mutant (i.e., Δpex16) would not produce the band. A second set of primers was designed to produce a band only when the Pex16 gene was deleted. Specifically, one primer (i.e., 3UTR-URA3; SEQ ID NO:62) binds to a region in the vector sequences of the introduced 6.0 kB *Asc*I/*Sph*I disruption fragment, and the other primer (i.e., PEX16-conf; SEQ ID NO:63) binds to the Pex16 terminator sequences of chromosome outside of the homologous region of the disruption fragment.

More specifically, the colony PCR was performed using a reaction mixture that contained: 20 mM Tris-HCl (pH 8.4), 50 mM KCI, 1.5 mM MgCl₂ 400 µM each of dGTP, dCTP, dATP, and dTTP, 2 µM of each primer, 20 µl water and 2 U *Taq* polymerase. Amplification was carried out as follows: initial denaturation at 94 °C for 120 sec, followed by 35 cycles of denaturation at 94 °C for 60 sec, annealing at 55 °C for 60 sec, and elongation at 72 °C fo 120 sec. A final elongation cycle at 72 °C for 5 min was carried out, followed by reaction termination at 4 °C.

Of 205 colonies screened, 195 had the Pex16 knockout fragment integrated at a random site in the chromosome and thus were not Δpex16 mutants (however, the cells could grow on ura- plates, due to the presence o pYRH13). Three of these random integrants, designated as Y4036U-17, Y4036U-19 and Y4036U-33, were used as controls in lipid production experiments (*infra*).

The remaining 10 colonies screened (i.e., of the total 205) contained the Pex16 knockout. These ten Δpex16 mutants within the Y4036U strain background were designated RHY25 through RHY34.

### Confirmation Of Yarrowia lipotytica Knockout Strain Y4036U (ΔPex16) By Quantitative Real Time PCR

Further confirmation of the Pex16 knockout in strains RHY25 through RHY34 was performed by quantitative real time PCR, with the *Yarrowia* translation elongation factor (tef-1) gene (GenBank Accession No. AF054510 used as the control.

First, real time PCR primers and TaqMan probes targeting the Pex16 gene and the tef-1 gene, respectively, were designed with Primer Express software v 2.0 (AppliedBiosystems, Foster City, CA). Specifically, real time PCR primers ef-324F (SEQ ID NO:64), ef-392R (SEQ ID NO:65), PEX16-741 F (SEQ ID NO:66) and PEX16-802R (SEQ ID NO:67) were designed, as well as the TaqMan probes ef-345T (i.e., 5' 6-FAM™-TGCTGGTGGTGTTGGTGAGTT-TAMRA™, wherein the nucleotide sequence is set forth as SEQ ID NO:68) and PEX16-760T (i.e., 5'-6FAM™-CTGTCCATTCTGCGACCCCTC-TAMRA™, wherein the nucleotide sequence is set forth as SEQ ID NO:69). The 5' end of the TaqMan fluorogenic probes have the 6FAM™ fluorescent reporter dye bound, while the 3' end comprises the TAMRA™ quencher. All primers and probes were obtained from Sigma-Genosys (Woodlands, TX).

Knockout candidate DNA was prepared by suspending 1 colony in 50 µl of water. Reactions for tef-1 and PEX16 were run separately, in triplicate for each sample. Real time PCR reactions included 20 pmoles each of forward and reverse primers (i.e., ef-324F, ef-392R, PEX16-741 F and PEX16-802R 5', *supra*), 5 pmoles TaqMan probe (i.e., ef-345T and PEX16-760T), 10 µl TaqMan Universal PCR Master Mix--No AmpErase^{®} Uracil-N-Glycosylase (UNG) (Catalog No. PN 4326614, Applied Biosystems), 1 µl colony suspension and 8.5 µl RNase/DNase free water for a total volume of 20 µl per reaction. Reactions were run on the ABI PRISM^{®} 7900 Sequence Detection System under the following conditions: initial denaturation at 95 °C for 10 min, followed by 40 cycles of denaturation at 95 °C for 15 sec and annealing at 60 °C for 1 min. Real time data was collected automatically during each cycle by monitoring 6-FAM™ fluorescence. Data analysis was performed using tef-1 gene threshold cycle (C_{T}) values for data normalization as per the ABI PRISM^{®} 7900 Sequence Detection System instruction manual

Based on this analysis, it was concluded that all ten of the Y4036U (Δpex16) colonies (i.e., RHY25 through RHY34) were valid Pex16 knockouts wherein the pYRH13 construct had integrated into the chromosomal YIPex16

### Evaluation Of Yarrowia lipotytica Strains Y4036U And Y4036U (ΔPex16) For DGLA Production

To evaluate the effect of the Pex16 knockout on the percent of PUFAs in the total lipid fraction and the total lipid content in the cells, the Y4036U an Y4036U (Δpex16) strains were grown under comparable oleaginous conditions. More specifically, strains Y4036U-17, Y4036U-19 and Y4036U-33 having the Pex16 knockout fragment integrated at a random site in the chromosome were considered as Pex16 wild type (i.e., Y4036U) and strains RHY25 through RHY34 were the Pex16 mutant strains (i.e., Y4036U (Δpex16)). Cultures of each strain were grown at a starting OD₆₀₀ of ∼0.1 in 25 mL of MM containing 90 mg/L L-leucine in a 125 mL flask for 48 hrs. The cells were harvested by centrifugation for 5 min at 4300 rpm in a 50 mL conical tube. The supernatant was discarded and the cells were resuspended in 25 mL of HGM and transferred to a new 125 mL flask. The cells were incubated with aeration for an additional 120 hrs at 30 °C.

The fatty acid composition (i.e., LA (18:2), ALA, EDA and DGLA) for each of the strains is shown below in Table 13; fatty acid composition is expressed as the weight percent (wt. %) of total fatty acids. The average fatty acid composition of strains Y4036U and Y4036U (Δpex16) are highlighted in gray and indicated with "Ave". None of the strains tested provided sufficient cell mass in MM + L-leucine media, and thus total lipid content was not analyzed.

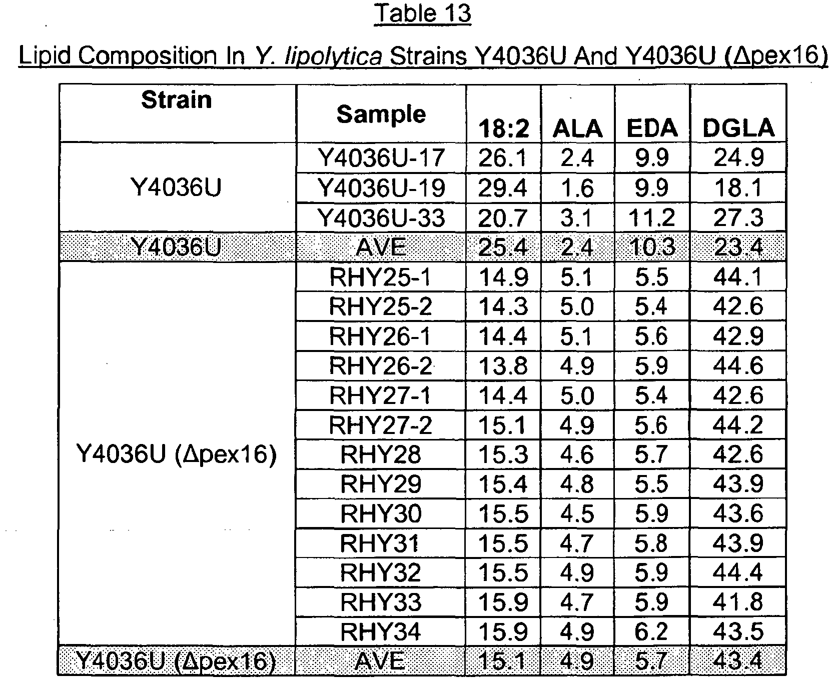

The results in Table 13 showed that knockout of the chromosomal Pex16 gene in Y4036U (Δpex16) increased the DGLA % TFAs approximately 85%, as compared to the DGLA % TFAs in strain Y4036U whose native Pex16p had not been knocked out. However, Y4036U (Δpex16) also had a ∼40% decrease in the LA (18:2) accumulation.

Thus, the results in Table 13 showed that compared to the parent strain Y4036, Y4036 (ΔPex16) strain had higher average DGLA % TFAs (43.4% versus 23.4%). Additionally, strain Y4036U (Δpex16) had a 1.65-fold increase in the amount of DGLA relative to the total PUFAs (62.8% of the PUFAs [as a % TFAs] versus 38.1 % of the PUFAs [as a % TFAs]) and a 1.3 fold increase in the amount of C20 PUFAs relative to the total PUFAs (71% of the PUFAs [as a % TFAs] versus 54.8% of the PUFAs [as a % TFAs]).

### EXAMPLE 10

### Generation Of Y4305 Strain To Produce About 53.2% EPA Of Total Lipids

*Y. lipolytica* strain Y4305U, having a *Ura-* phenotype, was used as the host in Example 11, *infra.* Strain Y4305 (a *Ura+* strain that was parent to Y4305U) was derived from *Y. lipolytica* ATCC #20362, and is capable of producing about 53.2% EPA relative to the total lipids via expression of a Δ9 elongase/Δ8 desaturase pathway.

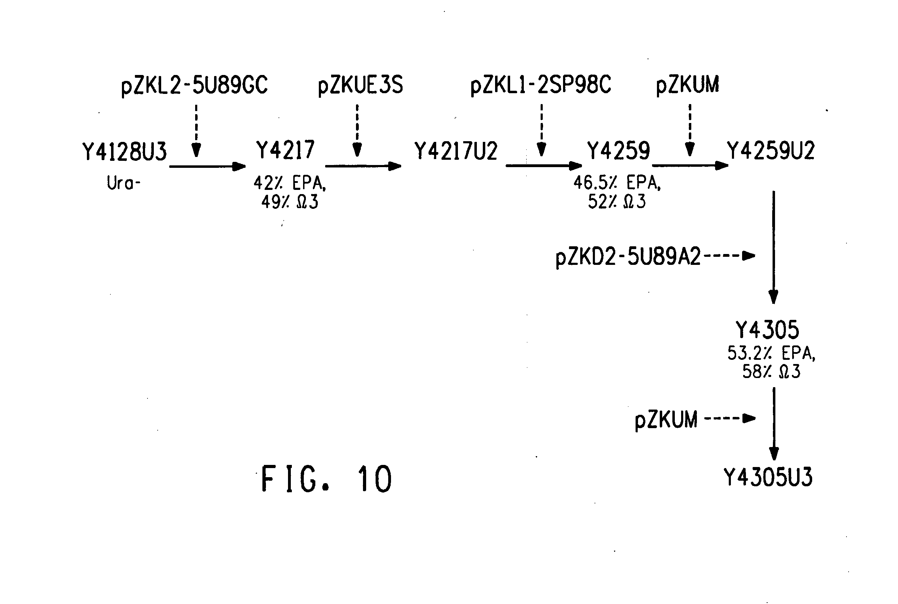

The development of strain Y4305U required the construction of strain Y2224, strain Y4001, strain Y4001 U, strain Y4036, strain Y4036U, strain Y4070 and strain Y4086 (*supra,* Example 1). Further development of strain Y4305U required construction of strain Y4086U1, strain Y4128 and strain Y4128U3 (*supra,* Example 2). Subsequently, development of strain Y4305U (diagrammed in FIG. 10) required construction of strain Y4217 (producing 42% EPA), strain Y4217U2 (*Ura*-), strain Y4259 (producing 46.5% EPA), strain Y4259U2 (*Ura*-) and strain Y4305 (producing 53.2% EPA).

Although the details concerning transformation and selection of the EPA-producing strains developed after strain Y4128U3 are not elaborated herein, the methodology used for isolation of strain Y4217, strain Y4217U2, strain Y4259, strain Y4259U2, strain Y4305 and strain Y4305U was as described in Examples 1 and 2.

Briefly, construct pZKL2-5U89GC (FIG. 8B; SEQ ID NO:55; described in Table 24 of Int'l. App. Pub. No. WO 2008/073367) was generated to integrate one Δ9 elongase gene (i.e., EgD9eS), one Δ8 desaturase gene (i.e. EgD8M), one Δ5 desaturase gene (i.e., EgD5S), and one *Yarrowia lipolytica* diacylglycerol cholinephosphotransferase (CPT1) gene into the *Lip2* loci (GenBank Accession No. AJ012632) of strain Y4128U3 to thereby enable higher level production of EPA. Six strains, designated as Y4215, Y4216, Y4217, Y4218, Y4219 and Y4220, produced about 41.1%, 41.8%, 41.7%, 41.1%, 41% and 41.1% EPA of total lipids, respectively.

Strain Y4217U1 and Y4217U2 were created by disrupting the *Ura3* gene in strain Y4217 via construct pZKUE3S (FIG. 5A; SEQ ID NO:31; described in Table 22 of Int'l. App. Pub. No. WO 2008/073367), comprising a chimeric EXP1::ME3S::Pex20 gene targeted for the *Ura3* gene. Construct pZKL1-2SP98C (FIG. 8A; SEQ ID NO:54; described in Table 23 of Int'l. App. Pub. No. WO 2008/073367) was utilized to integrate one Δ9 elongase gene (i.e., EgD9eS), one Δ8 desaturase gene (i.e., EgD8M), one Δ12 desaturase gene (i.e., FmD12S), and one *Yarrowia lipolytica* CPT1 gene into the *Lip1* locus (GenBank Accession No. Z50020) of strain Y4217U2, thereby resulting in isolation of strains Y4259, Y4260, Y4261, Y4262, Y4263 and Y4264, producing about 46.5%, 44.5%, 44.5%, 44.8%, 44.5% and 44.3% EPA of total lipids, respectively.

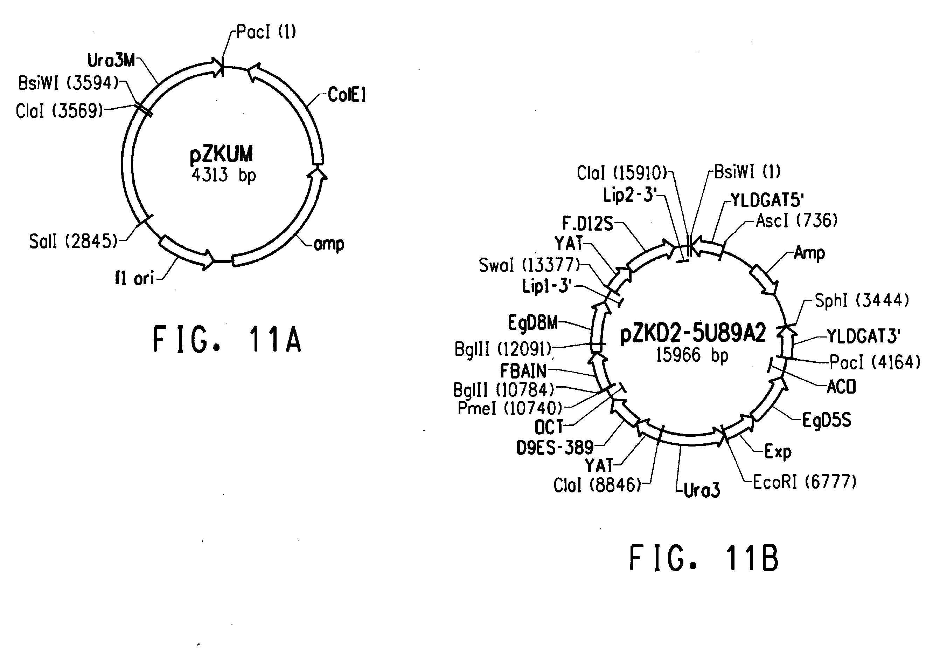

A *Ura-* derivative (i.e., strain Y4259U2) was then created, via transformation with construct pZKUM (FIG. 11A; SEQ ID NO:70; described in Table 33 of Int'l. App. Pub. No. WO 2008/073367) which integrated a *Ura3* mutant gene into the *Ura3* gene of strain Y4259, thereby resulting in isolation of strains Y4259U1, Y4259U2 and Y4259U3, respectively (collectively, Y4259U) (producing 31.4%, 31 % and 31.3% EPA of total lipids, respectively).

Finally, construct pZKD2-5U89A2 (FIG. 11B; SEQ ID NO:71) was generated to integrate one Δ9 elongase gene, one Δ5 desaturase gene, one Δ8 desaturase gene, and one Δ12 desaturase gene into the diacylglycerol acyltransferase (*DGAT2*) loci of strain Y4259U2, to thereby enable increased production of EPA. The pZKD2-5U89A2 plasmid contained the following components:

**Table 14**

| Description of Plasmid pZKD2-5U89A2 (SEQ ID NO:71) | |
|---|---|
| RE Sites And Nucleotides Within SEQ ID NO:71 | Description Of Fragment And Chimeric Gene Components |
| | |
| *Asc*I/*Bsi*WI (1-736) | 728 bp 5' portion of *Yarrowia DGAT2* gene (SEQ ID NO:72) (labeled as "YLDGAT5'" in Figure; U.S. Pat. No. 7,267,976) |
| *Pac*I/*Sph*I (4164-3444) | 714 bp 3' portion of *Yarrowia DGAT2* gene (SEQ ID NO:72) (labeled as "YLDGAT3"' in Figure; U.S. Pat. No. 7,267,976) |
| *Swa*I/*Bsi*WI (13377-1) | YAT1::FmD12S::Lip2, comprising: |
| | ● YAT1: *Yarrowia lipolytica* YAT1 promoter (labeled as "YAT" in Figure; Pat. Appl. Pub. No. US 2006/0094102-A1); |
| | ● FmD12S: codon-optimized Δ12 elongase (SEQ ID NO:74), derived from *Fusarium moniliforme* (labeled as "F.D12S" in Figure; Int'l. App. Pub. No. WO 2005/047485); |
| | ● Lip2: Lip2 terminator sequence from *Yarrowia Lip2* gene (GenBank Accession No. AJ012632) |
| P*me*I/*Swa*I (10740-13377) | FBAIN::EgD8M::Lip1 comprising: |
| | ● FBAIN: *Yarrowia lipolytica* FBAIN promoter (U.S. Pat. No. 7,202,356); |
| | ● EgDBM: Synthetic mutant Δ8 desaturase (SEQ ID NO:76; Pat. Appl. Pub. No. US 2008-0138868 A1), derived from *Euglena gracilis* ("EgD8S"; U.S. Pat. No. 7,256,033); |
| | ● Lip1: Lip1 terminator sequence from *Yarrowia Lip1* gene (GenBank Accession No. Z50020) |
| *Cla*I/*Pme*I (8846-10740) | YAT1::E389D9eS::OCT, comprising: |
| | ● YAT1: *Yarrowia lipolytica* YAT1 promoter (labeled as "YAT" in Figure; Pat. Appl. Pub. No. US 2006/0094102-A1); |
| | ● E389D9eS: codon-optimized Δ9 elongase (SEQ ID NO:78), derived from *Eutreptiella* sp. CCMP389 (labeled as "D9ES-389" in Figure; Int'l. App. Pub. No. WO 2007/061742); |
| | ● OCT: OCT terminator sequence from *Yarrowia OCT* gene (GenBank Accession No. X69988) |
| *Cla*I/*Eco*RI (8846-6777) | *Yarrowia* Ura3 gene (GenBank Accession No. AJ306421) |
| *Eco*RI/*Pac*I (6777-4164) | EXP1::EgD5S::ACO, comprising: |
| | ● EXP1: *Yarrowia lipolytica* export protein (EXP1) promoter (labeled as "Exp" in Figure; Int'l. App. Pub. No. WO 2006/052870); |
| | ● EgD5S: codon-optimized Δ5 desaturase (SEQ ID NO:80), derived from *Euglena gracilis* (Pat. Appl. Pub. No. US 2007-0292924-A1); |
| | ● Aco: Aco terminator sequence from *Yarrowia Aco* gene (GenBank Accession No. AJ001300) |

The pZKD2-5U89A2 plasmid was digested with *Asc*I/*Sph*I and then used for transformation of strain Y4259U2 according to the General Methods The transformed cells were plated onto MM plates, and plates were maintained at 30 °C for 3 to 4 days. Single colonies were re-streaked onto MM plates, and the resulting colonies were used to inoculate liquid MM. Liquid cultures were shaken at 250 rpm/min for 2 days at 30 °C. The cells were collected by centrifugation, resuspended in HGM, and then shaken at 250 rpm/min for 5 days. The cells were collected by centrifugation, and lipids were extracted. FAMEs were prepared by trans-esterification and subsequently analyzed with a Hewlett-Packard 6890 GC.

GC analyses showed that most of the selected 96 strains produced 40-46% EPA of total lipids. Four strains, designated as Y4305, Y4306, Y4307 and Y4308, produced about 53.2%, 46.4%, 46.8% and 47.8% EPA of total lipids, respectively. The complete lipid profile of Y4305 is as follows: 16:0 (2.8%), 16:1 (0.7%), 18:0 (1.3%), 18:1 (4.9%), 18:2 (17.6%), ALA (2.3%), EDA (3.4%), DGLA (2.0%), ARA (0.6%), ETA (1.7%) and EPA (53.2%). The total lipid % dry cell weight was 27.5.

The final genotype of strain Y4305 with respect to wild type *Yarrowia lipolytica* ATCC #20362 was SCP2- (YALI0E01298g), YALI0C18711g-, Pex10-, YALI0F24167g-, *unknown 1-, unknown 3-, unknown 8-,* GPD::FmD12::Pex20, YAT1::FmD12::OCT, GPM/FBAIN::FmD12S::OCT, EXP1::FmD12S::Aco, YAT1::FmD12S::Lip2, YAT1::ME3S::Pex16, EXP1::ME3S::Pex20 (3 copies), GPAT::EgD9e::Lip2, EXP1::EgD9eS::Lip1, FBAINm::EgD9eS::Lip2, FBA::EgD9eS::Pex20, GPD::EgD9eS::Lip2, YAT1::EgD9eS::Lip2, YAT1::E389D9eS::OCT, FBAINm::EgD8M::Pex20, FBAIN::EgD8M::Lip1 (2 copies), EXP1::EgD8M::Pex16, GPDIN::EgD8M::Lip1, YAT1::EgD8M::Aco, FBAIN::EgD5::Aco, EXP1::EgD5S::Pex20, YAT1::EgD5S::Aco, EXP1::EgD5S::ACO, YAT1::RD5S::OCT, YAT1::PaD17S::Lip1, EXP1::PaD17::Pex16, FBAINm::PaD17::Aco, YAT1::YICPT1::ACO, GPD::YICPT1::ACO.

In order to disrupt the *Ura3* gene in strain Y4305, construct pZKUM (FIG. 11A; SEQ ID NO:70; described in Table 33 of Int'l. App. Pub. No. WO 2008/073367) was used to integrate a *Ura3* mutant gene into the *Ura3* gene of strain Y4305. A total of 8 transformants grown on MM + 5-FOA plates were picked and re-streaked onto MM plates and MM + 5-FOA plates, separately. All 8 strains had a *Ura-* phenotype (i.e., cells could grow on MM 5-FOA plates, but not on MM plates). The cells were scraped from the MM + 5-FOA plates, and lipids were extracted. FAMEs were prepared by trans-esterification and subsequently analyzed with a Hewlett-Packard 6890 GC.

GC analyses showed the presence of 37.6%, 37.3% and 36.5% EPA of total lipids in pZKUM transformants #1, #6 and #7 grown on MM + 5-FOA plates. These three strains were designated as strains Y4305U1, Y4305U2 and Y4305U3, respectively (collectively, Y4305U). For clarity in Example 11, strain Y4305U is referred to as strain Y4305U (Δpex10).

### EXAMPLE 11

### Chromosomal Deletion Of Pex16 In Yarrowia lipolytica Strain Y4305U (Δpex10) Further Increased Percent EPA Accumulated (The Double Pex10-Pex16 Knockout)

The present Example describes use of construct pYRH13 (FIG. 9B; SEQ ID NO:59) to knock out the chromosomal Pex16 in *Yarrowia* strain Y4305U (Δpex10) (Example 10), to thereby result in a Pex10-Pex16 double mutant. The effect of the Pex10-Pex16 double knockout on total oil and EPA level was determined and compared. Specifically, the effect of the Pex10-Pex16 double mutation in strain Y4305U (Δpex10) (Δpex16) resulted in an increased amount of EPA in the cell (EPA % TFAs and EPA % DCW), as compared to the single mutant (i.e., strain Y4305U (Δpex10)).

### Generation Of Yarrowia lipolytica Knockout Strain Y4305U (Δpex10) (Δpex16)

Standard protocols were used to transform *Yarrowia lipolytica* strain Y4305U (Δpex10) (Example 10) with the purified 6.0 kB A*sc*I/*Sph*I fragment of Pex16 knockout construct pYRH13 (Example 9; SEQ ID NO:59). Screening and identification of cells having the Pex16 deletion was conducted by colony PCR, as described in Example 9.

Of 93 colonies screened, 88 had the Pex16 knockout fragment integrated at a random site in the chromosome and thus were not Δpex16 mutants (however, the cells could grow on Ura- plates, due to the presence of pYRH13). Two of these random integrants, designated as Y4305U-22 and Y4305U-25, were used as controls in lipid production experiments (*infra*).

The remaining 5 colonies screened (i.e., of the total 93) contained the Pex16 knockout. These five Δpex6 mutants within the Y4305U strain background were designated RHY20, RHY21, RHY22, RHY23 and RHY24. Further confirmation of the YIPex16 knockout was performed by quantitative real time PCR, as described in Example 9.

### Evaluation Of Yarrowia lipolytica Strains Y4305U (ΔPex10) And Y4305U (ΔPex10) (Δpex16) For EPA Production

To evaluate the effect of mutation in multiple Pex genes on the percent of PUFAs in the total lipid fraction and the total lipid content in the cells, Y4305U (Δpex10) and Y4305U (Δpex10) (Δpex6) strains were grown under comparable oleaginous conditions. More specifically, strains Y4305U-22 and Y4305U-25 having the Pex16 knockout fragment integrated at a random site in the chromosome were considered as Pex16 wild type, Pex10 knockouts (i.e., Y4305U (Δpex10)). Strains RHY22, RHY23 and RHY24 were the double knockout mutant strains (i.e., Y4305U (Δpex10) (Δpex16)). Cultures of each strain were grown in duplicate under comparable oleaginous conditions.

Specifically, cultures were grown at a starting OD₆₀₀ of ∼0.1 in 25 mL of synthetic dextrose media (SD) in a 125 mL flask for 48 hrs. The cells were harvested by centrifugation for 5 min at 4300 rpm in a 50 mL conical tube. The supernatant was discarded and the cells were re-suspended in 25 mL of HGM and transferred to a new 125 mL flask. The cells were incubated with aeration for an additional 120 hrs at 30 °C.

To determine the dry cell weight (DCW), the cells from 5 mL of the HGM-grown cultures were processed. The cultured cells were centrifuged for 5 min at 4300 rpm. The pellet was re-suspended using 10 mL of sterile water and was centrifuged under the same conditions for a second time. The pellet was then re-suspended using 1 mL of sterile H₂O (three times) and was transferred to a pre-weighed aluminum pan. The cell suspension was dried overnight in a vacuum oven at 80 °C. The weight of the cells was determined.

To determine the total lipid content, 1 mL of HGM cultured cells were collected by centrifugation for 1 min at 13,000 rpm, total lipids were extracted and FAMEs were prepared by trans-esterification, and subsequently analyzed with a Hewlett-Packard 6890 GC (General Methods).

The fatty acid composition (i.e., 16:0 (palmitate), 16:1 (palmitoleic acid), 18:0, 18:1 (oleic acid), 18:2 (LA), 18:3 (ALA), EDA, DGLA, ARA, ETrA, ETA and EPA) for each of the strains is shown below in Table 15 (expressed as the weight percent (wt. %) of total fatty acids (TFA)), as well as the DCW (g/L) and total lipid content (TFAs %DCW). The average fatty acid composition of strains Y4305U (Δpex10) and Y4305U (Δpex10) (Δpex16) are highlighted in gray and indicated with "Ave".

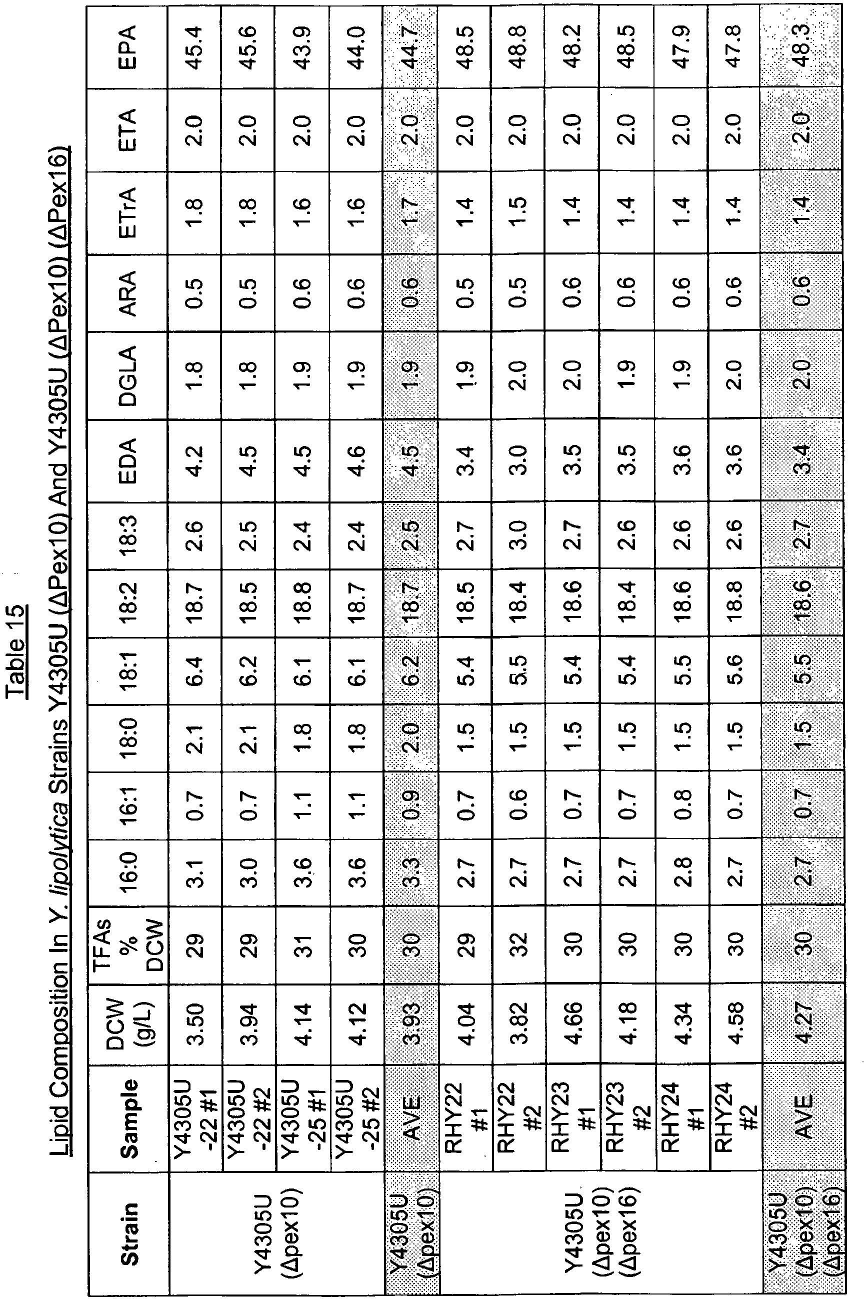

The results in Table 15 showed that knockout of the chromosomal Pex16 gene in Y4305U (Δpex10) (Δpex16) increased the EPA % TFAs approximately 8%, as compared to the EPA % TFAs in strain Y4305U (Δpex10) whose native Pex16p had not been knocked out. Additionally, the EPA % DCW was also increased in the double mutant as compared to in the single mutant strain, while the TFAs % DCW remained the same.

Thus, the results in Table 15 showed that compared to the control Y4305 (ΔPex10) strains, Y4305 (ΔPex10, ΔPex16) strains on average had higher EPA % TFAs (48.3% versus 44.7%) and higher EPA % DCW (14.57% versus 13.23%). Strain Y4305 (ΔPex10, ΔPex16) had only a 1.05-fold increase in the amount of EPA relative to the total PUFAs (61% of the PUFA [as a % TFAs] versus 58.3% of the PUFAs [as a % TFAs]) relative to strain Y4305 (ΔPex10), while the increase in the amount of C20 PUFAs relative to the total PUFAs was effectively identical (73% of the PUFAs [as a % TFAs] versus 72% of the PUFAs [as a % TFAs]).

### EXAMPLE 12

### Chromosomal Deletion Of Pex3 In Yarrowia lipolytica Strain Y4036U Increases Percent DGLA Accumulated

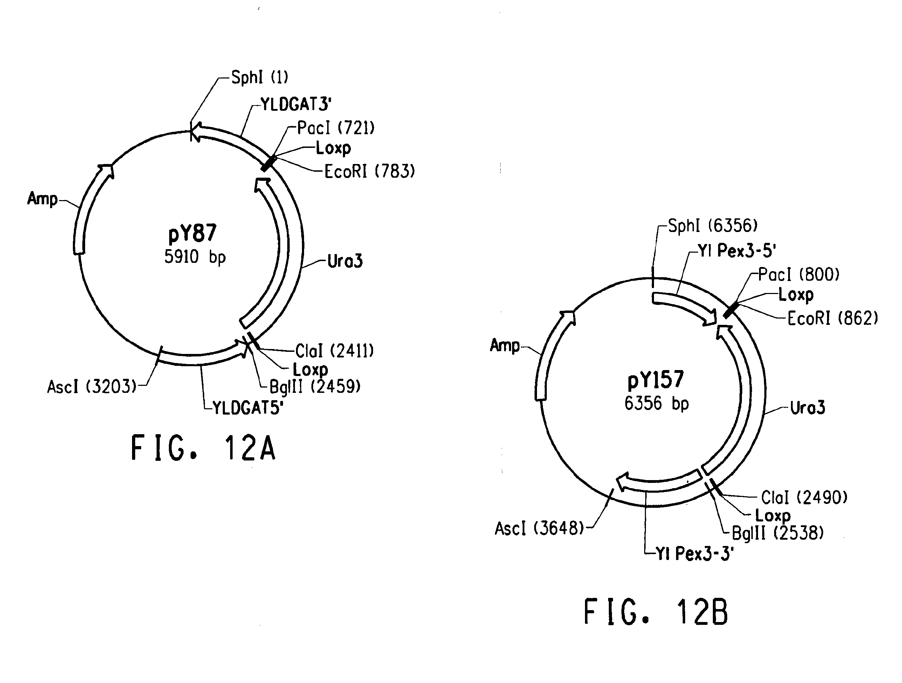

The present Example describes use of construct pY157 (FIG. 12B; SEQ ID NO:82) to knock out the chromosomal Pex3 gene (SEQ ID NO:3) in the Ura-, DGLA-producing *Yarrowia* strain Y4036U (Example 1). Transformation of *Y. lipolytica* strain Y4036U with the Pex3 knockout construct resulted in creation of strain Y4036 (Δpex3). The effect of the Pex3 knockout on DGLA level was determined and compared to the control strain Y4036 (a Ura+ strain that was parent to strain Y4036U). Specifically, knockout of Pex3 increased DGLA as a percentage of total fatty acids and improved ca. 3-fold DGLA % DCW, compared to the control.

### Construct pY157

Plasmid pY87 (FIG. 12A) contained a cassette to knock out the *Yarrowia lipolytica* diacylglycerol acyltransferase (*DGAT2*) gene, as described below in Table 16:

**Table 16**

| Description of Plasmid pY87 (SEQ ID NO:83) | |
|---|---|
| RE Sites And Nucleotides Within SEQ ID NO:83 | Description Of Fragment And Chimeric Gene Components |
| *Sph*I/*Pac*I (1-721) | 5' portion of *Yarrowia DGAT2* gene (bases 1-720 of SEQ NO:72) (U.S. Patent 7,267,976) |
| *Pac*I/ *Bg*/II (721-2459) | LoxP::Ura3::LoxP, comprising: |
| | ● LoxP sequence (SEQ ID NO:84); |
| | ● *Yarrowia* Ura3 gene (GenBank Accession No. AJ306421 |
| | ● LoxP sequence (SEQ ID NO:84) |
| *Bg*/II/*Asc*I (2459-3203) | 3' portion of *Yarrowia DGAT2* gene (bases 2468-3202 of SEQ ID NO:72) (U.S. Patent 7,267,976) |
| *Asc*I/*Sph*I (3203-5910) | Vector backbone including: |
| | ● *ColE1* plasmid origin of replication; |
| | ● ampicillin-resistance gene (Amp^{R}) for selection in *E*. *coli* (4191-5051); |
| | ● *E. coli* f1 origin of replication |

Plasmid pY157 was derived from plasmid pY87. Specifically, a 704 bp 5' promoter region of the *Yarrowia lipolytica* Pex3 gene replaced the *Sph*I/*Pac*I fragment of pY87 and a 448 bp 3' terminator region of the *Yarrowia lipolytica* Pex3 gene replaced the Bg*l*II/AscI fragment of pY87 to produce pY157 (SEQ ID NO:82; FIG. 12B).

### Generation Of Yarrowia lipolytica Knockout Strain Y4036 (ΔPex3)

Standard protocols were used to transform *Yarrowia lipolytica* strain Y4036U (Example 1) with the purified 3648 bp *Asc*I/*Sph*I fragment of Pex3 knockout construct pY157 (*supra*).

To screen for cells having the Pex3 deletion, colony PCR was performed using *Taq* polymerase (Invitrogen; Carlsbad, CA) and the PCR primers UP 768 (SEQ ID NO:85) and LP 769 (SEQ ID NO:86). This set of primers was designed to amplify a 2039 bp wild type band of the intact Pex3 gene and 3719 bp knockout-specific band when the Pex3 gene was disrupted by targeted knockout.

More specifically, the colony PCR was performed using a MasterAmp Taq kit (Epicentre Technologies, Madison, WI; Catalog No. 82250) and the manufacturer's instructions in a 25 µl reaction comprising: 2.5 µl of 10X MasterAmp Taq buffer, 2.0 µl of 25 mM MgCl₂, 7.5 µl of 10X MasterAmp Enhancer, 2.5 µl of 2.5 mM dNTPs (TaKaRa Bio Inc., Otsu Shiga, Japan), 1.0 µl of 10 µM Upper primer, 1.0 µl of 10 µM Lower primer, 0.25 µl of MasterAmp Taq DNA polymerase and 19.75 µl of water. Amplification was carried out as follows: initial denaturation at 95 °C for 5 min, followed by 40 cycles of denaturation at 95 °C for 30 sec, annealing at 56 °C for 60 sec, and elongation at 72 °C for 4 min. A final elongation cycle at 72 °C for 10 min was carried out, followed by reaction termination at 4 °C.

Of 48 colonies screened, 46 had the 2039 bp band expected from the wild type (i.e., undisrupted) Pex3 gene thus were not Δpex3 mutants. The remaining 2 colonies showed only a faint band of 2039 bp, suggesting that they were Δpex3 mutants with some contaminating untransformed cells present in the background. This was confirmed by streaking the 2 putative knockout colonies on selection plates to isolate single colonies. Then, genomic DNA was isolated from 3 single colonies from each putative knockout strain and screened by the same primer pair. i.e., UP 768 and LP 769 (SEQ ID NOs:85 and 86). This method was considered more sensitive than colony PCR. All three single colonies from both primary transformants lacked the 2039 bp wild type band and instead possessed the 3719 bp knockout-specific band. The two Δpex3 mutants within the Y4036U strain background were designated L134 and L135.

### Evaluation Of Yarrowia lipolytica Strains Y4036 And Y4036 (ΔPex3) For DGLA Production

To evaluate the effect of the Pex3 knockout on the percent of PUFAs in the total lipid fraction and the total lipid content in the cells, the Y4036 and Y4036 (Δpex3) strains were grown under comparable oleaginous conditions. Strains Y4036, L134 (i.e., Y4036 (Δpex3)) and L135 (i.e., Y4036 (Δpex3)) were inoculated into 25 mL of CSM-Ura and grown at 30 °C overnight in a shaker. The preculture was aliquoted into fresh 25 mL CSM-Ura flasks at a final OD₆₀₀ of 0.4. Cultures were grown at 30 °C in shaker. After 48 hrs, the cells (which barely grew) were spun down and resuspended in fresh 25 mL CSM-Ura and continued to grow for 72 hrs. Cells were spun down, re-suspended in 25 mL of HGM, and continued to grow as above for 72 hrs. Cells were harvested by centrifugation, washed once in distilled water and resuspended in 25 mL water to give a final volume of 20.5 mL. An aliquot (1.5 mL) was used for lipid content, following extraction of the total lipids, preparation of FAMEs by base trans-esterification, and analysis by a Hewlett-Packard 6890 GC (General Methods). The remaining aliquot was dried down to measure dry cell weight (DCW), as described in Example 11.

The fatty acid composition (i.e., 16:0 (palmitate), 16:1 (palmitoleic acid), 18:0, 18:1 (oleic acid), 18:2 (LA), EDA and DGLA) for each of the strains is shown below in Table 17 (expressed as the weight percent (wt. %) of total fatty acids (TFA)), as well as the total lipid content (TFA %DCW). The conversion efficiency ("CE") was measured according to the following formula: ([product]/[substrate+product])*100, where 'product' includes the immediate product and all products in the pathway derived from it. Thus, the Δ12 desaturase conversion efficiency (Δ12 % CE) was calculated as: ([LA+EDA+DGLA]/[18:1 +LA+EDA+DGLA])*100; the Δ9 elongase conversion efficiency (Δ9 elo % CE) was calculated as:
([EDA+DGLA]/[LA+EDA+DGLA])*100; and, the Δ8 desaturase conversion efficiency (Δ8 % CE) was calculated as: ([DGLA]/[EDA+DGLA])*100. The average fatty acid composition of strains Y4036, L134 and L135 are highlighted in gray and indicated with "Ave", while "S.D." indicates the Standard Deviation. As expected, the Δpex3 strains did not grow on plates with oleate as a sole source of carbon.

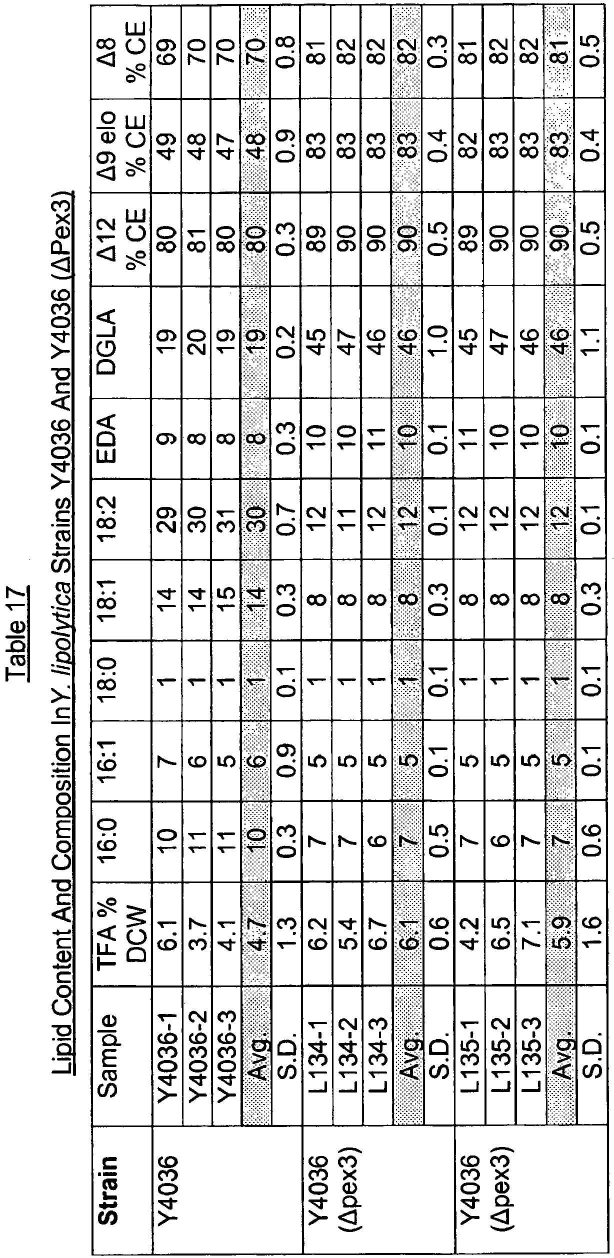

The results in Table 17 showed that knockout of the chromosomal Pex3 gene in Y4036 (Δpex3) increased the DGLA % TFAs approximately 142%, as compared to the DGLA % TFAs in strain Y4036 whose native Pex3p had not been knocked out. Specifically, the Pex3 knockout increased DGLA levels from ca. 19% in Y4036 to 46% in Y4036 (Δpex3) strains, L134 and L135. Additionally, the Δ9 elongase percent conversion efficiency increased from ca. 48% in Y4036 to 83% in Y4036 (Δpex3) strains, L134 and L135; and, TFA % DCW increased from 4.7% to 6% in the strains L134 and L135. The LA % TFAs decreased from 30% to 12%. Pex3 deletion indeed increases the flux of fatty acids and thus the substrate availability for Δ9 elongation.

Thus, the results in Table 17 showed that compared to the parent strain Y4036, Y4036 (ΔPex3) strain had on average higher lipid content (TFAs % DCW) (ca. 6.0% versus 4.7%), higher DGLA % TFAs (46% versus 19%), and higher DGLA % DCW (ca. 2.8% versus 0.9%). Additionally, strain Y4036 (ΔPex3) had a 2-fold increase in the amount of DGLA relative to the total PUFAs (67.7% of the PUFAs [as a % TFAs] versus 33.3% of the PUFAs [as a % TFAs]) and a 1.7-fold increase in the amount of C20 PUFAs relative to the total PUFAs (82% of the PUFAs [as a % TFAs] versus 47% of the PUFAs [as a % TFAs]).

It is hypothesized that the improved DGLA productivity would also result in improved EPA productivity in *Yarrowia lipolytica* strains engineered for EPA production (e.g., *Y*. *lipolytica* strain Y4305U, as described in Example, 10, and derivatives therefrom).

## Claims

1. A method of increasing the weight percent of at least one polyunsaturated fatty acid relative to the weight percent of total fatty acids in an oleaginous yeast or fungus having a total lipid content, a total lipid fraction and an oil fraction, comprising:
a) providing an oleaginous yeast or fungus which produces at least 25% of its dry cell weight as oil comprising:
1) genes encoding a functional polyunsaturated fatty acid biosynthetic pathway; and
2) a disruption in a native gene encoding a peroxisome biogenesis factor protein, thereby providing a PEX-disrupted organism, and
b) growing the PEX-disrupted organism under conditions as to increase the weight percent of at least one polyunsaturated fatty acid relative to the weight percent of total fatty acids in the total lipid fraction or in the oil fraction, when compared to the weight percent of the at least one polyunsaturated fatty acid relative to the weight percent of total fatty acids in the total lipid fraction or in the oil fraction in the oleaginous yeast or fungus in which no native gene encoding a peroxisome biogenesis factor protein has been disrupted.

2. A method of increasing the percent of at least one polyunsaturated fatty acid relative to the dry cell weight in an oleaginous yeast or fungus, comprising:
a) providing an oleaginous yeast or fungus which produces at least 25% of its dry cell weight as oil comprising:
1) genes encoding a functional polyunsaturated fatty acid biosynthetic pathway; and
2) a disruption in a native gene encoding a peroxisome biogenesis factor protein, thereby providing a PEX-disrupted organism, and
b) growing the PEX-disrupted organism under conditions as to increase the percent of at least one polyunsaturated fatty acid relative to the dry cell weight, when compared to the percent of the at least one polyunsaturated fatty acid relative to the dry cell weight in the oleaginous yeast or fungus in which no native gene encoding a peroxisome biogenesis factor protein has been disrupted.

3. The method of Claim 1 or 2, wherein the increase in the weight percent of the at least one polyunsaturated fatty acid relative to the weight percent of total fatty acids is at least 1.3, when compared to the weight percent of polyunsaturated fatty acids relative to the weight percent of total fatty acids in the total lipid fraction or in the oil fraction in an oleaginous yeast or fungus in which no native gene encoding a peroxisome biogenesis factor protein has been disrupted.

4. The method of Claim 1 or 2, wherein the at least one polyunsaturated fatty acid is selected from the group consisting of: linoleic acid, conjugated linoleic acid, γ-linolenic acid, dihomo-γ-linolenic acid, arachidonic acid, docosatetraenoic acid, ω-6 docosapentaenoic acid, α-linolenic acid, stearidonic acid, eicosatetraenoic acid, eicosapentaenoic acid, w-3 docosapentaenoic acid, eicosadienoic acid, eicosatrienoic acid, docosahexaenoic acid, hydroxylated or expoxy fatty acids of these, C₁₈ polyunsaturated fatty acids, C₂₀ polyunsaturated fatty acids, and C₂₂ polyunsaturated fatty acids.

5. The method of Claim 1, wherein the at least one polyunsaturated fatty acid consists of a combination of polyunsaturated fatty acids and wherein the weight percent of the combination is increased relative to the weight percent of total fatty acids.

6. The method of Claim 4, wherein the combination of polyunsaturated fatty acids consists of any combination of two or more polyunsaturated fatty acids selected from the group consisting of: linoleic acid, conjugated linoleic acid, γ-linolenic acid, dihomo-γ-linolenic acid arachidonic acid, docosatetraenoic acid, ω-6 docosapentaenoic acid, α-linolenic acid, stearidonic acid, eicosatetraenoic acid, eicosapentaenoic acid, ω-3 docosapentaenoic acid, eicosadienoic acid, eicosatrienoic acid, docosahexaenoic acid, hydroxylated or expoxy fatty acids of these, a combination of C₂₀ polyunsaturated fatty acids, a combination of C₂₀₋₂₂ polyunsaturated fatty acids, and a combination of C₂₂ polyunsaturated fatty acids.

7. The method of Claim 1 or 2, wherein the total lipid content in the PEX-disrupted organism is altered, when compared with the total lipid content in an oleaginous eukaryotic organism in which no native gene encoding a peroxisome biogenesis factor protein has been disrupted.

8. The method of Claim 1 or 2, wherein the PEX-disrupted organism is selected from the group consisting of: *Yarrowia, Candida, Rhodotorula, Rhodosporidium, Cryptococcus, Trichosporon, Lipomyces, Mortierella Thraustochytrium, Schizochytrium,* and *Saccharomyces* having the property of oleaginy.

9. The method of Claim 1 or 2, wherein the polyunsaturated fatty acid biosynthetic pathway comprises genes encoding enzymes selected from the group consisting of: Δ9 desaturase, Δ12 desaturase, Δ6 desaturase, Δ5 desaturase, Δ17 desaturase, Δ8 desaturase, Δ15 desaturase, Δ4 desaturase, C_{14/16} elongase, C_{16/18} elongase, C_{18/20} elongase, C_{20/22} elongase and Δ9 elongase.

10. The method of Claim 1 or 2, wherein the disruption in the native gene encoding a peroxisome biogenesis factor protein comprises a deletion selected from the group consisting of: a deletion in a portion of the gene encoding the C-terminal portion of the protein and a gene knockout.

11. The method of Claim 1 or 2, wherein the peroxisome biogenesis factor protein is selected from the group consisting of: Pex1 p, Pex 2p, Pex3p, Pex3Bp, Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21p, Pex21 Bp, Pex22p, Pex22p-like and Pex26p.

12. The method of Claim 11, wherein the peroxisome biogenesis factor protein is selected from the group consisting of: peroxisome biogenesis factor 2 protein (Pex2p), peroxisome biogenesis factor 10 protein (Pex10p) and peroxisome biogenesis factor 12 protein (Pex12p), and wherein the disruption is a gene knockout or a deletion in a portion of the gene encoding the C-terminal portion of the C₃HC₄ zinc ring finger motif of the protein.

13. The method of any one of claims 1 to 12, further comprising obtaining the oil fraction or the total oil fraction produced from the organism.

14. The method of any one of claims 1 to 12, further comprising extracting fatty acids from the organism.

15. A PEX-disrupted *Yarrowia lipolytica,* wherein:
a) a disruption occurs in a native gene encoding a peroxisome biogenesis factor protein selected from the group consisting of Pex3p, Pex10p and Pex16p;and
b) the *Yarrowia lipolytica* comprises heterologous genes encoding a functional polyunsaturated fatty acid biosynthesis pathway.

## Patentansprüche

1. Verfahren zur Erhöhung des gewichtsprozentualen Anteils von mindestens einer mehrfach ungesättigten Fettsäure bezogen auf den gewichtsprozentualen Anteil der Gesamtfettsäuren in einer ölhaltigen Hefe oder in einem ölhaltigen Pilz mit einem Gesamtlipidgehalt, einer Gesamtlipidfraktion und einer Ölfraktion, umfassend:
a) Bereitstellen einer ölhaltigen Hefe oder eines ölhaltigen Pilzes, die/der mindestens 25 % ihres/seines Trockenzellgewichts als Öl produziert, umfassend:
1) Gene, die für einen funktionellen Biosynthese-Weg einer mehrfach ungesättigten Fettsäure kodieren; und
2) eine Disruption in einem nativen Gen, das für ein peroxisomales Biogenese-Faktor-Protein kodiert, wodurch ein PEX-disruptierter Organismus bereitgestellt wird, und
b) Züchten des PEX-disruptierten Organismus unter Bedingungen zur Erhöhung des gewichtsprozentualen Anteils von mindestens einer mehrfach ungesättigten Fettsäure bezogen auf den gewichtsprozentualen Anteil der Gesamtfettsäuren in der Gesamtlipidfraktion oder in der Ölfraktion im Vergleich zum gewichtsprozentualen Anteil der mindestens einen mehrfach ungesättigten Fettsäure bezogen auf den gewichtsprozentualen Anteil der Gesamtfettsäuren in der Gesamtlipidfraktion oder in der Ölfraktion in der ölhaltigen Hefe oder dem ölhaltigen Pilz, in der/dem kein für ein peroxisomales Biogenese-Faktor-Protein kodierendes natives Gen disruptiert wurde.

2. Verfahren zur Erhöhung des gewichtsprozentualen Anteils von mindestens einer mehrfach ungesättigten Fettsäure bezogen auf das Trockenzellgewicht in einer ölhaltigen Hefe oder einem ölhaltigen Pilz, umfassend:
a) Bereitstellen einer ölhaltigen Hefe oder eines ölhaltigen Pilzes, die/der mindestens 25 % ihres/seines Trockenzellgewichts als Öl produziert, umfassend:
1) Gene, die für einen funktionellen Biosynthese-Weg einer mehrfach ungesättigten Fettsäure kodieren; und
2) eine Disruption in einem nativen Gen, das für ein peroxisomales Biogenese-Faktor-Protein kodiert, wobei ein PEX-disruptierter Organismus bereitgestellt wird, und
b) Züchten des PEX-disruptierten Organismus unter Bedingungen zur Erhöhung des prozentualen Anteils von mindestens einer mehrfach ungesättigten Fettsäure bezogen auf das Trockenzellgewicht im Vergleich zum prozentualen Anteil der mindestens einen mehrfach ungesättigten Fettsäure bezogen auf das Trockenzellgewicht in der ölhaltigen Hefe oder in dem ölhaltigen Pilz, in der/dem kein für ein peroxisomales Biogenese-Faktor-Protein kodierendes natives Gen disruptiert wurde.

3. Verfahren nach Anspruch 1 oder 2, wobei die Erhöhung des gewichtsprozentualen Anteils der mindestens einen mehrfach ungesättigten Fettsäure bezogen auf den gewichtsprozentualen Anteil der Gesamtfettsäuren mindestens 1,3 beträgt, im Vergleich zum gewichtsprozentualen Anteil der mehrfach ungesättigten Fettsäuren bezogen auf den gewichtsprozentualen Anteil der Gesamtfettsäuren in der Gesamtlipidfraktion oder in der Ölfraktion in einer ölhaltigen Hefe oder in einem ölhaltigen Pilz, in der/dem kein für ein peroxisomales Biogenese-Faktor-Protein kodierendes natives Gen disruptiert wurde.

4. Verfahren nach Anspruch 1 oder 2, wobei die mindestens eine mehrfach ungesättigte Fettsäure aus der Gruppe ausgewählt ist, bestehend aus: Linolsäure, konjugierter Linolsäure, γ-Linolensäure, Dihomo-γ-linolensäure, Arachidonsäure, Docosatetraensäure, ω-6-Docosapentaensäure, α-Linolensäure, Stearidonsäure, Eicosatetraensäure, Eicosapentaensäure, ω-3-Docosapentaensäure, Eicosadiensäure, Eicosatriensäure, Docosahexaensäure, hydroxylierten oder Expoxyfettsäuren von diesen, mehrfach ungesättigten C₁₈-Fettsäuren, mehrfach ungesättigten C₂₀-Fettsäuren und mehrfach ungesättigten C₂₂-Fettsäuren.

5. Verfahren nach Anspruch 1, wobei die mindestens eine mehrfach ungesättigte Fettsäure aus einer Kombination von mehrfach ungesättigten Fettsäuren besteht und wobei der gewichtsprozentuale Anteil der Kombination bezogen auf den gewichtsprozentualen Anteil der Gesamtfettsäuren erhöht ist.

6. Verfahren nach Anspruch 4, wobei die Kombination von mehrfach ungesättigten Fettsäuren aus jedweder Kombination von zwei oder mehr mehrfach ungesättigten Fettsäuren besteht, die aus der Gruppe ausgewählt sind, bestehend aus: Linolsäure, konjugierter Linolsäure, γ-Linolensäure, Dihomo-γ-linolensäure, Arachidonsäure, Docosatetraensäure, ω-6-Docosapentaensäure, α-Linolensäure, Stearidonsäure, Eicosatetraensäure, Eicosapentaensäure, ω-3-Docosapentaensäure, Eicosadiensäure, Eicosatriensäure, Docosahexaensäure, hydroxylierten oder Expoxyfettsäuren von diesen, einer Kombination von mehrfach ungesättigten C₂₀-Fettsäuren, einer Kombination von mehrfach ungesättigten C₂₀₋₂₂-Fettsäuren und einer Kombination von mehrfach ungesättigten C₂₂-Fettsäuren.

7. Verfahren nach Anspruch 1 oder 2, wobei der Gesamtlipidgehalt im PEX-disruptierten Organismus im Vergleich zum Gesamtlipidgehalt in einem ölhaltigen eukaryotischen Organismus verändert ist, in dem kein für ein peroxisomales Biogenese-Faktor-Protein kodierendes natives Gen disruptiert wurde.

8. Verfahren nach Anspruch 1 oder 2, wobei der PEX-disruptierte Organismus ausgewählt ist aus der Gruppe, bestehend aus *Yarrowia, Candida, Rhodotorula, Rhodosporidium, Cryptococcus,* Trichosporon, *Lipomyces, Mortierella, Thraustochytrium, Schizochytrium* und *Saccharomyces,* welche die Eigenschaft der Ölhaltigkeit aufweisen.

9. Verfahren nach Anspruch 1 oder 2, wobei der Biosynthese-Weg der mehrfach ungesättigten Fettsäure Gene umfasst, die für Enzyme kodieren, die aus der Gruppe ausgewählt sind, bestehend aus: Δ9-Desaturase, Δ12-Desaturase, Δ6-Desaturase, Δ5-Desaturase, Δ17-Desaturase, Δ8-Desaturase, Δ15-Desaturase, Δ4-Desaturase, C_{14/16}-Elongase, C_{16/18}-Elongase, C_{18/20}-Elongase, C_{20/22}-Elongase und Δ9-Elongase.

10. Verfahren nach Anspruch 1 oder 2, wobei die Disruption im nativen Gen, das für ein peroxisomales Biogenese-Faktor-Protein kodiert, eine Deletion umfasst, die aus der Gruppe ausgewählt ist, bestehend aus: einer Deletion in einem Anteil des Gens, das für den C-termialen Anteil des Proteins kodiert und einem Gen-Knockout.

11. Verfahren nach Anspruch 1 oder 2, wobei das peroxisomale Biogenese-Faktor-Protein aus der Gruppe ausgewählt ist, bestehend aus: Pex1p, Pex2p, Pex3p, Pex3Bp, Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21p, Pex21Bp, Pex22p, einem Pex22p ähnlichen und Pex26p.

12. Verfahren nach Anspruch 11, wobei das peroxisomale Biogenese-Faktor-Protein ausgewählt ist aus der Gruppe, bestehend aus: dem peroxisomalen Biogenese-Faktor-2-Protein (Pex2p), dem peroxisomalen Biogenese-Faktor-10-Protein (Pex10p) und dem peroxisomalen Biogenese-Faktor-12-Protein (Pex12p), und wobei die Disruption ein Gen-Knockout oder eine Deletion in einem Anteil des Gens ist, das für den C-terminalen Anteil des C₃HC₄-Zinkfingermotivs des Proteins kodiert.

13. Verfahren nach einem der Ansprüche 1 bis 12, das ferner den Erhalt der aus dem Organismus produzierten Ölfraktion oder der Gesamtölfraktion umfasst.

14. Verfahren nach einem der Ansprüche 1 bis 12, das ferner das Extrahieren von Fettsäuren aus dem Organismus umfasst.

15. PEX-disruptierte *Yarrowia lipolytica,* wobei:
a) eine Disruption in einem nativen Gen auftritt, das für ein peroxisomales Biogenese-Faktor-Protein kodiert, ausgewählt aus der Gruppe, die aus Pex3p, Pex10p und Pex16 besteht; und
b) *Yarrowia lipolytica* heterologe Gene umfasst, die für einen funktionellen Biosynthese-Weg einer mehrfach ungesättigten Fettsäure kodieren.

## Revendications

1. Procédé pour l'augmentation du pourcentage en poids d'au moins un acide gras polyinsaturé relativement au pourcentage en poids du total d'acides gras dans une levure ou un champignon oléagineux possédant une teneur totale en lipides, une fraction totale de lipides et une fraction d'huile, comprenant:
a) la fourniture d'une levure ou d'un champignon oléagineux qui produit au moins 25% de son poids cellulaire sec sous forme d'huile comprenant:
1) des gènes codant pour une voie de biosynthèse d'acide gras polyinsaturé fonctionnelle; et
2) une perturbation dans un gène naturel codant pour une protéine de facteur de biogénèse de peroxysome (PEX), fournissant ainsi un organisme à PEX perturbé, et
b) la croissance de l'organisme à PEX perturbé dans des conditions de manière à augmenter le pourcentage en poids d'au moins un acide gras polyinsaturé relativement au pourcentage en poids du total d'acides gras dans la fraction totale de lipides ou dans la fraction d'huile, lorsqu'il est comparé au pourcentage en poids du au moins un acide gras polyinsaturé relativement au pourcentage en poids du total d'acides gras dans la fraction totale de lipides ou dans la fraction d'huile dans la levure ou le champignon oléagineux dans lequel aucun gène naturel codant pour une protéine de facteur de biogénèse de peroxysome n'a été perturbé.

2. Procédé pour l'augmentation du pourcentage d'au moins un acide gras polyinsaturé relativement au poids cellulaire sec dans une levure ou un champignon oléagineux, comprenant:
a) la fourniture d'une levure ou d'un champignon oléagineux qui produit au moins 25% de son poids cellulaire sec sous forme d'huile comprenant:
1) des gènes codant pour une voie de biosynthèse d'acide gras polyinsaturé fonctionnelle; et
2) une perturbation dans un gène naturel codant pour une protéine de facteur de biogénèse de peroxysome, fournissant ainsi un organisme à PEX perturbé, et
b) la croissance de l'organisme à PEX perturbé dans des conditions de manière à augmenter le pourcentage d'au moins un acide gras polyinsaturé relativement au poids cellulaire sec, lorsqu'il est comparé au pourcentage du au moins un acide gras polyinsaturé relativement au poids cellulaire sec dans la levure ou le champignon oléagineux dans lequel aucun gène naturel codant pour une protéine de facteur de biogénèse de peroxysome n'a été perturbé.

3. Procédé selon la revendication 1 ou 2, dans lequel l'augmentation du pourcentage en poids du au moins un acide gras polyinsaturé relativement au pourcentage en poids du total d'acides gras est d'au moins 1,3, lorsqu'il est comparé au pourcentage en poids des acides gras polyinsaturés relativement au pourcentage en poids du total d'acides gras dans la fraction totale de lipides ou dans la fraction d'huile dans une levure ou un champignon oléagineux dans lequel aucun gène naturel codant pour une protéine de facteur de biogénèse de peroxysome n'a été perturbé.

4. Procédé selon la revendication 1 ou 2, dans lequel le au moins un acide gras polyinsaturé est choisi dans le groupe constitué de: acide linoléique, acide linoléique conjugué, acide γ-linolénique, acide dihomo-γ-linolénique, acide arachidonique, acide docosatétraénoïque, acide ω-6 docosapentaénoïque, acide α-linolénique, acide stéaridonique, acide eicosatétraénoïque, acide eicosapentaénoïque, acide ω-3 docosapentaénoïque, acide eicosadiénoïque, acide eicosatriénoïque, acide docosahexaénoïque, acides gras hydroxylés ou époxy de ceux-ci, acides gras C₁₈ polyinsaturés, acides gras C₂₀ polyinsaturés et acides gras C₂₂ polyinsaturés.

5. Procédé selon la revendication 1, dans lequel le au moins un acide gras polyinsaturé est constitué d'une combinaison d'acides gras polyinsaturés et dans lequel le pourcentage en poids de la combinaison est augmenté relativement au pourcentage en poids du total d'acides gras.

6. Procédé selon la revendication 4, dans lequel la combinaison d'acides gras polyinsaturés est constituée de toute combinaison de deux acides gras polyinsaturés ou plus choisis dans le groupe constitué de: acide linoléique, acide linoléique conjugué, acide γ-linolénique, acide dihomo-γ-linolénique, acide arachidonique, acide docosatétraénoïque, acide ω-6 docosapentaénoïque, acide α-linolénique, acide stéaridonique, acide eicosatétraénoïque, acide eicosapentaénoïque, acide ω-3 docosapentaénoïque, acide eicosadiénoïque, acide eicosatriénoïque, acide docosahexaénoïque, acides gras hydroxylés ou époxy de ceux-ci, une combinaison d'acides gras C₂₀ polyinsaturés, une combinaison d'acides gras C₂₀₋₂₂ polyinsaturés et une combinaison d'acides gras C₂₂ polyinsaturés.

7. Procédé selon la revendication 1 ou 2, dans lequel la teneur totale en lipides dans l'organisme à PEX perturbé est modifiée, lorsqu'elle est comparée à la teneur totale en lipides dans un organisme eucaryote oléagineux dans lequel aucun gène naturel codant pour une protéine de facteur de biogénèse de peroxysome n'a été perturbé.

8. Procédé selon la revendication 1 ou 2, dans lequel l'organisme à PEX perturbé est choisi dans le groupe constitué de: *Yarrowia, Candida, Rhodotorula, Rhodosporidium, Cryptococcus, Trichosporon, Lipomyces, Mortierella, Thraustochytrium, Schizochytrium* et *Saccharomyces* ayant la propriété d'être oléagineux.

9. Procédé selon la revendication 1 ou 2, dans lequel la voie de biosynthèse d'acide gras polyinsaturé comprend des gènes codant pour des enzymes choisies dans le groupe constitué de: Δ9 désaturase, Δ12 désaturase, Δ6 désaturase, Δ5 désaturase, Δ17 désaturase, Δ8 désaturase, Δ15 désaturase, Δ4 désaturase, C_{14/16} élongase, C_{16/18} élongase, C_{18/20} élongase, C_{20/22} élongase et Δ9 élongase.

10. Procédé selon la revendication 1 ou 2, dans lequel la perturbation dans le gène naturel codant pour une protéine de facteur de biogénèse de peroxysome comprend une délétion choisie dans le groupe constitué de: une délétion dans une portion du gène codant pour la portion C-terminale de la protéine et une inactivation de gène.

11. Procédé selon la revendication 1 ou 2, dans lequel la protéine de facteur de biogénèse de peroxysome est choisie dans le groupe constitué de: Pex1 p, Pex2p, Pex3p, Pex3Bp, Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21p, Pex21Bp, Pex22p, de type Pex22p et Pex26p.

12. Procédé selon la revendication 11, dans lequel la protéine de facteur de biogénèse de peroxysome est choisie dans le groupe constitué de: protéine de facteur de biogénèse de peroxysome 2 (Pex2p), protéine de facteur de biogénèse de peroxysome 10 (Pex10p) et protéine de facteur de biogénèse de peroxysome 12 (Pex12p) et dans lequel la perturbation est une inactivation de gène ou une délétion dans une portion du gène codant pour la portion C-terminale du motif de doigt de cycle de zinc C₃HC₄ de la protéine.

13. Procédé selon l'une quelconque des revendications 1 à 12, comprenant en outre l'obtention de la fraction d'huile ou de la fraction totale d'huile produite à partir de l'organisme.

14. Procédé selon l'une quelconque des revendications 1 à 12, comprenant en outre l'extraction d'acides gras à partir de l'organisme.

15. *Yarrowia lipolytica* à PEX perturbé, dans laquelle:
a) une perturbation se produit dans un gène naturel codant pour une protéine de facteur de biogénèse de peroxysome choisie dans le groupe constitué de: Pex3p, Pex10p et Pex16p; et
b) la *Yarrowia lipolytica* comprend des gènes hétérologues codant pour une voie de biosynthèse d'acide gras polyinsaturé fonctionnelle.


### SEQUENCE LISTING

<110> E. I. du Pont de Nemours & Co., Inc.
   Zhu, Quinn
   Xue, Zhixiong
<120> PEROXISOME BIOGENESIS FACTOR PROTEIN (PEX) DISRUPTIONS FOR ALTERING POLYUNSATURATED FATTY ACIDS AND TOTAL LIPID CONTENT IN OLEAGINOUS EUKARYOTIC ORGANISMS
<130> CL3847
<160> 89
<170> PatentIn version 3.4
<210> 1
   <211> 1024
   <212> PRT
   <213> Yarrowia lipolytica CLIB122 (GenBank Accession No. CAG82178)
<220>
   <221> MISC_FEATURE
   <222> (1)..(1024)
   <223> YlPex1p
<400> 1
   Met Thr Ser Lys Ser Asp Tyr Ser Gly Lys Asp Lys Ile Glu Leu Asp
   1               5                   10                  15
   Pro Val Phe Ala Lys Ser Ile Asp Leu Leu Pro Asn Thr Gln Val Val
               20                  25                  30
   Ile Asp Ile Gln Leu Asn Pro Lys Ile Ala His Thr Ile His Leu Glu
           35                  40                  45
   Pro Val Thr Val Ala Asp Trp Glu Ile Val Glu Leu His Ala Ala Tyr
       50                  55                  60
   Leu Glu Ser Arg Met Ile Asn Gln Val Arg Ala Val Ser Pro Asn Gln
   65                  70                  75                  80
   Pro Val Thr Val Tyr Pro Ser Ser Thr Thr Ser Ala Thr Leu Lys Val
                   85                  90                  95
   Ile Arg Ile Glu Pro Asp Leu Gly Ala Ala Gly Phe Ala Lys Leu Ser
               100                 105                 110
   Pro Asp Ser Glu Val Val Val Ala Pro Lys Gln Arg Lys Lys Glu Glu
           115                 120                 125
   Lys Gln Val Lys Lys Arg Ser Gly Ser Ala Arg Ser Thr Gly Ser Gln
   130 135 140
   Lys Arg Lys Gly Gly Arg Gly Pro His Ala Leu Arg Arg Ala Ile Ser
   145 150 155 160
   Glu Asp Phe Asp Gly His Leu Arg Leu Glu Val Ser Leu Asp Val Ser
   165 170 175
   Gln Leu Pro Pro Glu Phe His Gln Leu Lys Asn Val Ser Ile Lys Val
   180 185 190
   Ile Thr Pro Pro Asn Leu Ala Ser Pro Gln Gln Ala Ala Ser Ile Ala
   195 200 205
   Val Glu Glu Lys Ser Glu Glu Ser Leu Ser Gln Asn Lys Pro Pro Ser
   210 215 220
   Ser Glu Pro Lys Val Glu Val Pro Pro Asp Ile Ile Asn Pro Ala Ser
   225 230 235 240
   Glu Ile Val Ala Thr Leu Val Asn Asp Thr Thr Ser Pro Thr Gly His
   245 250 255
   Ala Lys Leu Ser Tyr Ala Leu Ala Asp Ala Leu Gly Ile Pro Ser Ser
   260 265 270
   Val Gly His Val Ile Arg Phe Glu Ser Ala Ser Lys Pro Leu Ser Gln
   275 280 285
   Lys Pro Gly Ala Leu Val Ile His Arg Phe Ile Thr Lys Thr Val Gly
   290 295 300
   Ala Ala Glu Gln Lys Ser Leu Arg Leu Lys Gly Glu Lys Asn Ala Asp
   305 310 315 320
   Asp Gly Val Ser Ala Asp Asp Gln Phe Ser Leu Leu Glu Glu Leu Lys
   325 330 335
   Lys Leu Gln Met Leu Glu Gly Pro Ile Thr Asn Phe Gln Arg Leu Pro
   340 345 350
   Pro Ile Pro Glu Leu Leu Pro Leu Gly Gly Val Ile Gly Leu Gln Asn
   355 360 365
   Ser Glu Gly Trp Ile Gln Gly Gly Tyr Leu Gly Glu Glu Pro Ile Pro
   370 375 380
   Phe Val Ser Gly Ser Glu Ile Leu Arg Ser Glu Ser Ser Leu Ser Pro
   385 390 395 400
   Ser Asn Ile Glu Ser Glu Asp Lys Arg Val Val Gly Leu Asp Asn Met
   405 410 415
   Leu Asn Lys Ile Asn Glu Val Leu Ser Arg Asp Ser Ile Gly Cys Leu
   420 425 430
   Val Tyr Gly Ser Arg Gly Ser Gly Lys Ser Ala Val Leu Asn His Ile
   435 440 445
   Lys Lys Glu Cys Lys Val Ser His Thr His Thr Val Ser Ile Ala Cys
   450 455 460
   Gly Leu Ile Ala Gln Asp Arg Val Gln Ala Val Arg Glu Ile Leu Thr
   465 470 475 480
   Lys Ala Phe Leu Glu Ala Ser Trp Phe Ser Pro Ser Val Leu Phe Leu
   485 490 495
   Asp Asp Ile Asp Ala Leu Met Pro Ala Glu Val Glu His Ala Asp Ser
   500 505 510
   Ser Arg Thr Arg Gln Leu Thr Gln Leu Phe Leu Glu Leu Ala Leu Pro
   515 520 525
   Ile Met Lys Ser Arg His Val Ser Val Val Ala Ser Ala Gln Ala Lys
   530 535 540
   Glu Ser Leu His Met Asn Leu Val Thr Gly His Val Phe Glu Glu Leu
   545 550 555 560
   Phe His Leu Lys Ser Pro Asp Lys Glu Ala Arg Leu Ala Ile Leu Ser
   565 570 575
   Glu Ala Val Lys Leu Met Asp Gln Asn Val Ser Phe Ser Gln Asn Asp
   580 585 590
   Val Leu Glu Ile Ala Ser Gln Val Asp Gly Tyr Leu Pro Gly Asp Leu
   595 600 605
   Trp Thr Leu Ser Glu Arg Ala Gln His Glu Met Ala Leu Arg Gln Ile
   610 615 620
   Glu Ile Gly Leu Glu Asn Pro Ser Ile Gln Leu Ala Asp Phe Met Lys
   625                 630                 635                 640
   Ala Leu Glu Asp Phe Val Pro Ser Ser Leu Arg Gly Val Lys Leu Gln
                   645                 650                 655
   Lys Ser Asn Val Lys Trp Asn Asp Ile Gly Gly Leu Lys Glu Thr Lys
               660                 665                 670
   Ala Val Leu Leu Glu Thr Leu Glu Trp Pro Thr Lys Tyr Ala Pro Ile
           675                 680                 685
   Phe Ala Ser Cys Pro Leu Arg Leu Arg Ser Gly Leu Leu Leu Tyr Gly
       690                 695                 700
   Tyr Pro Gly Cys Gly Lys Thr Tyr Leu Ala Ser Ala Val Ala Ala Gln
   705                 710                 715                 720
   Cys Gly Leu Asn Phe Ile Ser Ile Lys Gly Pro Glu Ile Leu Asn Lys
                   725                 730                 735
   Tyr Ile Gly Ala Ser Glu Gln Ser Val Arg Glu Leu Phe Glu Arg Ala
               740                 745                 750
   Gln Ala Ala Lys Pro Cys Ile Leu Phe Phe Asp Glu Phe Asp Ser Ile
           755                 760                 765
   Ala Pro Lys Arg Gly His Asp Ser Thr Gly Val Thr Asp Arg Val Val
       770                 775                 780
   Asn Gln Met Leu Thr Gln Met Asp Gly Ala Glu Gly Leu Asp Gly Val
   785                 790                 795                 800
   Tyr Val Leu Ala Ala Thr Ser Arg Pro Asp Leu Ile Asp Pro Ala Leu
                   805                 810                 815
   Leu Arg Pro Gly Arg Leu Asp Lys Met Leu Ile Cys Asp Leu Pro Ser
               820                 825                 830
   Tyr Glu Asp Arg Leu Asp Ile Leu Arg Ala Ile Val Asp Gly Lys Met
           835                 840                 845
   His Leu Asp Gly Glu Val Glu Leu Glu Tyr Val Ala Ser Arg Thr Asp
       850                 855                 860
   Gly Phe Ser Gly Ala Asp Leu Gln Ala Val Met Phe Asn Ala Tyr Leu
   865                 870                 875                 880
   Glu Ala Ile His Glu Val Val Asp Val Ala Asp Asp Thr Ala Ala Asp
                   885                 890                 895
   Thr Pro Ala Leu Glu Asp Lys Arg Leu Glu Phe Phe Gln Thr Thr Leu
               900                 905                 910
   Gly Asp Ala Lys Lys Asp Pro Ala Ala Val Gln Asn Glu Val Met Asn
           915                 920                 925
   Ala Arg Ala Ala Val Ala Glu Lys Ala Arg Val Thr Ala Lys Leu Glu
       930                 935                 940
   Ala Leu Phe Lys Gly Met Ser Val Gly Val Asp Asn Asp Asp Asp Lys
   945                 950                 955                 960
   Pro Arg Lys Lys Ala Val Val Val Ile Lys Pro Gln His Met Asn Lys
                   965                 970                 975
   Ser Leu Asp Glu Thr Ser Pro Ser Ile Ser Lys Lys Glu Leu Leu Lys
               980                 985                 990
   Leu Lys Gly Ile Tyr Ser Gln Phe  Val Ser Gly Arg Ser  Gly Asp Met
           995                 1000                 1005
   Pro Pro  Gly Thr Ala Ser Thr  Asp Val Gly Gly Arg  Ala Thr Leu
       1010                 1015                 1020
   Ala
<210> 2
   <211> 381
   <212> PRT
   <213> Yarrowia lipolytica CLIB122 (GenBank Accession No. CAG77647)
<220>
   <221> MISC_FEATURE
   <222> (1)..(381)
   <223> YlPex2p
<400> 2
   Met Ser Ser Val Leu Arg Leu Phe Lys Ile Gly Ala Pro Val Pro Asn
   1               5                   10                  15
   Val Arg Val His Gln Leu Asp Ala Ser Leu Leu Asp Ala Glu Leu Val
               20                  25                  30
   Asp Leu Leu Lys Asn Gln Leu Phe Lys Gly Phe Thr Asn Phe His Pro
           35                  40                  45
   Glu Phe Arg Asp Lys Tyr Glu Ser Glu Leu Val Leu Ala Leu Lys Leu
       50                  55                  60
   Ile Leu Phe Lys Leu Thr Val Trp Asp His Ala Ile Thr Tyr Gly Gly
   65                  70                  75                  80
   Lys Leu Gln Asn Leu Lys Phe Ile Asp Ser Arg His Ser Ser Lys Leu
                   85                  90                  95
   Gln Ile Gln Pro Ser Val Ile Gln Lys Leu Gly Tyr Gly Ile Leu Val
               100                 105                 110
   Val Gly Gly Gly Tyr Leu Trp Ser Lys Ile Glu Gly Tyr Leu Leu Ala
           115                 120                 125
   Arg Ser Glu Asp Asp Val Ala Thr Asp Gly Thr Ser Val Arg Gly Ala
       130                 135                 140
   Ser Ala Ala Arg Gly Ala Leu Lys Val Ala Asn Phe Ala Ser Leu Leu
   145                 150                 155                 160
   Tyr Ser Ala Ala Thr Leu Gly Asn Phe Val Ala Phe Leu Tyr Thr Gly
                   165                 170                 175
   Arg Tyr Ala Thr Val Ile Met Arg Leu Leu Arg Ile Arg Leu Val Pro
               180                 185                 190
   Ser Gln Arg Thr Ser Ser Arg Gln Val Ser Tyr Glu Phe Gln Asn Arg
           195                 200                 205
   Gln Leu Val Trp Asn Ala Phe Thr Glu Phe Leu Ile Phe Ile Leu Pro
       210                 215                 220
   Leu Leu Gln Leu Pro Lys Leu Lys Arg Arg Ile Glu Arg Lys Leu Gln
   225                 230                 235                 240
   Ser Leu Asn Val Thr Arg Val Gly Asn Val Glu Glu Ala Ser Glu Gly
                   245                 250                 255
   Glu Leu Ala His Leu Pro Gln Lys Thr Cys Ala Ile Cys Phe Arg Asp
   260 265 270
   Glu Glu Glu Gln Glu Gly Gly Gly Gly Ala Ser His Tyr Ser Thr Asp
   275 280 285
   Val Thr Asn Pro Tyr Gln Ala Asp Cys Gly His Val Tyr Cys Tyr Val
   290 295 300
   Cys Leu Val Thr Lys Leu Ala Gln Gly Asp Gly Asp Gly Trp Asn Cys
   305 310 315 320
   Tyr Arg Cys Ala Lys Gln Val Gln Lys Met Lys Pro Trp Val Asp Val
   325 330 335
   Asp Glu Ala Ala Val Val Gly Ala Ala Glu Met His Glu Lys Val Asp
   340 345 350
   Val Ile Glu His Ala Glu Asp Asn Glu Gln Glu Glu Glu Glu Phe Asp
   355 360 365
   Asp Asp Asp Glu Asp Ser Asn Phe Gln Leu Met Lys Asp
   370 375 380
<210> 3
   <211> 431
   <212> PRT
   <213> Yarrowia lipolytica CLIB122 (GenBank Accession No. CAG78565)
<220>
   <221> MISC_FEATURE
   <222> (1)..(431)
   <223> YlPex3p
<400> 3
   Met Asp Phe Phe Arg Arg His Gln Lys Lys Val Leu Ala Leu Val Gly
   1 5 10 15
   Val Ala Leu Ser Ser Tyr Leu Phe Ile Asp Tyr Val Lys Lys Lys Phe
   20 25 30
   Phe Glu Ile Gln Gly Arg Leu Ser Ser Glu Arg Thr Ala Lys Gln Asn
   35 40 45
   Leu Arg Arg Arg Phe Glu Gln Asn Gln Gln Asp Ala Asp Phe Thr Ile
   50 55 60
   Met Ala Leu Leu Ser Ser Leu Thr Thr Pro Val Met Glu Arg Tyr Pro
   65                  70                  75                  80
   Val Asp Gln Ile Lys Ala Glu Leu Gln Ser Lys Arg Arg Pro Thr Asp
                   85                  90                  95
   Arg Val Leu Ala Leu Glu Ser Ser Thr Ser Ser Ser Ala Thr Ala Gln
               100                 105                 110
   Thr Val Pro Thr Met Thr Ser Gly Ala Thr Glu Glu Gly Glu Lys Ser
           115                 120                 125
   Lys Thr Gln Leu Trp Gln Asp Leu Lys Arg Thr Thr Ile Ser Arg Ala
       130                 135                 140
   Phe Ser Leu Val Tyr Ala Asp Ala Leu Leu Ile Phe Phe Thr Arg Leu
   145                 150                 155                 160
   Gln Leu Asn Ile Leu Gly Arg Arg Asn Tyr Val Asn Ser Val Val Ala
                   165                 170                 175
   Leu Ala Gln Gln Gly Arg Glu Gly Asn Ala Glu Gly Arg Val Ala Pro
               180                 185                 190
   Ser Phe Gly Asp Leu Ala Asp Met Gly Tyr Phe Gly Asp Leu Ser Gly
           195                 200                 205
   Ser Ser Ser Phe Gly Glu Thr Ile Val Asp Pro Asp Leu Asp Glu Gln
       210                 215                 220
   Tyr Leu Thr Phe Ser Trp Trp Leu Leu Asn Glu Gly Trp Val Ser Leu
   225                 230                 235                 240
   Ser Glu Arg Val Glu Glu Ala Val Arg Arg Val Trp Asp Pro Val Ser
                   245                 250                 255
   Pro Lys Ala Glu Leu Gly Phe Asp Glu Leu Ser Glu Leu Ile Gly Arg
               260                 265                 270
   Thr Gln Met Leu Ile Asp Arg Pro Leu Asn Pro Ser Ser Pro Leu Asn
           275                 280                 285
   Phe Leu Ser Gln Leu Leu Pro Pro Arg Glu Gln Glu Glu Tyr Val Leu
       290                 295                 300
   Ala Gln Asn Pro Ser Asp Thr Ala Ala Pro Ile Val Gly Pro Thr Leu
   305 310 315 320
   Arg Arg Leu Leu Asp Glu Thr Ala Asp Phe Ile Glu Ser Pro Asn Ala
   325 330 335
   Ala Glu Val Ile Glu Arg Leu Val His Ser Gly Leu Ser Val Phe Met
   340 345 350
   Asp Lys Leu Ala Val Thr Phe Gly Ala Thr Pro Ala Asp Ser Gly Ser
   355 360 365
   Pro Tyr Pro Val Val Leu Pro Thr Ala Lys Val Lys Leu Pro Ser Ile
   370 375 380
   Leu Ala Asn Met Ala Arg Gln Ala Gly Gly Met Ala Gln Gly Ser Pro
   385 390 395 400
   Gly Val Glu Asn Glu Tyr Ile Asp Val Met Asn Gln Val Gln Glu Leu
   405 410 415
   Thr Ser Phe Ser Ala Val Val Tyr Ser Ser Phe Asp Trp Ala Leu
   420 425 430
<210> 4
   <211> 395
   <212> PRT
   <213> Yarrowia lipolytica CLIB122 (GenBank Accession No. CAG83356)
<220>
   <221> MISC_FEATURE
   <222> (1))..(395)
   <223> YlPex3Bp
<400> 4
   Met Leu Gln Ser Leu Asn Arg Asn Lys Lys Arg Leu Ala Val Ser Thr
   1 5 10 15
   Gly Leu Ile Ala Val Ala Tyr Val Val Ile Ser Tyr Thr Thr Lys Arg
   20 25 30
   Leu Ile Glu Lys Gln Glu Gln Lys Leu Glu Glu Glu Arg Ala Lys Glu
   35 40 45
   Arg Leu Lys Gln Leu Phe Ala Gln Thr Gln Asn Glu Ala Ala Phe His
   50 55 60
   Thr Ala Ser Val Leu Pro Gln Leu Cys Glu Gln Ile Met Glu Phe Val
   65 70 75 80
   Ala Val Glu Lys Ile Ala Glu Gln Leu Gln Asn Met Arg Ala Glu Lys
   85 90 95
   Arg Lys Lys Gln Asn Met Asp Asp Asp Lys His Ser Val Leu Ser Leu
   100 105 110
   Gly Thr Glu Thr Thr Ala Ser Met Ala Asp Gly Gln Lys Met Ser Lys
   115 120 125
   Ile Gln Leu Trp Asp Glu Leu Lys Ile Glu Ser Leu Thr Arg Ile Val
   130 135 140
   Thr Leu Ile Tyr Cys Val Ser Leu Leu Asn Tyr Leu Ile Arg Leu Gln
   145 150 155 160
   Thr Asn Ile Val Gly Arg Lys Arg Tyr Gln Asn Glu Ala Gly Pro Ala
   165 170 175
   Gly Ala Thr Tyr Asp Met Ser Leu Glu Gln Cys Tyr Thr Trp Leu Leu
   180 185 190
   Thr Arg Gly Trp Lys Ser Val Val Asp Asn Val Arg Arg Ser Val Gln
   195 200 205
   Gln Val Phe Thr Gly Val Asn Pro Arg Gln Asn Leu Ser Leu Asp Glu
   210 215 220
   Phe Ala Thr Leu Leu Lys Arg Val Gln Thr Leu Val Asn Ser Pro Pro
   225 230 235 240
   Tyr Ser Thr Thr Pro Asn Thr Phe Leu Thr Ser Leu Leu Pro Pro Arg
   245 250 255
   Glu Leu Glu Gln Leu Arg Leu Glu Lys Glu Lys Gln Ser Leu Ser Pro
   260 265 270
   Asn Tyr Thr Tyr Gly Ser Pro Leu Lys Asp Leu Val Phe Glu Ser Ala
   275 280 285
   Gln His Ile Gln Ser Pro Gln Gly Met Ser Ser Phe Arg Ala Ile Ile
   290 295 300
   Asp Gln Ser Phe Lys Val Phe Leu Glu Lys Val Asn Glu Ser Gln Tyr
   305 310 315 320
   Val Asn Pro Pro Ser Thr Gly Gly Lys Arg Ile Ala Val Gly Ala Leu
                   325                 330                 335
   Gln Pro Pro Ile Ile Ser Gly Gly Pro Lys Lys Val Lys Leu Ala Ser
               340                 345                 350
   Leu Leu Ser Val Ala Thr Arg Gln Ser Ser Val Ile Ser His Ala Gln
           355                 360                 365
   Pro Asn Pro Tyr Val Asp Ala Ile Asn Ser Val Ala Glu Tyr Asn Gly
       370                 375                 380
   Leu Cys Ala Val Ile Tyr Ser Ser Phe Glu Gln
   385                 390                 395
<210> 5
   <211> 153
   <212> PRT
   <213> Yarrowia lipolytica CLIB122 (GenBank Accession No. CAG79130)
<220>
   <221> MISC_FEATURE
   <222> (1)..(153)
   <223> YlPex4p
<400> 5
   Met Ala Ser Gln Lys Arg Leu Ile Lys Glu Leu Ala Ala Tyr Lys Lys
   1               5                   10                  15
   Asp Pro Asn Pro Cys Leu Ala Ser Leu Thr Ala Asp Gly Asp Ser Leu
               20                  25                  30
   Tyr Lys Trp Thr Ala Val Met Arg Gly Thr Glu Gly Thr Ala Tyr Glu
           35                  40                  45
   Asn Gly Leu Trp Gln Val Glu Ile Asn Ile Pro Glu Asn Tyr Pro Leu
       50                  55                  60
   Gln Pro Pro Thr Met Phe Phe Arg Thr Lys Ile Cys His Pro Asn Ile
   65                  70                  75                  80
   His Phe Glu Thr Gly Glu Val Cys Ile Asp Val Leu Lys Thr Gln Trp
                   85                  90                  95
   Ser Pro Ala Trp Thr Ile Ser Ser Ala Cys Thr Ala Val Ser Ala Met
               100                 105                 110
   Leu Ser Leu Pro Glu Pro Asp Ser Pro Leu Asn Ile Asp Ala Ala Asn
   115 120 125
   Leu Val Arg Cys Gly Asp Glu Ser Ala Met Glu Gly Leu Val Arg Tyr
   130 135 140
   Tyr Val Asn Lys Tyr Ala Ser Gly Asn
   145 150
<210> 6
   <211> 598
   <212> PRT
   <213> Yarrowia lipolytica CLIB122 (GenBank Accession No. CAG78803)
<220>
   <221> MISC_FEATURE
   <222> (1)..(598)
   <223> YlPex5p
<400> 6
   Met Ser Phe Met Arg Gly Gly Ser Glu Cys Ser Thr Gly Arg Asn Pro
   1 5 10 15
   Leu Ser Gln Phe Thr Lys His Thr Ala Glu Asp Arg Ser Leu Gln His
   20 25 30
   Asp Arg Val Ala Gly Pro Ser Gly Gly Arg Val Gly Gly Met Arg Ser
   35 40 45
   Asn Thr Gly Glu Met Ser Gln Gln Asp Arg Glu Met Met Ala Arg Phe
   50 55 60
   Gly Ala Ala Gly Pro Glu Gln Ser Ser Phe Asn Tyr Glu Gln Met Arg
   65 70 75 80
   His Glu Leu His Asn Met Gly Ala Gln Gly Gly Gln Ile Pro Gln Val
   85 90 95
   Pro Ser Gln Gln Gly Ala Ala Asn Gly Gly Gln Trp Ala Arg Asp Phe
   100 105 110
   Gly Gly Gln Gln Thr Ala Pro Gly Ala Ala Pro Gln Asp Ala Lys Asn
   115 120 125
   Trp Asn Ala Glu Phe Gln Arg Gly Gly Ser Pro Ala Glu Ala Met Gln
   130 135 140
   Gln Gln Gly Pro Gly Pro Met Gln Gly Gly Met Gly Met Gly Gly Met
   145                 150                 155                 160
   Pro Met Tyr Gly Met Ala Arg Pro Met Tyr Ser Gly Met Ser Ala Asn
                   165                 170                 175
   Met Ala Pro Gln Phe Gln Pro Gln Gln Ala Asn Ala Arg Val Val Glu
               180                 185                 190
   Leu Asp Glu Gln Asn Trp Glu Glu Gln Phe Lys Gln Met Asp Ser Ala
           195                 200                 205
   Val Gly Lys Gly Lys Glu Val Glu Glu Gln Thr Ala Glu Thr Ala Thr
       210                 215                 220
   Ala Thr Glu Thr Val Thr Glu Thr Glu Thr Thr Thr Glu Asp Lys Pro
   225                 230                 235                 240
   Met Asp Ile Lys Asn Met Asp Phe Glu Asn Ile Trp Lys Asn Leu Gln
                   245                 250                 255
   Val Asn Val Leu Asp Asn Met Asp Glu Trp Leu Glu Glu Thr Asn Ser
               260                 265                 270
   Pro Ala Trp Glu Arg Asp Phe His Glu Tyr Thr His Asn Arg Pro Glu
           275                 280                 285
   Phe Ala Asp Tyr Gln Phe Glu Glu Asn Asn Gln Phe Met Glu His Pro
       290                 295                 300
   Asp Pro Phe Lys Ile Gly Val Glu Leu Met Glu Thr Gly Gly Arg Leu
   305                 310                 315                 320
   Ser Glu Ala Ala Leu Ala Phe Glu Ala Ala Val Gln Lys Asn Thr Glu
                   325                 330                 335
   His Ala Glu Ala Trp Gly Arg Leu Gly Ala Cys Gln Ala Gln Asn Glu
               340                 345                 350
   Lys Glu Asp Pro Ala Ile Arg Ala Leu Glu Arg Cys Ile Lys Leu Glu
           355                 360                 365
   Pro Gly Asn Leu Ser Ala Leu Met Asn Leu Ser Val Ser Tyr Thr Asn
       370                 375                 380
   Glu Gly Tyr Glu Asn Ala Ala Tyr Ala Thr Leu Glu Arg Trp Leu Ala
   385                 390                 395                 400
   Thr Lys Tyr Pro Glu Val Val Asp Gln Ala Arg Asn Gln Glu Pro Arg
                   405                 410                 415
   Leu Gly Asn Glu Asp Lys Phe Gln Leu His Ser Arg Val Thr Glu Leu
               420                 425                 430
   Phe Ile Arg Ala Ala Gln Leu Ser Pro Asp Gly Ala Asn Ile Asp Ala
           435                 440                 445
   Asp Val Gln Val Gly Leu Gly Val Leu Phe Tyr Gly Asn Glu Glu Tyr
       450                 455                 460
   Asp Lys Ala Ile Asp Cys Phe Asn Ala Ala Ile Ala Val Arg Pro Asp
   465                 470                 475                 480
   Asp Ala Leu Leu Trp Asn Arg Leu Gly Ala Thr Leu Ala Asn Ser His
                   485                 490                 495
   Arg Ser Glu Glu Ala Ile Asp Ala Tyr Tyr Lys Ala Leu Glu Leu Arg
               500                 505                 510
   Pro Ser Phe Val Arg Ala Arg Tyr Asn Leu Gly Val Ser Cys Ile Asn
           515                 520                 525
   Ile Gly Cys Tyr Lys Glu Ala Ala Gln Tyr Leu Leu Gly Ala Leu Ser
       530                 535                 540
   Met His Lys Val Glu Gly Val Gln Asp Asp Val Leu Ala Asn Gln Ser
   545                 550                 555                 560
   Thr Asn Leu Tyr Asp Thr Leu Lys Arg Val Phe Leu Gly Met Asp Arg
                   565                 570                 575
   Arg Asp Leu Val Ala Lys Val Gly Asn Gly Met Asp Val Asn Gln Phe
               580                 585                 590
   Arg Asn Glu Phe Glu Phe
           595
<210> 7
   <211> 1024
   <212> PRT
   <213> Yarrowia lipolytica CLIB122 (GenBank Accession No. CAG82306)
<220>
   <221> MISC_FEATURE
   <222> (1)..(1024)
   <223> YlPex6p
<400> 7
   Met Pro Ser Ile Ser His Lys Pro Ile Thr Ala Lys Leu Val Ala Ala
   1               5                   10                  15
   Pro Asp Ala Thr Lys Leu Glu Leu Ser Ser Tyr Leu Tyr Gln Gln Leu
               20                  25                  30
   Phe Ser Asp Lys Pro Ala Glu Pro Tyr Val Ala Phe Glu Ala Pro Gly
           35                  40                  45
   Ile Lys Trp Ala Leu Tyr Pro Ala Ser Glu Asp Arg Ser Leu Pro Gln
       50                  55                  60
   Tyr Thr Cys Lys Ala Asp Ile Arg His Val Ala Gly Ser Leu Lys Lys
   65                  70                  75                  80
   Phe Met Pro Val Val Leu Lys Arg Val Asn Pro Val Thr Ile Glu His
                   85                  90                  95
   Ala Ile Val Thr Val Pro Ala Ser Gln Tyr Glu Thr Leu Asn Thr Pro
               100                 105                 110
   Glu Gln Val Leu Lys Ala Leu Glu Pro Gln Leu Asp Lys Asp Arg Pro
           115                 120                 125
   Val Ile Arg Gln Gly Asp Val Leu Leu Asn Gly Cys Arg Val Arg Leu
       130                 135                 140
   Cys Glu Pro Val Asn Gln Gly Lys Val Val Lys Gly Thr Thr Lys Leu
   145                 150                 155                 160
   Thr Val Ala Lys Glu Gln Glu Thr Ile Gln Pro Ala Asp Glu Ala Ala
                   165                 170                 175
   Asp Val Ala Phe Asp Ile Ala Glu Phe Leu Asp Phe Asp Thr Ser Val
               180                 185                 190
   Ala Lys Thr Arg Glu Ser Thr Asn Leu Gln Val Ala Pro Leu Glu Gly
           195                 200                 205
   Ala Ile Pro Thr Pro Leu Ser Asp Arg Phe Asp Asp Cys Glu Ser Arg
   210 215 220
   Gly Phe Val Lys Ser Glu Thr Met Ser Lys Leu Gly Val Phe Ser Gly
   225 230 235 240
   Asp Ile Val Ser Ile Lys Thr Lys Asn Gly Ala Glu Arg Val Leu Arg
   245 250 255
   Leu Phe Ala Tyr Pro Glu Pro Asn Thr Val Lys Tyr Asp Val Val Tyr
   260 265 270
   Val Ser Pro Ile Leu Tyr His Asn Ile Gly Asp Lys Glu Ile Glu Val
   275 280 285
   Thr Pro Asn Gly Glu Thr His Lys Ser Val Gly Glu Ala Leu Asp Ser
   290 295 300
   Val Leu Glu Ala Ala Glu Glu Val Lys Leu Ala Arg Val Leu Gly Pro
   305 310 315 320
   Thr Thr Thr Asp Arg Thr Phe Gln Thr Ala Tyr His Ala Gly Leu Gln
   325 330 335
   Ala Tyr Phe Lys Pro Val Lys Arg Ala Val Arg Val Gly Asp Leu Ile
   340 345 350
   Pro Ile Pro Phe Asp Ser Ile Leu Ala Arg Thr Ile Gly Glu Asp Pro
   355 360 365
   Glu Met Ser His Ile Pro Leu Glu Ala Leu Ala Val Lys Pro Asp Ser
   370 375 380
   Val Ala Trp Phe Gln Val Thr Ser Leu Asn Gly Ser Glu Asp Pro Ala
   385 390 395 400
   Ser Lys Gln Tyr Leu Val Asp Ser Ser Gln Thr Lys Leu Ile Glu Gly
   405 410 415
   Gly Thr Thr Ser Ser Ala Val Ile Pro Thr Ser Val Pro Trp Arg Glu
   420 425 430
   Tyr Leu Gly Leu Asp Thr Leu Pro Lys Phe Gly Ser Glu Phe Ala Tyr
   435 440 445
   Ala Asp Lys Ile Arg Asn Leu Val Gln Ile Ser Thr Ser Ala Leu Ser
   450 455 460
   His Ala Lys Leu Asn Thr Ser Val Leu Leu His Ser Ala Lys Arg Gly
   465 470 475 480
   Val Gly Lys Ser Thr Val Leu Arg Ser Val Ala Ala Gln Cys Gly Ile
   485 490 495
   Ser Val Phe Glu Ile Ser Cys Phe Gly Leu Ile Gly Asp Asn Glu Ala
   500 505 510
   Gln Thr Leu Gly Thr Leu Arg Ala Lys Leu Asp Arg Ala Tyr Gly Cys
   515 520 525
   Ser Pro Cys Val Val Val Leu Gln His Leu Glu Ser Ile Ala Lys Lys
   530 535 540
   Ser Asp Gln Asp Gly Lys Asp Glu Gly Ile Val Ser Lys Leu Val Asp
   545 550 555 560
   Val Leu Ala Asp Tyr Ser Gly His Gly Val Leu Leu Ala Ala Thr Ser
   565 570 575
   Asn Asp Pro Asp Lys Ile Ser Glu Ala Ile Arg Ser Arg Phe Gln Phe
   580 585 590
   Glu Ile Glu Ile Gly Val Pro Ser Glu Pro Gln Arg Arg Gln Ile Phe
   595 600 605
   Ser His Leu Thr Lys Ser Gly Pro Gly Gly Asp Ser Ile Arg Asn Ala
   610 615 620
   Pro Ile Ser Leu Arg Ser Asp Val Ser Val Glu Asn Leu Ala Leu Gln
   625 630 635 640
   Ser Ala Gly Leu Thr Pro Pro Asp Leu Thr Ala Ile Val Gln Thr Thr
   645 650 655
   Arg Leu Arg Ala Ile Asp Arg Leu Asn Lys Leu Thr Lys Asp Ser Asp
   660 665 670
   Thr Thr Leu Asp Asp Leu Leu Thr Leu Ser His Gly Thr Leu Gln Leu
   675 680 685
   Thr Pro Ser Asp Phe Asp Asp Ala Ile Ala Asp Ala Arg Gln Lys Tyr
   690 695 700
   Ser Asp Ser Ile Gly Ala Pro Arg Ile Pro Asn Val Gly Trp Asp Asp
   705 710 715 720
   Val Gly Gly Met Glu Gly Val Lys Lys Asp Ile Leu Asp Thr Ile Glu
   725 730 735
   Thr Pro Leu Lys Tyr Pro His Trp Phe Ser Asp Gly Val Lys Lys Arg
   740 745 750
   Ser Gly Ile Leu Phe Tyr Gly Pro Pro Gly Thr Gly Lys Thr Leu Leu
   755 760 765
   Ala Lys Ala Ile Ala Thr Thr Phe Ser Leu Asn Phe Phe Ser Val Lys
   770 775 780
   Gly Pro Glu Leu Leu Asn Met Tyr Ile Gly Glu Ser Glu Ala Asn Val
   785 790 795 800
   Arg Arg Val Phe Gln Lys Ala Arg Asp Ala Lys Pro Cys Val Val Phe
   805 810 815
   Phe Asp Glu Leu Asp Ser Val Ala Pro Gln Arg Gly Asn Gln Gly Asp
   820 825 830
   Ser Gly Gly Val Met Asp Arg Ile Val Ser Gln Leu Leu Ala Glu Leu
   835 840 845
   Asp Gly Met Ser Thr Ala Gly Gly Glu Gly Val Phe Val Val Gly Ala
   850 855 860
   Thr Asn Arg Pro Asp Leu Leu Asp Glu Ala Leu Leu Arg Pro Gly Arg
   865 870 875 880
   Phe Asp Lys Met Leu Tyr Leu Gly Ile Ser Asp Thr His Glu Lys Gln
   885 890 895
   Gln Thr Ile Met Glu Ala Leu Thr Arg Lys Phe Arg Leu Ala Ala Asp
   900 905 910
   Val Ser Leu Glu Ala Ile Ser Lys Arg Cys Pro Phe Thr Phe Thr Gly
   915 920 925
   Ala Asp Phe Tyr Ala Leu Cys Ser Asp Ala Met Leu Asn Ala Met Thr
   930 935 940
   Arg Thr Ala Asn Glu Val Asp Ala Lys Ile Lys Leu Leu Asn Lys Asn
   945 950 955 960
   Arg Glu Glu Ala Gly Glu Glu Pro Val Ser Ile Arg Trp Trp Phe Asp
   965 970 975
   His Glu Ala Thr Lys Ser Asp Ile Glu Val Glu Val Ala Gln Gln Asp
   980 985 990
   Phe Glu Lys Ala Lys Asp Glu Leu Ser Pro Ser Val Ser Ala Glu Glu
   995 1000 1005
   Leu Gln His Tyr Leu Lys Leu Arg Gln Gln Phe Glu Gly Gly Lys
   1010 1015 1020
   Lys
<210> 8
   <211> 356
   <212> PRT
   <213> Yarrowia lipolytica CLIB122 (GenBank Accession No. CAG78389)
<220>
   <221> MISC_FEATURE
   <222> (1)..(356)
   <223> YlPex7p
<400> 8
   Met Leu Gly Phe Lys Thr Gln Gly Phe Asn Gly Tyr Ala Ala Asn Tyr
   1 5 10 15
   Ser Pro Phe Phe Asn Asp Lys Ile Ala Val Gly Thr Ala Ala Asn Tyr
   20 25 30
   Gly Leu Val Gly Asn Gly Lys Leu Phe Ile Leu Gly Ile Ser Pro Glu
   35 40 45
   Gly Arg Met Val Cys Glu Gly Gln Phe Asp Thr Gln Asp Gly Ile Phe
   50 55 60
   Asp Val Ala Trp Ser Glu Gln His Glu Asn His Val Ala Thr Ala Cys
   65 70 75 80
   Gly Asp Gly Ser Val Lys Leu Phe Asp Ile Lys Ala Gly Ala Phe Pro
   85 90 95
   Leu Val Ser Phe Lys Glu His Thr Arg Glu Val Phe Ser Val Asn Trp
               100                 105                 110
   Asn Met Ala Asn Lys Ala Leu Phe Cys Thr Ser Ser Trp Asp Ser Thr
           115                 120                 125
   Ile Lys Ile Trp Thr Pro Glu Arg Thr Asn Ser Ile Met Thr Leu Gly
       130                 135                 140
   Gln Pro Ala Pro Ala Gln Gly Thr Asn Ala Ser Ala His Ile Gly Arg
   145                 150                 155                 160
   Gln Thr Ala Pro Asn Gln Ala Ala Ala Gln Glu Cys Ile Tyr Ser Ala
                   165                 170                 175
   Lys Phe Ser Pro His Thr Asp Ser Ile Ile Ala Ser Ala His Ser Thr
               180                 185                 190
   Gly Met Val Lys Val Trp Asp Thr Arg Ala Pro Gln Pro Leu Gln Gln
           195                 200                 205
   Gln Phe Ser Thr Gln Gln Thr Glu Ser Gly Gly Pro Pro Glu Val Leu
       210                 215                 220
   Ser Leu Asp Trp Asn Lys Tyr Arg Pro Thr Val Ile Ala Thr Gly Gly
   225                 230                 235                 240
   Val Asp Arg Ser Val Gln Val Tyr Asp Ile Arg Met Thr Gln Pro Ala
                   245                 250                 255
   Ala Asn Gln Pro Val Gln Pro Leu Ser Leu Ile Leu Gly His Arg Leu
               260                 265                 270
   Pro Val Arg Gly Val Ser Trp Ser Pro His His Ala Asp Leu Leu Leu
           275                 280                 285
   Ser Cys Ser Tyr Asp Met Thr Ala Arg Val Trp Arg Asp Ala Ser Thr
       290                 295                 300
   Gly Gly Asn Tyr Leu Ala Arg Gln Arg Gly Gly Thr Glu Val Lys Cys
   305                 310                 315                 320
   Met Asp Arg His Thr Glu Phe Val Ile Gly Gly Asp Trp Ser Leu Trp
                   325                 330                 335
   Gly Asp Pro Gly Trp Ile Thr Thr Val Gly Trp Asp Gln Met Val Tyr
   340 345 350
   Val Trp His Ala
   355
<210> 9
   <211> 671
   <212> PRT
   <213> Yarrowia lipolytica CLIB122 (GenBank Accession No. CAG80447)
<220>
   <221> MISC_FEATURE
   <222> (1)..(671)
   <223> YlPex8p
<400> 9
   Met Asn Lys Tyr Leu Val Pro Pro Pro Gln Ala Asn Arg Thr Val Thr
   1 5 10 15
   Asn Leu Asp Leu Leu Ile Asn Asn Leu Arg Gly Ser Ser Thr Pro Gly
   20 25 30
   Ala Ala Glu Val Asp Thr Arg Asp Ile Leu Gln Arg Ile Val Phe Ile
   35 40 45
   Leu Pro Thr Ile Lys Asn Pro Leu Asn Leu Asp Leu Val Ile Lys Glu
   50 55 60
   Ile Ile Asn Ser Pro Arg Leu Leu Pro Pro Leu Ile Asp Leu His Asp
   65 70 75 80
   Tyr Gln Gln Leu Thr Asp Ala Phe Arg Ala Thr Ile Lys Arg Lys Ala
   85 90 95
   Leu Val Thr Asp Pro Thr Ile Ser Phe Glu Ala Trp Leu Glu Thr Cys
   100 105 110
   Phe Gln Val Ile Thr Arg Phe Ala Gly Pro Gly Trp Lys Lys Leu Pro
   115 120 125
   Leu Leu Ala Gly Leu Ile Leu Ala Asp Tyr Asp Ile Ser Ala Asp Gly
   130 135 140
   Pro Thr Leu Glu Arg Lys Pro Gly Phe Pro Ser Lys Leu Lys His Leu
   145 150 155 160
   Leu Lys Arg Glu Phe Val Thr Thr Phe Asp Gln Cys Leu Ser Ile Asp
   165 170 175
   Thr Arg Asn Arg Ser Asp Ala Thr Lys Trp Val Pro Val Leu Ala Cys
   180 185 190
   Ile Ser Ile Ala Gln Val Tyr Ser Leu Leu Gly Asp Val Ala Ile Asn
   195 200 205
   Tyr Arg Arg Phe Leu Gln Val Gly Leu Asp Leu Ile Phe Ser Asn Tyr
   210 215 220
   Gly Leu Glu Met Gly Thr Ala Leu Ala Arg Leu His Ala Glu Ser Gly
   225 230 235 240
   Gly Asp Ala Thr Thr Ala Gly Gly Leu Ile Gly Lys Lys Leu Lys Glu
   245 250 255
   Pro Val Val Ala Leu Leu Asn Thr Phe Ala His Ile Ala Ser Ser Cys
   260 265 270
   Ile Val His Val Asp Ile Asp Tyr Ile Asp Arg Ile Gln Asn Lys Ile
   275 280 285
   Ile Leu Val Cys Glu Asn Gln Ala Glu Thr Trp Arg Ile Leu Thr Ile
   290 295 300
   Glu Ser Pro Thr Val Met His His Gln Glu Ser Val Gln Tyr Leu Lys
   305 310 315 320
   Trp Glu Leu Phe Thr Leu Cys Ile Ile Met Gln Gly Ile Ala Asn Met
   325 330 335
   Leu Leu Thr Gln Lys Met Asn Gln Phe Met Tyr Leu Gln Leu Ala Tyr
   340 345 350
   Lys Gln Leu Gln Ala Leu His Ser Ile Tyr Phe Ile Val Asp Gln Met
   355 360 365
   Gly Ser Gln Phe Ala Ala Tyr Asp Tyr Val Phe Phe Ser Ala Ile Asp
   370 375 380
   Val Leu Leu Ser Glu Tyr Ala Pro Tyr Ile Lys Asn Arg Gly Thr Ile
   385 390 395 400
   Pro Pro Asn Lys Glu Phe Val Ala Glu Arg Leu Ala Ala Asn Leu Ala
   405 410 415
   Gly Thr Ser Asn Val Gly Ser His Leu Pro Ile Asp Arg Ser Arg Val
   420 425 430
   Leu Phe Ala Leu Asn Tyr Tyr Glu Gln Leu Val Thr Val Cys His Asp
   435 440 445
   Ser Cys Val Glu Thr Ile Ile Tyr Pro Met Ala Arg Ser Phe Leu Tyr
   450 455 460
   Pro Thr Ser Asp Ile Gln Gln Leu Lys Pro Leu Val Glu Ala Ala His
   465 470 475 480
   Ser Val Ile Leu Ala Gly Leu Ala Val Pro Thr Asn Ala Val Val Asn
   485 490 495
   Ala Lys Leu Ile Pro Glu Tyr Met Gly Gly Val Leu Pro Leu Phe Pro
   500 505 510
   Gly Val Phe Ser Trp Asn Gln Phe Val Leu Ala Ile Gln Ser Ile Val
   515 520 525
   Asn Thr Val Ser Pro Pro Ser Glu Val Phe Lys Thr Asn Gln Lys Leu
   530 535 540
   Phe Arg Leu Val Leu Asp Ser Leu Met Lys Lys Cys Arg Asp Thr Pro
   545 550 555 560
   Val Gly Ile Pro Val Pro His Ser Val Thr Val Ser Gln Glu Gln Glu
   565 570 575
   Asp Ile Pro Pro Thr Gln Arg Ala Val Val Met Leu Ala Leu Ile Asn
   580 585 590
   Ser Leu Pro Tyr Val Asp Ile Arg Ser Phe Glu Leu Trp Leu Gln Glu
   595 600 605
   Thr Trp Asn Met Ile Glu Ala Thr Pro Met Leu Ala Glu Asn Ala Pro
   610 615 620
   Asn Lys Glu Leu Ala His Ala Glu His Glu Phe Leu Val Leu Glu Met
   625 630 635 640
   Trp Lys Met Ile Ser Gly Asn Ile Asp Gln Arg Leu Asn Asp Val Ala
   645 650 655
   Ile Arg Trp Trp Tyr Lys Lys Asn Ala Arg Val His Gly Thr Leu
               660                     665                 670
<210> 10
   <211> 377
   <212> PRT
   <213> Yarrowia lipolytica CLIB122 (GenBank Accession No. CAG81606)
<220>
   <221> MISC_FEATURE
   <222> (1)..(377)
   <223> YlPex10p
<400> 10
   Met Trp Gly Ser Ser His Ala Phe Ala Gly Glu Ser Asp Leu Thr Leu
   1               5                   10                  15
   Gln Leu His Thr Arg Ser Asn Met Ser Asp Asn Thr Thr Ile Lys Lys
               20                  25                  30
   Pro Ile Arg Pro Lys Pro Ile Arg Thr Glu Arg Leu Pro Tyr Ala Gly
           35                  40                  45
   Ala Ala Glu Ile Ile Arg Ala Asn Gln Lys Asp His Tyr Phe Glu Ser
       50                  55                  60
   Val Leu Glu Gln His Leu Val Thr Phe Leu Gln Lys Trp Lys Gly Val
   65                  70                  75                  80
   Arg Phe Ile His Gln Tyr Lys Glu Glu Leu Glu Thr Ala Ser Lys Phe
                   85                  90                  95
   Ala Tyr Leu Gly Leu Cys Thr Leu Val Gly Ser Lys Thr Leu Gly Glu
               100                 105                 110
   Glu Tyr Thr Asn Leu Met Tyr Thr Ile Arg Asp Arg Thr Ala Leu Pro
           115                 120                 125
   Gly Val Val Arg Arg Phe Gly Tyr Val Leu Ser Asn Thr Leu Phe Pro
       130                 135                 140
   Tyr Leu Phe Val Arg Tyr Met Gly Lys Leu Arg Ala Lys Leu Met Arg
   145                 150                 155                 160
   Glu Tyr Pro His Leu Val Glu Tyr Asp Glu Asp Glu Pro Val Pro Ser
                   165                 170                 175
   Pro Glu Thr Trp Lys Glu Arg Val Ile Lys Thr Phe Val Asn Lys Phe
               180                 185                 190
   Asp Lys Phe Thr Ala Leu Glu Gly Phe Thr Ala Ile His Leu Ala Ile
           195                 200                 205
   Phe Tyr Val Tyr Gly Ser Tyr Tyr Gln Leu Ser Lys Arg Ile Trp Gly
       210                 215                 220
   Met Arg Tyr Val Phe Gly His Arg Leu Asp Lys Asn Glu Pro Arg Ile
   225                 230                 235                 240
   Gly Tyr Glu Met Leu Gly Leu Leu Ile Phe Ala Arg Phe Ala Thr Ser
                   245                 250                 255
   Phe Val Gln Thr Gly Arg Glu Tyr Leu Gly Ala Leu Leu Glu Lys Ser
               260                 265                 270
   Val Glu Lys Glu Ala Gly Glu Lys Glu Asp Glu Lys Glu Ala Val Val
           275                 280                 285
   Pro Lys Lys Lys Ser Ser Ile Pro Phe Ile Glu Asp Thr Glu Gly Glu
       290                 295                 300
   Thr Glu Asp Lys Ile Asp Leu Glu Asp Pro Arg Gln Leu Lys Phe Ile
   305                 310                 315                 320
   Pro Glu Ala Ser Arg Ala Cys Thr Leu Cys Leu Ser Tyr Ile Ser Ala
                   325                 330                 335
   Pro Ala Cys Thr Pro Cys Gly His Phe Phe Cys Trp Asp Cys Ile Ser
               340                 345                 350
   Glu Trp Val Arg Glu Lys Pro Glu Cys Pro Leu Cys Arg Gln Gly Val
           355                 360                 365
   Arg Glu Gln Asn Leu Leu Pro Ile Arg
       370                 375
<210> 11
   <211> 408
   <212> PRT
   <213> Yarrowia lipolytica CLIB122 (GenBank Accession No. CAG81532)
<220>
   <221> MISC_FEATURE
   <222> (1)..(408)
   <223> YlPex12p
<400> 11
   Met Asp Tyr Phe Ser Ser Leu Asn Ala Ser Gln Leu Asp Pro Asp Val
   1               5                   10                  15
   Pro Thr Leu Phe Glu Leu Leu Ser Ala Lys Gln Leu Glu Gly Leu Ile
               20                  25                  30
   Ala Pro Ser Val Arg Tyr Ile Leu Ala Phe Tyr Ala Gln Arg His Pro
           35                  40                  45
   Arg Tyr Leu Leu Arg Ile Val Asn Arg Tyr Asp Glu Leu Tyr Ala Leu
       50                  55                  60
   Phe Met Gly Leu Val Glu Tyr Tyr Asn Leu Lys Thr Trp Asn Ala Ser
   65                  70                  75                  80
   Phe Thr Glu Lys Phe Tyr Gly Leu Lys Arg Thr Gln Ile Leu Thr Asn
                   85                  90                  95
   Pro Ala Leu Arg Thr Arg Gln Ala Val Pro Asp Leu Val Glu Ala Glu
               100                 105                 110
   Lys Arg Leu Ser Lys Lys Lys Ile Trp Gly Ser Leu Phe Phe Leu Ile
           115                 120                 125
   Val Val Pro Tyr Val Lys Glu Lys Leu Asp Ala Arg Tyr Glu Arg Leu
       130                 135                 140
   Lys Gly Arg Tyr Leu Ala Arg Asp Ile Asn Glu Glu Arg Ile Glu Ile
   145                 150                 155                 160
   Lys Arg Thr Gly Thr Ala Gln Gln Ile Ala Val Phe Glu Phe Asp Tyr
                   165                 170                 175
   Trp Leu Leu Lys Leu Tyr Pro Ile Val Thr Met Gly Cys Thr Thr Ala
               180                 185                 190
   Thr Leu Ala Phe His Met Leu Phe Leu Phe Ser Val Thr Arg Ala Tyr
           195                 200                 205
   Ser Ile Asp Asp Phe Leu Leu Asn Ile Gln Phe Ser Arg Met Thr Arg
       210                 215                 220
   Tyr Asp Tyr Gln Met Glu Thr Gln Arg Asp Ser Arg Asn Ala Ala Asn
   225                 230                 235                 240
   Val Ala His Thr Met Lys Ser Ile Ser Glu Tyr Pro Val Ala Glu Arg
                   245                 250                 255
   Val Met Leu Leu Leu Thr Thr Lys Ala Gly Ala Asn Ala Met Arg Ser
               260                 265                 270
   Ala Ala Leu Ser Gly Leu Ser Tyr Val Leu Pro Thr Ser Ile Phe Ala
           275                 280                 285
   Leu Lys Phe Leu Glu Trp Trp Tyr Ala Ser Asp Phe Ala Arg Gln Leu
       290                 295                 300
   Asn Gln Lys Arg Arg Gly Asp Leu Glu Asp Asn Leu Pro Val Pro Asp
   305                 310                 315                 320
   Lys Val Lys Gly Ala Asp Lys Leu Ala Glu Ser Val Ala Lys Trp Lys
                   325                 330                 335
   Glu Asp Thr Ser Lys Cys Pro Leu Cys Ser Lys Glu Leu Val Asn Pro
               340                 345                 350
   Thr Val Ile Glu Ser Gly Tyr Val Phe Cys Tyr Thr Cys Ile Tyr Arg
           355                 360                 365
   His Leu Glu Asp Gly Asp Glu Glu Thr Gly Gly Arg Cys Pro Val Thr
       370                 375                 380
   Gly Gln Lys Leu Leu Gly Cys Arg Trp Gln Asp Asp Val Trp Gln Val
   385                 390                 395                 400
   Thr Gly Leu Arg Arg Leu Met Val
                   405
<210> 12
   <211> 412
   <212> PRT
   <213> Yarrowia lipolytica CLIB122 (GenBank Accession No. CAG81789)
<220>
   <221> MISC_FEATURE
   <222> (1)..(412)
   <223> YlPex13p
<400> 12
   Met Ser Val Pro Arg Pro Lys Pro Trp Glu Gly Ala Ser Gly Ser Ser
   1 5 10 15
   Ala Ala Thr Ala Thr Pro Ala Ala Thr Ala Thr Pro Ala Ser Thr Asp
   20 25 30
   Ala Val Ser Ser Ser Ala Gly Ser Ala Thr Gly Ala Pro Glu Leu Pro
   35 40 45
   Ser Arg Pro Ser Ala Met Gly Ser Thr Ser Asn Ala Leu Ser Ser Pro
   50 55 60
   Met Gly Ser Ser Met Asn Ser Gly Tyr Gly Gly Met Asn Ser Gly Tyr
   65 70 75 80
   Gly Gly Met Gly Ser Ser Tyr Gly Ser Gly Tyr Gly Ser Ser Tyr Gly
   85 90 95
   Met Gly Ser Ser Tyr Gly Ser Gly Tyr Gly Ser Gly Leu Gly Gly Tyr
   100 105 110
   Gly Ser Tyr Gly Gly Met Gly Gly Met Gly Gly Met Tyr Gly Ser Arg
   115 120 125
   Tyr Gly Gly Tyr Gly Ser Tyr Gly Gly Met Gly Gly Tyr Gly Gly Tyr
   130 135 140
   Gly Gly Met Gly Gly Gly Pro Met Gly Gln Asn Gly Leu Ala Gly Gly
   145 150 155 160
   Thr Gln Ala Thr Phe Gln Leu Ile Glu Ser Ile Val Gly Ala Val Gly
   165 170 175
   Gly Phe Ala Gln Met Leu Glu Ser Thr Tyr Met Ala Thr Gln Ser Ser
   180 185 190
   Phe Phe Ala Met Val Ser Val Ala Glu Gln Phe Gly Asn Leu Lys Asn
   195 200 205
   Thr Leu Gly Ser Leu Leu Gly Ile Tyr Ala Ile Met Arg Trp Ala Arg
   210 215 220
   Arg Leu Val Ala Lys Leu Ser Gly Gln Pro Val Thr Gly Ala Asn Gly
   225 230 235 240
   Ile Thr Pro Ala Gly Phe Ala Lys Phe Glu Ala Thr Gly Gly Ala Ala
   245 250 255
   Gly Pro Gly Arg Gly Pro Arg Pro Ser Tyr Lys Pro Leu Leu Phe Phe
   260 265 270
   Leu Thr Ala Val Phe Gly Leu Pro Tyr Leu Leu Gly Arg Leu Ile Lys
   275 280 285
   Ala Leu Ala Ala Lys Gln Glu Gly Met Tyr Asp Glu His Gly Asn Leu
   290 295 300
   Leu Pro Gly Ala Gln Met Gly Met Gly Gly Pro Gly Met Glu Gly Gly
   305 310 315 320
   Ala Glu Ile Asp Pro Ser Lys Leu Glu Phe Cys Arg Ala Asn Phe Asp
   325 330 335
   Phe Val Pro Glu Asn Pro Gln Leu Glu Leu Glu Leu Arg Lys Gly Asp
   340 345 350
   Leu Val Ala Val Leu Ala Lys Thr Asp Pro Met Gly Asn Pro Ser Gln
   355 360 365
   Trp Trp Arg Val Arg Thr Arg Asp Gly Arg Ser Gly Tyr Val Pro Ala
   370 375 380
   Asn Tyr Leu Glu Val Ile Pro Arg Pro Ala Val Glu Ala Pro Lys Lys
   385 390 395 400
   Val Glu Glu Ile Gly Ala Ser Ala Val Pro Val Asn
   405 410
<210> 13
   <211> 380
   <212> PRT
   <213> Yarrowia lipolytica CLIB122 (GenBank Accession No. CAG79323)
<220>
   <221> MISC_FEATURE
   <222> (1)..(380)
   <223> YlPex14p
<400> 13
   Met Ile Pro Ser Cys Leu Ser Thr Gln His Met Ala Pro Arg Glu Asp
   1 5 10 15
   Leu Val Gln Ser Ala Val Ala Phe Leu Asn Asp Pro Gln Ala Ala Thr
   20 25 30
   Ala Pro Leu Ala Lys Arg Ile Glu Phe Leu Glu Ser Lys Asp Met Thr
   35 40 45
   Pro Glu Glu Ile Glu Glu Ala Leu Lys Arg Ala Gly Ser Gly Ser Ala
   50 55 60
   Gln Ser His Pro Gly Ser Val Val Ser His Gly Gly Ala Ala Pro Thr
   65 70 75 80
   Val Pro Ala Ser Tyr Ala Phe Gln Ser Ala Pro Pro Leu Pro Glu Arg
   85 90 95
   Asp Trp Lys Asp Val Phe Ile Met Ala Thr Val Thr Val Gly Val Gly
   100 105 110
   Phe Gly Leu Tyr Thr Val Ala Lys Arg Tyr Leu Met Pro Leu Ile Leu
   115 120 125
   Pro Pro Thr Pro Pro Ser Leu Glu Ala Asp Lys Glu Ala Leu Glu Ala
   130 135 140
   Glu Phe Ala Arg Val Gln Gly Leu Leu Asp Gln Val Gln Gln Asp Thr
   145 150 155 160
   Glu Glu Val Lys Asn Ser Gln Val Glu Val Ala Lys Arg Val Thr Asp
   165 170 175
   Ala Leu Lys Gly Val Glu Glu Thr Ile Asp Gln Leu Lys Ser Gln Thr
   180 185 190
   Lys Lys Arg Asp Asp Glu Met Lys Leu Val Thr Ala Glu Val Glu Arg
   195 200 205
   Ile Arg Asp Arg Leu Pro Lys Asn Ile Asp Lys Leu Lys Asp Ser Gln
   210 215 220
   Glu Gln Gly Leu Ala Asp Ile Gln Ser Glu Leu Lys Ser Leu Lys Gln
   225 230 235 240
   Leu Leu Ser Thr Arg Thr Ala Ala Ser Ser Gly Pro Lys Leu Pro Pro
   245 250 255
   Ile Pro Pro Pro Ser Ser Tyr Leu Thr Arg Lys Ala Ser Pro Ala Val
               260                 265                 270
   Pro Ala Ala Ala Pro Ala Pro Val Thr Pro Gly Ser Pro Val His Asn
           275                 280                 285
   Val Ser Ser Ser Ser Thr Val Pro Ala Asp Arg Asp Asp Phe Ile Pro
       290                 295                 300
   Thr Pro Ala Gly Ala Val Pro Met Ile Pro Gln Pro Ala Ser Met Ser
   305                 310                 315                 320
   Ser Ser Ser Thr Ser Thr Val Pro Asn Ser Ala Ile Ser Ser Ala Pro
                   325                 330                 335
   Ser Pro Ile Gln Glu Pro Glu Pro Phe Val Pro Glu Pro Gly Asn Ser
               340                 345                 350
   Ala Val Lys Lys Pro Ala Pro Lys Ala Ser Ile Pro Ala Trp Gln Leu
           355                 360                 365
   Ala Ala Leu Glu Lys Glu Lys Glu Lys Glu Lys Glu
       370                 375                 380
<210> 14
   <211> 391
   <212> PRT
   <213> Yarrowia lipolytica CLIB122 (GenBank Accession No. CAG79622)
<220>
   <221> MISC_FEATURE
   <222> (1)..(391)
   <223> YlPex16p
<400> 14
   Met Thr Asp Lys Leu Val Lys Val Met Gln Lys Lys Lys Ser Ala Pro
   1               5                   10                  15
   Gln Thr Trp Leu Asp Ser Tyr Asp Lys Phe Leu Val Arg Asn Ala Ala
               20                  25                  30
   Ser Ile Gly Ser Ile Glu Ser Thr Leu Arg Thr Val Ser Tyr Val Leu
           35                  40                  45
   Pro Gly Arg Phe Asn Asp Val Glu Ile Ala Thr Glu Thr Leu Tyr Ala
       50                  55                  60
   Val Leu Asn Val Leu Gly Leu Tyr His Asp Thr Ile Ile Ala Arg Ala
   65 70 75 80
   Val Ala Ala Ser Pro Asn Ala Ala Ala Val Tyr Arg Pro Ser Pro His
   85 90 95
   Asn Arg Tyr Thr Asp Trp Phe Ile Lys Asn Arg Lys Gly Tyr Lys Tyr
   100 105 110
   Ala Ser Arg Ala Val Thr Phe Val Lys Phe Gly Glu Leu Val Ala Glu
   115 120 125
   Met Val Ala Lys Lys Asn Gly Gly Glu Met Ala Arg Trp Lys Cys Ile
   130 135 140
   Ile Gly Ile Glu Gly Ile Lys Ala Gly Leu Arg Ile Tyr Met Leu Gly
   145 150 155 160
   Ser Thr Leu Tyr Gln Pro Leu Cys Thr Thr Pro Tyr Pro Asp Arg Glu
   165 170 175
   Val Thr Gly Glu Leu Leu Glu Thr Ile Cys Arg Asp Glu Gly Glu Leu
   180 185 190
   Asp Ile Glu Lys Gly Leu Met Asp Pro Gln Trp Lys Met Pro Arg Thr
   195 200 205
   Gly Arg Thr Ile Pro Glu Ile Ala Pro Thr Asn Val Glu Gly Tyr Leu
   210 215 220
   Leu Thr Lys Val Leu Arg Ser Glu Asp Val Asp Arg Pro Tyr Asn Leu
   225 230 235 240
   Leu Ser Arg Leu Asp Asn Trp Gly Val Val Ala Glu Leu Leu Ser Ile
   245 250 255
   Leu Arg Pro Leu Ile Tyr Ala Cys Leu Leu Phe Arg Gln His Val Asn
   260 265 270
   Lys Thr Val Pro Ala Ser Thr Lys Ser Lys Phe Pro Phe Leu Asn Ser
   275 280 285
   Pro Trp Ala Pro Trp Ile Ile Gly Leu Val Ile Glu Ala Leu Ser Arg
   290 295 300
   Lys Met Met Gly Ser Trp Leu Leu Arg Gln Arg Gln Ser Gly Lys Thr
   305 310 315 320
   Pro Thr Ala Leu Asp Gln Met Glu Val Lys Gly Arg Thr Asn Leu Leu
   325 330 335
   Gly Trp Trp Leu Phe Arg Gly Glu Phe Tyr Gln Ala Tyr Thr Arg Pro
   340 345 350
   Leu Leu Tyr Ser Ile Val Ala Arg Leu Glu Lys Ile Pro Gly Leu Gly
   355 360 365
   Leu Phe Gly Ala Leu Ile Ser Asp Tyr Leu Tyr Leu Phe Asp Arg Tyr
   370 375 380
   Tyr Phe Thr Ala Ser Thr Leu
   385 390
<210> 15
   <211> 225
   <212> PRT
   <213> Yarrowia lipolytica CLIB122 (GenBank Accession No. CAG84025)
<220>
   <221> MISC_FEATURE
   <222> (1)..(225)
   <223> YlPexl7p
<400> 15 Arg
225
<210> 16
   <211> 324
   <212> PRT
   <213> Yarrowia lipolytica (GenBank Accession No. AAK84827)
<220>
   <221> MISC_FEATURE
   <222> (1)..(324)
   <223> YlPexl9p
<400> 16
   Met Ser His Glu Glu Asp Leu Asp Asp Leu Asp Asp Phe Leu Asp Glu
   1               5                   10                  15
   Phe Asp Glu Gln Val Leu Ser Lys Pro Pro Gly Ala Gln Lys Asp Ala
               20                  25                  30
   Thr Pro Thr Thr Ser Thr Ala Pro Thr Thr Ala Glu Ala Lys Pro Asp
           35                  40                  45
   Ala Thr Lys Lys Ser Thr Glu Thr Ser Gly Thr Asp Ser Lys Thr Glu
       50                  55                  60
   Gly Ala Asp Thr Ala Asp Lys Asn Ala Ala Thr Asp Ser Ala Glu Ala
   65 70 75 80
   Gly Ala Glu Lys Val Ser Leu Pro Asn Leu Glu Asp Gln Leu Ala Gly
   85 90 95
   Leu Lys Met Asp Asp Phe Leu Lys Asp Ile Glu Ala Asp Pro Glu Ser
   100 105 110
   Lys Ala Gln Phe Glu Ser Leu Leu Lys Glu Ile Asn Asn Val Thr Ser
   115 120 125
   Ala Thr Ala Ser Glu Lys Ala Gln Gln Pro Lys Ser Phe Lys Glu Thr
   130 135 140
   Ile Ser Ala Thr Ala Asp Arg Leu Asn Gln Ser Asn Gln Glu Met Gly
   145 150 155 160
   Asp Met Pro Leu Gly Asp Asp Met Leu Ala Gly Leu Met Glu Gln Leu
   165 170 175
   Ser Gly Ala Gly Gly Phe Gly Glu Gly Gly Glu Gly Asp Phe Gly Asp
   180 185 190
   Met Leu Gly Gly Ile Met Arg Gln Leu Ala Ser Lys Glu Val Leu Tyr
   195 200 205
   Gln Pro Leu Lys Glu Met His Asp Asn Tyr Pro Lys Trp Trp Asp Glu
   210 215 220
   His Gly Ser Lys Val Thr Glu Glu Lys Glu Arg Asp Arg Leu Lys Leu
   225 230 235 240
   Gln Gln Asp Ile Val Gly Lys Ile Cys Ala Lys Phe Glu Asp Pro Ser
   245 250 255
   Tyr Ser Asp Asp Ser Glu Ala Asp Arg Ala Val Ile Thr Gln Leu Met
   260 265 270
   Asp Glu Met Gln Glu Thr Gly Ala Pro Pro Asp Glu Ile Met Ser Asn
   275 280 285
   Val Ala Asp Gly Ser Ile Pro Gly Gly Leu Asp Gly Leu Gly Leu Gly
   290 295 300
   Gly Leu Gly Gly Gly Lys Met Pro Glu Met Pro Glu Asn Met Pro Glu
   305 310 315 320
   Cys Asn Gln Gln
<210> 17
   <211> 417
   <212> PRT
   <213> Yarrowia lipolytica CLIB122 (GenBank Accession No. CAG79226)
<220>
   <221> MISC_FEATURE
   <222> (1)..(417)
   <223> YlPex20p
<400> 17
   Met Ala Ser Cys Gly Pro Ser Asn Ala Leu Gln Asn Leu Ser Lys His
   1 5 10 15
   Ala Ser Ala Asp Arg Ser Leu Gln His Asp Arg Met Ala Pro Gly Gly
   20 25 30
   Ala Pro Gly Ala Gln Arg Gln Gln Phe Arg Ser Gln Thr Gln Gly Gly
   35 40 45
   Gln Leu Asn Asn Glu Phe Gln Gln Phe Ala Gln Ala Gly Pro Ala His
   50 55 60
   Asn Ser Phe Glu Gln Ser Gln Met Gly Pro His Phe Gly Gln Gln His
   65 70 75 80
   Phe Gly Gln Pro His Gln Pro Gln Met Gly Gln His Ala Pro Met Ala
   85 90 95
   His Gly Gln Gln Ser Asp Trp Ala Gln Ser Phe Ser Gln Leu Asn Leu
   100 105 110
   Gly Pro Gln Thr Gly Pro Gln His Thr Gln Gln Ser Asn Trp Gly Gln
   115 120 125
   Asp Phe Met Arg Gln Ser Pro Gln Ser His Gln Val Gln Pro Gln Met
   130 135 140
   Ala Asn Gly Val Met Gly Ser Met Ser Gly Met Ser Ser Phe Gly Pro
   145 150 155 160
   Met Tyr Ser Asn Ser Gln Leu Met Asn Ser Thr Tyr Gly Leu Gln Thr
                   165                 170                 175
   Glu His Gln Gln Thr His Lys Thr Glu Thr Lys Ser Ser Gln Asp Ala
               180                 185                 190
   Ala Phe Glu Ala Ala Phe Gly Ala Val Glu Glu Ser Ile Thr Lys Thr
           195                 200                 205
   Ser Asp Lys Gly Lys Glu Val Glu Lys Asp Pro Met Glu Gln Thr Tyr
       210                 215                 220
   Arg Tyr Asp Gln Ala Asp Ala Leu Asn Arg Gln Ala Glu His Ile Ser
   225                 230                 235                 240
   Asp Asn Ile Ser Arg Glu Glu Val Asp Ile Lys Thr Asp Glu Asn Gly
                   245                 250                 255
   Glu Phe Ala Ser Ile Ala Arg Gln Ile Ala Ser Ser Leu Glu Glu Ala
               260                 265                 270
   Asp Lys Ser Lys Phe Glu Lys Ser Thr Phe Met Asn Leu Met Arg Arg
           275                 280                 285
   Ile Gly Asn His Glu Val Thr Leu Asp Gly Asp Lys Leu Val Asn Lys
       290                 295                 300
   Glu Gly Glu Asp Ile Arg Glu Glu Val Arg Asp Glu Leu Leu Arg Glu
   305                 310                 315                 320
   Gly Ala Ser Gln Glu Asn Gly Phe Gln Ser Glu Ala Gln Gln Thr Ala
                   325                 330                 335
   Pro Leu Pro Val His His Glu Ala Pro Pro Pro Glu Gln Ile His Pro
               340                 345                 350
   His Thr Glu Thr Gly Asp Lys Gln Leu Glu Asp Pro Met Val Tyr Ile
           355                 360                 365
   Glu Gln Glu Ala Ala Arg Arg Ala Ala Glu Ser Gly Arg Thr Val Glu
       370                 375                 380
   Glu Glu Lys Leu Asn Phe Tyr Ser Pro Phe Glu Tyr Ala Gln Lys Leu
   385                 390                 395                 400
   Gly Pro Gln Gly Val Ala Lys Gln Ser Asn Trp Glu Glu Asp Tyr Asp
   405 410 415
   Phe
<210> 18
   <211> 195
   <212> PRT
   <213> Yarrowia lipolytica CLIB122 (GenBank Accession No. CAG77876)
<220>
   <221> MISC_FEATURE
   <222> (1)..(195)
   <223> YlPex22p
<400> 18
   Val Pro Arg Cys Thr Ser His Pro Cys Asn Leu Thr Leu His Leu Pro
   1 5 10 15
   Val Thr Thr Met Ala Pro Arg Lys Thr Arg Leu Pro Ala Val Ile Gly
   20 25 30
   Ala Ala Ala Ala Ala Ala Ala Val Ala Tyr Leu Val Tyr Ser Phe Val
   35 40 45
   Ala Lys Ser Asn Ser Asp Gln Asp Thr Phe Asp Ser Ser Val Gln Ser
   50 55 60
   Ser Ser Lys Ser Ser Thr Lys Ser Pro Lys Ser Thr Ala Thr Asn Ser
   65 70 75 80
   Lys Ile Thr Val Val Val Ser Gln Glu Leu Val Gln Ser Gln Leu Val
   85 90 95
   Asp Phe Lys His Leu Met Ser Val His Pro Asn Leu Val Val Ile Val
   100 105 110
   Pro Pro Met Val Ala Asn Lys Phe His Arg Ala Leu Lys Ser Ser Val
   115 120 125
   Gly His Asp His Gly Val Lys Val Ile Arg Cys Asp Thr Asp Val Gly
   130 135 140
   Val Ile His Val Ile Lys His Ile Arg Pro Asp Leu Ala Leu Ile Ala
   145 150 155 160
   Asp Gly Val Gly Asp Asn Ile Gln Gly Glu Ile Lys Arg Phe Val Gly
   165 170 175
   Ser Ser Glu Ala Leu Ser Gly Asp Val Asn Leu Ala Ala Glu Arg Leu
   180 185 190
   Thr Gly Leu
   195
<210> 19
   <211> 386
   <212> PRT
   <213> Yarrowia lipolytica CLIB122 (GenBank Accession No. NC_006072, antisense
   translation of nucleotides 117230-118387)
<220>
   <221> MISC_FEATURE
   <222> (1)..(386)
   <223> YlPex26p
<400> 19
   Met Pro Pro Ala Met Pro Gln Met Thr Thr Ser Thr Leu Leu Thr Asp
   1 5 10 15
   Ser Val Thr Ser Ala Val Asn Gln Ala Ala Thr Pro Lys Val Asp Gln
   20 25 30
   Met Tyr Gln Thr Phe Gly Glu Ser Ala Arg Glu Phe Val Asn Lys Asn
   35 40 45
   Phe Tyr Asn Ser Tyr Glu Leu Ile Arg Pro Phe Phe Asp Glu Ile Thr
   50 55 60
   Ala Lys Gly Ala Gln Gln Asn Gly Ser Thr Val Leu Asp Ala Glu Asn
   65 70 75 80
   Pro His Asn Ile Pro Leu Ser Leu Trp Ile Lys Val Trp Ser Leu Tyr
   85 90 95
   Leu Ala Ile Leu Asp Ala Ser Cys Lys Gln Ala Gly Glu Ala Leu Leu
   100 105 110
   Asn Ser Thr Gly Asp Leu Ser Gly Ser Asp Ser Gly Glu Trp Asn Gln
   115 120 125
   Thr Arg Lys Leu Leu Ala Arg Lys Leu Thr Ser Gly Ser Val Trp Asp
   130 135 140
   Glu Leu Val Thr Ala Ser Gly Gly Thr Gly Asn Ile His Pro Thr Ile
   145                 150                 155                 160
   Leu Ala Leu Leu Ala Ser Leu Ser Ile Arg His Asp Thr Asp Ala Lys
                   165                 170                 175
   Leu Met Ala Asp Asn Leu Glu Lys Phe Ile Val Thr Tyr Asn Asp Asn
               180                 185                 190
   Gly Ser Asp Asp Val Lys Thr Lys Thr Ala Phe Tyr Lys Val Leu Asp
           195                 200                 205
   Leu Tyr Leu Leu Arg Val Leu Pro Asp Leu Gly Gln Trp Asp Val Ala
       210                 215                 220
   His Ser Phe Val Asn Asn Thr Asn Leu Phe Ser His Glu Gln Lys Lys
   225                 230                 235                 240
   Glu Met Thr His Lys Leu Asp Gln Ser Gln Lys His Ala Glu Gln Glu
                   245                 250                 255
   His Lys Arg Leu Leu Glu Glu Ala Gln Glu Lys Glu Lys Ser Asp Ala
               260                 265                 270
   Lys Glu Lys Glu Arg Glu Glu Arg Val Ser Arg Asp Thr Gln Ser Arg
           275                 280                 285
   Glu Ile Lys Ser Pro Ile Val Asp Ser Ser Thr Ser Ser Arg Asp Val
       290                 295                 300
   Thr Arg Asp Thr Thr Arg Glu Leu Ser Lys Ser Ser Arg Gln Pro Arg
   305                 310                 315                 320
   Thr Leu Ser Gln Ile Ile Ser Thr Ser Leu Lys Ser Gln Phe Asp Gly
                   325                 330                 335
   Asn Ala Ile Phe Arg Thr Leu Ala Leu Ile Val Ile Val Ser Leu Ser
               340                 345                 350
   Ala Ala Asn Pro Leu Ile Arg Lys Arg Val Val Asp Thr Leu Lys Met
           355                 360                 365
   Leu Trp Ile Lys Ile Leu Gln Thr Leu Ser Met Gly Phe Lys Val Ser
       370                 375                 380
   Tyr Leu
   385
<210> 20
   <211> 3387
   <212> DNA
   <213> Yarrowia lipolytica (GenBank Accession No. AB036770)
<400> 20
   ggtaccatca agggtaaaat caaggctatc atcaagggcc atatatcgca agtttggggg 60
   aagataatat gttcatagtg aatcgggttg tggatttcct catctaacgg cattataact 120
   agtcctggag ggtctttttt atggataacc tccatgtacg atgtatccaa gatctccacg 180
   tactgtgttc tgtttcctaa gtaataccca acaacctctc caacaaacac ttgggaagat 240
   gcacttgtgc tgagatgtca agatgttaga gagtagagac agtagcaagc gtaaaaggcg 300
   gccgaggcca ccgagagaac agcgtagcag ggcgcgtagt caccacaggg gacgcagaac 360
   caaacaaatg acgaagaaga accacaagga gacgttttca aaggcaatgc aaacgaagag 420
   ggcaatggaa ggattgagat tagagaactg gagactggag tggcgttttc ccgatgaacg 480
   aacaaacacg cgaagctatg tggaccaaca tacaacacgg actgaaccag gtttttttat 540
   gattttttta ctggaaatag gtacgtgcca agttggacca tgacactaaa cgtgtttaat 600
   tagtaatatt cgtgtaagcg tacattcatt tcaaaggtta ttctttcacg gcaaagttat 660
   aattaaatga atgtatatgc agaaaaaaaa aaaaaaagta ctgtactgga tggagagaat 720
   attaataaat aattgttacc caactacatc ttgtcgattg aaagagaccc ctaagacaga 780
   taggatatct gcaacccgag gaatgaaccc cccagcaccg gcaccctttc tattaacaaa 840
   atgccaactg aaatttgaaa agttcaacta aacttatttg acccacaaaa actcgtcaaa 900
   agtggcggcg aaagctggca aatgatgaca tccccttgga accatgatat cctctcggaa 960
   tcttcgtccc catttgccac atctacttgc aacgccacat ctgcttacta agcaacccaa 1020
   atctgcctcg gctcaaaatg tggggaagtt cacatgcatt cgctggtgaa tctgatctga 1080
   cactacaact acacaccagg tccaacatga gcgacaatac gacaatcaaa aagccgatcc 1140
   gacccaaacc gatccggacg gaacgcctgc cttacgctgg ggccgcagaa atcatccgag 1200
   ccaaccagaa agaccactac tttgagtccg tgcttgaaca gcatctcgtc acgtttctgc 1260
   agaaatggaa gggagtacga tttatccacc agtacaagga ggagctggag acggcgtcca 1320
   agtttgcata tctcggtttg tgtacgcttg tgggctccaa gactctcgga gaagagtaca 1380
   ccaatctcat gtacactatc agagaccgaa cagctctacc gggggtggtg agacggtttg 1440
   gctacgtgct ttccaacact ctgtttccat acctgtttgt gcgctacatg ggcaagttgc 1500
   gcgccaaact gatgcgcgag tatccccatc tggtggagta cgacgaagat gagcctgtgc 1560
   ccagcccgga aacatggaag gagcgggtca tcaagacgtt tgtgaacaag tttgacaagt 1620
   tcacggcgct ggaggggttt accgcgatcc acttggcgat tttctacgtc tacggctcgt 1680
   actaccagct cagtaagcgg atctggggca tgcgttatgt atttggacac cgactggaca 1740
   agaatgagcc tcgaatcggt tacgagatgc tcggtctgct gattttcgcc cggtttgcca 1800
   cgtcatttgt gcagacggga agagagtacc tcggagcgct gctggaaaag agcgtggaga 1860
   aagaggcagg ggagaaggaa gatgaaaagg aagcggttgt gccgaaaaag aagtcgtcaa 1920
   ttccgttcat tgaggataca gaaggggaga cggaagacaa gatcgatctg gaggaccctc 1980
   gacagctcaa gttcattcct gaggcgtcca gagcgtgcac tctgtgtctg tcatacatta 2040
   gtgcgccggc atgtacgcca tgtggacact ttttctgttg ggactgtatt tccgaatggg 2100
   tgagagagaa gcccgagtgt cccttgtgtc ggcagggtgt gagagagcag aacttgttgc 2160
   ctatcagata atgacgaggt ctggatggaa ggactagtca gcgagacaca gagcatcagg 2220
   gaccagacac gaccaattca atcgacaaca ctgtgctgca tagcagtgca cagaggtcct 2280
   gggcatgaat atattttagc attggagata tgagtggtag agcgtataca gtattaattg 2340
   tggaggtatc tcgtcgcatt gatagagcaa tacagttact gctgaaggga atgataccga 2400
   gtatttcggc ccgattcagt tcttgatatc gtcattttgt ctctattgtc tacttttcag 2460
   ataacctcaa caaatcttca acaaatctcc cagtaaacag tcagagatca tatccgagat 2520
   catatcagat atgtcacgat ccgagtacaa taatggatat taatctgctt gattttgaat 2580
   tctgttgcga ttatgatttc tttgatttcg atatgaacac atacggcgac tcccagacct 2640
   ttagaagctc cagtttggat tcttagcaat ggttacactc aactatatcc caagtaatac 2700
   ttggtaacaa tatgccaagt tagtcattca ttcgttatag gagttagcaa gtgtttgtca 2760
   gctaaaaatg gttagtcggt cgattaccac ttagatcttt tcagcgtgga acttgatggt 2820
   acgcttgaac cgacacttgg agtagtcggg gctgttgatg acgtagatga cgtttcgctc 2880
   agggtgagga gtgcaatagt agtactcctt ggggccgtct ctcagctcaa aggttccatc 2940
   ggcggcaatg tcaaagaccg agccctggag cttgtagccg tagtcgccgg tccagaacaa 3000
   agcctgcagc tccagatagg cgatgggcat gtcgttaaca gagaaggtgt tgccctcgcc 3060
   ctcggtgatg gtgatgggtt cgccgtcggt ggaggcggtg atcaggtcat cttggtaggt 3120
   gacgggcaga gattcgaccg attgggcgtc tgatctggta taggtcagct tgtacttgtc 3180
   tccgacagcc gccagagcgg tggtagcgac ggtgatgagg gagatgagtt tcatattggc 3240
   ggcaagttta gcaaaagatg gcagtgggat tgagggacaa gagtgtttat atagatatag 3300
   atacaacaca acgagtctga atgagacaac cgagacaacc actcccgaag cctcactaat 3360
   agttactaac ggcatatttc aggtacc 3387
<210> 21
   <211> 1134
   <212> DNA
   <213> Yarrowia lipolytica (GenBank Accession No. AB036770, nucleotides 1038-2171)
<220>
   <221> CDS
   <222> (1)..(1134)
   <223> Pex10
<400> 21
   atg tgg gga agt tca cat gca ttc gct ggt gaa tct gat ctg aca cta      48
   Met Trp Gly Ser Ser His Ala Phe Ala Gly Glu Ser Asp Leu Thr Leu
   1               5                   10                  15
   caa cta cac acc agg tcc aac atg agc gac aat acg aca atc aaa aag      96
   Gln Leu His Thr Arg Ser Asn Met Ser Asp Asn Thr Thr Ile Lys Lys
               20                  25                  30
   ccg atc cga ccc aaa ccg atc cgg acg gaa cgc ctg cct tac gct ggg     144
   Pro Ile Arg Pro Lys Pro Ile Arg Thr Glu Arg Leu Pro Tyr Ala Gly
           35                  40                  45
   gcc gca gaa atc atc cga gcc aac cag aaa gac cac tac ttt gag tcc     192
   Ala Ala Glu Ile Ile Arg Ala Asn Gln Lys Asp His Tyr Phe Glu Ser
       50                  55                  60
   gtg ctt gaa cag cat ctc gtc acg ttt ctg cag aaa tgg aag gga gta     240
   Val Leu Glu Gln His Leu Val Thr Phe Leu Gln Lys Trp Lys Gly Val
   65                  70                  75                  80
   cga ttt atc cac cag tac aag gag gag ctg gag acg gcg tcc aag ttt     288
   Arg Phe Ile His Gln Tyr Lys Glu Glu Leu Glu Thr Ala Ser Lys Phe
                   85                  90                  95
   gca tat ctc ggt ttg tgt acg ctt gtg ggc tcc aag act ctc gga gaa     336
   Ala Tyr Leu Gly Leu Cys Thr Leu Val Gly Ser Lys Thr Leu Gly Glu
               100                 105                 110
   gag tac acc aat ctc atg tac act atc aga gac cga aca gct cta ccg     384
   Glu Tyr Thr Asn Leu Met Tyr Thr Ile Arg Asp Arg Thr Ala Leu Pro
           115                 120                 125
   ggg gtg gtg aga cgg ttt ggc tac gtg ctt tcc aac act ctg ttt cca     432
   Gly Val Val Arg Arg Phe Gly Tyr Val Leu Ser Asn Thr Leu Phe Pro
       130                 135                 140
   tac ctg ttt gtg cgc tac atg ggc aag ttg cgc gcc aaa ctg atg cgc     480
   Tyr Leu Phe Val Arg Tyr Met Gly Lys Leu Arg Ala Lys Leu Met Arg
   145                 150                 155                 160
   gag tat ccc cat ctg gtg gag tac gac gaa gat gag cct gtg ccc agc     528
   Glu Tyr Pro His Leu Val Glu Tyr Asp Glu Asp Glu Pro Val Pro Ser
                   165                 170                 175
   ccg gaa aca tgg aag gag cgg gtc atc aag acg ttt gtg aac aag ttt     576
   Pro Glu Thr Trp Lys Glu Arg Val Ile Lys Thr Phe Val Asn Lys Phe
                   180                 185                 190
   gac aag ttc acg gcg ctg gag ggg ttt acc gcg atc cac ttg gcg att      624
   Asp Lys Phe Thr Ala Leu Glu Gly Phe Thr Ala Ile His Leu Ala Ile
           195                 200                 205
   ttc tac gtc tac ggc tcg tac tac cag ctc agt aag cgg atc tgg ggc      672
   Phe Tyr Val Tyr Gly Ser Tyr Tyr Gln Leu Ser Lys Arg Ile Trp Gly
       210                 215                 220
   atg cgt tat gta ttt gga cac cga ctg gac aag aat gag cct cga atc      720
   Met Arg Tyr Val Phe Gly His Arg Leu Asp Lys Asn Glu Pro Arg Ile
   225                 230                 235                 240
   ggt tac gag atg ctc ggt ctg ctg att ttc gcc cgg ttt gcc acg tca      768
   Gly Tyr Glu Met Leu Gly Leu Leu Ile Phe Ala Arg Phe Ala Thr Ser
                   245                 250                 255
   ttt gtg cag acg gga aga gag tac ctc gga gcg ctg ctg gaa aag agc      816
   Phe Val Gln Thr Gly Arg Glu Tyr Leu Gly Ala Leu Leu Glu Lys Ser
               260                 265                 270
   gtg gag aaa gag gca ggg gag aag gaa gat gaa aag gaa gcg gtt gtg      864
   Val Glu Lys Glu Ala Gly Glu Lys Glu Asp Glu Lys Glu Ala Val Val
           275                 280                 285
   ccg aaa aag aag tcg tca att ccg ttc att gag gat aca gaa ggg gag      912
   Pro Lys Lys Lys Ser Ser Ile Pro Phe Ile Glu Asp Thr Glu Gly Glu
       290                 295                 300
   acg gaa gac aag atc gat ctg gag gac cct cga cag ctc aag ttc att      960
   Thr Glu Asp Lys Ile Asp Leu Glu Asp Pro Arg Gln Leu Lys Phe Ile
   305                 310                 315                 320
   cct gag gcg tcc aga gcg tgc act ctg tgt ctg tca tac att agt gcg     1008
   Pro Glu Ala Ser Arg Ala Cys Thr Leu Cys Leu Ser Tyr Ile Ser Ala
                   325                 330                 335
   ccg gca tgt acg cca tgt gga cac ttt ttc tgt tgg gac tgt att tcc     1056
   Pro Ala Cys Thr Pro Cys Gly His Phe Phe Cys Trp Asp Cys Ile Ser
               340                 345                 350
   gaa tgg gtg aga gag aag ccc gag tgt ccc ttg tgt cgg cag ggt gtg     1104
   Glu Trp Val Arg Glu Lys Pro Glu Cys Pro Leu Cys Arg Gln Gly Val
           355                 360                 365
   aga gag cag aac ttg ttg cct atc aga taa                             1134
   Arg Glu Gln Asn Leu Leu Pro Ile Arg
       370                 375
<210> 22
   <211> 377
   <212> PRT
   <213> Yarrowia lipolytica (GenBank Accession No. AB036770, nucleotides 1038-2171)
<400> 22
   Met Trp Gly Ser Ser His Ala Phe Ala Gly Glu Ser Asp Leu Thr Leu
   1               5                   10                  15
   Gln Leu His Thr Arg Ser Asn Met Ser Asp Asn Thr Thr Ile Lys Lys
               20                  25                  30
   Pro Ile Arg Pro Lys Pro Ile Arg Thr Glu Arg Leu Pro Tyr Ala Gly
           35                  40                  45
   Ala Ala Glu Ile Ile Arg Ala Asn Gln Lys Asp His Tyr Phe Glu Ser
       50                  55                  60
   Val Leu Glu Gln His Leu Val Thr Phe Leu Gln Lys Trp Lys Gly Val
   65                  70                  75                  80
   Arg Phe Ile His Gln Tyr Lys Glu Glu Leu Glu Thr Ala Ser Lys Phe
                   85                  90                  95
   Ala Tyr Leu Gly Leu Cys Thr Leu Val Gly Ser Lys Thr Leu Gly Glu
               100                 105                 110
   Glu Tyr Thr Asn Leu Met Tyr Thr Ile Arg Asp Arg Thr Ala Leu Pro
           115                 120                 125
   Gly Val Val Arg Arg Phe Gly Tyr Val Leu Ser Asn Thr Leu Phe Pro
       130                 135                 140
   Tyr Leu Phe Val Arg Tyr Met Gly Lys Leu Arg Ala Lys Leu Met Arg
   145                 150                 155                 160
   Glu Tyr Pro His Leu Val Glu Tyr Asp Glu Asp Glu Pro Val Pro Ser
                   165                 170                 175
   Pro Glu Thr Trp Lys Glu Arg Val Ile Lys Thr Phe Val Asn Lys Phe
               180                 185                 190
   Asp Lys Phe Thr Ala Leu Glu Gly Phe Thr Ala Ile His Leu Ala Ile
           195                 200                 205
   Phe Tyr Val Tyr Gly Ser Tyr Tyr Gln Leu Ser Lys Arg Ile Trp Gly
       210                 215                 220
   Met Arg Tyr Val Phe Gly His Arg Leu Asp Lys Asn Glu Pro Arg Ile
   225                 230                 235                 240
   Gly Tyr Glu Met Leu Gly Leu Leu Ile Phe Ala Arg Phe Ala Thr Ser
                   245                 250                 255
   Phe Val Gln Thr Gly Arg Glu Tyr Leu Gly Ala Leu Leu Glu Lys Ser
               260                 265                 270
   Val Glu Lys Glu Ala Gly Glu Lys Glu Asp Glu Lys Glu Ala Val Val
           275                 280                 285
   Pro Lys Lys Lys Ser Ser Ile Pro Phe Ile Glu Asp Thr Glu Gly Glu
       290                 295                 300
   Thr Glu Asp Lys Ile Asp Leu Glu Asp Pro Arg Gln Leu Lys Phe Ile
   305                 310                 315                 320
   Pro Glu Ala Ser Arg Ala Cys Thr Leu Cys Leu Ser Tyr Ile Ser Ala
                   325                 330                 335
   Pro Ala Cys Thr Pro Cys Gly His Phe Phe Cys Trp Asp Cys Ile Ser
               340                 345                 350
   Glu Trp Val Arg Glu Lys Pro Glu Cys Pro Leu Cys Arg Gln Gly Val
           355                 360                 365
   Arg Glu Gln Asn Leu Leu Pro Ile Arg
       370                 375
<210> 23
   <211> 1065
   <212> DNA
   <213> Yarrowia lipolytica (GenBank Accession No. AJ012084, which corresponds to
   nucleotides 1107-2171 of GenBank Accession No. AB036770)
<220>
   <221> CDS
   <222> (1)..(1065)
   <223> YlPEX10
<400> 23
   atg agc gac aat acg aca atc aaa aag ccg atc cga ccc aaa ccg atc      48
   Met Ser Asp Asn Thr Thr Ile Lys Lys Pro Ile Arg Pro Lys Pro Ile
   1               5                   10                  15
   cgg acg gaa cgc ctg cct tac gct ggg gcc gca gaa atc atc cga gcc      96
   Arg Thr Glu Arg Leu Pro Tyr Ala Gly Ala Ala Glu Ile Ile Arg Ala
               20                  25                  30
   aac cag aaa gac cac tac ttt gag tcc gtg ctt gaa cag cat ctc gtc     144
   Asn Gln Lys Asp His Tyr Phe Glu Ser Val Leu Glu Gln His Leu Val
           35                  40                  45
   acg ttt ctg cag aaa tgg aag gga gta cga ttt atc cac cag tac aag     192
   Thr Phe Leu Gln Lys Trp Lys Gly Val Arg Phe Ile His Gln Tyr Lys
           50                  55                  60
   gag gag ctg gag acg gcg tcc aag ttt gca tat ctc ggt ttg tgt acg        240
   Glu Glu Leu Glu Thr Ala Ser Lys Phe Ala Tyr Leu Gly Leu Cys Thr
   65                  70                  75                  80
   ctt gtg ggc tcc aag act ctc gga gaa gag tac acc aat ctc atg tac        288
   Leu Val Gly Ser Lys Thr Leu Gly Glu Glu Tyr Thr Asn Leu Met Tyr
                   85                  90                  95
   act atc aga gac cga aca gct cta ccg ggg gtg gtg aga cgg ttt ggc        336
   Thr Ile Arg Asp Arg Thr Ala Leu Pro Gly Val Val Arg Arg Phe Gly
               100                 105                 110
   tac gtg ctt tcc aac act ctg ttt cca tac ctg ttt gtg cgc tac atg        384
   Tyr Val Leu Ser Asn Thr Leu Phe Pro Tyr Leu Phe Val Arg Tyr Met
           115                 120                 125
   ggc aag ttg cgc gcc aaa ctg atg cgc gag tat ccc cat ctg gtg gag        432
   Gly Lys Leu Arg Ala Lys Leu Met Arg Glu Tyr Pro His Leu Val Glu
       130                 135                 140
   tac gac gaa gat gag cct gtg ccc agc ccg gaa aca tgg aag gag cgg        480
   Tyr Asp Glu Asp Glu Pro Val Pro Ser Pro Glu Thr Trp Lys Glu Arg
   145                 150                 155                 160
   gtc atc aag acg ttt gtg aac aag ttt gac aag ttc acg gcg ctg gag        528
   Val Ile Lys Thr Phe Val Asn Lys Phe Asp Lys Phe Thr Ala Leu Glu
                   165                 170                 175
   ggg ttt acc gcg atc cac ttg gcg att ttc tac gtc tac ggc tcg tac        576
   Gly Phe Thr Ala Ile His Leu Ala Ile Phe Tyr Val Tyr Gly Ser Tyr
               180                 185                 190
   tac cag ctc agt aag cgg atc tgg ggc atg cgt tat gta ttt gga cac        624
   Tyr Gln Leu Ser Lys Arg Ile Trp Gly Met Arg Tyr Val Phe Gly His
           195                 200                 205
   cga ctg gac aag aat gag cct cga atc ggt tac gag atg ctc ggt ctg        672
   Arg Leu Asp Lys Asn Glu Pro Arg Ile Gly Tyr Glu Met Leu Gly Leu
       210                 215                 220
   ctg att ttc gcc cgg ttt gcc acg tca ttt gtg cag acg gga aga gag        720
   Leu Ile Phe Ala Arg Phe Ala Thr Ser Phe Val Gln Thr Gly Arg Glu
   225                 230                 235                 240
   tac ctc gga gcg ctg ctg gaa aag agc gtg gag aaa gag gca ggg gag        768
   Tyr Leu Gly Ala Leu Leu Glu Lys Ser Val Glu Lys Glu Ala Gly Glu
                   245                 250                 255
   aag gaa gat gaa aag gaa gcg gtt gtg ccg aaa aag aag tcg tca att        816
   Lys Glu Asp Glu Lys Glu Ala Val Val Pro Lys Lys Lys Ser Ser Ile
               260                 265                 270
   ccg ttc att gag gat aca gaa ggg gag acg gaa gac aag atc gat ctg        864
   Pro Phe Ile Glu Asp Thr Glu Gly Glu Thr Glu Asp Lys Ile Asp Leu
           275                 280                 285
   gag gac cct cga cag ctc aag ttc att cct gag gcg tcc aga gcg tgc        912
   Glu Asp Pro Arg Gln Leu Lys Phe Ile Pro Glu Ala Ser Arg Ala Cys
       290                 295                 300
   act ctg tgt ctg tca tac att agt gcg ccg gca tgt acg cca tgt gga      960
   Thr Leu Cys Leu Ser Tyr Ile Ser Ala Pro Ala Cys Thr Pro Cys Gly
   305                 310                 315                 320
   cac ttt ttc tgt tgg gac tgt att tcc gaa tgg gtg aga gag aag ccc     1008
   His Phe Phe Cys Trp Asp Cys Ile Ser Glu Trp Val Arg Glu Lys Pro
                   325                 330                 335
   gag tgt ccc ttg tgt cgg cag ggt gtg aga gag cag aac ttg ttg cct     1056
   Glu Cys Pro Leu Cys Arg Gln Gly Val Arg Glu Gln Asn Leu Leu Pro
               340                 345                 350
   atc aga taa                                                         1065
   Ile Arg
<210> 24
   <211> 354
   <212> PRT
   <213> Yarrowia lipolytica (GenBank Accession No. AJ012084, which corresponds to
   nucleotides 1107-2171 of GenBank Accession No. AB036770)
<400> 24
   Met Ser Asp Asn Thr Thr Ile Lys Lys Pro Ile Arg Pro Lys Pro Ile
   1               5                   10                  15
   Arg Thr Glu Arg Leu Pro Tyr Ala Gly Ala Ala Glu Ile Ile Arg Ala
               20                  25                  30
   Asn Gln Lys Asp His Tyr Phe Glu Ser Val Leu Glu Gln His Leu Val
           35                  40                  45
   Thr Phe Leu Gln Lys Trp Lys Gly Val Arg Phe Ile His Gln Tyr Lys
       50                  55                  60
   Glu Glu Leu Glu Thr Ala Ser Lys Phe Ala Tyr Leu Gly Leu Cys Thr
   65                  70                  75                  80
   Leu Val Gly Ser Lys Thr Leu Gly Glu Glu Tyr Thr Asn Leu Met Tyr
                   85                  90                  95
   Thr Ile Arg Asp Arg Thr Ala Leu Pro Gly Val Val Arg Arg Phe Gly
               100                 105                 110
   Tyr Val Leu Ser Asn Thr Leu Phe Pro Tyr Leu Phe Val Arg Tyr Met
           115                 120                 125
   Gly Lys Leu Arg Ala Lys Leu Met Arg Glu Tyr Pro His Leu Val Glu
       130                 135                 140
   Tyr Asp Glu Asp Glu Pro Val Pro Ser Pro Glu Thr Trp Lys Glu Arg
   145                 150                 155                 160
   Val Ile Lys Thr Phe Val Asn Lys Phe Asp Lys Phe Thr Ala Leu Glu
                   165                 170                 175
   Gly Phe Thr Ala Ile His Leu Ala Ile Phe Tyr Val Tyr Gly Ser Tyr
               180                 185                 190
   Tyr Gln Leu Ser Lys Arg Ile Trp Gly Met Arg Tyr Val Phe Gly His
           195                 200                 205
   Arg Leu Asp Lys Asn Glu Pro Arg Ile Gly Tyr Glu Met Leu Gly Leu
       210                 215                 220
   Leu Ile Phe Ala Arg Phe Ala Thr Ser Phe Val Gln Thr Gly Arg Glu
   225                 230                 235                 240
   Tyr Leu Gly Ala Leu Leu Glu Lys Ser Val Glu Lys Glu Ala Gly Glu
                   245                 250                 255
   Lys Glu Asp Glu Lys Glu Ala Val Val Pro Lys Lys Lys Ser Ser Ile
               260                 265                 270
   Pro Phe Ile Glu Asp Thr Glu Gly Glu Thr Glu Asp Lys Ile Asp Leu
           275                 280                 285
   Glu Asp Pro Arg Gln Leu Lys Phe Ile Pro Glu Ala Ser Arg Ala Cys
       290                 295                 300
   Thr Leu Cys Leu Ser Tyr Ile Ser Ala Pro Ala Cys Thr Pro Cys Gly
   305                 310                 315                 320
   His Phe Phe Cys Trp Asp Cys Ile Ser Glu Trp Val Arg Glu Lys Pro
                   325                 330                 335
   Glu Cys Pro Leu Cys Arg Gln Gly Val Arg Glu Gln Asn Leu Leu Pro
               340                 345                 350
   Ile Arg
<210> 25
   <211> 38
   <212> PRT
   <213> Yarrowia lipolytica
<220>
   <221> misc_feature
   <222> (2)..(3)
   <223> Xaa can be any naturally occurring amino acid
<220>
   <221> misc_feature
   <222> (5).. (15)
   <223> Xaa can be any naturally occurring amino acid
<220>
   <221> misc_feature
   <222> (17)..(17)
   <223> Xaa can be any naturally occurring amino acid
<220>
   <221> misc_feature
   <222> (19)..(20)
   <223> Xaa can be any naturally occurring amino acid
<220>
   <221> misc_feature
   <222> (22)..(23)
   <223> Xaa can be any naturally occurring amino acid
<220>
   <221> misc_feature
   <222> (25)..(34)
   <223> Xaa can be any naturally occurring amino acid
<220>
   <221> misc_feature
   <222> (36)..(37)
   <223> Xaa can be any naturally occurring amino acid
<400> 25
   Cys Xaa Xaa Cys Xaa Xaa Xaa Xaa Xaa Xaa Xaa Xaa Xaa Xaa Xaa Cys
   1               5                   10                  15
   Xaa His Xaa Xaa Cys Xaa Xaa Cys Xaa Xaa Xaa Xaa Xaa Xaa Xaa Xaa
               20                  25                  30
   Xaa Xaa Cys Xaa Xaa Cys
           35
<210> 26
   <211> 345
   <212> PRT
   <213> Yarrowia lipolytica
<400> 26
   Met Trp Gly Ser Ser His Ala Phe Ala Gly Glu Ser Asp Leu Thr Leu
   1               5                   10                  15
   Gln Leu His Thr Arg Ser Asn Met Ser Asp Asn Thr Thr Ile Lys Lys
               20                  25                  30
   Pro Ile Arg Pro Lys Pro Ile Arg Thr Glu Arg Leu Pro Tyr Ala Gly
           35                  40                  45
   Ala Ala Glu Ile Ile Arg Ala Asn Gln Lys Asp His Tyr Phe Glu Ser
       50                  55                  60
   Val Leu Glu Gln His Leu Val Thr Phe Leu Gln Lys Trp Lys Gly Val
   65                  70                  75                  80
   Arg Phe Ile His Gln Tyr Lys Glu Glu Leu Glu Thr Ala Ser Lys Phe
                   85                  90                  95
   Ala Tyr Leu Gly Leu Cys Thr Leu Val Gly Ser Lys Thr Leu Gly Glu
               100                 105                 110
   Glu Tyr Thr Asn Leu Met Tyr Thr Ile Arg Asp Arg Thr Ala Leu Pro
           115                 120                 125
   Gly Val Val Arg Arg Phe Gly Tyr Val Leu Ser Asn Thr Leu Phe Pro
       130                 135                 140
   Tyr Leu Phe Val Arg Tyr Met Gly Lys Leu Arg Ala Lys Leu Met Arg
   145                 150                 155                 160
   Glu Tyr Pro His Leu Val Glu Tyr Asp Glu Asp Glu Pro Val Pro Ser
                   165                 170                 175
   Pro Glu Thr Trp Lys Glu Arg Val Ile Lys Thr Phe Val Asn Lys Phe
               180                 185                 190
   Asp Lys Phe Thr Ala Leu Glu Gly Phe Thr Ala Ile His Leu Ala Ile
           195                 200                 205
   Phe Tyr Val Tyr Gly Ser Tyr Tyr Gln Leu Ser Lys Arg Ile Trp Gly
       210                 215                 220
   Met Arg Tyr Val Phe Gly His Arg Leu Asp Lys Asn Glu Pro Arg Ile
   225                 230                 235                 240
   Gly Tyr Glu Met Leu Gly Leu Leu Ile Phe Ala Arg Phe Ala Thr Ser
                   245                 250                 255
   Phe Val Gln Thr Gly Arg Glu Tyr Leu Gly Ala Leu Leu Glu Lys Ser
               260                 265                 270
   Val Glu Lys Glu Ala Gly Glu Lys Glu Asp Glu Lys Glu Ala Val Val
           275                 280                 285
   Pro Lys Lys Lys Ser Ser Ile Pro Phe Ile Glu Asp Thr Glu Gly Glu
       290                 295                 300
   Thr Glu Asp Lys Ile Asp Leu Glu Asp Pro Arg Gln Leu Lys Phe Ile
   305                 310                 315                 320
   Pro Glu Ala Ser Arg Ala Cys Thr Leu Cys Leu Ser Tyr Ile Ser Ala
                   325                 330                 335
   Pro Ala Cys Thr Pro Cys Gly His Phe
               340                 345
<210> 27
   <211> 2987
   <212> DNA
   <213> Yarrowia lipolytica
<220>
   <221> misc_feature
   <223> mutant acetohydroxyacid synthase (AHAS) with W497L mutation
<300>
   <302> HIGH EICOSAPENTAENOIC ACID PRODUCING STRAINS OF YARROWIA LIPOLYTICA
<310> US 2006-0115881-A1
   <311> 2005-11-02
   <312> 2006-06-01
   <313> (1)..(2987)
<300>
   <302> HIGH EICOSAPENTAENOIC ACID PRODUCING STRAINS OF YARROWIA LIPOLYTICA
<310> WO 2006/052870
   <311> 2005-11-03
   <312> 2006-05-18
   <313> (1)..(2987)
<400> 27
   ttccctagtc ccagtgtaca cccgccgata tcgcttaccc tgcagccgga ttaaggttgg     60
   caatttttca cgtccttgtc tccgcaatta ctcaccgggt ggtttataag attgcaagcg    120
   tcttgatttg tctctgtata ctaacatgca atcgcgactc gcccgacggg ccactaacct    180
   ggccagaatc tccagatcca agtattctct tggtctgcga tatgtttcca acacaaaagc    240
   ccctgctgcc cagccggcaa ctgctgagtg agtattcctt gccataaacg acccagaacc    300
   actgtatagt gtttggaagc actagtcaga agaccagcga aaacaggtgg aaaaaactga 360
   gacgaaaagc aacgaccaga aatgtaatgt gtggaaaagc gacacacaca gagcagataa 420
   agaggtgaca aataacgaca aatgaaatat cagtatcttc ccacaatcac tacctctcag 480
   ctgtctgaag gtgcggctga tatatccatc ccacgtctaa cgtatggagt gtgatagaat 540
   atgacgacac aagcatgaga actcgctctc tatccaacca ccgaaacact gtcactacag 600
   ccgttcttgt tgctccattc gcttttgtga ttccatgcct tctctggtga ctgacaacat 660
   tccttccttt tctccagccc tgttgttatc tgctcatgac ctacggccac tctctatcgc 720
   atactaacat agacgatccc agcccgctcc ccacttccag ggcaccgttg gcaagcctcc 780
   tatcctcaag aaggctgagg ctgccaacgc tgacatggac gagtccttca tcggaatgtc 840
   tggaggagag atcttccacg agatgatgct gcgacacaac gtcgacactg tcttcggtta 900
   ccccggtgga gccattctcc ccgtctttga cgccattcac aactctgagt acttcaactt 960
   tgtgctccct cgacacgagc agggtgccgg ccacatggcc gagggctacg ctcgagcctc 1020
   tggtaagccc ggtgtcgttc tcgtcacctc tggccccggt gccaccaacg tcatcacccc 1080
   catgcaggac gctctttccg atggtacccc catggttgtc ttcaccggtc aggtcctgac 1140
   ctccgttatc ggcactgacg ccttccagga ggccgatgtt gtcggcatct cccgatcttg 1200
   caccaagtgg aacgtcatgg tcaagaacgt tgctgagctc ccccgacgaa tcaacgaggc 1260
   ctttgagatt gctacttccg gccgacccgg tcccgttctc gtcgatctgc ccaaggatgt 1320
   tactgctgcc atcctgcgag agcccatccc caccaagtcc accattccct cgcattctct 1380
   gaccaacctc acctctgccg ccgccaccga gttccagaag caggctatcc agcgagccgc 1440
   caacctcatc aaccagtcca agaagcccgt cctttacgtc ggacagggta tccttggctc 1500
   cgaggagggt cctaagctgc ttaaggagct ggctgagaag gccgagattc ccgtcaccac 1560
   tactctgcag ggtcttggtg cctttgacga gcgagacccc aagtctctgc acatgctcgg 1620
   tatgcacggt tccggctacg ccaacatggc catgcagaac gctgactgta tcattgctct 1680
   cggcgcccga tttgatgacc gagttaccgg ctccatcccc aagtttgccc ccgaggctcg 1740
   agccgctgcc cttgagggtc gaggtggtat tgttcacttt gagatccagg ccaagaacat 1800
   caacaaggtt gttcaggcca ccgaagccgt tgagggagac gttaccgagt ctgtccgaca 1860
   gctcatcccc ctcatcaaca aggtctctgc cgctgagcga gctccctgga ctgagactat 1920
   ccagtcctgg aagcagcagt tccccttcct cttcgaggct gaaggtgagg atggtgttat 1980
   caagccccag tccgtcattg ctctgctctc tgacctgaca gagaacaaca aggacaagac 2040
   catcatcacc accggtgttg gtcagcatca gatgtggact gcccagcatt tccgatggcg 2100
   acaccctcga accatgatca cttctggtgg tcttggaact atgggttacg gcctgcccgc 2160
   cgctatcggc gccaaggttg cccgacctga ctgcgacgtc attgacatcg atggtgacgc 2220
   ttctttcaac atgactctga ccgagctgtc caccgccgtt cagttcaaca ttggcgtcaa 2280
   ggctattgtc ctcaacaacg aggaacaggg tatggtcacc cagctgcagt ctctcttcta 2340
   cgagaaccga tactgccaca ctcatcagaa gaaccccgac ttcatgaagc tggccgagtc 2400
   catgggcatg aagggtatcc gaatcactca cattgaccag ctggaggccg gtctcaagga 2460
   gatgctcgca tacaagggcc ctgtgctcgt tgaggttgtt gtcgacaaga agatccccgt 2520
   tcttcccatg gttcccgctg gtaaggcttt gcatgagttc cttgtctacg acgctgacgc 2580
   cgaggctgct tctcgacccg atcgactgaa gaatgccccc gcccctcacg tccaccagac 2640
   cacctttgag aactaagtgg aaaggaacac aagcaatccg aaccaaaaat aattggggtc 2700
   ccgtgcccac agagtctagt gcagacctaa aatgaccaca gtaaattata gctgttatta 2760
   aacatgagat tttgaccaac aagagcgtag gaatgttatt agctactact tgtacataca 2820
   cagcatttgt tttaaataat gttgcctcca ggggcagtga gatcaggacc cagatccgtg 2880
   gccagctctc tgacttcaga ccgcttgtac ttaagcagct cgcaacactg ttgtcgagga 2940
   ttgaacttgc catattcgat tttgtggtca tgaatccagc acacctc 2987
<210> 28
   <211> 14688
   <212> DNA
   <213> Artificial Sequence
<220>
   <223> Plasmid pZKLeuN-29E3
<400> 28
   cgattgttgt ctactaacta tcgtacgata acttcgtata gcatacatta tacgaagtta      60
   tcgcgtcgac gagtatctgt ctgactcgtc attgccgcct ttggagtacg actccaacta     120
   tgagtgtgct tggatcactt tgacgataca ttcttcgttg gaggctgtgg gtctgacagc     180
   tgcgttttcg gcgcggttgg ccgacaacaa tatcagctgc aacgtcattg ctggctttca     240
   tcatgatcac atttttgtcg gcaaaggcga cgcccagaga gccattgacg ttctttctaa     300
   tttggaccga tagccgtata gtccagtcta tctataagtt caactaactc gtaactatta     360
   ccataacata tacttcactg ccccagataa ggttccgata aaaagttctg cagactaaat     420
   ttatttcagt ctcctcttca ccaccaaaat gccctcctac gaagctcgag ctaacgtcca     480
   caagtccgcc tttgccgctc gagtgctcaa gctcgtggca gccaagaaaa ccaacctgtg     540
   tgcttctctg gatgttacca ccaccaagga gctcattgag cttgccgata aggtcggacc     600
   ttatgtgtgc atgatcaaaa cccatatcga catcattgac gacttcacct acgccggcac     660
   tgtgctcccc ctcaaggaac ttgctcttaa gcacggtttc ttcctgttcg aggacagaaa 720
   gttcgcagat attggcaaca ctgtcaagca ccagtaccgg tgtcaccgaa tcgccgagtg 780
   gtccgatatc accaacgccc acggtgtacc cggaaccgga atcattgctg gcctgcgagc 840
   tggtgccgag gaaactgtct ctgaacagaa gaaggaggac gtctctgact acgagaactc 900
   ccagtacaag gagttcctag tcccctctcc caacgagaag ctggccagag gtctgctcat 960
   gctggccgag ctgtcttgca agggctctct ggccactggc gagtactcca agcagaccat 1020
   tgagcttgcc cgatccgacc ccgagtttgt ggttggcttc attgcccaga accgacctaa 1080
   gggcgactct gaggactggc ttattctgac cccgggggtg ggtcttgacg acaagggaga 1140
   cgctctcgga cagcagtacc gaactgttga ggatgtcatg tctaccggaa cggatatcat 1200
   aattgtcggc cgaggtctgt acggccagaa ccgagatcct attgaggagg ccaagcgata 1260
   ccagaaggct ggctgggagg cttaccagaa gattaactgt tagaggttag actatggata 1320
   tgtaatttaa ctgtgtatat agagagcgtg caagtatgga gcgcttgttc agcttgtatg 1380
   atggtcagac gacctgtctg atcgagtatg tatgatactg cacaacctgt gtatccgcat 1440
   gatctgtcca atggggcatg ttgttgtgtt tctcgatacg gagatgctgg gtacagtgct 1500
   aatacgttga actacttata cttatatgag gctcgaagaa agctgacttg tgtatgactt 1560
   attctcaact acatccccag tcacaatacc accactgcac taccactaca ccaaaaccat 1620
   gatcaaacca cccatggact tcctggaggc agaagaactt gttatggaaa agctcaagag 1680
   agagatcata acttcgtata gcatacatta tacgaagtta tcctgcaggt aaaggaattc 1740
   tggagtttct gagagaaaaa ggcaagatac gtatgtaaca aagcgacgca tggtacaata 1800
   ataccggagg catgtatcat agagagttag tggttcgatg atggcactgg tgcctggtat 1860
   gactttatac ggctgactac atatttgtcc tcagacatac aattacagtc aagcacttac 1920
   ccttggacat ctgtaggtac cccccggcca agacgatctc agcgtgtcgt atgtcggatt 1980
   ggcgtagctc cctcgctcgt caattggctc ccatctactt tcttctgctt ggctacaccc 2040
   agcatgtctg ctatggctcg ttttcgtgcc ttatctatcc tcccagtatt accaactcta 2100
   aatgacatga tgtgattggg tctacacttt catatcagag ataaggagta gcacagttgc 2160
   ataaaaagcc caactctaat cagcttcttc ctttcttgta attagtacaa aggtgattag 2220
   cgaaatctgg aagcttagtt ggccctaaaa aaatcaaaaa aagcaaaaaa cgaaaaacga 2280
   aaaaccacag ttttgagaac agggaggtaa cgaaggatcg tatatatata tatatatata 2340
   tatacccacg gatcccgaga ccggcctttg attcttccct acaaccaacc attctcacca 2400
   ccctaattca caaccatgga gtctggaccc atgcctgctg gcattccctt ccctgagtac 2460
   tatgacttct ttatggactg gaagactccc ctggccatcg ctgccaccta cactgctgcc 2520
   gtcggtctct tcaaccccaa ggttggcaag gtctcccgag tggttgccaa gtcggctaac 2580
   gcaaagcctg ccgagcgaac ccagtccgga gctgccatga ctgccttcgt ctttgtgcac 2640
   aacctcattc tgtgtgtcta ctctggcatc accttctact acatgtttcc tgctatggtc 2700
   aagaacttcc gaacccacac actgcacgaa gcctactgcg acacggatca gtccctctgg 2760
   aacaacgcac ttggctactg gggttacctc ttctacctgt ccaagttcta cgaggtcatt 2820
   gacaccatca tcatcatcct gaagggacga cggtcctcgc tgcttcagac ctaccaccat 2880
   gctggagcca tgattaccat gtggtctggc atcaactacc aagccactcc catttggatc 2940
   tttgtggtct tcaactcctt cattcacacc atcatgtact gttactatgc cttcacctct 3000
   atcggattcc atcctcctgg caaaaagtac ctgacttcga tgcagattac tcagtttctg 3060
   gtcggtatca ccattgccgt gtcctacctc ttcgttcctg gctgcatccg aacacccggt 3120
   gctcagatgg ctgtctggat caacgtcggc tacctgtttc ccttgaccta tctgttcgtg 3180
   gactttgcca agcgaaccta ctccaagcga tctgccattg ccgctcagaa aaaggctcag 3240
   taagcggccg cattgatgat tggaaacaca cacatgggtt atatctaggt gagagttagt 3300
   tggacagtta tatattaaat cagctatgcc aacggtaact tcattcatgt caacgaggaa 3360
   ccagtgactg caagtaatat agaatttgac caccttgcca ttctcttgca ctcctttact 3420
   atatctcatt tatttcttat atacaaatca cttcttcttc ccagcatcga gctcggaaac 3480
   ctcatgagca ataacatcgt ggatctcgtc aatagagggc tttttggact ccttgctgtt 3540
   ggccaccttg tccttgctgt ctggctcatt ctgtttcaac gccttttaat taacggagta 3600
   ggtctcggtg tcggaagcga cgccagatcc gtcatcctcc tttcgctctc caaagtagat 3660
   acctccgacg agctctcgga caatgatgaa gtcggtgccc tcaacgtttc ggatggggga 3720
   gagatcggcg agcttgggcg acagcagctg gcagggtcgc aggttggcgt acaggttcag 3780
   gtcctttcgc agcttgagga gaccctgctc gggtcgcacg tcggttcgtc cgtcgggagt 3840
   ggtccatacg gtgttggcag cgcctccgac agcaccgagc ataatagagt cagcctttcg 3900
   gcagatgtcg agagtagcgt cggtgatggg ctcgccctcc ttctcaatgg cagctcctcc 3960
   aatgagtcgg tcctcaaaca caaactcggt gccggaggcc tcagcaacag acttgagcac 4020
   cttgacggcc tcggcaatca cctcggggcc acagaagtcg ccgccgagaa gaacaatctt 4080
   cttggagtca gtcttggtct tcttagtttc gggttccatt gtggatgtgt gtggttgtat 4140
   gtgtgatgtg gtgtgtggag tgaaaatctg tggctggcaa acgctcttgt atatatacgc 4200
   acttttgccc gtgctatgtg gaagactaaa cctccgaaga ttgtgactca ggtagtgcgg 4260
   tatcggctag ggacccaaac cttgtcgatg ccgatagcat gcgacgtcgg gcccaattcg 4320
   ccctatagtg agtcgtatta caattcactg gccgtcgttt tacaacgtcg tgactgggaa 4380
   aaccctggcg ttacccaact taatcgcctt gcagcacatc cccctttcgc cagctggcgt 4440
   aatagcgaag aggcccgcac cgatcgccct tcccaacagt tgcgcagcct gaatggcgaa 4500
   tggacgcgcc ctgtagcggc gcattaagcg cggcgggtgt ggtggttacg cgcagcgtga 4560
   ccgctacact tgccagcgcc ctagcgcccg ctcctttcgc tttcttccct tcctttctcg 4620
   ccacgttcgc cggctttccc cgtcaagctc taaatcgggg gctcccttta gggttccgat 4680
   ttagtgcttt acggcacctc gaccccaaaa aacttgatta gggtgatggt tcacgtagtg 4740
   ggccatcgcc ctgatagacg gtttttcgcc ctttgacgtt ggagtccacg ttctttaata 4800
   gtggactctt gttccaaact ggaacaacac tcaaccctat ctcggtctat tcttttgatt 4860
   tataagggat tttgccgatt tcggcctatt ggttaaaaaa tgagctgatt taacaaaaat 4920
   ttaacgcgaa ttttaacaaa atattaacgc ttacaatttc ctgatgcggt attttctcct 4980
   tacgcatctg tgcggtattt cacaccgcat caggtggcac ttttcgggga aatgtgcgcg 5040
   gaacccctat ttgtttattt ttctaaatac attcaaatat gtatccgctc atgagacaat 5100
   aaccctgata aatgcttcaa taatattgaa aaaggaagag tatgagtatt caacatttcc 5160
   gtgtcgccct tattcccttt tttgcggcat tttgccttcc tgtttttgct cacccagaaa 5220
   cgctggtgaa agtaaaagat gctgaagatc agttgggtgc acgagtgggt tacatcgaac 5280
   tggatctcaa cagcggtaag atccttgaga gttttcgccc cgaagaacgt tttccaatga 5340
   tgagcacttt taaagttctg ctatgtggcg cggtattatc ccgtattgac gccgggcaag 5400
   agcaactcgg tcgccgcata cactattctc agaatgactt ggttgagtac tcaccagtca 5460
   cagaaaagca tcttacggat ggcatgacag taagagaatt atgcagtgct gccataacca 5520
   tgagtgataa cactgcggcc aacttacttc tgacaacgat cggaggaccg aaggagctaa 5580
   ccgctttttt gcacaacatg ggggatcatg taactcgcct tgatcgttgg gaaccggagc 5640
   tgaatgaagc cataccaaac gacgagcgtg acaccacgat gcctgtagca atggcaacaa 5700
   cgttgcgcaa actattaact ggcgaactac ttactctagc ttcccggcaa caattaatag 5760
   actggatgga ggcggataaa gttgcaggac cacttctgcg ctcggccctt ccggctggct 5820
   ggtttattgc tgataaatct ggagccggtg agcgtgggtc tcgcggtatc attgcagcac 5880
   tggggccaga tggtaagccc tcccgtatcg tagttatcta cacgacgggg agtcaggcaa 5940
   ctatggatga acgaaataga cagatcgctg agataggtgc ctcactgatt aagcattggt 6000
   aactgtcaga ccaagtttac tcatatatac tttagattga tttaaaactt catttttaat 6060
   ttaaaaggat ctaggtgaag atcctttttg ataatctcat gaccaaaatc ccttaacgtg 6120
   agttttcgtt ccactgagcg tcagaccccg tagaaaagat caaaggatct tcttgagatc 6180
   ctttttttct gcgcgtaatc tgctgcttgc aaacaaaaaa accaccgcta ccagcggtgg 6240
   tttgtttgcc ggatcaagag ctaccaactc tttttccgaa ggtaactggc ttcagcagag 6300
   cgcagatacc aaatactgtt cttctagtgt agccgtagtt aggccaccac ttcaagaact 6360
   ctgtagcacc gcctacatac ctcgctctgc taatcctgtt accagtggct gctgccagtg 6420
   gcgataagtc gtgtcttacc gggttggact caagacgata gttaccggat aaggcgcagc 6480
   ggtcgggctg aacggggggt tcgtgcacac agcccagctt ggagcgaacg acctacaccg 6540
   aactgagata cctacagcgt gagctatgag aaagcgccac gcttcccgaa gggagaaagg 6600
   cggacaggta tccggtaagc ggcagggtcg gaacaggaga gcgcacgagg gagcttccag 6660
   ggggaaacgc ctggtatctt tatagtcctg tcgggtttcg ccacctctga cttgagcgtc 6720
   gatttttgtg atgctcgtca ggggggcgga gcctatggaa aaacgccagc aacgcggcct 6780
   ttttacggtt cctggccttt tgctggcctt ttgctcacat gttctttcct gcgttatccc 6840
   ctgattctgt ggataaccgt attaccgcct ttgagtgagc tgataccgct cgccgcagcc 6900
   gaacgaccga gcgcagcgag tcagtgagcg aggaagcgga agagcgccca atacgcaaac 6960
   cgcctctccc cgcgcgttgg ccgattcatt aatgcagctg gcgcgcccac tgagctcgtc 7020
   taacggactt gatatacaac caattaaaac aaatgaaaag aaatacagtt ctttgtatca 7080
   tttgtaacaa ttaccctgta caaactaagg tattgaaatc ccacaatatt cccaaagtcc 7140
   acccctttcc aaattgtcat gcctacaact catataccaa gcactaacct accaaacacc 7200
   actaaaaccc cacaaaatat atcttaccga atatacagta acaagctacc accacactcg 7260
   ttgggtgcag tcgccagctt aaagatatct atccacatca gccacaactc ccttccttta 7320
   ataaaccgac tacacccttg gctattgagg ttatgagtga atatactgta gacaagacac 7380
   tttcaagaag actgtttcca aaacgtacca ctgtcctcca ctacaaacac acccaatctg 7440
   cttcttctag tcaaggttgc tacaccggta aattataaat catcatttca ttagcagggc 7500
   agggcccttt ttatagagtc ttatacacta gcggaccctg ccggtagacc aacccgcagg 7560
   cgcgtcagtt tgctccttcc atcaatgcgt cgtagaaacg acttactcct tcttgagcag 7620
   ctccttgacc ttgttggcaa caagtctccg acctcggagg tggaggaaga gcctccgata 7680
   tcggcggtag tgataccagc ctcgacggac tccttgacgg cagcctcaac agcgtcaccg 7740
   gcgggcttca tgttaagaga gaacttgagc atcatggcgg cagacagaat ggtggcgtac 7800
   gcaactaaca tgaatgaata cgatatacat caaagactat gatacgcagt attgcacact 7860
   gtacgagtaa gagcactagc cactgcactc aagtgaaacc gttgcccggg tacgagtatg 7920
   agtatgtaca gtatgtttag tattgtactt ggacagtgct tgtatcgtac attctcaagt 7980
   gtcaaacata aatatccgtt gctatatcct cgcaccacca cgtagctcgc tatatccctg 8040
   tgttgaatcc atccatcttg gattgccaat tgtgcacaca gaaccgggca ctcacttccc 8100
   catccacact tgcggccgct taagcaacgg gcttgataac agcggggggg gtgcccacgt 8160
   tgttgcggtt gcggaagaac agaacaccct taccagcacc ctcggcacca gcgctgggct 8220
   caacccactg gcacatacgc gcactgcggt acatggcgcg gatgaagcca cgaggaccat 8280
   cctggacatc agcccggtag tgcttgccca tgatgggctt aatggcctcg gtggcctcgt 8340
   ccgcgttgta gaaggggatg ctgctgacgt agtggtggag gacatgagtc tcgatgatgc 8400
   cgtggagaag gtggcggccg atgaagccca tctcacggtc aatggtagca gcggcaccac 8460
   ggacgaagtt ccactcgtcg ttggtgtagt ggggaagggt agggtcggtg tgctggagga 8520
   aggtgatggc aacgagccag tggttaaccc agaggtaggg aacaaagtac cagatggcca 8580
   tgttgtagaa accgaacttc tgaacgagga agtacagagc agtggccatc agaccgatac 8640
   caatatcgct gaggacgatg agcttagcgt cactgttctc gtacagaggg ctgcggggat 8700
   cgaagtggtt aacaccaccg ccgaggccgt tatgcttgcc cttgccgcga ccctcacgct 8760
   ggcgctcgtg gtagttgtgg ccggtaacat tggtgatgag gtagttgggc cagccaacga 8820
   gctgctgaag gacgagcatg agaagagtga aagcgggggt ctcctcagta agatgagcga 8880
   gctcgtgggt catctttccg agacgagtag cctgctgctc gcgggttcgg ggaacgaaga 8940
   ccatgtcacg ctccatgttg ccagtggcct tgtggtgctt tcggtgggag atttgccagc 9000
   tgaagtaggg gacaaggagg gaagagtgaa gaacccagcc agtaatgtcg ttgatgatgc 9060
   gagaatcgga gaaagcaccg tgaccgcact catgggcaat aacccagaga ccagtaccga 9120
   aaagaccctg aagaacggtg tacacggccc acagaccagc gcgggcgggg gtggagggga 9180
   tatattcggg ggtcacaaag ttgtaccaga tgctgaaagt ggtagtcagg aggacaatgt 9240
   cgcggaggat ataaccgtat cccttgagag cggagcgctt gaagcagtgc ttagggatgg 9300
   cattgtagat gtccttgatg gtaaagtcgg gaacctcgaa ctggttgccg taggtgtcga 9360
   gcatgacacc atactcggac ttgggcttgg cgatatcaac ctcggacatg gacgagagcg 9420
   atgtggaaga ggccgagtgg cggggagagt ctgaaggaga gacggcggca gactcagaat 9480
   ccgtcacagt agttgaggtg acggtgcgtc taagcgcagg gttctgcttg ggcagagccg 9540
   aagtggacgc catggttgat gtgtgtttaa ttcaagaatg aatatagaga agagaagaag 9600
   aaaaaagatt caattgagcc ggcgatgcag acccttatat aaatgttgcc ttggacagac 9660
   ggagcaagcc cgcccaaacc tacgttcggt ataatatgtt aagcttttta acacaaaggt 9720
   ttggcttggg gtaacctgat gtggtgcaaa agaccgggcg ttggcgagcc attgcgcggg 9780
   cgaatggggc cgtgactcgt ctcaaattcg agggcgtgcc tcaattcgtg cccccgtggc 9840
   tttttcccgc cgtttccgcc ccgtttgcac cactgcagcc gcttctttgg ttcggacacc 9900
   ttgctgcgag ctaggtgcct tgtgctactt aaaaagtggc ctcccaacac caacatgaca 9960
   tgagtgcgtg ggccaagaca cgttggcggg gtcgcagtcg gctcaatggc ccggaaaaaa 10020
   cgctgctgga gctggttcgg acgcagtccg ccgcggcgta tggatatccg caaggttcca 10080
   tagcgccatt gccctccgtc ggcgtctatc ccgcaacctc taaatagagc gggaatataa 10140
   cccaagcttc ttttttttcc tttaacacgc acacccccaa ctatcatgtt gctgctgctg 10200
   tttgactcta ctctgtggag gggtgctccc acccaaccca acctacaggt ggatccggcg 10260
   ctgtgattgg ctgataagtc tcctatccgg actaattctg accaatggga catgcgcgca 10320
   ggacccaaat gccgcaatta cgtaacccca acgaaatgcc tacccctctt tggagcccag 10380
   cggccccaaa tccccccaag cagcccggtt ctaccggctt ccatctccaa gcacaagcag 10440
   cccggttcta ccggcttcca tctccaagca cccctttctc cacaccccac aaaaagaccc 10500
   gtgcaggaca tcctactgcg tcgacatcat ttaaattcct tcacttcaag ttcattcttc 10560
   atctgcttct gttttacttt gacaggcaaa tgaagacatg gtacgacttg atggaggcca 10620
   agaacgccat ttcaccccga gacaccgaag tgcctgaaat cctggctgcc cccattgata 10680
   acatcggaaa ctacggtatt ccggaaagtg tatatagaac ctttccccag cttgtgtctg 10740
   tggatatgga tggtgtaatc ccctttgagt actcgtcttg gcttctctcc gagcagtatg 10800
   aggctctcta atctagcgca tttaatatct caatgtattt atatatttat cttctcatgc 10860
   ggccgctcac tgaatctttt tggctccctt gtgcttcctg acgatatacg tttgcacata 10920
   gaaattcaag aacaaacaca agactgtgcc aacataaaag taattgaaga accagccaaa 10980
   catcctcatc ccatcttggc gataacaggg aatgttcctg tacttccaga caatgtagaa 11040
   accaacattg aattgaatga tctgcattga tgtaatcagg gattttggca tggggaactt 11100
   cagcttgatc aatctggtcc aataataacc gtacatgatc cagtggatga aaccattcaa 11160
   cagcacaaaa atccaaacag cttcatttcg gtaattatag aacagccaca tatccatcgg 11220
   tgcccccaaa tgatggaaga attgcaacca ggtcagaggc ttgcccatca gtggcaaata 11280
   gaaggagtca atatactcca ggaacttgct caaatagaac aactgcgtgg tgatcctgaa 11340
   gacgttgttg tcaaaagcct tctcgcagtt gtcagacata acaccgatgg tgtacatggc 11400
   atatgccatt gagaggaatg atcccaacga ataaatggac atgagaaggt tgtaattggt 11460
   gaaaacaaac ttcatacgag actgaccttt tggaccaagg gggccaagag tgaacttcaa 11520
   gatgacaaat gcgatggaca agtaaagcac ctcacagtga ctggcatcac tccagagttg 11580
   ggcataatca actggttggg taaaacttcc tgcccaattg agactatttc attcaccacc 11640
   tccatggcca ttgctgtaga tatgtcttgt gtgtaagggg gttggggtgg ttgtttgtgt 11700
   tcttgacttt tgtgttagca agggaagacg ggcaaaaaag tgagtgtggt tgggagggag 11760
   agacgagcct tatatataat gcttgtttgt gtttgtgcaa gtggacgccg aaacgggcag 11820
   gagccaaact aaacaaggca gacaatgcga gcttaattgg attgcctgat gggcaggggt 11880
   tagggctcga tcaatggggg tgcgaagtga caaaattggg aattaggttc gcaagcaagg 11940
   ctgacaagac tttggcccaa acatttgtac gcggtggaca acaggagcca cccatcgtct 12000
   gtcacgggct agccggtcgt gcgtcctgtc aggctccacc taggctccat gccactccat 12060
   acaatcccac tagtgtaccg ctaggccgct tttagctccc atctaagacc cccccaaaac 12120
   ctccactgta cagtgcactg tactgtgtgg cgatcaaggg caagggaaaa aaggcgcaaa 12180
   catgcacgca tggaatgacg taggtaaggc gttactagac tgaaaagtgg cacatttcgg 12240
   cgtgccaaag ggtcctaggt gcgtttcgcg agctgggcgc caggccaagc cgctccaaaa 12300
   cgcctctccg actccctcca gcggcctcca tatccccatc cctctccaca gcaatgttgt 12360
   taagccttgc aaacgaaaaa atagaaaggc taataagctt ccaatattgt ggtgtacgct 12420
   gcataacgca acaatgagcg ccaaacaaca cacacacaca gcacacagca gcattaacca 12480
   cgatgaacag catgacatta caggtgggtg tgtaatcagg gccctgattg ctggtggtgg 12540
   gagcccccat catgggcaga tctgcgtaca ctgtttaaac agtgtacgca gatctactat 12600
   agaggaacat ttaaattgcc ccggagaaga cggccaggcc gcctagatga caaattcaac 12660
   aactcacagc tgactttctg ccattgccac tagggggggg cctttttata tggccaagcc 12720
   aagctctcca cgtcggttgg gctgcaccca acaataaatg ggtagggttg caccaacaaa 12780
   gggatgggat ggggggtaga agatacgagg ataacggggc tcaatggcac aaataagaac 12840
   gaatactgcc attaagactc gtgatccagc gactgacacc attgcatcat ctaagggcct 12900
   caaaactacc tcggaactgc tgcgctgatc tggacaccac agaggttccg agcactttag 12960
   gttgcaccaa atgtcccacc aggtgcaggc agaaaacgct ggaacagcgt gtacagtttg 13020
   tcttaacaaa aagtgagggc gctgaggtcg agcagggtgg tgtgacttgt tatagccttt 13080
   agagctgcga aagcgcgtat ggatttggct catcaggcca gattgagggt ctgtggacac 13140
   atgtcatgtt agtgtacttc aatcgccccc tggatatagc cccgacaata ggccgtggcc 13200
   tcattttttt gccttccgca catttccatt gctcgatacc cacaccttgc ttctcctgca 13260
   cttgccaacc ttaatactgg tttacattga ccaacatctt acaagcgggg ggcttgtcta 13320
   gggtatatat aaacagtggc tctcccaatc ggttgccagt ctcttttttc ctttctttcc 13380
   ccacagattc gaaatctaaa ctacacatca cagaattccg agccgtgagt atccacgaca 13440
   agatcagtgt cgagacgacg cgttttgtgt aatgacacaa tccgaaagtc gctagcaaca 13500
   cacactctct acacaaacta acccagctct ggtaccatgg aggtcgtgaa cgaaatcgtc  13560
   tccattggcc aggaggttct tcccaaggtc gactatgctc agctctggtc tgatgcctcg  13620
   cactgcgagg tgctgtacct ctccatcgcc ttcgtcatcc tgaagttcac ccttggtcct  13680
   ctcggaccca agggtcagtc tcgaatgaag tttgtgttca ccaactacaa cctgctcatg  13740
   tccatctact cgctgggctc cttcctctct atggcctacg ccatgtacac cattggtgtc  13800
   atgtccgaca actgcgagaa ggctttcgac aacaatgtct tccgaatcac cactcagctg  13860
   ttctacctca gcaagttcct cgagtacatt gactccttct atctgcccct catgggcaag  13920
   cctctgacct ggttgcagtt ctttcaccat ctcggagctc ctatggacat gtggctgttc  13980
   tacaactacc gaaacgaagc cgtttggatc tttgtgctgc tcaacggctt cattcactgg  14040
   atcatgtacg gctactattg gacccgactg atcaagctca agttccctat gcccaagtcc  14100
   ctgattactt ctatgcagat cattcagttc aacgttggct tctacatcgt ctggaagtac  14160
   cggaacattc cctgctaccg acaagatgga atgagaatgt ttggctggtt tttcaactac  14220
   ttctacgttg gtactgtcct gtgtctgttc ctcaacttct acgtgcagac ctacatcgtc  14280
   cgaaagcaca agggagccaa aaagattcag tgagcggccg catgtacata caagattatt  14340
   tatagaaatg aatcgcgatc gaacaaagag tacgagtgta cgagtagggg atgatgataa  14400
   aagtggaaga agttccgcat ctttggattt atcaacgtgt aggacgatac ttcctgtaaa  14460
   aatgcaatgt ctttaccata ggttctgctg tagatgttat taactaccat taacatgtct  14520
   acttgtacag ttgcagacca gttggagtat agaatggtac acttaccaaa aagtgttgat  14580
   ggttgtaact acgatatata aaactgttga cgggatcccc gctgatatgc ctaaggaaca  14640
   atcaaagagg aagatattaa ttcagaatgc tagtatacag ttagggat                14688
<210> 29
   <211> 1434
   <212> DNA
   <213> Fusarium monoliforme
<220>
   <221> CDS
   <222> (1)..(1434)
   <223> delta-12 desaturase
<300>
   <302> DELTA-12 DESATURASES SUITABLE FOR ALTERING LEVELS OF POLYUNSATURATED FATTY ACIDS IN OLEAGINOUS YEAST
<310> WO 2005/047485
   <311> 2004-11-12
   <312> 2005-05-26
   <313> (1)..(1434)
<300>
   <302> DELTA-12 DESATURASES SUITABLE FOR ALTERING LEVELS OF POLYUNSATURATED FATTY ACIDS IN OLEAGINOUS YEAST
<310> US 2005-0216975-A1
   <311> 2004-11-10
   <312> 2005-09-29
   <313> (1)..(1434)
<400> 29
   atg gcg tcc act tcg gct ctg ccc aag cag aac cct gcg ctt aga cgc      48
   Met Ala Ser Thr Ser Ala Leu Pro Lys Gln Asn Pro Ala Leu Arg Arg
   1               5                   10                  15
   acc gtc acc tca act act gtg acg gat tct gag tct gcc gcc gtc tct      96
   Thr Val Thr Ser Thr Thr Val Thr Asp Ser Glu Ser Ala Ala Val Ser
               20                  25                  30
   cct tca gac tct ccc cgc cac tcg gcc tct tcc aca tcg ctc tcg tcc     144
   Pro Ser Asp Ser Pro Arg His Ser Ala Ser Ser Thr Ser Leu Ser Ser
           35                  40                  45
   atg tcc gag gtt gat atc gcc aag ccc aag tcc gag tat ggt gtc atg     192
   Met Ser Glu Val Asp Ile Ala Lys Pro Lys Ser Glu Tyr Gly Val Met
       50                  55                  60
   ctc gac acc tac ggc aac cag ttc gag gtt ccc gac ttt acc atc aag     240
   Leu Asp Thr Tyr Gly Asn Gln Phe Glu Val Pro Asp Phe Thr Ile Lys
   65                  70                  75                  80
   gac atc tac aat gcc atc cct aag cac tgc ttc aag cgc tcc gct ctc     288
   Asp Ile Tyr Asn Ala Ile Pro Lys His Cys Phe Lys Arg Ser Ala Leu
                   85                  90                  95
   aag gga tac ggt tat atc ctc cgc gac att gtc ctc ctg act acc act     336
   Lys Gly Tyr Gly Tyr Ile Leu Arg Asp Ile Val Leu Leu Thr Thr Thr
               100                 105                 110
   ttc agc atc tgg tac aac ttt gtg acc ccc gaa tat atc ccc tcc acc     384
   Phe Ser Ile Trp Tyr Asn Phe Val Thr Pro Glu Tyr Ile Pro Ser Thr
           115                 120                 125
   ccc gcc cgc gct ggt ctg tgg gcc gtg tac acc gtt ctt cag ggt ctt     432
   Pro Ala Arg Ala Gly Leu Trp Ala Val Tyr Thr Val Leu Gln Gly Leu
       130                 135                 140
   ttc ggt act ggt ctc tgg gtt att gcc cat gag tgc ggt cac ggt gct     480
   Phe Gly Thr Gly Leu Trp Val Ile Ala His Glu Cys Gly His Gly Ala
   145                 150                 155                 160
   ttc tcc gat tct cgc atc atc aac gac att act ggc tgg gtt ctt cac     528
   Phe Ser Asp Ser Arg Ile Ile Asn Asp Ile Thr Gly Trp Val Leu His
                   165                 170                 175
   tct tcc ctc ctt gtc ccc tac ttc agc tgg caa atc tcc cac cga aag     576
   Ser Ser Leu Leu Val Pro Tyr Phe Ser Trp Gln Ile Ser His Arg Lys
               180                 185                 190
   cac cac aag gcc act ggc aac atg gag cgt gac atg gtc ttc gtt ccc     624
   His His Lys Ala Thr Gly Asn Met Glu Arg Asp Met Val Phe Val Pro
           195                 200                 205
   cga acc cgc gag cag cag gct act cgt ctc gga aag atg acc cac gag     672
   Arg Thr Arg Glu Gln Gln Ala Thr Arg Leu Gly Lys Met Thr His Glu
       210                 215                 220
   ctc gct cat ctt act gag gag acc ccc gct ttc act ctt ctc atg ctc     720
   Leu Ala His Leu Thr Glu Glu Thr Pro Ala Phe Thr Leu Leu Met Leu
   225                 230                 235                 240
   gtc ctt cag cag ctc gtt ggc tgg ccc aac tac ctc atc acc aat gtt     768
   Val Leu Gln Gln Leu Val Gly Trp Pro Asn Tyr Leu Ile Thr Asn Val
                   245                 250                 255
   acc ggc cac aac tac cac gag cgc cag cgt gag ggt cgc ggc aag ggc     816
   Thr Gly His Asn Tyr His Glu Arg Gln Arg Glu Gly Arg Gly Lys Gly
               260                 265                 270
   aag cat aac ggc ctc ggc ggt ggt gtt aac cac ttc gat ccc cgc agc     864
   Lys His Asn Gly Leu Gly Gly Gly Val Asn His Phe Asp Pro Arg Ser
           275                 280                 285
   cct ctg tac gag aac agt gac gct aag ctc atc gtc ctc agc gat att     912
   Pro Leu Tyr Glu Asn Ser Asp Ala Lys Leu Ile Val Leu Ser Asp Ile
       290                 295                 300
   ggt atc ggt ctg atg gcc act gct ctg tac ttc ctc gtt cag aag ttc     960
   Gly Ile Gly Leu Met Ala Thr Ala Leu Tyr Phe Leu Val Gln Lys Phe
   305                 310                 315                 320
   ggt ttc tac aac atg gcc atc tgg tac ttt gtt ccc tac ctc tgg gtt    1008
   Gly Phe Tyr Asn Met Ala Ile Trp Tyr Phe Val Pro Tyr Leu Trp Val
                   325                 330                 335
   aac cac tgg ctc gtt gcc atc acc ttc ctc cag cac acc gac cct acc    1056
   Asn His Trp Leu Val Ala Ile Thr Phe Leu Gln His Thr Asp Pro Thr
               340                 345                 350
   ctt ccc cac tac acc aac gac gag tgg aac ttc gtc cgt ggt gcc gct    1104
   Leu Pro His Tyr Thr Asn Asp Glu Trp Asn Phe Val Arg Gly Ala Ala
           355                 360                 365
   gct acc att gac cgt gag atg ggc ttc atc ggc cgc cac ctt ctc cac    1152
   Ala Thr Ile Asp Arg Glu Met Gly Phe Ile Gly Arg His Leu Leu His
       370                 375                 380
   ggc atc atc gag act cat gtc ctc cac cac tac gtc agc agc atc ccc    1200
   Gly Ile Ile Glu Thr His Val Leu His His Tyr Val Ser Ser Ile Pro
   385                 390                 395                 400
   ttc tac aac gcg gac gag gcc acc gag gcc att aag ccc atc atg ggc    1248
   Phe Tyr Asn Ala Asp Glu Ala Thr Glu Ala Ile Lys Pro Ile Met Gly
                   405                 410                 415
   aag cac tac cgg gct gat gtc cag gat ggt cct cgt ggc ttc atc cgc    1296
   Lys His Tyr Arg Ala Asp Val Gln Asp Gly Pro Arg Gly Phe Ile Arg
               420                 425                 430
   gcc atg tac cgc agt gcg cgt atg tgc cag tgg gtt gag ccc agc gct    1344
   Ala Met Tyr Arg Ser Ala Arg Met Cys Gln Trp Val Glu Pro Ser Ala
           435                 440                 445
   ggt gcc gag ggt gct ggt aag ggt gtt ctg ttc ttc cgc aac cgc aac    1392
   Gly Ala Glu Gly Ala Gly Lys Gly Val Leu Phe Phe Arg Asn Arg Asn
       450                 455                 460
   aac gtg ggc acc ccc ccc gct gtt atc aag ccc gtt gct taa            1434
   Asn Val Gly Thr Pro Pro Ala Val Ile Lys Pro Val Ala
   465                 470                 475
<210> 30
   <211> 477
   <212> PRT
   <213> Fusarium monoliforme
<400> 30
   Met Ala Ser Thr Ser Ala Leu Pro Lys Gln Asn Pro Ala Leu Arg Arg
   1               5                   10                  15
   Thr Val Thr Ser Thr Thr Val Thr Asp Ser Glu Ser Ala Ala Val Ser
               20                  25                  30
   Pro Ser Asp Ser Pro Arg His Ser Ala Ser Ser Thr Ser Leu Ser Ser
           35                  40                  45
   Met Ser Glu Val Asp Ile Ala Lys Pro Lys Ser Glu Tyr Gly Val Met
       50                  55                  60
   Leu Asp Thr Tyr Gly Asn Gln Phe Glu Val Pro Asp Phe Thr Ile Lys
   65                  70                  75                  80
   Asp Ile Tyr Asn Ala Ile Pro Lys His Cys Phe Lys Arg Ser Ala Leu
                   85                  90                  95
   Lys Gly Tyr Gly Tyr Ile Leu Arg Asp Ile Val Leu Leu Thr Thr Thr
               100                 105                 110
   Phe Ser Ile Trp Tyr Asn Phe Val Thr Pro Glu Tyr Ile Pro Ser Thr
           115                 120                 125
   Pro Ala Arg Ala Gly Leu Trp Ala Val Tyr Thr Val Leu Gln Gly Leu
       130                 135                 140
   Phe Gly Thr Gly Leu Trp Val Ile Ala His Glu Cys Gly His Gly Ala
   145                 150                 155                 160
   Phe Ser Asp Ser Arg Ile Ile Asn Asp Ile Thr Gly Trp Val Leu His
                   165                 170                 175
   Ser Ser Leu Leu Val Pro Tyr Phe Ser Trp Gln Ile Ser His Arg Lys
               180                 185                 190
   His His Lys Ala Thr Gly Asn Met Glu Arg Asp Met Val Phe Val Pro
   195 200 205
   Arg Thr Arg Glu Gln Gln Ala Thr Arg Leu Gly Lys Met Thr His Glu
   210 215 220
   Leu Ala His Leu Thr Glu Glu Thr Pro Ala Phe Thr Leu Leu Met Leu
   225 230 235 240
   Val Leu Gln Gln Leu Val Gly Trp Pro Asn Tyr Leu Ile Thr Asn Val
   245 250 255
   Thr Gly His Asn Tyr His Glu Arg Gln Arg Glu Gly Arg Gly Lys Gly
   260 265 270
   Lys His Asn Gly Leu Gly Gly Gly Val Asn His Phe Asp Pro Arg Ser
   275 280 285
   Pro Leu Tyr Glu Asn Ser Asp Ala Lys Leu Ile Val Leu Ser Asp Ile
   290 295 300
   Gly Ile Gly Leu Met Ala Thr Ala Leu Tyr Phe Leu Val Gln Lys Phe
   305 310 315 320
   Gly Phe Tyr Asn Met Ala Ile Trp Tyr Phe Val Pro Tyr Leu Trp Val
   325 330 335
   Asn His Trp Leu Val Ala Ile Thr Phe Leu Gln His Thr Asp Pro Thr
   340 345 350
   Leu Pro His Tyr Thr Asn Asp Glu Trp Asn Phe Val Arg Gly Ala Ala
   355 360 365
   Ala Thr Ile Asp Arg Glu Met Gly Phe Ile Gly Arg His Leu Leu His
   370 375 380
   Gly Ile Ile Glu Thr His Val Leu His His Tyr Val Ser Ser Ile Pro
   385 390 395 400
   Phe Tyr Asn Ala Asp Glu Ala Thr Glu Ala Ile Lys Pro Ile Met Gly
   405 410 415
   Lys His Tyr Arg Ala Asp Val Gln Asp Gly Pro Arg Gly Phe Ile Arg
   420 425 430
   Ala Met Tyr Arg Ser Ala Arg Met Cys Gln Trp Val Glu Pro Ser Ala
           435                 440                 445
   Gly Ala Glu Gly Ala Gly Lys Gly Val Leu Phe Phe Arg Asn Arg Asn
       450                 455                 460
   Asn Val Gly Thr Pro Pro Ala Val Ile Lys Pro Val Ala
   465                 470                 475
<210> 31
   <211> 777
   <212> DNA
   <213> Euglena gracilis
<220>
   <221> CDS
   <222> (1)..(777)
   <223> synthetic delta-9 elongase (codon-optimized for Yarrowia lipolytica)
<300>
   <302> DELTA-9 ELONGASES AND THEIR USE IN MAKING POLYUNSATURATED FATTY ACIDS
<310> WO 2007/061742
   <311> 2006-11-16
   <312> 2007-05-31
   <313> (1)..(777)
<300>
   <302> DELTA-9 ELONGASES AND THEIR USE IN MAKING POLYUNSATURATED FATTY ACIDS
<310> US-2007-0117190-A1
   <311> 2006-11-16
   <312> 2007-05-24
   <313> (1) .. (777)
<400> 31
   atg gag gtc gtg aac gaa atc gtc tcc att ggc cag gag gtt ctt ccc       48
   Met Glu Val Val Asn Glu Ile Val Ser Ile Gly Gln Glu Val Leu Pro
   1               5                   10                  15
   aag gtc gac tat gct cag ctc tgg tct gat gcc tcg cac tgc gag gtg       96
   Lys Val Asp Tyr Ala Gln Leu Trp Ser Asp Ala Ser His Cys Glu Val
               20                  25                  30
   ctg tac ctc tcc atc gcc ttc gtc atc ctg aag ttc acc ctt ggt cct      144
   Leu Tyr Leu Ser Ile Ala Phe Val Ile Leu Lys Phe Thr Leu Gly Pro
           35                  40                  45
   ctc gga ccc aag ggt cag tct cga atg aag ttt gtg ttc acc aac tac      192
   Leu Gly Pro Lys Gly Gln Ser Arg Met Lys Phe Val Phe Thr Asn Tyr
       50                  55                  60
   aac ctg ctc atg tcc atc tac tcg ctg ggc tcc ttc ctc tct atg gcc      240
   Asn Leu Leu Met Ser Ile Tyr Ser Leu Gly Ser Phe Leu Ser Met Ala
   65                  70                  75                  80
   tac gcc atg tac acc att ggt gtc atg tcc gac aac tgc gag aag gct      288
   Tyr Ala Met Tyr Thr Ile Gly Val Met Ser Asp Asn Cys Glu Lys Ala
                   85                  90                  95
   ttc gac aac aat gtc ttc cga atc acc act cag ctg ttc tac ctc agc      336
   Phe Asp Asn Asn Val Phe Arg Ile Thr Thr Gln Leu Phe Tyr Leu Ser
               100                 105                 110
   aag ttc ctc gag tac att gac tcc ttc tat ctg ccc ctc atg ggc aag      384
   Lys Phe Leu Glu Tyr Ile Asp Ser Phe Tyr Leu Pro Leu Met Gly Lys
           115                 120                 125
   cct ctg acc tgg ttg cag ttc ttt cac cat ctc gga gct cct atg gac      432
   Pro Leu Thr Trp Leu Gln Phe Phe His His Leu Gly Ala Pro Met Asp
       130                 135                 140
   atg tgg ctg ttc tac aac tac cga aac gaa gcc gtt tgg atc ttt gtg      480
   Met Trp Leu Phe Tyr Asn Tyr Arg Asn Glu Ala Val Trp Ile Phe Val
   145                 150                 155                 160
   ctg ctc aac ggc ttc att cac tgg atc atg tac ggc tac tat tgg acc      528
   Leu Leu Asn Gly Phe Ile His Trp Ile Met Tyr Gly Tyr Tyr Trp Thr
                   165                 170                 175
   cga ctg atc aag ctc aag ttc cct atg ccc aag tcc ctg att act tct      576
   Arg Leu Ile Lys Leu Lys Phe Pro Met Pro Lys Ser Leu Ile Thr Ser
               180                 185                 190
   atg cag atc att cag ttc aac gtt ggc ttc tac atc gtc tgg aag tac      624
   Met Gln Ile Ile Gln Phe Asn Val Gly Phe Tyr Ile Val Trp Lys Tyr
           195                 200                 205
   cgg aac att ccc tgc tac cga caa gat gga atg aga atg ttt ggc tgg      672
   Arg Asn Ile Pro Cys Tyr Arg Gln Asp Gly Met Arg Met Phe Gly Trp
       210                 215                 220
   ttt ttc aac tac ttc tac gtt ggt act gtc ctg tgt ctg ttc ctc aac      720
   Phe Phe Asn Tyr Phe Tyr Val Gly Thr Val Leu Cys Leu Phe Leu Asn
   225                 230                 235                 240
   ttc tac gtg cag acc tac atc gtc cga aag cac aag gga gcc aaa aag      768
   Phe Tyr Val Gln Thr Tyr Ile Val Arg Lys His Lys Gly Ala Lys Lys
                   245                 250                 255
   att cag tga                                                          777
   Ile Gln
<210> 32
   <211> 258
   <212> PRT
   <213> Euglena gracilis
<400> 32
   Met Glu Val Val Asn Glu Ile Val Ser Ile Gly Gln Glu Val Leu Pro
   1               5                   10                  15
   Lys Val Asp Tyr Ala Gln Leu Trp Ser Asp Ala Ser His Cys Glu Val
               20                  25                  30
   Leu Tyr Leu Ser Ile Ala Phe Val Ile Leu Lys Phe Thr Leu Gly Pro
           35                  40                  45
   Leu Gly Pro Lys Gly Gln Ser Arg Met Lys Phe Val Phe Thr Asn Tyr
       50                  55                  60
   Asn Leu Leu Met Ser Ile Tyr Ser Leu Gly Ser Phe Leu Ser Met Ala
   65                  70                  75                  80
   Tyr Ala Met Tyr Thr Ile Gly Val Met Ser Asp Asn Cys Glu Lys Ala
                   85                  90                  95
   Phe Asp Asn Asn Val Phe Arg Ile Thr Thr Gln Leu Phe Tyr Leu Ser
               100                 105                 110
   Lys Phe Leu Glu Tyr Ile Asp Ser Phe Tyr Leu Pro Leu Met Gly Lys
           115                 120                 125
   Pro Leu Thr Trp Leu Gln Phe Phe His His Leu Gly Ala Pro Met Asp
       130                 135                 140
   Met Trp Leu Phe Tyr Asn Tyr Arg Asn Glu Ala Val Trp Ile Phe Val
   145                 150                 155                 160
   Leu Leu Asn Gly Phe Ile His Trp Ile Met Tyr Gly Tyr Tyr Trp Thr
                   165                 170                 175
   Arg Leu Ile Lys Leu Lys Phe Pro Met Pro Lys Ser Leu Ile Thr Ser
               180                 185                 190
   Met Gln Ile Ile Gln Phe Asn Val Gly Phe Tyr Ile Val Trp Lys Tyr
           195                 200                 205
   Arg Asn Ile Pro Cys Tyr Arg Gln Asp Gly Met Arg Met Phe Gly Trp
       210                 215                 220
   Phe Phe Asn Tyr Phe Tyr Val Gly Thr Val Leu Cys Leu Phe Leu Asn
   225                 230                 235                 240
   Phe Tyr Val Gln Thr Tyr Ile Val Arg Lys His Lys Gly Ala Lys Lys
                   245                 250                 255
   Ile Gln
<210> 33
   <211> 34
   <212> DNA
   <213> Escherichia coli
<400> 33
   ataacttcgt ataatgtatg ctatacgaag ttat 34
<210> 34
   <211> 828
   <212> DNA
   <213> Mortierella alpina
<220>
   <221> CDS
   <222> (1)..(828)
   <223> synthetic C16/18 elongase (codon-optimized for Yarrowia lipolytica)
<300>
   <302> A MORTIERELLA ALPINA C16/18 FATTY ACID ELONGASE
<310> US 2007-0087420-A1
   <311> 2005-10-19
   <312> 2007-04-19
   <313> (1)..(828)
<300>
   <302> A MORTIERELLA ALPINA C16/18 FATTY ACID ELONGASE
<310> WO 2007/046817
   <311> 2005-11-04
   <312> 2007-04-26
   <313> (1)..(828)
<400> 34
   atg gag tct gga ccc atg cct gct ggc att ccc ttc cct gag tac tat      48
   Met Glu Ser Gly Pro Met Pro Ala Gly Ile Pro Phe Pro Glu Tyr Tyr
   1               5                   10                  15
   gac ttc ttt atg gac tgg aag act ccc ctg gcc atc gct gcc acc tac      96
   Asp Phe Phe Met Asp Trp Lys Thr Pro Leu Ala Ile Ala Ala Thr Tyr
               20                  25                  30
   act gct gcc gtc ggt ctc ttc aac ccc aag gtt ggc aag gtc tcc cga      144
   Thr Ala Ala Val Gly Leu Phe Asn Pro Lys Val Gly Lys Val Ser Arg
           35                  40                  45
   gtg gtt gcc aag tcg gct aac gca aag cct gcc gag cga acc cag tcc      192
   Val Val Ala Lys Ser Ala Asn Ala Lys Pro Ala Glu Arg Thr Gln Ser
       50                  55                  60
   gga gct gcc atg act gcc ttc gtc ttt gtg cac aac ctc att ctg tgt      240
   Gly Ala Ala Met Thr Ala Phe Val Phe Val His Asn Leu Ile Leu Cys
   65                  70                  75                  80
   gtc tac tct ggc atc acc ttc tac tac atg ttt cct gct atg gtc aag      288
   Val Tyr Ser Gly Ile Thr Phe Tyr Tyr Met Phe Pro Ala Met Val Lys
               85                  90                  95
   aac ttc cga acc cac aca ctg cac gaa gcc tac tgc gac acg gat cag      336
   Asn Phe Arg Thr His Thr Leu His Glu Ala Tyr Cys Asp Thr Asp Gln
               100                 105                 110
   tcc ctc tgg aac aac gca ctt ggc tac tgg ggt tac ctc ttc tac ctg      384
   Ser Leu Trp Asn Asn Ala Leu Gly Tyr Trp Gly Tyr Leu Phe Tyr Leu
           115                 120                 125
   tcc aag ttc tac gag gtc att gac acc atc atc atc atc ctg aag gga      432
   Ser Lys Phe Tyr Glu Val Ile Asp Thr Ile Ile Ile Ile Leu Lys Gly
       130                 135                 140
   cga cgg tcc tcg ctg ctt cag acc tac cac cat gct gga gcc atg att      480
   Arg Arg Ser Ser Leu Leu Gln Thr Tyr His His Ala Gly Ala Met Ile
   145                 150                 155                 160
   acc atg tgg tct ggc atc aac tac caa gcc act ccc att tgg atc ttt      528
   Thr Met Trp Ser Gly Ile Asn Tyr Gln Ala Thr Pro Ile Trp Ile Phe
                   165                 170                 175
   gtg gtc ttc aac tcc ttc att cac acc atc atg tac tgt tac tat gcc      576
   Val Val Phe Asn Ser Phe Ile His Thr Ile Met Tyr Cys Tyr Tyr Ala
               180                 185                 190
   ttc acc tct atc gga ttc cat cct cct ggc aaa aag tac ctg act tcg      624
   Phe Thr Ser Ile Gly Phe His Pro Pro Gly Lys Lys Tyr Leu Thr Ser
           195                 200                 205
   atg cag att act cag ttt ctg gtc ggt atc acc att gcc gtg tcc tac      672
   Met Gln Ile Thr Gln Phe Leu Val Gly Ile Thr Ile Ala Val Ser Tyr
       210                 215                 220
   ctc ttc gtt cct ggc tgc atc cga aca ccc ggt gct cag atg gct gtc      720
   Leu Phe Val Pro Gly Cys Ile Arg Thr Pro Gly Ala Gln Met Ala Val
   225                 230                 235                 240
   tgg atc aac gtc ggc tac ctg ttt ccc ttg acc tat ctg ttc gtg gac      768
   Trp Ile Asn Val Gly Tyr Leu Phe Pro Leu Thr Tyr Leu Phe Val Asp
                   245                 250                 255
   ttt gcc aag cga acc tac tcc aag cga tct gcc att gcc gct cag aaa      816
   Phe Ala Lys Arg Thr Tyr Ser Lys Arg Ser Ala Ile Ala Ala Gln Lys
               260                 265                 270
   aag gct cag taa                                                      828
   Lys Ala Gln
           275
<210> 35
   <211> 275
   <212> PRT
   <213> Mortierella alpina
<400> 35
   Met Glu Ser Gly Pro Met Pro Ala Gly Ile Pro Phe Pro Glu Tyr Tyr
   1               5                   10                  15
   Asp Phe Phe Met Asp Trp Lys Thr Pro Leu Ala Ile Ala Ala Thr Tyr
   20 25 30
   Thr Ala Ala Val Gly Leu Phe Asn Pro Lys Val Gly Lys Val Ser Arg
   35 40 45
   Val Val Ala Lys Ser Ala Asn Ala Lys Pro Ala Glu Arg Thr Gln Ser
   50 55 60
   Gly Ala Ala Met Thr Ala Phe Val Phe Val His Asn Leu Ile Leu Cys
   65 70 75 80
   Val Tyr Ser Gly Ile Thr Phe Tyr Tyr Met Phe Pro Ala Met Val Lys
   85 90 95
   Asn Phe Arg Thr His Thr Leu His Glu Ala Tyr Cys Asp Thr Asp Gln
   100 105 110
   Ser Leu Trp Asn Asn Ala Leu Gly Tyr Trp Gly Tyr Leu Phe Tyr Leu
   115 120 125
   Ser Lys Phe Tyr Glu Val Ile Asp Thr Ile Ile Ile Ile Leu Lys Gly
   130 135 140
   Arg Arg Ser Ser Leu Leu Gln Thr Tyr His His Ala Gly Ala Met Ile
   145 150 155 160
   Thr Met Trp Ser Gly Ile Asn Tyr Gln Ala Thr Pro Ile Trp Ile Phe
   165 170 175
   Val Val Phe Asn Ser Phe Ile His Thr Ile Met Tyr Cys Tyr Tyr Ala
   180 185 190
   Phe Thr Ser Ile Gly Phe His Pro Pro Gly Lys Lys Tyr Leu Thr Ser
   195 200 205
   Met Gln Ile Thr Gln Phe Leu Val Gly Ile Thr Ile Ala Val Ser Tyr
   210 215 220
   Leu Phe Val Pro Gly Cys Ile Arg Thr Pro Gly Ala Gln Met Ala Val
   225 230 235 240
   Trp Ile Asn Val Gly Tyr Leu Phe Pro Leu Thr Tyr Leu Phe Val Asp
   245 250 255
   Phe Ala Lys Arg Thr Tyr Ser Lys Arg Ser Ala Ile Ala Ala Gln Lys
               260                 265                 270
   Lys Ala Gln
           275
<210> 36
   <211> 8739
   <212> DNA
   <213> Artificial Sequence
<220>
   <223> Plasmid pY116
<400> 36
   ggccgccacc gcggcccgag attccggcct cttcggccgc caagcgaccc gggtggacgt 60
   ctagaggtac ctagcaatta acagatagtt tgccggtgat aattctctta acctcccaca 120
   ctcctttgac ataacgattt atgtaacgaa actgaaattt gaccagatat tgtgtccgcg 180
   gtggagctcc agcttttgtt ccctttagtg agggtttaaa cgagcttggc gtaatcatgg 240
   tcatagctgt ttcctgtgtg aaattgttat ccgctcacaa ttccacacaa cgtacgagcc 300
   ggaagcataa agtgtaaagc ctggggtgcc taatgagtga gctaactcac attaattgcg 360
   ttgcgctcac tgcccgcttt ccagtcggga aacctgtcgt gccagctgca ttaatgaatc 420
   ggccaacgcg cggggagagg cggtttgcgt attgggcgct cttccgcttc ctcgctcact 480
   gactcgctgc gctcggtcgt tcggctgcgg cgagcggtat cagctcactc aaaggcggta 540
   atacggttat ccacagaatc aggggataac gcaggaaaga acatgtgagc aaaaggccag 600
   caaaaggcca ggaaccgtaa aaaggccgcg ttgctggcgt ttttccatag gctccgcccc 660
   cctgacgagc atcacaaaaa tcgacgctca agtcagaggt ggcgaaaccc gacaggacta 720
   taaagatacc aggcgtttcc ccctggaagc tccctcgtgc gctctcctgt tccgaccctg 780
   ccgcttaccg gatacctgtc cgcctttctc ccttcgggaa gcgtggcgct ttctcatagc 840
   tcacgctgta ggtatctcag ttcggtgtag gtcgttcgct ccaagctggg ctgtgtgcac 900
   gaaccccccg ttcagcccga ccgctgcgcc ttatccggta actatcgtct tgagtccaac 960
   ccggtaagac acgacttatc gccactggca gcagccactg gtaacaggat tagcagagcg 1020
   aggtatgtag gcggtgctac agagttcttg aagtggtggc ctaactacgg ctacactaga 1080
   aggacagtat ttggtatctg cgctctgctg aagccagtta ccttcggaaa aagagttggt 1140
   agctcttgat ccggcaaaca aaccaccgct ggtagcggtg gtttttttgt ttgcaagcag 1200
   cagattacgc gcagaaaaaa aggatctcaa gaagatcctt tgatcttttc tacggggtct 1260
   gacgctcagt ggaacgaaaa ctcacgttaa gggattttgg tcatgagatt atcaaaaagg 1320
   atcttcacct agatcctttt aaattaaaaa tgaagtttta aatcaatcta aagtatatat 1380
   gagtaaactt ggtctgacag ttaccaatgc ttaatcagtg aggcacctat ctcagcgatc 1440
   tgtctatttc gttcatccat agttgcctga ctccccgtcg tgtagataac tacgatacgg 1500
   gagggcttac catctggccc cagtgctgca atgataccgc gagacccacg ctcaccggct 1560
   ccagatttat cagcaataaa ccagccagcc ggaagggccg agcgcagaag tggtcctgca 1620
   actttatccg cctccatcca gtctattaat tgttgccggg aagctagagt aagtagttcg 1680
   ccagttaata gtttgcgcaa cgttgttgcc attgctacag gcatcgtggt gtcacgctcg 1740
   tcgtttggta tggcttcatt cagctccggt tcccaacgat caaggcgagt tacatgatcc 1800
   cccatgttgt gcaaaaaagc ggttagctcc ttcggtcctc cgatcgttgt cagaagtaag 1860
   ttggccgcag tgttatcact catggttatg gcagcactgc ataattctct tactgtcatg 1920
   ccatccgtaa gatgcttttc tgtgactggt gagtactcaa ccaagtcatt ctgagaatag 1980
   tgtatgcggc gaccgagttg ctcttgcccg gcgtcaatac gggataatac cgcgccacat 2040
   agcagaactt taaaagtgct catcattgga aaacgttctt cggggcgaaa actctcaagg 2100
   atcttaccgc tgttgagatc cagttcgatg taacccactc gtgcacccaa ctgatcttca 2160
   gcatctttta ctttcaccag cgtttctggg tgagcaaaaa caggaaggca aaatgccgca 2220
   aaaaagggaa taagggcgac acggaaatgt tgaatactca tactcttcct ttttcaatat 2280
   tattgaagca tttatcaggg ttattgtctc atgagcggat acatatttga atgtatttag 2340
   aaaaataaac aaataggggt tccgcgcaca tttccccgaa aagtgccacc tgacgcgccc 2400
   tgtagcggcg cattaagcgc ggcgggtgtg gtggttacgc gcagcgtgac cgctacactt 2460
   gccagcgccc tagcgcccgc tcctttcgct ttcttccctt cctttctcgc cacgttcgcc 2520
   ggctttcccc gtcaagctct aaatcggggg ctccctttag ggttccgatt tagtgcttta 2580
   cggcacctcg accccaaaaa acttgattag ggtgatggtt cacgtagtgg gccatcgccc 2640
   tgatagacgg tttttcgccc tttgacgttg gagtccacgt tctttaatag tggactcttg 2700
   ttccaaactg gaacaacact caaccctatc tcggtctatt cttttgattt ataagggatt 2760
   ttgccgattt cggcctattg gttaaaaaat gagctgattt aacaaaaatt taacgcgaat 2820
   tttaacaaaa tattaacgct tacaatttcc attcgccatt caggctgcgc aactgttggg 2880
   aagggcgatc ggtgcgggcc tcttcgctat tacgccagct ggcgaaaggg ggatgtgctg 2940
   caaggcgatt aagttgggta acgccagggt tttcccagtc acgacgttgt aaaacgacgg 3000
   ccagtgaatt gtaatacgac tcactatagg gcgaattggg taccgggccc cccctcgagg 3060
   tcgatggtgt cgataagctt gatatcgaat tcatgtcaca caaaccgatc ttcgcctcaa 3120
   ggaaacctaa ttctacatcc gagagactgc cgagatccag tctacactga ttaattttcg 3180
   ggccaataat ttaaaaaaat cgtgttatat aatattatat gtattatata tatacatcat 3240
   gatgatactg acagtcatgt cccattgcta aatagacaga ctccatctgc cgcctccaac 3300
   tgatgttctc aatatttaag gggtcatctc gcattgttta ataataaaca gactccatct 3360
   accgcctcca aatgatgttc tcaaaatata ttgtatgaac ttatttttat tacttagtat 3420
   tattagacaa cttacttgct ttatgaaaaa cacttcctat ttaggaaaca atttataatg 3480
   gcagttcgtt catttaacaa tttatgtaga ataaatgtta taaatgcgta tgggaaatct 3540
   taaatatgga tagcataaat gatatctgca ttgcctaatt cgaaatcaac agcaacgaaa 3600
   aaaatccctt gtacaacata aatagtcatc gagaaatatc aactatcaaa gaacagctat 3660
   tcacacgtta ctattgagat tattattgga cgagaatcac acactcaact gtctttctct 3720
   cttctagaaa tacaggtaca agtatgtact attctcattg ttcatacttc tagtcatttc 3780
   atcccacata ttccttggat ttctctccaa tgaatgacat tctatcttgc aaattcaaca 3840
   attataataa gatataccaa agtagcggta tagtggcaat caaaaagctt ctctggtgtg 3900
   cttctcgtat ttatttttat tctaatgatc cattaaaggt atatatttat ttcttgttat 3960
   ataatccttt tgtttattac atgggctgga tacataaagg tattttgatt taattttttg 4020
   cttaaattca atcccccctc gttcagtgtc aactgtaatg gtaggaaatt accatacttt 4080
   tgaagaagca aaaaaaatga aagaaaaaaa aaatcgtatt tccaggttag acgttccgca 4140
   gaatctagaa tgcggtatgc ggtacattgt tcttcgaacg taaaagttgc gctccctgag 4200
   atattgtaca tttttgcttt tacaagtaca agtacatcgt acaactatgt actactgttg 4260
   atgcatccac aacagtttgt tttgtttttt tttgtttttt ttttttctaa tgattcatta 4320
   ccgctatgta tacctacttg tacttgtagt aagccgggtt attggcgttc aattaatcat 4380
   agacttatga atctgcacgg tgtgcgctgc gagttacttt tagcttatgc atgctacttg 4440
   ggtgtaatat tgggatctgt tcggaaatca acggatgctc aaccgatttc gacagtaatt 4500
   aattaatttg aatcgaatcg gagcctaaaa tgaacccgag tatatctcat aaaattctcg 4560
   gtgagaggtc tgtgactgtc agtacaaggt gccttcatta tgccctcaac cttaccatac 4620
   ctcactgaat gtagtgtacc tctaaaaatg aaatacagtg ccaaaagcca aggcactgag 4680
   ctcgtctaac ggacttgata tacaaccaat taaaacaaat gaaaagaaat acagttcttt 4740
   gtatcatttg taacaattac cctgtacaaa ctaaggtatt gaaatcccac aatattccca 4800
   aagtccaccc ctttccaaat tgtcatgcct acaactcata taccaagcac taacctacca 4860
   aacaccacta aaaccccaca aaatatatct taccgaatat acagtaacaa gctaccacca 4920
   cactcgttgg gtgcagtcgc cagcttaaag atatctatcc acatcagcca caactccctt 4980
   cctttaataa accgactaca cccttggcta ttgaggttat gagtgaatat actgtagaca 5040
   agacactttc aagaagactg tttccaaaac gtaccactgt cctccactac aaacacaccc 5100
   aatctgcttc ttctagtcaa ggttgctaca ccggtaaatt ataaatcatc atttcattag 5160
   cagggcaggg ccctttttat agagtcttat acactagcgg accctgccgg tagaccaacc 5220
   cgcaggcgcg tcagtttgct ccttccatca atgcgtcgta gaaacgactt actccttctt 5280
   gagcagctcc ttgaccttgt tggcaacaag tctccgacct cggaggtgga ggaagagcct 5340
   ccgatatcgg cggtagtgat accagcctcg acggactcct tgacggcagc ctcaacagcg 5400
   tcaccggcgg gcttcatgtt aagagagaac ttgagcatca tggcggcaga cagaatggtg 5460
   gcaatggggt tgaccttctg cttgccgaga tcgggggcag atccgtgaca gggctcgtac 5520
   agaccgaacg cctcgttggt gtcgggcaga gaagccagag aggcggaggg cagcagaccc 5580
   agagaaccgg ggatgacgga ggcctcgtcg gagatgatat cgccaaacat gttggtggtg 5640
   atgatgatac cattcatctt ggagggctgc ttgatgagga tcatggcggc cgagtcgatc 5700
   agctggtggt tgagctcgag ctgggggaat tcgtccttga ggactcgagt gacagtcttt 5760
   cgccaaagtc gagaggaggc cagcacgttg gccttgtcaa gagaccacac gggaagaggg 5820
   gggttgtgct gaagggccag gaaggcggcc attcgggcaa ttcgctcaac ctcaggaacg 5880
   gagtaggtct cggtgtcgga agcgacgcca gatccgtcat cctcctttcg ctctccaaag 5940
   tagatacctc cgacgagctc tcggacaatg atgaagtcgg tgccctcaac gtttcggatg 6000
   ggggagagat cggcgagctt gggcgacagc agctggcagg gtcgcaggtt ggcgtacagg 6060
   ttcaggtcct ttcgcagctt gaggagaccc tgctcgggtc gcacgtcggt tcgtccgtcg 6120
   ggagtggtcc atacggtgtt ggcagcgcct ccgacagcac cgagcataat agagtcagcc 6180
   tttcggcaga tgtcgagagt agcgtcggtg atgggctcgc cctccttctc aatggcagct 6240
   cctccaatga gtcggtcctc aaacacaaac tcggtgccgg aggcctcagc aacagacttg 6300
   agcaccttga cggcctcggc aatcacctcg gggccacaga agtcgccgcc gagaagaaca 6360
   atcttcttgg agtcagtctt ggtcttctta gtttcgggtt ccattgtgga tgtgtgtggt 6420
   tgtatgtgtg atgtggtgtg tggagtgaaa atctgtggct ggcaaacgct cttgtatata 6480
   tacgcacttt tgcccgtgct atgtggaaga ctaaacctcc gaagattgtg actcaggtag 6540
   tgcggtatcg gctagggacc caaaccttgt cgatgccgat agcgctatcg aacgtacccc 6600
   agccggccgg gagtatgtcg gaggggacat acgagatcgt caagggtttg tggccaactg 6660
   gtatttaaat gtagctaacg gtagcaggcg aactactggt acatacctcc cccggaatat 6720
   gtacaggcat aatgcgtatc tgtgggacat gtggtcgttg cgccattatg taagcagcgt 6780
   gtactcctct gactgtccat atggtttgct ccatctcacc ctcatcgttt tcattgttca 6840
   caggcggcca caaaaaaact gtcttctctc cttctctctt cgccttagtc tactcggacc 6900
   agttttagtt tagcttggcg ccactggata aatgagacct caggccttgt gatgaggagg 6960
   tcacttatga agcatgttag gaggtgcttg tatggataga gaagcaccca aaataataag 7020
   aataataata aaacaggggg cgttgtcatt tcatatcgtg ttttcaccat caatacacct 7080
   ccaaacaatg cccttcatgt ggccagcccc aatattgtcc tgtagttcaa ctctatgcag 7140
   ctcgtatctt attgagcaag taaaactctg tcagccgata ttgcccgacc cgcgacaagg 7200
   gtcaacaagg tggtgtaagg ccttcgcaga agtcaaaact gtgccaaaca aacatctaga 7260
   gtctctttgg tgtttctcgc atatatttwa tcggctgtct tacgtatttg cgcctcggta 7320
   ccggactaat ttcggatcat ccccaatacg ctttttcttc gcagctgtca acagtgtcca 7380
   tgatctatcc acctaaatgg gtcatatgag gcgtataatt tcgtggtgct gataataatt 7440
   cccatatatt tgacacaaaa cttccccccc tagacataca tctcacaatc tcacttcttg 7500
   tgcttctgtc acacatctcc tccagctgac ttcaactcac acctctgccc cagttggtct 7560
   acagcggtat aaggtttctc cgcatagagg tgcaccactc ctcccgatac ttgtttgtgt 7620
   gacttgtggg tcacgacata tatatctaca cacattgcgc caccctttgg ttcttccagc 7680
   acaacaaaaa cacgacacgc taaccatggc caatttactg accgtacacc aaaatttgcc 7740
   tgcattaccg gtcgatgcaa cgagtgatga ggttcgcaag aacctgatgg acatgttcag 7800
   ggatcgccag gcgttttctg agcatacctg gaaaatgctt ctgtccgttt gccggtcgtg 7860
   ggcggcatgg tgcaagttga ataaccggaa atggtttccc gcagaacctg aagatgttcg 7920
   cgattatctt ctatatcttc aggcgcgcgg tctggcagta aaaactatcc agcaacattt 7980
   gggccagcta aacatgcttc atcgtcggtc cgggctgcca cgaccaagtg acagcaatgc 8040
   tgtttcactg gttatgcggc ggatccgaaa agaaaacgtt gatgccggtg aacgtgcaaa 8100
   acaggctcta gcgttcgaac gcactgattt cgaccaggtt cgttcactca tggaaaatag 8160
   cgatcgctgc caggatatac gtaatctggc atttctgggg attgcttata acaccctgtt 8220
   acgtatagcc gaaattgcca ggatcagggt taaagatatc tcacgtactg acggtgggag 8280
   aatgttaatc catattggca gaacgaaaac gctggttagc accgcaggtg tagagaaggc 8340
   acttagcctg ggggtaacta aactggtcga gcgatggatt tccgtctctg gtgtagctga 8400
   tgatccgaat aactacctgt tttgccgggt cagaaaaaat ggtgttgccg cgccatctgc 8460
   caccagccag ctatcaactc gcgccctgga agggattttt gaagcaactc atcgattgat 8520
   ttacggcgct aaggatgact ctggtcagag atacctggcc tggtctggac acagtgcccg 8580
   tgtcggagcc gcgcgagata tggcccgcgc tggagtttca ataccggaga tcatgcaagc 8640
   tggtggctgg accaatgtaa atattgtcat gaactatatc cgtaacctgg atagtgaaac 8700
   aggggcaatg gtgcgcctgc tggaagatgg cgattaagc 8739
<210> 37
   <211> 15337
   <212> DNA
   <213> Artificial Sequence
<220>
   <223> Plasmid pK02UF8289
<400> 37
   cgatcgagga agaggacaag cggctgcttc ttaagtttgt gacatcagta tccaaggcac     60
   cattgcaagg attcaaggct ttgaacccgt catttgccat tcgtaacgct ggtagacagg    120
   ttgatcggtt ccctacggcc tccacctgtg tcaatcttct caagctgcct gactatcagg    180
   acattgatca acttcggaag aaacttttgt atgccattcg atcacatgct ggtttcgatt    240
   tgtcttagag gaacgcatat acagtaatca tagagaataa acgatattca tttattaaag    300
   tagatagttg aggtagaagt tgtaaagagt gataaatagc ggccgctcac tgaatctttt    360
   tggctccctt gtgcttcctg acgatatacg tttgcacata gaaattcaag aacaaacaca    420
   agactgtgcc aacataaaag taattgaaga accagccaaa catcctcatc ccatcttggc    480
   gataacaggg aatgttcctg tacttccaga caatgtagaa accaacattg aattgaatga    540
   tctgcattga tgtaatcagg gattttggca tggggaactt cagcttgatc aatctggtcc    600
   aataataacc gtacatgatc cagtggatga aaccattcaa cagcacaaaa atccaaacag    660
   cttcatttcg gtaattatag aacagccaca tatccatcgg tgcccccaaa tgatggaaga    720
   attgcaacca ggtcagaggc ttgcccatca gtggcaaata gaaggagtca atatactcca    780
   ggaacttgct caaatagaac aactgcgtgg tgatcctgaa gacgttgttg tcaaaagcct    840
   tctcgcagtt gtcagacata acaccgatgg tgtacatggc atatgccatt gagaggaatg    900
   atcccaacga ataaatggac atgagaaggt tgtaattggt gaaaacaaac ttcatacgag    960
   actgaccttt tggaccaagg gggccaagag tgaacttcaa gatgacaaat gcgatggaca   1020
   agtaaagcac ctcacagtga ctggcatcac tccagagttg ggcataatca actggttggg   1080
   taaaacttcc tgcccaattg agactatttc attcaccacc tccatggtta gcgtgtcgtg   1140
   tttttgttgt gctggaagaa ccaaagggtg gcgcaatgtg tgtagatata tatgtcgtga   1200
   cccacaagtc acacaaacaa gtatcgggag gagtggtgca cctctatgcg gagaaacctt   1260
   ataccgctgt agaccaactg gggcagaggt gtgagttgaa gtcagctgga ggagatgtgt   1320
   gacagaagca caagaagtga gattgtgaga tgtatgtcta gggggggaag ttttgtgtca  1380
   aatatatggg aattattatc agcaccacga aattatacgc ctcatatgac ccatttaggt   1440
   ggatagatca tggacactgt tgacagctgc gaagaaaaag cgtattgggg atgatccgaa 1500
   attagtccgg taccgaggcg caaatacgta agacagccga twaaatatat gcgagaaaca 1560
   ccaaagagac tctagatgtt tgtttggcac agttttgact tctgcgaagg ccttacacca 1620
   ccttgttgac ccttgtcgcg ggtcgggcaa tatcggctga cagagtttta cttgctcaat 1680
   aagatacgag ctgcatagag ttgaactaca ggacaatatt ggggctggcc acatgaaggg 1740
   cattgtttgg aggtgtattg atggtgaaaa cacgatatga aatgacaacg ccccctgttt 1800
   tattattatt cttattattt tgggtgcttc tctatccata caagcacctc ctaacatgct 1860
   tcataagtga cctcctcatc acaaggcctg aggtctcatt tatccagtgg cgccaagcta 1920
   aactaaaact ggtccgagta gactaaggcg aagagagaag gagagaagac agttttttgg 1980
   tggccgcctg tgaacaatga aaacgatgag ggtgagatgg agcaaaccat atggtttaaa 2040
   cagtcagagg agtacacgct gcttacataa tggcgcaacg accacatgtc ccacagatac 2100
   gcatcgattc gattcaaatt aattaaaagg cgttgaaaca gaatgagcca gacagcaagg 2160
   acaaggtggc caacagcaag gagtccaaaa agccctctat tgacgagatc cacgatgtta 2220
   ttgctcatga ggtttccgag ctcgatgctg ggaagaagaa gtgatttgta tataagaaat 2280
   aaatgagata tagtaaagga gtgcaagaga atggcaaggt ggtcaaattc tatattactt 2340
   gcagtcactg gttcctcgtt gacatgaatg aagttaccgt tggcatagct gatttaatat 2400
   ataactgtcc aactaactct cacctagata taacccatgt gtgtgtttcc aatcatcaat 2460
   gcggccgctt actgagcctt ggcaccgggc tgcttctcgg ccattcgagc gaactgggac 2520
   aggtatcgga gcaggatgac gagaccttca tggggcagag ggtttcggta ggggaggttg 2580
   tgcttctggc acagctgttc cacctggtag gaaacggcag tgaggttgtg tcgaggcagg 2640
   gtgggccaga gatggtgctc gatctggtag ttcaggcctc caaagaacca gtcagtaatg 2700
   atgcctcgtc gaatgttcat ggtctcatgg atctgaccca cagagaagcc atgtccgtcc 2760
   cagacggaat caccgatctt ctccagaggg tagtggttca tgaagaccac gatggcaatt 2820
   ccgaagccac cgacgagctc ggaaacaaag aacaccagca tcgaggtcag gatggagggc 2880
   ataaagaaga ggtggaacag ggtcttgaga gtccagtgca gagcgagtcc aatggcctct 2940
   ttcttgtact gagatcggta gaactggttg tctcggtcct tgagggatcg aacggtcagc 3000
   acagactgga aacaccagat gaatcgcagg agaatacaga tgaccaggaa atagtactgt 3060
   tggaactgaa tgagctttcg ggagatggga gaagctcgag tgacatcgtc ctcggaccag 3120
   gcgagcagag gcaggttatc aatgtcggga tcgtgaccct gaacgttggt agcagaatga 3180
   tgggcgttgt gtctgtcctt ccaccaggtc acggagaagc cctggagtcc gttgccaaag 3240
   accagaccca ggacgttatt ccagtttcgg ttcttgaagg tctggtggtg gcagatgtca 3300
   tgagacagcc atcccatttg ctggtagtgc ataccgagca cgagagcacc aatgaagtac 3360
   aggtggtact ggaccagcat gaagaaggca agcacgccaa gacccagggt ggtcaagatc 3420
   ttgtacgagt accagagggg agaggcgtca aacatgccag tggcgatcag ctcttctcgg 3480
   agctttcgga aatcctcctg agcttcgttg acggcagcct ggggaggcag ctcggaagcc 3540
   tggttgatct tgggcattcg cttgagcttg tcgaaggctt cctgagagtg cataaccatg 3600
   aaggcgtcag tagcatctcg tccctggtag ttctcaatga tttcagctcc accagggtgg 3660
   aagttcaccc aagcggagac gtcgtacacc tttccgtcga tgacgagggg cagagcctgt 3720
   cgagaagcct tcaccatggc cattgctgta gatatgtctt gtgtgtaagg gggttggggt 3780
   ggttgtttgt gttcttgact tttgtgttag caagggaaga cgggcaaaaa agtgagtgtg 3840
   gttgggaggg agagacgagc cttatatata atgcttgttt gtgtttgtgc aagtggacgc 3900
   cgaaacgggc aggagccaaa ctaaacaagg cagacaatgc gagcttaatt ggattgcctg 3960
   atgggcaggg gttagggctc gatcaatggg ggtgcgaagt gacaaaattg ggaattaggt 4020
   tcgcaagcaa ggctgacaag actttggccc aaacatttgt acgcggtgga caacaggagc 4080
   cacccatcgt ctgtcacggg ctagccggtc gtgcgtcctg tcaggctcca cctaggctcc 4140
   atgccactcc atacaatccc actagtgtac cgctaggccg cttttagctc ccatctaaga 4200
   cccccccaaa acctccactg tacagtgcac tgtactgtgt ggcgatcaag ggcaagggaa 4260
   aaaaggcgca aacatgcacg catggaatga cgtaggtaag gcgttactag actgaaaagt 4320
   ggcacatttc ggcgtgccaa agggtcctag gtgcgtttcg cgagctgggc gccaggccaa 4380
   gccgctccaa aacgcctctc cgactccctc cagcggcctc catatcccca tccctctcca 4440
   cagcaatgtt gttaagcctt gcaaacgaaa aaatagaaag gctaataagc ttccaatatt 4500
   gtggtgtacg ctgcataacg caacaatgag cgccaaacaa cacacacaca cagcacacag 4560
   cagcattaac cacgatgttt aaacagtgta cgcagatccc gtcaacagtt ttatatatcg 4620
   tagttacaac catcaacact ttttggtaag tgtaccattc tatactccaa ctggtctgca 4680
   actgtacaag tagacatgtt aatggtagtt aataacatct acagcagaac ctatggtaaa 4740
   gacattgcat ttttacagga agtatcgtcc tacacgttga taaatccaaa gatgcggaac 4800
   ttcttccact tttatcatca tcccctactc gtacactcgt actctttgtt cgatcgcgat 4860
   tcatttctat aaataatctt gtatgtacat gcggccgctt aagcaacggg cttgataaca 4920
   gcgggggggg tgcccacgtt gttgcggttg cggaagaaca gaacaccctt accagcaccc 4980
   tcggcaccag cgctgggctc aacccactgg cacatacgcg cactgcggta catggcgcgg 5040
   atgaagccac gaggaccatc ctggacatca gcccggtagt gcttgcccat gatgggctta 5100
   atggcctcgg tggcctcgtc cgcgttgtag aaggggatgc tgctgacgta gtggtggagg 5160
   acatgagtct cgatgatgcc gtggagaagg tggcggccga tgaagcccat ctcacggtca 5220
   atggtagcag cggcaccacg gacgaagttc cactcgtcgt tggtgtagtg gggaagggta 5280
   gggtcggtgt gctggaggaa ggtgatggca acgagccagt ggttaaccca gaggtaggga 5340
   acaaagtacc agatggccat gttgtagaaa ccgaacttct gaacgaggaa gtacagagca 5400
   gtggccatca gaccgatacc aatatcgctg aggacgatga gcttagcgtc actgttctcg 5460
   tacagagggc tgcggggatc gaagtggtta acaccaccgc cgaggccgtt atgcttgccc 5520
   ttgccgcgac cctcacgctg gcgctcgtgg tagttgtggc cggtaacatt ggtgatgagg 5580
   tagttgggcc agccaacgag ctgctgaagg acgagcatga gaagagtgaa agcgggggtc 5640
   tcctcagtaa gatgagcgag ctcgtgggtc atctttccga gacgagtagc ctgctgctcg 5700
   cgggttcggg gaacgaagac catgtcacgc tccatgttgc cagtggcctt gtggtgcttt 5760
   cggtgggaga tttgccagct gaagtagggg acaaggaggg aagagtgaag aacccagcca 5820
   gtaatgtcgt tgatgatgcg agaatcggag aaagcaccgt gaccgcactc atgggcaata 5880
   acccagagac cagtaccgaa aagaccctga agaacggtgt acacggccca cagaccagcg 5940
   cgggcggggg tggaggggat atattcgggg gtcacaaagt tgtaccagat gctgaaagtg 6000
   gtagtcagga ggacaatgtc gcggaggata taaccgtatc ccttgagagc ggagcgcttg 6060
   aagcagtgct tagggatggc attgtagatg tccttgatgg taaagtcggg aacctcgaac 6120
   tggttgccgt aggtgtcgag catgacacca tactcggact tgggcttggc gatatcaacc 6180
   tcggacatgg acgagagcga tgtggaagag gccgagtggc ggggagagtc tgaaggagag 6240
   acggcggcag actcagaatc cgtcacagta gttgaggtga cggtgcgtct aagcgcaggg 6300
   ttctgcttgg gcagagccga agtggacgcc atggttgtga attagggtgg tgagaatggt 6360
   tggttgtagg gaagaatcaa aggccggtct cgggatccgt gggtatatat atatatatat 6420
   atatatacga tccttcgtta cctccctgtt ctcaaaactg tggtttttcg tttttcgttt 6480
   tttgcttttt ttgatttttt tagggccaac taagcttcca gatttcgcta atcacctttg 6540
   tactaattac aagaaaggaa gaagctgatt agagttgggc tttttatgca actgtgctac 6600
   tccttatctc tgatatgaaa gtgtagaccc aatcacatca tgtcatttag agttggtaat 6660
   actgggagga tagataaggc acgaaaacga gccatagcag acatgctggg tgtagccaag 6720
   cagaagaaag tagatgggag ccaattgacg agcgagggag ctacgccaat ccgacatacg 6780
   acacgctgag atcgtcttgg ccggggggta cctacagatg tccaagggta agtgcttgac 6840
   tgtaattgta tgtctgagga caaatatgta gtcagccgta taaagtcata ccaggcacca 6900
   gtgccatcat cgaaccacta actctctatg atacatgcct ccggtattat tgtaccatgc 6960
   gtcgctttgt tacatacgta tcttgccttt ttctctcaga aactccagac tttggctatt 7020
   ggtcgagata agcccggacc atagtgagtc tttcacactc tacatttctc ccttgctcca 7080
   actatttaaa ttgccccgga gaagacggcc aggccgccta gatgacaaat tcaacaactc 7140
   acagctgact ttctgccatt gccactaggg gggggccttt ttatatggcc aagccaagct 7200
   ctccacgtcg gttgggctgc acccaacaat aaatgggtag ggttgcacca acaaagggat 7260
   gggatggggg gtagaagata cgaggataac ggggctcaat ggcacaaata agaacgaata 7320
   ctgccattaa gactcgtgat ccagcgactg acaccattgc atcatctaag ggcctcaaaa 7380
   ctacctcgga actgctgcgc tgatctggac accacagagg ttccgagcac tttaggttgc 7440
   accaaatgtc ccaccaggtg caggcagaaa acgctggaac agcgtgtaca gtttgtctta 7500
   acaaaaagtg agggcgctga ggtcgagcag ggtggtgtga cttgttatag cctttagagc 7560
   tgcgaaagcg cgtatggatt tggctcatca ggccagattg agggtctgtg gacacatgtc 7620
   atgttagtgt acttcaatcg ccccctggat atagccccga caataggccg tggcctcatt 7680
   tttttgcctt ccgcacattt ccattgctcg gtacccacac cttgcttctc ctgcacttgc 7740
   caaccttaat actggtttac attgaccaac atcttacaag cggggggctt gtctagggta 7800
   tatataaaca gtggctctcc caatcggttg ccagtctctt ttttcctttc tttccccaca 7860
   gattcgaaat ctaaactaca catcacagaa ttccgagccg tgagtatcca cgacaagatc 7920
   agtgtcgaga cgacgcgttt tgtgtaatga cacaatccga aagtcgctag caacacacac 7980
   tctctacaca aactaaccca gctctggtac catggtgaag gcttctcgac aggctctgcc 8040
   cctcgtcatc gacggaaagg tgtacgacgt ctccgcttgg gtgaacttcc accctggtgg 8100
   agctgaaatc attgagaact accagggacg agatgctact gacgccttca tggttatgca 8160
   ctctcaggaa gccttcgaca agctcaagcg aatgcccaag atcaaccagg cttccgagct 8220
   gcctccccag gctgccgtca acgaagctca ggaggatttc cgaaagctcc gagaagagct 8280
   gatcgccact ggcatgtttg acgcctctcc cctctggtac tcgtacaaga tcttgaccac 8340
   cctgggtctt ggcgtgcttg ccttcttcat gctggtccag taccacctgt acttcattgg 8400
   tgctctcgtg ctcggtatgc actaccagca aatgggatgg ctgtctcatg acatctgcca 8460
   ccaccagacc ttcaagaacc gaaactggaa taacgtcctg ggtctggtct ttggcaacgg 8520
   actccagggc ttctccgtga cctggtggaa ggacagacac aacgcccatc attctgctac 8580
   caacgttcag ggtcacgatc ccgacattga taacctgcct ctgctcgcct ggtccgagga 8640
   cgatgtcact cgagcttctc ccatctcccg aaagctcatt cagttccaac agtactattt 8700
   cctggtcatc tgtattctcc tgcgattcat ctggtgtttc cagtctgtgc tgaccgttcg 8760
   atccctcaag gaccgagaca accagttcta ccgatctcag tacaagaaag aggccattgg 8820
   actcgctctg cactggactc tcaagaccct gttccacctc ttctttatgc cctccatcct 8880
   gacctcgatg ctggtgttct ttgtttccga gctcgtcggt ggcttcggaa ttgccatcgt 8940
   ggtcttcatg aaccactacc ctctggagaa gatcggtgat tccgtctggg acggacatgg 9000
   cttctctgtg ggtcagatcc atgagaccat gaacattcga cgaggcatca ttactgactg 9060
   gttctttgga ggcctgaact accagatcga gcaccatctc tggcccaccc tgcctcgaca 9120
   caacctcact gccgtttcct accaggtgga acagctgtgc cagaagcaca acctccccta 9180
   ccgaaaccct ctgccccatg aaggtctcgt catcctgctc cgatacctgt cccagttcgc 9240
   tcgaatggcc gagaagcagc ccggtgccaa ggctcagtaa gcggccgcaa gtgtggatgg 9300
   ggaagtgagt gcccggttct gtgtgcacaa ttggcaatcc aagatggatg gattcaacac 9360
   agggatatag cgagctacgt ggtggtgcga ggatatagca acggatattt atgtttgaca 9420
   cttgagaatg tacgatacaa gcactgtcca agtacaatac taaacatact gtacatactc 9480
   atactcgtac ccgggcaacg gtttcacttg agtgcagtgg ctagtgctct tactcgtaca 9540
   gtgtgcaata ctgcgtatca tagtctttga tgtatatcgt attcattcat gttagttgcg 9600
   tacgggtgaa gcttccactg gtcggcgtgg tagtggggca gagtggggtc ggtgtgctgc 9660
   aggtaggtga tggccacgag ccagtggttg acccacaggt aggggatcag gtagtagagg 9720
   gtgacggaag ccaggcccca tcggttgatg gagtatgcga tgacggacat ggtgatacca 9780
   ataccgacgt tagagatcca gatgttgaac cagtccttct tctcaaacag cggggcgttg 9840
   gggttgaagt ggttgacagc ccatttgttg agcttggggt acttctgtcc ggtaacgtaa 9900
   gacagcagat acagaggcca tccaaacacc tgctgggtga tgaggccgta gagggtcatg 9960
   aggggagcgt cctcagcaag ctcagaccag tcatgggcgc ctcggttctc cataaactcc 10020
   tttcggtcct tgggcacaaa caccatatca cgggtgaggt gaccagtgga cttgtggtgc 10080
   atggagtggg tcagcttcca ggcgtagtaa gggaccagca tggaggagtg cagaacccat 10140
   ccggtgacgt tgttgacggt gttagagtcg gagaaagcag agtggccaca ctcgtgggca 10200
   agaacccaca gaccggtgcc aaacagaccc tggacaatgg agtacatggc ccaggccaca 10260
   gctcggccgg aagccgaggg aataagaggc aggtacgcgt aggccatgta ggcaaaaacg 10320
   gcgataaaga agcaggcgcg ccagctgcat taatgaatcg gccaacgcgc ggggagaggc 10380
   ggtttgcgta ttgggcgctc ttccgcttcc tcgctcactg actcgctgcg ctcggtcgtt 10440
   cggctgcggc gagcggtatc agctcactca aaggcggtaa tacggttatc cacagaatca 10500
   ggggataacg caggaaagaa catgtgagca aaaggccagc aaaaggccag gaaccgtaaa 10560
   aaggccgcgt tgctggcgtt tttccatagg ctccgccccc ctgacgagca tcacaaaaat 10620
   cgacgctcaa gtcagaggtg gcgaaacccg acaggactat aaagatacca ggcgtttccc 10680
   cctggaagct ccctcgtgcg ctctcctgtt ccgaccctgc cgcttaccgg atacctgtcc 10740
   gcctttctcc cttcgggaag cgtggcgctt tctcatagct cacgctgtag gtatctcagt 10800
   tcggtgtagg tcgttcgctc caagctgggc tgtgtgcacg aaccccccgt tcagcccgac 10860
   cgctgcgcct tatccggtaa ctatcgtctt gagtccaacc cggtaagaca cgacttatcg 10920
   ccactggcag cagccactgg taacaggatt agcagagcga ggtatgtagg cggtgctaca 10980
   gagttcttga agtggtggcc taactacggc tacactagaa gaacagtatt tggtatctgc 11040
   gctctgctga agccagttac cttcggaaaa agagttggta gctcttgatc cggcaaacaa 11100
   accaccgctg gtagcggtgg tttttttgtt tgcaagcagc agattacgcg cagaaaaaaa 11160
   ggatctcaag aagatccttt gatcttttct acggggtctg acgctcagtg gaacgaaaac 11220
   tcacgttaag ggattttggt catgagatta tcaaaaagga tcttcaccta gatcctttta 11280
   aattaaaaat gaagttttaa atcaatctaa agtatatatg agtaaacttg gtctgacagt 11340
   taccaatgct taatcagtga ggcacctatc tcagcgatct gtctatttcg ttcatccata 11400
   gttgcctgac tccccgtcgt gtagataact acgatacggg agggcttacc atctggcccc 11460
   agtgctgcaa tgataccgcg agacccacgc tcaccggctc cagatttatc agcaataaac 11520
   cagccagccg gaagggccga gcgcagaagt ggtcctgcaa ctttatccgc ctccatccag 11580
   tctattaatt gttgccggga agctagagta agtagttcgc cagttaatag tttgcgcaac 11640
   gttgttgcca ttgctacagg catcgtggtg tcacgctcgt cgtttggtat ggcttcattc 11700
   agctccggtt cccaacgatc aaggcgagtt acatgatccc ccatgttgtg caaaaaagcg 11760
   gttagctcct tcggtcctcc gatcgttgtc agaagtaagt tggccgcagt gttatcactc 11820
   atggttatgg cagcactgca taattctctt actgtcatgc catccgtaag atgcttttct 11880
   gtgactggtg agtactcaac caagtcattc tgagaatagt gtatgcggcg accgagttgc 11940
   tcttgcccgg cgtcaatacg ggataatacc gcgccacata gcagaacttt aaaagtgctc 12000
   atcattggaa aacgttcttc ggggcgaaaa ctctcaagga tcttaccgct gttgagatcc 12060
   agttcgatgt aacccactcg tgcacccaac tgatcttcag catcttttac tttcaccagc 12120
   gtttctgggt gagcaaaaac aggaaggcaa aatgccgcaa aaaagggaat aagggcgaca 12180
   cggaaatgtt gaatactcat actcttcctt tttcaatatt attgaagcat ttatcagggt 12240
   tattgtctca tgagcggata catatttgaa tgtatttaga aaaataaaca aataggggtt 12300
   ccgcgcacat ttccccgaaa agtgccacct gatgcggtgt gaaataccgc acagatgcgt 12360
   aaggagaaaa taccgcatca ggaaattgta agcgttaata ttttgttaaa attcgcgtta 12420
   aatttttgtt aaatcagctc attttttaac caataggccg aaatcggcaa aatcccttat 12480
   aaatcaaaag aatagaccga gatagggttg agtgttgttc cagtttggaa caagagtcca 12540
   ctattaaaga acgtggactc caacgtcaaa gggcgaaaaa ccgtctatca gggcgatggc 12600
   ccactacgtg aaccatcacc ctaatcaagt tttttggggt cgaggtgccg taaagcacta 12660
   aatcggaacc ctaaagggag cccccgattt agagcttgac ggggaaagcc ggcgaacgtg 12720
   gcgagaaagg aagggaagaa agcgaaagga gcgggcgcta gggcgctggc aagtgtagcg 12780
   gtcacgctgc gcgtaaccac cacacccgcc gcgcttaatg cgccgctaca gggcgcgtcc 12840
   attcgccatt caggctgcgc aactgttggg aagggcgatc ggtgcgggcc tcttcgctat 12900
   tacgccagct ggcgaaaggg ggatgtgctg caaggcgatt aagttgggta acgccagggt 12960
   tttcccagtc acgacgttgt aaaacgacgg ccagtgaatt gtaatacgac tcactatagg 13020
   gcgaattggg cccgacgtcg catgcttgaa tctacaagta ggagggttgg agtgattaag 13080
   tgaaacttct ttaacggctc tatgccagtt ctattgatat ccgaaacatc agtatgaagg 13140
   tctgataagg gtgacttctt cccacagatt cgtatcagta cgagtacgag accggtactt 13200
   gtaacagtat tgatactaaa gggaaactac aacggttgtc agcgtaatgt gacttcgccc 13260
   atgaacgcag acacgcagtg ccgagtgcgg tgatatcgcc tactcgttac gtccatggac 13320
   tacacaaccc ctcggcttcg cttggcttag cctcgggctc ggtgctgttc agttaaaaca 13380
   caatcaaata acatttctac tttttagaag gcaggccgtc aggagcaact ccgactccat 13440
   tgacgtttct aaacatctga atgccttcct taccttcaac aaactggcag gttcgggcga 13500
   cagtgtaaag agacttgatg aagttggtgt cgtcgtgtcg gtagtgcttg cccatgacct 13560
   tcttgatctt ctcagtggcg attcgggcgt tgtagaaggg aattccttta cctgcaggat 13620
   aacttcgtat aatgtatgct atacgaagtt atgatctctc tcttgagctt ttccataaca 13680
   agttcttctg cctccaggaa gtccatgggt ggtttgatca tggttttggt gtagtggtag 13740
   tgcagtggtg gtattgtgac tggggatgta gttgagaata agtcatacac aagtcagctt 13800
   tcttcgagcc tcatataagt ataagtagtt caacgtatta gcactgtacc cagcatctcc 13860
   gtatcgagaa acacaacaac atgccccatt ggacagatca tgcggataca caggttgtgc 13920
   agtatcatac atactcgatc agacaggtcg tctgaccatc atacaagctg aacaagcgct 13980
   ccatacttgc acgctctcta tatacacagt taaattacat atccatagtc taacctctaa 14040
   cagttaatct tctggtaagc ctcccagcca gccttctggt atcgcttggc ctcctcaata 14100
   ggatctcggt tctggccgta cagacctcgg ccgacaatta tgatatccgt tccggtagac 14160
   atgacatcct caacagttcg gtactgctgt ccgagagcgt ctcccttgtc gtcaagaccc 14220
   accccggggg tcagaataag ccagtcctca gagtcgccct taggtcggtt ctgggcaatg  14280
   aagccaacca caaactcggg gtcggatcgg gcaagctcaa tggtctgctt ggagtactcg  14340
   ccagtggcca gagagccctt gcaagacagc tcggccagca tgagcagacc tctggccagc  14400
   ttctcgttgg gagaggggac taggaactcc ttgtactggg agttctcgta gtcagagacg  14460
   tcctccttct tctgttcaga gacagtttcc tcggcaccag ctcgcaggcc agcaatgatt  14520
   ccggttccgg gtacaccgtg ggcgttggtg atatcggacc actcggcgat tcggtgacac  14580
   cggtactggt gcttgacagt gttgccaata tctgcgaact ttctgtcctc gaacaggaag  14640
   aaaccgtgct taagagcaag ttccttgagg gggagcacag tgccggcgta ggtgaagtcg  14700
   tcaatgatgt cgatatgggt tttgatcatg cacacataag gtccgacctt atcggcaagc  14760
   tcaatgagct ccttggtggt ggtaacatcc agagaagcac acaggttggt tttcttggct  14820
   gccacgagct tgagcactcg agcggcaaag gcggacttgt ggacgttagc tcgagcttcg  14880
   taggagggca ttttggtggt gaagaggaga ctgaaataaa tttagtctgc agaacttttt  14940
   atcggaacct tatctggggc agtgaagtat atgttatggt aatagttacg agttagttga  15000
   acttatagat agactggact atacggctat cggtccaaat tagaaagaac gtcaatggct  15060
   ctctgggcgt cgcctttgcc gacaaaaatg tgatcatgat gaaagccagc aatgacgttg  15120
   cagctgatat tgttgtcggc caaccgcgcc gaaaacgcag ctgtcagacc cacagcctcc  15180
   aacgaagaat gtatcgtcaa agtgatccaa gcacactcat agttggagtc gtactccaaa  15240
   ggcggcaatg acgagtcaga cagatactcg tcgacgcgat aacttcgtat aatgtatgct  15300
   atacgaagtt atcgtacgat agttagtaga caacaat                           15337
<210> 38
   <211> 1272
   <212> DNA
   <213> Artificial Sequence
<220>
   <223> mutant EgD8M delta-8 desaturase (also designated as "EgD8S-23")
<220>
   <221> CDS
   <222> (2)..(1270)
<400> 38
   c atg gtg aag gct tct cga cag gct ctg ccc ctc gtc atc gac gga aag     49
     Met Val Lys Ala Ser Arg Gln Ala Leu Pro Leu Val Ile Asp Gly Lys
     1               5                   10                  15
   gtg tac gac gtc tcc gct tgg gtg aac ttc cac cct ggt gga gct gaa       97
   Val Tyr Asp Val Ser Ala Trp Val Asn Phe His Pro Gly Gly Ala Glu
               20                  25                  30
   atc att gag aac tac cag gga cga gat gct act gac gcc ttc atg gtt      145
   Ile Ile Glu Asn Tyr Gln Gly Arg Asp Ala Thr Asp Ala Phe Met Val
           35                  40                  45
   atg cac tct cag gaa gcc ttc gac aag ctc aag cga atg ccc aag atc      193
   Met His Ser Gln Glu Ala Phe Asp Lys Leu Lys Arg Met Pro Lys Ile
       50                  55                  60
   aac cag gct tcc gag ctg cct ccc cag gct gcc gtc aac gaa gct cag      241
   Asn Gln Ala Ser Glu Leu Pro Pro Gln Ala Ala Val Asn Glu Ala Gln
   65                  70                  75                  80
   gag gat ttc cga aag ctc cga gaa gag ctg atc gcc act ggc atg ttt      289
   Glu Asp Phe Arg Lys Leu Arg Glu Glu Leu Ile Ala Thr Gly Met Phe
                   85                  90                  95
   gac gcc tct ccc ctc tgg tac tcg tac aag atc ttg acc acc ctg ggt      337
   Asp Ala Ser Pro Leu Trp Tyr Ser Tyr Lys Ile Leu Thr Thr Leu Gly
               100                 105                 110
   ctt ggc gtg ctt gcc ttc ttc atg ctg gtc cag tac cac ctg tac ttc      385
   Leu Gly Val Leu Ala Phe Phe Met Leu Val Gln Tyr His Leu Tyr Phe
           115                 120                 125
   att ggt gct ctc gtg ctc ggt atg cac tac cag caa atg gga tgg ctg      433
   Ile Gly Ala Leu Val Leu Gly Met His Tyr Gln Gln Met Gly Trp Leu
       130                 135                 140
   tct cat gac atc tgc cac cac cag acc ttc aag aac cga aac tgg aat      481
   Ser His Asp Ile Cys His His Gln Thr Phe Lys Asn Arg Asn Trp Asn
   145                 150                 155                 160
   aac gtc ctg ggt ctg gtc ttt ggc aac gga ctc cag ggc ttc tcc gtg      529
   Asn Val Leu Gly Leu Val Phe Gly Asn Gly Leu Gln Gly Phe Ser Val
                   165                 170                 175
   acc tgg tgg aag gac aga cac aac gcc cat cat tct gct acc aac gtt      577
   Thr Trp Trp Lys Asp Arg His Asn Ala His His Ser Ala Thr Asn Val
               180                 185                 190
   cag ggt cac gat ccc gac att gat aac ctg cct ctg ctc gcc tgg tcc      625
   Gln Gly His Asp Pro Asp Ile Asp Asn Leu Pro Leu Leu Ala Trp Ser
           195                 200                 205
   gag gac gat gtc act cga gct tct ccc atc tcc cga aag ctc att cag      673
   Glu Asp Asp Val Thr Arg Ala Ser Pro Ile Ser Arg Lys Leu Ile Gln
       210                 215                 220
   ttc caa cag tac tat ttc ctg gtc atc tgt att ctc ctg cga ttc atc      721
   Phe Gln Gln Tyr Tyr Phe Leu Val Ile Cys Ile Leu Leu Arg Phe Ile
   225                 230                 235                 240
   tgg tgt ttc cag tct gtg ctg acc gtt cga tcc ctc aag gac cga gac      769
   Trp Cys Phe Gln Ser Val Leu Thr Val Arg Ser Leu Lys Asp Arg Asp
                   245                 250                 255
   aac cag ttc tac cga tct cag tac aag aaa gag gcc att gga ctc gct      817
   Asn Gln Phe Tyr Arg Ser Gln Tyr Lys Lys Glu Ala Ile Gly Leu Ala
               260                 265                 270
   ctg cac tgg act ctc aag acc ctg ttc cac ctc ttc ttt atg ccc tcc      865
   Leu His Trp Thr Leu Lys Thr Leu Phe His Leu Phe Phe Met Pro Ser
           275                 280                 285
   atc ctg acc tcg atg ctg gtg ttc ttt gtt tcc gag ctc gtc ggt ggc      913
   Ile Leu Thr Ser Met Leu Val Phe Phe Val Ser Glu Leu Val Gly Gly
       290                 295                 300
   ttc gga att gcc atc gtg gtc ttc atg aac cac tac cct ctg gag aag      961
   Phe Gly Ile Ala Ile Val Val Phe Met Asn His Tyr Pro Leu Glu Lys
   305                 310                 315                 320
   atc ggt gat tcc gtc tgg gac gga cat ggc ttc tct gtg ggt cag atc     1009
   Ile Gly Asp Ser Val Trp Asp Gly His Gly Phe Ser Val Gly Gln Ile
                   325                 330                 335
   cat gag acc atg aac att cga cga ggc atc att act gac tgg ttc ttt     1057
   His Glu Thr Met Asn Ile Arg Arg Gly Ile Ile Thr Asp Trp Phe Phe
               340                 345                 350
   gga ggc ctg aac tac cag atc gag cac cat ctc tgg ccc acc ctg cct     1105
   Gly Gly Leu Asn Tyr Gln Ile Glu His His Leu Trp Pro Thr Leu Pro
           355                 360                 365
   cga cac aac ctc act gcc gtt tcc tac cag gtg gaa cag ctg tgc cag     1153
   Arg His Asn Leu Thr Ala Val Ser Tyr Gln Val Glu Gln Leu Cys Gln
       370                 375                 380
   aag cac aac ctc ccc tac cga aac cct ctg ccc cat gaa ggt ctc gtc     1201
   Lys His Asn Leu Pro Tyr Arg Asn Pro Leu Pro His Glu Gly Leu Val
   385                 390                 395                 400
   atc ctg ctc cga tac ctg tcc cag ttc gct cga atg gcc gag aag cag     1249
   Ile Leu Leu Arg Tyr Leu Ser Gln Phe Ala Arg Met Ala Glu Lys Gln
                   405                 410                 415
   ccc ggt gcc aag gct cag taa gc                                      1272
   Pro Gly Ala Lys Ala Gln
               420
<210> 39
   <211> 422
   <212> PRT
   <213> Artificial Sequence
<220>
   <223> Synthetic Construct
<400> 39
   Met Val Lys Ala Ser Arg Gln Ala Leu Pro Leu Val Ile Asp Gly Lys
   1 5 10 15
   Val Tyr Asp Val Ser Ala Trp Val Asn Phe His Pro Gly Gly Ala Glu
   20 25 30
   Ile Ile Glu Asn Tyr Gln Gly Arg Asp Ala Thr Asp Ala Phe Met Val
   35 40 45
   Met His Ser Gln Glu Ala Phe Asp Lys Leu Lys Arg Met Pro Lys Ile
       50                  55                  60
   Asn Gln Ala Ser Glu Leu Pro Pro Gln Ala Ala Val Asn Glu Ala Gln
   65                  70                  75                  80
   Glu Asp Phe Arg Lys Leu Arg Glu Glu Leu Ile Ala Thr Gly Met Phe
                   85                  90                  95
   Asp Ala Ser Pro Leu Trp Tyr Ser Tyr Lys Ile Leu Thr Thr Leu Gly
               100                 105                 110
   Leu Gly Val Leu Ala Phe Phe Met Leu Val Gln Tyr His Leu Tyr Phe
           115                 120                 125
   Ile Gly Ala Leu Val Leu Gly Met His Tyr Gln Gln Met Gly Trp Leu
       130                 135                 140
   Ser His Asp Ile Cys His His Gln Thr Phe Lys Asn Arg Asn Trp Asn
   145                 150                 155                 160
   Asn Val Leu Gly Leu Val Phe Gly Asn Gly Leu Gln Gly Phe Ser Val
                   165                 170                 175
   Thr Trp Trp Lys Asp Arg His Asn Ala His His Ser Ala Thr Asn Val
               180                 185                 190
   Gln Gly His Asp Pro Asp Ile Asp Asn Leu Pro Leu Leu Ala Trp Ser
           195                 200                 205
   Glu Asp Asp Val Thr Arg Ala Ser Pro Ile Ser Arg Lys Leu Ile Gln
       210                 215                 220
   Phe Gln Gln Tyr Tyr Phe Leu Val Ile Cys Ile Leu Leu Arg Phe Ile
   225                 230                 235                 240
   Trp Cys Phe Gln Ser Val Leu Thr Val Arg Ser Leu Lys Asp Arg Asp
                   245                 250                 255
   Asn Gln Phe Tyr Arg Ser Gln Tyr Lys Lys Glu Ala Ile Gly Leu Ala
               260                 265                 270
   Leu His Trp Thr Leu Lys Thr Leu Phe His Leu Phe Phe Met Pro Ser
           275                 280                 285
   Ile Leu Thr Ser Met Leu Val Phe Phe Val Ser Glu Leu Val Gly Gly
   290 295 300
   Phe Gly Ile Ala Ile Val Val Phe Met Asn His Tyr Pro Leu Glu Lys
   305 310 315 320
   Ile Gly Asp Ser Val Trp Asp Gly His Gly Phe Ser Val Gly Gln Ile
   325 330 335
   His Glu Thr Met Asn Ile Arg Arg Gly Ile Ile Thr Asp Trp Phe Phe
   340 345 350
   Gly Gly Leu Asn Tyr Gln Ile Glu His His Leu Trp Pro Thr Leu Pro
   355 360 365
   Arg His Asn Leu Thr Ala Val Ser Tyr Gln Val Glu Gln Leu Cys Gln
   370 375 380
   Lys His Asn Leu Pro Tyr Arg Asn Pro Leu Pro His Glu Gly Leu Val
   385 390 395 400
   Ile Leu Leu Arg Tyr Leu Ser Gln Phe Ala Arg Met Ala Glu Lys Gln
   405 410 415
   Pro Gly Ala Lys Ala Gln
   420
<210> 40
   <211> 777
   <212> DNA
   <213> Euglena gracilis
<220>
   <221> CDS
   <222> (1)..(777)
   <223> delta-9 elongase
<300>
   <302> DELTA-9 ELONGASES AND THEIR USE IN MAKING POLYUNSATURATED FATTY ACIDS
<310> WO 2007/061742
   <311> 2006-11-16
   <312> 2007-05-31
   <313> (1)..(777)
<300>
   <302> DELTA-9 ELONGASES AND THEIR USE IN MAKING POLYUNSATURATED FATTY ACIDS
<310> US 2007-0117190-A1
   <311> 2006-11-16
   <312> 2007-05-24
   <313> (1)..(777)
<400> 40
   atg gag gtg gtg aat gaa ata gtc tca att ggg cag gaa gtt tta ccc       48
   Met Glu Val Val Asn Glu Ile Val Ser Ile Gly Gln Glu Val Leu Pro
   1               5                   10                  15
   aaa gtt gat tat gcc caa ctc tgg agt gat gcc agt cac tgt gag gtg       96
   Lys Val Asp Tyr Ala Gln Leu Trp Ser Asp Ala Ser His Cys Glu Val
               20                  25                  30
   ctt tac ttg tcc atc gca ttt gtc atc ttg aag ttc act ctt ggc ccc      144
   Leu Tyr Leu Ser Ile Ala Phe Val Ile Leu Lys Phe Thr Leu Gly Pro
           35                  40                  45
   ctt ggt cca aaa ggt cag tct cgt atg aag ttt gtt ttc acc aat tac      192
   Leu Gly Pro Lys Gly Gln Ser Arg Met Lys Phe Val Phe Thr Asn Tyr
       50                  55                  60
   aac ctt ctc atg tcc att tat tcg ttg gga tca ttc ctc tca atg gca      240
   Asn Leu Leu Met Ser Ile Tyr Ser Leu Gly Ser Phe Leu Ser Met Ala
   65                  70                  75                  80
   tat gcc atg tac acc atc ggt gtt atg tct gac aac tgc gag aag gct      288
   Tyr Ala Met Tyr Thr Ile Gly Val Met Ser Asp Asn Cys Glu Lys Ala
                   85                  90                  95
   ttt gac aac aac gtc ttc agg atc acc acg cag ttg ttc tat ttg agc      336
   Phe Asp Asn Asn Val Phe Arg Ile Thr Thr Gln Leu Phe Tyr Leu Ser
               100                 105                 110
   aag ttc ctg gag tat att gac tcc ttc tat ttg cca ctg atg ggc aag      384
   Lys Phe Leu Glu Tyr Ile Asp Ser Phe Tyr Leu Pro Leu Met Gly Lys
           115                 120                 125
   cct ctg acc tgg ttg caa ttc ttc cat cat ttg ggg gca ccg atg gat      432
   Pro Leu Thr Trp Leu Gln Phe Phe His His Leu Gly Ala Pro Met Asp
       130                 135                 140
   atg tgg ctg ttc tat aat tac cga aat gaa gct gtt tgg att ttt gtg      480
   Met Trp Leu Phe Tyr Asn Tyr Arg Asn Glu Ala Val Trp Ile Phe Val
   145                 150                 155                 160
   ctg ttg aat ggt ttc atc cac tgg atc atg tac ggt tat tat tgg acc      528
   Leu Leu Asn Gly Phe Ile His Trp Ile Met Tyr Gly Tyr Tyr Trp Thr
                   165                 170                 175
   aga ttg atc aag ctg aag ttc ccc atg cca aaa tcc ctg att aca tca      576
   Arg Leu Ile Lys Leu Lys Phe Pro Met Pro Lys Ser Leu Ile Thr Ser
               180                 185                 190
   atg cag atc att caa ttc aat gtt ggt ttc tac att gtc tgg aag tac      624
   Met Gln Ile Ile Gln Phe Asn Val Gly Phe Tyr Ile Val Trp Lys Tyr
           195                 200                 205
   agg aac att ccc tgt tat cgc caa gat ggg atg agg atg ttt ggc tgg      672
   Arg Asn Ile Pro Cys Tyr Arg Gln Asp Gly Met Arg Met Phe Gly Trp
       210                 215                 220
   ttc ttc aat tac ttt tat gtt ggc aca gtc ttg tgt ttg ttc ttg aat      720
   Phe Phe Asn Tyr Phe Tyr Val Gly Thr Val Leu Cys Leu Phe Leu Asn
   225                 230                 235                 240
   ttc tat gtg caa acg tat atc gtc agg aag cac aag gga gcc aaa aag      768
   Phe Tyr Val Gln Thr Tyr Ile Val Arg Lys His Lys Gly Ala Lys Lys
                   245                 250                 255
   att cag tga                                                          777
   Ile Gln
<210> 41
   <211> 258
   <212> PRT
   <213> Euglena gracilis
<400> 41
   Met Glu Val Val Asn Glu Ile Val Ser Ile Gly Gln Glu Val Leu Pro
   1               5                   10                  15
   Lys Val Asp Tyr Ala Gln Leu Trp Ser Asp Ala Ser His Cys Glu Val
               20                  25                  30
   Leu Tyr Leu Ser Ile Ala Phe Val Ile Leu Lys Phe Thr Leu Gly Pro
           35                  40                  45
   Leu Gly Pro Lys Gly Gln Ser Arg Met Lys Phe Val Phe Thr Asn Tyr
       50                  55                  60
   Asn Leu Leu Met Ser Ile Tyr Ser Leu Gly Ser Phe Leu Ser Met Ala
   65                  70                  75                  80
   Tyr Ala Met Tyr Thr Ile Gly Val Met Ser Asp Asn Cys Glu Lys Ala
                   85                  90                  95
   Phe Asp Asn Asn Val Phe Arg Ile Thr Thr Gln Leu Phe Tyr Leu Ser
               100                 105                 110
   Lys Phe Leu Glu Tyr Ile Asp Ser Phe Tyr Leu Pro Leu Met Gly Lys
           115                 120                 125
   Pro Leu Thr Trp Leu Gln Phe Phe His His Leu Gly Ala Pro Met Asp
       130                 135                 140
   Met Trp Leu Phe Tyr Asn Tyr Arg Asn Glu Ala Val Trp Ile Phe Val
   145                 150                 155                 160
   Leu Leu Asn Gly Phe Ile His Trp Ile Met Tyr Gly Tyr Tyr Trp Thr
                   165                 170                 175
   Arg Leu Ile Lys Leu Lys Phe Pro Met Pro Lys Ser Leu Ile Thr Ser
   180 185 190
   Met Gln Ile Ile Gln Phe Asn Val Gly Phe Tyr Ile Val Trp Lys Tyr
   195 200 205
   Arg Asn Ile Pro Cys Tyr Arg Gln Asp Gly Met Arg Met Phe Gly Trp
   210 215 220
   Phe Phe Asn Tyr Phe Tyr Val Gly Thr Val Leu Cys Leu Phe Leu Asn
   225 230 235 240
   Phe Tyr Val Gln Thr Tyr Ile Val Arg Lys His Lys Gly Ala Lys Lys
   245 250 255
   Ile Gln
<210> 42
   <211> 13707
   <212> DNA
   <213> Artificial Sequence
<220>
   <223> Plasmid pZKSL-555R
<400> 42
   aaacagtgta cgcagatctg cccatgatgg gggctcccac caccagcaat cagggccctg      60
   attacacacc cacctgtaat gtcatgctgt tcatcgtggt taatgctgct gtgtgctgtg     120
   tgtgtgtgtt gtttggcgct cattgttgcg ttatgcagcg tacaccacaa tattggaagc     180
   ttattagcct ttctattttt tcgtttgcaa ggcttaacaa cattgctgtg gagagggatg     240
   gggatatgga ggccgctgga gggagtcgga gaggcgtttt ggagcggctt ggcctggcgc     300
   ccagctcgcg aaacgcacct aggacccttt ggcacgccga aatgtgccac ttttcagtct     360
   agtaacgcct tacctacgtc attccatgcg tgcatgtttg cgcctttttt cccttgccct     420
   tgatcgccac acagtacagt gcactgtaca gtggaggttt tgggggggtc ttagatggga      480
   gctaaaagcg gcctagcggt acactagtgg gattgtatgg agtggcatgg agcctaggtg     540
   gagcctgaca ggacgcacga ccggctagcc cgtgacagac gatgggtggc tcctgttgtc     600
   caccgcgtac aaatgtttgg gccaaagtct tgtcagcctt gcttgcgaac ctaattccca     660
   attttgtcac ttcgcacccc cattgatcga gccctaaccc ctgcccatca ggcaatccaa     720
   ttaagctcgc attgtctgcc ttgtttagtt tggctcctgc ccgtttcggc gtccacttgc     780
   acaaacacaa acaagcatta tatataaggc tcgtctctcc ctcccaacca cactcacttt     840
   tttgcccgtc ttcccttgct aacacaaaag tcaagaacac aaacaaccac cccaaccccc     900
   ttacacacaa gacatatcta cagcaatggc catggctctc tcccttacta ccgagcagct     960
   gctcgagcga cccgacctgg ttgccatcga cggcattctc tacgatctgg aaggtcttgc    1020
   caaggtccat cccggaggcg acttgatcct cgcttctggt gcctccgatg cttctcctct    1080
   gttctactcc atgcaccctt acgtcaagcc cgagaactcg aagctgcttc aacagttcgt    1140
   gcgaggcaag cacgaccgaa cctccaagga cattgtctac acctacgact ctccctttgc    1200
   acaggacgtc aagcgaacta tgcgagaggt catgaaaggt cggaactggt atgccacacc    1260
   tggattctgg ctgcgaaccg ttggcatcat tgctgtcacc gccttttgcg agtggcactg    1320
   ggctactacc ggaatggtgc tgtggggtct cttgactgga ttcatgcaca tgcagatcgg    1380
   cctgtccatt cagcacgatg cctctcatgg tgccatcagc aaaaagccct gggtcaacgc    1440
   tctctttgcc tacggcatcg acgtcattgg atcgtccaga tggatctggc tgcagtctca    1500
   catcatgcga catcacacct acaccaatca gcatggtctc gacctggatg ccgagtccgc    1560
   agaaccattc cttgtgttcc acaactaccc tgctgccaac actgctcgaa agtggtttca    1620
   ccgattccag gcctggtaca tgtacctcgt gcttggagcc tacggcgttt cgctggtgta    1680
   caaccctctc tacatcttcc gaatgcagca caacgacacc attcccgagt ctgtcacagc    1740
   catgcgagag aacggctttc tgcgacggta ccgaaccctt gcattcgtta tgcgagcttt    1800
   cttcatcttt cgaaccgcct tcttgccctg gtatctcact ggaacctccc tgctcatcac    1860
   cattcctctg gtgcccactg ctaccggtgc cttcctcacc ttctttttca tcttgtctca    1920
   caacttcgat ggctcggagc gaatccccga caagaactgc aaggtcaaga gctccgagaa    1980
   ggacgttgaa gccgatcaga tcgactggta cagagctcag gtggagacct cttccaccta    2040
   cggtggaccc attgccatgt tctttactgg cggtctcaac ttccagatcg agcatcacct    2100
   ctttcctcga atgtcgtctt ggcactatcc cttcgtgcag caagctgtcc gagagtgttg    2160
   cgaacgacac ggagttcggt acgtcttcta ccctaccatt gtgggcaaca tcatttccac    2220
   cctcaagtac atgcacaaag tcggtgtggt tcactgtgtc aaggacgctc aggattccta    2280
   agcggccgca agtgtggatg gggaagtgag tgcccggttc tgtgtgcaca attggcaatc    2340
   caagatggat ggattcaaca cagggatata gcgagctacg tggtggtgcg aggatatagc    2400
   aacggatatt tatgtttgac acttgagaat gtacgataca agcactgtcc aagtacaata    2460
   ctaaacatac tgtacatact catactcgta cccgggcaac ggtttcactt gagtgcagtg    2520
   gctagtgctc ttactcgtac agtgtgcaat actgcgtatc atagtctttg atgtatatcg    2580
   tattcattca tgttagttgc gtacgctgtg ttgttgtatg tggtgaagct tgacaatgga    2640
   tggtgtgtcg tatcaggctg gggaacaatt gtgcttaagt atgctgcagt tgagtaagag 2700
   tcatcgctcc accaaaataa agtttgccat tagggttgga gagagagatg gtggctggaa 2760
   gaattaaatg acatcaagct gaggattgtg ggtgtgcaat aacacatgtt aggggtgacc 2820
   tgtggctcga aatctgataa ttattttgta actttatgat tattcttaga ttttttaata 2880
   ttcctctata taacacataa gtagctgtcg tctagttgtt catagcctga ctcctgcaat 2940
   agattagtgc agagtgattt tgtgcaattg agagccacgg ttgagtcaag tgactttgtg 3000
   tgtgaagtca tcttacgttt caagtctcac aggttactca attggttggt tgtctgccct 3060
   ttacagatat ttacagtacc tgagcgtaaa gtcgttcatc cacggaatga ctgttcctgt 3120
   cacgcagtca tgatcatgga tgtggctggt caggaaccat tttggatagg agacttaggg 3180
   attggactat tattgaaaaa actgagccga atatgatata gttctatttg aatgcagaac 3240
   ttctgatggt caattcactt atttcaggca tatcggtcat ggtggcagct gccacgatgt 3300
   tatctcgttg gaaacctcgg cgcgccagct gcattaatga atcggccaac gcgcggggag 3360
   aggcggtttg cgtattgggc gctcttccgc ttcctcgctc actgactcgc tgcgctcggt 3420
   cgttcggctg cggcgagcgg tatcagctca ctcaaaggcg gtaatacggt tatccacaga 3480
   atcaggggat aacgcaggaa agaacatgtg agcaaaaggc cagcaaaagg ccaggaaccg 3540
   taaaaaggcc gcgttgctgg cgtttttcca taggctccgc ccccctgacg agcatcacaa 3600
   aaatcgacgc tcaagtcaga ggtggcgaaa cccgacagga ctataaagat accaggcgtt 3660
   tccccctgga agctccctcg tgcgctctcc tgttccgacc ctgccgctta ccggatacct 3720
   gtccgccttt ctcccttcgg gaagcgtggc gctttctcat agctcacgct gtaggtatct 3780
   cagttcggtg taggtcgttc gctccaagct gggctgtgtg cacgaacccc ccgttcagcc 3840
   cgaccgctgc gccttatccg gtaactatcg tcttgagtcc aacccggtaa gacacgactt 3900
   atcgccactg gcagcagcca ctggtaacag gattagcaga gcgaggtatg taggcggtgc 3960
   tacagagttc ttgaagtggt ggcctaacta cggctacact agaagaacag tatttggtat 4020
   ctgcgctctg ctgaagccag ttaccttcgg aaaaagagtt ggtagctctt gatccggcaa 4080
   acaaaccacc gctggtagcg gtggtttttt tgtttgcaag cagcagatta cgcgcagaaa 4140
   aaaaggatct caagaagatc ctttgatctt ttctacgggg tctgacgctc agtggaacga 4200
   aaactcacgt taagggattt tggtcatgag attatcaaaa aggatcttca cctagatcct 4260
   tttaaattaa aaatgaagtt ttaaatcaat ctaaagtata tatgagtaaa cttggtctga 4320
   cagttaccaa tgcttaatca gtgaggcacc tatctcagcg atctgtctat ttcgttcatc 4380
   catagttgcc tgactccccg tcgtgtagat aactacgata cgggagggct taccatctgg 4440
   ccccagtgct gcaatgatac cgcgagaccc acgctcaccg gctccagatt tatcagcaat 4500
   aaaccagcca gccggaaggg ccgagcgcag aagtggtcct gcaactttat ccgcctccat 4560
   ccagtctatt aattgttgcc gggaagctag agtaagtagt tcgccagtta atagtttgcg 4620
   caacgttgtt gccattgcta caggcatcgt ggtgtcacgc tcgtcgtttg gtatggcttc 4680
   attcagctcc ggttcccaac gatcaaggcg agttacatga tcccccatgt tgtgcaaaaa 4740
   agcggttagc tccttcggtc ctccgatcgt tgtcagaagt aagttggccg cagtgttatc 4800
   actcatggtt atggcagcac tgcataattc tcttactgtc atgccatccg taagatgctt 4860
   ttctgtgact ggtgagtact caaccaagtc attctgagaa tagtgtatgc ggcgaccgag 4920
   ttgctcttgc ccggcgtcaa tacgggataa taccgcgcca catagcagaa ctttaaaagt 4980
   gctcatcatt ggaaaacgtt cttcggggcg aaaactctca aggatcttac cgctgttgag 5040
   atccagttcg atgtaaccca ctcgtgcacc caactgatct tcagcatctt ttactttcac 5100
   cagcgtttct gggtgagcaa aaacaggaag gcaaaatgcc gcaaaaaagg gaataagggc 5160
   gacacggaaa tgttgaatac tcatactctt cctttttcaa tattattgaa gcatttatca 5220
   gggttattgt ctcatgagcg gatacatatt tgaatgtatt tagaaaaata aacaaatagg 5280
   ggttccgcgc acatttcccc gaaaagtgcc acctgatgcg gtgtgaaata ccgcacagat 5340
   gcgtaaggag aaaataccgc atcaggaaat tgtaagcgtt aatattttgt taaaattcgc 5400
   gttaaatttt tgttaaatca gctcattttt taaccaatag gccgaaatcg gcaaaatccc 5460
   ttataaatca aaagaataga ccgagatagg gttgagtgtt gttccagttt ggaacaagag 5520
   tccactatta aagaacgtgg actccaacgt caaagggcga aaaaccgtct atcagggcga 5580
   tggcccacta cgtgaaccat caccctaatc aagttttttg gggtcgaggt gccgtaaagc 5640
   actaaatcgg aaccctaaag ggagcccccg atttagagct tgacggggaa agccggcgaa 5700
   cgtggcgaga aaggaaggga agaaagcgaa aggagcgggc gctagggcgc tggcaagtgt 5760
   agcggtcacg ctgcgcgtaa ccaccacacc cgccgcgctt aatgcgccgc tacagggcgc 5820
   gtccattcgc cattcaggct gcgcaactgt tgggaagggc gatcggtgcg ggcctcttcg 5880
   ctattacgcc agctggcgaa agggggatgt gctgcaaggc gattaagttg ggtaacgcca 5940
   gggttttccc agtcacgacg ttgtaaaacg acggccagtg aattgtaata cgactcacta 6000
   tagggcgaat tgggcccgac gtcgcatgca ttccatagcc acacctttgc ctatggcttc 6060
   acaaccgaag gcaattcgag aggtcgcgct tatggaatcg actcgtataa agctgaaggg 6120
   aaagggagac gttccgagcg ctcagatgca atagtcgtcc agctaatgtg gattcaaaaa 6180
   caaccccaac agtaatcttg aaaatttgaa cggatcaatc tgaacactct tgctccaggt 6240
   cattcttcta acgcacatcc ccagagtcta gagggagttg tgttgtgaac atcctaataa 6300
   acaatgcaat ggattcggga tatcttctgt ctcgccccct actcgatgtc gagtaaaccg 6360
   atcaccaact aacaatactc ctccgcgttc tgccattgac tctcaaacag acatcgctat 6420
   caacggaaca gcatatttta gcttcttagg acaataaata ttgataatgc cggctctccc 6480
   tcggtatatt aagcaatcca ttcatacact cattcatcag gttaatttta tatatataat 6540
   ttgtctattc aaacaccgta aattactggt accatcatct cctccttttc aaatacacgt 6600
   ctatttgcat taatgaaatt actcgccaat tcgcagaacg tgtttgtcga acagagcctt 6660
   agctcgggtc cagacaggag cagtgtctcg ctgaggaagc tgcaggagag ttaattaact 6720
   cacctgcagg attgagacta tgaatggatt cccgtgcccg tattactcta ctaatttgat 6780
   cttggaacgc gaaaatacgt ttctaggact ccaaagaatc tcaactcttg tccttactaa 6840
   atatactacc catagttgat ggtttacttg aacagagagg acatgttcac ttgacccaaa 6900
   gtttctcgca tctcttggat atttgaacaa cggcgtccac tgaccgtcag ttatccagtc 6960
   acaaaacccc cacattcata cattcccatg tacgtttaca aagttctcaa ttccatcgtg 7020
   caaatcaaaa tcacatctat tcattcatca tatataaacc catcatgtct actaacactc 7080
   acaactccat agaaaacatc gactcagaac acacgctcca tgcggccgct taggaatcct 7140
   gtgcgtcctt cacgcagtgg acgacaccca ccttatgcat gtacttcagg gtggagatga 7200
   tgttgccgac gatggtaggg tagaaaacat atcgcactcc atgtcgttcg caacactccc 7260
   ggaccgcctg ctggacgaag gggtagtgcc aagacgacat ccggggaaag aggtggtgct 7320
   cgatctggaa attgagaccg ccagtgaaga acatggcgat ggggccaccg tatgtggagg 7380
   acgtctccac ctgcgcccga taccagtcaa tttggtcagc ctcaacgtcc ttctcagatc 7440
   gcttaacctt gcagttcttg tcggggatcc gttcggagcc atcaaaattg tgggacaaaa 7500
   tgaagaagaa cgtcaagaag gcaccagttg cggtgggcac cagaggaatg gtgatcagca 7560
   atgaggtccc agtgaggtac cagggcaaga atgcggtccg gaagatgaag aaagctcgca 7620
   tcacgaatgc aagtgtgcgg tagcgccgca gaaagccatt ttcccgcatg gccgtgacag 7680
   actctgggat ggtgtcattg tgctgcatcc ggaaaatgta gagcgggttg tacaccagcg 7740
   ataccccgta tgcccccagc acaaggtaca tgtaccaagc ctggaagcgg tggaaccact 7800
   ttcgggcggt gtttgcggcg gggtagttgt ggaacaccag gaacggctct gccgactccg 7860
   catccaggtc gaggccgtgc tggttggtgt aggtgtggtg ccgcatgatg tgcgactgca 7920
   gccaaatcca ccgggacgat ccgatgacgt caatgccgta ggcgaagagg gcgttgaccc 7980
   aaggcttctt gctgatggcc ccgtgggacg catcatgctg gatggataag ccgatctgca 8040
   tgtgcatgaa tccagtcaac aggccccaca gcaccatccc cgtggtagcc cagtgccact 8100
   cgcaaaaggc cgtcacggcg atgatcccaa cggtgcgcag ccagaagcca ggggttgcgt 8160
   accagttcct ccctttcatc acctcgcgca ttgtccgctt aacgtcttgt gcgaagggag 8220
   aatcatacgt gtagacaatg tccttcgagg tgcggtcatg cttccctcgg acgaactgtt 8280
   gaagcaattt ggagttctcc ggtttgacgt atggatgcat tgaataaaag agaggggagg 8340
   catcagaggc accagaagcg agaatcaaat ctcctcctgg atgaactttg gcaagccctt 8400
   caaggtcgta gaggatgcca tcaatcgcaa ccaaatcagg gcgttctaac agctgttctg 8460
   tggtaagact gagagccatg gagagctggg ttagtttgtg tagagagtgt gtgttgctag 8520
   cgactttcgg attgtgtcat tacacaaaac gcgtcgtctc gacactgatc ttgtcgtgga 8580
   tactcacggc tcggacatcg tcgccgacga tgacaccgga ctttcgctta aggacgtcag 8640
   taacaggcat tgtgtgatgt gtagtttaga tttcgaatct gtggggaaag aaaggaaaaa 8700
   agagactggc aaccgattgg gagagccact gtttatatat accctagaca agccccccgc 8760
   ttgtaagatg ttggtcaatg taaaccagta ttaaggttgg caagtgcagg agaagcaagg 8820
   tgtgggtacc gagcaatgga aatgtgcgga aggcaaaaaa atgaggccac ggcctattgt 8880
   cggggctata tccagggggc gattgaagta cactaacatg acatgtgtcc acagaccctc 8940
   aatctggcct gatgagccaa atccatacgc gctttcgcag ctctaaaggc tataacaagt 9000
   cacaccaccc tgctcgacct cagcgccctc actttttgtt aagacaaact gtacacgctg 9060
   ttccagcgtt ttctgcctgc acctggtggg acatttggtg caacctaaag tgctcggaac 9120
   ctctgtggtg tccagatcag cgcagcagtt ccgaggtagt tttgaggccc ttagatgatg 9180
   caatggtgtc agtcgctgga tcacgagtct taatggcagt attcgttctt atttgtgcca 9240
   ttgagccccg ttatcctcgt atcttctacc ccccatccca tccctttgtt ggtgcaaccc 9300
   tacccattta ttgttgggtg cagcccaacc gacgtggaga gcttggcttg gccatataaa 9360
   aaggcccccc cctagtggca atggcagaaa gtcagctgtg agttgttgaa tttgtcatct 9420
   aggcggcctg gccgtcttct ccggggcaat tggggctgtt ttttgggaca caaatacgcc 9480
   gccaacccgg tctctcctga attccgtcgt cgcctgagtc gacatcattt atttaccagt 9540
   tggccacaaa cccttgacga tctcgtatgt cccctccgac atactcccgg ccggctgggg 9600
   tacgttcgat agcgctatcg gcatcgacaa ggtttgggtc cctagccgat accgcactac 9660
   ctgagtcaca atcttcggag gtttagtctt ccacatagca cgggcaaaag tgcgtatata 9720
   tacaagagcg tttgccagcc acagattttc actccacaca ccacatcaca catacaacca 9780
   cacacatcca caatggaacc cgaaactaag aagaccaaga ctgactccaa gaagattgtt 9840
   cttctcggcg gcgacttctg tggccccgag gtgattgccg aggccgtcaa ggtgctcaag 9900
   tctgttgctg aggcctccgg caccgagttt gtgtttgagg accgactcat tggaggagct 9960
   gccattgaga aggagggcga gcccatcacc gacgctactc tcgacatctg ccgaaaggct 10020
   gactctatta tgctcggtgc tgtcggaggc gctgccaaca ccgtatggac cactcccgac 10080
   ggacgaaccg acgtgcgacc cgagcagggt ctcctcaagc tgcgaaagga cctgaacctg 10140
   tacgccaacc tgcgaccctg ccagctgctg tcgcccaagc tcgccgatct ctcccccatc 10200
   cgaaacgttg agggcaccga cttcatcatt gtccgagagc tcgtcggagg tatctacttt 10260
   ggagagcgaa aggaggatga cggatctggc gtcgcttccg acaccgagac ctactccgtt 10320
   cctgaggttg agcgaattgc ccgaatggcc gccttcctgg cccttcagca caacccccct 10380
   cttcccgtgt ggtctcttga caaggccaac gtgctggcct cctctcgact ttggcgaaag 10440
   actgtcactc gagtcctcaa ggacgaactc ccccagctcg agctcaacca ccagctgatc 10500
   gactcggccg ccatgatcct catcaagcag ccctccaaga tgaatggtat catcatcacc 10560
   accaacatgt ttggcgatat catctccgac gaggcctccg tcatccccgg ttctctgggt 10620
   ctgctgccct ccgcctctct ggcttctctg cccgacacca acgaggcgtt cggtctgtac 10680
   gagccctgtc acggatctgc ccccgatctc ggcaagcaga aggtcaaccc cattgccacc 10740
   attctgtctg ccgccatgat gctcaagttc tctcttaaca tgaagcccgc cggtgacgct 10800
   gttgaggctg ccgtcaagga gtccgtcgag gctggtatca ctaccgccga tatcggaggc 10860
   tcttcctcca cctccgaggt cggagacttg ttgccaacaa ggtcaaggag ctgctcaaga 10920
   aggagtaagt cgtttctacg acgcattgat ggaaggagca aactgacgcg cctgcgggtt 10980
   ggtctaccgg cagggtccgc tagtgtataa gactctataa aaagggccct gccctgctaa 11040
   tgaaatgatg atttataatt taccggtgta gcaaccttga ctagaagaag cagattgggt 11100
   gtgtttgtag tggaggacag tggtacgttt tggaaacagt cttcttgaaa gtgtcttgtc 11160
   tacagtatat tcactcataa cctcaatagc caagggtgta gtcggtttat taaaggaagg 11220
   gagttgtggc tgatgtggat atcgatagtt ggagcaaggg agaaatgtag agtgtgaaag 11280
   actcactatg gtccgggctt atctcgacca atagccaaag tctggagttt ctgagagaaa 11340
   aaggcaagat acgtatgtaa caaagcgacg catggtacaa taataccgga ggcatgtatc 11400
   atagagagtt agtggttcga tgatggcact ggtgcctggt atgactttat acggctgact 11460
   acatatttgt cctcagacat acaattacag tcaagcactt acccttggac atctgtaggt 11520
   accccccggc caagacgatc tcagcgtgtc gtatgtcgga ttggcgtagc tccctcgctc 11580
   gtcaattggc tcccatctac tttcttctgc ttggctacac ccagcatgtc tgctatggct 11640
   cgttttcgtg ccttatctat cctcccagta ttaccaactc taaatgacat gatgtgattg 11700
   ggtctacact ttcatatcag agataaggag tagcacagtt gcataaaaag cccaactcta 11760
   atcagcttct tcctttcttg taattagtac aaaggtgatt agcgaaatct ggaagcttag 11820
   ttggccctaa aaaaatcaaa aaaagcaaaa aacgaaaaac gaaaaaccac agttttgaga 11880
   acagggaggt aacgaaggat cgtatatata tatatatata tatataccca cggatcccga 11940
   gaccggcctt tgattcttcc ctacaaccaa ccattctcac caccctaatt cacaaccatg 12000
   gctcccgacg ccgacaagct gcgacagcga aaggctcagt ccatccagga cactgccgat 12060
   tctcaggcta ccgagctcaa gattggcacc ctgaagggtc tccaaggcac cgagatcgtc 12120
   attgatggcg acatctacga catcaaagac ttcgatcacc ctggaggcga atccatcatg 12180
   acctttggtg gcaacgacgt tactgccacc tacaagatga ttcatcccta ccactcgaag 12240
   catcacctgg agaagatgaa aaaggtcggt cgagtgcccg actacacctc cgagtacaag 12300
   ttcgatactc ccttcgaacg agagatcaaa caggaggtct tcaagattgt gcgaagaggt 12360
   cgagagtttg gaacacctgg ctacttcttt cgagccttct gctacatcgg tctcttcttt 12420
   tacctgcagt atctctgggt taccactcct accactttcg cccttgctat cttctacggt 12480
   gtgtctcagg ccttcattgg cctgaacgtc cagcacgacg ccaaccacgg agctgcctcc 12540
   aaaaagccct ggatcaacaa tttgctcggc ctgggtgccg actttatcgg aggctccaag 12600
   tggctctgga tgaaccagca ctggacccat cacacttaca ccaaccatca cgagaaggat 12660
   cccgacgccc tgggtgcaga gcctatgctg ctcttcaacg actatccctt gggtcacccc 12720
   aagcgaaccc tcattcatca cttccaagcc ttctactatc tgtttgtcct tgctggctac 12780
   tgggtgtctt cggtgttcaa ccctcagatc ctggacctcc agcaccgagg tgcccaggct 12840
   gtcggcatga agatggagaa cgactacatt gccaagtctc gaaagtacgc tatcttcctg 12900
   cgactcctgt acatctacac caacattgtg gctcccatcc agaaccaagg cttttcgctc 12960
   accgtcgttg ctcacattct tactatgggt gtcgcctcca gcctgaccct cgctactctg 13020
   ttcgccctct cccacaactt cgagaacgca gatcgggatc ccacctacga ggctcgaaag 13080
   ggaggcgagc ctgtctgttg gttcaagtcg caggtggaaa cctcctctac ttacggtggc 13140
   ttcatttccg gttgccttac aggcggactc aactttcagg tcgagcatca cctgtttcct 13200
   cgaatgtcct ctgcctggta cccctacatc gctcctaccg ttcgagaggt ctgcaaaaag 13260
   cacggcgtca agtacgccta ctatccctgg gtgtggcaga acctcatctc gaccgtcaag 13320
   tacctgcatc agtccggaac tggctcgaac tggaagaacg gtgccaatcc ctactctggc 13380
   aagctgtaag cggccgcatg tacatacaag attatttata gaaatgaatc gcgatcgaac 13440
   aaagagtacg agtgtacgag taggggatga tgataaaagt ggaagaagtt ccgcatcttt 13500
   ggatttatca acgtgtagga cgatacttcc tgtaaaaatg caatgtcttt accataggtt 13560
   ctgctgtaga tgttattaac taccattaac atgtctactt gtacagttgc agaccagttg 13620
   gagtatagaa tggtacactt accaaaaagt gttgatggtt gtaactacga tatataaaac   13680
   tgttgacggg atctgcgtac actgttt                                       13707
<210> 43
   <211> 1350
   <212> DNA
   <213> Euglena gracilis
<220>
   <221> CDS
   <222> (1)..(1350)
   <223> synthetic delta-5 desaturase (codon-optimized for Yarrowia lipolytica)
<400> 43
   atg gct ctc tcc ctt act acc gag cag ctg ctc gag cga ccc gac ctg        48
   Met Ala Leu Ser Leu Thr Thr Glu Gln Leu Leu Glu Arg Pro Asp Leu
   1               5                   10                  15
   gtt gcc atc gac ggc att ctc tac gat ctg gaa ggt ctt gcc aag gtc        96
   Val Ala Ile Asp Gly Ile Leu Tyr Asp Leu Glu Gly Leu Ala Lys Val
               20                  25                  30
   cat ccc gga ggc gac ttg atc ctc gct tct ggt gcc tcc gat gct tct       144
   His Pro Gly Gly Asp Leu Ile Leu Ala Ser Gly Ala Ser Asp Ala Ser
           35                  40                  45
   cct ctg ttc tac tcc atg cac cct tac gtc aag ccc gag aac tcg aag       192
   Pro Leu Phe Tyr Ser Met His Pro Tyr Val Lys Pro Glu Asn Ser Lys
       50                  55                  60
   ctg ctt caa cag ttc gtg cga ggc aag cac gac cga acc tcc aag gac       240
   Leu Leu Gln Gln Phe Val Arg Gly Lys His Asp Arg Thr Ser Lys Asp
   65                  70                  75                  80
   att gtc tac acc tac gac tct ccc ttt gca cag gac gtc aag cga act       288
   Ile Val Tyr Thr Tyr Asp Ser Pro Phe Ala Gln Asp Val Lys Arg Thr
                   85                  90                  95
   atg cga gag gtc atg aaa ggt cgg aac tgg tat gcc aca cct gga ttc       336
   Met Arg Glu Val Met Lys Gly Arg Asn Trp Tyr Ala Thr Pro Gly Phe
               100                 105                 110
   tgg ctg cga acc gtt ggc atc att gct gtc acc gcc ttt tgc gag tgg       384
   Trp Leu Arg Thr Val Gly Ile Ile Ala Val Thr Ala Phe Cys Glu Trp
           115                 120                 125
   cac tgg gct act acc gga atg gtg ctg tgg ggt ctc ttg act gga ttc       432
   His Trp Ala Thr Thr Gly Met Val Leu Trp Gly Leu Leu Thr Gly Phe
       130                 135                 140
   atg cac atg cag atc ggc ctg tcc att cag cac gat gcc tct cat ggt       480
   Met His Met Gln Ile Gly Leu Ser Ile Gln His Asp Ala Ser His Gly
   145                 150                 155                 160
   gcc atc agc aaa aag ccc tgg gtc aac gct ctc ttt gcc tac ggc atc       528
   Ala Ile Ser Lys Lys Pro Trp Val Asn Ala Leu Phe Ala Tyr Gly Ile
                   165                 170                 175
   gac gtc att gga tcg tcc aga tgg atc tgg ctg cag tct cac atc atg      576
   Asp Val Ile Gly Ser Ser Arg Trp Ile Trp Leu Gln Ser His Ile Met
               180                 185                 190
   cga cat cac acc tac acc aat cag cat ggt ctc gac ctg gat gcc gag      624
   Arg His His Thr Tyr Thr Asn Gln His Gly Leu Asp Leu Asp Ala Glu
           195                 200                 205
   tcc gca gaa cca ttc ctt gtg ttc cac aac tac cct gct gcc aac act      672
   Ser Ala Glu Pro Phe Leu Val Phe His Asn Tyr Pro Ala Ala Asn Thr
       210                 215                 220
   gct cga aag tgg ttt cac cga ttc cag gcc tgg tac atg tac ctc gtg      720
   Ala Arg Lys Trp Phe His Arg Phe Gln Ala Trp Tyr Met Tyr Leu Val
   225                 230                 235                 240
   ctt gga gcc tac ggc gtt tcg ctg gtg tac aac cct ctc tac atc ttc      768
   Leu Gly Ala Tyr Gly Val Ser Leu Val Tyr Asn Pro Leu Tyr Ile Phe
                   245                 250                 255
   cga atg cag cac aac gac acc att ccc gag tct gtc aca gcc atg cga      816
   Arg Met Gln His Asn Asp Thr Ile Pro Glu Ser Val Thr Ala Met Arg
               260                 265                 270
   gag aac ggc ttt ctg cga cgg tac cga acc ctt gca ttc gtt atg cga      864
   Glu Asn Gly Phe Leu Arg Arg Tyr Arg Thr Leu Ala Phe Val Met Arg
           275                 280                 285
   gct ttc ttc atc ttt cga acc gcc ttc ttg ccc tgg tat ctc act gga      912
   Ala Phe Phe Ile Phe Arg Thr Ala Phe Leu Pro Trp Tyr Leu Thr Gly
       290                 295                 300
   acc tcc ctg ctc atc acc att cct ctg gtg ccc act gct acc ggt gcc      960
   Thr Ser Leu Leu Ile Thr Ile Pro Leu Val Pro Thr Ala Thr Gly Ala
   305                 310                 315                 320
   ttc ctc acc ttc ttt ttc atc ttg tct cac aac ttc gat ggc tcg gag     1008
   Phe Leu Thr Phe Phe Phe Ile Leu Ser His Asn Phe Asp Gly Ser Glu
                   325                 330                 335
   cga atc ccc gac aag aac tgc aag gtc aag agc tcc gag aag gac gtt     1056
   Arg Ile Pro Asp Lys Asn Cys Lys Val Lys Ser Ser Glu Lys Asp Val
               340                 345                 350
   gaa gcc gat cag atc gac tgg tac aga gct cag gtg gag acc tct tcc     1104
   Glu Ala Asp Gln Ile Asp Trp Tyr Arg Ala Gln Val Glu Thr Ser Ser
           355                 360                 365
   acc tac ggt gga ccc att gcc atg ttc ttt act ggc ggt ctc aac ttc     1152
   Thr Tyr Gly Gly Pro Ile Ala Met Phe Phe Thr Gly Gly Leu Asn Phe
       370                 375                 380
   cag atc gag cat cac ctc ttt cct cga atg tcg tct tgg cac tat ccc     1200
   Gln Ile Glu His His Leu Phe Pro Arg Met Ser Ser Trp His Tyr Pro
   385                 390                 395                 400
   ttc gtg cag caa gct gtc cga gag tgt tgc gaa cga cac gga gtt cgg     1248
   Phe Val Gln Gln Ala Val Arg Glu Cys Cys Glu Arg His Gly Val Arg
                   405                 410                 415
   tac gtc ttc tac cct acc att gtg ggc aac atc att tcc acc ctc aag      1296
   Tyr Val Phe Tyr Pro Thr Ile Val Gly Asn Ile Ile Ser Thr Leu Lys
               420                 425                 430
   tac atg cac aaa gtc ggt gtg gtt cac tgt gtc aag gac gct cag gat      1344
   Tyr Met His Lys Val Gly Val Val His Cys Val Lys Asp Ala Gln Asp
           435                 440                 445
   tcc taa                                                              1350
   Ser
<210> 44
   <211> 449
   <212> PRT
   <213> Euglena gracilis
<400> 44
   Met Ala Leu Ser Leu Thr Thr Glu Gln Leu Leu Glu Arg Pro Asp Leu
   1               5                   10                  15
   Val Ala Ile Asp Gly Ile Leu Tyr Asp Leu Glu Gly Leu Ala Lys Val
               20                  25                  30
   His Pro Gly Gly Asp Leu Ile Leu Ala Ser Gly Ala Ser Asp Ala Ser
           35                  40                  45
   Pro Leu Phe Tyr Ser Met His Pro Tyr Val Lys Pro Glu Asn Ser Lys
       50                  55                  60
   Leu Leu Gln Gln Phe Val Arg Gly Lys His Asp Arg Thr Ser Lys Asp
   65                  70                  75                  80
   Ile Val Tyr Thr Tyr Asp Ser Pro Phe Ala Gln Asp Val Lys Arg Thr
                   85                  90                  95
   Met Arg Glu Val Met Lys Gly Arg Asn Trp Tyr Ala Thr Pro Gly Phe
               100                 105                 110
   Trp Leu Arg Thr Val Gly Ile Ile Ala Val Thr Ala Phe Cys Glu Trp
           115                 120                 125
   His Trp Ala Thr Thr Gly Met Val Leu Trp Gly Leu Leu Thr Gly Phe
       130                 135                 140
   Met His Met Gln Ile Gly Leu Ser Ile Gln His Asp Ala Ser His Gly
   145                 150                 155                 160
   Ala Ile Ser Lys Lys Pro Trp Val Asn Ala Leu Phe Ala Tyr Gly Ile
   165 170 175
   Asp Val Ile Gly Ser Ser Arg Trp Ile Trp Leu Gln Ser His Ile Met
   180 185 190
   Arg His His Thr Tyr Thr Asn Gln His Gly Leu Asp Leu Asp Ala Glu
   195 200 205
   Ser Ala Glu Pro Phe Leu Val Phe His Asn Tyr Pro Ala Ala Asn Thr
   210 215 220
   Ala Arg Lys Trp Phe His Arg Phe Gln Ala Trp Tyr Met Tyr Leu Val
   225 230 235 240
   Leu Gly Ala Tyr Gly Val Ser Leu Val Tyr Asn Pro Leu Tyr Ile Phe
   245 250 255
   Arg Met Gln His Asn Asp Thr Ile Pro Glu Ser Val Thr Ala Met Arg
   260 265 270
   Glu Asn Gly Phe Leu Arg Arg Tyr Arg Thr Leu Ala Phe Val Met Arg
   275 280 285
   Ala Phe Phe Ile Phe Arg Thr Ala Phe Leu Pro Trp Tyr Leu Thr Gly
   290 295 300
   Thr Ser Leu Leu Ile Thr Ile Pro Leu Val Pro Thr Ala Thr Gly Ala
   305 310 315 320
   Phe Leu Thr Phe Phe Phe Ile Leu Ser His Asn Phe Asp Gly Ser Glu
   325 330 335
   Arg Ile Pro Asp Lys Asn Cys Lys Val Lys Ser Ser Glu Lys Asp Val
   340 345 350
   Glu Ala Asp Gln Ile Asp Trp Tyr Arg Ala Gln Val Glu Thr Ser Ser
   355 360 365
   Thr Tyr Gly Gly Pro Ile Ala Met Phe Phe Thr Gly Gly Leu Asn Phe
   370 375 380
   Gln Ile Glu His His Leu Phe Pro Arg Met Ser Ser Trp His Tyr Pro
   385 390 395 400
   Phe Val Gln Gln Ala Val Arg Glu Cys Cys Glu Arg His Gly Val Arg
   405 410 415
   Tyr Val Phe Tyr Pro Thr Ile Val Gly Asn Ile Ile Ser Thr Leu Lys
               420                 425                 430
   Tyr Met His Lys Val Gly Val Val His Cys Val Lys Asp Ala Gln Asp
           435                 440                 445
   Ser
<210> 45
   <211> 1392
   <212> DNA
   <213> Peridinium sp. CCMP626
<220>
   <221> CDS
   <222> (1)..(1392)
   <223> synthetic delta-5 desaturase (codon-optimized for Yarrowia lipolytica)
<400> 45
   atg gct ccc gac gcc gac aag ctg cga cag cga aag gct cag tcc atc      48
   Met Ala Pro Asp Ala Asp Lys Leu Arg Gln Arg Lys Ala Gln Ser Ile
   1               5                   10                  15
   cag gac act gcc gat tct cag gct acc gag ctc aag att ggc acc ctg      96
   Gln Asp Thr Ala Asp Ser Gln Ala Thr Glu Leu Lys Ile Gly Thr Leu
               20                  25                  30
   aag ggt ctc caa ggc acc gag atc gtc att gat ggc gac atc tac gac     144
   Lys Gly Leu Gln Gly Thr Glu Ile Val Ile Asp Gly Asp Ile Tyr Asp
           35                  40                  45
   atc aaa gac ttc gat cac cct gga ggc gaa tcc atc atg acc ttt ggt     192
   Ile Lys Asp Phe Asp His Pro Gly Gly Glu Ser Ile Met Thr Phe Gly
       50                  55                  60
   ggc aac gac gtt act gcc acc tac aag atg att cat ccc tac cac tcg     240
   Gly Asn Asp Val Thr Ala Thr Tyr Lys Met Ile His Pro Tyr His Ser
   65                  70                  75                  80
   aag cat cac ctg gag aag atg aaa aag gtc ggt cga gtg ccc gac tac     288
   Lys His His Leu Glu Lys Met Lys Lys Val Gly Arg Val Pro Asp Tyr
                   85                  90                  95
   acc tcc gag tac aag ttc gat act ccc ttc gaa cga gag atc aaa cag     336
   Thr Ser Glu Tyr Lys Phe Asp Thr Pro Phe Glu Arg Glu Ile Lys Gln
               100                 105                 110
   gag gtc ttc aag att gtg cga aga ggt cga gag ttt gga aca cct ggc     384
   Glu Val Phe Lys Ile Val Arg Arg Gly Arg Glu Phe Gly Thr Pro Gly
           115                 120                 125
   tac ttc ttt cga gcc ttc tgc tac atc ggt ctc ttc ttt tac ctg cag     432
   Tyr Phe Phe Arg Ala Phe Cys Tyr Ile Gly Leu Phe Phe Tyr Leu Gln
       130                 135                 140
   tat ctc tgg gtt acc act cct acc act ttc gcc ctt gct atc ttc tac      480
   Tyr Leu Trp Val Thr Thr Pro Thr Thr Phe Ala Leu Ala Ile Phe Tyr
   145                 150                 155                 160
   ggt gtg tct cag gcc ttc att ggc ctg aac gtc cag cac gac gcc aac      528
   Gly Val Ser Gln Ala Phe Ile Gly Leu Asn Val Gln His Asp Ala Asn
                   165                 170                 175
   cac gga gct gcc tcc aaa aag ccc tgg atc aac aat ttg ctc ggc ctg      576
   His Gly Ala Ala Ser Lys Lys Pro Trp Ile Asn Asn Leu Leu Gly Leu
               180                 185                 190
   ggt gcc gac ttt atc gga ggc tcc aag tgg ctc tgg atg aac cag cac      624
   Gly Ala Asp Phe Ile Gly Gly Ser Lys Trp Leu Trp Met Asn Gln His
           195                 200                 205
   tgg acc cat cac act tac acc aac cat cac gag aag gat ccc gac gcc      672
   Trp Thr His His Thr Tyr Thr Asn His His Glu Lys Asp Pro Asp Ala
       210                 215                 220
   ctg ggt gca gag cct atg ctg ctc ttc aac gac tat ccc ttg ggt cac      720
   Leu Gly Ala Glu Pro Met Leu Leu Phe Asn Asp Tyr Pro Leu Gly His
   225                 230                 235                 240
   ccc aag cga acc ctc att cat cac ttc caa gcc ttc tac tat ctg ttt      768
   Pro Lys Arg Thr Leu Ile His His Phe Gln Ala Phe Tyr Tyr Leu Phe
                   245                 250                 255
   gtc ctt gct ggc tac tgg gtg tct tcg gtg ttc aac cct cag atc ctg      816
   Val Leu Ala Gly Tyr Trp Val Ser Ser Val Phe Asn Pro Gln Ile Leu
               260                 265                 270
   gac ctc cag cac cga ggt gcc cag gct gtc ggc atg aag atg gag aac      864
   Asp Leu Gln His Arg Gly Ala Gln Ala Val Gly Met Lys Met Glu Asn
           275                 280                 285
   gac tac att gcc aag tct cga aag tac gct atc ttc ctg cga ctc ctg      912
   Asp Tyr Ile Ala Lys Ser Arg Lys Tyr Ala Ile Phe Leu Arg Leu Leu
       290                 295                 300
   tac atc tac acc aac att gtg gct ccc atc cag aac caa ggc ttt tcg      960
   Tyr Ile Tyr Thr Asn Ile Val Ala Pro Ile Gln Asn Gln Gly Phe Ser
   305                 310                 315                 320
   ctc acc gtc gtt gct cac att ctt act atg ggt gtc gcc tcc agc ctg     1008
   Leu Thr Val Val Ala His Ile Leu Thr Met Gly Val Ala Ser Ser Leu
                   325                 330                 335
   acc ctc gct act ctg ttc gcc ctc tcc cac aac ttc gag aac gca gat     1056
   Thr Leu Ala Thr Leu Phe Ala Leu Ser His Asn Phe Glu Asn Ala Asp
               340                 345                 350
   cgg gat ccc acc tac gag gct cga aag gga ggc gag cct gtc tgt tgg     1104
   Arg Asp Pro Thr Tyr Glu Ala Arg Lys Gly Gly Glu Pro Val Cys Trp
           355                 360                 365
   ttc aag tcg cag gtg gaa acc tcc tct act tac ggt ggc ttc att tcc     1152
   Phe Lys Ser Gln Val Glu Thr Ser Ser Thr Tyr Gly Gly Phe Ile Ser
       370                 375                 380
   ggt tgc ctt aca ggc gga ctc aac ttt cag gtc gag cat cac ctg ttt      1200
   Gly Cys Leu Thr Gly Gly Leu Asn Phe Gln Val Glu His His Leu Phe
   385                 390                 395                 400
   cct cga atg tcc tct gcc tgg tac ccc tac atc gct cct acc gtt cga      1248
   Pro Arg Met Ser Ser Ala Trp Tyr Pro Tyr Ile Ala Pro Thr Val Arg
                   405                 410                 415
   gag gtc tgc aaa aag cac ggc gtc aag tac gcc tac tat ccc tgg gtg      1296
   Glu Val Cys Lys Lys His Gly Val Lys Tyr Ala Tyr Tyr Pro Trp Val
               420                 425                 430
   tgg cag aac ctc atc tcg acc gtc aag tac ctg cat cag tcc gga act      1344
   Trp Gln Asn Leu Ile Ser Thr Val Lys Tyr Leu His Gln Ser Gly Thr
           435                 440                 445
   ggc tcg aac tgg aag aac ggt gcc aat ccc tac tct ggc aag ctg taa      1392
   Gly Ser Asn Trp Lys Asn Gly Ala Asn Pro Tyr Ser Gly Lys Leu
       450                 455                 460
<210> 46
   <211> 463
   <212> PRT
   <213> Peridinium sp. CCMP626
<400> 46
   Met Ala Pro Asp Ala Asp Lys Leu Arg Gln Arg Lys Ala Gln Ser Ile
   1               5                   10                  15
   Gln Asp Thr Ala Asp Ser Gln Ala Thr Glu Leu Lys Ile Gly Thr Leu
               20                  25                  30
   Lys Gly Leu Gln Gly Thr Glu Ile Val Ile Asp Gly Asp Ile Tyr Asp
           35                  40                  45
   Ile Lys Asp Phe Asp His Pro Gly Gly Glu Ser Ile Met Thr Phe Gly
       50                  55                  60
   Gly Asn Asp Val Thr Ala Thr Tyr Lys Met Ile His Pro Tyr His Ser
   65                  70                  75                  80
   Lys His His Leu Glu Lys Met Lys Lys Val Gly Arg Val Pro Asp Tyr
                   85                  90                  95
   Thr Ser Glu Tyr Lys Phe Asp Thr Pro Phe Glu Arg Glu Ile Lys Gln
               100                 105                 110
   Glu Val Phe Lys Ile Val Arg Arg Gly Arg Glu Phe Gly Thr Pro Gly
           115                 120                 125
   Tyr Phe Phe Arg Ala Phe Cys Tyr Ile Gly Leu Phe Phe Tyr Leu Gln
       130                 135                 140
   Tyr Leu Trp Val Thr Thr Pro Thr Thr Phe Ala Leu Ala Ile Phe Tyr
   145                 150                 155                 160
   Gly Val Ser Gln Ala Phe Ile Gly Leu Asn Val Gln His Asp Ala Asn
                   165                 170                 175
   His Gly Ala Ala Ser Lys Lys Pro Trp Ile Asn Asn Leu Leu Gly Leu
               180                 185                 190
   Gly Ala Asp Phe Ile Gly Gly Ser Lys Trp Leu Trp Met Asn Gln His
           195                 200                 205
   Trp Thr His His Thr Tyr Thr Asn His His Glu Lys Asp Pro Asp Ala
       210                 215                 220
   Leu Gly Ala Glu Pro Met Leu Leu Phe Asn Asp Tyr Pro Leu Gly His
   225                 230                 235                 240
   Pro Lys Arg Thr Leu Ile His His Phe Gln Ala Phe Tyr Tyr Leu Phe
                   245                 250                 255
   Val Leu Ala Gly Tyr Trp Val Ser Ser Val Phe Asn Pro Gln Ile Leu
               260                 265                 270
   Asp Leu Gln His Arg Gly Ala Gln Ala Val Gly Met Lys Met Glu Asn
           275                 280                 285
   Asp Tyr Ile Ala Lys Ser Arg Lys Tyr Ala Ile Phe Leu Arg Leu Leu
       290                 295                 300
   Tyr Ile Tyr Thr Asn Ile Val Ala Pro Ile Gln Asn Gln Gly Phe Ser
   305                 310                 315                 320
   Leu Thr Val Val Ala His Ile Leu Thr Met Gly Val Ala Ser Ser Leu
                   325                 330                 335
   Thr Leu Ala Thr Leu Phe Ala Leu Ser His Asn Phe Glu Asn Ala Asp
               340                 345                 350
   Arg Asp Pro Thr Tyr Glu Ala Arg Lys Gly Gly Glu Pro Val Cys Trp
           355                 360                 365
   Phe Lys Ser Gln Val Glu Thr Ser Ser Thr Tyr Gly Gly Phe Ile Ser
   370 375 380
   Gly Cys Leu Thr Gly Gly Leu Asn Phe Gln Val Glu His His Leu Phe
   385 390 395 400
   Pro Arg Met Ser Ser Ala Trp Tyr Pro Tyr Ile Ala Pro Thr Val Arg
   405 410 415
   Glu Val Cys Lys Lys His Gly Val Lys Tyr Ala Tyr Tyr Pro Trp Val
   420 425 430
   Trp Gln Asn Leu Ile Ser Thr Val Lys Tyr Leu His Gln Ser Gly Thr
   435 440 445
   Gly Ser Asn Trp Lys Asn Gly Ala Asn Pro Tyr Ser Gly Lys Leu
   450 455 460
<210> 47
   <211> 1350
   <212> DNA
   <213> Euglena gracilis
<220>
   <221> CDS
   <222> (1)..(1350)
   <223> delta-5 desaturase
<400> 47
   atg gct ctc agt ctt acc aca gaa cag ctg tta gaa cgc cct gat ttg          48
   Met Ala Leu Ser Leu Thr Thr Glu Gln Leu Leu Glu Arg Pro Asp Leu
   1               5                   10                  15
   gtt gcg att gat ggc atc ctc tac gac ctt gaa ggg ctt gcc aaa gtt          96
   Val Ala Ile Asp Gly Ile Leu Tyr Asp Leu Glu Gly Leu Ala Lys Val
               20                  25                  30
   cat cca gga gga gat ttg att ctc gct tct ggt gcc tct gat gcc tcc         144
   His Pro Gly Gly Asp Leu Ile Leu Ala Ser Gly Ala Ser Asp Ala Ser
           35                  40                  45
   cct ctc ttt tat tca atg cat cca tac gtc aaa ccg gag aat tcc aaa         192
   Pro Leu Phe Tyr Ser Met His Pro Tyr Val Lys Pro Glu Asn Ser Lys
       50                  55                  60
   ttg ctt caa cag ttc gtc cga ggg aag cat gac cgc acc tcg aag gac         240
   Leu Leu Gln Gln Phe Val Arg Gly Lys His Asp Arg Thr Ser Lys Asp
   65                  70                  75                  80
   att gtc tac acg tat gat tct ccc ttc gca caa gac gtt aag cgg aca         288
   Ile Val Tyr Thr Tyr Asp Ser Pro Phe Ala Gln Asp Val Lys Arg Thr
                   85                  90                  95
   atg cgc gag gtg atg aaa ggg agg aac tgg tac gca acc cct ggc ttc         336
   Met Arg Glu Val Met Lys Gly Arg Asn Trp Tyr Ala Thr Pro Gly Phe
                   100                 105                 110
   tgg ctg cgc acc gtt ggg atc atc gcc gtg acg gcc ttt tgc gag tgg      384
   Trp Leu Arg Thr Val Gly Ile Ile Ala Val Thr Ala Phe Cys Glu Trp
           115                 120                 125
   cac tgg gct acc acg ggg atg gtg ctg tgg ggc ctg ttg act gga ttc      432
   His Trp Ala Thr Thr Gly Met Val Leu Trp Gly Leu Leu Thr Gly Phe
       130                 135                 140
   atg cac atg cag atc ggc tta tcc atc cag cat gat gcg tcc cac ggg      480
   Met His Met Gln Ile Gly Leu Ser Ile Gln His Asp Ala Ser His Gly
   145                 150                 155                 160
   gcc atc agc aag aag cct tgg gtc aac gcc ctc ttc gcc tac ggc att      528
   Ala Ile Ser Lys Lys Pro Trp Val Asn Ala Leu Phe Ala Tyr Gly Ile
                   165                 170                 175
   gac gtc atc gga tcg tcc cgg tgg att tgg ctg cag tcg cac atc atg      576
   Asp Val Ile Gly Ser Ser Arg Trp Ile Trp Leu Gln Ser His Ile Met
               180                 185                 190
   cgg cac cac acc tac acc aac cag cac ggc ctc gac ctg gat gcg gag      624
   Arg His His Thr Tyr Thr Asn Gln His Gly Leu Asp Leu Asp Ala Glu
           195                 200                 205
   tcg gca gag ccg ttc ctg gtg ttc cac aac tac ccc gcc gca aac acc      672
   Ser Ala Glu Pro Phe Leu Val Phe His Asn Tyr Pro Ala Ala Asn Thr
       210                 215                 220
   gcc cga aag tgg ttc cac cgc ttc caa gct tgg tac atg tac ctt gtg      720
   Ala Arg Lys Trp Phe His Arg Phe Gln Ala Trp Tyr Met Tyr Leu Val
   225                 230                 235                 240
   ctg ggg gca tac ggg gta tcg ctg gtg tac aac ccg ctc tac att ttc      768
   Leu Gly Ala Tyr Gly Val Ser Leu Val Tyr Asn Pro Leu Tyr Ile Phe
                   245                 250                 255
   cgg atg cag cac aat gac acc atc cca gag tct gtc acg gcc atg cgg      816
   Arg Met Gln His Asn Asp Thr Ile Pro Glu Ser Val Thr Ala Met Arg
               260                 265                 270
   gag aat ggc ttt ctg cgg cgc tac cgc aca ctt gca ttc gtg atg cga      864
   Glu Asn Gly Phe Leu Arg Arg Tyr Arg Thr Leu Ala Phe Val Met Arg
           275                 280                 285
   gct ttc ttc atc ttc cgg acc gca ttc ttg ccc tgg tac ctc act ggg      912
   Ala Phe Phe Ile Phe Arg Thr Ala Phe Leu Pro Trp Tyr Leu Thr Gly
       290                 295                 300
   acc tca ttg ctg atc acc att cct ctg gtg ccc act gca act ggt gcc      960
   Thr Ser Leu Leu Ile Thr Ile Pro Leu Val Pro Thr Ala Thr Gly Ala
   305                 310                 315                 320
   ttc ttg acg ttc ttc ttc att ttg tcc cac aat ttt gat ggc tcc gaa     1008
   Phe Leu Thr Phe Phe Phe Ile Leu Ser His Asn Phe Asp Gly Ser Glu
                   325                 330                 335
   cgg atc ccc gac aag aac tgc aag gtt aag agc tct gag aag gac gtt     1056
   Arg Ile Pro Asp Lys Asn Cys Lys Val Lys Ser Ser Glu Lys Asp Val
               340                 345                 350
   gag gct gac caa att gac tgg tat cgg gcg cag gtg gag acg tcc tcc      1104
   Glu Ala Asp Gln Ile Asp Trp Tyr Arg Ala Gln Val Glu Thr Ser Ser
           355                 360                 365
   aca tac ggt ggc ccc atc gcc atg ttc ttc act ggc ggt ctc aat ttc      1152
   Thr Tyr Gly Gly Pro Ile Ala Met Phe Phe Thr Gly Gly Leu Asn Phe
       370                 375                 380
   cag atc gag cac cac ctc ttt ccc cgg atg tcg tct tgg cac tac ccc      1200
   Gln Ile Glu His His Leu Phe Pro Arg Met Ser Ser Trp His Tyr Pro
   385                 390                 395                 400
   ttc gtc cag cag gcg gtc cgg gag tgt tgc gaa cgc cat gga gtg cga      1248
   Phe Val Gln Gln Ala Val Arg Glu Cys Cys Glu Arg His Gly Val Arg
                   405                 410                 415
   tat gtt ttc tac cct acc atc gtc ggc aac atc atc tcc acc ctg aag      1296
   Tyr Val Phe Tyr Pro Thr Ile Val Gly Asn Ile Ile Ser Thr Leu Lys
               420                 425                 430
   tac atg cat aag gtg ggt gtc gtc cac tgc gtg aag gac gca cag gat      1344
   Tyr Met His Lys Val Gly Val Val His Cys Val Lys Asp Ala Gln Asp
           435                 440                 445
   tcc tga                                                              1350
   Ser
<210> 48
   <211> 449
   <212> PRT
   <213> Euglena gracilis
<400> 48
   Met Ala Leu Ser Leu Thr Thr Glu Gln Leu Leu Glu Arg Pro Asp Leu
   1               5                   10                  15
   Val Ala Ile Asp Gly Ile Leu Tyr Asp Leu Glu Gly Leu Ala Lys Val
               20                  25                  30
   His Pro Gly Gly Asp Leu Ile Leu Ala Ser Gly Ala Ser Asp Ala Ser
           35                  40                  45
   Pro Leu Phe Tyr Ser Met His Pro Tyr Val Lys Pro Glu Asn Ser Lys
       50                  55                  60
   Leu Leu Gln Gln Phe Val Arg Gly Lys His Asp Arg Thr Ser Lys Asp
   65                  70                  75                  80
   Ile Val Tyr Thr Tyr Asp Ser Pro Phe Ala Gln Asp Val Lys Arg Thr
                   85                  90                  95
   Met Arg Glu Val Met Lys Gly Arg Asn Trp Tyr Ala Thr Pro Gly Phe
               100                 105                 110
   Trp Leu Arg Thr Val Gly Ile Ile Ala Val Thr Ala Phe Cys Glu Trp
           115                 120                 125
   His Trp Ala Thr Thr Gly Met Val Leu Trp Gly Leu Leu Thr Gly Phe
       130                 135                 140
   Met His Met Gln Ile Gly Leu Ser Ile Gln His Asp Ala Ser His Gly
   145                 150                 155                 160
   Ala Ile Ser Lys Lys Pro Trp Val Asn Ala Leu Phe Ala Tyr Gly Ile
                   165                 170                 175
   Asp Val Ile Gly Ser Ser Arg Trp Ile Trp Leu Gln Ser His Ile Met
               180                 185                 190
   Arg His His Thr Tyr Thr Asn Gln His Gly Leu Asp Leu Asp Ala Glu
           195                 200                 205
   Ser Ala Glu Pro Phe Leu Val Phe His Asn Tyr Pro Ala Ala Asn Thr
       210                 215                 220
   Ala Arg Lys Trp Phe His Arg Phe Gln Ala Trp Tyr Met Tyr Leu Val
   225                 230                 235                 240
   Leu Gly Ala Tyr Gly Val Ser Leu Val Tyr Asn Pro Leu Tyr Ile Phe
                   245                 250                 255
   Arg Met Gln His Asn Asp Thr Ile Pro Glu Ser Val Thr Ala Met Arg
               260                 265                 270
   Glu Asn Gly Phe Leu Arg Arg Tyr Arg Thr Leu Ala Phe Val Met Arg
           275                 280                 285
   Ala Phe Phe Ile Phe Arg Thr Ala Phe Leu Pro Trp Tyr Leu Thr Gly
       290                 295                 300
   Thr Ser Leu Leu Ile Thr Ile Pro Leu Val Pro Thr Ala Thr Gly Ala
   305                 310                 315                 320
   Phe Leu Thr Phe Phe Phe Ile Leu Ser His Asn Phe Asp Gly Ser Glu
                   325                 330                 335
   Arg Ile Pro Asp Lys Asn Cys Lys Val Lys Ser Ser Glu Lys Asp Val
   340 345 350
   Glu Ala Asp Gln Ile Asp Trp Tyr Arg Ala Gln Val Glu Thr Ser Ser
   355 360 365
   Thr Tyr Gly Gly Pro Ile Ala Met Phe Phe Thr Gly Gly Leu Asn Phe
   370 375 380
   Gln Ile Glu His His Leu Phe Pro Arg Met Ser Ser Trp His Tyr Pro
   385 390 395 400
   Phe Val Gln Gln Ala Val Arg Glu Cys Cys Glu Arg His Gly Val Arg
   405 410 415
   Tyr Val Phe Tyr Pro Thr Ile Val Gly Asn Ile Ile Ser Thr Leu Lys
   420 425 430
   Tyr Met His Lys Val Gly Val Val His Cys Val Lys Asp Ala Gln Asp
   435 440 445
   Ser
<210> 49
   <211> 13066
   <212> DNA
   <213> Artificial Sequence
<220>
   <223> Plasmid pZP3-Pa777U
<400> 49
   tctcggtcta ttcttttgat ttataaggga ttttgccgat ttcggcctat tggttaaaaa 60
   atgagctgat ttaacaaaaa tttaacgcga attttaacaa aatattaacg cttacaattt 120
   cctgatgcgg tattttctcc ttacgcatct gtgcggtatt tcacaccgca tcaggtggca 180
   cttttcgggg aaatgtgcgc ggaaccccta tttgtttatt tttctaaata cattcaaata 240
   tgtatccgct catgagacaa taaccctgat aaatgcttca ataatattga aaaaggaaga 300
   gtatgagtat tcaacatttc cgtgtcgccc ttattccctt ttttgcggca ttttgccttc 360
   ctgtttttgc tcacccagaa acgctggtga aagtaaaaga tgctgaagat cagttgggtg 420
   cacgagtggg ttacatcgaa ctggatctca acagcggtaa gatccttgag agttttcgcc 480
   ccgaagaacg ttttccaatg atgagcactt ttaaagttct gctatgtggc gcggtattat 540
   cccgtattga cgccgggcaa gagcaactcg gtcgccgcat acactattct cagaatgact 600
   tggttgagta ctcaccagtc acagaaaagc atcttacgga tggcatgaca gtaagagaat 660
   tatgcagtgc tgccataacc atgagtgata acactgcggc caacttactt ctgacaacga 720
   tcggaggacc gaaggagcta accgcttttt tgcacaacat gggggatcat gtaactcgcc 780
   ttgatcgttg ggaaccggag ctgaatgaag ccataccaaa cgacgagcgt gacaccacga 840
   tgcctgtagc aatggcaaca acgttgcgca aactattaac tggcgaacta cttactctag 900
   cttcccggca acaattaata gactggatgg aggcggataa agttgcagga ccacttctgc 960
   gctcggccct tccggctggc tggtttattg ctgataaatc tggagccggt gagcgtgggt 1020
   ctcgcggtat cattgcagca ctggggccag atggtaagcc ctcccgtatc gtagttatct 1080
   acacgacggg gagtcaggca actatggatg aacgaaatag acagatcgct gagataggtg 1140
   cctcactgat taagcattgg taactgtcag accaagttta ctcatatata ctttagattg 1200
   atttaaaact tcatttttaa tttaaaagga tctaggtgaa gatccttttt gataatctca 1260
   tgaccaaaat cccttaacgt gagttttcgt tccactgagc gtcagacccc gtagaaaaga 1320
   tcaaaggatc ttcttgagat cctttttttc tgcgcgtaat ctgctgcttg caaacaaaaa 1380
   aaccaccgct accagcggtg gtttgtttgc cggatcaaga gctaccaact ctttttccga 1440
   aggtaactgg cttcagcaga gcgcagatac caaatactgt tcttctagtg tagccgtagt 1500
   taggccacca cttcaagaac tctgtagcac cgcctacata cctcgctctg ctaatcctgt 1560
   taccagtggc tgctgccagt ggcgataagt cgtgtcttac cgggttggac tcaagacgat 1620
   agttaccgga taaggcgcag cggtcgggct gaacgggggg ttcgtgcaca cagcccagct 1680
   tggagcgaac gacctacacc gaactgagat acctacagcg tgagctatga gaaagcgcca 1740
   cgcttcccga agggagaaag gcggacaggt atccggtaag cggcagggtc ggaacaggag 1800
   agcgcacgag ggagcttcca gggggaaacg cctggtatct ttatagtcct gtcgggtttc 1860
   gccacctctg acttgagcgt cgatttttgt gatgctcgtc aggggggcgg agcctatgga 1920
   aaaacgccag caacgcggcc tttttacggt tcctggcctt ttgctggcct tttgctcaca 1980
   tgttctttcc tgcgttatcc cctgattctg tggataaccg tattaccgcc tttgagtgag 2040
   ctgataccgc tcgccgcagc cgaacgaccg agcgcagcga gtcagtgagc gaggaagcgg 2100
   aagagcgccc aatacgcaaa ccgcctctcc ccgcgcgttg gccgattcat taatgcagct 2160
   ggcgcgccac caatcacaat tctgaaaagc acatcttgat ctcctcattg cggggagtcc 2220
   aacggtggtc ttattccccc gaatttcccg ctcaatctcg ttccagaccg acccggacac 2280
   agtgcttaac gccgttccga aactctaccg cagatatgct ccaacggact gggctgcata 2340
   gatgtgatcc tcggcttgga gaaatggata aaagccggcc aaaaaaaaag cggaaaaaag 2400
   cggaaaaaaa gagaaaaaaa atcgcaaaat ttgaaaaata gggggaaaag acgcaaaaac 2460
   gcaaggaggg gggagtatat gacactgata agcaagctca caacggttcc tcttattttt 2520
   ttcctcatct tctgcctagg ttcccaaaat cccagatgct tctctccagt gccaaaagta 2580
   agtaccccac aggttttcgg ccgaaaattc cacgtgcagc aacgtcgtgt ggggtgttaa 2640
   aatgtggggg gggggaacca ggacaagagg ctcttgtggg agccgaatga gagcacaaag 2700
   cgggcgggtg tgataagggc atttttgccc attttccctt ctcctgtctc tccgacggtg 2760
   atggcgttgt gcgtcctcta tttcttttta tttctttttg ttttatttct ctgactaccg 2820
   atttggtttg atttcctcaa ccccacacaa ataagctcgg gccgaggaat atatatatac 2880
   acggacacag tcgccctgtg gacaacacgt cactacctct acgatacaca ccgtacgttg 2940
   tgtggaagct tgtgagcgga taacaatttc acacaggaaa cagctatgac catgattacg 3000
   ccaagctcga aattaaccct cactaaaggg aacaaaagct ggagctccac cgcggacaca 3060
   atatctggtc aaatttcagt ttcgttacat ttaaattcct tcacttcaag ttcattcttc 3120
   atctgcttct gttttacttt gacaggcaaa tgaagacatg gtacgacttg atggaggcca 3180
   agaacgccat ttcaccccga gacaccgaag tgcctgaaat cctggctgcc cccattgata 3240
   acatcggaaa ctacggtatt ccggaaagtg tatatagaac ctttccccag cttgtgtctg 3300
   tggatatgga tggtgtaatc ccctttgagt actcgtcttg gcttctctcc gagcagtatg 3360
   aggctctcta atctagcgca tttaatatct caatgtattt atatatttat cttctcatgc 3420
   ggccgcttag ttggctttgg tcttggcagc cttggcctcc ttgagggtaa acatcttggc 3480
   atccttgtcg accacgccgt acttggcgta cataagacca attcggatga aggtgggaat 3540
   gatgggagaa gccgactttc gcaccagttc gggaaaggcc tgagcgaagg cagcagtggc 3600
   ctcgttgagc ttgtagtgag gaatgatggg aaacagatgg tggatctgat gtgtaccaat 3660
   gttgtgggac aggttgtcga tgagggctcc gtagcttcgg tccacagagg acaagttgcc 3720
   cttgacatag gtccactccg aatcggcgta ccagggagtt tcctcgtcgt tgtgatggag 3780
   gaaggtagtg acaaccagca tggtggcgaa tccaaagaga ggtgcgaagt aatacagagc 3840
   catggtcttg aggccgtaga cgtaggtaag gtaggcgtac agaccagcaa aggccacgag 3900
   agagccgagg gaaatgatga cggcagacat tcttcgcagg tagagaggct cccagggatt 3960
   gaagtggttg acctttcggg gaggaaatcc agcaacgagg taggcaaacc aagccgaacc 4020
   aagggagatg accatgtgtc gggacagggg atgagagtcg gcttctcgct gagggtagaa 4080
   gatctcatcc ttgtcgatgt tgccggtgtt cttgtgatgg tgtcgatggc tgatcttcca 4140
   cgactcgtag ggagtcagaa tgatggagtg aatgagtgtg ccaacagaga agttgagcag 4200
   gtgggatcgc gagaaggcac catgtccaca gtcgtgaccg atggtaaaga atccccagaa 4260
   cacgataccc tggagcagaa tgtagccagt gcaaaggacg gcatcgagca gtgcaaactc 4320
   ctgcacgata gcaagggctc gagcatagta cagtccgaga gcaagggaac cggcaatgcc 4380
   cagagctcgc acggtatagt agagggacca gggaacagag gcttcgaagc agtgggcagg 4440
   cagggatcgc ttgatctcgg tgagagtagg gaactcgtag ggagcggcaa cggtagagga 4500
   agccatggtt gtgaattagg gtggtgagaa tggttggttg tagggaagaa tcaaaggccg 4560
   gtctcgggat ccgtgggtat atatatatat atatatatat acgatccttc gttacctccc 4620
   tgttctcaaa actgtggttt ttcgtttttc gttttttgct ttttttgatt tttttagggc 4680
   caactaagct tccagatttc gctaatcacc tttgtactaa ttacaagaaa ggaagaagct 4740
   gattagagtt gggcttttta tgcaactgtg ctactcctta tctctgatat gaaagtgtag 4800
   acccaatcac atcatgtcat ttagagttgg taatactggg aggatagata aggcacgaaa 4860
   acgagccata gcagacatgc tgggtgtagc caagcagaag aaagtagatg ggagccaatt 4920
   gacgagcgag ggagctacgc caatccgaca tacgacacgc tgagatcgtc ttggccgggg 4980
   ggtacctaca gatgtccaag ggtaagtgct tgactgtaat tgtatgtctg aggacaaata 5040
   tgtagtcagc cgtataaagt cataccaggc accagtgcca tcatcgaacc actaactctc 5100
   tatgatacat gcctccggta ttattgtacc atgcgtcgct ttgttacata cgtatcttgc 5160
   ctttttctct cagaaactcc agactttggc tattggtcga gataagcccg gaccatagtg 5220
   agtctttcac actctacatt tctcccttgc tccaactatc gattgttgtc tactaactat 5280
   cgtacgataa cttcgtatag catacattat acgaagttat cgcgtcgacg agtatctgtc 5340
   tgactcgtca ttgccgcctt tggagtacga ctccaactat gagtgtgctt ggatcacttt 5400
   gacgatacat tcttcgttgg aggctgtggg tctgacagct gcgttttcgg cgcggttggc 5460
   cgacaacaat atcagctgca acgtcattgc tggctttcat catgatcaca tttttgtcgg 5520
   caaaggcgac gcccagagag ccattgacgt tctttctaat ttggaccgat agccgtatag 5580
   tccagtctat ctataagttc aactaactcg taactattac cataacatat acttcactgc 5640
   cccagataag gttccgataa aaagttctgc agactaaatt tatttcagtc tcctcttcac 5700
   caccaaaatg ccctcctacg aagctcgagc taacgtccac aagtccgcct ttgccgctcg 5760
   agtgctcaag ctcgtggcag ccaagaaaac caacctgtgt gcttctctgg atgttaccac 5820
   caccaaggag ctcattgagc ttgccgataa ggtcggacct tatgtgtgca tgatcaaaac 5880
   ccatatcgac atcattgacg acttcaccta cgccggcact gtgctccccc tcaaggaact 5940
   tgctcttaag cacggtttct tcctgttcga ggacagaaag ttcgcagata ttggcaacac 6000
   tgtcaagcac cagtaccggt gtcaccgaat cgccgagtgg tccgatatca ccaacgccca 6060
   cggtgtaccc ggaaccggaa tcattgctgg cctgcgagct ggtgccgagg aaactgtctc 6120
   tgaacagaag aaggaggacg tctctgacta cgagaactcc cagtacaagg agttcctagt 6180
   cccctctccc aacgagaagc tggccagagg tctgctcatg ctggccgagc tgtcttgcaa 6240
   gggctctctg gccactggcg agtactccaa gcagaccatt gagcttgccc gatccgaccc 6300
   cgagtttgtg gttggcttca ttgcccagaa ccgacctaag ggcgactctg aggactggct 6360
   tattctgacc cccggggtgg gtcttgacga caagggagac gctctcggac agcagtaccg 6420
   aactgttgag gatgtcatgt ctaccggaac ggatatcata attgtcggcc gaggtctgta 6480
   cggccagaac cgagatccta ttgaggaggc caagcgatac cagaaggctg gctgggaggc 6540
   ttaccagaag attaactgtt agaggttaga ctatggatat gtaatttaac tgtgtatata 6600
   gagagcgtgc aagtatggag cgcttgttca gcttgtatga tggtcagacg acctgtctga 6660
   tcgagtatgt atgatactgc acaacctgtg tatccgcatg atctgtccaa tggggcatgt 6720
   tgttgtgttt ctcgatacgg agatgctggg tacagtgcta atacgttgaa ctacttatac 6780
   ttatatgagg ctcgaagaaa gctgacttgt gtatgactta ttctcaacta catccccagt 6840
   cacaatacca ccactgcact accactacac caaaaccatg atcaaaccac ccatggactt 6900
   cctggaggca gaagaacttg ttatggaaaa gctcaagaga gagatcataa cttcgtatag 6960
   catacattat acgaagttat cctgcaggta aaggaattca tgctgttcat cgtggttaat 7020
   gctgctgtgt gctgtgtgtg tgtgttgttt ggcgctcatt gttgcgttat gcagcgtaca 7080
   ccacaatatt ggaagcttat tagcctttct attttttcgt ttgcaaggct taacaacatt 7140
   gctgtggaga gggatgggga tatggaggcc gctggaggga gtcggagagg cgttttggag 7200
   cggcttggcc tggcgcccag ctcgcgaaac gcacctagga ccctttggca cgccgaaatg 7260
   tgccactttt cagtctagta acgccttacc tacgtcattc catgcgtgca tgtttgcgcc 7320
   ttttttccct tgcccttgat cgccacacag tacagtgcac tgtacagtgg aggttttggg 7380
   ggggtcttag atgggagcta aaagcggcct agcggtacac tagtgggatt gtatggagtg 7440
   gcatggagcc taggtggagc ctgacaggac gcacgaccgg ctagcccgtg acagacgatg 7500
   ggtggctcct gttgtccacc gcgtacaaat gtttgggcca aagtcttgtc agccttgctt 7560
   gcgaacctaa ttcccaattt tgtcacttcg cacccccatt gatcgagccc taacccctgc 7620
   ccatcaggca atccaattaa gctcgcattg tctgccttgt ttagtttggc tcctgcccgt 7680
   ttcggcgtcc acttgcacaa acacaaacaa gcattatata taaggctcgt ctctccctcc 7740
   caaccacact cacttttttg cccgtcttcc cttgctaaca caaaagtcaa gaacacaaac 7800
   aaccacccca acccccttac acacaagaca tatctacagc aatggccatg gcttcttcca 7860
   ctgttgctgc gccgtacgag ttcccgacgc tgacggagat caagcgctcg ctgccagcgc 7920
   actgctttga ggcctcggtc ccgtggtcgc tctactacac cgtgcgcgcg ctgggcatcg 7980
   ccggctcgct cgcgctcggc ctctactacg cgcgcgcgct cgcgatcgtg caggagtttg 8040
   ccctgctgga tgcggtgctc tgcacggggt acattctgct gcagggcatc gtattctggg 8100
   ggttcttcac catcggccat gactgcggcc acggcgcgtt ctcgcgttcg cacctgctca 8160
   acttcagcgt cggcacgctc attcactcga tcatcctcac gccgtacgag tcatggaaga 8220
   tctcgcaccg ccaccaccac aagaacacgg gcaacatcga caaggacgag attttctacc 8280
   cgcagcgcga ggccgactcg cacccactgt cccgacacat ggtgatctcg ctcggctcgg 8340
   cctggttcgc gtacctcgtt gcgggcttcc ctcctcgcaa ggtgaaccac ttcaaccctt 8400
   gggaaccgtt gtacctgcgc cgcatgtctg ccgtcatcat ctcactcggc tcgctcgtgg 8460
   cgttcgcggg cttgtatgcg tatctcacct acgtctatgg ccttaagacc atggcgctgt 8520
   actacttcgc ccctctcttt gggttcgcca cgatgctcgt ggtcactacc tttttgcacc 8580
   acaatgacga ggaaacgcca tggtacgccg actcggagtg gacgtacgtc aagggcaacc 8640
   tctcgtccgt ggaccgctcg tacggcgcgc tcatcgacaa cctgagccac aacatcggca 8700
   cgcaccagat ccaccacctg tttccgatca tcccgcacta caagctgaac gaggcgacgg 8760
   cagcgttcgc gcaggcgttc ccggagctcg tgcgcaagag cgcgtcgccg atcatcccga 8820
   cgttcatccg catcgggctc atgtacgcca agtacggcgt cgtggacaag gacgccaaga 8880
   tgtttacgct caaggaggcc aaggccgcca agaccaaggc caactaggcg gccgcattga 8940
   tgattggaaa cacacacatg ggttatatct aggtgagagt tagttggaca gttatatatt 9000
   aaatcagcta tgccaacggt aacttcattc atgtcaacga ggaaccagtg actgcaagta 9060
   atatagaatt tgaccacctt gccattctct tgcactcctt tactatatct catttatttc 9120
   ttatatacaa atcacttctt cttcccagca tcgagctcgg aaacctcatg agcaataaca 9180
   tcgtggatct cgtcaataga gggctttttg gactccttgc tgttggccac cttgtccttg 9240
   ctgtttaaac agtgtacgca gatctactat agaggaacat ttaaattgcc ccggagaaga 9300
   cggccaggcc gcctagatga caaattcaac aactcacagc tgactttctg ccattgccac 9360
   tagggggggg cctttttata tggccaagcc aagctctcca cgtcggttgg gctgcaccca 9420
   acaataaatg ggtagggttg caccaacaaa gggatgggat ggggggtaga agatacgagg 9480
   ataacggggc tcaatggcac aaataagaac gaatactgcc attaagactc gtgatccagc 9540
   gactgacacc attgcatcat ctaagggcct caaaactacc tcggaactgc tgcgctgatc 9600
   tggacaccac agaggttccg agcactttag gttgcaccaa atgtcccacc aggtgcaggc 9660
   agaaaacgct ggaacagcgt gtacagtttg tcttaacaaa aagtgagggc gctgaggtcg 9720
   agcagggtgg tgtgacttgt tatagccttt agagctgcga aagcgcgtat ggatttggct 9780
   catcaggcca gattgagggt ctgtggacac atgtcatgtt agtgtacttc aatcgccccc 9840
   tggatatagc cccgacaata ggccgtggcc tcattttttt gccttccgca catttccatt 9900
   gctcggtacc cacaccttgc ttctcctgca cttgccaacc ttaatactgg tttacattga 9960
   ccaacatctt acaagcgggg ggcttgtcta gggtatatat aaacagtggc tctcccaatc 10020
   ggttgccagt ctcttttttc ctttctttcc ccacagattc gaaatctaaa ctacacatca 10080
   cagaattccg agccgtgagt atccacgaca agatcagtgt cgagacgacg cgttttgtgt 10140
   aatgacacaa tccgaaagtc gctagcaaca cacactctct acacaaacta acccagctct 10200
   ggtaccatgg cttcttccac tgttgctgcg ccgtacgagt tcccgacgct gacggagatc 10260
   aagcgctcgc tgccagcgca ctgctttgag gcctcggtcc cgtggtcgct ctactacacc 10320
   gtgcgcgcgc tgggcatcgc cggctcgctc gcgctcggcc tctactacgc gcgcgcgctc 10380
   gcgatcgtgc aggagtttgc cctgctggat gcggtgctct gcacggggta cattctgctg 10440
   cagggcatcg tattctgggg gttcttcacc atcggccatg actgcggcca cggcgcgttc 10500
   tcgcgttcgc acctgctcaa cttcagcgtc ggcacgctca ttcactcgat catcctcacg 10560
   ccgtacgagt catggaagat ctcgcaccgc caccaccaca agaacacggg caacatcgac 10620
   aaggacgaga ttttctaccc gcagcgcgag gccgactcgc acccactgtc ccgacacatg 10680
   gtgatctcgc tcggctcggc ctggttcgcg tacctcgttg cgggcttccc tcctcgcaag 10740
   gtgaaccact tcaacccttg ggaaccgttg tacctgcgcc gcatgtctgc cgtcatcatc 10800
   tcactcggct cgctcgtggc gttcgcgggc ttgtatgcgt atctcaccta cgtctatggc 10860
   cttaagacca tggcgctgta ctacttcgcc cctctctttg ggttcgccac gatgctcgtg 10920
   gtcactacct ttttgcacca caatgacgag gaaacgccat ggtacgccga ctcggagtgg 10980
   acgtacgtca agggcaacct ctcgtccgtg gaccgctcgt acggcgcgct catcgacaac 11040
   ctgagccaca acatcggcac gcaccagatc caccacctgt ttccgatcat cccgcactac 11100
   aagctgaacg aggcgacggc agcgttcgcg caggcgttcc cggagctcgt gcgcaagagc 11160
   gcgtcgccga tcatcccgac gttcatccgc atcgggctca tgtacgccaa gtacggcgtc 11220
   gtggacaagg acgccaagat gtttacgctc aaggaggcca aggccgccaa gaccaaggcc 11280
   aactaggcgg ccgcatggag cgtgtgttct gagtcgatgt tttctatgga gttgtgagtg 11340
   ttagtagaca tgatgggttt atatatgatg aatgaataga tgtgattttg atttgcacga 11400
   tggaattgag aactttgtaa acgtacatgg gaatgtatga atgtgggggt tttgtgactg 11460
   gataactgac ggtcagtgga cgccgttgtt caaatatcca agagatgcga gaaactttgg 11520
   gtcaagtgaa catgtcctct ctgttcaagt aaaccatcaa ctatgggtag tatatttagt 11580
   aaggacaaga gttgagattc tttggagtcc tagaaacgta ttttcgcgtt ccaagatcaa 11640
   attagtagag taatacgggc acgggaatcc attcatagtc tcaatcctgc aggtgagtta 11700
   attaagatga cgacatttgc gagctggacg aggaatagat ggagcgtgtg ttctgagtcg 11760
   atgttttcta tggagttgtg agtgttagta gacatgatgg gtttatatat gatgaatgaa 11820
   tagatgtgat tttgatttgc acgatggaat tgagaacttt gtaaacgtac atgggaatgt 11880
   atgaatgtgg gggttttgtg actggataac tgacggtcag tggacgccgt tgttcaaata 11940
   tccaagagat gcgagaaact ttgggtcaag tgaacatgtc ctctctgttc aagtaaacca 12000
   tcaactatgg gtagtatatt tagtaaggac aagagttgag attctttgga gtcctagaaa 12060
   cgtattttcg cgttccaaga tcaaattagt agagtaatac gggcacggga atccattcat 12120
   agtctcaatt ttcccatagg tgtgctacaa ggtgttgaga tgtggtacag taccaccatg 12180
   attcgaggta aagagcccag aagtcattga tgaggtcaag aaatacacag atctacagct 12240
   caatacaatg aatatcttct ttcatattct tcaggtgaca ccaagggtgt ctattttccc 12300
   cagaaatgcg tgaaaaggcg cgtgtgtagc gtggagtatg ggttcggttg gcgtatcctt 12360
   catatatcga cgaaatagta gggcaagaga tgacaaaaag tatctatatg tagacagcgt 12420
   agaatatgga tttgattggt ataaattcat ttattgcgtg tctcacaaat actctcgata 12480
   agttggggtt aaactggaga tggaacaatg tcgatatctc gacgcatgcg acgtcgggcc 12540
   caattcgccc tatagtgagt cgtattacaa ttcactggcc gtcgttttac aacgtcgtga 12600
   ctgggaaaac cctggcgtta cccaacttaa tcgccttgca gcacatcccc ctttcgccag 12660
   ctggcgtaat agcgaagagg cccgcaccga tcgcccttcc caacagttgc gcagcctgaa 12720
   tggcgaatgg acgcgccctg tagcggcgca ttaagcgcgg cgggtgtggt ggttacgcgc 12780
   agcgtgaccg ctacacttgc cagcgcccta gcgcccgctc ctttcgcttt cttcccttcc 12840
   tttctcgcca cgttcgccgg ctttccccgt caagctctaa atcgggggct ccctttaggg 12900
   ttccgattta gtgctttacg gcacctcgac cccaaaaaac ttgattaggg tgatggttca 12960
   cgtagtgggc catcgccctg atagacggtt tttcgccctt tgacgttgga gtccacgttc 13020
   tttaatagtg gactcttgtt ccaaactgga acaacactca acccta 13066
<210> 50
   <211> 1080
   <212> DNA
   <213> Pythium aphanidermatum
<220>
   <221> CDS
   <222> (1)..(1080)
   <223> synthetic delta-17 desaturase (codon-optimized for Yarrowia lipolytica)
<400> 50
   atg gct tcc tct acc gtt gcc gct ccc tac gag ttc cct act ctc acc      48
   Met Ala Ser Ser Thr Val Ala Ala Pro Tyr Glu Phe Pro Thr Leu Thr
   1               5                   10                  15
   gag atc aag cga tcc ctg cct gcc cac tgc ttc gaa gcc tct gtt ccc      96
   Glu Ile Lys Arg Ser Leu Pro Ala His Cys Phe Glu Ala Ser Val Pro
               20                  25                  30
   tgg tcc ctc tac tat acc gtg cga gct ctg ggc att gcc ggt tcc ctt     144
   Trp Ser Leu Tyr Tyr Thr Val Arg Ala Leu Gly Ile Ala Gly Ser Leu
           35                  40                  45
   gct ctc gga ctg tac tat gct cga gcc ctt gct atc gtg cag gag ttt     192
   Ala Leu Gly Leu Tyr Tyr Ala Arg Ala Leu Ala Ile Val Gln Glu Phe
       50                  55                  60
   gca ctg ctc gat gcc gtc ctt tgc act ggc tac att ctg ctc cag ggt     240
   Ala Leu Leu Asp Ala Val Leu Cys Thr Gly Tyr Ile Leu Leu Gln Gly
   65                  70                  75                  80
   atc gtg ttc tgg gga ttc ttt acc atc ggt cac gac tgt gga cat ggt     288
   Ile Val Phe Trp Gly Phe Phe Thr Ile Gly His Asp Cys Gly His Gly
                   85                  90                  95
   gcc ttc tcg cga tcc cac ctg ctc aac ttc tct gtt ggc aca ctc att     336
   Ala Phe Ser Arg Ser His Leu Leu Asn Phe Ser Val Gly Thr Leu Ile
               100                 105                 110
   cac tcc atc att ctg act ccc tac gag tcg tgg aag atc agc cat cga     384
   His Ser Ile Ile Leu Thr Pro Tyr Glu Ser Trp Lys Ile Ser His Arg
           115                 120                 125
   cac cat cac aag aac acc ggc aac atc gac aag gat gag atc ttc tac     432
   His His His Lys Asn Thr Gly Asn Ile Asp Lys Asp Glu Ile Phe Tyr
       130                 135                 140
   cct cag cga gaa gcc gac tct cat ccc ctg tcc cga cac atg gtc atc     480
   Pro Gln Arg Glu Ala Asp Ser His Pro Leu Ser Arg His Met Val Ile
   145                 150                 155                 160
   tcc ctt ggt tcg gct tgg ttt gcc tac ctc gtt gct gga ttt cct ccc     528
   Ser Leu Gly Ser Ala Trp Phe Ala Tyr Leu Val Ala Gly Phe Pro Pro
                   165                 170                 175
   cga aag gtc aac cac ttc aat ccc tgg gag cct ctc tac ctg cga aga     576
   Arg Lys Val Asn His Phe Asn Pro Trp Glu Pro Leu Tyr Leu Arg Arg
               180                 185                 190
   atg tct gcc gtc atc att tcc ctc ggc tct ctc gtg gcc ttt gct ggt     624
   Met Ser Ala Val Ile Ile Ser Leu Gly Ser Leu Val Ala Phe Ala Gly
           195                 200                 205
   ctg tac gcc tac ctt acc tac gtc tac ggc ctc aag acc atg gct ctg     672
   Leu Tyr Ala Tyr Leu Thr Tyr Val Tyr Gly Leu Lys Thr Met Ala Leu
       210                 215                 220
   tat tac ttc gca cct ctc ttt gga ttc gcc acc atg ctg gtt gtc act     720
   Tyr Tyr Phe Ala Pro Leu Phe Gly Phe Ala Thr Met Leu Val Val Thr
   225                 230                 235                 240
   acc ttc ctc cat cac aac gac gag gaa act ccc tgg tac gcc gat tcg      768
   Thr Phe Leu His His Asn Asp Glu Glu Thr Pro Trp Tyr Ala Asp Ser
                   245                 250                 255
   gag tgg acc tat gtc aag ggc aac ttg tcc tct gtg gac cga agc tac      816
   Glu Trp Thr Tyr Val Lys Gly Asn Leu Ser Ser Val Asp Arg Ser Tyr
               260                 265                 270
   gga gcc ctc atc gac aac ctg tcc cac aac att ggt aca cat cag atc      864
   Gly Ala Leu Ile Asp Asn Leu Ser His Asn Ile Gly Thr His Gln Ile
           275                 280                 285
   cac cat ctg ttt ccc atc att cct cac tac aag ctc aac gag gcc act      912
   His His Leu Phe Pro Ile Ile Pro His Tyr Lys Leu Asn Glu Ala Thr
       290                 295                 300
   gct gcc ttc gct cag gcc ttt ccc gaa ctg gtg cga aag tcg gct tct      960
   Ala Ala Phe Ala Gln Ala Phe Pro Glu Leu Val Arg Lys Ser Ala Ser
   305                 310                 315                 320
   ccc atc att ccc acc ttc atc cga att ggt ctt atg tac gcc aag tac     1008
   Pro Ile Ile Pro Thr Phe Ile Arg Ile Gly Leu Met Tyr Ala Lys Tyr
                   325                 330                 335
   ggc gtg gtc gac aag gat gcc aag atg ttt acc ctc aag gag gcc aag     1056
   Gly Val Val Asp Lys Asp Ala Lys Met Phe Thr Leu Lys Glu Ala Lys
               340                 345                 350
   gct gcc aag acc aaa gcc aac taa                                     1080
   Ala Ala Lys Thr Lys Ala Asn
           355
<210> 51
   <211> 359
   <212> PRT
   <213> Pythium aphanidermatum
<400> 51
   Met Ala Ser Ser Thr Val Ala Ala Pro Tyr Glu Phe Pro Thr Leu Thr
   1               5                   10                  15
   Glu Ile Lys Arg Ser Leu Pro Ala His Cys Phe Glu Ala Ser Val Pro
               20                  25                  30
   Trp Ser Leu Tyr Tyr Thr Val Arg Ala Leu Gly Ile Ala Gly Ser Leu
           35                  40                  45
   Ala Leu Gly Leu Tyr Tyr Ala Arg Ala Leu Ala Ile Val Gln Glu Phe
       50                  55                  60
   Ala Leu Leu Asp Ala Val Leu Cys Thr Gly Tyr Ile Leu Leu Gln Gly
   65                  70                  75                  80
   Ile Val Phe Trp Gly Phe Phe Thr Ile Gly His Asp Cys Gly His Gly
   85 90 95
   Ala Phe Ser Arg Ser His Leu Leu Asn Phe Ser Val Gly Thr Leu Ile
   100 105 110
   His Ser Ile Ile Leu Thr Pro Tyr Glu Ser Trp Lys Ile Ser His Arg
   115 120 125
   His His His Lys Asn Thr Gly Asn Ile Asp Lys Asp Glu Ile Phe Tyr
   130 135 140
   Pro Gln Arg Glu Ala Asp Ser His Pro Leu Ser Arg His Met Val Ile
   145 150 155 160
   Ser Leu Gly Ser Ala Trp Phe Ala Tyr Leu Val Ala Gly Phe Pro Pro
   165 170 175
   Arg Lys Val Asn His Phe Asn Pro Trp Glu Pro Leu Tyr Leu Arg Arg
   180 185 190
   Met Ser Ala Val Ile Ile Ser Leu Gly Ser Leu Val Ala Phe Ala Gly
   195 200 205
   Leu Tyr Ala Tyr Leu Thr Tyr Val Tyr Gly Leu Lys Thr Met Ala Leu
   210 215 220
   Tyr Tyr Phe Ala Pro Leu Phe Gly Phe Ala Thr Met Leu Val Val Thr
   225 230 235 240
   Thr Phe Leu His His Asn Asp Glu Glu Thr Pro Trp Tyr Ala Asp Ser
   245 250 255
   Glu Trp Thr Tyr Val Lys Gly Asn Leu Ser Ser Val Asp Arg Ser Tyr
   260 265 270
   Gly Ala Leu Ile Asp Asn Leu Ser His Asn Ile Gly Thr His Gln Ile
   275 280 285
   His His Leu Phe Pro Ile Ile Pro His Tyr Lys Leu Asn Glu Ala Thr
   290 295 300
   Ala Ala Phe Ala Gln Ala Phe Pro Glu Leu Val Arg Lys Ser Ala Ser
   305 310 315 320
   Pro Ile Ile Pro Thr Phe Ile Arg Ile Gly Leu Met Tyr Ala Lys Tyr
   325 330 335
   Gly Val Val Asp Lys Asp Ala Lys Met Phe Thr Leu Lys Glu Ala Lys
   340 345 350
   Ala Ala Lys Thr Lys Ala Asn
   355
<210> 52
   <211> 1080
   <212> DNA
   <213> Pythium aphanidermatum
<220>
   <221> CDS
   <222> (1)..(1080)
   <223> delta-17 desaturase
<400> 52
   atg gct tct tcc act gtt gct gcg ccg tac gag ttc ccg acg ctg acg      48
   Met Ala Ser Ser Thr Val Ala Ala Pro Tyr Glu Phe Pro Thr Leu Thr
   1               5                   10                  15
   gag atc aag cgc tcg ctg cca gcg cac tgc ttt gag gcc tcg gtc ccg      96
   Glu Ile Lys Arg Ser Leu Pro Ala His Cys Phe Glu Ala Ser Val Pro
               20                  25                  30
   tgg tcg ctc tac tac acc gtg cgc gcg ctg ggc atc gcc ggc tcg ctc     144
   Trp Ser Leu Tyr Tyr Thr Val Arg Ala Leu Gly Ile Ala Gly Ser Leu
           35                  40                  45
   gcg ctc ggc ctc tac tac gcg cgc gcg ctc gcg atc gtg cag gag ttt     192
   Ala Leu Gly Leu Tyr Tyr Ala Arg Ala Leu Ala Ile Val Gln Glu Phe
       50                  55                  60
   gcc ctg ctg gat gcg gtg ctc tgc acg ggg tac att ctg ctg cag ggc     240
   Ala Leu Leu Asp Ala Val Leu Cys Thr Gly Tyr Ile Leu Leu Gln Gly
   65                  70                  75                  80
   atc gta ttc tgg ggg ttc ttc acc atc ggc cat gac tgc ggc cac ggc     288
   Ile Val Phe Trp Gly Phe Phe Thr Ile Gly His Asp Cys Gly His Gly
                   85                  90                  95
   gcg ttc tcg cgt tcg cac ctg ctc aac ttc agc gtc ggc acg ctc att     336
   Ala Phe Ser Arg Ser His Leu Leu Asn Phe Ser Val Gly Thr Leu Ile
               100                 105                 110
   cac tcg atc atc ctc acg ccg tac gag tca tgg aag atc tcg cac cgc     384
   His Ser Ile Ile Leu Thr Pro Tyr Glu Ser Trp Lys Ile Ser His Arg
           115                 120                 125
   cac cac cac aag aac acg ggc aac atc gac aag gac gag att ttc tac     432
   His His His Lys Asn Thr Gly Asn Ile Asp Lys Asp Glu Ile Phe Tyr
       130                 135                 140
   ccg cag cgc gag gcc gac tcg cac cca ctg tcc cga cac atg gtg atc     480
   Pro Gln Arg Glu Ala Asp Ser His Pro Leu Ser Arg His Met Val Ile
   145                 150                 155                 160
   tcg ctc ggc tcg gcc tgg ttc gcg tac ctc gtt gcg ggc ttc cct cct         528
   Ser Leu Gly Ser Ala Trp Phe Ala Tyr Leu Val Ala Gly Phe Pro Pro
                   165                 170                 175
   cgc aag gtg aac cac ttc aac cct tgg gaa ccg ttg tac ctg cgc cgc         576
   Arg Lys Val Asn His Phe Asn Pro Trp Glu Pro Leu Tyr Leu Arg Arg
               180                 185                 190
   atg tct gcc gtc atc atc tca ctc ggc tcg ctc gtg gcg ttc gcg ggc         624
   Met Ser Ala Val Ile Ile Ser Leu Gly Ser Leu Val Ala Phe Ala Gly
           195                 200                 205
   ttg tat gcg tat ctc acc tac gtc tat ggc ctt aag acc atg gcg ctg         672
   Leu Tyr Ala Tyr Leu Thr Tyr Val Tyr Gly Leu Lys Thr Met Ala Leu
       210                 215                 220
   tac tac ttc gcc cct ctc ttt ggg ttc gcc acg atg ctc gtg gtc act         720
   Tyr Tyr Phe Ala Pro Leu Phe Gly Phe Ala Thr Met Leu Val Val Thr
   225                 230                 235                 240
   acc ttt ttg cac cac aat gac gag gaa acg cca tgg tac gcc gac tcg         768
   Thr Phe Leu His His Asn Asp Glu Glu Thr Pro Trp Tyr Ala Asp Ser
                   245                 250                 255
   gag tgg acg tac gtc aag ggc aac ctc tcg tcc gtg gac cgc tcg tac         816
   Glu Trp Thr Tyr Val Lys Gly Asn Leu Ser Ser Val Asp Arg Ser Tyr
               260                 265                 270
   ggc gcg ctc atc gac aac ctg agc cac aac atc ggc acg cac cag atc         864
   Gly Ala Leu Ile Asp Asn Leu Ser His Asn Ile Gly Thr His Gln Ile
           275                 280                 285
   cac cac ctg ttt ccg atc atc ccg cac tac aag ctg aac gag gcg acg         912
   His His Leu Phe Pro Ile Ile Pro His Tyr Lys Leu Asn Glu Ala Thr
       290                 295                 300
   gca gcg ttc gcg cag gcg ttc ccg gag ctc gtg cgc aag agc gcg tcg         960
   Ala Ala Phe Ala Gln Ala Phe Pro Glu Leu Val Arg Lys Ser Ala Ser
   305                 310                 315                 320
   ccg atc atc ccg acg ttc atc cgc atc ggg ctc atg tac gcc aag tac        1008
   Pro Ile Ile Pro Thr Phe Ile Arg Ile Gly Leu Met Tyr Ala Lys Tyr
                   325                 330                 335
   ggc gtc gtg gac aag gac gcc aag atg ttt acg ctc aag gag gcc aag        1056
   Gly Val Val Asp Lys Asp Ala Lys Met Phe Thr Leu Lys Glu Ala Lys
               340                 345                 350
   gcc gcc aag acc aag gcc aac tag                                        1080
   Ala Ala Lys Thr Lys Ala Asn
           355
<210> 53
   <211> 359
   <212> PRT
   <213> Pythium aphanidermatum
<400> 53
   Met Ala Ser Ser Thr Val Ala Ala Pro Tyr Glu Phe Pro Thr Leu Thr
   1               5                   10                  15
   Glu Ile Lys Arg Ser Leu Pro Ala His Cys Phe Glu Ala Ser Val Pro
               20                  25                  30
   Trp Ser Leu Tyr Tyr Thr Val Arg Ala Leu Gly Ile Ala Gly Ser Leu
           35                  40                  45
   Ala Leu Gly Leu Tyr Tyr Ala Arg Ala Leu Ala Ile Val Gln Glu Phe
       50                  55                  60
   Ala Leu Leu Asp Ala Val Leu Cys Thr Gly Tyr Ile Leu Leu Gln Gly
   65                  70                  75                  80
   Ile Val Phe Trp Gly Phe Phe Thr Ile Gly His Asp Cys Gly His Gly
                   85                  90                  95
   Ala Phe Ser Arg Ser His Leu Leu Asn Phe Ser Val Gly Thr Leu Ile
               100                 105                 110
   His Ser Ile Ile Leu Thr Pro Tyr Glu Ser Trp Lys Ile Ser His Arg
           115                 120                 125
   His His His Lys Asn Thr Gly Asn Ile Asp Lys Asp Glu Ile Phe Tyr
       130                 135                 140
   Pro Gln Arg Glu Ala Asp Ser His Pro Leu Ser Arg His Met Val Ile
   145                 150                 155                 160
   Ser Leu Gly Ser Ala Trp Phe Ala Tyr Leu Val Ala Gly Phe Pro Pro
                   165                 170                 175
   Arg Lys Val Asn His Phe Asn Pro Trp Glu Pro Leu Tyr Leu Arg Arg
               180                 185                 190
   Met Ser Ala Val Ile Ile Ser Leu Gly Ser Leu Val Ala Phe Ala Gly
           195                 200                 205
   Leu Tyr Ala Tyr Leu Thr Tyr Val Tyr Gly Leu Lys Thr Met Ala Leu
       210                 215                 220
   Tyr Tyr Phe Ala Pro Leu Phe Gly Phe Ala Thr Met Leu Val Val Thr
   225                 230                 235                 240
   Thr Phe Leu His His Asn Asp Glu Glu Thr Pro Trp Tyr Ala Asp Ser
   245 250 255
   Glu Trp Thr Tyr Val Lys Gly Asn Leu Ser Ser Val Asp Arg Ser Tyr
   260 265 270
   Gly Ala Leu Ile Asp Asn Leu Ser His Asn Ile Gly Thr His Gln Ile
   275 280 285
   His His Leu Phe Pro Ile Ile Pro His Tyr Lys Leu Asn Glu Ala Thr
   290 295 300
   Ala Ala Phe Ala Gln Ala Phe Pro Glu Leu Val Arg Lys Ser Ala Ser
   305 310 315 320
   Pro Ile Ile Pro Thr Phe Ile Arg Ile Gly Leu Met Tyr Ala Lys Tyr
   325 330 335
   Gly Val Val Asp Lys Asp Ala Lys Met Phe Thr Leu Lys Glu Ala Lys
   340 345 350
   Ala Ala Lys Thr Lys Ala Asn
   355
<210> 54
   <211> 9570
   <212> DNA
   <213> Artificial Sequence
<220>
   <223> Plasmid pY117
<400> 54
   ggccgccacc gcggcccgag attccggcct cttcggccgc caagcgaccc gggtggacgt 60
   ctagaggtac ctagcaatta acagatagtt tgccggtgat aattctctta acctcccaca 120
   ctcctttgac ataacgattt atgtaacgaa actgaaattt gaccagatat tgtgtccgcg 180
   gtggagctcc agcttttgtt ccctttagtg agggtttaaa cgagcttggc gtaatcatgg 240
   tcatagctgt ttcctgtgtg aaattgttat ccgctcacaa ttccacacaa cgtacgagcc 300
   ggaagcataa agtgtaaagc ctggggtgcc taatgagtga gctaactcac attaattgcg 360
   ttgcgctcac tgcccgcttt ccagtcggga aacctgtcgt gccagctgca ttaatgaatc 420
   ggccaacgcg cggggagagg cggtttgcgt attgggcgct cttccgcttc ctcgctcact 480
   gactcgctgc gctcggtcgt tcggctgcgg cgagcggtat cagctcactc aaaggcggta 540
   atacggttat ccacagaatc aggggataac gcaggaaaga acatgtgagc aaaaggccag 600
   caaaaggcca ggaaccgtaa aaaggccgcg ttgctggcgt ttttccatag gctccgcccc 660
   cctgacgagc atcacaaaaa tcgacgctca agtcagaggt ggcgaaaccc gacaggacta 720
   taaagatacc aggcgtttcc ccctggaagc tccctcgtgc gctctcctgt tccgaccctg 780
   ccgcttaccg gatacctgtc cgcctttctc ccttcgggaa gcgtggcgct ttctcatagc 840
   tcacgctgta ggtatctcag ttcggtgtag gtcgttcgct ccaagctggg ctgtgtgcac 900
   gaaccccccg ttcagcccga ccgctgcgcc ttatccggta actatcgtct tgagtccaac 960
   ccggtaagac acgacttatc gccactggca gcagccactg gtaacaggat tagcagagcg 1020
   aggtatgtag gcggtgctac agagttcttg aagtggtggc ctaactacgg ctacactaga 1080
   aggacagtat ttggtatctg cgctctgctg aagccagtta ccttcggaaa aagagttggt 1140
   agctcttgat ccggcaaaca aaccaccgct ggtagcggtg gtttttttgt ttgcaagcag 1200
   cagattacgc gcagaaaaaa aggatctcaa gaagatcctt tgatcttttc tacggggtct 1260
   gacgctcagt ggaacgaaaa ctcacgttaa gggattttgg tcatgagatt atcaaaaagg 1320
   atcttcacct agatcctttt aaattaaaaa tgaagtttta aatcaatcta aagtatatat 1380
   gagtaaactt ggtctgacag ttaccaatgc ttaatcagtg aggcacctat ctcagcgatc 1440
   tgtctatttc gttcatccat agttgcctga ctccccgtcg tgtagataac tacgatacgg 1500
   gagggcttac catctggccc cagtgctgca atgataccgc gagacccacg ctcaccggct 1560
   ccagatttat cagcaataaa ccagccagcc ggaagggccg agcgcagaag tggtcctgca 1620
   actttatccg cctccatcca gtctattaat tgttgccggg aagctagagt aagtagttcg 1680
   ccagttaata gtttgcgcaa cgttgttgcc attgctacag gcatcgtggt gtcacgctcg 1740
   tcgtttggta tggcttcatt cagctccggt tcccaacgat caaggcgagt tacatgatcc 1800
   cccatgttgt gcaaaaaagc ggttagctcc ttcggtcctc cgatcgttgt cagaagtaag 1860
   ttggccgcag tgttatcact catggttatg gcagcactgc ataattctct tactgtcatg 1920
   ccatccgtaa gatgcttttc tgtgactggt gagtactcaa ccaagtcatt ctgagaatag 1980
   tgtatgcggc gaccgagttg ctcttgcccg gcgtcaatac gggataatac cgcgccacat 2040
   agcagaactt taaaagtgct catcattgga aaacgttctt cggggcgaaa actctcaagg 2100
   atcttaccgc tgttgagatc cagttcgatg taacccactc gtgcacccaa ctgatcttca 2160
   gcatctttta ctttcaccag cgtttctggg tgagcaaaaa caggaaggca aaatgccgca 2220
   aaaaagggaa taagggcgac acggaaatgt tgaatactca tactcttcct ttttcaatat 2280
   tattgaagca tttatcaggg ttattgtctc atgagcggat acatatttga atgtatttag 2340
   aaaaataaac aaataggggt tccgcgcaca tttccccgaa aagtgccacc tgacgcgccc 2400
   tgtagcggcg cattaagcgc ggcgggtgtg gtggttacgc gcagcgtgac cgctacactt 2460
   gccagcgccc tagcgcccgc tcctttcgct ttcttccctt cctttctcgc cacgttcgcc   2520
   ggctttcccc gtcaagctct aaatcggggg ctccctttag ggttccgatt tagtgcttta   2580
   cggcacctcg accccaaaaa acttgattag ggtgatggtt cacgtagtgg gccatcgccc   2640
   tgatagacgg tttttcgccc tttgacgttg gagtccacgt tctttaatag tggactcttg   2700
   ttccaaactg gaacaacact caaccctatc tcggtctatt cttttgattt ataagggatt   2760
   ttgccgattt cggcctattg gttaaaaaat gagctgattt aacaaaaatt taacgcgaat   2820
   tttaacaaaa tattaacgct tacaatttcc attcgccatt caggctgcgc aactgttggg   2880
   aagggcgatc ggtgcgggcc tcttcgctat tacgccagct ggcgaaaggg ggatgtgctg   2940
   caaggcgatt aagttgggta acgccagggt tttcccagtc acgacgttgt aaaacgacgg   3000
   ccagtgaatt gtaatacgac tcactatagg gcgaattggg taccgggccc cccctcgagg   3060
   tcgatggtgt cgataagctt gatatcgaat tcatgtcaca caaaccgatc ttcgcctcaa   3120
   ggaaacctaa ttctacatcc gagagactgc cgagatccag tctacactga ttaattttcg   3180
   ggccaataat ttaaaaaaat cgtgttatat aatattatat gtattatata tatacatcat   3240
   gatgatactg acagtcatgt cccattgcta aatagacaga ctccatctgc cgcctccaac   3300
   tgatgttctc aatatttaag gggtcatctc gcattgttta ataataaaca gactccatct   3360
   accgcctcca aatgatgttc tcaaaatata ttgtatgaac ttatttttat tacttagtat   3420
   tattagacaa cttacttgct ttatgaaaaa cacttcctat ttaggaaaca atttataatg   3480
   gcagttcgtt catttaacaa tttatgtaga ataaatgtta taaatgcgta tgggaaatct   3540
   taaatatgga tagcataaat gatatctgca ttgcctaatt cgaaatcaac agcaacgaaa   3600
   aaaatccctt gtacaacata aatagtcatc gagaaatatc aactatcaaa gaacagctat   3660
   tcacacgtta ctattgagat tattattgga cgagaatcac acactcaact gtctttctct   3720
   cttctagaaa tacaggtaca agtatgtact attctcattg ttcatacttc tagtcatttc   3780
   atcccacata ttccttggat ttctctccaa tgaatgacat tctatcttgc aaattcaaca   3840
   attataataa gatataccaa agtagcggta tagtggcaat caaaaagctt ctctggtgtg   3900
   cttctcgtat ttatttttat tctaatgatc cattaaaggt atatatttat ttcttgttat   3960
   ataatccttt tgtttattac atgggctgga tacataaagg tattttgatt taattttttg   4020
   cttaaattca atcccccctc gttcagtgtc aactgtaatg gtaggaaatt accatacttt   4080
   tgaagaagca aaaaaaatga aagaaaaaaa aaatcgtatt tccaggttag acgttccgca   4140
   gaatctagaa tgcggtatgc ggtacattgt tcttcgaacg taaaagttgc gctccctgag   4200
   atattgtaca tttttgcttt tacaagtaca agtacatcgt acaactatgt actactgttg   4260
   atgcatccac aacagtttgt tttgtttttt tttgtttttt ttttttctaa tgattcatta 4320
   ccgctatgta tacctacttg tacttgtagt aagccgggtt attggcgttc aattaatcat 4380
   agacttatga atctgcacgg tgtgcgctgc gagttacttt tagcttatgc atgctacttg 4440
   ggtgtaatat tgggatctgt tcggaaatca acggatgctc aaccgatttc gacagtaatt 4500
   aattaattcc ctagtcccag tgtacacccg ccgatatcgc ttaccctgca gccggattaa 4560
   ggttggcaat ttttcacgtc cttgtctccg caattactca ccgggtggtt tataagattg 4620
   caagcgtctt gatttgtctc tgtatactaa catgcaatcg cgactcgccc gacgggccac 4680
   taacctggcc agaatctcca gatccaagta ttctcttggt ctgcgatatg tttccaacac 4740
   aaaagcccct gctgcccagc cggcaactgc tgagtgagta ttccttgcca taaacgaccc 4800
   agaaccactg tatagtgttt ggaagcacta gtcagaagac cagcgaaaac aggtggaaaa 4860
   aactgagacg aaaagcaacg accagaaatg taatgtgtgg aaaagcgaca cacacagagc 4920
   agataaagag gtgacaaata acgacaaatg aaatatcagt atcttcccac aatcactacc 4980
   tctcagctgt ctgaaggtgc ggctgatata tccatcccac gtctaacgta tggagtgtga 5040
   tagaatatga cgacacaagc atgagaactc gctctctatc caaccaccga aacactgtca 5100
   ctacagccgt tcttgttgct ccattcgctt ttgtgattcc atgccttctc tggtgactga 5160
   caacattcct tccttttctc cagccctgtt gttatctgct catgacctac ggccactctc 5220
   tatcgcatac taacatagac gatcccagcc cgctccccac ttccagggca ccgttggcaa 5280
   gcctcctatc ctcaagaagg ctgaggctgc caacgctgac atggacgagt ccttcatcgg 5340
   aatgtctgga ggagagatct tccacgagat gatgctgcga cacaacgtcg acactgtctt 5400
   cggttacccc ggtggagcca ttctccccgt ctttgacgcc attcacaact ctgagtactt 5460
   caactttgtg ctccctcgac acgagcaggg tgccggccac atggccgagg gctacgctcg 5520
   agcctctggt aagcccggtg tcgttctcgt cacctctggc cccggtgcca ccaacgtcat 5580
   cacccccatg caggacgctc tttccgatgg tacccccatg gttgtcttca ccggtcaggt 5640
   cctgacctcc gttatcggca ctgacgcctt ccaggaggcc gatgttgtcg gcatctcccg 5700
   atcttgcacc aagtggaacg tcatggtcaa gaacgttgct gagctccccc gacgaatcaa 5760
   cgaggccttt gagattgcta cttccggccg acccggtccc gttctcgtcg atctgcccaa 5820
   ggatgttact gctgccatcc tgcgagagcc catccccacc aagtccacca ttccctcgca 5880
   ttctctgacc aacctcacct ctgccgccgc caccgagttc cagaagcagg ctatccagcg 5940
   agccgccaac ctcatcaacc agtccaagaa gcccgtcctt tacgtcggac agggtatcct 6000
   tggctccgag gagggtccta agctgcttaa ggagctggct gagaaggccg agattcccgt 6060
   caccactact ctgcagggtc ttggtgcctt tgacgagcga gaccccaagt ctctgcacat 6120
   gctcggtatg cacggttccg gctacgccaa catggccatg cagaacgctg actgtatcat 6180
   tgctctcggc gcccgatttg atgaccgagt taccggctcc atccccaagt ttgcccccga 6240
   ggctcgagcc gctgcccttg agggtcgagg tggtattgtt cactttgaga tccaggccaa 6300
   gaacatcaac aaggttgttc aggccaccga agccgttgag ggagacgtta ccgagtctgt 6360
   ccgacagctc atccccctca tcaacaaggt ctctgccgct gagcgagctc cctggactga 6420
   gactatccag tcctggaagc agcagttccc cttcctcttc gaggctgaag gtgaggatgg 6480
   tgttatcaag ccccagtccg tcattgctct gctctctgac ctgacagaga acaacaagga 6540
   caagaccatc atcaccaccg gtgttggtca gcatcagatg tggactgccc agcatttccg 6600
   atggcgacac cctcgaacca tgatcacttc tggtggtctt ggaactatgg gttacggcct 6660
   gcccgccgct atcggcgcca aggttgcccg acctgactgc gacgtcattg acatcgatgg 6720
   tgacgcttct ttcaacatga ctctgaccga gctgtccacc gccgttcagt tcaacattgg 6780
   cgtcaaggct attgtcctca acaacgagga acagggtatg gtcacccagc tgcagtctct 6840
   cttctacgag aaccgatact gccacactca tcagaagaac cccgacttca tgaagctggc 6900
   cgagtccatg ggcatgaagg gtatccgaat cactcacatt gaccagctgg aggccggtct 6960
   caaggagatg ctcgcataca agggccctgt gctcgttgag gttgttgtcg acaagaagat 7020
   ccccgttctt cccatggttc ccgctggtaa ggctttgcat gagttccttg tctacgacgc 7080
   tgacgccgag gctgcttctc gacccgatcg actgaagaat gcccccgccc ctcacgtcca 7140
   ccagaccacc tttgagaact aagtggaaag gaacacaagc aatccgaacc aaaaataatt 7200
   ggggtcccgt gcccacagag tctagtgcag acctaaaatg accacagtaa attatagctg 7260
   ttattaaaca tgagattttg accaacaaga gcgtaggaat gttattagct actacttgta 7320
   catacacagc atttgtttta aataatgttg cctccagggg cagtgagatc aggacccaga 7380
   tccgtggcca gctctctgac ttcagaccgc ttgtacttaa gcagctcgca acactgttgt 7440
   cgaggattga acttgccata ttcgattttg tggtcatgaa tccagcacac ctcatttaaa 7500
   tgtagctaac ggtagcaggc gaactactgg tacatacctc ccccggaata tgtacaggca 7560
   taatgcgtat ctgtgggaca tgtggtcgtt gcgccattat gtaagcagcg tgtactcctc 7620
   tgactgtcca tatggtttgc tccatctcac cctcatcgtt ttcattgttc acaggcggcc 7680
   acaaaaaaac tgtcttctct ccttctctct tcgccttagt ctactcggac cagttttagt 7740
   ttagcttggc gccactggat aaatgagacc tcaggccttg tgatgaggag gtcacttatg 7800
   aagcatgtta ggaggtgctt gtatggatag agaagcaccc aaaataataa gaataataat 7860
   aaaacagggg gcgttgtcat ttcatatcgt gttttcacca tcaatacacc tccaaacaat 7920
   gcccttcatg tggccagccc caatattgtc ctgtagttca actctatgca gctcgtatct 7980
   tattgagcaa gtaaaactct gtcagccgat attgcccgac ccgcgacaag ggtcaacaag 8040
   gtggtgtaag gccttcgcag aagtcaaaac tgtgccaaac aaacatctag agtctctttg 8100
   gtgtttctcg catatatttw atcggctgtc ttacgtattt gcgcctcggt accggactaa 8160
   tttcggatca tccccaatac gctttttctt cgcagctgtc aacagtgtcc atgatctatc 8220
   cacctaaatg ggtcatatga ggcgtataat ttcgtggtgc tgataataat tcccatatat 8280
   ttgacacaaa acttcccccc ctagacatac atctcacaat ctcacttctt gtgcttctgt 8340
   cacacatctc ctccagctga cttcaactca cacctctgcc ccagttggtc tacagcggta 8400
   taaggtttct ccgcatagag gtgcaccact cctcccgata cttgtttgtg tgacttgtgg 8460
   gtcacgacat atatatctac acacattgcg ccaccctttg gttcttccag cacaacaaaa 8520
   acacgacacg ctaaccatgg ccaatttact gaccgtacac caaaatttgc ctgcattacc 8580
   ggtcgatgca acgagtgatg aggttcgcaa gaacctgatg gacatgttca gggatcgcca 8640
   ggcgttttct gagcatacct ggaaaatgct tctgtccgtt tgccggtcgt gggcggcatg 8700
   gtgcaagttg aataaccgga aatggtttcc cgcagaacct gaagatgttc gcgattatct 8760
   tctatatctt caggcgcgcg gtctggcagt aaaaactatc cagcaacatt tgggccagct 8820
   aaacatgctt catcgtcggt ccgggctgcc acgaccaagt gacagcaatg ctgtttcact 8880
   ggttatgcgg cggatccgaa aagaaaacgt tgatgccggt gaacgtgcaa aacaggctct 8940
   agcgttcgaa cgcactgatt tcgaccaggt tcgttcactc atggaaaata gcgatcgctg 9000
   ccaggatata cgtaatctgg catttctggg gattgcttat aacaccctgt tacgtatagc 9060
   cgaaattgcc aggatcaggg ttaaagatat ctcacgtact gacggtggga gaatgttaat 9120
   ccatattggc agaacgaaaa cgctggttag caccgcaggt gtagagaagg cacttagcct 9180
   gggggtaact aaactggtcg agcgatggat ttccgtctct ggtgtagctg atgatccgaa 9240
   taactacctg ttttgccggg tcagaaaaaa tggtgttgcc gcgccatctg ccaccagcca 9300
   gctatcaact cgcgccctgg aagggatttt tgaagcaact catcgattga tttacggcgc 9360
   taaggatgac tctggtcaga gatacctggc ctggtctgga cacagtgccc gtgtcggagc 9420
   cgcgcgagat atggcccgcg ctggagtttc aataccggag atcatgcaag ctggtggctg 9480
   gaccaatgta aatattgtca tgaactatat ccgtaacctg gatagtgaaa caggggcaat 9540
   ggtgcgcctg ctggaagatg gcgattaagc 9570
<210> 55
   <211> 15743
   <212> DNA
   <213> Artificial Sequence
<220>
   <223> Plasmid pZP2-2988
<400> 55
   ggccgcatgt acatacaaga ttatttatag aaatgaatcg cgatcgaaca aagagtacga 60
   gtgtacgagt aggggatgat gataaaagtg gaagaagttc cgcatctttg gatttatcaa 120
   cgtgtaggac gatacttcct gtaaaaatgc aatgtcttta ccataggttc tgctgtagat 180
   gttattaact accattaaca tgtctacttg tacagttgca gaccagttgg agtatagaat 240
   ggtacactta ccaaaaagtg ttgatggttg taactacgat atataaaact gttgacggga 300
   tctgtatatt cggtaagata tattttgtgg ggttttagtg gtgtttaaac agtgtacgca 360
   gtactataga ggaacaattg ccccggagaa gacggccagg ccgcctagat gacaaattca 420
   acaactcaca gctgactttc tgccattgcc actagggggg ggccttttta tatggccaag 480
   ccaagctctc cacgtcggtt gggctgcacc caacaataaa tgggtagggt tgcaccaaca 540
   aagggatggg atggggggta gaagatacga ggataacggg gctcaatggc acaaataaga 600
   acgaatactg ccattaagac tcgtgatcca gcgactgaca ccattgcatc atctaagggc 660
   ctcaaaacta cctcggaact gctgcgctga tctggacacc acagaggttc cgagcacttt 720
   aggttgcacc aaatgtccca ccaggtgcag gcagaaaacg ctggaacagc gtgtacagtt 780
   tgtcttaaca aaaagtgagg gcgctgaggt cgagcagggt ggtgtgactt gttatagcct 840
   ttagagctgc gaaagcgcgt atggatttgg ctcatcaggc cagattgagg gtctgtggac 900
   acatgtcatg ttagtgtact tcaatcgccc cctggatata gccccgacaa taggccgtgg 960
   cctcattttt ttgccttccg cacatttcca ttgctcggta cccacacctt gcttctcctg 1020
   cacttgccaa ccttaatact ggtttacatt gaccaacatc ttacaagcgg ggggcttgtc 1080
   tagggtatat ataaacagtg gctctcccaa tcggttgcca gtctcttttt tcctttcttt 1140
   ccccacagat tcgaaatcta aactacacat cacaccatgg aggtcgtgaa cgaaatcgtc 1200
   tccattggcc aggaggttct tcccaaggtc gactatgctc agctctggtc tgatgcctcg 1260
   cactgcgagg tgctgtacct ctccatcgcc ttcgtcatcc tgaagttcac ccttggtcct 1320
   ctcggaccca agggtcagtc tcgaatgaag tttgtgttca ccaactacaa cctgctcatg 1380
   tccatctact cgctgggctc cttcctctct atggcctacg ccatgtacac cattggtgtc 1440
   atgtccgaca actgcgagaa ggctttcgac aacaatgtct tccgaatcac cactcagctg 1500
   ttctacctca gcaagttcct cgagtacatt gactccttct atctgcccct catgggcaag 1560
   cctctgacct ggttgcagtt ctttcaccat ctcggagctc ctatggacat gtggctgttc 1620
   tacaactacc gaaacgaagc cgtttggatc tttgtgctgc tcaacggctt cattcactgg 1680
   atcatgtacg gctactattg gacccgactg atcaagctca agttccctat gcccaagtcc 1740
   ctgattactt ctatgcagat cattcagttc aacgttggct tctacatcgt ctggaagtac 1800
   cggaacattc cctgctaccg acaagatgga atgagaatgt ttggctggtt tttcaactac 1860
   ttctacgttg gtactgtcct gtgtctgttc ctcaacttct acgtgcagac ctacatcgtc 1920
   cgaaagcaca agggagccaa aaagattcag tgagcggccg caagtgtgga tggggaagtg 1980
   agtgcccggt tctgtgtgca caattggcaa tccaagatgg atggattcaa cacagggata 2040
   tagcgagcta cgtggtggtg cgaggatata gcaacggata tttatgtttg acacttgaga 2100
   atgtacgata caagcactgt ccaagtacaa tactaaacat actgtacata ctcatactcg 2160
   tacccgggca acggtttcac ttgagtgcag tggctagtgc tcttactcgt acagtgtgca 2220
   atactgcgta tcatagtctt tgatgtatat cgtattcatt catgttagtt gcgtacgggc 2280
   gtcgttgctt gtgtgatttt tgaggaccca tccctttggt atataagtat actctggggt 2340
   taaggttgcc cgtgtagtct aggttatagt tttcatgtga aataccgaga gccgagggag 2400
   aataaacggg ggtatttgga cttgtttttt tcgcggaaaa gcgtcgaatc aaccctgcgg 2460
   gccttgcacc atgtccacga cgtgtttctc gccccaattc gccccttgca cgtcaaaatt 2520
   aggcctccat ctagacccct ccataacatg tgactgtggg gaaaagtata agggaaacca 2580
   tgcaaccata gacgacgtga aagacgggga ggaaccaatg gaggccaaag aaatggggta 2640
   gcaacagtcc aggagacaga caaggagaca aggagagggc gcccgaaaga tcggaaaaac 2700
   aaacatgtcc aattggggca gtgacggaaa cgacacggac acttcagtac aatggaccga 2760
   ccatctccaa gccagggtta ttccggtatc accttggccg taacctcccg ctggtacctg 2820
   atattgtaca cgttcacatt caatatactt tcagctacaa taagagaggc tgtttgtcgg 2880
   gcatgtgtgt ccgtcgtatg gggtgatgtc cgagggcgaa attcgctaca agcttaactc 2940
   tggcgcttgt ccagtatgaa tagacaagtc aagaccagtg gtgccatgat tgacagggag 3000
   gtacaagact tcgatactcg agcattactc ggacttgtgg cgattgaaca gacgggcgat 3060
   cgcttctccc ccgtattgcc ggcgcgccag ctgcattaat gaatcggcca acgcgcgggg 3120
   agaggcggtt tgcgtattgg gcgctcttcc gcttcctcgc tcactgactc gctgcgctcg 3180
   gtcgttcggc tgcggcgagc ggtatcagct cactcaaagg cggtaatacg gttatccaca 3240
   gaatcagggg ataacgcagg aaagaacatg tgagcaaaag gccagcaaaa ggccaggaac 3300
   cgtaaaaagg ccgcgttgct ggcgtttttc cataggctcc gcccccctga cgagcatcac 3360
   aaaaatcgac gctcaagtca gaggtggcga aacccgacag gactataaag ataccaggcg 3420
   tttccccctg gaagctccct cgtgcgctct cctgttccga ccctgccgct taccggatac 3480
   ctgtccgcct ttctcccttc gggaagcgtg gcgctttctc atagctcacg ctgtaggtat 3540
   ctcagttcgg tgtaggtcgt tcgctccaag ctgggctgtg tgcacgaacc ccccgttcag 3600
   cccgaccgct gcgccttatc cggtaactat cgtcttgagt ccaacccggt aagacacgac 3660
   ttatcgccac tggcagcagc cactggtaac aggattagca gagcgaggta tgtaggcggt 3720
   gctacagagt tcttgaagtg gtggcctaac tacggctaca ctagaagaac agtatttggt 3780
   atctgcgctc tgctgaagcc agttaccttc ggaaaaagag ttggtagctc ttgatccggc 3840
   aaacaaacca ccgctggtag cggtggtttt tttgtttgca agcagcagat tacgcgcaga 3900
   aaaaaaggat ctcaagaaga tcctttgatc ttttctacgg ggtctgacgc tcagtggaac 3960
   gaaaactcac gttaagggat tttggtcatg agattatcaa aaaggatctt cacctagatc 4020
   cttttaaatt aaaaatgaag ttttaaatca atctaaagta tatatgagta aacttggtct 4080
   gacagttacc aatgcttaat cagtgaggca cctatctcag cgatctgtct atttcgttca 4140
   tccatagttg cctgactccc cgtcgtgtag ataactacga tacgggaggg cttaccatct 4200
   ggccccagtg ctgcaatgat accgcgagac ccacgctcac cggctccaga tttatcagca 4260
   ataaaccagc cagccggaag ggccgagcgc agaagtggtc ctgcaacttt atccgcctcc 4320
   atccagtcta ttaattgttg ccgggaagct agagtaagta gttcgccagt taatagtttg 4380
   cgcaacgttg ttgccattgc tacaggcatc gtggtgtcac gctcgtcgtt tggtatggct 4440
   tcattcagct ccggttccca acgatcaagg cgagttacat gatcccccat gttgtgcaaa 4500
   aaagcggtta gctccttcgg tcctccgatc gttgtcagaa gtaagttggc cgcagtgtta 4560
   tcactcatgg ttatggcagc actgcataat tctcttactg tcatgccatc cgtaagatgc 4620
   ttttctgtga ctggtgagta ctcaaccaag tcattctgag aatagtgtat gcggcgaccg 4680
   agttgctctt gcccggcgtc aatacgggat aataccgcgc cacatagcag aactttaaaa 4740
   gtgctcatca ttggaaaacg ttcttcgggg cgaaaactct caaggatctt accgctgttg 4800
   agatccagtt cgatgtaacc cactcgtgca cccaactgat cttcagcatc ttttactttc 4860
   accagcgttt ctgggtgagc aaaaacagga aggcaaaatg ccgcaaaaaa gggaataagg 4920
   gcgacacgga aatgttgaat actcatactc ttcctttttc aatattattg aagcatttat 4980
   cagggttatt gtctcatgag cggatacata tttgaatgta tttagaaaaa taaacaaata 5040
   ggggttccgc gcacatttcc ccgaaaagtg ccacctgatg cggtgtgaaa taccgcacag 5100
   atgcgtaagg agaaaatacc gcatcaggaa attgtaagcg ttaatatttt gttaaaattc 5160
   gcgttaaatt tttgttaaat cagctcattt tttaaccaat aggccgaaat cggcaaaatc 5220
   ccttataaat caaaagaata gaccgagata gggttgagtg ttgttccagt ttggaacaag 5280
   agtccactat taaagaacgt ggactccaac gtcaaagggc gaaaaaccgt ctatcagggc 5340
   gatggcccac tacgtgaacc atcaccctaa tcaagttttt tggggtcgag gtgccgtaaa 5400
   gcactaaatc ggaaccctaa agggagcccc cgatttagag cttgacgggg aaagccggcg 5460
   aacgtggcga gaaaggaagg gaagaaagcg aaaggagcgg gcgctagggc gctggcaagt 5520
   gtagcggtca cgctgcgcgt aaccaccaca cccgccgcgc ttaatgcgcc gctacagggc 5580
   gcgtccattc gccattcagg ctgcgcaact gttgggaagg gcgatcggtg cgggcctctt 5640
   cgctattacg ccagctggcg aaagggggat gtgctgcaag gcgattaagt tgggtaacgc 5700
   cagggttttc ccagtcacga cgttgtaaaa cgacggccag tgaattgtaa tacgactcac 5760
   tatagggcga attgggcccg acgtcgcatg cgctgatgac actttggtct gaaagagatg 5820
   cattttgaat cccaaacttg cagtgcccaa gtgacataca tctccgcgtt ttggaaaatg 5880
   ttcagaaaca gttgattgtg ttggaatggg gaatggggaa tggaaaaatg actcaagtat 5940
   caattccaaa aacttctctg gctggcagta cctactgtcc atactactgc attttctcca 6000
   gtcaggccac tctatactcg acgacacagt agtaaaaccc agataatttc gacataaaca 6060
   agaaaacaga cccaataata tttatatata gtcagccgtt tgtccagttc agactgtaat 6120
   agccgaaaaa aaatccaaag tttctattct aggaaaatat attccaatat ttttaattct 6180
   taatctcatt tattttattc tagcgaaata catttcagct acttgagaca tgtgataccc 6240
   acaaatcgga ttcggactcg gttgttcaga agagcatatg gcattcgtgc tcgcttgttc 6300
   acgtattctt cctgttccat ctcttggccg acaatcacac aaaaatgggg tttttttttt 6360
   aattctaatg attcattaca gcaaaattga gatatagcag accacgtatt ccataatcac 6420
   caaggaagtt cttgggcgtc ttaattaact cacctgcagg attgagacta tgaatggatt 6480
   cccgtgcccg tattactcta ctaatttgat cttggaacgc gaaaatacgt ttctaggact 6540
   ccaaagaatc tcaactcttg tccttactaa atatactacc catagttgat ggtttacttg 6600
   aacagagagg acatgttcac ttgacccaaa gtttctcgca tctcttggat atttgaacaa 6660
   cggcgtccac tgaccgtcag ttatccagtc acaaaacccc cacattcata cattcccatg 6720
   tacgtttaca aagttctcaa ttccatcgtg caaatcaaaa tcacatctat tcattcatca 6780
   tatataaacc catcatgtct actaacactc acaactccat agaaaacatc gactcagaac 6840
   acacgctcca tgcggccgct tactgagcct tggcaccggg ctgcttctcg gccattcgag 6900
   cgaactggga caggtatcgg agcaggatga cgagaccttc atggggcaga gggtttcggt 6960
   aggggaggtt gtgcttctgg cacagctgtt ccacctggta ggaaacggca gtgaggttgt 7020
   gtcgaggcag ggtgggccag agatggtgct cgatctggta gttcaggcct ccaaagaacc 7080
   agtcagtaat gatgcctcgt cgaatgttca tggtctcatg gatctgaccc acagagaagc 7140
   catgtccgtc ccagacggaa tcaccgatct tctccagagg gtagtggttc atgaagacca 7200
   cgatggcaat tccgaagcca ccgacgagct cggaaacaaa gaacaccagc atcgaggtca 7260
   ggatggaggg cataaagaag aggtggaaca gggtcttgag agtccagtgc agagcgagtc 7320
   caatggcctc tttcttgtac tgagatcggt agaactggtt gtctcggtcc ttgagggatc 7380
   gaacggtcag cacagactgg aaacaccaga tgaatcgcag gagaatacag atgaccagga 7440
   aatagtactg ttggaactga atgagctttc gggagatggg agaagctcga gtgacatcgt 7500
   cctcggacca ggcgagcaga ggcaggttat caatgtcggg atcgtgaccc tgaacgttgg 7560
   tagcagaatg atgggcgttg tgtctgtcct tccaccaggt cacggagaag ccctggagtc 7620
   cgttgccaaa gaccagaccc aggacgttat tccagtttcg gttcttgaag gtctggtggt 7680
   ggcagatgtc atgagacagc catcccattt gctggtagtg cataccgagc acgagagcac 7740
   caatgaagta caggtggtac tggaccagca tgaagaaggc aagcacgcca agacccaggg 7800
   tggtcaagat cttgtacgag taccagaggg gagaggcgtc aaacatgcca gtggcgatca 7860
   gctcttctcg gagctttcgg aaatcctcct gagcttcgtt gacggcagcc tggggaggca 7920
   gctcggaagc ctggttgatc ttgggcattc gcttgagctt gtcgaaggct tcctgagagt 7980
   gcataaccat gaaggcgtca gtagcatctc gtccctggta gttctcaatg atttcagctc 8040
   caccagggtg gaagttcacc caagcggaga cgtcgtacac ctttccgtcg atgacgaggg 8100
   gcagagcctg tcgagaagcc ttcaccatgg ttgtgaatta gggtggtgag aatggttggt 8160
   tgtagggaag aatcaaaggc cggtctcggg atccgtgggt atatatatat atatatatat 8220
   atacgatcct tcgttacctc cctgttctca aaactgtggt ttttcgtttt tcgttttttg 8280
   ctttttttga tttttttagg gccaactaag cttccagatt tcgctaatca cctttgtact 8340
   aattacaaga aaggaagaag ctgattagag ttgggctttt tatgcaactg tgctactcct 8400
   tatctctgat atgaaagtgt agacccaatc acatcatgtc atttagagtt ggtaatactg 8460
   ggaggataga taaggcacga aaacgagcca tagcagacat gctgggtgta gccaagcaga 8520
   agaaagtaga tgggagccaa ttgacgagcg agggagctac gccaatccga catacgacac 8580
   gctgagatcg tcttggccgg ggggtaccta cagatgtcca agggtaagtg cttgactgta 8640
   attgtatgtc tgaggacaaa tatgtagtca gccgtataaa gtcataccag gcaccagtgc 8700
   catcatcgaa ccactaactc tctatgatac atgcctccgg tattattgta ccatgcgtcg 8760
   ctttgttaca tacgtatctt gcctttttct ctcagaaact ccagactttg gctattggtc 8820
   gagataagcc cggaccatag tgagtctttc acactctaca tttctccctt gctccaacta 8880
   tttaaattcc ttcacttcaa gttcattctt catctgcttc tgttttactt tgacaggcaa 8940
   atgaagacat ggtacgactt gatggaggcc aagaacgcca tttcaccccg agacaccgaa 9000
   gtgcctgaaa tcctggctgc ccccattgat aacatcggaa actacggtat tccggaaagt 9060
   gtatatagaa cctttcccca gcttgtgtct gtggatatgg atggtgtaat cccctttgag 9120
   tactcgtctt ggcttctctc cgagcagtat gaggctctct aatctagcgc atttaatatc 9180
   tcaatgtatt tatatattta tcttctcatg cggccgctta ctgagccttg gcaccgggct 9240
   gcttctcggc cattcgagcg aactgggaca ggtatcggag caggatgacg agaccttcat 9300
   ggggcagagg gtttcggtag gggaggttgt gcttctggca cagctgttcc acctggtagg 9360
   aaacggcagt gaggttgtgt cgaggcaggg tgggccagag atggtgctcg atctggtagt 9420
   tcaggcctcc aaagaaccag tcagtaatga tgcctcgtcg aatgttcatg gtctcatgga 9480
   tctgacccac agagaagcca tgtccgtccc agacggaatc accgatcttc tccagagggt 9540
   agtggttcat gaagaccacg atggcaattc cgaagccacc gacgagctcg gaaacaaaga 9600
   acaccagcat cgaggtcagg atggagggca taaagaagag gtggaacagg gtcttgagag 9660
   tccagtgcag agcgagtcca atggcctctt tcttgtactg agatcggtag aactggttgt 9720
   ctcggtcctt gagggatcga acggtcagca cagactggaa acaccagatg aatcgcagga 9780
   gaatacagat gaccaggaaa tagtactgtt ggaactgaat gagctttcgg gagatgggag 9840
   aagctcgagt gacatcgtcc tcggaccagg cgagcagagg caggttatca atgtcgggat 9900
   cgtgaccctg aacgttggta gcagaatgat gggcgttgtg tctgtccttc caccaggtca 9960
   cggagaagcc ctggagtccg ttgccaaaga ccagacccag gacgttattc cagtttcggt 10020
   tcttgaaggt ctggtggtgg cagatgtcat gagacagcca tcccatttgc tggtagtgca 10080
   taccgagcac gagagcacca atgaagtaca ggtggtactg gaccagcatg aagaaggcaa 10140
   gcacgccaag acccagggtg gtcaagatct tgtacgagta ccagagggga gaggcgtcaa 10200
   acatgccagt ggcgatcagc tcttctcgga gctttcggaa atcctcctga gcttcgttga 10260
   cggcagcctg gggaggcagc tcggaagcct ggttgatctt gggcattcgc ttgagcttgt 10320
   cgaaggcttc ctgagagtgc ataaccatga aggcgtcagt agcatctcgt ccctggtagt 10380
   tctcaatgat ttcagctcca ccagggtgga agttcaccca agcggagacg tcgtacacct 10440
   ttccgtcgat gacgaggggc agagcctgtc gagaagcctt caccatgggc aggacctgtg 10500
   ttagtacatt gtcggggagt catcaattgg ttcgacaggt tgtcgactgt tagtatgagc 10560
   tcaattgggc tctggtgggt cgatgacact tgtcatctgt ttctgttggg tcatgtttcc 10620
   atcaccttct atggtactca caattcgtcc gattcgcccg aatccgttaa taccgacttt 10680
   gatggccatg ttgatgtgtg tttaattcaa gaatgaatat agagaagaga agaagaaaaa 10740
   agattcaatt gagccggcga tgcagaccct tatataaatg ttgccttgga cagacggagc 10800
   aagcccgccc aaacctacgt tcggtataat atgttaagct ttttaacaca aaggtttggc 10860
   ttggggtaac ctgatgtggt gcaaaagacc gggcgttggc gagccattgc gcgggcgaat 10920
   ggggccgtga ctcgtctcaa attcgagggc gtgcctcaat tcgtgccccc gtggcttttt 10980
   cccgccgttt ccgccccgtt tgcaccactg cagccgcttc tttggttcgg acaccttgct 11040
   gcgagctagg tgccttgtgc tacttaaaaa gtggcctccc aacaccaaca tgacatgagt 11100
   gcgtgggcca agacacgttg gcggggtcgc agtcggctca atggcccgga aaaaacgctg 11160
   ctggagctgg ttcggacgca gtccgccgcg gcgtatggat atccgcaagg ttccatagcg 11220
   ccattgccct ccgtcggcgt ctatcccgca acctctaaat agagcgggaa tataacccaa 11280
   gcttcttttt tttcctttaa cacgcacacc cccaactatc atgttgctgc tgctgtttga 11340
   ctctactctg tggaggggtg ctcccaccca acccaaccta caggtggatc cggcgctgtg 11400
   attggctgat aagtctccta tccggactaa ttctgaccaa tgggacatgc gcgcaggacc 11460
   caaatgccgc aattacgtaa ccccaacgaa atgcctaccc ctctttggag cccagcggcc 11520
   ccaaatcccc ccaagcagcc cggttctacc ggcttccatc tccaagcaca agcagcccgg 11580
   aattccttta cctgcaggat aacttcgtat aatgtatgct atacgaagtt atgatctctc 11640
   tcttgagctt ttccataaca agttcttctg cctccaggaa gtccatgggt ggtttgatca 11700
   tggttttggt gtagtggtag tgcagtggtg gtattgtgac tggggatgta gttgagaata 11760
   agtcatacac aagtcagctt tcttcgagcc tcatataagt ataagtagtt caacgtatta 11820
   gcactgtacc cagcatctcc gtatcgagaa acacaacaac atgccccatt ggacagatca 11880
   tgcggataca caggttgtgc agtatcatac atactcgatc agacaggtcg tctgaccatc 11940
   atacaagctg aacaagcgct ccatacttgc acgctctcta tatacacagt taaattacat 12000
   atccatagtc taacctctaa cagttaatct tctggtaagc ctcccagcca gccttctggt 12060
   atcgcttggc ctcctcaata ggatctcggt tctggccgta cagacctcgg ccgacaatta 12120
   tgatatccgt tccggtagac atgacatcct caacagttcg gtactgctgt ccgagagcgt 12180
   ctcccttgtc gtcaagaccc accccggggg tcagaataag ccagtcctca gagtcgccct 12240
   taggtcggtt ctgggcaatg aagccaacca caaactcggg gtcggatcgg gcaagctcaa 12300
   tggtctgctt ggagtactcg ccagtggcca gagagccctt gcaagacagc tcggccagca 12360
   tgagcagacc tctggccagc ttctcgttgg gagaggggac taggaactcc ttgtactggg 12420
   agttctcgta gtcagagacg tcctccttct tctgttcaga gacagtttcc tcggcaccag 12480
   ctcgcaggcc agcaatgatt ccggttccgg gtacaccgtg ggcgttggtg atatcggacc 12540
   actcggcgat tcggtgacac cggtactggt gcttgacagt gttgccaata tctgcgaact 12600
   ttctgtcctc gaacaggaag aaaccgtgct taagagcaag ttccttgagg gggagcacag 12660
   tgccggcgta ggtgaagtcg tcaatgatgt cgatatgggt tttgatcatg cacacataag 12720
   gtccgacctt atcggcaagc tcaatgagct ccttggtggt ggtaacatcc agagaagcac 12780
   acaggttggt tttcttggct gccacgagct tgagcactcg agcggcaaag gcggacttgt 12840
   ggacgttagc tcgagcttcg taggagggca ttttggtggt gaagaggaga ctgaaataaa 12900
   tttagtctgc agaacttttt atcggaacct tatctggggc agtgaagtat atgttatggt 12960
   aatagttacg agttagttga acttatagat agactggact atacggctat cggtccaaat 13020
   tagaaagaac gtcaatggct ctctgggcgt cgcctttgcc gacaaaaatg tgatcatgat 13080
   gaaagccagc aatgacgttg cagctgatat tgttgtcggc caaccgcgcc gaaaacgcag 13140
   ctgtcagacc cacagcctcc aacgaagaat gtatcgtcaa agtgatccaa gcacactcat 13200
   agttggagtc gtactccaaa ggcggcaatg acgagtcaga cagatactcg tcgacgcgat 13260
   aacttcgtat aatgtatgct atacgaagtt atcgtacgat agttagtaga caacaatcga 13320
   taacgtctcg taccaaccac agattacgac ccattcgcag tcacagttca ctagggtttg 13380
   ggttgcatcc gttgagagcg gtttgttttt aaccttctcc atgtgctcac tcaggttttg 13440
   ggttcagatc aaatcaaggc gtgaaccact ttgtttgagg acaaatgtga cacaaccaac 13500
   cagtgtcagg ggcaagtccg tgacaaaggg gaagatacaa tgcaattact gacagttaca 13560
   gactgcctcg atgccctaac cttgccccaa aataagacaa ctgtcctcgt ttaagcgcaa 13620
   ccctattcag cgtcacgtca taatagcgtt tggatagcac tagtctatga ggagcgtttt 13680
   atgttgcggt gagggcgatt ggtgctcata tgggttcaat tgaggtggcg gaacgagctt 13740
   agtcttcaat tgaggtgcga gcgacacaat tgggtgtcac gtggcctaat tgacctcggg 13800
   tcgtggagtc cccagttata cagcaaccac gaggtgcatg ggtaggagac gtcaccagac 13860
   aatagggttt tttttggact ggagagggtt gggcaaaagc gctcaacggg ctgtttgggg 13920
   agctgtgggg gaggaattgg cgatatttgt gaggttaacg gctccgattt gcgtgttttg 13980
   tcgctcctgc atctccccat acccatatct tccctcccca cctctttcca cgataatttt 14040
   acggatcagc aataaggttc cttctcctag tttccacgtc catatatatc tatgctgcgt 14100
   cgtccttttc gtgacatcac caaaacacat acaacaatgg ctgttactga cgtccttaag 14160
   cgaaagtccg gtgtcatcgt cggcgacgat gtccgagccg tgagtatcca cgacaagatc 14220
   agtgtcgaga cgacgcgttt tgtgtaatga cacaatccga aagtcgctag caacacacac 14280
   tctctacaca aactaaccca gctctccatg gcctccacct cggctctgcc caagcagaac 14340
   cctgccctcc gacgaaccgt cacttccacc actgtgaccg actcggagtc tgctgccgtc 14400
   tctccctccg attctcccag acactcggcc tcctctacat cgctgtcttc catgtccgag 14460
   gtggacattg ccaagcccaa gtccgagtac ggtgtcatgc tggataccta cggcaaccag 14520
   ttcgaagttc ccgacttcac catcaaggac atctacaacg ctattcccaa gcactgcttc 14580
   aagcgatctg ctctcaaggg atacggctac attcttcgag acattgtcct cctgactacc 14640
   actttcagca tctggtacaa ctttgtgaca cccgagtaca ttccctccac tcctgctcga 14700
   gccggtctgt gggctgtgta caccgttctt cagggactct tcggtactgg actgtgggtc 14760
   attgcccacg agtgtggaca tggtgctttc tccgattccc gaatcatcaa cgacattact 14820
   ggctgggtgc ttcactcttc cctgcttgtt ccctacttca gctggcaaat ctcccaccgg 14880
   aagcatcaca aggccactgg aaacatggag cgagacatgg tcttcgttcc tcgaacccga 14940
   gagcagcaag ctactcgact cggcaagatg acccacgaac tcgcccatct taccgaggaa 15000
   actcctgctt tcaccctgct catgcttgtg cttcagcaac tggtcggttg gcccaactat 15060
   ctcattacca acgttactgg acacaactac catgagcggc agcgagaggg tcgaggcaag 15120
   ggaaagcaca acggtcttgg cggtggagtt aaccatttcg atccccgatc tcctctgtac 15180
   gagaacagcg acgccaagct catcgtgctc tccgacattg gcattggtct tatggccacc 15240
   gctctgtact ttctcgttca gaagttcgga ttctacaaca tggccatctg gtacttcgtt 15300
   ccctacttgt gggttaacca ctggctcgtc gccattacct ttctgcagca cacagatcct 15360
   actcttcccc actacaccaa cgacgagtgg aactttgtgc gaggtgccgc tgcaaccatc 15420
   gaccgagaga tgggcttcat tggacgtcat ctgctccacg gcattatcga gactcacgtc 15480
   ctgcatcact acgtctcttc cattcccttc tacaatgcgg acgaagctac cgaggccatc 15540
   aaacctatca tgggcaagca ctatcgagct gatgtccagg acggtcctcg aggattcatt 15600
   cgagccatgt accgatctgc acgaatgtgc cagtgggttg aaccctccgc tggtgccgag 15660
   ggagctggca agggtgtcct gttctttcga aaccgaaaca atgtgggcac tcctcccgct 15720
   gtcatcaagc ccgttgccta agc 15743
<210> 56
   <211> 1434
   <212> DNA
   <213> Fusarium moniliforme
<220>
   <221> CDS
   <222> (1)..(1434)
   <223> synthetic delta-12 desaturase (codon-optimized for Yarrowia lipolytica)
<300>
   <302> DELTA-12 DESATURASES SUITABLE FOR ALTERING LEVELS OF POLYUNSATURATED FATTY ACIDS IN OLEAGINOUS YEAST
<310> WO 2005/047485
   <311> 2004-11-12
   <312> 2005-05-26
   <313> (1)..(1434)
<300>
   <302> DELTA-12 DESATURASES SUITABLE FOR ALTERING LEVELS OF POLYUNSATURATED FATTY ACIDS IN OLEAGINOUS YEAST
<310> US 2005-0216975-A1
   <311> 2004-11-10
   <312> 2005-09-29
   <313> (1)..(1434)
<400> 56
   atg gcc tcc acc tcg gct ctg ccc aag cag aac cct gcc ctc cga cga      48
   Met Ala Ser Thr Ser Ala Leu Pro Lys Gln Asn Pro Ala Leu Arg Arg
   1               5                   10                  15
   acc gtc act tcc acc act gtg acc gac tcg gag tct gct gcc gtc tct      96
   Thr Val Thr Ser Thr Thr Val Thr Asp Ser Glu Ser Ala Ala Val Ser
               20                  25                  30
   ccc tcc gat tct ccc aga cac tcg gcc tcc tct aca tcg ctg tct tcc     144
   Pro Ser Asp Ser Pro Arg His Ser Ala Ser Ser Thr Ser Leu Ser Ser
           35                  40                  45
   atg tcc gag gtg gac att gcc aag ccc aag tcc gag tac ggt gtc atg     192
   Met Ser Glu Val Asp Ile Ala Lys Pro Lys Ser Glu Tyr Gly Val Met
       50                  55                  60
   ctg gat acc tac ggc aac cag ttc gaa gtt ccc gac ttc acc atc aag     240
   Leu Asp Thr Tyr Gly Asn Gln Phe Glu Val Pro Asp Phe Thr Ile Lys
   65                  70                  75                  80
   gac atc tac aac gct att ccc aag cac tgc ttc aag cga tct gct ctc     288
   Asp Ile Tyr Asn Ala Ile Pro Lys His Cys Phe Lys Arg Ser Ala Leu
                   85                  90                  95
   aag gga tac ggc tac att ctt cga gac att gtc ctc ctg act acc act     336
   Lys Gly Tyr Gly Tyr Ile Leu Arg Asp Ile Val Leu Leu Thr Thr Thr
               100                 105                 110
   ttc agc atc tgg tac aac ttt gtg aca ccc gag tac att ccc tcc act     384
   Phe Ser Ile Trp Tyr Asn Phe Val Thr Pro Glu Tyr Ile Pro Ser Thr
           115                 120                 125
   cct gct cga gcc ggt ctg tgg gct gtg tac acc gtt ctt cag gga ctc     432
   Pro Ala Arg Ala Gly Leu Trp Ala Val Tyr Thr Val Leu Gln Gly Leu
       130                 135                 140
   ttc ggt act gga ctg tgg gtc att gcc cac gag tgt gga cat ggt gct     480
   Phe Gly Thr Gly Leu Trp Val Ile Ala His Glu Cys Gly His Gly Ala
   145                 150                 155                 160
   ttc tcc gat tcc cga atc atc aac gac att act ggc tgg gtg ctt cac     528
   Phe Ser Asp Ser Arg Ile Ile Asn Asp Ile Thr Gly Trp Val Leu His
                   165                 170                 175
   tct tcc ctg ctt gtt ccc tac ttc agc tgg caa atc tcc cac cgg aag     576
   Ser Ser Leu Leu Val Pro Tyr Phe Ser Trp Gln Ile Ser His Arg Lys
               180                 185                 190
   cat cac aag gcc act gga aac atg gag cga gac atg gtc ttc gtt cct     624
   His His Lys Ala Thr Gly Asn Met Glu Arg Asp Met Val Phe Val Pro
           195                 200                 205
   cga acc cga gag cag caa gct act cga ctc ggc aag atg acc cac gaa       672
   Arg Thr Arg Glu Gln Gln Ala Thr Arg Leu Gly Lys Met Thr His Glu
       210                 215                 220
   ctc gcc cat ctt acc gag gaa act cct gct ttc acc ctg ctc atg ctt       720
   Leu Ala His Leu Thr Glu Glu Thr Pro Ala Phe Thr Leu Leu Met Leu
   225                 230                 235                 240
   gtg ctt cag caa ctg gtc ggt tgg ccc aac tat ctc att acc aac gtt       768
   Val Leu Gln Gln Leu Val Gly Trp Pro Asn Tyr Leu Ile Thr Asn Val
                   245                 250                 255
   act gga cac aac tac cat gag cgg cag cga gag ggt cga ggc aag gga       816
   Thr Gly His Asn Tyr His Glu Arg Gln Arg Glu Gly Arg Gly Lys Gly
               260                 265                 270
   aag cac aac ggt ctt ggc ggt gga gtt aac cat ttc gat ccc cga tct       864
   Lys His Asn Gly Leu Gly Gly Gly Val Asn His Phe Asp Pro Arg Ser
           275                 280                 285
   cct ctg tac gag aac agc gac gcc aag ctc atc gtg ctc tcc gac att       912
   Pro Leu Tyr Glu Asn Ser Asp Ala Lys Leu Ile Val Leu Ser Asp Ile
       290                 295                 300
   ggc att ggt ctt atg gcc acc gct ctg tac ttt ctc gtt cag aag ttc       960
   Gly Ile Gly Leu Met Ala Thr Ala Leu Tyr Phe Leu Val Gln Lys Phe
   305                 310                 315                 320
   gga ttc tac aac atg gcc atc tgg tac ttc gtt ccc tac ttg tgg gtt      1008
   Gly Phe Tyr Asn Met Ala Ile Trp Tyr Phe Val Pro Tyr Leu Trp Val
                   325                 330                 335
   aac cac tgg ctc gtc gcc att acc ttt ctg cag cac aca gat cct act      1056
   Asn His Trp Leu Val Ala Ile Thr Phe Leu Gln His Thr Asp Pro Thr
               340                 345                 350
   ctt ccc cac tac acc aac gac gag tgg aac ttt gtg cga ggt gcc gct      1104
   Leu Pro His Tyr Thr Asn Asp Glu Trp Asn Phe Val Arg Gly Ala Ala
           355                 360                 365
   gca acc atc gac cga gag atg ggc ttc att gga cgt cat ctg ctc cac      1152
   Ala Thr Ile Asp Arg Glu Met Gly Phe Ile Gly Arg His Leu Leu His
       370                 375                 380
   ggc att atc gag act cac gtc ctg cat cac tac gtc tct tcc att ccc      1200
   Gly Ile Ile Glu Thr His Val Leu His His Tyr Val Ser Ser Ile Pro
   385                 390                 395                 400
   ttc tac aat gcg gac gaa gct acc gag gcc atc aaa cct atc atg ggc      1248
   Phe Tyr Asn Ala Asp Glu Ala Thr Glu Ala Ile Lys Pro Ile Met Gly
                   405                 410                 415
   aag cac tat cga gct gat gtc cag gac ggt cct cga gga ttc att cga      1296
   Lys His Tyr Arg Ala Asp Val Gln Asp Gly Pro Arg Gly Phe Ile Arg
               420                 425                 430
   gcc atg tac cga tct gca cga atg tgc cag tgg gtt gaa ccc tcc gct      1344
   Ala Met Tyr Arg Ser Ala Arg Met Cys Gln Trp Val Glu Pro Ser Ala
           435                 440                 445
   ggt gcc gag gga gct ggc aag ggt gtc ctg ttc ttt cga aac cga aac      1392
   Gly Ala Glu Gly Ala Gly Lys Gly Val Leu Phe Phe Arg Asn Arg Asn
       450                 455                 460
   aat gtg ggc act cct ccc gct gtc atc aag ccc gtt gcc taa              1434
   Asn Val Gly Thr Pro Pro Ala Val Ile Lys Pro Val Ala
   465                 470                 475
<210> 57
   <211> 477
   <212> PRT
   <213> Fusarium moniliforme
<400> 57
   Met Ala Ser Thr Ser Ala Leu Pro Lys Gln Asn Pro Ala Leu Arg Arg
   1               5                   10                  15
   Thr Val Thr Ser Thr Thr Val Thr Asp Ser Glu Ser Ala Ala Val Ser
               20                  25                  30
   Pro Ser Asp Ser Pro Arg His Ser Ala Ser Ser Thr Ser Leu Ser Ser
           35                  40                  45
   Met Ser Glu Val Asp Ile Ala Lys Pro Lys Ser Glu Tyr Gly Val Met
       50                  55                  60
   Leu Asp Thr Tyr Gly Asn Gln Phe Glu Val Pro Asp Phe Thr Ile Lys
   65                  70                  75                  80
   Asp Ile Tyr Asn Ala Ile Pro Lys His Cys Phe Lys Arg Ser Ala Leu
                   85                  90                  95
   Lys Gly Tyr Gly Tyr Ile Leu Arg Asp Ile Val Leu Leu Thr Thr Thr
               100                 105                 110
   Phe Ser Ile Trp Tyr Asn Phe Val Thr Pro Glu Tyr Ile Pro Ser Thr
           115                 120                 125
   Pro Ala Arg Ala Gly Leu Trp Ala Val Tyr Thr Val Leu Gln Gly Leu
       130                 135                 140
   Phe Gly Thr Gly Leu Trp Val Ile Ala His Glu Cys Gly His Gly Ala
   145                 150                 155                 160
   Phe Ser Asp Ser Arg Ile Ile Asn Asp Ile Thr Gly Trp Val Leu His
                   165                 170                 175
   Ser Ser Leu Leu Val Pro Tyr Phe Ser Trp Gln Ile Ser His Arg Lys
   180 185 190
   His His Lys Ala Thr Gly Asn Met Glu Arg Asp Met Val Phe Val Pro
   195 200 205
   Arg Thr Arg Glu Gln Gln Ala Thr Arg Leu Gly Lys Met Thr His Glu
   210 215 220
   Leu Ala His Leu Thr Glu Glu Thr Pro Ala Phe Thr Leu Leu Met Leu
   225 230 235 240
   Val Leu Gln Gln Leu Val Gly Trp Pro Asn Tyr Leu Ile Thr Asn Val
   245 250 255
   Thr Gly His Asn Tyr His Glu Arg Gln Arg Glu Gly Arg Gly Lys Gly
   260 265 270
   Lys His Asn Gly Leu Gly Gly Gly Val Asn His Phe Asp Pro Arg Ser
   275 280 285
   Pro Leu Tyr Glu Asn Ser Asp Ala Lys Leu Ile Val Leu Ser Asp Ile
   290 295 300
   Gly Ile Gly Leu Met Ala Thr Ala Leu Tyr Phe Leu Val Gln Lys Phe
   305 310 315 320
   Gly Phe Tyr Asn Met Ala Ile Trp Tyr Phe Val Pro Tyr Leu Trp Val
   325 330 335
   Asn His Trp Leu Val Ala Ile Thr Phe Leu Gln His Thr Asp Pro Thr
   340 345 350
   Leu Pro His Tyr Thr Asn Asp Glu Trp Asn Phe Val Arg Gly Ala Ala
   355 360 365
   Ala Thr Ile Asp Arg Glu Met Gly Phe Ile Gly Arg His Leu Leu His
   370 375 380
   Gly Ile Ile Glu Thr His Val Leu His His Tyr Val Ser Ser Ile Pro
   385 390 395 400
   Phe Tyr Asn Ala Asp Glu Ala Thr Glu Ala Ile Lys Pro Ile Met Gly
   405 410 415
   Lys His Tyr Arg Ala Asp Val Gln Asp Gly Pro Arg Gly Phe Ile Arg
               420                 425                 430
   Ala Met Tyr Arg Ser Ala Arg Met Cys Gln Trp Val Glu Pro Ser Ala
           435                 440                 445
   Gly Ala Glu Gly Ala Gly Lys Gly Val Leu Phe Phe Arg Asn Arg Asn
       450                 455                 460
   Asn Val Gly Thr Pro Pro Ala Val Ile Lys Pro Val Ala
   465                 470                 475
<210> 58
   <211> 6303
   <212> DNA
   <213> Artificial Sequence
<220>
   <223> Plasmid pZKUE3S
<400> 58
   ggccgcaagt gtggatgggg aagtgagtgc ccggttctgt gtgcacaatt ggcaatccaa     60
   gatggatgga ttcaacacag ggatatagcg agctacgtgg tggtgcgagg atatagcaac    120
   ggatatttat gtttgacact tgagaatgta cgatacaagc actgtccaag tacaatacta    180
   aacatactgt acatactcat actcgtaccc gggcaacggt ttcacttgag tgcagtggct    240
   agtgctctta ctcgtacagt gtgcaatact gcgtatcata gtctttgatg tatatcgtat    300
   tcattcatgt tagttgcgta cgaggaaact gtctctgaac agaagaagga ggacgtctct    360
   gactacgaga actcccagta caaggagttc ctagtcccct ctcccaacga gaagctggcc    420
   agaggtctgc tcatgctggc cgagctgtct tgcaagggct ctctggccac tggcgagtac    480
   tccaagcaga ccattgagct tgcccgatcc gaccccgagt ttgtggttgg cttcattgcc    540
   cagaaccgac ctaagggcga ctctgaggac tggcttattc tgacccccgg ggtgggtctt    600
   gacgacaagg gagacgctct cggacagcag taccgaactg ttgaggatgt catgtctacc    660
   ggaacggata tcataattgt cggccgaggt ctgtacggcc agaaccgaga tcctattgag    720
   gaggccaagc gataccagaa ggctggctgg gaggcttacc agaagattaa ctgttagagg    780
   ttagactatg gatatgtaat ttaactgtgt atatagagag cgtgcaagta tggagcgctt    840
   gttcagcttg tatgatggtc agacgacctg tctgatcgag tatgtatgat actgcacaac    900
   ctgtgtatcc gcatgatctg tccaatgggg catgttgttg tgtttctcga tacggagatg    960
   ctgggtacag tgctaatacg ttgaactact tatacttata tgaggctcga agaaagctga   1020
   cttgtgtatg acttaattaa tcgagcttgg cgtaatcatg gtcatagctg tttcctgtgt   1080
   gaaattgtta tccgctcaca attccacaca acatacgagc cggaagcata aagtgtaaag 1140
   cctggggtgc ctaatgagtg agctaactca cattaattgc gttgcgctca ctgcccgctt 1200
   tccagtcggg aaacctgtcg tgccagctgc attaatgaat cggccaacgc gcggggagag 1260
   gcggtttgcg tattgggcgc tcttccgctt cctcgctcac tgactcgctg cgctcggtcg 1320
   ttcggctgcg gcgagcggta tcagctcact caaaggcggt aatacggtta tccacagaat 1380
   caggggataa cgcaggaaag aacatgtgag caaaaggcca gcaaaaggcc aggaaccgta 1440
   aaaaggccgc gttgctggcg tttttccata ggctccgccc ccctgacgag catcacaaaa 1500
   atcgacgctc aagtcagagg tggcgaaacc cgacaggact ataaagatac caggcgtttc 1560
   cccctggaag ctccctcgtg cgctctcctg ttccgaccct gccgcttacc ggatacctgt 1620
   ccgcctttct cccttcggga agcgtggcgc tttctcatag ctcacgctgt aggtatctca 1680
   gttcggtgta ggtcgttcgc tccaagctgg gctgtgtgca cgaacccccc gttcagcccg 1740
   accgctgcgc cttatccggt aactatcgtc ttgagtccaa cccggtaaga cacgacttat 1800
   cgccactggc agcagccact ggtaacagga ttagcagagc gaggtatgta ggcggtgcta 1860
   cagagttctt gaagtggtgg cctaactacg gctacactag aaggacagta tttggtatct 1920
   gcgctctgct gaagccagtt accttcggaa aaagagttgg tagctcttga tccggcaaac 1980
   aaaccaccgc tggtagcggt ggtttttttg tttgcaagca gcagattacg cgcagaaaaa 2040
   aaggatctca agaagatcct ttgatctttt ctacggggtc tgacgctcag tggaacgaaa 2100
   actcacgtta agggattttg gtcatgagat tatcaaaaag gatcttcacc tagatccttt 2160
   taaattaaaa atgaagtttt aaatcaatct aaagtatata tgagtaaact tggtctgaca 2220
   gttaccaatg cttaatcagt gaggcaccta tctcagcgat ctgtctattt cgttcatcca 2280
   tagttgcctg actccccgtc gtgtagataa ctacgatacg ggagggctta ccatctggcc 2340
   ccagtgctgc aatgataccg cgagacccac gctcaccggc tccagattta tcagcaataa 2400
   accagccagc cggaagggcc gagcgcagaa gtggtcctgc aactttatcc gcctccatcc 2460
   agtctattaa ttgttgccgg gaagctagag taagtagttc gccagttaat agtttgcgca 2520
   acgttgttgc cattgctaca ggcatcgtgg tgtcacgctc gtcgtttggt atggcttcat 2580
   tcagctccgg ttcccaacga tcaaggcgag ttacatgatc ccccatgttg tgcaaaaaag 2640
   cggttagctc cttcggtcct ccgatcgttg tcagaagtaa gttggccgca gtgttatcac 2700
   tcatggttat ggcagcactg cataattctc ttactgtcat gccatccgta agatgctttt 2760
   ctgtgactgg tgagtactca accaagtcat tctgagaata gtgtatgcgg cgaccgagtt 2820
   gctcttgccc ggcgtcaata cgggataata ccgcgccaca tagcagaact ttaaaagtgc 2880
   tcatcattgg aaaacgttct tcggggcgaa aactctcaag gatcttaccg ctgttgagat 2940
   ccagttcgat gtaacccact cgtgcaccca actgatcttc agcatctttt actttcacca 3000
   gcgtttctgg gtgagcaaaa acaggaaggc aaaatgccgc aaaaaaggga ataagggcga 3060
   cacggaaatg ttgaatactc atactcttcc tttttcaata ttattgaagc atttatcagg 3120
   gttattgtct catgagcgga tacatatttg aatgtattta gaaaaataaa caaatagggg 3180
   ttccgcgcac atttccccga aaagtgccac ctgacgcgcc ctgtagcggc gcattaagcg 3240
   cggcgggtgt ggtggttacg cgcagcgtga ccgctacact tgccagcgcc ctagcgcccg 3300
   ctcctttcgc tttcttccct tcctttctcg ccacgttcgc cggctttccc cgtcaagctc 3360
   taaatcgggg gctcccttta gggttccgat ttagtgcttt acggcacctc gaccccaaaa 3420
   aacttgatta gggtgatggt tcacgtagtg ggccatcgcc ctgatagacg gtttttcgcc 3480
   ctttgacgtt ggagtccacg ttctttaata gtggactctt gttccaaact ggaacaacac 3540
   tcaaccctat ctcggtctat tcttttgatt tataagggat tttgccgatt tcggcctatt 3600
   ggttaaaaaa tgagctgatt taacaaaaat ttaacgcgaa ttttaacaaa atattaacgc 3660
   ttacaatttc cattcgccat tcaggctgcg caactgttgg gaagggcgat cggtgcgggc 3720
   ctcttcgcta ttacgccagc tggcgaaagg gggatgtgct gcaaggcgat taagttgggt 3780
   aacgccaggg ttttcccagt cacgacgttg taaaacgacg gccagtgaat tgtaatacga 3840
   ctcactatag ggcgaattgg gtaccgggcc ccccctcgag gtcgacgagt atctgtctga 3900
   ctcgtcattg catgcctttg gagtacgact ccaactatga gtgtgcttgg atcactttga 3960
   cgatacattc ttcgttggag gctgtgggtc tgacagctgc gttttcggcg cggttggccg 4020
   acaacaatat cagctgcaac gtcattgctg gctttcatca tgatcacatt tttgtcggca 4080
   aaggcgacgc ccagagagcc attgacgttc tttctaattt ggaccgatag ccgtatagtc 4140
   cagtctatct ataagttcaa ctaactcgta actattacca taacatatac ttcactgccc 4200
   cagataaggt tccgataaaa agttctgcag actaaattta tttcagtctc ctcttcacca 4260
   ccaaaatgcc ctcctacgaa gctcgagtgc tcaagctcgt ggcagccaag aaaaccaacc 4320
   tgtgtgcttc tctggatgtt accaccacca aggagctcat tgagcttgcc gataaggtcg 4380
   gaccttatgt gtgcatgatc aaaacccata tcgacatcat tgacgacttc acctacgccg 4440
   gcactgtgct ccccctcaag gaacttgctc ttaagcacgg tttcttcctg ttcgaggaca 4500
   gaaagttcgc agatattggc aacactgtca agcaccagta ccggtgtcac cgaatcgccg 4560
   agtggtccga tatcaccaac gcccacggtg tttaaacccg gaaccggaat cgataagctt 4620
   gatatcgaat tcatgctgtt catcgtggtt aatgctgctg tgtgctgtgt gtgtgtgttg 4680
   tttggcgctc attgttgcgt tatgcagcgt acaccacaat attggaagct tattagcctt 4740
   tctatttttt cgtttgcaag gcttaacaac attgctgtgg agagggatgg ggatatggag 4800
   gccgctggag ggagtcggag aggcgttttg gagcggcttg gcctggcgcc cagctcgcga 4860
   aacgcaccta ggaccctttg gcacgccgaa atgtgccact tttcagtcta gtaacgcctt 4920
   acctacgtca ttccatgcgt gcatgtttgc gccttttttc ccttgccctt gatcgccaca 4980
   cagtacagtg cactgtacag tggaggtttt gggggggtct tagatgggag ctaaaagcgg 5040
   cctagcggta cactagtggg attgtatgga gtggcatgga gcctaggtgg agcctgacag 5100
   gacgcacgac cggctagccc gtgacagacg atgggtggct cctgttgtcc accgcgtaca 5160
   aatgtttggg ccaaagtctt gtcagccttg cttgcgaacc taattcccaa ttttgtcact 5220
   tcgcaccccc attgatcgag ccctaacccc tgcccatcag gcaatccaat taagctcgca 5280
   ttgtctgcct tgtttagttt ggctcctgcc cgtttcggcg tccacttgca caaacacaaa 5340
   caagcattat atataaggct cgtctctccc tcccaaccac actcactttt ttgcccgtct 5400
   tcccttgcta acacaaaagt caagaacaca aacaaccacc ccaaccccct tacacacaag 5460
   acatatctac accatggagt ctggacccat gcctgctggc attcccttcc ctgagtacta 5520
   tgacttcttt atggactgga agactcccct ggccatcgct gccacctaca ctgctgccgt 5580
   cggtctcttc aaccccaagg ttggcaaggt ctcccgagtg gttgccaagt cggctaacgc 5640
   aaagcctgcc gagcgaaccc agtccggagc tgccatgact gccttcgtct ttgtgcacaa 5700
   cctcattctg tgtgtctact ctggcatcac cttctactac atgtttcctg ctatggtcaa 5760
   gaacttccga acccacacac tgcacgaagc ctactgcgac acggatcagt ccctctggaa 5820
   caacgcactt ggctactggg gttacctctt ctacctgtcc aagttctacg aggtcattga 5880
   caccatcatc atcatcctga agggacgacg gtcctcgctg cttcagacct accaccatgc 5940
   tggagccatg attaccatgt ggtctggcat caactaccaa gccactccca tttggatctt 6000
   tgtggtcttc aactccttca ttcacaccat catgtactgt tactatgcct tcacctctat 6060
   cggattccat cctcctggca aaaagtacct gacttcgatg cagattactc agtttctggt 6120
   cggtatcacc attgccgtgt cctacctctt cgttcctggc tgcatccgaa cacccggtgc 6180
   tcagatggct gtctggatca acgtcggcta cctgtttccc ttgacctatc tgttcgtgga 6240
   ctttgccaag cgaacctact ccaagcgatc tgccattgcc gctcagaaaa aggctcagta 6300
   agc 6303
<210> 59
   <211> 21
   <212> DNA
   <213> Artificial Sequence
<220>
   <223> Primer pZP-GW-5-1
<400> 59
   cgacaagatg gaatgagaat g 21
<210> 60
   <211> 22
   <212> DNA
   <213> Artificial Sequence
<220>
   <223> Primer pZP-GW-5-2
<400> 60
   ctggtttttc aactacttct ac 22
<210> 61
   <211> 21
   <212> DNA
   <213> Artificial Sequence
<220>
   <223> Primer pZP-GW-5-3
<400> 61
   gtactgtcct gtgtctgttc c 21
<210> 62
   <211> 22
   <212> DNA
   <213> Artificial Sequence
<220>
   <223> Primer pZP-GW-5-4
<400> 62
   ctacatcgtc cgaaagcaca ag 22
<210> 63
   <211> 24
   <212> DNA
   <213> Artificial Sequence
<220>
   <223> Primer pZP-GW-3-1
<400> 63
   ctaccagatc gagcaccatc tctg 24
<210> 64
   <211> 21
   <212> DNA
   <213> Artificial Sequence
<220>
   <223> Primer pZP-GW-3-2
<400> 64
   ctaccaggtg gaacagctgt g 21
<210> 65
   <211> 22
   <212> DNA
   <213> Artificial Sequence
<220>
   <223> Primer pZP-GW-3-3
<400> 65
   tctgccccat gaaggtctcg tc 22
<210> 66
   <211> 22
   <212> DNA
   <213> Artificial Sequence
<220>
   <223> Primer pZP-GW-3-4
<400> 66
   cctgtcccag ttcgctcgaa tg 22
<210> 67
   <211> 44
   <212> DNA
   <213> Artificial Sequence
<220>
   <223> Genome Walker adaptor-1
<400> 67
   gtaatacgac tatagggcac gcgtggtcga cggcccgggc tggt 44
<210> 68
   <211> 8
   <212> DNA
   <213> Artificial Sequence
<220>
   <223> Genome Walker adaptor-2
<220>
   <221> misc_feature
   <222> (1)..(1)
   <223> 5' end is associated with a -P04 group
<220>
   <221> misc_feature
   <222> (8)..(8)
   <223> 3' end is associated with a -H2N group
<400> 68
   accagccc 8
<210> 69
   <211> 22
   <212> DNA
   <213> Artificial Sequence
<220>
   <223> Nested adaptor primer
<400> 69
   gtaatacgac tcactatagg gc 22
<210> 70
   <211> 36
   <212> DNA
   <213> Artificial Sequence
<220>
   <223> Primer Per10F1
<400> 70
   gatcaaccat ggggggaagt tcacatgcat tcgctg 36
<210> 71
   <211> 29
   <212> DNA
   <213> Artificial Sequence
<220>
   <223> Primer ZPGW-5-5
<400> 71
   gttatagttt tcatgtgaaa taccgagag 29
<210> 72
   <211> 37
   <212> DNA
   <213> Artificial Sequence
<220>
   <223> Primer Per10R
<400> 72
   gatcaagcgg ccgccagacc tcgtcattat ctgatag 37
<210> 73
   <211> 7222
   <212> DNA
   <213> Artificial Sequence
<220>
   <223> Plasmid pFBAIn-MOD-1
<400> 73
   catggatcca ggcctgttaa cggccattac ggcctgcagg atccgaaaaa acctcccaca 60
   cctccccctg aacctgaaac ataaaatgaa tgcaattgtt gttgttaact tgtttattgc 120
   agcttataat ggttacaaat aaagcaatag catcacaaat ttcacaaata aagcattttt 180
   ttcactgcat tctagttgtg gtttgtccaa actcatcaat gtatcttatc atgtctgcgg 240
   ccgcaagtgt ggatggggaa gtgagtgccc ggttctgtgt gcacaattgg caatccaaga 300
   tggatggatt caacacaggg atatagcgag ctacgtggtg gtgcgaggat atagcaacgg 360
   atatttatgt ttgacacttg agaatgtacg atacaagcac tgtccaagta caatactaaa 420
   catactgtac atactcatac tcgtacccgg gcaacggttt cacttgagtg cagtggctag 480
   tgctcttact cgtacagtgt gcaatactgc gtatcatagt ctttgatgta tatcgtattc 540
   attcatgtta gttgcgtacg agccggaagc ataaagtgta aagcctgggg tgcctaatga 600
   gtgagctaac tcacattaat tgcgttgcgc tcactgcccg ctttccagtc gggaaacctg 660
   tcgtgccagc tgcattaatg aatcggccaa cgcgcgggga gaggcggttt gcgtattggg 720
   cgctcttccg cttcctcgct cactgactcg ctgcgctcgg tcgttcggct gcggcgagcg 780
   gtatcagctc actcaaaggc ggtaatacgg ttatccacag aatcagggga taacgcagga 840
   aagaacatgt gagcaaaagg ccagcaaaag gccaggaacc gtaaaaaggc cgcgttgctg 900
   gcgtttttcc ataggctccg cccccctgac gagcatcaca aaaatcgacg ctcaagtcag 960
   aggtggcgaa acccgacagg actataaaga taccaggcgt ttccccctgg aagctccctc 1020
   gtgcgctctc ctgttccgac cctgccgctt accggatacc tgtccgcctt tctcccttcg 1080
   ggaagcgtgg cgctttctca tagctcacgc tgtaggtatc tcagttcggt gtaggtcgtt 1140
   cgctccaagc tgggctgtgt gcacgaaccc cccgttcagc ccgaccgctg cgccttatcc 1200
   ggtaactatc gtcttgagtc caacccggta agacacgact tatcgccact ggcagcagcc 1260
   actggtaaca ggattagcag agcgaggtat gtaggcggtg ctacagagtt cttgaagtgg 1320
   tggcctaact acggctacac tagaaggaca gtatttggta tctgcgctct gctgaagcca 1380
   gttaccttcg gaaaaagagt tggtagctct tgatccggca aacaaaccac cgctggtagc 1440
   ggtggttttt ttgtttgcaa gcagcagatt acgcgcagaa aaaaaggatc tcaagaagat 1500
   cctttgatct tttctacggg gtctgacgct cagtggaacg aaaactcacg ttaagggatt 1560
   ttggtcatga gattatcaaa aaggatcttc acctagatcc ttttaaatta aaaatgaagt 1620
   tttaaatcaa tctaaagtat atatgagtaa acttggtctg acagttacca atgcttaatc 1680
   agtgaggcac ctatctcagc gatctgtcta tttcgttcat ccatagttgc ctgactcccc 1740
   gtcgtgtaga taactacgat acgggagggc ttaccatctg gccccagtgc tgcaatgata 1800
   ccgcgagacc cacgctcacc ggctccagat ttatcagcaa taaaccagcc agccggaagg 1860
   gccgagcgca gaagtggtcc tgcaacttta tccgcctcca tccagtctat taattgttgc 1920
   cgggaagcta gagtaagtag ttcgccagtt aatagtttgc gcaacgttgt tgccattgct 1980
   acaggcatcg tggtgtcacg ctcgtcgttt ggtatggctt cattcagctc cggttcccaa 2040
   cgatcaaggc gagttacatg atcccccatg ttgtgcaaaa aagcggttag ctccttcggt 2100
   cctccgatcg ttgtcagaag taagttggcc gcagtgttat cactcatggt tatggcagca 2160
   ctgcataatt ctcttactgt catgccatcc gtaagatgct tttctgtgac tggtgagtac 2220
   tcaaccaagt cattctgaga atagtgtatg cggcgaccga gttgctcttg cccggcgtca 2280
   atacgggata ataccgcgcc acatagcaga actttaaaag tgctcatcat tggaaaacgt 2340
   tcttcggggc gaaaactctc aaggatctta ccgctgttga gatccagttc gatgtaaccc 2400
   actcgtgcac ccaactgatc ttcagcatct tttactttca ccagcgtttc tgggtgagca 2460
   aaaacaggaa ggcaaaatgc cgcaaaaaag ggaataaggg cgacacggaa atgttgaata 2520
   ctcatactct tcctttttca atattattga agcatttatc agggttattg tctcatgagc 2580
   ggatacatat ttgaatgtat ttagaaaaat aaacaaatag gggttccgcg cacatttccc 2640
   cgaaaagtgc cacctgacgc gccctgtagc ggcgcattaa gcgcggcggg tgtggtggtt 2700
   acgcgcagcg tgaccgctac acttgccagc gccctagcgc ccgctccttt cgctttcttc 2760
   ccttcctttc tcgccacgtt cgccggcttt ccccgtcaag ctctaaatcg ggggctccct 2820
   ttagggttcc gatttagtgc tttacggcac ctcgacccca aaaaacttga ttagggtgat 2880
   ggttcacgta gtgggccatc gccctgatag acggtttttc gccctttgac gttggagtcc 2940
   acgttcttta atagtggact cttgttccaa actggaacaa cactcaaccc tatctcggtc 3000
   tattcttttg atttataagg gattttgccg atttcggcct attggttaaa aaatgagctg 3060
   atttaacaaa aatttaacgc gaattttaac aaaatattaa cgcttacaat ttccattcgc 3120
   cattcaggct gcgcaactgt tgggaagggc gatcggtgcg ggcctcttcg ctattacgcc 3180
   agctggcgaa agggggatgt gctgcaaggc gattaagttg ggtaacgcca gggttttccc 3240
   agtcacgacg ttgtaaaacg acggccagtg aattgtaata cgactcacta tagggcgaat 3300
   tgggtaccgg gccccccctc gaggtcgatg gtgtcgataa gcttgatatc gaattcatgt 3360
   cacacaaacc gatcttcgcc tcaaggaaac ctaattctac atccgagaga ctgccgagat 3420
   ccagtctaca ctgattaatt ttcgggccaa taatttaaaa aaatcgtgtt atataatatt 3480
   atatgtatta tatatataca tcatgatgat actgacagtc atgtcccatt gctaaataga 3540
   cagactccat ctgccgcctc caactgatgt tctcaatatt taaggggtca tctcgcattg 3600
   tttaataata aacagactcc atctaccgcc tccaaatgat gttctcaaaa tatattgtat 3660
   gaacttattt ttattactta gtattattag acaacttact tgctttatga aaaacacttc 3720
   ctatttagga aacaatttat aatggcagtt cgttcattta acaatttatg tagaataaat   3780
   gttataaatg cgtatgggaa atcttaaata tggatagcat aaatgatatc tgcattgcct   3840
   aattcgaaat caacagcaac gaaaaaaatc ccttgtacaa cataaatagt catcgagaaa   3900
   tatcaactat caaagaacag ctattcacac gttactattg agattattat tggacgagaa   3960
   tcacacactc aactgtcttt ctctcttcta gaaatacagg tacaagtatg tactattctc   4020
   attgttcata cttctagtca tttcatccca catattcctt ggatttctct ccaatgaatg   4080
   acattctatc ttgcaaattc aacaattata ataagatata ccaaagtagc ggtatagtgg   4140
   caatcaaaaa gcttctctgg tgtgcttctc gtatttattt ttattctaat gatccattaa   4200
   aggtatatat ttatttcttg ttatataatc cttttgttta ttacatgggc tggatacata   4260
   aaggtatttt gatttaattt tttgcttaaa ttcaatcccc cctcgttcag tgtcaactgt   4320
   aatggtagga aattaccata cttttgaaga agcaaaaaaa atgaaagaaa aaaaaaatcg   4380
   tatttccagg ttagacgttc cgcagaatct agaatgcggt atgcggtaca ttgttcttcg   4440
   aacgtaaaag ttgcgctccc tgagatattg tacatttttg cttttacaag tacaagtaca   4500
   tcgtacaact atgtactact gttgatgcat ccacaacagt ttgttttgtt tttttttgtt   4560
   tttttttttt ctaatgattc attaccgcta tgtataccta cttgtacttg tagtaagccg   4620
   ggttattggc gttcaattaa tcatagactt atgaatctgc acggtgtgcg ctgcgagtta   4680
   cttttagctt atgcatgcta cttgggtgta atattgggat ctgttcggaa atcaacggat   4740
   gctcaatcga tttcgacagt aattaattaa gtcatacaca agtcagcttt cttcgagcct   4800
   catataagta taagtagttc aacgtattag cactgtaccc agcatctccg tatcgagaaa   4860
   cacaacaaca tgccccattg gacagatcat gcggatacac aggttgtgca gtatcataca   4920
   tactcgatca gacaggtcgt ctgaccatca tacaagctga acaagcgctc catacttgca   4980
   cgctctctat atacacagtt aaattacata tccatagtct aacctctaac agttaatctt   5040
   ctggtaagcc tcccagccag ccttctggta tcgcttggcc tcctcaatag gatctcggtt   5100
   ctggccgtac agacctcggc cgacaattat gatatccgtt ccggtagaca tgacatcctc   5160
   aacagttcgg tactgctgtc cgagagcgtc tcccttgtcg tcaagaccca ccccgggggt   5220
   cagaataagc cagtcctcag agtcgccctt aggtcggttc tgggcaatga agccaaccac   5280
   aaactcgggg tcggatcggg caagctcaat ggtctgcttg gagtactcgc cagtggccag   5340
   agagcccttg caagacagct cggccagcat gagcagacct ctggccagct tctcgttggg   5400
   agaggggact aggaactcct tgtactggga gttctcgtag tcagagacgt cctccttctt   5460
   ctgttcagag acagtttcct cggcaccagc tcgcaggcca gcaatgattc cggttccggg   5520
   tacaccgtgg gcgttggtga tatcggacca ctcggcgatt cggtgacacc ggtactggtg 5580
   cttgacagtg ttgccaatat ctgcgaactt tctgtcctcg aacaggaaga aaccgtgctt 5640
   aagagcaagt tccttgaggg ggagcacagt gccggcgtag gtgaagtcgt caatgatgtc 5700
   gatatgggtt ttgatcatgc acacataagg tccgacctta tcggcaagct caatgagctc 5760
   cttggtggtg gtaacatcca gagaagcaca caggttggtt ttcttggctg ccacgagctt 5820
   gagcactcga gcggcaaagg cggacttgtg gacgttagct cgagcttcgt aggagggcat 5880
   tttggtggtg aagaggagac tgaaataaat ttagtctgca gaacttttta tcggaacctt 5940
   atctggggca gtgaagtata tgttatggta atagttacga gttagttgaa cttatagata 6000
   gactggacta tacggctatc ggtccaaatt agaaagaacg tcaatggctc tctgggcgtc 6060
   gcctttgccg acaaaaatgt gatcatgatg aaagccagca atgacgttgc agctgatatt 6120
   gttgtcggcc aaccgcgccg aaaacgcagc tgtcagaccc acagcctcca acgaagaatg 6180
   tatcgtcaaa gtgatccaag cacactcata gttggagtcg tactccaaag gcggcaatga 6240
   cgagtcagac agatactcgt cgaaaacagt gtacgcagat ctactataga ggaacattta 6300
   aattgccccg gagaagacgg ccaggccgcc tagatgacaa attcaacaac tcacagctga 6360
   ctttctgcca ttgccactag gggggggcct ttttatatgg ccaagccaag ctctccacgt 6420
   cggttgggct gcacccaaca ataaatgggt agggttgcac caacaaaggg atgggatggg 6480
   gggtagaaga tacgaggata acggggctca atggcacaaa taagaacgaa tactgccatt 6540
   aagactcgtg atccagcgac tgacaccatt gcatcatcta agggcctcaa aactacctcg 6600
   gaactgctgc gctgatctgg acaccacaga ggttccgagc actttaggtt gcaccaaatg 6660
   tcccaccagg tgcaggcaga aaacgctgga acagcgtgta cagtttgtct taacaaaaag 6720
   tgagggcgct gaggtcgagc agggtggtgt gacttgttat agcctttaga gctgcgaaag 6780
   cgcgtatgga tttggctcat caggccagat tgagggtctg tggacacatg tcatgttagt 6840
   gtacttcaat cgccccctgg atatagcccc gacaataggc cgtggcctca tttttttgcc 6900
   ttccgcacat ttccattgct cggtacccac accttgcttc tcctgcactt gccaacctta 6960
   atactggttt acattgacca acatcttaca agcggggggc ttgtctaggg tatatataaa 7020
   cagtggctct cccaatcggt tgccagtctc tttttccttt tctttcccca cagattcgaa 7080
   atctaaacta cacatcacag aattccgagc cgtgagtatc cacgacaaga tcagtgtcga 7140
   gacgacgcgt tttgtgtaat gacacaatcc gaaagtcgct agcaacacac actctctaca 7200
   caaactaacc cagctctggt ac 7222
<210> 74
   <211> 8133
   <212> DNA
   <213> Artificial Sequence
<220>
   <223> Plasmid pFBAIN-Pex10
<400> 74
   ggccgcaagt gtggatgggg aagtgagtgc ccggttctgt gtgcacaatt ggcaatccaa 60
   gatggatgga ttcaacacag ggatatagcg agctacgtgg tggtgcgagg atatagcaac 120
   ggatatttat gtttgacact tgagaatgta cgatacaagc actgtccaag tacaatacta 180
   aacatactgt acatactcat actcgtaccc gggcaacggt ttcacttgag tgcagtggct 240
   agtgctctta ctcgtacagt gtgcaatact gcgtatcata gtctttgatg tatatcgtat 300
   tcattcatgt tagttgcgta cgagccggaa gcataaagtg taaagcctgg ggtgcctaat 360
   gagtgagcta actcacatta attgcgttgc gctcactgcc cgctttccag tcgggaaacc 420
   tgtcgtgcca gctgcattaa tgaatcggcc aacgcgcggg gagaggcggt ttgcgtattg 480
   ggcgctcttc cgcttcctcg ctcactgact cgctgcgctc ggtcgttcgg ctgcggcgag 540
   cggtatcagc tcactcaaag gcggtaatac ggttatccac agaatcaggg gataacgcag 600
   gaaagaacat gtgagcaaaa ggccagcaaa aggccaggaa ccgtaaaaag gccgcgttgc 660
   tggcgttttt ccataggctc cgcccccctg acgagcatca caaaaatcga cgctcaagtc 720
   agaggtggcg aaacccgaca ggactataaa gataccaggc gtttccccct ggaagctccc 780
   tcgtgcgctc tcctgttccg accctgccgc ttaccggata cctgtccgcc tttctccctt 840
   cgggaagcgt ggcgctttct catagctcac gctgtaggta tctcagttcg gtgtaggtcg 900
   ttcgctccaa gctgggctgt gtgcacgaac cccccgttca gcccgaccgc tgcgccttat 960
   ccggtaacta tcgtcttgag tccaacccgg taagacacga cttatcgcca ctggcagcag 1020
   ccactggtaa caggattagc agagcgaggt atgtaggcgg tgctacagag ttcttgaagt 1080
   ggtggcctaa ctacggctac actagaagga cagtatttgg tatctgcgct ctgctgaagc 1140
   cagttacctt cggaaaaaga gttggtagct cttgatccgg caaacaaacc accgctggta 1200
   gcggtggttt ttttgtttgc aagcagcaga ttacgcgcag aaaaaaagga tctcaagaag 1260
   atcctttgat cttttctacg gggtctgacg ctcagtggaa cgaaaactca cgttaaggga 1320
   ttttggtcat gagattatca aaaaggatct tcacctagat ccttttaaat taaaaatgaa 1380
   gttttaaatc aatctaaagt atatatgagt aaacttggtc tgacagttac caatgcttaa 1440
   tcagtgaggc acctatctca gcgatctgtc tatttcgttc atccatagtt gcctgactcc 1500
   ccgtcgtgta gataactacg atacgggagg gcttaccatc tggccccagt gctgcaatga 1560
   taccgcgaga cccacgctca ccggctccag atttatcagc aataaaccag ccagccggaa 1620
   gggccgagcg cagaagtggt cctgcaactt tatccgcctc catccagtct attaattgtt 1680
   gccgggaagc tagagtaagt agttcgccag ttaatagttt gcgcaacgtt gttgccattg 1740
   ctacaggcat cgtggtgtca cgctcgtcgt ttggtatggc ttcattcagc tccggttccc 1800
   aacgatcaag gcgagttaca tgatccccca tgttgtgcaa aaaagcggtt agctccttcg 1860
   gtcctccgat cgttgtcaga agtaagttgg ccgcagtgtt atcactcatg gttatggcag 1920
   cactgcataa ttctcttact gtcatgccat ccgtaagatg cttttctgtg actggtgagt 1980
   actcaaccaa gtcattctga gaatagtgta tgcggcgacc gagttgctct tgcccggcgt 2040
   caatacggga taataccgcg ccacatagca gaactttaaa agtgctcatc attggaaaac 2100
   gttcttcggg gcgaaaactc tcaaggatct taccgctgtt gagatccagt tcgatgtaac 2160
   ccactcgtgc acccaactga tcttcagcat cttttacttt caccagcgtt tctgggtgag 2220
   caaaaacagg aaggcaaaat gccgcaaaaa agggaataag ggcgacacgg aaatgttgaa 2280
   tactcatact cttccttttt caatattatt gaagcattta tcagggttat tgtctcatga 2340
   gcggatacat atttgaatgt atttagaaaa ataaacaaat aggggttccg cgcacatttc 2400
   cccgaaaagt gccacctgac gcgccctgta gcggcgcatt aagcgcggcg ggtgtggtgg 2460
   ttacgcgcag cgtgaccgct acacttgcca gcgccctagc gcccgctcct ttcgctttct 2520
   tcccttcctt tctcgccacg ttcgccggct ttccccgtca agctctaaat cgggggctcc 2580
   ctttagggtt ccgatttagt gctttacggc acctcgaccc caaaaaactt gattagggtg 2640
   atggttcacg tagtgggcca tcgccctgat agacggtttt tcgccctttg acgttggagt 2700
   ccacgttctt taatagtgga ctcttgttcc aaactggaac aacactcaac cctatctcgg 2760
   tctattcttt tgatttataa gggattttgc cgatttcggc ctattggtta aaaaatgagc 2820
   tgatttaaca aaaatttaac gcgaatttta acaaaatatt aacgcttaca atttccattc 2880
   gccattcagg ctgcgcaact gttgggaagg gcgatcggtg cgggcctctt cgctattacg 2940
   ccagctggcg aaagggggat gtgctgcaag gcgattaagt tgggtaacgc cagggttttc 3000
   ccagtcacga cgttgtaaaa cgacggccag tgaattgtaa tacgactcac tatagggcga 3060
   attgggtacc gggccccccc tcgaggtcga tggtgtcgat aagcttgata tcgaattcat 3120
   gtcacacaaa ccgatcttcg cctcaaggaa acctaattct acatccgaga gactgccgag 3180
   atccagtcta cactgattaa ttttcgggcc aataatttaa aaaaatcgtg ttatataata 3240
   ttatatgtat tatatatata catcatgatg atactgacag tcatgtccca ttgctaaata 3300
   gacagactcc atctgccgcc tccaactgat gttctcaata tttaaggggt catctcgcat 3360
   tgtttaataa taaacagact ccatctaccg cctccaaatg atgttctcaa aatatattgt 3420
   atgaacttat ttttattact tagtattatt agacaactta cttgctttat gaaaaacact 3480
   tcctatttag gaaacaattt ataatggcag ttcgttcatt taacaattta tgtagaataa 3540
   atgttataaa tgcgtatggg aaatcttaaa tatggatagc ataaatgata tctgcattgc 3600
   ctaattcgaa atcaacagca acgaaaaaaa tcccttgtac aacataaata gtcatcgaga 3660
   aatatcaact atcaaagaac agctattcac acgttactat tgagattatt attggacgag 3720
   aatcacacac tcaactgtct ttctctcttc tagaaataca ggtacaagta tgtactattc 3780
   tcattgttca tacttctagt catttcatcc cacatattcc ttggatttct ctccaatgaa 3840
   tgacattcta tcttgcaaat tcaacaatta taataagata taccaaagta gcggtatagt 3900
   ggcaatcaaa aagcttctct ggtgtgcttc tcgtatttat ttttattcta atgatccatt 3960
   aaaggtatat atttatttct tgttatataa tccttttgtt tattacatgg gctggataca 4020
   taaaggtatt ttgatttaat tttttgctta aattcaatcc cccctcgttc agtgtcaact 4080
   gtaatggtag gaaattacca tacttttgaa gaagcaaaaa aaatgaaaga aaaaaaaaat 4140
   cgtatttcca ggttagacgt tccgcagaat ctagaatgcg gtatgcggta cattgttctt 4200
   cgaacgtaaa agttgcgctc cctgagatat tgtacatttt tgcttttaca agtacaagta 4260
   catcgtacaa ctatgtacta ctgttgatgc atccacaaca gtttgttttg tttttttttg 4320
   tttttttttt ttctaatgat tcattaccgc tatgtatacc tacttgtact tgtagtaagc 4380
   cgggttattg gcgttcaatt aatcatagac ttatgaatct gcacggtgtg cgctgcgagt 4440
   tacttttagc ttatgcatgc tacttgggtg taatattggg atctgttcgg aaatcaacgg 4500
   atgctcaatc gatttcgaca gtaattaatt aagtcataca caagtcagct ttcttcgagc 4560
   ctcatataag tataagtagt tcaacgtatt agcactgtac ccagcatctc cgtatcgaga 4620
   aacacaacaa catgccccat tggacagatc atgcggatac acaggttgtg cagtatcata 4680
   catactcgat cagacaggtc gtctgaccat catacaagct gaacaagcgc tccatacttg 4740
   cacgctctct atatacacag ttaaattaca tatccatagt ctaacctcta acagttaatc 4800
   ttctggtaag cctcccagcc agccttctgg tatcgcttgg cctcctcaat aggatctcgg 4860
   ttctggccgt acagacctcg gccgacaatt atgatatccg ttccggtaga catgacatcc 4920
   tcaacagttc ggtactgctg tccgagagcg tctcccttgt cgtcaagacc caccccgggg 4980
   gtcagaataa gccagtcctc agagtcgccc ttaggtcggt tctgggcaat gaagccaacc 5040
   acaaactcgg ggtcggatcg ggcaagctca atggtctgct tggagtactc gccagtggcc 5100
   agagagccct tgcaagacag ctcggccagc atgagcagac ctctggccag cttctcgttg 5160
   ggagagggga ctaggaactc cttgtactgg gagttctcgt agtcagagac gtcctccttc 5220
   ttctgttcag agacagtttc ctcggcacca gctcgcaggc cagcaatgat tccggttccg 5280
   ggtacaccgt gggcgttggt gatatcggac cactcggcga ttcggtgaca ccggtactgg 5340
   tgcttgacag tgttgccaat atctgcgaac tttctgtcct cgaacaggaa gaaaccgtgc 5400
   ttaagagcaa gttccttgag ggggagcaca gtgccggcgt aggtgaagtc gtcaatgatg 5460
   tcgatatggg ttttgatcat gcacacataa ggtccgacct tatcggcaag ctcaatgagc 5520
   tccttggtgg tggtaacatc cagagaagca cacaggttgg ttttcttggc tgccacgagc 5580
   ttgagcactc gagcggcaaa ggcggacttg tggacgttag ctcgagcttc gtaggagggc 5640
   attttggtgg tgaagaggag actgaaataa atttagtctg cagaactttt tatcggaacc 5700
   ttatctgggg cagtgaagta tatgttatgg taatagttac gagttagttg aacttataga 5760
   tagactggac tatacggcta tcggtccaaa ttagaaagaa cgtcaatggc tctctgggcg 5820
   tcgcctttgc cgacaaaaat gtgatcatga tgaaagccag caatgacgtt gcagctgata 5880
   ttgttgtcgg ccaaccgcgc cgaaaacgca gctgtcagac ccacagcctc caacgaagaa 5940
   tgtatcgtca aagtgatcca agcacactca tagttggagt cgtactccaa aggcggcaat 6000
   gacgagtcag acagatactc gtcgaaaaca gtgtacgcag atctactata gaggaacatt 6060
   taaattgccc cggagaagac ggccaggccg cctagatgac aaattcaaca actcacagct 6120
   gactttctgc cattgccact aggggggggc ctttttatat ggccaagcca agctctccac 6180
   gtcggttggg ctgcacccaa caataaatgg gtagggttgc accaacaaag ggatgggatg 6240
   gggggtagaa gatacgagga taacggggct caatggcaca aataagaacg aatactgcca 6300
   ttaagactcg tgatccagcg actgacacca ttgcatcatc taagggcctc aaaactacct 6360
   cggaactgct gcgctgatct ggacaccaca gaggttccga gcactttagg ttgcaccaaa 6420
   tgtcccacca ggtgcaggca gaaaacgctg gaacagcgtg tacagtttgt cttaacaaaa 6480
   agtgagggcg ctgaggtcga gcagggtggt gtgacttgtt atagccttta gagctgcgaa 6540
   agcgcgtatg gatttggctc atcaggccag attgagggtc tgtggacaca tgtcatgtta 6600
   gtgtacttca atcgccccct ggatatagcc ccgacaatag gccgtggcct catttttttg 6660
   ccttccgcac atttccattg ctcggtaccc acaccttgct tctcctgcac ttgccaacct 6720
   taatactggt ttacattgac caacatctta caagcggggg gcttgtctag ggtatatata 6780
   aacagtggct ctcccaatcg gttgccagtc tcttttttcc tttctttccc cacagattcg 6840
   aaatctaaac tacacatcac agaattccga gccgtgagta tccacgacaa gatcagtgtc 6900
   gagacgacgc gttttgtgta atgacacaat ccgaaagtcg ctagcaacac acactctcta 6960
   cacaaactaa cccagctctg gtaccatggg gggaagttca catgcattcg ctggtgaatc 7020
   tgatctgaca ctacaactac acaccaggtc caacatgagc gacaatacga caatcaaaaa 7080
   gccgatccga cccaaaccga tccggacgga acgcctgcct tacgctgggg ccgcagaaat 7140
   catccgagcc aaccagaaag accactactt tgagtccgtg cttgaacagc atctcgtcac 7200
   gtttctgcag aaatggaagg gagtacgatt tatccaccag tacaaggagg agctggagac 7260
   ggcgtccaag tttgcatatc tcggtttgtg tacgcttgtg ggctccaaga ctctcggaga 7320
   agagtacacc aatctcatgt acactatcag agaccgaaca gctctaccgg gggtggtgag 7380
   acggtttggc tacgtgcttt ccaacactct gtttccatac ctgtttgtgc gctacatggg 7440
   caagttgcgc gccaaactga tgcgcgagta tccccatctg gtggagtacg acgaagatga 7500
   gcctgtgccc agcccggaaa catggaagga gcgggtcatc aagacgtttg tgaacaagtt 7560
   tgacaagttc acggcgctgg aggggtttac cgcgatccac ttggcgattt tctacgtcta 7620
   cggctcgtac taccagctca gtaagcggat ctggggcatg cgttatgtat ttggacaccg 7680
   actggacaag aatgagcctc gaatcggtta cgagatgctc ggtctgctga ttttcgcccg 7740
   gtttgccacg tcatttgtgc agacgggaag agagtacctc ggagcgctgc tggaaaagag 7800
   cgtggagaaa gaggcagggg agaaggaaga tgaaaaggaa gcggttgtgc cgaaaaagaa 7860
   gtcgtcaatt ccgttcattg aggatacaga aggggagacg gaagacaaga tcgatctgga 7920
   ggaccctcga cagctcaagt tcattcctga ggcgtccaga gcgtgcactc tgtgtctgtc 7980
   atacattagt gcgccggcat gtacgccatg tggacacttt ttctgttggg actgtatttc 8040
   cgaatgggtg agagagaagc ccgagtgtcc cttgtgtcgg cagggtgtga gagagcagaa 8100
   cttgttgcct atcagataat gacgaggtct ggc 8133
<210> 75
   <211> 35
   <212> DNA
   <213> Artificial Sequence
<220>
   <223> Primer PEX10-R-BsiWI
<400> 75
   gatcaacgta cgcttcagca gtaactgtat tgctc 35
<210> 76
   <211> 35
   <212> DNA
   <213> Artificial Sequence
<220>
   <223> Primer PEX10-F1-SalI
<400> 76
   gatcaagtcg acattgtaac tagtcctgga gggtc 35
<210> 77
   <211> 36
   <212> DNA
   <213> Artificial Sequence
<220>
   <223> Primer PEX10-F2-SalI
<400> 77
   gatcaagtcg acgtcttagc gtcatgtatt ctcaag 36
<210> 78
   <211> 7277
   <212> DNA
   <213> Artificial Sequence
<220>
   <223> Plasmid pEXP-MOD1
<400> 78
   catggatcca ggcctgttaa cggccattac ggcctgcagg atccgaaaaa acctcccaca      60
   cctccccctg aacctgaaac ataaaatgaa tgcaattgtt gttgttaact tgtttattgc     120
   agcttataat ggttacaaat aaagcaatag catcacaaat ttcacaaata aagcattttt     180
   ttcactgcat tctagttgtg gtttgtccaa actcatcaat gtatcttatc atgtctgcgg     240
   ccgcaagtgt ggatggggaa gtgagtgccc ggttctgtgt gcacaattgg caatccaaga     300
   tggatggatt caacacaggg atatagcgag ctacgtggtg gtgcgaggat atagcaacgg     360
   atatttatgt ttgacacttg agaatgtacg atacaagcac tgtccaagta caatactaaa     420
   catactgtac atactcatac tcgtacccgg gcaacggttt cacttgagtg cagtggctag     480
   tgctcttact cgtacagtgt gcaatactgc gtatcatagt ctttgatgta tatcgtattc     540
   attcatgtta gttgcgtacg agccggaagc ataaagtgta aagcctgggg tgcctaatga     600
   gtgagctaac tcacattaat tgcgttgcgc tcactgcccg ctttccagtc gggaaacctg     660
   tcgtgccagc tgcattaatg aatcggccaa cgcgcgggga gaggcggttt gcgtattggg     720
   cgctcttccg cttcctcgct cactgactcg ctgcgctcgg tcgttcggct gcggcgagcg     780
   gtatcagctc actcaaaggc ggtaatacgg ttatccacag aatcagggga taacgcagga     840
   aagaacatgt gagcaaaagg ccagcaaaag gccaggaacc gtaaaaaggc cgcgttgctg     900
   gcgtttttcc ataggctccg cccccctgac gagcatcaca aaaatcgacg ctcaagtcag     960
   aggtggcgaa acccgacagg actataaaga taccaggcgt ttccccctgg aagctccctc    1020
   gtgcgctctc ctgttccgac cctgccgctt accggatacc tgtccgcctt tctcccttcg    1080
   ggaagcgtgg cgctttctca tagctcacgc tgtaggtatc tcagttcggt gtaggtcgtt    1140
   cgctccaagc tgggctgtgt gcacgaaccc cccgttcagc ccgaccgctg cgccttatcc    1200
   ggtaactatc gtcttgagtc caacccggta agacacgact tatcgccact ggcagcagcc    1260
   actggtaaca ggattagcag agcgaggtat gtaggcggtg ctacagagtt cttgaagtgg 1320
   tggcctaact acggctacac tagaaggaca gtatttggta tctgcgctct gctgaagcca 1380
   gttaccttcg gaaaaagagt tggtagctct tgatccggca aacaaaccac cgctggtagc 1440
   ggtggttttt ttgtttgcaa gcagcagatt acgcgcagaa aaaaaggatc tcaagaagat 1500
   cctttgatct tttctacggg gtctgacgct cagtggaacg aaaactcacg ttaagggatt 1560
   ttggtcatga gattatcaaa aaggatcttc acctagatcc ttttaaatta aaaatgaagt 1620
   tttaaatcaa tctaaagtat atatgagtaa acttggtctg acagttacca atgcttaatc 1680
   agtgaggcac ctatctcagc gatctgtcta tttcgttcat ccatagttgc ctgactcccc 1740
   gtcgtgtaga taactacgat acgggagggc ttaccatctg gccccagtgc tgcaatgata 1800
   ccgcgagacc cacgctcacc ggctccagat ttatcagcaa taaaccagcc agccggaagg 1860
   gccgagcgca gaagtggtcc tgcaacttta tccgcctcca tccagtctat taattgttgc 1920
   cgggaagcta gagtaagtag ttcgccagtt aatagtttgc gcaacgttgt tgccattgct 1980
   acaggcatcg tggtgtcacg ctcgtcgttt ggtatggctt cattcagctc cggttcccaa 2040
   cgatcaaggc gagttacatg atcccccatg ttgtgcaaaa aagcggttag ctccttcggt 2100
   cctccgatcg ttgtcagaag taagttggcc gcagtgttat cactcatggt tatggcagca 2160
   ctgcataatt ctcttactgt catgccatcc gtaagatgct tttctgtgac tggtgagtac 2220
   tcaaccaagt cattctgaga atagtgtatg cggcgaccga gttgctcttg cccggcgtca 2280
   atacgggata ataccgcgcc acatagcaga actttaaaag tgctcatcat tggaaaacgt 2340
   tcttcggggc gaaaactctc aaggatctta ccgctgttga gatccagttc gatgtaaccc 2400
   actcgtgcac ccaactgatc ttcagcatct tttactttca ccagcgtttc tgggtgagca 2460
   aaaacaggaa ggcaaaatgc cgcaaaaaag ggaataaggg cgacacggaa atgttgaata 2520
   ctcatactct tcctttttca atattattga agcatttatc agggttattg tctcatgagc 2580
   ggatacatat ttgaatgtat ttagaaaaat aaacaaatag gggttccgcg cacatttccc 2640
   cgaaaagtgc cacctgacgc gccctgtagc ggcgcattaa gcgcggcggg tgtggtggtt 2700
   acgcgcagcg tgaccgctac acttgccagc gccctagcgc ccgctccttt cgctttcttc 2760
   ccttcctttc tcgccacgtt cgccggcttt ccccgtcaag ctctaaatcg ggggctccct 2820
   ttagggttcc gatttagtgc tttacggcac ctcgacccca aaaaacttga ttagggtgat 2880
   ggttcacgta gtgggccatc gccctgatag acggtttttc gccctttgac gttggagtcc 2940
   acgttcttta atagtggact cttgttccaa actggaacaa cactcaaccc tatctcggtc 3000
   tattcttttg atttataagg gattttgccg atttcggcct attggttaaa aaatgagctg 3060
   atttaacaaa aatttaacgc gaattttaac aaaatattaa cgcttacaat ttccattcgc 3120
   cattcaggct gcgcaactgt tgggaagggc gatcggtgcg ggcctcttcg ctattacgcc 3180
   agctggcgaa agggggatgt gctgcaaggc gattaagttg ggtaacgcca gggttttccc 3240
   agtcacgacg ttgtaaaacg acggccagtg aattgtaata cgactcacta tagggcgaat 3300
   tgggtaccgg gccccccctc gaggtcgatg gtgtcgataa gcttgatatc gaattcatgt 3360
   cacacaaacc gatcttcgcc tcaaggaaac ctaattctac atccgagaga ctgccgagat 3420
   ccagtctaca ctgattaatt ttcgggccaa taatttaaaa aaatcgtgtt atataatatt 3480
   atatgtatta tatatataca tcatgatgat actgacagtc atgtcccatt gctaaataga 3540
   cagactccat ctgccgcctc caactgatgt tctcaatatt taaggggtca tctcgcattg 3600
   tttaataata aacagactcc atctaccgcc tccaaatgat gttctcaaaa tatattgtat 3660
   gaacttattt ttattactta gtattattag acaacttact tgctttatga aaaacacttc 3720
   ctatttagga aacaatttat aatggcagtt cgttcattta acaatttatg tagaataaat 3780
   gttataaatg cgtatgggaa atcttaaata tggatagcat aaatgatatc tgcattgcct 3840
   aattcgaaat caacagcaac gaaaaaaatc ccttgtacaa cataaatagt catcgagaaa 3900
   tatcaactat caaagaacag ctattcacac gttactattg agattattat tggacgagaa 3960
   tcacacactc aactgtcttt ctctcttcta gaaatacagg tacaagtatg tactattctc 4020
   attgttcata cttctagtca tttcatccca catattcctt ggatttctct ccaatgaatg 4080
   acattctatc ttgcaaattc aacaattata ataagatata ccaaagtagc ggtatagtgg 4140
   caatcaaaaa gcttctctgg tgtgcttctc gtatttattt ttattctaat gatccattaa 4200
   aggtatatat ttatttcttg ttatataatc cttttgttta ttacatgggc tggatacata 4260
   aaggtatttt gatttaattt tttgcttaaa ttcaatcccc cctcgttcag tgtcaactgt 4320
   aatggtagga aattaccata cttttgaaga agcaaaaaaa atgaaagaaa aaaaaaatcg 4380
   tatttccagg ttagacgttc cgcagaatct agaatgcggt atgcggtaca ttgttcttcg 4440
   aacgtaaaag ttgcgctccc tgagatattg tacatttttg cttttacaag tacaagtaca 4500
   tcgtacaact atgtactact gttgatgcat ccacaacagt ttgttttgtt tttttttgtt 4560
   tttttttttt ctaatgattc attaccgcta tgtataccta cttgtacttg tagtaagccg 4620
   ggttattggc gttcaattaa tcatagactt atgaatctgc acggtgtgcg ctgcgagtta 4680
   cttttagctt atgcatgcta cttgggtgta atattgggat ctgttcggaa atcaacggat 4740
   gctcaatcga tttcgacagt aattaattaa gtcatacaca agtcagcttt cttcgagcct 4800
   catataagta taagtagttc aacgtattag cactgtaccc agcatctccg tatcgagaaa 4860
   cacaacaaca tgccccattg gacagatcat gcggatacac aggttgtgca gtatcataca 4920
   tactcgatca gacaggtcgt ctgaccatca tacaagctga acaagcgctc catacttgca 4980
   cgctctctat atacacagtt aaattacata tccatagtct aacctctaac agttaatctt 5040
   ctggtaagcc tcccagccag ccttctggta tcgcttggcc tcctcaatag gatctcggtt 5100
   ctggccgtac agacctcggc cgacaattat gatatccgtt ccggtagaca tgacatcctc 5160
   aacagttcgg tactgctgtc cgagagcgtc tcccttgtcg tcaagaccca ccccgggggt 5220
   cagaataagc cagtcctcag agtcgccctt aggtcggttc tgggcaatga agccaaccac 5280
   aaactcgggg tcggatcggg caagctcaat ggtctgcttg gagtactcgc cagtggccag 5340
   agagcccttg caagacagct cggccagcat gagcagacct ctggccagct tctcgttggg 5400
   agaggggact aggaactcct tgtactggga gttctcgtag tcagagacgt cctccttctt 5460
   ctgttcagag acagtttcct cggcaccagc tcgcaggcca gcaatgattc cggttccggg 5520
   tacaccgtgg gcgttggtga tatcggacca ctcggcgatt cggtgacacc ggtactggtg 5580
   cttgacagtg ttgccaatat ctgcgaactt tctgtcctcg aacaggaaga aaccgtgctt 5640
   aagagcaagt tccttgaggg ggagcacagt gccggcgtag gtgaagtcgt caatgatgtc 5700
   gatatgggtt ttgatcatgc acacataagg tccgacctta tcggcaagct caatgagctc 5760
   cttggtggtg gtaacatcca gagaagcaca caggttggtt ttcttggctg ccacgagctt 5820
   gagcactcga gcggcaaagg cggacttgtg gacgttagct cgagcttcgt aggagggcat 5880
   tttggtggtg aagaggagac tgaaataaat ttagtctgca gaacttttta tcggaacctt 5940
   atctggggca gtgaagtata tgttatggta atagttacga gttagttgaa cttatagata 6000
   gactggacta tacggctatc ggtccaaatt agaaagaacg tcaatggctc tctgggcgtc 6060
   gcctttgccg acaaaaatgt gatcatgatg aaagccagca atgacgttgc agctgatatt 6120
   gttgtcggcc aaccgcgccg aaaacgcagc tgtcagaccc acagcctcca acgaagaatg 6180
   tatcgtcaaa gtgatccaag cacactcata gttggagtcg tactccaaag gcggcaatga 6240
   cgagtcagac agatactcgt cgaccgtacg gggagtttgg cgcccgtttt ttcgagcccc 6300
   acacgtttcg gtgagtatga gcggcggcag attcgagcgt ttccggtttc cgcggctgga 6360
   cgagagccca tgatgggggc tcccaccacc agcaatcagg gccctgatta cacacccacc 6420
   tgtaatgtca tgctgttcat cgatggttaa tgctgctgtg tgctgtgtgt gtgtgttgtt 6480
   tggcgctcat tgttgcgtta tgcagcgtac accacaatat tggaagctta ttagcctttc 6540
   tattttttcg tttgcaaggc ttaacaacat tgctgtggag agggatgggg atatggaggc 6600
   cgctggaggg agtcggagag gcgttttgga gcggcttggc ctggcgccca gctcgcgaaa 6660
   cgcacctagg accctttggc acgccgaaat gtgccacttt tcagtctagt aacgccttac 6720
   ctacgtcatt ccatgcgtgc atgtttgcgc cttttttccc ttgcccttga tcgccacaca 6780
   gtacagtgca ctgtacagtg gaggttttgg gggggtctta gatgggagct aaaagcggcc 6840
   tagcggtaca ctagtgggat tgtatggagt ggcatggagc ctaggtggag cctgacagga 6900
   cgcacgaccg gctagcccgt gacagacgat gggtggctcc tgttgtccac cgcgtacaaa 6960
   tgtttgggcc aaagtcttgt cagccttgct tgcgaaccta attcccaatt ttgtcacttc 7020
   gcacccccat tgatcgagcc ctaacccctg cccatcaggc aatccaatta agctcgcatt 7080
   gtctgccttg tttagtttgg ctcctgcccg tttcggcgtc cacttgcaca aacacaaaca 7140
   agcattatat ataaggctcg tctctccctc ccaaccacac tcactttttt gcccgtcttc 7200
   ccttgctaac acaaaagtca agaacacaaa caaccacccc aaccccctta cacacaagac 7260
   atatctacag caatggc 7277
<210> 79
   <211> 7559
   <212> DNA
   <213> Artificial Sequence
<220>
   <223> Plasmid pPEX10-1
<400> 79
   gtacgagccg gaagcataaa gtgtaaagcc tggggtgcct aatgagtgag ctaactcaca 60
   ttaattgcgt tgcgctcact gcccgctttc cagtcgggaa acctgtcgtg ccagctgcat 120
   taatgaatcg gccaacgcgc ggggagaggc ggtttgcgta ttgggcgctc ttccgcttcc 180
   tcgctcactg actcgctgcg ctcggtcgtt cggctgcggc gagcggtatc agctcactca 240
   aaggcggtaa tacggttatc cacagaatca ggggataacg caggaaagaa catgtgagca 300
   aaaggccagc aaaaggccag gaaccgtaaa aaggccgcgt tgctggcgtt tttccatagg 360
   ctccgccccc ctgacgagca tcacaaaaat cgacgctcaa gtcagaggtg gcgaaacccg 420
   acaggactat aaagatacca ggcgtttccc cctggaagct ccctcgtgcg ctctcctgtt 480
   ccgaccctgc cgcttaccgg atacctgtcc gcctttctcc cttcgggaag cgtggcgctt 540
   tctcatagct cacgctgtag gtatctcagt tcggtgtagg tcgttcgctc caagctgggc 600
   tgtgtgcacg aaccccccgt tcagcccgac cgctgcgcct tatccggtaa ctatcgtctt 660
   gagtccaacc cggtaagaca cgacttatcg ccactggcag cagccactgg taacaggatt 720
   agcagagcga ggtatgtagg cggtgctaca gagttcttga agtggtggcc taactacggc 780
   tacactagaa ggacagtatt tggtatctgc gctctgctga agccagttac cttcggaaaa 840
   agagttggta gctcttgatc cggcaaacaa accaccgctg gtagcggtgg tttttttgtt 900
   tgcaagcagc agattacgcg cagaaaaaaa ggatctcaag aagatccttt gatcttttct 960
   acggggtctg acgctcagtg gaacgaaaac tcacgttaag ggattttggt catgagatta 1020
   tcaaaaagga tcttcaccta gatcctttta aattaaaaat gaagttttaa atcaatctaa 1080
   agtatatatg agtaaacttg gtctgacagt taccaatgct taatcagtga ggcacctatc 1140
   tcagcgatct gtctatttcg ttcatccata gttgcctgac tccccgtcgt gtagataact 1200
   acgatacggg agggcttacc atctggcccc agtgctgcaa tgataccgcg agacccacgc 1260
   tcaccggctc cagatttatc agcaataaac cagccagccg gaagggccga gcgcagaagt 1320
   ggtcctgcaa ctttatccgc ctccatccag tctattaatt gttgccggga agctagagta 1380
   agtagttcgc cagttaatag tttgcgcaac gttgttgcca ttgctacagg catcgtggtg 1440
   tcacgctcgt cgtttggtat ggcttcattc agctccggtt cccaacgatc aaggcgagtt 1500
   acatgatccc ccatgttgtg caaaaaagcg gttagctcct tcggtcctcc gatcgttgtc 1560
   agaagtaagt tggccgcagt gttatcactc atggttatgg cagcactgca taattctctt 1620
   actgtcatgc catccgtaag atgcttttct gtgactggtg agtactcaac caagtcattc 1680
   tgagaatagt gtatgcggcg accgagttgc tcttgcccgg cgtcaatacg ggataatacc 1740
   gcgccacata gcagaacttt aaaagtgctc atcattggaa aacgttcttc ggggcgaaaa 1800
   ctctcaagga tcttaccgct gttgagatcc agttcgatgt aacccactcg tgcacccaac 1860
   tgatcttcag catcttttac tttcaccagc gtttctgggt gagcaaaaac aggaaggcaa 1920
   aatgccgcaa aaaagggaat aagggcgaca cggaaatgtt gaatactcat actcttcctt 1980
   tttcaatatt attgaagcat ttatcagggt tattgtctca tgagcggata catatttgaa 2040
   tgtatttaga aaaataaaca aataggggtt ccgcgcacat ttccccgaaa agtgccacct 2100
   gacgcgccct gtagcggcgc attaagcgcg gcgggtgtgg tggttacgcg cagcgtgacc 2160
   gctacacttg ccagcgccct agcgcccgct cctttcgctt tcttcccttc ctttctcgcc 2220
   acgttcgccg gctttccccg tcaagctcta aatcgggggc tccctttagg gttccgattt 2280
   agtgctttac ggcacctcga ccccaaaaaa cttgattagg gtgatggttc acgtagtggg 2340
   ccatcgccct gatagacggt ttttcgccct ttgacgttgg agtccacgtt ctttaatagt 2400
   ggactcttgt tccaaactgg aacaacactc aaccctatct cggtctattc ttttgattta 2460
   taagggattt tgccgatttc ggcctattgg ttaaaaaatg agctgattta acaaaaattt 2520
   aacgcgaatt ttaacaaaat attaacgctt acaatttcca ttcgccattc aggctgcgca 2580
   actgttggga agggcgatcg gtgcgggcct cttcgctatt acgccagctg gcgaaagggg 2640
   gatgtgctgc aaggcgatta agttgggtaa cgccagggtt ttcccagtca cgacgttgta 2700
   aaacgacggc cagtgaattg taatacgact cactataggg cgaattgggt accgggcccc 2760
   ccctcgaggt cgatggtgtc gataagcttg atatcgaatt catgtcacac aaaccgatct 2820
   tcgcctcaag gaaacctaat tctacatccg agagactgcc gagatccagt ctacactgat 2880
   taattttcgg gccaataatt taaaaaaatc gtgttatata atattatatg tattatatat 2940
   atacatcatg atgatactga cagtcatgtc ccattgctaa atagacagac tccatctgcc 3000
   gcctccaact gatgttctca atatttaagg ggtcatctcg cattgtttaa taataaacag 3060
   actccatcta ccgcctccaa atgatgttct caaaatatat tgtatgaact tatttttatt 3120
   acttagtatt attagacaac ttacttgctt tatgaaaaac acttcctatt taggaaacaa 3180
   tttataatgg cagttcgttc atttaacaat ttatgtagaa taaatgttat aaatgcgtat 3240
   gggaaatctt aaatatggat agcataaatg atatctgcat tgcctaattc gaaatcaaca 3300
   gcaacgaaaa aaatcccttg tacaacataa atagtcatcg agaaatatca actatcaaag 3360
   aacagctatt cacacgttac tattgagatt attattggac gagaatcaca cactcaactg 3420
   tctttctctc ttctagaaat acaggtacaa gtatgtacta ttctcattgt tcatacttct 3480
   agtcatttca tcccacatat tccttggatt tctctccaat gaatgacatt ctatcttgca 3540
   aattcaacaa ttataataag atataccaaa gtagcggtat agtggcaatc aaaaagcttc 3600
   tctggtgtgc ttctcgtatt tatttttatt ctaatgatcc attaaaggta tatatttatt 3660
   tcttgttata taatcctttt gtttattaca tgggctggat acataaaggt attttgattt 3720
   aattttttgc ttaaattcaa tcccccctcg ttcagtgtca actgtaatgg taggaaatta 3780
   ccatactttt gaagaagcaa aaaaaatgaa agaaaaaaaa aatcgtattt ccaggttaga 3840
   cgttccgcag aatctagaat gcggtatgcg gtacattgtt cttcgaacgt aaaagttgcg 3900
   ctccctgaga tattgtacat ttttgctttt acaagtacaa gtacatcgta caactatgta 3960
   ctactgttga tgcatccaca acagtttgtt ttgttttttt ttgttttttt tttttctaat 4020
   gattcattac cgctatgtat acctacttgt acttgtagta agccgggtta ttggcgttca 4080
   attaatcata gacttatgaa tctgcacggt gtgcgctgcg agttactttt agcttatgca 4140
   tgctacttgg gtgtaatatt gggatctgtt cggaaatcaa cggatgctca atcgatttcg 4200
   acagtaatta attaagtcat acacaagtca gctttcttcg agcctcatat aagtataagt 4260
   agttcaacgt attagcactg tacccagcat ctccgtatcg agaaacacaa caacatgccc 4320
   cattggacag atcatgcgga tacacaggtt gtgcagtatc atacatactc gatcagacag 4380
   gtcgtctgac catcatacaa gctgaacaag cgctccatac ttgcacgctc tctatataca 4440
   cagttaaatt acatatccat agtctaacct ctaacagtta atcttctggt aagcctccca 4500
   gccagccttc tggtatcgct tggcctcctc aataggatct cggttctggc cgtacagacc 4560
   tcggccgaca attatgatat ccgttccggt agacatgaca tcctcaacag ttcggtactg 4620
   ctgtccgaga gcgtctccct tgtcgtcaag acccaccccg ggggtcagaa taagccagtc   4680
   ctcagagtcg cccttaggtc ggttctgggc aatgaagcca accacaaact cggggtcgga   4740
   tcgggcaagc tcaatggtct gcttggagta ctcgccagtg gccagagagc ccttgcaaga   4800
   cagctcggcc agcatgagca gacctctggc cagcttctcg ttgggagagg ggactaggaa   4860
   ctccttgtac tgggagttct cgtagtcaga gacgtcctcc ttcttctgtt cagagacagt   4920
   ttcctcggca ccagctcgca ggccagcaat gattccggtt ccgggtacac cgtgggcgtt   4980
   ggtgatatcg gaccactcgg cgattcggtg acaccggtac tggtgcttga cagtgttgcc   5040
   aatatctgcg aactttctgt cctcgaacag gaagaaaccg tgcttaagag caagttcctt   5100
   gagggggagc acagtgccgg cgtaggtgaa gtcgtcaatg atgtcgatat gggttttgat   5160
   catgcacaca taaggtccga ccttatcggc aagctcaatg agctccttgg tggtggtaac   5220
   atccagagaa gcacacaggt tggttttctt ggctgccacg agcttgagca ctcgagcggc   5280
   aaaggcggac ttgtggacgt tagctcgagc ttcgtaggag ggcattttgg tggtgaagag   5340
   gagactgaaa taaatttagt ctgcagaact ttttatcgga accttatctg gggcagtgaa   5400
   gtatatgtta tggtaatagt tacgagttag ttgaacttat agatagactg gactatacgg   5460
   ctatcggtcc aaattagaaa gaacgtcaat ggctctctgg gcgtcgcctt tgccgacaaa   5520
   aatgtgatca tgatgaaagc cagcaatgac gttgcagctg atattgttgt cggccaaccg   5580
   cgccgaaaac gcagctgtca gacccacagc ctccaacgaa gaatgtatcg tcaaagtgat   5640
   ccaagcacac tcatagttgg agtcgtactc caaaggcggc aatgacgagt cagacagata   5700
   ctcgtcgaca ttgtaactag tcctggaggg tcttttttat ggataacctc catgtacgat   5760
   gtatccaaga tctccacgta ctgtgttctg tttcctaagt aatacccaac aacctctcca   5820
   acaaacactt gggaagatgc acttgtgctg agatgtcaag atgttagtac tgtactggat   5880
   ggagagaata ttaataaata attgttaccc aactacatct tgtcgattga aagagatacc   5940
   cctaagacag ataggatatc tgcaacccga ggaatgaacc ccccagcacc ggcacccttt   6000
   ctattaacaa aatgccaact gaaatttgaa aagttcaact aaacttattt gacccacaaa   6060
   aactcgtcaa aagtggcggc gaaagctggc aaatgatgac atccccttgg aactatgata   6120
   tcccctcgga atcttcgtcc ccatttgcca catctacttg caacgccacg tctgcttact   6180
   aagcaaccca aatctgcctc ggctcaaaat gtggggaagt tcacatgcat tcgctggtga   6240
   atctgatctg acactacaac tacacaccag gtccaacatg agcgacaata cgacaatcaa   6300
   aaagccgatc cgacccaaac cgatccggac ggaacgcctg ccttacgctg gggccgcaga    6360
   aatcatccga gccaaccaga aagaccacta ctttgagtcc gtgcttgaac agcatctcgt   6420
   cacgtttctg cagaaatgga agggagtacg atttatccac cagtacaagg aggagctgga   6480
   gacggcgtcc aagtttgcat atctcggttt gtgtacgctt gtgggctcca agactctcgg   6540
   agaagagtac accaatctca tgtacactat cagagaccga acagctctac cgggggtggt   6600
   gagacggttt ggctacgtgc tttccaacac tctgtttcca tacctgtttg tgcgctacat   6660
   gggcaagttg cgcgccaaac tgatgcgcga gtatccccat ctggtggagt acgacgaaga   6720
   tgagcctgtg cccagcccgg aaacatggaa ggagcgggtc atcaagacgt ttgtgaacaa   6780
   gtttgacaag ttcacggcgc tggaggggtt taccgcgatc cacttggcga ttttctacgt   6840
   ctacggctcg tactaccagc tcagtaagcg gatctggggc atgcgttatg tatttggaca   6900
   ccgactggac aagaatgagc ctcgaatcgg ttacgagatg ctcggtctgc tgattttcgc   6960
   ccggtttgcc acgtcatttg tgcagacggg aagagagtac ctcggagcgc tgctggaaaa   7020
   gagcgtggag aaagaggcag gggagaagga agatgaaaag gaagcggttg tgccgaaaaa   7080
   gaagtcgtca attccgttca ttgaggatac agaaggggag acggaagaca agatcgatct   7140
   ggaggaccct cgacagctca agttcattcc tgaggcgtcc agagcgtgca ctctgtgtct   7200
   gtcatacatt agtgcgccgg catgtacgcc atgtggacac tttttctgtt gggactgtat   7260
   ttccgaatgg gtgagagaga agcccgagtg tcccttgtgt cggcagggtg tgagagagca   7320
   gaacttgttg cctatcagat aatgacgagg tctggatgga aggactagtc agcgagacac   7380
   agagcatcag ggaccagaca cgaccaattc aatcgacaac actgtgctgc atagcagtgc   7440
   acagaggtcc tgggcatgaa tatattttag cattggagat atgagtggta gagcgtatac   7500
   agtattaatt gtggaggtat ctcgtcgcat tgatagagca atacagttac tgctgaagc    7559
<210> 80
   <211> 8051
   <212> DNA
   <213> Artificial Sequence
<220>
   <223> Plasmid pPEX10-2
<400> 80
   gtacgagccg gaagcataaa gtgtaaagcc tggggtgcct aatgagtgag ctaactcaca     60
   ttaattgcgt tgcgctcact gcccgctttc cagtcgggaa acctgtcgtg ccagctgcat    120
   taatgaatcg gccaacgcgc ggggagaggc ggtttgcgta ttgggcgctc ttccgcttcc    180
   tcgctcactg actcgctgcg ctcggtcgtt cggctgcggc gagcggtatc agctcactca    240
   aaggcggtaa tacggttatc cacagaatca ggggataacg caggaaagaa catgtgagca    300
   aaaggccagc aaaaggccag gaaccgtaaa aaggccgcgt tgctggcgtt tttccatagg    360
   ctccgccccc ctgacgagca tcacaaaaat cgacgctcaa gtcagaggtg gcgaaacccg    420
   acaggactat aaagatacca ggcgtttccc cctggaagct ccctcgtgcg ctctcctgtt 480
   ccgaccctgc cgcttaccgg atacctgtcc gcctttctcc cttcgggaag cgtggcgctt 540
   tctcatagct cacgctgtag gtatctcagt tcggtgtagg tcgttcgctc caagctgggc 600
   tgtgtgcacg aaccccccgt tcagcccgac cgctgcgcct tatccggtaa ctatcgtctt 660
   gagtccaacc cggtaagaca cgacttatcg ccactggcag cagccactgg taacaggatt 720
   agcagagcga ggtatgtagg cggtgctaca gagttcttga agtggtggcc taactacggc 780
   tacactagaa ggacagtatt tggtatctgc gctctgctga agccagttac cttcggaaaa 840
   agagttggta gctcttgatc cggcaaacaa accaccgctg gtagcggtgg tttttttgtt 900
   tgcaagcagc agattacgcg cagaaaaaaa ggatctcaag aagatccttt gatcttttct 960
   acggggtctg acgctcagtg gaacgaaaac tcacgttaag ggattttggt catgagatta 1020
   tcaaaaagga tcttcaccta gatcctttta aattaaaaat gaagttttaa atcaatctaa 1080
   agtatatatg agtaaacttg gtctgacagt taccaatgct taatcagtga ggcacctatc 1140
   tcagcgatct gtctatttcg ttcatccata gttgcctgac tccccgtcgt gtagataact 1200
   acgatacggg agggcttacc atctggcccc agtgctgcaa tgataccgcg agacccacgc 1260
   tcaccggctc cagatttatc agcaataaac cagccagccg gaagggccga gcgcagaagt 1320
   ggtcctgcaa ctttatccgc ctccatccag tctattaatt gttgccggga agctagagta 1380
   agtagttcgc cagttaatag tttgcgcaac gttgttgcca ttgctacagg catcgtggtg 1440
   tcacgctcgt cgtttggtat ggcttcattc agctccggtt cccaacgatc aaggcgagtt 1500
   acatgatccc ccatgttgtg caaaaaagcg gttagctcct tcggtcctcc gatcgttgtc 1560
   agaagtaagt tggccgcagt gttatcactc atggttatgg cagcactgca taattctctt 1620
   actgtcatgc catccgtaag atgcttttct gtgactggtg agtactcaac caagtcattc 1680
   tgagaatagt gtatgcggcg accgagttgc tcttgcccgg cgtcaatacg ggataatacc 1740
   gcgccacata gcagaacttt aaaagtgctc atcattggaa aacgttcttc ggggcgaaaa 1800
   ctctcaagga tcttaccgct gttgagatcc agttcgatgt aacccactcg tgcacccaac 1860
   tgatcttcag catcttttac tttcaccagc gtttctgggt gagcaaaaac aggaaggcaa 1920
   aatgccgcaa aaaagggaat aagggcgaca cggaaatgtt gaatactcat actcttcctt 1980
   tttcaatatt attgaagcat ttatcagggt tattgtctca tgagcggata catatttgaa 2040
   tgtatttaga aaaataaaca aataggggtt ccgcgcacat ttccccgaaa agtgccacct 2100
   gacgcgccct gtagcggcgc attaagcgcg gcgggtgtgg tggttacgcg cagcgtgacc 2160
   gctacacttg ccagcgccct agcgcccgct cctttcgctt tcttcccttc ctttctcgcc 2220
   acgttcgccg gctttccccg tcaagctcta aatcgggggc tccctttagg gttccgattt 2280
   agtgctttac ggcacctcga ccccaaaaaa cttgattagg gtgatggttc acgtagtggg 2340
   ccatcgccct gatagacggt ttttcgccct ttgacgttgg agtccacgtt ctttaatagt 2400
   ggactcttgt tccaaactgg aacaacactc aaccctatct cggtctattc ttttgattta 2460
   taagggattt tgccgatttc ggcctattgg ttaaaaaatg agctgattta acaaaaattt 2520
   aacgcgaatt ttaacaaaat attaacgctt acaatttcca ttcgccattc aggctgcgca 2580
   actgttggga agggcgatcg gtgcgggcct cttcgctatt acgccagctg gcgaaagggg 2640
   gatgtgctgc aaggcgatta agttgggtaa cgccagggtt ttcccagtca cgacgttgta 2700
   aaacgacggc cagtgaattg taatacgact cactataggg cgaattgggt accgggcccc 2760
   ccctcgaggt cgatggtgtc gataagcttg atatcgaatt catgtcacac aaaccgatct 2820
   tcgcctcaag gaaacctaat tctacatccg agagactgcc gagatccagt ctacactgat 2880
   taattttcgg gccaataatt taaaaaaatc gtgttatata atattatatg tattatatat 2940
   atacatcatg atgatactga cagtcatgtc ccattgctaa atagacagac tccatctgcc 3000
   gcctccaact gatgttctca atatttaagg ggtcatctcg cattgtttaa taataaacag 3060
   actccatcta ccgcctccaa atgatgttct caaaatatat tgtatgaact tatttttatt 3120
   acttagtatt attagacaac ttacttgctt tatgaaaaac acttcctatt taggaaacaa 3180
   tttataatgg cagttcgttc atttaacaat ttatgtagaa taaatgttat aaatgcgtat 3240
   gggaaatctt aaatatggat agcataaatg atatctgcat tgcctaattc gaaatcaaca 3300
   gcaacgaaaa aaatcccttg tacaacataa atagtcatcg agaaatatca actatcaaag 3360
   aacagctatt cacacgttac tattgagatt attattggac gagaatcaca cactcaactg 3420
   tctttctctc ttctagaaat acaggtacaa gtatgtacta ttctcattgt tcatacttct 3480
   agtcatttca tcccacatat tccttggatt tctctccaat gaatgacatt ctatcttgca 3540
   aattcaacaa ttataataag atataccaaa gtagcggtat agtggcaatc aaaaagcttc 3600
   tctggtgtgc ttctcgtatt tatttttatt ctaatgatcc attaaaggta tatatttatt 3660
   tcttgttata taatcctttt gtttattaca tgggctggat acataaaggt attttgattt 3720
   aattttttgc ttaaattcaa tcccccctcg ttcagtgtca actgtaatgg taggaaatta 3780
   ccatactttt gaagaagcaa aaaaaatgaa agaaaaaaaa aatcgtattt ccaggttaga 3840
   cgttccgcag aatctagaat gcggtatgcg gtacattgtt cttcgaacgt aaaagttgcg 3900
   ctccctgaga tattgtacat ttttgctttt acaagtacaa gtacatcgta caactatgta 3960
   ctactgttga tgcatccaca acagtttgtt ttgttttttt ttgttttttt tttttctaat 4020
   gattcattac cgctatgtat acctacttgt acttgtagta agccgggtta ttggcgttca 4080
   attaatcata gacttatgaa tctgcacggt gtgcgctgcg agttactttt agcttatgca 4140
   tgctacttgg gtgtaatatt gggatctgtt cggaaatcaa cggatgctca atcgatttcg 4200
   acagtaatta attaagtcat acacaagtca gctttcttcg agcctcatat aagtataagt 4260
   agttcaacgt attagcactg tacccagcat ctccgtatcg agaaacacaa caacatgccc 4320
   cattggacag atcatgcgga tacacaggtt gtgcagtatc atacatactc gatcagacag 4380
   gtcgtctgac catcatacaa gctgaacaag cgctccatac ttgcacgctc tctatataca 4440
   cagttaaatt acatatccat agtctaacct ctaacagtta atcttctggt aagcctccca 4500
   gccagccttc tggtatcgct tggcctcctc aataggatct cggttctggc cgtacagacc 4560
   tcggccgaca attatgatat ccgttccggt agacatgaca tcctcaacag ttcggtactg 4620
   ctgtccgaga gcgtctccct tgtcgtcaag acccaccccg ggggtcagaa taagccagtc 4680
   ctcagagtcg cccttaggtc ggttctgggc aatgaagcca accacaaact cggggtcgga 4740
   tcgggcaagc tcaatggtct gcttggagta ctcgccagtg gccagagagc ccttgcaaga 4800
   cagctcggcc agcatgagca gacctctggc cagcttctcg ttgggagagg ggactaggaa 4860
   ctccttgtac tgggagttct cgtagtcaga gacgtcctcc ttcttctgtt cagagacagt 4920
   ttcctcggca ccagctcgca ggccagcaat gattccggtt ccgggtacac cgtgggcgtt 4980
   ggtgatatcg gaccactcgg cgattcggtg acaccggtac tggtgcttga cagtgttgcc 5040
   aatatctgcg aactttctgt cctcgaacag gaagaaaccg tgcttaagag caagttcctt 5100
   gagggggagc acagtgccgg cgtaggtgaa gtcgtcaatg atgtcgatat gggttttgat 5160
   catgcacaca taaggtccga ccttatcggc aagctcaatg agctccttgg tggtggtaac 5220
   atccagagaa gcacacaggt tggttttctt ggctgccacg agcttgagca ctcgagcggc 5280
   aaaggcggac ttgtggacgt tagctcgagc ttcgtaggag ggcattttgg tggtgaagag 5340
   gagactgaaa taaatttagt ctgcagaact ttttatcgga accttatctg gggcagtgaa 5400
   gtatatgtta tggtaatagt tacgagttag ttgaacttat agatagactg gactatacgg 5460
   ctatcggtcc aaattagaaa gaacgtcaat ggctctctgg gcgtcgcctt tgccgacaaa 5520
   aatgtgatca tgatgaaagc cagcaatgac gttgcagctg atattgttgt cggccaaccg 5580
   cgccgaaaac gcagctgtca gacccacagc ctccaacgaa gaatgtatcg tcaaagtgat 5640
   ccaagcacac tcatagttgg agtcgtactc caaaggcggc aatgacgagt cagacagata 5700
   ctcgtcgacg tcttagcgtc atgtattctc aagcttagtc agagagaagg actatggagg 5760
   agaaggggag aattgagaag ggtatttgaa gggactttga aggtcgcgtg gaagaggtac 5820
   ttgaagaggt atttgaaggt cacgtggaag aggtatttga agatcacgtg gaagaagtac 5880
   ttgttttaca gagaatatcg gggtgatttt gacagtggga ttgtctccca agtcctaatc 5940
   gtttgacatg ggagcagtga aaagtcgggc taaaaaaggg aatatcggaa atcggaaaga 6000
   cggaaagaat tactggactc atgtttagta gatctgagca cttcaaattt gaaaatatct 6060
   cttcaaacag cagatcggtt ggtcgtggag gtaccatcaa gggtaaaatc aaggctatca 6120
   tcaagggcca tatatcgcaa gtttggggga agataatatg ttcatagtga atcagggttg 6180
   tggatttcct catctaacgg cattgtaact agtcctggag ggtctttttt atggataacc 6240
   tccatgtacg atgtatccaa gatctccacg tactgtgttc tgtttcctaa gtaataccca 6300
   acaacctctc caacaaacac ttgggaagat gcacttgtgc tgagatgtca agatgttagt 6360
   actgtactgg atggagagaa tattaataaa taattgttac ccaactacat cttgtcgatt 6420
   gaaagagata cccctaagac agataggata tctgcaaccc gaggaatgaa ccccccagca 6480
   ccggcaccct ttctattaac aaaatgccaa ctgaaatttg aaaagttcaa ctaaacttat 6540
   ttgacccaca aaaactcgtc aaaagtggcg gcgaaagctg gcaaatgatg acatcccctt 6600
   ggaactatga tatcccctcg gaatcttcgt ccccatttgc cacatctact tgcaacgcca 6660
   cgtctgctta ctaagcaacc caaatctgcc tcggctcaaa atgtggggaa gttcacatgc 6720
   attcgctggt gaatctgatc tgacactaca actacacacc aggtccaaca tgagcgacaa 6780
   tacgacaatc aaaaagccga tccgacccaa accgatccgg acggaacgcc tgccttacgc 6840
   tggggccgca gaaatcatcc gagccaacca gaaagaccac tactttgagt ccgtgcttga 6900
   acagcatctc gtcacgtttc tgcagaaatg gaagggagta cgatttatcc accagtacaa 6960
   ggaggagctg gagacggcgt ccaagtttgc atatctcggt ttgtgtacgc ttgtgggctc 7020
   caagactctc ggagaagagt acaccaatct catgtacact atcagagacc gaacagctct 7080
   accgggggtg gtgagacggt ttggctacgt gctttccaac actctgtttc catacctgtt 7140
   tgtgcgctac atgggcaagt tgcgcgccaa actgatgcgc gagtatcccc atctggtgga 7200
   gtacgacgaa gatgagcctg tgcccagccc ggaaacatgg aaggagcggg tcatcaagac 7260
   gtttgtgaac aagtttgaca agttcacggc gctggagggg tttaccgcga tccacttggc 7320
   gattttctac gtctacggct cgtactacca gctcagtaag cggatctggg gcatgcgtta 7380
   tgtatttgga caccgactgg acaagaatga gcctcgaatc ggttacgaga tgctcggtct 7440
   gctgattttc gcccggtttg ccacgtcatt tgtgcagacg ggaagagagt acctcggagc 7500
   gctgctggaa aagagcgtgg agaaagaggc aggggagaag gaagatgaaa aggaagcggt 7560
   tgtgccgaaa aagaagtcgt caattccgtt cattgaggat acagaagggg agacggaaga 7620
   caagatcgat ctggaggacc ctcgacagct caagttcatt cctgaggcgt ccagagcgtg 7680
   cactctgtgt ctgtcataca ttagtgcgcc ggcatgtacg ccatgtggac actttttctg 7740
   ttgggactgt atttccgaat gggtgagaga gaagcccgag tgtcccttgt gtcggcaggg    7800
   tgtgagagag cagaacttgt tgcctatcag ataatgacga ggtctggatg gaaggactag    7860
   tcagcgagac acagagcatc agggaccaga cacgaccaat tcaatcgaca acactgtgct    7920
   gcatagcagt gcacagaggt cctgggcatg aatatatttt agcattggag atatgagtgg    7980
   tagagcgtat acagtattaa ttgtggaggt atctcgtcgc attgatagag caatacagtt    8040
   actgctgaag c                                                         8051
<210> 81
   <211> 15877
   <212> DNA
   <213> Artificial Sequence
<220>
   <223> Plasmid pZKL1-2SP98C
<400> 81
   aaatgatgtc gacgcagtag gatgtcctgc acgggtcttt ttgtggggtg tggagaaagg      60
   ggtgcttgga tcgatggaag ccggtagaac cgggctgctt gtgcttggag atggaagccg     120
   gtagaaccgg gctgcttggg gggatttggg gccgctgggc tccaaagagg ggtaggcatt     180
   tcgttggggt tacgtaattg cggcatttgg gtcctgcgcg catgtcccat tggtcagaat     240
   tagtccggat aggagactta tcagccaatc acagcgccgg atccacctgt aggttgggtt     300
   gggtgggagc acccctccac agagtagagt caaacagcag cagcaacatg atagttgggg     360
   gtgtgcgtgt taaaggaaaa aaaagaagct tgggttatat tcccgctcta tttagaggtt     420
   gcgggataga cgccgacgga gggcaatggc gctatggaac cttgcggata tccatacgcc     480
   gcggcggact gcgtccgaac cagctccagc agcgtttttt ccgggccatt gagccgactg     540
   cgaccccgcc aacgtgtctt ggcccacgca ctcatgtcat gttggtgttg ggaggccact     600
   ttttaagtag cacaaggcac ctagctcgca gcaaggtgtc cgaaccaaag aagcggctgc     660
   agtggtgcaa acggggcgga aacggcggga aaaagccacg ggggcacgaa ttgaggcacg     720
   ccctcgaatt tgagacgagt cacggcccca ttcgcccgcg caatggctcg ccaacgcccg     780
   gtcttttgca ccacatcagg ttaccccaag ccaaaccttt gtgttaaaaa gcttaacata     840
   ttataccgaa cgtaggtttg ggcgggcttg ctccgtctgt ccaaggcaac atttatataa     900
   gggtctgcat cgccggctca attgaatctt ttttcttctt ctcttctcta tattcattct     960
   tgaattaaac acacatcaac catgggcgta ttcattaaac aggagcagct tccggctctc    1020
   aagaagtaca agtactccgc cgaggatcac tcgttcatct ccaacaacat tctgcgcccc    1080
   ttctggcgac agtttgtcaa aatcttccct ctgtggatgg cccccaacat ggtgactctg    1140
   ctgggcttct tctttgtcat tgtgaacttc atcaccatgc tcattgttga tcccacccac 1200
   gaccgcgagc ctcccagatg ggtctacctc acctacgctc tgggtctgtt cctttaccag 1260
   acatttgatg cctgtgacgg atcccatgcc cgacgaactg gccagagtgg accccttgga 1320
   gagctgtttg accactgtgt cgacgccatg aatacctctc tgattctcac ggtggtggtg 1380
   tccaccaccc atatgggata taacatgaag ctactgattg tgcagattgc cgctctcgga 1440
   aacttctacc tgtcgacctg ggagacctac cataccggaa ctctgtacct ttctggcttc 1500
   tctggtcctg ttgaaggtat cttgattctg gtggctcttt tcgtcctcac cttcttcact 1560
   ggtcccaacg tgtacgctct gaccgtctac gaggctcttc ccgagtccat cacttcgctg 1620
   ctgcctgcca gcttcctgga cgtcaccatc acccagatct acattggatt cggagtgctg 1680
   ggcatggtgt tcaacatcta cggcgcctgc ggaaacgtga tcaagtacta caacaacaag 1740
   ggcaagagcg ctctccccgc cattctcgga atcgccccct ttggcatctt ctacgtcggc 1800
   gtctttgcct gggcccatgt tgctcctctg cttctctcca agtacgccat cgtctatctg 1860
   tttgccattg gggctgcctt tgccatgcaa gtcggccaga tgattcttgc ccatctcgtg 1920
   cttgctccct ttccccactg gaacgtgctg ctcttcttcc cctttgtggg actggcagtg 1980
   cactacattg cacccgtgtt tggctgggac gccgatatcg tgtcggttaa cactctcttc 2040
   acctgttttg gcgccaccct ctccatttac gccttctttg tgcttgagat catcgacgag 2100
   atcaccaact acctcgatat ctggtgtctg cgaatcaagt accctcagga gaagaagacc 2160
   gaataagcgg ccgcatggag cgtgtgttct gagtcgatgt ttctatggga gttgtgagtg 2220
   ttagtagaca tgatgggttt atatatgatg aatgaataga tgtgattttg atttgcacga 2280
   tggaattgag aactttgtaa acgtacatgg gaatgtatga atgtgggggt tttgtgactg 2340
   gataactgac ggtcagtgga cgccgttgtt caaatatcca agagatgcga gaaactttgg 2400
   gtcaagtgaa catgtcctct ctgttcaagt aaaccatcaa ctatgggtag tatatttagt 2460
   aaggacaaga gttgagattc tttggagtcc tagaaacgta ttttcgcgtt ccaagatcaa 2520
   attagtagag taatacgggc acgggaatcc attcatagtc tcaatcctgc aggtgagtta 2580
   attaatcgag cttggcgtaa tcatggtcat agctgtttcc tgtgtgaaat tgttatccgc 2640
   tcacaattcc acacaacgta cgatagttag tagacaacaa tcagaacatc tccctcctta 2700
   tataatcaca caggccagaa cgcgctaaac taaagcgctt tggacactat gttacattgg 2760
   cattgattga actgaaacca cagtctccct cgcctgaatc gagcaatgga tgttgtcgga 2820
   agtcaacttc actagaagag cggttctatg ccttgtcaag atcatatcat aaactcactc 2880
   tgtattaccc catctataga acacttgtta tgaatgggcg gaaacattcc gctatatgca 2940
   cctttccaca ctaatgcaaa gatgtgcatc ttcaacgggt agtaagactg gttccgactt 3000
   ccgttgcatg gagagcaatg acctcgataa tgcgaacatc ccccacatat acactcttac 3060
   acaggccaat ataatctgtg catttactaa atatttaagt ctatgcacct gcttgatgaa 3120
   aagcggcacg gatggtatca tctagtttcc gccaatccaa gaaccaactg tgttggcagt 3180
   ggtgtagccc atggcacaca gaccaaagat gaaaatacag acatcggcgg ttcgagccgt 3240
   ggtgcctcga gcaacaccct tgtaatgcaa aagaggaggg taaatgtaca ccagaggcac 3300
   acatgcaaac gatccggtga gagcgacgaa ccgatcgaga tcgtcggcac ctccccatgc 3360
   aacaaaggcg gtgacaaaca caaggaagaa ccggaaaatg ttcttctgcc acttgatggt 3420
   agagttgtac ttgcctgatc gggtgaagag accattctcg atgattcgga tggcgcgcca 3480
   gctgcattaa tgaatcggcc aacgcgcggg gagaggcggt ttgcgtattg ggcgctcttc 3540
   cgcttcctcg ctcactgact cgctgcgctc ggtcgttcgg ctgcggcgag cggtatcagc 3600
   tcactcaaag gcggtaatac ggttatccac agaatcaggg gataacgcag gaaagaacat 3660
   gtgagcaaaa ggccagcaaa aggccaggaa ccgtaaaaag gccgcgttgc tggcgttttt 3720
   ccataggctc cgcccccctg acgagcatca caaaaatcga cgctcaagtc agaggtggcg 3780
   aaacccgaca ggactataaa gataccaggc gtttccccct ggaagctccc tcgtgcgctc 3840
   tcctgttccg accctgccgc ttaccggata cctgtccgcc tttctccctt cgggaagcgt 3900
   ggcgctttct catagctcac gctgtaggta tctcagttcg gtgtaggtcg ttcgctccaa 3960
   gctgggctgt gtgcacgaac cccccgttca gcccgaccgc tgcgccttat ccggtaacta 4020
   tcgtcttgag tccaacccgg taagacacga cttatcgcca ctggcagcag ccactggtaa 4080
   caggattagc agagcgaggt atgtaggcgg tgctacagag ttcttgaagt ggtggcctaa 4140
   ctacggctac actagaagaa cagtatttgg tatctgcgct ctgctgaagc cagttacctt 4200
   cggaaaaaga gttggtagct cttgatccgg caaacaaacc accgctggta gcggtggttt 4260
   ttttgtttgc aagcagcaga ttacgcgcag aaaaaaagga tctcaagaag atcctttgat 4320
   cttttctacg gggtctgacg ctcagtggaa cgaaaactca cgttaaggga ttttggtcat 4380
   gagattatca aaaaggatct tcacctagat ccttttaaat taaaaatgaa gttttaaatc 4440
   aatctaaagt atatatgagt aaacttggtc tgacagttac caatgcttaa tcagtgaggc 4500
   acctatctca gcgatctgtc tatttcgttc atccatagtt gcctgactcc ccgtcgtgta 4560
   gataactacg atacgggagg gcttaccatc tggccccagt gctgcaatga taccgcgaga 4620
   cccacgctca ccggctccag atttatcagc aataaaccag ccagccggaa gggccgagcg 4680
   cagaagtggt cctgcaactt tatccgcctc catccagtct attaattgtt gccgggaagc 4740
   tagagtaagt agttcgccag ttaatagttt gcgcaacgtt gttgccattg ctacaggcat 4800
   cgtggtgtca cgctcgtcgt ttggtatggc ttcattcagc tccggttccc aacgatcaag 4860
   gcgagttaca tgatccccca tgttgtgcaa aaaagcggtt agctccttcg gtcctccgat 4920
   cgttgtcaga agtaagttgg ccgcagtgtt atcactcatg gttatggcag cactgcataa 4980
   ttctcttact gtcatgccat ccgtaagatg cttttctgtg actggtgagt actcaaccaa 5040
   gtcattctga gaatagtgta tgcggcgacc gagttgctct tgcccggcgt caatacggga 5100
   taataccgcg ccacatagca gaactttaaa agtgctcatc attggaaaac gttcttcggg 5160
   gcgaaaactc tcaaggatct taccgctgtt gagatccagt tcgatgtaac ccactcgtgc 5220
   acccaactga tcttcagcat cttttacttt caccagcgtt tctgggtgag caaaaacagg 5280
   aaggcaaaat gccgcaaaaa agggaataag ggcgacacgg aaatgttgaa tactcatact 5340
   cttccttttt caatattatt gaagcattta tcagggttat tgtctcatga gcggatacat 5400
   atttgaatgt atttagaaaa ataaacaaat aggggttccg cgcacatttc cccgaaaagt 5460
   gccacctgat gcggtgtgaa ataccgcaca gatgcgtaag gagaaaatac cgcatcagga 5520
   aattgtaagc gttaatattt tgttaaaatt cgcgttaaat ttttgttaaa tcagctcatt 5580
   ttttaaccaa taggccgaaa tcggcaaaat cccttataaa tcaaaagaat agaccgagat 5640
   agggttgagt gttgttccag tttggaacaa gagtccacta ttaaagaacg tggactccaa 5700
   cgtcaaaggg cgaaaaaccg tctatcaggg cgatggccca ctacgtgaac catcacccta 5760
   atcaagtttt ttggggtcga ggtgccgtaa agcactaaat cggaacccta aagggagccc 5820
   ccgatttaga gcttgacggg gaaagccggc gaacgtggcg agaaaggaag ggaagaaagc 5880
   gaaaggagcg ggcgctaggg cgctggcaag tgtagcggtc acgctgcgcg taaccaccac 5940
   acccgccgcg cttaatgcgc cgctacaggg cgcgtccatt cgccattcag gctgcgcaac 6000
   tgttgggaag ggcgatcggt gcgggcctct tcgctattac gccagctggc gaaaggggga 6060
   tgtgctgcaa ggcgattaag ttgggtaacg ccagggtttt cccagtcacg acgttgtaaa 6120
   acgacggcca gtgaattgta atacgactca ctatagggcg aattgggccc gacgtcgcat 6180
   gcttagaagt gaggattaca agaagcctct ggatatcaat gatgaacgta ctcagcggct 6240
   ggtcaagcat ttcgaccgtc gaatcgacga ggtgttcacc tttgacaagc gagggttccc 6300
   aattgatcac gttctcgagt tgttcaaatc ttctctcaac atctctctgc atgaactatc 6360
   tctgttgacg aacgtgtcac ccactgttcc tcgaacgccc ttctccgagt ttggtctgaa 6420
   catcttcgat ctcaaactga cccccgcagt gatcaatagt gccatgccac tgccgatgcg 6480
   gtgcgaacat ccctggaggg attctcggag ctctacacaa tgcagattct gtcgtcgagt 6540
   actctctacc ttgctcgaat gacttattgt gctactactg cactcatgct tcgatcatgt 6600
   gccctactgc accccaaatt tggtgatctg attgagacag agtaccctct tcagctgatt 6660
   cagaagatca tcagcaacat gaatgatgtg gttgaccagg caggctgttg tagtcacgtc 6720
   cttcacttca agttcattct tcatctgctt ctgttttact ttgacaggca aatgaagaca 6780
   tggtacgact tgatggaggc caagaacgcc atttcacccc gagacaccga agtgcctgaa 6840
   atcctggctg cccccattga taacatcgga aactacggta ttccggaaag tgtatataga 6900
   acctttcccc agcttgtgtc tgtggatatg gatggtgtaa tccccttaat taactcacct 6960
   gcaggattga gactatgaat ggattcccgt gcccgtatta ctctactaat ttgatcttgg 7020
   aacgcgaaaa tacgtttcta ggactccaaa gaatctcaac tcttgtcctt actaaatata 7080
   ctacccatag ttgatggttt acttgaacag agaggacatg ttcacttgac ccaaagtttc 7140
   tcgcatctct tggatatttg aacaacggcg tccactgacc gtcagttatc cagtcacaaa 7200
   acccccacat tcatacattc ccatgtacgt ttacaaagtt ctcaattcca tcgtgcaaat 7260
   caaaatcaca tctattcatt catcatatat aaacccatca tgtctactaa cactcacaac 7320
   tccatagaaa acatcgactc agaacacacg ctccatgcgg ccgcttaggc aacgggcttg 7380
   atgacagcgg gaggagtgcc cacattgttt cggtttcgaa agaacaggac acccttgcca 7440
   gctccctcgg caccagcgga gggttcaacc cactggcaca ttcgtgcaga tcggtacatg 7500
   gctcgaatga atcctcgagg accgtcctgg acatcagctc gatagtgctt gcccatgata 7560
   ggtttgatgg cctcggtagc ttcgtccgca ttgtagaagg gaatggaaga gacgtagtga 7620
   tgcaggacgt gagtctcgat aatgccgtgg agcagatgac gtccaatgaa gcccatctct 7680
   cggtcgatgg ttgcagcggc acctcgcaca aagttccact cgtcgttggt gtagtgggga 7740
   agagtaggat ctgtgtgctg cagaaaggta atggcgacga gccagtggtt aacccacaag 7800
   tagggaacga agtaccagat ggccatgttg tagaatccga acttctgaac gagaaagtac 7860
   agagcggtgg ccataagacc aatgccaatg tcggagagca cgatgagctt ggcgtcgctg 7920
   ttctcgtaca gaggagatcg gggatcgaaa tggttaactc caccgccaag accgttgtgc 7980
   tttcccttgc ctcgaccctc tcgctgccgc tcatggtagt tgtgtccagt aacgttggta 8040
   atgagatagt tgggccaacc gaccagttgc tgaagcacaa gcatgagcag ggtgaaagca 8100
   ggagtttcct cggtaagatg ggcgagttcg tgggtcatct tgccgagtcg agtagcttgc 8160
   tgctctcggg ttcgaggaac gaagaccatg tctcgctcca tgtttccagt ggccttgtga 8220
   tgcttccggt gggagatttg ccagctgaag tagggaacaa gcagggaaga gtgaagcacc 8280
   cagccagtaa tgtcgttgat gattcgggaa tcggagaaag caccatgtcc acactcgtgg 8340
   gcaatgaccc acagtccagt accgaagagt ccctgaagaa cggtgtacac agcccacaga 8400
   ccggctcgag caggagtgga gggaatgtac tcgggtgtca caaagttgta ccagatgctg 8460
   aaagtggtag tcaggaggac aatgtctcga agaatgtagc cgtatccctt gagagcagat 8520
   cgcttgaagc agtgcttggg aatagcgttg tagatgtcct tgatggtgaa gtcgggaact 8580
   tcgaactggt tgccgtaggt atccagcatg acaccgtact cggacttggg cttggcaatg 8640
   tccacctcgg acatggaaga cagcgatgta gaggaggccg agtgtctggg agaatcggag 8700
   ggagagacgg cagcagactc cgagtcggtc acagtggtgg aagtgacggt tcgtcggagg 8760
   gcagggttct gcttgggcag agccgaggtg gaggccatgg ccattgctgt agatatgtct 8820
   tgtgtgtaag ggggttgggg tggttgtttg tgttcttgac ttttgtgtta gcaagggaag 8880
   acgggcaaaa aagtgagtgt ggttgggagg gagagacgag ccttatatat aatgcttgtt 8940
   tgtgtttgtg caagtggacg ccgaaacggg caggagccaa actaaacaag gcagacaatg 9000
   cgagcttaat tggattgcct gatgggcagg ggttagggct cgatcaatgg gggtgcgaag 9060
   tgacaaaatt gggaattagg ttcgcaagca aggctgacaa gactttggcc caaacatttg 9120
   tacgcggtgg acaacaggag ccacccatcg tctgtcacgg gctagccggt cgtgcgtcct 9180
   gtcaggctcc acctaggctc catgccactc catacaatcc cactagtgta ccgctaggcc 9240
   gcttttagct cccatctaag acccccccaa aacctccact gtacagtgca ctgtactgtg 9300
   tggcgatcaa gggcaaggga aaaaaggcgc aaacatgcac gcatggaatg acgtaggtaa 9360
   ggcgttacta gactgaaaag tggcacattt cggcgtgcca aagggtccta ggtgcgtttc 9420
   gcgagctggg cgccaggcca agccgctcca aaacgcctct ccgactccct ccagcggcct 9480
   ccatatcccc atccctctcc acagcaatgt tgttaagcct tgcaaacgaa aaaatagaaa 9540
   ggctaataag cttccaatat tgtggtgtac gctgcataac gcaacaatga gcgccaaaca 9600
   acacacacac acagcacaca gcagcattaa ccacgatgaa cagcatgaat tcctttacct 9660
   gcaggataac ttcgtataat gtatgctata cgaagttatg atctctctct tgagcttttc 9720
   cataacaagt tcttctgcct ccaggaagtc catgggtggt ttgatcatgg ttttggtgta 9780
   gtggtagtgc agtggtggta ttgtgactgg ggatgtagtt gagaataagt catacacaag 9840
   tcagctttct tcgagcctca tataagtata agtagttcaa cgtattagca ctgtacccag 9900
   catctccgta tcgagaaaca caacaacatg ccccattgga cagatcatgc ggatacacag 9960
   gttgtgcagt atcatacata ctcgatcaga caggtcgtct gaccatcata caagctgaac 10020
   aagcgctcca tacttgcacg ctctctatat acacagttaa attacatatc catagtctaa 10080
   cctctaacag ttaatcttct ggtaagcctc ccagccagcc ttctggtatc gcttggcctc 10140
   ctcaatagga tctcggttct ggccgtacag acctcggccg acaattatga tatccgttcc 10200
   ggtagacatg acatcctcaa cagttcggta ctgctgtccg agagcgtctc ccttgtcgtc 10260
   aagacccacc ccgggggtca gaataagcca gtcctcagag tcgcccttag gtcggttctg 10320
   ggcaatgaag ccaaccacaa actcggggtc ggatcgggca agctcaatgg tctgcttgga 10380
   gtactcgcca gtggccagag agcccttgca agacagctcg gccagcatga gcagacctct 10440
   ggccagcttc tcgttgggag aggggactag gaactccttg tactgggagt tctcgtagtc 10500
   agagacgtcc tccttcttct gttcagagac agtttcctcg gcaccagctc gcaggccagc 10560
   aatgattccg gttccgggta caccgtgggc gttggtgata tcggaccact cggcgattcg 10620
   gtgacaccgg tactggtgct tgacagtgtt gccaatatct gcgaactttc tgtcctcgaa 10680
   caggaagaaa ccgtgcttaa gagcaagttc cttgaggggg agcacagtgc cggcgtaggt 10740
   gaagtcgtca atgatgtcga tatgggtttt gatcatgcac acataaggtc cgaccttatc 10800
   ggcaagctca atgagctcct tggtggtggt aacatccaga gaagcacaca ggttggtttt 10860
   cttggctgcc acgagcttga gcactcgagc ggcaaaggcg gacttgtgga cgttagctcg 10920
   agcttcgtag gagggcattt tggtggtgaa gaggagactg aaataaattt agtctgcaga 10980
   actttttatc ggaaccttat ctggggcagt gaagtatatg ttatggtaat agttacgagt 11040
   tagttgaact tatagataga ctggactata cggctatcgg tccaaattag aaagaacgtc 11100
   aatggctctc tgggcgtcgc ctttgccgac aaaaatgtga tcatgatgaa agccagcaat 11160
   gacgttgcag ctgatattgt tgtcggccaa ccgcgccgaa aacgcagctg tcagacccac 11220
   agcctccaac gaagaatgta tcgtcaaagt gatccaagca cactcatagt tggagtcgta 11280
   ctccaaaggc ggcaatgacg agtcagacag atactcgtcg acgcgataac ttcgtataat 11340
   gtatgctata cgaagttatc gtacgatagt tagtagacaa caatcgatcg aggaagagga 11400
   caagcggctg cttcttaagt ttgtgacatc agtatccaag gcaccattgc aaggattcaa 11460
   ggctttgaac ccgtcatttg ccattcgtaa cgctggtaga caggttgatc ggttccctac 11520
   ggcctccacc tgtgtcaatc ttctcaagct gcctgactat caggacattg atcaacttcg 11580
   gaagaaactt ttgtatgcca ttcgatcaca tgctggtttc gatttgtctt agaggaacgc 11640
   atatacagta atcatagaga ataaacgata ttcatttatt aaagtagata gttgaggtag 11700
   aagttgtaaa gagtgataaa tagcggccgc tcactgaatc tttttggctc ccttgtgctt 11760
   tcggacgatg taggtctgca cgtagaagtt gaggaacaga cacaggacag taccaacgta 11820
   gaagtagttg aaaaaccagc caaacattct cattccatct tgtcggtagc agggaatgtt 11880
   ccggtacttc cagacgatgt agaagccaac gttgaactga atgatctgca tagaagtaat 11940
   cagggacttg ggcataggga acttgagctt gatcagtcgg gtccaatagt agccgtacat 12000
   gatccagtga atgaagccgt tgagcagcac aaagatccaa acggcttcgt ttcggtagtt 12060
   gtagaacagc cacatgtcca taggagctcc gagatggtga aagaactgca accaggtcag 12120
   aggcttgccc atgaggggca gatagaagga gtcaatgtac tcgaggaact tgctgaggta 12180
   gaacagctga gtggtgattc ggaagacatt gttgtcgaaa gccttctcgc agttgtcgga 12240
   catgacacca atggtgtaca tggcgtaggc catagagagg aaggagccca gcgagtagat 12300
   ggacatgagc aggttgtagt tggtgaacac aaacttcatt cgagactgac ccttgggtcc 12360
   gagaggacca agggtgaact tcaggatgac gaaggcgatg gagaggtaca gcacctcgca 12420
   gtgcgaggca tcagaccaga gctgagcata gtcgaccttg ggaagaacct cctggccaat 12480
   ggagacgatt tcgttcacga cctccatggt tgtgaattag ggtggtgaga atggttggtt 12540
   gtagggaaga atcaaaggcc ggtctcggga tccgtgggta tatatatata tatatatata 12600
   tacgatcctt cgttacctcc ctgttctcaa aactgtggtt tttcgttttt cgttttttgc 12660
   tttttttgat ttttttaggg ccaactaagc ttccagattt cgctaatcac ctttgtacta 12720
   attacaagaa aggaagaagc tgattagagt tgggcttttt atgcaactgt gctactcctt 12780
   atctctgata tgaaagtgta gacccaatca catcatgtca tttagagttg gtaatactgg 12840
   gaggatagat aaggcacgaa aacgagccat agcagacatg ctgggtgtag ccaagcagaa 12900
   gaaagtagat gggagccaat tgacgagcga gggagctacg ccaatccgac atacgacacg 12960
   ctgagatcgt cttggccggg gggtacctac agatgtccaa gggtaagtgc ttgactgtaa 13020
   ttgtatgtct gaggacaaat atgtagtcag ccgtataaag tcataccagg caccagtgcc 13080
   atcatcgaac cactaactct ctatgataca tgcctccggt attattgtac catgcgtcgc 13140
   tttgttacat acgtatcttg cctttttctc tcagaaactc cagactttgg ctattggtcg 13200
   agataagccc ggaccatagt gagtctttca cactctgttt aaacaccact aaaaccccac 13260
   aaaatatatc ttaccgaata tacagatcta ctatagagga acaattgccc cggagaagac 13320
   ggccaggccg cctagatgac aaattcaaca actcacagct gactttctgc cattgccact 13380
   aggggggggc ctttttatat ggccaagcca agctctccac gtcggttggg ctgcacccaa 13440
   caataaatgg gtagggttgc accaacaaag ggatgggatg gggggtagaa gatacgagga 13500
   taacggggct caatggcaca aataagaacg aatactgcca ttaagactcg tgatccagcg 13560
   actgacacca ttgcatcatc taagggcctc aaaactacct cggaactgct gcgctgatct 13620
   ggacaccaca gaggttccga gcactttagg ttgcaccaaa tgtcccacca ggtgcaggca 13680
   gaaaacgctg gaacagcgtg tacagtttgt cttaacaaaa agtgagggcg ctgaggtcga 13740
   gcagggtggt gtgacttgtt atagccttta gagctgcgaa agcgcgtatg gatttggctc 13800
   atcaggccag attgagggtc tgtggacaca tgtcatgtta gtgtacttca atcgccccct 13860
   ggatatagcc ccgacaatag gccgtggcct catttttttg ccttccgcac atttccattg 13920
   ctcggtaccc acaccttgct tctcctgcac ttgccaacct taatactggt ttacattgac 13980
   caacatctta caagcggggg gcttgtctag ggtatatata aacagtggct ctcccaatcg 14040
   gttgccagtc tcttttttcc tttctttccc cacagattcg aaatctaaac tacacatcac 14100
   acaatgcctg ttactgacgt ccttaagcga aagtccggtg tcatcgtcgg cgacgatgtc 14160
   cgagccgtga gtatccacga caagatcagt gtcgagacga cgcgttttgt gtaatgacac 14220
   aatccgaaag tcgctagcaa cacacactct ctacacaaac taacccagct ctccatggtg 14280
   aaggcttctc gacaggctct gcccctcgtc atcgacggaa aggtgtacga cgtctccgct 14340
   tgggtgaact tccaccctgg tggagctgaa atcattgaga actaccaggg acgagatgct 14400
   actgacgcct tcatggttat gcactctcag gaagccttcg acaagctcaa gcgaatgccc 14460
   aagatcaacc aggcttccga gctgcctccc caggctgccg tcaacgaagc tcaggaggat 14520
   ttccgaaagc tccgagaaga gctgatcgcc actggcatgt ttgacgcctc tcccctctgg 14580
   tactcgtaca agatcttgac caccctgggt cttggcgtgc ttgccttctt catgctggtc 14640
   cagtaccacc tgtacttcat tggtgctctc gtgctcggta tgcactacca gcaaatggga 14700
   tggctgtctc atgacatctg ccaccaccag accttcaaga accgaaactg gaataacgtc 14760
   ctgggtctgg tctttggcaa cggactccag ggcttctccg tgacctggtg gaaggacaga 14820
   cacaacgccc atcattctgc taccaacgtt cagggtcacg atcccgacat tgataacctg 14880
   cctctgctcg cctggtccga ggacgatgtc actcgagctt ctcccatctc ccgaaagctc 14940
   attcagttcc aacagtacta tttcctggtc atctgtattc tcctgcgatt catctggtgt 15000
   ttccagtctg tgctgaccgt tcgatccctc aaggaccgag acaaccagtt ctaccgatct 15060
   cagtacaaga aagaggccat tggactcgct ctgcactgga ctctcaagac cctgttccac 15120
   ctcttcttta tgccctccat cctgacctcg atgctggtgt tctttgtttc cgagctcgtc 15180
   ggtggcttcg gaattgccat cgtggtcttc atgaaccact accctctgga gaagatcggt 15240
   gattccgtct gggacggaca tggcttctct gtgggtcaga tccatgagac catgaacatt 15300
   cgacgaggca tcattactga ctggttcttt ggaggcctga actaccagat cgagcaccat 15360
   ctctggccca ccctgcctcg acacaacctc actgccgttt cctaccaggt ggaacagctg 15420
   tgccagaagc acaacctccc ctaccgaaac cctctgcccc atgaaggtct cgtcatcctg 15480
   ctccgatacc tgtcccagtt cgctcgaatg gccgagaagc agcccggtgc caaggctcag 15540
   taagcggccg catgagaaga taaatatata aatacattga gatattaaat gcgctagatt 15600
   agagagcctc atactgctcg gagagaagcc aagacgagta ctcaaagggg attacaccat 15660
   ccatatccac agacacaagc tggggaaagg ttctatatac actttccgga ataccgtagt 15720
   ttccgatgtt atcaatgggg gcagccagga tttcaggcac ttcggtgtct cggggtgaaa 15780
   tggcgttctt ggcctccatc aagtcgtacc atgtcttcat ttgcctgtca aagtaaaaca  15840
   gaagcagatg aagaatgaac ttgaagtgaa ggaattt                           15877
<210> 82
   <211> 1185
   <212> DNA
   <213> Yarrowia lipolytica
<220>
   <221> CDS
   <222> (1)..(1185)
   <223> diacylglycerol cholinephosphotransferase (YlCPT1)
<300>
   <302> HIGH EICOSAPENTAENOIC ACID PRODUCING STRAINS OF
<310> WO 2006/052870
   <311> 2005-11-03
   <312> 2006-05-18
   <313> (1)..(1185)
<300>
   <302> HIGH EICOSAPENTAENOIC ACID PRODUCING STRAINS OF
<310> US 2006-0115881-A1
   <311> 2005-11-02
   <312> 2006-06-01
   <313> (1)..(1185)
<400> 82
   atg ggc gta ttc att aaa cag gag cag ctt ccg gct ctc aag aag tac      48
   Met Gly Val Phe Ile Lys Gln Glu Gln Leu Pro Ala Leu Lys Lys Tyr
   1               5                   10                  15
   aag tac tcc gcc gag gat cac tcg ttc atc tcc aac aac att ctg cgc      96
   Lys Tyr Ser Ala Glu Asp His Ser Phe Ile Ser Asn Asn Ile Leu Arg
               20                  25                  30
   ccc ttc tgg cga cag ttt gtc aaa atc ttc cct ctg tgg atg gcc ccc     144
   Pro Phe Trp Arg Gln Phe Val Lys Ile Phe Pro Leu Trp Met Ala Pro
           35                  40                  45
   aac atg gtg act ctg ttg ggc ttc ttc ttt gtc att gtg aac ttc atc     192
   Asn Met Val Thr Leu Leu Gly Phe Phe Phe Val Ile Val Asn Phe Ile
       50                  55                  60
   acc atg ctc att gtt gat ccc acc cac gac cgc gag cct ccc aga tgg     240
   Thr Met Leu Ile Val Asp Pro Thr His Asp Arg Glu Pro Pro Arg Trp
   65                  70                  75                  80
   gtc tac ctc acc tac gct ctg ggt ctg ttc ctt tac cag aca ttt gat     288
   Val Tyr Leu Thr Tyr Ala Leu Gly Leu Phe Leu Tyr Gln Thr Phe Asp
                   85                  90                  95
   gcc tgt gac gga tcc cat gcc cga cga act ggc cag agt gga ccc ctt     336
   Ala Cys Asp Gly Ser His Ala Arg Arg Thr Gly Gln Ser Gly Pro Leu
               100                 105                 110
   gga gag ctg ttt gac cac tgt gtc gac gcc atg aat acc tct ctg att     384
   Gly Glu Leu Phe Asp His Cys Val Asp Ala Met Asn Thr Ser Leu Ile
               115                 120                 125
   ctc acg gtg gtg gtg tcc acc acc cat atg gga tat aac atg aag ctg      432
   Leu Thr Val Val Val Ser Thr Thr His Met Gly Tyr Asn Met Lys Leu
       130                 135                 140
   ctg att gtg cag att gcc gct ctc gga aac ttc tac ctg tcg acc tgg      480
   Leu Ile Val Gln Ile Ala Ala Leu Gly Asn Phe Tyr Leu Ser Thr Trp
   145                 150                 155                 160
   gag acc tac cat acc gga act ctg tac ctt tct ggc ttc tct ggt cct      528
   Glu Thr Tyr His Thr Gly Thr Leu Tyr Leu Ser Gly Phe Ser Gly Pro
                   165                 170                 175
   gtt gaa ggt atc ttg att ctg gtg gct ctt ttc gtc ctc acc ttc ttc      576
   Val Glu Gly Ile Leu Ile Leu Val Ala Leu Phe Val Leu Thr Phe Phe
               180                 185                 190
   act ggt ccc aac gtg tac gct ctg acc gtc tac gag gct ctt ccc gaa      624
   Thr Gly Pro Asn Val Tyr Ala Leu Thr Val Tyr Glu Ala Leu Pro Glu
           195                 200                 205
   tcc atc act tcg ctg ctg cct gcc agc ttc ctg gac gtc acc atc acc      672
   Ser Ile Thr Ser Leu Leu Pro Ala Ser Phe Leu Asp Val Thr Ile Thr
       210                 215                 220
   cag atc tac att gga ttc gga gtg ctg ggc atg gtg ttc aac atc tac      720
   Gln Ile Tyr Ile Gly Phe Gly Val Leu Gly Met Val Phe Asn Ile Tyr
   225                 230                 235                 240
   ggc gcc tgc gga aac gtg atc aag tac tac aac aac aag ggc aag agc      768
   Gly Ala Cys Gly Asn Val Ile Lys Tyr Tyr Asn Asn Lys Gly Lys Ser
                   245                 250                 255
   gct ctc ccc gcc att ctc gga atc gcc ccc ttt ggc atc ttc tac gtc      816
   Ala Leu Pro Ala Ile Leu Gly Ile Ala Pro Phe Gly Ile Phe Tyr Val
               260                 265                 270
   ggc gtc ttt gcc tgg gcc cat gtt gct cct ctg ctt ctc tcc aag tac      864
   Gly Val Phe Ala Trp Ala His Val Ala Pro Leu Leu Leu Ser Lys Tyr
           275                 280                 285
   gcc atc gtc tat ctg ttt gcc att ggg gct gcc ttt gcc atg caa gtc      912
   Ala Ile Val Tyr Leu Phe Ala Ile Gly Ala Ala Phe Ala Met Gln Val
       290                 295                 300
   ggc cag atg att ctt gcc cat ctc gtg ctt gct ccc ttc ccc cac tgg      960
   Gly Gln Met Ile Leu Ala His Leu Val Leu Ala Pro Phe Pro His Trp
   305                 310                 315                 320
   aac gtg ctg ctc ttc ttc ccc ttt gtg gga ctg gca gtg cac tac att     1008
   Asn Val Leu Leu Phe Phe Pro Phe Val Gly Leu Ala Val His Tyr Ile
                   325                 330                 335
   gca ccc gtg ttt ggc tgg gac gcc gat atc gtg tcg gtt aac act ctc     1056
   Ala Pro Val Phe Gly Trp Asp Ala Asp Ile Val Ser Val Asn Thr Leu
               340                 345                 350
   ttc acc tgt ttt ggc gcc acc ctc tcc att tac gcc ttc ttt gtg ctt     1104
   Phe Thr Cys Phe Gly Ala Thr Leu Ser Ile Tyr Ala Phe Phe Val Leu
           355                 360                 365
   gag atc atc gac gag atc acc aac tac ctc gat atc tgg tgt ctg cga      1152
   Glu Ile Ile Asp Glu Ile Thr Asn Tyr Leu Asp Ile Trp Cys Leu Arg
       370                 375                 380
   atc aag tac cct cag gag aag aag act gag taa                          1185
   Ile Lys Tyr Pro Gln Glu Lys Lys Thr Glu
   385                 390
<210> 83
   <211> 394
   <212> PRT
   <213> Yarrowia lipolytica
<210> 84
   <211> 15812
   <212> DNA
   <213> Artificial Sequence
<220>
   <223> Plasmid pZKL2-5U89GC
<400> 84
   gtacgttatc atttgaacag tgaaaggcta cagtaacaga agcagttgta aacttcattc 60
   cgttgattct gtactacagt accccactac gccgcttccg ctgacactgt tcaacccaaa 120
   aactacatct gcgtgcgctg tgtaaggcta tcatcagata catactgtag attctgtaga 180
   tgcgaacctg cttgtatcat atacatcccc ctccccctga cctgcacaag caagcaatgt 240
   gacattgata ttgctgctta tctagtgccg aggatgtgaa agccgagact caaacatttc 300
   ttttactctc ttgttcctga ccagacctgg cggagattac gccagtatga ttcttgcagg 360
   tctgagacaa gcctggaaca gccaacattt atttttcgaa gcgagaaaca tgccacaccc 420
   cggcacgttc agagatgcat atgatttgtt tttcgagtaa cagtaccccc cccccccccc 480
   ccaatgaaac cagtattact cacaccatcc tcattcaaag cgttacactg attacgcgcc 540
   catcaacgac agcatgaggg gactgctgat ctgatctaat caaatgacta caaaaatcgc 600
   aataatgaag agcaaacgac aaaaaagaaa caggttaacc aatcccgctt caatgtctca 660
   ccacaatcca gcactgtttc tcattacctc ctccctctaa tttcagagtt gcatcagggt 720
   ccttgatggc gcgccagctg cattaatgaa tcggccaacg cgcggggaga ggcggtttgc 780
   gtattgggcg ctcttccgct tcctcgctca ctgactcgct gcgctcggtc gttcggctgc 840
   ggcgagcggt atcagctcac tcaaaggcgg taatacggtt atccacagaa tcaggggata 900
   acgcaggaaa gaacatgtga gcaaaaggcc agcaaaaggc caggaaccgt aaaaaggccg 960
   cgttgctggc gtttttccat aggctccgcc cccctgacga gcatcacaaa aatcgacgct 1020
   caagtcagag gtggcgaaac ccgacaggac tataaagata ccaggcgttt ccccctggaa 1080
   gctccctcgt gcgctctcct gttccgaccc tgccgcttac cggatacctg tccgcctttc 1140
   tcccttcggg aagcgtggcg ctttctcata gctcacgctg taggtatctc agttcggtgt 1200
   aggtcgttcg ctccaagctg ggctgtgtgc acgaaccccc cgttcagccc gaccgctgcg 1260
   ccttatccgg taactatcgt cttgagtcca acccggtaag acacgactta tcgccactgg 1320
   cagcagccac tggtaacagg attagcagag cgaggtatgt aggcggtgct acagagttct 1380
   tgaagtggtg gcctaactac ggctacacta gaagaacagt atttggtatc tgcgctctgc 1440
   tgaagccagt taccttcgga aaaagagttg gtagctcttg atccggcaaa caaaccaccg 1500
   ctggtagcgg tggttttttt gtttgcaagc agcagattac gcgcagaaaa aaaggatctc 1560
   aagaagatcc tttgatcttt tctacggggt ctgacgctca gtggaacgaa aactcacgtt 1620
   aagggatttt ggtcatgaga ttatcaaaaa ggatcttcac ctagatcctt ttaaattaaa 1680
   aatgaagttt taaatcaatc taaagtatat atgagtaaac ttggtctgac agttaccaat 1740
   gcttaatcag tgaggcacct atctcagcga tctgtctatt tcgttcatcc atagttgcct 1800
   gactccccgt cgtgtagata actacgatac gggagggctt accatctggc cccagtgctg 1860
   caatgatacc gcgagaccca cgctcaccgg ctccagattt atcagcaata aaccagccag 1920
   ccggaagggc cgagcgcaga agtggtcctg caactttatc cgcctccatc cagtctatta 1980
   attgttgccg ggaagctaga gtaagtagtt cgccagttaa tagtttgcgc aacgttgttg 2040
   ccattgctac aggcatcgtg gtgtcacgct cgtcgtttgg tatggcttca ttcagctccg 2100
   gttcccaacg atcaaggcga gttacatgat cccccatgtt gtgcaaaaaa gcggttagct 2160
   ccttcggtcc tccgatcgtt gtcagaagta agttggccgc agtgttatca ctcatggtta 2220
   tggcagcact gcataattct cttactgtca tgccatccgt aagatgcttt tctgtgactg 2280
   gtgagtactc aaccaagtca ttctgagaat agtgtatgcg gcgaccgagt tgctcttgcc 2340
   cggcgtcaat acgggataat accgcgccac atagcagaac tttaaaagtg ctcatcattg 2400
   gaaaacgttc ttcggggcga aaactctcaa ggatcttacc gctgttgaga tccagttcga 2460
   tgtaacccac tcgtgcaccc aactgatctt cagcatcttt tactttcacc agcgtttctg 2520
   ggtgagcaaa aacaggaagg caaaatgccg caaaaaaggg aataagggcg acacggaaat 2580
   gttgaatact catactcttc ctttttcaat attattgaag catttatcag ggttattgtc 2640
   tcatgagcgg atacatattt gaatgtattt agaaaaataa acaaataggg gttccgcgca 2700
   catttccccg aaaagtgcca cctgatgcgg tgtgaaatac cgcacagatg cgtaaggaga 2760
   aaataccgca tcaggaaatt gtaagcgtta atattttgtt aaaattcgcg ttaaattttt 2820
   gttaaatcag ctcatttttt aaccaatagg ccgaaatcgg caaaatccct tataaatcaa 2880
   aagaatagac cgagataggg ttgagtgttg ttccagtttg gaacaagagt ccactattaa 2940
   agaacgtgga ctccaacgtc aaagggcgaa aaaccgtcta tcagggcgat ggcccactac 3000
   gtgaaccatc accctaatca agttttttgg ggtcgaggtg ccgtaaagca ctaaatcgga 3060
   accctaaagg gagcccccga tttagagctt gacggggaaa gccggcgaac gtggcgagaa 3120
   aggaagggaa gaaagcgaaa ggagcgggcg ctagggcgct ggcaagtgta gcggtcacgc 3180
   tgcgcgtaac caccacaccc gccgcgctta atgcgccgct acagggcgcg tccattcgcc 3240
   attcaggctg cgcaactgtt gggaagggcg atcggtgcgg gcctcttcgc tattacgcca 3300
   gctggcgaaa gggggatgtg ctgcaaggcg attaagttgg gtaacgccag ggttttccca 3360
   gtcacgacgt tgtaaaacga cggccagtga attgtaatac gactcactat agggcgaatt 3420
   gggcccgacg tcgcatgctg gtttcgattt gtcttagagg aacgcatata cagtaatcat 3480
   agagaataaa cgatattcat ttattaaagt agatagttga ggtagaagtt gtaaagagtg 3540
   ataaatagct tagataccac agacaccctc ggtgacgaag tactgcagat ggtttccaat 3600
   cacattgacc tgctggagca gagtgttacc ggcagagcac tgtttattgc tctggccctg 3660
   gcacatgaca acgttggaga gaggagggtg gatcaggggc cagtcaataa agacctcacc 3720
   agagcagtgc tggtaaccgt cccagaaggg cacttgaggg acgatatctc ctcggtgggt 3780
   gattcggtag agctttcggt ctttggacac cttggagaca tcggggttct cctggccaaa 3840
   gaagagttta tcgacccagt tagcaaagcc agcgttaccg acaatgggct gaccaagagt 3900
   aacaacgagg ggatcgtggc cgttaacctt gaggttgatt ccgaacagaa gggctgcagc 3960
   tcctccgaga gagtgaccgg tgacagcaat ctggtagtcg ggatactgct caatcacaga 4020
   gtcgagcttg gggccgatct gattgtaggt gttgttgtag gactggatga agccattgtg 4080
   gacaagacag tcatcacaag tagcagtaga agagatgtta gcagcaagat caaagttaat 4140
   taactcacct gcaggattga gactatgaat ggattcccgt gcccgtatta ctctactaat 4200
   ttgatcttgg aacgcgaaaa tacgtttcta ggactccaaa gaatctcaac tcttgtcctt 4260
   actaaatata ctacccatag ttgatggttt acttgaacag agaggacatg ttcacttgac 4320
   ccaaagtttc tcgcatctct tggatatttg aacaacggcg tccactgacc gtcagttatc 4380
   cagtcacaaa acccccacat tcatacattc ccatgtacgt ttacaaagtt ctcaattcca 4440
   tcgtgcaaat caaaatcaca tctattcatt catcatatat aaacccatca tgtctactaa 4500
   cactcacaac tccatagaaa acatcgactc agaacacacg ctccatgcgg ccgcttagga 4560
   atcctgagcg tccttgacac agtgaaccac accgactttg tgcatgtact tgagggtgga 4620
   aatgatgttg cccacaatgg tagggtagaa gacgtaccga actccgtgtc gttcgcaaca 4680
   ctctcggaca gcttgctgca cgaagggata gtgccaagac gacattcgag gaaagaggtg 4740
   atgctcgatc tggaagttga gaccgccagt aaagaacatg gcaatgggtc caccgtaggt 4800
   ggaagaggtc tccacctgag ctctgtacca gtcgatctga tcggcttcaa cgtccttctc 4860
   ggagctcttg accttgcagt tcttgtcggg gattcgctcc gagccatcga agttgtgaga 4920
   caagatgaaa aagaaggtga ggaaggcacc ggtagcagtg ggcaccagag gaatggtgat 4980
   gagcagggag gttccagtga gataccaggg caagaaggcg gttcgaaaga tgaagaaagc 5040
   tcgcataacg aatgcaaggg ttcggtaccg tcgcagaaag ccgttctctc gcatggctgt 5100
   gacagactcg ggaatggtgt cgttgtgctg cattcggaag atgtagagag ggttgtacac 5160
   cagcgaaacg ccgtaggctc caagcacgag gtacatgtac caggcctgga atcggtgaaa 5220
   ccactttcga gcagtgttgg cagcagggta gttgtggaac acaaggaatg gttctgcgga 5280
   ctcggcatcc aggtcgagac catgctgatt ggtgtaggtg tgatgtcgca tgatgtgaga 5340
   ctgcagccag atccatctgg acgatccaat gacgtcgatg ccgtaggcaa agagagcgtt 5400
   gacccagggc tttttgctga tggcaccatg agaggcatcg tgctgaatgg acaggccgat 5460
   ctgcatgtgc atgaatccag tcaagagacc ccacagcacc attccggtag tagcccagtg 5520
   ccactcgcaa aaggcggtga cagcaatgat gccaacggtt cgcagccaga atccaggtgt 5580
   ggcataccag ttccgacctt tcatgacctc tcgcatagtt cgcttgacgt cctgtgcaaa 5640
   gggagagtcg taggtgtaga caatgtcctt ggaggttcgg tcgtgcttgc ctcgcacgaa 5700
   ctgttgaagc agcttcgagt tctcgggctt gacgtaaggg tgcatggagt agaacagagg 5760
   agaagcatcg gaggcaccag aagcgaggat caagtcgcct ccgggatgga ccttggcaag 5820
   accttccaga tcgtagagaa tgccgtcgat ggcaaccagg tcgggtcgct cgagcagctg 5880
   ctcggtagta agggagagag ccatggttgt gaattagggt ggtgagaatg gttggttgta 5940
   gggaagaatc aaaggccggt ctcgggatcc gtgggtatat atatatatat atatatatac 6000
   gatccttcgt tacctccctg ttctcaaaac tgtggttttt cgtttttcgt tttttgcttt 6060
   ttttgatttt tttagggcca actaagcttc cagatttcgc taatcacctt tgtactaatt 6120
   acaagaaagg aagaagctga ttagagttgg gctttttatg caactgtgct actccttatc 6180
   tctgatatga aagtgtagac ccaatcacat catgtcattt agagttggta atactgggag 6240
   gatagataag gcacgaaaac gagccatagc agacatgctg ggtgtagcca agcagaagaa 6300
   agtagatggg agccaattga cgagcgaggg agctacgcca atccgacata cgacacgctg 6360
   agatcgtctt ggccgggggg tacctacaga tgtccaaggg taagtgcttg actgtaattg 6420
   tatgtctgag gacaaatatg tagtcagccg tataaagtca taccaggcac cagtgccatc 6480
   atcgaaccac taactctcta tgatacatgc ctccggtatt attgtaccat gcgtcgcttt 6540
   gttacatacg tatcttgcct ttttctctca gaaactccag aattctctct cttgagcttt 6600
   tccataacaa gttcttctgc ctccaggaag tccatgggtg gtttgatcat ggttttggtg 6660
   tagtggtagt gcagtggtgg tattgtgact ggggatgtag ttgagaataa gtcatacaca 6720
   agtcagcttt cttcgagcct catataagta taagtagttc aacgtattag cactgtaccc 6780
   agcatctccg tatcgagaaa cacaacaaca tgccccattg gacagatcat gcggatacac 6840
   aggttgtgca gtatcataca tactcgatca gacaggtcgt ctgaccatca tacaagctga 6900
   acaagcgctc catacttgca cgctctctat atacacagtt aaattacata tccatagtct 6960
   aacctctaac agttaatctt ctggtaagcc tcccagccag ccttctggta tcgcttggcc 7020
   tcctcaatag gatctcggtt ctggccgtac agacctcggc cgacaattat gatatccgtt 7080
   ccggtagaca tgacatcctc aacagttcgg tactgctgtc cgagagcgtc tcccttgtcg 7140
   tcaagaccca ccccgggggt cagaataagc cagtcctcag agtcgccctt aggtcggttc 7200
   tgggcaatga agccaaccac aaactcgggg tcggatcggg caagctcaat ggtctgcttg 7260
   gagtactcgc cagtggccag agagcccttg caagacagct cggccagcat gagcagacct 7320
   ctggccagct tctcgttggg agaggggact aggaactcct tgtactggga gttctcgtag 7380
   tcagagacgt cctccttctt ctgttcagag acagtttcct cggcaccagc tcgcaggcca 7440
   gcaatgattc cggttccggg tacaccgtgg gcgttggtga tatcggacca ctcggcgatt 7500
   cggtgacacc ggtactggtg cttgacagtg ttgccaatat ctgcgaactt tctgtcctcg 7560
   aacaggaaga aaccgtgctt aagagcaagt tccttgaggg ggagcacagt gccggcgtag 7620
   gtgaagtcgt caatgatgtc gatatgggtt ttgatcatgc acacataagg tccgacctta 7680
   tcggcaagct caatgagctc cttggtggtg gtaacatcca gagaagcaca caggttggtt 7740
   ttcttggctg ccacgagctt gagcactcga gcggcaaagg cggacttgtg gacgttagct 7800
   cgagcttcgt aggagggcat tttggtggtg aagaggagac tgaaataaat ttagtctgca 7860
   gaacttttta tcggaacctt atctggggca gtgaagtata tgttatggta atagttacga 7920
   gttagttgaa cttatagata gactggacta tacggctatc ggtccaaatt agaaagaacg 7980
   tcaatggctc tctgggcgtc gcctttgccg acaaaaatgt gatcatgatg aaagccagca 8040
   atgacgttgc agctgatatt gttgtcggcc aaccgcgccg aaaacgcagc tgtcagaccc 8100
   acagcctcca acgaagaatg tatcgtcaaa gtgatccaag cacactcata gttggagtcg 8160
   tactccaaag gcggcaatga cgagtcagac agatactcgt cgaccttttc cttgggaacc 8220
   accaccgtca gcccttctga ctcacgtatt gtagccaccg acacaggcaa cagtccgtgg 8280
   atagcagaat atgtcttgtc ggtccatttc tcaccaactt taggcgtcaa gtgaatgttg 8340
   cagaagaagt atgtgccttc attgagaatc ggtgttgctg atttcaataa agtcttgaga 8400
   tcagtttggc cagtcatgtt gtggggggta attggattga gttatcgcct acagtctgta 8460
   caggtatact cgctgcccac tttatacttt ttgattccgc tgcacttgaa gcaatgtcgt 8520
   ttaccaaaag tgagaatgct ccacagaaca caccccaggg tatggttgag caaaaaataa 8580
   acactccgat acggggaatc gaaccccggt ctccacggtt ctcaagaagt attcttgatg 8640
   agagcgtatc gatcgaggaa gaggacaagc ggctgcttct taagtttgtg acatcagtat 8700
   ccaaggcacc attgcaagga ttcaaggctt tgaacccgtc atttgccatt cgtaacgctg 8760
   gtagacaggt tgatcggttc cctacggcct ccacctgtgt caatcttctc aagctgcctg 8820
   actatcagga cattgatcaa cttcggaaga aacttttgta tgccattcga tcacatgctg 8880
   gtttcgattt gtcttagagg aacgcatata cagtaatcat agagaataaa cgatattcat 8940
   ttattaaagt agatagttga ggtagaagtt gtaaagagtg ataaatagcg gccgctcact 9000
   gaatcttttt ggctcccttg tgctttcgga cgatgtaggt ctgcacgtag aagttgagga 9060
   acagacacag gacagtacca acgtagaagt agttgaaaaa ccagccaaac attctcattc 9120
   catcttgtcg gtagcaggga atgttccggt acttccagac gatgtagaag ccaacgttga 9180
   actgaatgat ctgcatagaa gtaatcaggg acttgggcat agggaacttg agcttgatca 9240
   gtcgggtcca atagtagccg tacatgatcc agtgaatgaa gccgttgagc agcacaaaga 9300
   tccaaacggc ttcgtttcgg tagttgtaga acagccacat gtccatagga gctccgagat 9360
   ggtgaaagaa ctgcaaccag gtcagaggct tgcccatgag gggcagatag aaggagtcaa 9420
   tgtactcgag gaacttgctg aggtagaaca gctgagtggt gattcggaag acattgttgt 9480
   cgaaagcctt ctcgcagttg tcggacatga caccaatggt gtacatggcg taggccatag 9540
   agaggaagga gcccagcgag tagatggaca tgagcaggtt gtagttggtg aacacaaact 9600
   tcattcgaga ctgacccttg ggtccgagag gaccaagggt gaacttcagg atgacgaagg 9660
   cgatggagag gtacagcacc tcgcagtgcg aggcatcaga ccagagctga gcatagtcga 9720
   ccttgggaag aacctcctgg ccaatggaga cgatttcgtt cacgacctcc atggttgatg 9780
   tgtgtttaat tcaagaatga atatagagaa gagaagaaga aaaaagattc aattgagccg 9840
   gcgatgcaga cccttatata aatgttgcct tggacagacg gagcaagccc gcccaaacct 9900
   acgttcggta taatatgtta agctttttaa cacaaaggtt tggcttgggg taacctgatg 9960
   tggtgcaaaa gaccgggcgt tggcgagcca ttgcgcgggc gaatggggcc gtgactcgtc 10020
   tcaaattcga gggcgtgcct caattcgtgc cccgtggctt ttttcccgcc gtttccgccc 10080
   cgtttgcacc actgcagccg cttctttggt tcggacacct tgctgcgagc taggtgcctt 10140
   gtgctactta aaaagtggcc tcccaacacc aacatgacat gagtgcgtgg gccaagacac 10200
   gttggcgggg tcgcagtcgg ctcaatggcc cggaaaaaac gctgctggag ctggttcgga 10260
   cgcagtccgc cgcggcgtat ggatatccgc aaggttccat agcgccattg ccctccgtcg 10320
   gcgtctatcc cgcaacctct aaatagagcg ggaatataac ccaagcttct tttttttcct 10380
   ttaacacgca cacccccaac tatcatgttg ctgctgctgt ttgactctac tctgtggagg 10440
   ggtgctccca cccaacccaa cctacaggtg gatccggcgc tgtgattggc tgataagtct 10500
   cctatccgga ctaattctga ccaatgggac atgcgcgcag gacccaaatg ccgcaattac 10560
   gtaaccccaa cgaaatgcct acccctcttt ggagcccagc ggccccaaat ccccccaagc 10620
   agcccggttc taccggcttc catctccaag cacaagcagc ccggttctac cggcttccat 10680
   ctccaagcac ccctttctcc acaccccaca aaaagacccg tgcaggacat cctactgcgt 10740
   gtttaaacac cactaaaacc ccacaaaata tatcttaccg aatatacaga tctactatag 10800
   aggaacaatt gccccggaga agacggccag gccgcctaga tgacaaattc aacaactcac 10860
   agctgacttt ctgccattgc cactaggggg gggccttttt atatggccaa gccaagctct 10920
   ccacgtcggt tgggctgcac ccaacaataa atgggtaggg ttgcaccaac aaagggatgg 10980
   gatggggggt agaagatacg aggataacgg ggctcaatgg cacaaataag aacgaatact 11040
   gccattaaga ctcgtgatcc agcgactgac accattgcat catctaaggg cctcaaaact 11100
   acctcggaac tgctgcgctg atctggacac cacagaggtt ccgagcactt taggttgcac 11160
   caaatgtccc accaggtgca ggcagaaaac gctggaacag cgtgtacagt ttgtcttaac 11220
   aaaaagtgag ggcgctgagg tcgagcaggg tggtgtgact tgttatagcc tttagagctg 11280
   cgaaagcgcg tatggatttg gctcatcagg ccagattgag ggtctgtgga cacatgtcat 11340
   gttagtgtac ttcaatcgcc ccctggatat agccccgaca ataggccgtg gcctcatttt 11400
   tttgccttcc gcacatttcc attgctcggt acccacacct tgcttctcct gcacttgcca 11460
   accttaatac tggtttacat tgaccaacat cttacaagcg gggggcttgt ctagggtata 11520
   tataaacagt ggctctccca atcggttgcc agtctctttt ttcctttctt tccccacaga 11580
   ttcgaaatct aaactacaca tcacacaatg cctgttactg acgtccttaa gcgaaagtcc 11640
   ggtgtcatcg tcggcgacga tgtccgagcc gtgagtatcc acgacaagat cagtgtcgag 11700
   acgacgcgtt ttgtgtaatg acacaatccg aaagtcgcta gcaacacaca ctctctacac 11760
   aaactaaccc agctctccat ggtgaaggct tctcgacagg ctctgcccct cgtcatcgac 11820
   ggaaaggtgt acgacgtctc cgcttgggtg aacttccacc ctggtggagc tgaaatcatt 11880
   gagaactacc agggacgaga tgctactgac gccttcatgg ttatgcactc tcaggaagcc 11940
   ttcgacaagc tcaagcgaat gcccaagatc aaccaggctt ccgagctgcc tccccaggct 12000
   gccgtcaacg aagctcagga ggatttccga aagctccgag aagagctgat cgccactggc 12060
   atgtttgacg cctctcccct ctggtactcg tacaagatct tgaccaccct gggtcttggc 12120
   gtgcttgcct tcttcatgct ggtccagtac cacctgtact tcattggtgc tctcgtgctc 12180
   ggtatgcact accagcaaat gggatggctg tctcatgaca tctgccacca ccagaccttc 12240
   aagaaccgaa actggaataa cgtcctgggt ctggtctttg gcaacggact ccagggcttc 12300
   tccgtgacct ggtggaagga cagacacaac gcccatcatt ctgctaccaa cgttcagggt 12360
   cacgatcccg acattgataa cctgcctctg ctcgcctggt ccgaggacga tgtcactcga 12420
   gcttctccca tctcccgaaa gctcattcag ttccaacagt actatttcct ggtcatctgt 12480
   attctcctgc gattcatctg gtgtttccag tctgtgctga ccgttcgatc cctcaaggac 12540
   cgagacaacc agttctaccg atctcagtac aagaaagagg ccattggact cgctctgcac 12600
   tggactctca agaccctgtt ccacctcttc tttatgccct ccatcctgac ctcgatgctg 12660
   gtgttctttg tttccgagct cgtcggtggc ttcggaattg ccatcgtggt cttcatgaac 12720
   cactaccctc tggagaagat cggtgattcc gtctgggacg gacatggctt ctctgtgggt 12780
   cagatccatg agaccatgaa cattcgacga ggcatcatta ctgactggtt ctttggaggc 12840
   ctgaactacc agatcgagca ccatctctgg cccaccctgc ctcgacacaa cctcactgcc 12900
   gtttcctacc aggtggaaca gctgtgccag aagcacaacc tcccctaccg aaaccctctg 12960
   ccccatgaag gtctcgtcat cctgctccga tacctgtccc agttcgctcg aatggccgag 13020
   aagcagcccg gtgccaaggc tcagtaagcg gccgcatgag aagataaata tataaataca 13080
   ttgagatatt aaatgcgcta gattagagag cctcatactg ctcggagaga agccaagacg 13140
   agtactcaaa ggggattaca ccatccatat ccacagacac aagctgggga aaggttctat 13200
   atacactttc cggaataccg tagtttccga tgttatcaat gggggcagcc aggatttcag 13260
   gcacttcggt gtctcggggt gaaatggcgt tcttggcctc catcaagtcg taccatgtct 13320
   tcatttgcct gtcaaagtaa aacagaagca gatgaagaat gaacttgaag tgaaggaatt 13380
   taaatagttg gagcaaggga gaaatgtaga gtgtgaaaga ctcactatgg tccgggctta 13440
   tctcgaccaa tagccaaagt ctggagtttc tgagagaaaa aggcaagata cgtatgtaac 13500
   aaagcgacgc atggtacaat aataccggag gcatgtatca tagagagtta gtggttcgat 13560
   gatggcactg gtgcctggta tgactttata cggctgacta catatttgtc ctcagacata 13620
   caattacagt caagcactta cccttggaca tctgtaggta ccccccggcc aagacgatct 13680
   cagcgtgtcg tatgtcggat tggcgtagct ccctcgctcg tcaattggct cccatctact 13740
   ttcttctgct tggctacacc cagcatgtct gctatggctc gttttcgtgc cttatctatc 13800
   ctcccagtat taccaactct aaatgacatg atgtgattgg gtctacactt tcatatcaga 13860
   gataaggagt agcacagttg cataaaaagc ccaactctaa tcagcttctt cctttcttgt 13920
   aattagtaca aaggtgatta gcgaaatctg gaagcttagt tggccctaaa aaaatcaaaa 13980
   aaagcaaaaa acgaaaaacg aaaaaccaca gttttgagaa cagggaggta acgaaggatc 14040
   gtatatatat atatatatat atatacccac ggatcccgag accggccttt gattcttccc 14100
   tacaaccaac cattctcacc accctaattc acaaccatgg gcgtattcat taaacaggag 14160
   cagcttccgg ctctcaagaa gtacaagtac tccgccgagg atcactcgtt catctccaac 14220
   aacattctgc gccccttctg gcgacagttt gtcaaaatct tccctctgtg gatggccccc 14280
   aacatggtga ctctgctggg cttcttcttt gtcattgtga acttcatcac catgctcatt 14340
   gttgatccca cccacgaccg cgagcctccc agatgggtct acctcaccta cgctctgggt 14400
   ctgttccttt accagacatt tgatgcctgt gacggatccc atgcccgacg aactggccag 14460
   agtggacccc ttggagagct gtttgaccac tgtgtcgacg ccatgaatac ctctctgatt 14520
   ctcacggtgg tggtgtccac cacccatatg ggatataaca tgaagctact gattgtgcag 14580
   attgccgctc tcggaaactt ctacctgtcg acctgggaga cctaccatac cggaactctg 14640
   tacctttctg gcttctctgg tcctgttgaa ggtatcttga ttctggtggc tcttttcgtc 14700
   ctcaccttct tcactggtcc caacgtgtac gctctgaccg tctacgaggc tcttcccgag 14760
   tccatcactt cgctgctgcc tgccagcttc ctggacgtca ccatcaccca gatctacatt 14820
   ggattcggag tgctgggcat ggtgttcaac atctacggcg cctgcggaaa cgtgatcaag 14880
   tactacaaca acaagggcaa gagcgctctc cccgccattc tcggaatcgc cccctttggc 14940
   atcttctacg tcggcgtctt tgcctgggcc catgttgctc ctctgcttct ctccaagtac 15000
   gccatcgtct atctgtttgc cattggggct gcctttgcca tgcaagtcgg ccagatgatt 15060
   cttgcccatc tcgtgcttgc tccctttccc cactggaacg tgctgctctt cttccccttt 15120
   gtgggactgg cagtgcacta cattgcaccc gtgtttggct gggacgccga tatcgtgtcg 15180
   gttaacactc tcttcacctg ttttggcgcc accctctcca tttacgcctt ctttgtgctt 15240
   gagatcatcg acgagatcac caactacctc gatatctggt gtctgcgaat caagtaccct 15300
   caggagaaga agaccgaata agcggccgca tggagcgtgt gttctgagtc gatgttttct 15360
   atggagttgt gagtgttagt agacatgatg ggtttatata tgatgaatga atagatgtga 15420
   ttttgatttg cacgatggaa ttgagaactt tgtaaacgta catgggaatg tatgaatgtg 15480
   ggggttttgt gactggataa ctgacggtca gtggacgccg ttgttcaaat atccaagaga 15540
   tgcgagaaac tttgggtcaa gtgaacatgt cctctctgtt caagtaaacc atcaactatg 15600
   ggtagtatat ttagtaagga caagagttga gattctttgg agtcctagaa acgtatttcc 15660
   gcgttccaag atcaaattag tagagtaata cgggcacggg aatccattca tagtctcaat 15720
   cctgcaggtg agttaattaa tcgagcttgg cgtaatcatg gtcatagctg tttcctgtgt 15780
   gaaattgtta tccgctcaca attccacaca ac 15812
<210> 85
   <211> 4313
   <212> DNA
   <213> Artificial Sequence
<220>
   <223> Plasmid pZKUM
<400> 85
   taatcgagct tggcgtaatc atggtcatag ctgtttcctg tgtgaaattg ttatccgctc      60
   acaattccac acaacatacg agccggaagc ataaagtgta aagcctgggg tgcctaatga     120
   gtgagctaac tcacattaat tgcgttgcgc tcactgcccg ctttccagtc gggaaacctg     180
   tcgtgccagc tgcattaatg aatcggccaa cgcgcgggga gaggcggttt gcgtattggg     240
   cgctcttccg cttcctcgct cactgactcg ctgcgctcgg tcgttcggct gcggcgagcg 300
   gtatcagctc actcaaaggc ggtaatacgg ttatccacag aatcagggga taacgcagga 360
   aagaacatgt gagcaaaagg ccagcaaaag gccaggaacc gtaaaaaggc cgcgttgctg 420
   gcgtttttcc ataggctccg cccccctgac gagcatcaca aaaatcgacg ctcaagtcag 480
   aggtggcgaa acccgacagg actataaaga taccaggcgt ttccccctgg aagctccctc 540
   gtgcgctctc ctgttccgac cctgccgctt accggatacc tgtccgcctt tctcccttcg 600
   ggaagcgtgg cgctttctca tagctcacgc tgtaggtatc tcagttcggt gtaggtcgtt 660
   cgctccaagc tgggctgtgt gcacgaaccc cccgttcagc ccgaccgctg cgccttatcc 720
   ggtaactatc gtcttgagtc caacccggta agacacgact tatcgccact ggcagcagcc 780
   actggtaaca ggattagcag agcgaggtat gtaggcggtg ctacagagtt cttgaagtgg 840
   tggcctaact acggctacac tagaaggaca gtatttggta tctgcgctct gctgaagcca 900
   gttaccttcg gaaaaagagt tggtagctct tgatccggca aacaaaccac cgctggtagc 960
   ggtggttttt ttgtttgcaa gcagcagatt acgcgcagaa aaaaaggatc tcaagaagat 1020
   cctttgatct tttctacggg gtctgacgct cagtggaacg aaaactcacg ttaagggatt 1080
   ttggtcatga gattatcaaa aaggatcttc acctagatcc ttttaaatta aaaatgaagt 1140
   tttaaatcaa tctaaagtat atatgagtaa acttggtctg acagttacca atgcttaatc 1200
   agtgaggcac ctatctcagc gatctgtcta tttcgttcat ccatagttgc ctgactcccc 1260
   gtcgtgtaga taactacgat acgggagggc ttaccatctg gccccagtgc tgcaatgata 1320
   ccgcgagacc cacgctcacc ggctccagat ttatcagcaa taaaccagcc agccggaagg 1380
   gccgagcgca gaagtggtcc tgcaacttta tccgcctcca tccagtctat taattgttgc 1440
   cgggaagcta gagtaagtag ttcgccagtt aatagtttgc gcaacgttgt tgccattgct 1500
   acaggcatcg tggtgtcacg ctcgtcgttt ggtatggctt cattcagctc cggttcccaa 1560
   cgatcaaggc gagttacatg atcccccatg ttgtgcaaaa aagcggttag ctccttcggt 1620
   cctccgatcg ttgtcagaag taagttggcc gcagtgttat cactcatggt tatggcagca 1680
   ctgcataatt ctcttactgt catgccatcc gtaagatgct tttctgtgac tggtgagtac 1740
   tcaaccaagt cattctgaga atagtgtatg cggcgaccga gttgctcttg cccggcgtca 1800
   atacgggata ataccgcgcc acatagcaga actttaaaag tgctcatcat tggaaaacgt 1860
   tcttcggggc gaaaactctc aaggatctta ccgctgttga gatccagttc gatgtaaccc 1920
   actcgtgcac ccaactgatc ttcagcatct tttactttca ccagcgtttc tgggtgagca 1980
   aaaacaggaa ggcaaaatgc cgcaaaaaag ggaataaggg cgacacggaa atgttgaata 2040
   ctcatactct tcctttttca atattattga agcatttatc agggttattg tctcatgagc 2100
   ggatacatat ttgaatgtat ttagaaaaat aaacaaatag gggttccgcg cacatttccc 2160
   cgaaaagtgc cacctgacgc gccctgtagc ggcgcattaa gcgcggcggg tgtggtggtt 2220
   acgcgcagcg tgaccgctac acttgccagc gccctagcgc ccgctccttt cgctttcttc 2280
   ccttcctttc tcgccacgtt cgccggcttt ccccgtcaag ctctaaatcg ggggctccct 2340
   ttagggttcc gatttagtgc tttacggcac ctcgacccca aaaaacttga ttagggtgat 2400
   ggttcacgta gtgggccatc gccctgatag acggtttttc gccctttgac gttggagtcc 2460
   acgttcttta atagtggact cttgttccaa actggaacaa cactcaaccc tatctcggtc 2520
   tattcttttg atttataagg gattttgccg atttcggcct attggttaaa aaatgagctg 2580
   atttaacaaa aatttaacgc gaattttaac aaaatattaa cgcttacaat ttccattcgc 2640
   cattcaggct gcgcaactgt tgggaagggc gatcggtgcg ggcctcttcg ctattacgcc 2700
   agctggcgaa agggggatgt gctgcaaggc gattaagttg ggtaacgcca gggttttccc 2760
   agtcacgacg ttgtaaaacg acggccagtg aattgtaata cgactcacta tagggcgaat 2820
   tgggtaccgg gccccccctc gaggtcgacg agtatctgtc tgactcgtca ttgccgcctt 2880
   tggagtacga ctccaactat gagtgtgctt ggatcacttt gacgatacat tcttcgttgg 2940
   aggctgtggg tctgacagct gcgttttcgg cgcggttggc cgacaacaat atcagctgca 3000
   acgtcattgc tggctttcat catgatcaca tttttgtcgg caaaggcgac gcccagagag 3060
   ccattgacgt tctttctaat ttggaccgat agccgtatag tccagtctat ctataagttc 3120
   aactaactcg taactattac cataacatat acttcactgc cccagataag gttccgataa 3180
   aaagttctgc agactaaatt tatttcagtc tcctcttcac caccaaaatg ccctcctacg 3240
   aagctcgagt gctcaagctc gtggcagcca agaaaaccaa cctgtgtgct tctctggatg 3300
   ttaccaccac caaggagctc attgagcttg ccgataaggt cggaccttat gtgtgcatga 3360
   tcaaaaccca tatcgacatc attgacgact tcacctacgc cggcactgtg ctccccctca 3420
   aggaacttgc tcttaagcac ggtttcttcc tgttcgagga cagaaagttc gcagatattg 3480
   gcaacactgt caagcaccag taccggtgtc accgaatcgc cgagtggtcc gatatcacca 3540
   acgcccacgg tgtacccgga accggaatcg attgctggcc tgcgagctgg tgcgtacgag 3600
   gaaactgtct ctgaacagaa gaaggaggac gtctctgact acgagaactc ccagtacaag 3660
   gagttcctag tcccctctcc caacgagaag ctggccagag gtctgctcat gctggccgag 3720
   ctgtcttgca agggctctct ggccactggc gagtactcca agcagaccat tgagcttgcc 3780
   cgatccgacc ccgagtttgt ggttggcttc attgcccaga accgacctaa gggcgactct 3840
   gaggactggc ttattctgac ccccggggtg ggtcttgacg acaagggaga cgctctcgga 3900
   cagcagtacc gaactgttga ggatgtcatg tctaccggaa cggatatcat aattgtcggc   3960
   cgaggtctgt acggccagaa ccgagatcct attgaggagg ccaagcgata ccagaaggct   4020
   ggctgggagg cttaccagaa gattaactgt tagaggttag actatggata tgtaatttaa   4080
   ctgtgtatat agagagcgtg caagtatgga gcgcttgttc agcttgtatg atggtcagac   4140
   gacctgtctg atcgagtatg tatgatactg cacaacctgt gtatccgcat gatctgtcca   4200
   atggggcatg ttgttgtgtt tctcgatacg gagatgctgg gtacagtgct aatacgttga   4260
   actacttata cttatatgag gctcgaagaa agctgacttg tgtatgactt aat          4313
<210> 86
   <211> 1459
   <212> DNA
   <213> Artificial Sequence
<220>
   <223> Synthetic mutant Ura3 gene, comprising a 33 bp deletion from +21 to +53, a 1 bp deletion at +376 and a 3 bp deletion from +400 to 403 of the Yarrowia Ura3 coding region (GenBank Accession No. AJ306421)
<400> 86
   gagtatctgt ctgactcgtc attgccgcct ttggagtacg actccaacta tgagtgtgct     60
   tggatcactt tgacgataca ttcttcgttg gaggctgtgg gtctgacagc tgcgttttcg    120
   gcgcggttgg ccgacaacaa tatcagctgc aacgtcattg ctggctttca tcatgatcac    180
   atttttgtcg gcaaaggcga cgcccagaga gccattgacg ttctttctaa tttggaccga    240
   tagccgtata gtccagtcta tctataagtt caactaactc gtaactatta ccataacata    300
   tacttcactg ccccagataa ggttccgata aaaagttctg cagactaaat ttatttcagt    360
   ctcctcttca ccaccaaaat gccctcctac gaagctcgag tgctcaagct cgtggcagcc    420
   aagaaaacca acctgtgtgc ttctctggat gttaccacca ccaaggagct cattgagctt    480
   gccgataagg tcggacctta tgtgtgcatg atcaaaaccc atatcgacat cattgacgac    540
   ttcacctacg ccggcactgt gctccccctc aaggaacttg ctcttaagca cggtttcttc    600
   ctgttcgagg acagaaagtt cgcagatatt ggcaacactg tcaagcacca gtaccggtgt    660
   caccgaatcg ccgagtggtc cgatatcacc aacgcccacg gtgtacccgg aaccggaatc    720
   gattgctggc ctgcgagctg gtgcgtacga ggaaactgtc tctgaacaga agaaggagga    780
   cgtctctgac tacgagaact cccagtacaa ggagttccta gtcccctctc ccaacgagaa    840
   gctggccaga ggtctgctca tgctggccga gctgtcttgc aagggctctc tggccactgg    900
   cgagtactcc aagcagacca ttgagcttgc ccgatccgac cccgagtttg tggttggctt    960
   cattgcccag aaccgaccta agggcgactc tgaggactgg cttattctga cccccggggt   1020
   gggtcttgac gacaagggag acgctctcgg acagcagtac cgaactgttg aggatgtcat 1080
   gtctaccgga acggatatca taattgtcgg ccgaggtctg tacggccaga accgagatcc 1140
   tattgaggag gccaagcgat accagaaggc tggctgggag gcttaccaga agattaactg 1200
   ttagaggtta gactatggat atgtaattta actgtgtata tagagagcgt gcaagtatgg 1260
   agcgcttgtt cagcttgtat gatggtcaga cgacctgtct gatcgagtat gtatgatact 1320
   gcacaacctg tgtatccgca tgatctgtcc aatggggcat gttgttgtgt ttctcgatac 1380
   ggagatgctg ggtacagtgc taatacgttg aactacttat acttatatga ggctcgaaga 1440
   aagctgactt gtgtatgac 1459
<210> 87
   <211> 7966
   <212> DNA
   <213> Artificial Sequence
<220>
   <223> Plasmid pYPS161
<400> 87
   aaatgtaacg aaactgaaat ttgaccagat attgtgtccg cggtggagct ccagcttttg 60
   ttccctttag tgagggttaa tttcgagctt ggcgtaatca tggtcatagc tgtttcctgt 120
   gtgaaattgt tatccgctca caagcttcca cacaacgtac gttctggttg gctcggatga 180
   tttctgcggc cccagcgtaa ggcaggcgtt ccgtccggat cggtttgggt cggatcggct 240
   ttttgattgt cgtattgtcg ctcatgttgg acctggtgtg tagttgtagt gtcagatcag 300
   attcaccagc gaatgcatgt gaacttcccc acattttgag ccgaggcaga tttgggttgc 360
   ttagtaagca gacgtggcgt tgcaagtaga tgtggcaaat ggggacgaag attccgaggg 420
   gatatcatag ttccaagggg atgtcatcat ttgccagctt tcgccgccac ttttgacgag 480
   tttttgtggg tcaaataagt ttagttgaac ttttcaaatt tcagttggca ttttgttaat 540
   agaaagggtg ccggtgctgg ggggttcatt cctcgggttg cagatatcct atctgtctta 600
   ggggtatctc tttcaatcga caagatgtag ttgggtaaca attatttatt aatattctct 660
   ccatccagta cagtactaac atcttgacat ctcagcacaa gtgcatcttc ccaagtgttt 720
   gttggagagg ttgttgggta ttacttagga aacagaacac agtacgtgga gatcttggat 780
   acatcgtaca tggaggttat ccataaaaaa gaccctccag gactagttac aatgccgtta 840
   gatgaggaaa tccacaaccc tgattcacta tgaacatatt atcttccccc aaacttgcga 900
   tatatggccc ttgatgatag ccttgatttt acccttgatg gtacctccac gaccaaccga 960
   tctgctgttt gaagagatat tttcaaattt gaagtgctca gatctactaa acatgagtcc 1020
   agtaattctt tccgtctttc cgatttccga tattcccttt tttagcccga cttttcactg 1080
   ctcccatgtc aaacgattag gacttgggag acaatcccac tgtcaaaatc accccgatat 1140
   tctctgtaaa acaagtactt cttccacgtg atcttcaaat acctcttcca cgtgaccttc 1200
   aaatacctct tcaagtacct cttccacgcg accttcaaag tcccttcaaa tacccttctc 1260
   aattctcccc ttctcctcca tagtccttct ctctgactaa gcttgagaat acatgacgct 1320
   aagacgaaaa cacactagag accctgagag cctgaacatg catccactct gcagttgcgc 1380
   acgtgcctac agcaactatc gggtccagtg ctggatctga cactgcgtct ccctatgaag 1440
   aaactgataa acagatctgc actcataaca atgatctgag cgatgaaaac gtgacctcca 1500
   cagccacaag tcataatcgg cgcgccagct gcattaatga atcggccaac gcgcggggag 1560
   aggcggtttg cgtattgggc gctcttccgc ttcctcgctc actgactcgc tgcgctcggt 1620
   cgttcggctg cggcgagcgg tatcagctca ctcaaaggcg gtaatacggt tatccacaga 1680
   atcaggggat aacgcaggaa agaacatgtg agcaaaaggc cagcaaaagg ccaggaaccg 1740
   taaaaaggcc gcgttgctgg cgtttttcca taggctccgc ccccctgacg agcatcacaa 1800
   aaatcgacgc tcaagtcaga ggtggcgaaa cccgacagga ctataaagat accaggcgtt 1860
   tccccctgga agctccctcg tgcgctctcc tgttccgacc ctgccgctta ccggatacct 1920
   gtccgccttt ctcccttcgg gaagcgtggc gctttctcat agctcacgct gtaggtatct 1980
   cagttcggtg taggtcgttc gctccaagct gggctgtgtg cacgaacccc ccgttcagcc 2040
   cgaccgctgc gccttatccg gtaactatcg tcttgagtcc aacccggtaa gacacgactt 2100
   atcgccactg gcagcagcca ctggtaacag gattagcaga gcgaggtatg taggcggtgc 2160
   tacagagttc ttgaagtggt ggcctaacta cggctacact agaagaacag tatttggtat 2220
   ctgcgctctg ctgaagccag ttaccttcgg aaaaagagtt ggtagctctt gatccggcaa 2280
   acaaaccacc gctggtagcg gtggtttttt tgtttgcaag cagcagatta cgcgcagaaa 2340
   aaaaggatct caagaagatc ctttgatctt ttctacgggg tctgacgctc agtggaacga 2400
   aaactcacgt taagggattt tggtcatgag attatcaaaa aggatcttca cctagatcct 2460
   tttaaattaa aaatgaagtt ttaaatcaat ctaaagtata tatgagtaaa cttggtctga 2520
   cagttaccaa tgcttaatca gtgaggcacc tatctcagcg atctgtctat ttcgttcatc 2580
   catagttgcc tgactccccg tcgtgtagat aactacgata cgggagggct taccatctgg 2640
   ccccagtgct gcaatgatac cgcgagaccc acgctcaccg gctccagatt tatcagcaat 2700
   aaaccagcca gccggaaggg ccgagcgcag aagtggtcct gcaactttat ccgcctccat 2760
   ccagtctatt aattgttgcc gggaagctag agtaagtagt tcgccagtta atagtttgcg 2820
   caacgttgtt gccattgcta caggcatcgt ggtgtcacgc tcgtcgtttg gtatggcttc 2880
   attcagctcc ggttcccaac gatcaaggcg agttacatga tcccccatgt tgtgcaaaaa 2940
   agcggttagc tccttcggtc ctccgatcgt tgtcagaagt aagttggccg cagtgttatc 3000
   actcatggtt atggcagcac tgcataattc tcttactgtc atgccatccg taagatgctt 3060
   ttctgtgact ggtgagtact caaccaagtc attctgagaa tagtgtatgc ggcgaccgag 3120
   ttgctcttgc ccggcgtcaa tacgggataa taccgcgcca catagcagaa ctttaaaagt 3180
   gctcatcatt ggaaaacgtt cttcggggcg aaaactctca aggatcttac cgctgttgag 3240
   atccagttcg atgtaaccca ctcgtgcacc caactgatct tcagcatctt ttactttcac 3300
   cagcgtttct gggtgagcaa aaacaggaag gcaaaatgcc gcaaaaaagg gaataagggc 3360
   gacacggaaa tgttgaatac tcatactctt cctttttcaa tattattgaa gcatttatca 3420
   gggttattgt ctcatgagcg gatacatatt tgaatgtatt tagaaaaata aacaaatagg 3480
   ggttccgcgc acatttcccc gaaaagtgcc acctgatgcg gtgtgaaata ccgcacagat 3540
   gcgtaaggag aaaataccgc atcaggaaat tgtaagcgtt aatattttgt taaaattcgc 3600
   gttaaatttt tgttaaatca gctcattttt taaccaatag gccgaaatcg gcaaaatccc 3660
   ttataaatca aaagaataga ccgagatagg gttgagtgtt gttccagttt ggaacaagag 3720
   tccactatta aagaacgtgg actccaacgt caaagggcga aaaaccgtct atcagggcga 3780
   tggcccacta cgtgaaccat caccctaatc aagttttttg gggtcgaggt gccgtaaagc 3840
   actaaatcgg aaccctaaag ggagcccccg atttagagct tgacggggaa agccggcgaa 3900
   cgtggcgaga aaggaaggga agaaagcgaa aggagcgggc gctagggcgc tggcaagtgt 3960
   agcggtcacg ctgcgcgtaa ccaccacacc cgccgcgctt aatgcgccgc tacagggcgc 4020
   gtccattcgc cattcaggct gcgcaactgt tgggaagggc gatcggtgcg ggcctcttcg 4080
   ctattacgcc agctggcgaa agggggatgt gctgcaaggc gattaagttg ggtaacgcca 4140
   gggttttccc agtcacgacg ttgtaaaacg acggccagtg aattgtaata cgactcacta 4200
   tagggcgaat tgggcccgac gtcgcatgca actattagtg aggcttcggg agtggttgtc 4260
   tcggttgtct cattcagact cgttgtgttg tatctatatc tatataaaca ctcttgtccc 4320
   tcaatcccac tgccatcttt tgctaaactt gccgccaata tgaaactcat ctccctcatc 4380
   accgtcgcta ccaccgctct ggcggctgtc ggagacaagt acaagctgac ctataccaga 4440
   tcagacgccc aatcggtcga atctctgccc gtcacctacc aagatgacct gatcaccgcc 4500
   tccaccgacg gcgaacccat caccatcacc gagggcgagg gcaacacctt ctctgttaac 4560
   gacatgccca tcgcctatct ggagctgcag gctttgttct ggaccggcga ctacggctac 4620
   aagctccagg gctcggtctt tgacattgcc gccgatggaa cctttgagct gagagacggc 4680
   cccaaggagt actactattg cactcctcac cctgagcgaa acgtcatcta cgtcatcaac 4740
   agccccgact actccaagtg tcggttcaag cgtaccatca agttccacgc tgaaaagatc 4800
   taagtggtaa tcgaccgact aaccattttt agctgacaaa cacttgctaa ctcctataac 4860
   gaatgaatga ctaacttggc atattgttac caagtattac ttgggatata gttgagtgta 4920
   accattgcta agaatccaaa ctggagcttc taaaggtctg ggagtcgccg tatgtgttca 4980
   tatcgaaatc aaagaaatca taatcgcaac agaattcaaa atcaagcaga ttaatatcca 5040
   ttattgtact cggatcgtga catatctgat atgatctcgg atatgatctc tgactgttta 5100
   ctgggagatt tgttgaagat ttgttgaggt tatctgaaaa gtagacaata gagacaaaat 5160
   gacgatatca agaactgaat cgggccgaaa tactcggtat cattcccttc agcagtaact 5220
   gtattgctct atcaatgcga cgagatacct ccacaattaa tactgtatac gctctaccac 5280
   tcatatctcc aatgctaaaa tatattcatg cccaggacct ctgtgcactg ctatgcagca 5340
   cagtgttgtc gattgaattg gtcgtgtctg gtccctgatg ctctgtgtct cgctgactag 5400
   tccttccatc cagacctcgt cattatctga taggcaacaa gttctgctct ctcacaccct 5460
   gccgacacaa gggacactcg ggcttctctc tcacccattc ggaaatacag tccttaatta 5520
   agttgcgaca catgtcttga tagtatcttg aattctctct cttgagcttt tccataacaa 5580
   gttcttctgc ctccaggaag tccatgggtg gtttgatcat ggttttggtg tagtggtagt 5640
   gcagtggtgg tattgtgact ggggatgtag ttgagaataa gtcatacaca agtcagcttt 5700
   cttcgagcct catataagta taagtagttc aacgtattag cactgtaccc agcatctccg 5760
   tatcgagaaa cacaacaaca tgccccattg gacagatcat gcggatacac aggttgtgca 5820
   gtatcataca tactcgatca gacaggtcgt ctgaccatca tacaagctga acaagcgctc 5880
   catacttgca cgctctctat atacacagtt aaattacata tccatagtct aacctctaac 5940
   agttaatctt ctggtaagcc tcccagccag ccttctggta tcgcttggcc tcctcaatag 6000
   gatctcggtt ctggccgtac agacctcggc cgacaattat gatatccgtt ccggtagaca 6060
   tgacatcctc aacagttcgg tactgctgtc cgagagcgtc tcccttgtcg tcaagaccca 6120
   ccccgggggt cagaataagc cagtcctcag agtcgccctt aggtcggttc tgggcaatga 6180
   agccaaccac aaactcgggg tcggatcggg caagctcaat ggtctgcttg gagtactcgc 6240
   cagtggccag agagcccttg caagacagct cggccagcat gagcagacct ctggccagct 6300
   tctcgttggg agaggggact aggaactcct tgtactggga gttctcgtag tcagagacgt 6360
   cctccttctt ctgttcagag acagtttcct cggcaccagc tcgcaggcca gcaatgattc 6420
   cggttccggg tacaccgtgg gcgttggtga tatcggacca ctcggcgatt cggtgacacc 6480
   ggtactggtg cttgacagtg ttgccaatat ctgcgaactt tctgtcctcg aacaggaaga 6540
   aaccgtgctt aagagcaagt tccttgaggg ggagcacagt gccggcgtag gtgaagtcgt 6600
   caatgatgtc gatatgggtt ttgatcatgc acacataagg tccgacctta tcggcaagct 6660
   caatgagctc cttggtggtg gtaacatcca gagaagcaca caggttggtt ttcttggctg 6720
   ccacgagctt gagcactcga gcggcaaagg cggacttgtg gacgttagct cgagcttcgt 6780
   aggagggcat tttggtggtg aagaggagac tgaaataaat ttagtctgca gaacttttta 6840
   tcggaacctt atctggggca gtgaagtata tgttatggta atagttacga gttagttgaa 6900
   cttatagata gactggacta tacggctatc ggtccaaatt agaaagaacg tcaatggctc 6960
   tctgggcgtc gcctttgccg acaaaaatgt gatcatgatg aaagccagca atgacgttgc 7020
   agctgatatt gttgtcggcc aaccgcgccg aaaacgcagc tgtcagaccc acagcctcca 7080
   acgaagaatg tatcgtcaaa gtgatccaag cacactcata gttggagtcg tactccaaag 7140
   gcggcaatga cgagtcagac agatactcgt cgaccttttc cttgggaacc accaccgtca 7200
   gcccttctga ctcacgtatt gtagccaccg acacaggcaa cagtccgtgg atagcagaat 7260
   atgtcttgtc ggtccatttc tcaccaactt taggcgtcaa gtgaatgttg cagaagaagt 7320
   atgtgccttc attgagaatc ggtgttgctg atttcaataa agtcttgaga tcagtttggc 7380
   cagtcatgtt gtggggggta attggattga gttatcgcct acagtctgta caggtatact 7440
   cgctgcccac tttatacttt ttgattccgc tgcacttgaa gcaatgtcgt ttaccaaaag 7500
   tgagaatgct ccacagaaca caccccaggg tatggttgag caaaaaataa acactccgat 7560
   acggggaatc gaaccccggt ctccacggtt ctcaagaagt attcttgatg agagcgtatc 7620
   gatgagccta aaatgaaccc gagtatatct cataaaattc tcggtgagag gtctgtgact 7680
   gtcagtacaa ggtgccttca ttatgccctc aaccttacca tacctcactg aatgtagtgt 7740
   acctctaaaa atgaaataca gtgccaaaag ccaaggcact gagctcgtct aacggacttg 7800
   atatacaacc aattaaaaca aatgaaaaga aatacagttc tttgtatcat ttgtaacaat 7860
   taccctgtac aaactaaggt attgaaatcc cacaatattc ccaaagtcca cccctttcca 7920
   aattgtcatg cctacaactc atataccaag cactaaccta ccgttt 7966
<210> 88
   <211> 20
   <212> DNA
   <213> Artificial Sequence
<220>
   <223> Primer Pex-10dell 3'.Forward
<400> 88
   ccaacatgag cgacaatacg 20
<210> 89
   <211> 20
   <212> DNA
   <213> Artificial Sequence
<220>
   <223> Primer Pex-10del2 5'.Reverse
<400> 89
   caagttctgc tctctcacac 20